# EUROPEAN PATENT SPECIFICATION

(11) **EP 2 646 571 B1**
(45) Date of publication and mention of the grant of the patent: **01.07.2015**
(21) Application number: 11790625.5
(22) Date of filing: 01.12.2011
(51) Int. Cl.: C12Q 1/68

(54) **METHOD FOR PREDICTING THE OUTCOME OF COLON CANCER BY ANALYSING MIRNA EXPRESSION**
VERFAHREN ZUR VORHERSAGE DES ERGEBNISSES EINES KOLONKARZINOMAS DURCH ANALYSE DER MIRNA-EXPRESSION
PROCÉDÉ DE PRÉDICTION DE L'ISSUE D'UN CANCER DU CÔLON PAR L'ANALYSE DE L'EXPRESSION DE MIARN

(30) Priority: 01.12.2010 EP 10306329; 20.01.2011 US 201161434498 P
(43) Date of publication of application: 09.10.2013
(60) Divisional application: 15168304.2
(73) Proprietor: INSERM (Institut National de la Santé et de la Recherche Médicale), 75013 Paris Cédex 13 (FR); Université Paris Descartes, 75006 Paris (FR); Assistance Publique-Hôpitaux de Paris (APHP), 75004 Paris (FR)
(72) Inventor: GALON, Jérôme, 75006 Paris (FR); MLECNIK, Bernhard, 75006 Paris (FR); PAGES, Franck, 75006 Paris (FR); FRIDMAN, Hervé, 75006 Paris (FR)
(74) Representative: Cabinet Plasseraud
(86) International application number: PCT/EP2011/071530
(87) International publication number: WO 2012/072750

(56) References cited:
- WO-A2-2006/137941
- WO-A2-2008/008430
- WO-A2-2009/008720
- WO-A2-2009/111643
- WO-A2-2009/140670
- WO-A2-2010/058393
- DÍAZ RAQUEL ET AL: "Deregulated expression of miR-106a predicts survival in human colon cancer patients.", GENES, CHROMOSOMES & CANCER SEP 2008 LNKD- PUBMED:18521848, vol. 47, no. 9, September 2008 (2008-09), pages 794-802, XP002629635, ISSN: 1098-2264
- SCHETTER AARON J ET AL: "MicroRNA expression profiles associated with prognosis and therapeutic outcome in colon adenocarcinoma", JAMA THE JOURNAL OF THE AMERICAN MEDICAL ASSOCIATION, AMERICAN MEDICAL ASSOCIATION, US, vol. 299, no. 4, 30 January 2008 (2008-01-30), pages 425-436, XP002529936, ISSN: 0098-7484, DOI: DOI:10.1001/JAMA.299.4.425 cited in the application
- LIU M ET AL: "The role of microRNAs in colorectal cancer", JOURNAL OF GENETICS AND GENOMICS, ELSEVIER BV, NL; CN, vol. 37, no. 6, 1 June 2010 (2010-06-01), pages 347-358, XP027119586, ISSN: 1673-8527 [retrieved on 2010-06-01]
- SCHEPELER TROELS ET AL: "Diagnostic and prognostic microRNAs in stage II colon cancer", CANCER RESEARCH, AMERICAN ASSOCIATION FOR CANCER RESEARCH, US, vol. 68, no. 15, 1 August 2008 (2008-08-01), pages 6416-6424, XP002529939, ISSN: 0008-5472, DOI: 10.1158/0008-5472.CAN-07-6110
- CUMMINS JORDAN M ET AL: "The colorectal microRNAome", PROCEEDINGS OF THE NATIONAL ACADEMY OF SCIENCES OF THE UNITED STATES (PNAS), NATIONAL ACADEMY OF SCIENCE, US, vol. 103, no. 10, 7 March 2006 (2006-03-07), pages 3687-3692, XP002539256, ISSN: 0027-8424, DOI: DOI:10.1073/PNAS.0511155103 cited in the application
- ZHAOHUI HUANG ET AL: "Plasma microRNAs are promising novel biomarkers for early detection of colorectal cancer", INTERNATIONAL JOURNAL OF CANCER, vol. 127, no. 1, 28 October 2009 (2009-10-28), pages 118-126, XP55024209, ISSN: 0020-7136, DOI: 10.1002/ijc.25007
- BANDRÉS E ET AL: "Identification by Real-time PCR of 13 mature microRNAs differentially expressed in colorectal cancer and non-tumoral tissues", MOLECULAR CANCER, BIOMED CENTRAL, LONDON, GB, vol. 5, no. 1, 19 July 2006 (2006-07-19), page 29, XP021018291, ISSN: 1476-4598, DOI: 10.1186/1476-4598-5-29
- HOL L ET AL: "W1985 MicroRNA Expression Profiling of Colorectal Cancer and Its Precancerous Lesions Using Lna(TM) Oligonucleotide Arrays", GASTROENTEROLOGY, ELSEVIER, PHILADELPHIA, PA, vol. 134, no. 4, 1 April 2008 (2008-04-01) , pages A-748, XP023435289, ISSN: 0016-5085, DOI: 10.1016/S0016-5085(08)63492-7 [retrieved on 2008-04-01]

## Description

### FIELD OF THE INVENTION:

The present invention relates to a method for predicting the outcome of a cancer.

### BACKGROUND OF THE INVENTION:

Cancer remains a serious public health problem in developed countries. Accordingly, to be most effective, cancer treatment requires not only early detection and treatment or removal of the malignancy, but a reliable assessment of the severity of the malignancy and a prediction of the likelihood of cancer recurrence. The stage of a cancer indicates how far a cancer has spread and as consequences what the oucome of the canser will be. Staging is important because treatment is often decided according to the stage of a cancer. To date, cancers are generally classified according to the UICC-TNM system. The TNM (for "Tumor-Node-Metastasis") classification system uses the size of the tumor, the presence or absence of tumor in regional lymph nodes, and the presence or absence of distant metastases, to assign a stage and an outcome to the tumor. The TNM system developed from the observation that patients with small tumours have better prognosis than those with tumours of greater size at the primary site. In general, patients with tumours confined to the primary site have better prognosis than those with regional lymph node involvement, which in turn is better than for those with distant spread of disease to other organs. Accordingly, cancer can be generally divided into four stages. Stage I is very localized cancer with no cancer in the lymph nodes. Stage II cancer has spread to the lymph nodes. Stage III cancer has spread near to where the cancer started. Stage IV cancer has spread to another part of the body. The assigned stage is used as a basis for selection of appropriate therapy and for prognostic purposes.

The above clinical classifications, although they are to be useful, are imperfect and do not allow a reliable prognosis of the outcome of the cancers. Recently, a study has suggested that density, and location of immune cells in colorectal cancer had a prognostic value that was superior to and independent of those of the UICC-TNM classification (Science. 2006 Sep 29;313(5795):1960-4). However there is still a need for other reliable methods that will help physicians for predicting the outcome of a cancer in a patient.

miRNAs have been suggested to be helpful for the diagnosis and predicting the outcome of a cancer. For example Cummins et al. (2006) describe miRNAs that are differentially expressed in colon cancer and some that are associated with clinical outcome (Cummins et al, Proc. Natl. Acad. Sci. USA, 103 (10):3687-3692, 2006). Michael et al. (2003) describe miRNAs that are down-regulated in colorectal adenocarcinomas as compared to matched normal tissues, in particular miR.143 and miR145 (Michael et al, Mol. Cancer. Res., 1:882-891, 2003). Bandres et al. (2006) describe miRNA in paired colorectal tumor and normal adjacent tissue and reported the differential expression of miR.31 in Stage II and Stage IV colorectal cancer cells (Bandres et al, Molec. Cancer, 5:29, 2006. Xi et al. (2006a, 2006b)), evaluated the prognostic value of several miRNAs in colorectal cancer and showed that higher expression of miR.200c was associated with shorter survival time (Xi et al, Biomarklnsights, 2:113-121, 2006a. Xi et al, Clin Cancer Res., 12:2014-2024, 2006b). Schetter et al., (2008) reported that a high expression of miR.21 is associated with a poor survival and poor therapeutic response in colorectal cancer patients (Schetter et al, JAMA, 299(4):425-436, 2008).

However, there is no current method based on miRNAs that allow having a prognostic value that is superior to and independent of those of the UICC-TNM classification.

### SUMMARY OF THE INVENTION:

The present invention relates to a method for predicting the outcome of a colorectal cancer in a patient comprising a step consisting of determining the expression level of a miRNA cluster in a sample obtained from said patient, wherein said miRNA cluster comprises miR.609. In particular the present invention is defined by the claims.

### DETAILED DESCRIPTION OF THE INVENTION:

From a cohort of 77 Stage I/II/III patients the inventors have determined various miRNA clusters that can be suitable for predicting the outcome of a colorectal cancer in a patient (Table 1 to Table 17). They demonstrated, by determining the ROC curves for each of said clusters that most of them provide greater sensitivities and selectivities than the UICC-TNM classification does for predicting the outcome of a colorectal cancer.

The term "miRNAs" refers to microRNA molecules that are generally 21 to 22 nucleotides in length, even though lengths of 19 and up to 23 nucleotides have been reported. miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved in animals by a ribonuclease Ill-like nuclease enzyme called Dicer. The processed miRNA is typically a portion of the stem. The processed miRNA (also referred to as "mature miRNA") become part of a large complex to down-regulate a particular target gene. All the miRNAs pertaining to the invention are known per se and sequences of them are publicly available from the data base http://microrna.sanger.ac.uk/sequences/. The miRNAs of the invention are listed in Table A:

**Table A: list of the miRNAs according to the invention**

| **miRNA** | **miRBase** |
|---|---|
| miR.609 | MI0003622 |
| miR.519b | MI0003151 |
| miR.520f | MI0003146 |
| miR.558 | MI0003564 |
| miR.603 | MI0003616 |
| miR.220a | MI0000297 |
| miR.376a* | MI0000784 |
| miR.639 | MI0003654 |
| miR.130a | MI0000448 |
| miR.338 | MI0000814 |
| miR.26a | MI0000083 |
| miR.29a | MI0000087 |
| miR.494 | MI0003134 |
| miR.518c | MI0003159 |
| miR.660 | MI0003684 |
| miR.369-3p | MI0000777 |
| miR.362 | MI0000762 |

The term "miRNA cluster" refers to a set of at least one miRNA selected from the group consisting in the miRNAs of Table A. Accordingly, said miRNA cluster may comprise 1, 2, 3, 4, 5, 6, 7, 8, 9, 10, 11, 12, 13, 14, 15, 16, or 17 miRNAs of Table A.

As used herein, the term "patient" denotes a mammal. In a preferred embodiment of the invention, a patient according to the invention is a human.

As used herein, the term "sample" refers to a sample that contains nucleic acid materials.

As used herein, the term "nucleic acid" is meant a polymeric compound comprising nucleoside or nucleoside analogs which have nitrogenous heterocyclic bases, or base analogs, linked together by nucleic acid backbone linkages (e.g., phosphodiester bonds) to form a polynucleotide. Conventional RNA and DNA are included in the term "nucleic acid" as are analogs thereof. The nucleic acid backbone may include a variety of linkages, for example, one or more of sugar-phosphodiester linkages, peptide-nucleic acid bonds, phosphorothioate or methylphosphonate linkages or mixtures of such linkages in a single oligonucleotide. Sugar moieties in the nucleic acid may be either ribose or deoxyribose, or similar compounds with known substitutions. Conventional nitrogenous bases (A, G, C, T, U), known base analogs (eg inosine), derivatives of purine or pyrimidine bases and "abasic" residues (i.e., no nitrogenous base for one or more backbone positions) are included in the term nucleic acid. That is, a nucleic acid may comprise only conventional sugars, bases and linkages found in RNA and DNA, or may include both conventional components and substitutions (e.g., conventional bases and analogs linked via a methoxy backbone, or conventional bases and one or more base analogs linked via an RNA or DNA backbone).

Typically the "sample" means any tissue sample derived from the patient. Said tissue sample is obtained for the purpose of the in vitro evaluation. The sample can be fresh, frozen, fixed (e.g., formalin fixed), or embedded (e.g., paraffin embedded). In a particular embodiment the sample results from biopsy performed in the tumour sample of the patient. For example an endoscopical biopsy performed in the bowel of the patient affected by a colorectal cancer.

### miRNA clusters based on miR.609:

The invention relates to a method for predicting the outcome of a colorectal cancer in a patient comprising a step consisting of determining the expression level of a miRNA cluster in a sample obtained from said patient, wherein said miRNA cluster comprises miR.609.

The inventors have indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.520f, miR.558, miR.603, miR.220a, miR.376a*, miR.639, miR.130a, miR.338, miR.26a, miR.29a, miR.494, miR.518c, miR.660, miR.369-3p and miR.362.

In a particular embodiment, the miRNA cluster consists in the combination of 2 miRNAs. For example, said miRNA cluster may be selected according to any combination described in Table 2 which comprises miR.609. In a particular embodiment, the miRNA cluster consists in the combination miR.609 + miR.29a.

In a particular embodiment, the miRNA cluster consists in the combination of 3 miRNAs. For example, said miRNA cluster may be selected according to any combination described in Table 3 which comprises miR.609. In a particular embodiment, the miRNA cluster consists in the combination miR.558 + miR.609 + miR.518c.

In a particular embodiment, the miRNA cluster consists in the combination of 4 miRNAs. For example, said miRNA cluster may be selected according to any combination described in Table 4 which comprises miR.609. In a particular embodiment, the miRNA cluster consists in the combination miR.609 + miR.29a + miR.376a* + miR.518c.

In a particular embodiment, the miRNA cluster consists in the combination of 5 miRNAs. For example, said miRNA cluster may be selected according to any combination described in Table 5 which comprises miR.609. In a particular embodiment, the miRNA cluster consists in the combination miR.558 + miR.609 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.609. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.609. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.609. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.609. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.609. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.609. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.609. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.609. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.609. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.609. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.609. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a*+ miR.494 + miR.518c + miR.639 + miR.660 + miR.338.

### Techniques for measuring the expression level of the miRNA clusters:

Measuring the expression level of the miRNA clusters of the invention in the sample obtained form the patient can be performed by a variety of techniques.

For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted miRNAs is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Other methods of Amplification include ligase chain reaction (LCR), transcription-mediated amplification (TMA), strand displacement amplification (SDA) and nucleic acid sequence based amplification (NASBA).

In a particular embodiment, the determination comprises contacting the sample with selective reagents such as probes or primers and thereby detecting the presence, or measuring the amount of miRNAs originally in the sample. Contacting may be performed in any suitable device, such as a plate, microtiter dish, test tube, well, glass, column, and so forth In specific embodiments, the contacting is performed on a substrate coated with the reagent, such as a miRNA array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a miRNAs hybrid, to be formed between the reagent and the miRNAs of the sample.

Nucleic acids exhibiting sequence complementarity or homology to the miRNAs of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

The probes and primers are "specific" to the miRNAs they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Accordingly, the present invention concerns the preparation and use of miRNA arrays or miRNA probe arrays, which are macroarrays or microarrays of nucleic acid molecules (probes) that are fully or nearly complementary or identical to a plurality of miRNA molecules positioned on a support or support material in a spatially separated organization. Macroarrays are typically sheets of nitrocellulose or nylon upon which probes have been spotted. Microarrays position the nucleic acid probes more densely such that up to 10,000 nucleic acid molecules can be fit into a region typically 1 to 4 square centimeters. Microarrays can be fabricated by spotting nucleic acid molecules, e.g., genes, oligonucleotides, etc., onto substrates or fabricating oligonucleotide sequences in situ on a substrate. Spotted or fabricated nucleic acid molecules can be applied in a high density matrix pattern of up to about 30 non-identical nucleic acid molecules per square centimeter or higher, e.g. up to about 100 or even 1000 per square centimeter. Microarrays typically use coated glass as the solid support, in contrast to the nitrocellulose-based material of filter arrays. By having an ordered array of miRNA-complementing nucleic acid samples, the position of each sample can be tracked and linked to the original sample. A variety of different array devices in which a plurality of distinct nucleic acid probes are stably associated with the surface of a solid support are known to those of skill in the art. Useful substrates for arrays include nylon, glass, metal, plastic, latex, and silicon. Such arrays may vary in a number of different ways, including average probe length, sequence or types of probes, nature of bond between the probe and the array surface, e.g. covalent or non-covalent, and the like.

After an array or a set of miRNA probes is prepared and/or the miRNA in the sample or miRNA probe is labeled, the population of target nucleic acids is contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook et al. (2001). Of particular interest in many embodiments is the use of stringent conditions during hybridization. Stringent conditions are known to those of skill in the art.

Alternatively, miRNAs quantification method may be performed by using stem-loop primers for reverse transcription (RT) followed by a real-time TaqMan® probe. Typically, said method comprises a first step wherein the stem-loop primers are annealed to miRNA targets and extended in the presence of reverse transcriptase. Then miRNA-specific forward primer, TaqMan® probe, and reverse primer are used for PCR reactions. Quantitation of miRNAs is estimated based on measured CT values.

Many miRNA quantification assays are commercially available from Qiagen (S. A. Courtaboeuf, France) or Applied Biosystems (Foster City, USA).

Expression level of a miRNA may be expressed as absolute expression level or normalized expression level. Typically, expression levels are normalized by correcting the absolute expression level of a miRNA by comparing its expression to the expression of a mRNA that is not a relevant for determining the outcome of the cancer in the patient, e.g., a housekeeping mRNA that is constitutively expressed. Suitable mRNA for normalization include housekeeping mRNAs such as the U6, U24, U48 and S18. This normalization allows the comparison of the expression level in one sample, e.g., a patient sample, to another sample, or between samples from different sources.

### Reference levels

It is specifically contemplated that the invention can be used for predicting the outcome of a colorectal cancer in a patient.

Therefore the methods of the invention further comprises a step consisting of comparing the expression level of one miRNA cluster (as above described) determined in the sample of the patient with a reference expression level of said miRNA cluster, wherein a difference between said expression levels is indicative of the outcome of the colorectal cancer in the patient.

Typically, the reference expression levels according to the invention are correlated with an outcome. The reference expression levels may thus consist in the expression level of said miRNA cluster in a tissue representative of an outcome. Accordingly, the reference levels may be predetermined by carrying out a method comprising the steps of a) providing at least one collection of tumor tissue samples selected from the group consisting of a collection of tumor tissue samples from cancer patients having different outcomes, b) determining for each tumor tissue sample comprised in a collection of tumor tissue samples provided at step a), the expression level of said miRNA clusters.

Typically, the expression level determined for one miRNA cluster may be also expressed as a score. Typically said score may be obtained by i) determining for every gene of the cluster their expression level in the sample ii) allocating for every miRNA a positive coefficient (e.g. +1) when the miRNA is associated with a good prognosis and a negative coefficient (e.g. -1) when the miRNA is associated with a bad prognosis and iii) multiplying the expression level of one miRNA with the coefficient allocated in step ii) and iv) adding up all values determining at step iii) for obtaining said score. Typically a coefficient of +1 is allocated to the miRNAs associated with a good prognosis, namely miR.609, miR.519b, miR.520f, miR.558, miR.603 and miR.220a and a coefficient of -1 is allocated to the genes associates with a bad prognosis, namely miR.376a*, miR.639, miR.130a, miR.338, miR.26a, miR.29a, miR.494, miR.518c, miR.660, miR.369-3p and miR.362. Preferably, the expression levels are expressed as normalized expression levels as above described.

The advantage of said score is to make easier the comparison step with the reference levels that may be expressed as "cut-off values". For example the reference ("cut-off') value represents the score calculated for the miRNA cluster of interest in a tissue sample representative of a specific colorectal cancer outcome. The cut-off values may also be predetermined by carrying out a method comprising the steps of:
a) selecting a miRNA cluster for which a reference value is to be determined;
b) providing a collection of tumor tissue samples from cancer patients;
c) providing, for each tumor sample provided at step b), information relating to the actual clinical outcome for the corresponding cancer patient;
d) providing a serial of arbitrary values for said miRNA cluster obtained at step a)
e) calculated the score of said miRNA cluster provided at step a) for each tumor tissue sample contained in the collection provided at step b)
f) classifying said tumor samples in two groups for one specific arbitrary value provided at step c), respectively :
   (i) a first group comprising tumor samples that exhibit a score for said miRNA cluster that is lower than the said arbitrary value contained in the said serial of values;
   (ii) a second group comprising tumor samples that exhibit a score for said miRNA cluster that is higher than said arbitrary value contained in the said serial of values;
   whereby two groups of tumor samples are obtained for the said specific value, wherein the tumors samples of each group are separately enumerated;
g) calculating the statistical significance between (i) the score for said miRNA cluster obtained at step e) and (ii) the actual clinical outcome of the patients from which tumor samples contained in the first and second groups defined at step f) derive;
h) reiterating steps f) and g) until every arbitrary value provided at step d) is tested;
i) setting the said reference value ("cut-off" value) as consisting of the arbitrary value for which the highest statistical significance (most significant) has been calculated at step g). As it is disclosed above, said method allows the setting of a single "cut-off" value permitting discrimination between bad and good outcome prognosis. Practically, high statistical significance values (e.g. low P values) are generally obtained for a range of successive arbitrary values, and not only for a single arbitrary value. Thus, in one alternative embodiment of the method of determining "cut-off" values above, a minimal statistical significance value (minimal threshold of significance, e.g. maximal threshold P value) is arbitrarily set and the range of arbitrary values for which the statistical significance value calculated at step g) is higher (more significant, e.g. lower P value) are retained, whereby a range of values is provided. Said range of values consist of a "cut-off" value according to the invention. According to this specific embodiment of a "cut-off" value, bad or good clinical outcome prognosis can be determined by comparing the score obtained for the miRNA cluster with the range of values delimiting said "cut-off" value. In certain embodiments, a cut-off value consisting of a range of values for the considered miRNA cluster, consists of a range of values centred on the value for which the highest statistical significance value is found (e.g. generally the minimum P value which is found). Typically, the cut-off values as above described are determined from a cohort of patient that has a sufficient size enough for allowing a reproducible and accurate discrimination between patients with good prognosis and patients with bad prognosis.

In particular embodiment, the cut-off values as described above may be reported in a table, so that the physician can compare the score obtained for a patient with said values and can easily determined the cancer outcome for said patient.

### Colorectal Cancer:

In a particular embodiment, the colorectal cancer is at Stage I, II, III, or IV as determined by the TNM classification, but however the present invention is accurately useful for predicting the outcome of the colorectal cancer when said colorectal cancer has been classified as Stage I, II or III by the TNM classification.

Methods of the invention can be applied for monitoring the treatment (e.g., drug compounds) of the patient. For example, the effectiveness of an agent to affect the expression level of the miRNA cluster (as herein after described) according to the invention can be monitored during treatments of patients receiving anti-cancer treatments.

The "anti-cancer treatment" that is referred to in the definition of step a) above relate to any type of cancer therapy undergone by the cancer patients previously to collecting the tumor tissue samples, including radiotherapy, chemotherapy and surgery, e.g. surgical resection of the tumor.

Accordingly, the present disclosure also relates to a method for monitoring the treatment of patient affected with a colorectal cancer, said method comprising the steps consisting of:
i) predicting the outcome of said cancer before said treatment by performing the method of the invention
ii) predicting the outcome of said cancer after said treatment by performing the method of the invention
iii) and comparing the outcome predicted a step i) with the outcome predicted at step ii) wherein a difference between said outcomes is indicative of the effectiveness of the treatment.

The present disclosure also relates to a method for predicting the outcome of a colorectal cancer in a patient, wherein said method may be used independently from conventional clinicopatholological cancer staging methods, and which method comprising determining the expression level of a miRNA cluster according to the invention.

The invention will be further illustrated by the following figures and examples. However, these examples and figures should not be interpreted in any way as limiting the scope of the present invention.

### FIGURES:

**Figure 1****: DFS KM curves for the two clusters. Cluster1 and Cluster 2 with good outcome are merged.** The hazard ratio (HR) is 10.1, the concordance index (CI) is 70.2 and the Brier Score (BS) is 0.23.
**Figure 2****: Pearson correlation matrix for the final selected 17 miRNAs for the deltaCt qPCR data on 77 patients, clustered using euclidean distance.**
**Figure 3****: DFS KM curves based on the dichotomized linear predictor of the fitted Cox model for all 17 miRNAs at 0 months follow-up.** The all over hazard ratio (HR) is 5.12 (logrank P value < 0.001) and the Brier Score (BS) is 0.205.

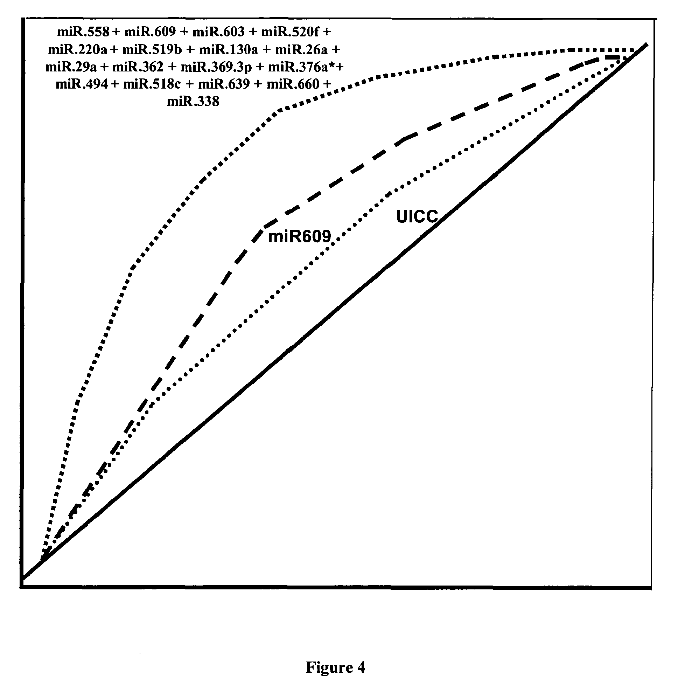

**Figure 4****: ROC curves for the final selected 17 miRNAs, miR609, and UICC.**

### EXAMPLE:

### Material & Methods

**Patients and data:** The records of colorectal cancer (CRC) patients who underwent a primary resection of their tumor at the Laennec-HEGP Hospitals between 1996 and 2004 were reviewed and previously described (Galon et al. 2006). All frozen tumor samples (n=77) from UICC-TNM Stage I-III patients available from Laennec-HEGP Hospitals from 1996-2004, with sufficient RNA quality and quantity, were selected. The RNA samples analyzed were from 77 different patients. These patients were used for miRNA expression experiments (Taqman qPCR). The observation time in the cohorts was the interval between diagnosis and last contact (death or last follow-up). Data were censored at the last follow-up for patients without relapse, or death. The min:max values until progression/death or last follow-up were (0:136) months, respectively. Eight patients with incomplete data records were excluded from the analysis. Time to recurrence or disease-free time was defined as the interval from the date of surgery to confirmed tumor relapse date for relapsed patients and from the date of surgery to the date of last follow-up for disease-free patients.

Histopathological and clinical findings were scored according to the UICC-TNM staging system. Follow-up data were collected prospectively and updated. A secure Web-based database, TME.db (Tumor MicroEnvironment Database), was built on a 3-tier architecture using Java-2 Enterprise-Edition (J2EE) to integrate the clinical data and the data from high-throughput technologies. Ethical, Legal and Social Implications were approved by ethical review board.

**Statistical analysis:** All signatures were build based on 69 patients (8 patients were removed due to missing values) from a UICC-TNM Stage I-III CRC patient cohort and all 17 selected final miRs. The predictive performance for each miRNA combination was assessed by the Harrel's concordance index (c-index) and time-dependent c-index (C_{τ} index) (R risksetROC package) and correspond to the area under the receiver operator curve (AUC) (c-index (Harrell FE et al. 1996) and iAUC (Heagerty PJ et al., 2005), respectively). A summary table for each possible model combination length from 1 to 16 miRs was created and sorted by the highest C_{τ} index. Additionally each table shows the number of miRNAs remaining significant in the cox model and a P-value (U-statistics, Pencina MJ et al., 2008) comparing the performance of all models of a certain number of miRNAs with the one with the highest performance (largest C_{τ} index) (R risksetROC and Hmisc package).

Time-dependent area under the ROC(t) (iAUC): Since the event occurrence is time-dependent, time-dependent ROC curves are more appropriate than conventional ones (Heagerty PJ et al., 2005). Heagerty PJ et al. proposed to summarize the discrimination potential of a risk score, estimated at the diagnosis/surgery time t=0, by calculating ROC curves for cumulative event occurrence by time t. From the ROC curve ROC(t) the integrated area under the curve over time (iAUC) can be calculated. The larger the iAUC at time t, the better is the predictability of the time to event (TTE) at time t as well as the average predictability of the TTE.

The concordance index (c-index): The concordance index (c-index) calculates the probability that, for a pair of randomly chosen comparable patients, the patient with the higher risk prediction will experience an event before the lower risk patient. The c-index is a generalization of the AUC(t) (iAUC), an can not represent the evolution of performance with respect to time (Harrell FE et al., 1996). The larger c-index, the better is the predictability of the time to event (TTE).

### Results

**Selection of the relevant miRNAs:** In a cohort of 77 Stage I/II/III patients for 365 micro-RNAs (miRNAs) we determined the logrank significance of each single marker using the minimal P-value approach for the dichotomization. The obtained P-values were corrected using Altman et al. (Altman, D. G., Lausen, B., Sauerbrei, W. & Schumacher, M. Dangers of using "optimal" cutpoints in the evaluation of prognostic factors. J Natl Cancer Inst, 1994;86:829-35.) and were additionally cross validated. The Hazard ratios obtained with the minimal P-value dichotomization were corrected using Hollaender et al. (Hollaender N, Sauerbrei W, Schumacher M. Confidence intervals for the effect of a prognostic factor after selection of an 'optimal' cutpoint. Stat Med, 2004;23(11):1701-13.). The final selection of the miRNAs was based on the median cross validation P-value. We removed from the final miRNA signature miRNAs which were not expressed (none of the determined values were larger than deltaCt > 32) although they where logrank significant. We finally end up with 17 markers, where 6 show a good out come when high expressed and 11 show a bad outcome when high expressed (see Table B). All analyzes were performed using the R survival package.

**Table B: Final selected miRNAs**

| **Marker** | **median Hazard ratio (HR)** | **median logrank P value** | **optimal Hazard ratio (HR)** | **optimal Hazard ratio corrected** | **optimal logrank P value** | **optimal logrank P value corrected** | **optimal logrank P value cross validated** |
|---|---|---|---|---|---|---|---|
| **miR.609** | 0.239 | 0.0057 | 0.194 | 0.24 | 0.0012 | 0.0576 | 0.0276 |
| **miR.519b** | 0.312 | 0.0209 | 0.251 | 0.31 | 0.0033 | 0.1359 | 0.0431 |
| **miR.520f** | 0.321 | 0.0217 | 0.252 | 0.31 | 0.0016 | 0.0731 | 0.0651 |
| **miR.558** | 0.334 | 0.0198 | 0.137 | 0.19 | 0.0019 | 0.0866 | 0.0389 |
| **miR.603** | 0.337 | 0.0213 | 0.132 | 0.18 | 0.0014 | 0.0682 | 0.0299 |
| **miR.220a** | 0.351 | 0.029 | 0.144 | 0.20 | 0.0027 | 0.1162 | 0.0531 |
| **miR.376a*** | 1.199 | 0.6935 | 3.812 | 3.23 | 0.0023 | 0.1022 | 0.0336 |
| **miR.639** | 1.339 | 0.5253 | 6.338 | 4.55 | 0.0047 | 0.1774 | 0.0231 |
| **miR.130a** | 1.635 | 0.2868 | 6.545 | 4.76 | 0.0041 | 0.1596 | 0.0316 |
| **miR.338** | 1.687 | 0.2861 | 4.863 | 3.70 | 0.0005 | 0.0278 | 0.0146 |
| **miR.26a** | 1.861 | 0.1857 | 5.04 | 3.85 | 0.0045 | 0.1707 | 0.0416 |
| **miR.29a** | 2.562 | 0.0483 | 7.257 | 5.26 | 0.0019 | 0.0877 | 0.0547 |
| **miR.494** | 3.333 | 0.0149 | 4.415 | 3.57 | 0.0042 | 0.1616 | 0.0406 |
| **miR.518c** | 3.514 | 0.0103 | 4.694 | 3.85 | 0.0011 | 0.0563 | 0.0214 |
| **miR.660** | 3.743 | 0.0119 | 4.67 | 3.85 | 0.0026 | 0.1118 | 0.054: |
| **miR.369-3p** | 4.533 | 0.0033 | 5.934 | 4.76 | 0.0013 | 0.0649 | 0.012 |
| **miR.362** | 4.634 | 0.0028 | 4.899 | 4.00 | 0.0018 | 0.0818 | 0.0215 |

6 miRNAs show a good out come (HR < 1) when high expressed: miR.609, miR.519b, miR.520f, miR.558, miR.603 and miR.220a.

11 miRNAs show a bad outcome (HR > 1) when high expressed: miR.376a*, miR.639, miR.130a, miR.338, miR.26a, miR.29a, miR.494, miR.518c, miR.660, miR.369-3p and miR.362.

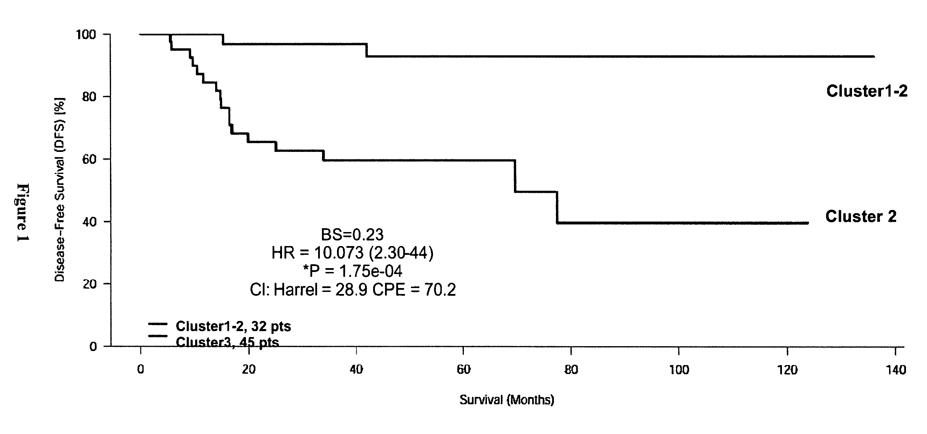

**Cluster for the 17 final selected signature miRNA qPCR data clustered on 77 patients (Stage I-III):** Before clustering the deltaCt qPCR data for each miRNA was mean centered and divided by its standard deviation. The miRNAs were hierarchical clustered using the complete linkage algorithm and Pearson correlation as distance measurement. Three major patient clusters were defined, two with good outcome (Cluster1-2) and one with poor outcome (Cluster3) (Figure 1).

**Time-dependent ROC Curves and iAUC (Time-dependent predictive accuracy) using Cox regression models for disease-free survival (DFS):** In the cohorts of Stage I/II, Stage I/II/III and Stage III patients using the 17 final selected micro-RNAs (miRNAs) as well as Version1 and Version2 of the clustered data we determined the time-dependent predictive accuracy (integrated Area Under the ROC curves, iAUC) (Heagerty PJ, Zheng Y. Survival model predictive accuracy and ROC curves. Biometrics, 2005, Mar;61(1):92-105.)

All signatures show stable time-dependent ROC curves and none of the signatures violate the cox proportional hazards assumption.

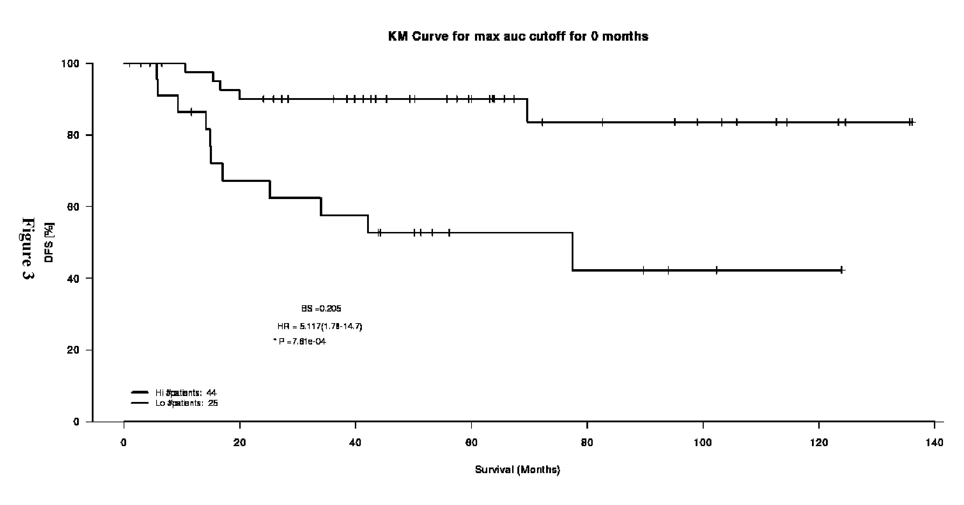

The ROC and AUC curves were drawn based on the linear predictor of a fitted Cox regression model for the respective miRNA or miRNA combination were the miRNA data entered un-dichotimized into the model. Each circle in the iAUC plot indicates an event (relapse) of a patient (Figure3). The ROC and AUC curves were calculated based on the LocalCox method in case the marker was violating the Hazards assumptions, otherwise the Cox method was used. The analyzes were performed using the R survival and risksetROC packages. All AUC characteristics for all combination markers are described in Table 1, 2, 3, 4, 5, 6, 7, 8, 9, 10, 11, 12, 13, 14, 15, and 16. The best combinations are described in Table 17.

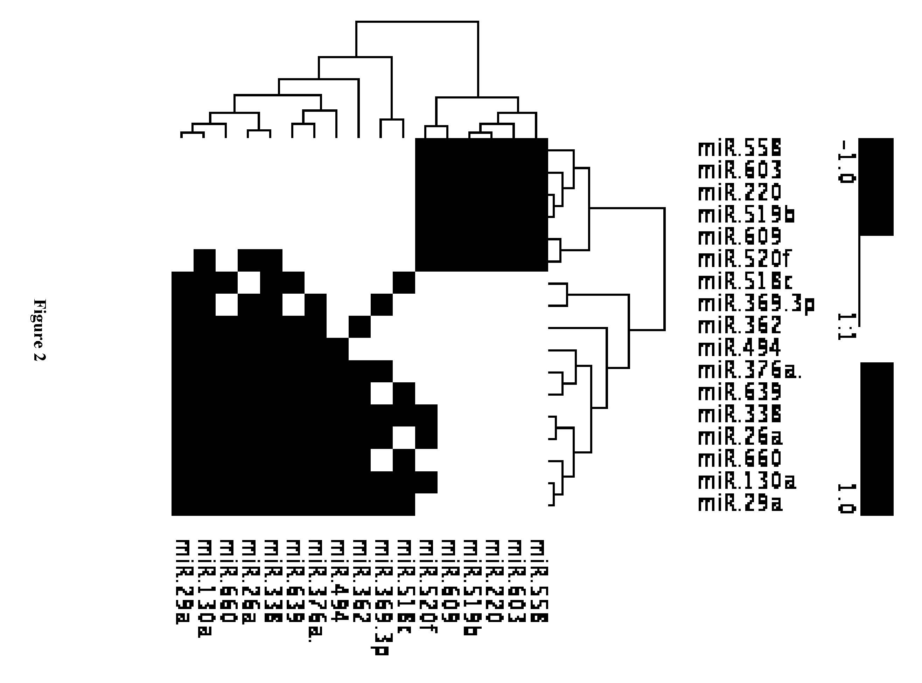

**Correlation matrix cluster for the 17 final selected signature miRNA qPCR data clustered on 77patients (Stage I-III):** The correlation matrix cluster show two major cluster (Figure 2). A high correlation cluster with the six miRs with good outcome and a high correlation cluster with the poor outcome miRNAs. Negative correlation can be seen between those two clusters showing an inverse expression of those miRNAs. A high correlation pattern between miRNAs show a probable redundancy among those miRNAs. E.g. miR.29a and miR130a have a highly similar correlation pattern as well as miR.26a and miR.338, which would suggest a possible replacement by one of them. Other examples are described in the followings Tables:

**Table C**

| 4 Marker Combination all miRs | c_tau |
|---|---|
| miR.558 + **miR.603 + miR.220a** + miR.518c | 0.7646 |
| miR.558 + **miR.609 + miR.519b** + miR.518c | 0.7639 |

**Table D**

| 4 Marker Combination all miRs | c_tau |
|---|---|
| miR.609 + miR.26a + **miR.29a** + miR.518c | 0.7609 |
| miR.609 + miR.603 + **miR.130a** + miR.518c | 0.7608 |

**Table E**

| 5 Marker Combination all miRs | c_tau |
|---|---|
| miR.609 + **miR.603** + miR.29a + miR.376a* + miR.518c | 0.7983 |
| miR.609 + **miR.558** + miR.29a + miR.376a* + miR.518c | 0.7913 |

**Table F**

| 6 Marker Combination all miRs | c_tau |
|---|---|
| miR.558 + miR.609 + **miR.220a** + miR.29a + miR.376a* + miR.518c | 0.8060 |
| miR.558 + miR.609 + **miR.520f +** miR.29a + miR.376a* + miR.518c | 0.8050 |

**Table G**

| 7 Marker_Combination all patients | c_tau |
|---|---|
| miR.558 + miR.609 + miR.220a + miR.29a + **miR.660** + miR.376a* + miR.518c | 0.8066 |
| miR.558 + miR.609 + miR.220a + miR.29a + **miR.130a** + miR.376a* + miR.518c | 0.8063 |

**Table H**

| 8 Marker_Combination all patients | c_tau |
|---|---|
| miR.558 + miR.609 + miR.220a + **miR.26a** + miR.29a + miR.369.3p + miR.376a* + miR.518c | 0.8113 |
| miR.558 + miR.609 + miR.220a + **miR.660** + miR.29a + miR.369.3p + miR.376a* + miR.518c | 0.8111 |

Some of the miRNAs are redundant and may share common biological functions which makes them replaceable among each other. This is of advantage in case some the miRNAs are not expressed or are not measurable because of technical reasons.

**Table 1: clusters of 1 miRNA**

| **Cluster of 1 miR** | **c_tau level** | **pv compared to top signature miR.609** |
|---|---|---|
| miR.609 | 0,66 | NaN |
| miR.558 | 0,66 | 0,418938974 |
| miR.603 | 0,66 | 0,190043508 |
| miR.518c | 0,65 | 0,394113397 |
| miR.520f | 0,64 | 0,483494013 |
| miR.362 | 0,64 | 0,432657709 |
| miR.220a | 0,63 | 0,436890827 |
| miR.29a | 0,62 | 0,312018624 |
| miR.519b | 0,62 | 0,226161064 |
| miR.494 | 0,61 | 0,158371367 |
| miR.130a | 0,6 | 0,189293501 |
| miR.639 | 0,6 | 0,168935983 |
| miR.660 | 0,59 | 0,170064551 |

| **UICC** | **0,59** | **NaN** |
|---|---|---|
| miR.26a | 0,57 | 0,059277098 |
| miR.369.3p | 0,55 | 0,08341555 |
| miR.338 | 0,54 | 0,054392511 |
| miR.376a* | 0,54 | 0,015577754 |

**Table 2: clusters of 2 miRNAs**

| **Cluster of 2 miRs** | **c_tau level** | **pv compared to top signature miR.603 + miR.518c** |
|---|---|---|
| miR.603 + miR.518c | 0,73 | NaN |
| miR.558 + miR.518c | 0,73 | 0,009268576 |
| miR.609 + miR.29a | 0,72 | 0,423370924 |
| miR.558 + miR.609 | 0,72 | 0,482705956 |
| miR.220a + miR.518c | 0,72 | 0,001073457 |
| miR.609 + miR.518c | 0,72 | 0,134186365 |
| miR.558 + miR.520f | 0,7 | 0,171004128 |
| miR.609 + miR.520f | 0,7 | 0,167116274 |
| miR.558 + miR.603 | 0,7 | 0,188187249 |
| miR.519b + miR.518c | 0,69 | 0,000904262 |
| miR.558 + miR.362 | 0,69 | 0,086851866 |
| miR.558 + miR.29a | 0,69 | 0,122648778 |
| miR.520f + miR.29a | 0,69 | 0,14900018 |
| miR.603 + miR.29a | 0,68 | 0,02436506 |
| miR.603 + miR.494 | 0,68 | 0,066852154 |
| miR.520f + miR.130a | 0,68 | 0,1378426 |
| miR.520f + miR.26a | 0,68 | 0,138396835 |
| miR.603 + miR.130a | 0,68 | 0,044534014 |
| miR.603 + miR.362 | 0,68 | 0,01236297 |
| miR.558 + miR.130a | 0,68 | 0,104285811 |
| miR.603 + miR.639 | 0,68 | 0,038208676 |
| miR.603 + miR.369.3p | 0,67 | 0,094691498 |
| miR.603 + miR.660 | 0,67 | 0,01385758 |
| miR.603 + miR.220a | 0,67 | 0,033359616 |
| miR.29a + miR.518c | 0,67 | 0,038492703 |
| miR.220a + miR.29a | 0,66 | 0,03205418 |
| miR.369.3p + miR.518c | 0,66 | 0,014816909 |
| miR.603 + miR.26a | 0,66 | 0,044908534 |
| miR.220a + miR.362 | 0,66 | 0,011719433 |
| miR.603 + miR.338 | 0,66 | 0,04870573 |
| miR.519b + miR.29a | 0,65 | 0,009625124 |
| miR.220a + miR.130a | 0,65 | 0,032322123 |
| miR.130a + miR.362 | 0,65 | 0,033378678 |

**Table 3: clusters of 3 miRNAs**

| **Cluster of 3 miRs** | **c_tau level** | **pv compared to top signature miR.558 + miR.609 + miR.518c** |
|---|---|---|
| miR.558 + miR.609 + miR.518c | 0,76 | NaN |
| miR.558 + miR.603 + miR.518c | 0,75 | 0,247784245 |
| miR.609 + miR.29a + miR.518c | 0,75 | 0,164812866 |
| miR.603 + miR.220a + miR.518c | 0,75 | 0,488976647 |
| miR.609 + miR.603 + miR.518c | 0,75 | 0,34714351 |
| miR.558 + miR.520f + miR.518c | 0,75 | 0,362175562 |
| miR.558 + miR.609 + miR.29a | 0,75 | 0,355216853 |
| miR.609 + miR.520f + miR.29a | 0,75 | 0,348153894 |
| miR.603 + miR.520f + miR.518c | 0,74 | 0,425658902 |
| miR.609 + miR.29a + miR.376a* | 0,74 | 0,194993588 |
| miR.609 + miR.220a + miR.518c | 0,74 | 0,049347643 |
| miR.558 + miR.220a + miR.518c | 0,74 | 0,101338262 |
| miR.603 + miR.376a* + miR.518c | 0,74 | 0,346484887 |
| miR.558 + miR.609 + miR.520f | 0,74 | 0,140417039 |
| miR.609 + miR.518c + miR.639 | 0,74 | 0,351186289 |
| miR.603 + miR.518c + miR.639 | 0,74 | 0,280054707 |
| miR.609 + miR.130a + miR.518c | 0,74 | 0,449352384 |
| miR.520f + miR.220a + miR.518c | 0,74 | 0,402122181 |
| miR.603 + miR.369.3p + miR.518c | 0,74 | 0,273671635 |
| miR.558 + miR.609 + miR.603 | 0,73 | 0,108306893 |
| miR.603 + miR.362 + miR.518c | 0,73 | 0,293305717 |
| miR.520f + miR.518c + miR.639 | 0,73 | 0,35243061 |
| miR.609 + miR.520f + miR.518c | 0,73 | 0,30176526 |
| miR.603 + miR.29a + miR.518c | 0,73 | 0,271112606 |
| miR.603 + miR.26a + miR.518c | 0,73 | 0,285703084 |
| miR.558 + miR.376a* + miR.518c | 0,73 | 0,315129807 |
| miR.558 + miR.609 + miR.639 | 0,73 | 0,104170851 |
| miR.609 + miR.520f + miR.130a | 0,73 | 0,370027972 |
| miR.603 + miR.518c + miR.660 | 0,73 | 0,216991425 |
| miR.603 + miR.130a + miR.518c | 0,73 | 0,251376103 |
| miR.603 + miR.518c + miR.338 | 0,73 | 0,212164512 |
| miR.603 + miR.519b + miR.518c | 0,73 | 0,185914027 |
| miR.558 + miR.609 + miR.130a | 0,73 | 0,078091571 |
| miR.603 + miR.494 + miR.518c | 0,73 | 0,173052451 |
| miR.558 + miR.362 + miR.518c | 0,73 | 0,069189409 |
| miR.558 + miR.609 + miR.220a | 0,73 | 0,039602125 |
| miR.558 + miR.609 + miR.362 | 0,73 | 0,021740781 |
| miR.609 + miR.603 + miR.29a | 0,73 | 0,397990999 |
| miR.520f + miR.29a + miR.518c | 0,73 | 0,404704361 |
| miR.558 + miR.518c + miR.639 | 0,73 | 0,091698305 |
| miR.558 + miR.26a + miR.518c | 0,73 | 0,299248291 |
| miR.558 + miR.29a + miR.518c | 0,73 | 0,09574238 |
| miR.609 + miR.519b + miR.518c | 0,73 | 0,005919178 |
| miR.558 + miR.609 + miR.369.3p | 0,73 | 0,265320988 |
| miR.609 + miR.29a + miR.494 | 0,73 | 0,329298298 |
| miR.609 + miR.220a + miR.29a | 0,73 | 0,471997025 |
| miR.609 + miR.362 + miR.518c | 0,73 | 0,256139437 |
| miR.558 + miR.369.3p + miR.518c | 0,73 | 0,153094875 |
| miR.558 + miR.519b + miR.518c | 0,73 | 0,202589552 |
| miR.558 + miR.518c + miR.660 | 0,73 | 0,090211228 |
| miR.609 + miR.520f + miR.639 | 0,73 | 0,248436815 |
| miR.520f + miR.26a + miR.518c | 0,73 | 0,248670032 |
| miR.558 + miR.494 + miR.518c | 0,73 | 0,102215116 |
| miR.558 + miR.609 + miR.494 | 0,73 | 0,112580737 |
| miR.558 + miR.518c + miR.338 | 0,73 | 0,111295798 |
| miR.558 + miR.130a + miR.518c | 0,73 | 0,097771847 |
| miR.609 + miR.369.3p + miR.518c | 0,73 | 0,292047668 |
| miR.520f + miR.130a + miR.518c | 0,73 | 0,325326497 |
| miR.558 + miR.609 + miR.376a* | 0,73 | 0,260189737 |
| miR.609 + miR.494 + miR.518c | 0,73 | 0,098487012 |
| miR.609 + miR.29a + miR.369.3p | 0,72 | 0,335139361 |
| miR.558 + miR.609 + miR.660 | 0,72 | 0,025079336 |
| miR.609 + miR.26a + miR.29a | 0,72 | 0,43507738 |
| miR.609 + miR.519b + miR.29a | 0,72 | 0,332887111 |
| miR.609 + miR.29a + miR.639 | 0,72 | 0,363228171 |
| miR.520f + miR.519b + miR.518c | 0,72 | 0,170474636 |
| miR.609 + miR.29a + miR.338 | 0,72 | 0,321558317 |
| miR.220a + miR.518c + miR.338 | 0,72 | 0,178086869 |
| miR.220a + miR.29a + miR.518c | 0,72 | 0,137410978 |
| miR.609 + miR.29a + miR.362 | 0,72 | 0,37955572 |
| miR.220a + miR.369.3p + miR.518c | 0,72 | 0,153896555 |
| miR.609 + miR.29a + miR.660 | 0,72 | 0,284984411 |
| miR.558 + miR.609 + miR.26a | 0,72 | 0,057440468 |
| miR.220a + miR.518c + miR.639 | 0,72 | 0,125459545 |
| miR.520f + miR.362 + miR.518c | 0,72 | 0,301355412 |
| miR.609 + miR.520f + miR.26a | 0,72 | 0,312363545 |
| miR.558 + miR.609 + miR.338 | 0,72 | 0,047008735 |
| miR.609 + miR.603 + miR.130a | 0,72 | 0,23533357 |
| miR.609 + miR.130a + miR.29a | 0,72 | 0,370784436 |
| miR.220a + miR.362 + miR.518c | 0,72 | 0,16166902 |
| miR.609 + miR.518c + miR.660 | 0,72 | 0,112066562 |
| miR.220a + miR.376a* + miR.518c | 0,72 | 0,198135987 |
| miR.220a + miR.130a + miR.518c | 0,72 | 0,148439546 |
| miR.558 + miR.609 + miR.519b | 0,72 | 0,088041913 |
| miR.220a + miR.26a + miR.518c | 0,72 | 0,190004241 |
| miR.220a + miR.519b + miR.518c | 0,72 | 0,142304577 |
| miR.220a + miR.494 + miR.518c | 0,72 | 0,157006033 |
| miR.220a + miR.518c + miR.660 | 0,72 | 0,161323893 |
| miR.609 + miR.603 + miR.494 | 0,72 | 0,241519739 |
| miR.609 + miR.603 + miR.639 | 0,72 | 0,251517332 |
| miR.609 + miR.26a + miR.518c | 0,72 | 0,066030989 |
| miR.558 + miR.603 + miR.520f | 0,72 | 0,142937829 |
| miR.520f + miR.518c + miR.660 | 0,72 | 0,243462668 |
| miR.558 + miR.520f + miR.29a | 0,72 | 0,061445518 |
| miR.520f + miR.369.3p + miR.518c | 0,72 | 0,221082215 |
| miR.520f + miR.518c + miR.338 | 0,72 | 0,277129663 |
| miR.609 + miR.362 + miR.369.3p | 0,72 | 0,256027229 |
| miR.609 + miR.603 + miR.520f | 0,72 | 0,165161932 |
| miR.558 + miR.520f + miR.639 | 0,72 | 0,10668753 |
| miR.609 + miR.518c + miR.338 | 0,72 | 0,049918113 |
| miR.609 + miR.130a + miR.376a* | 0,72 | 0,314938145 |
| miR.520f + miR.494 + miR.518c | 0,72 | 0,161170186 |
| miR.609 + miR.603 + miR.220a | 0,72 | 0,17485756 |
| miR.609 + miR.376a* + miR.518c | 0,71 | 0,021158932 |
| miR.558 + miR.520f + miR.362 | 0,71 | 0,042970193 |
| miR.609 + miR.220a + miR.130a | 0,71 | 0,128890701 |
| miR.609 + miR.603 + miR.369.3p | 0,71 | 0,196936889 |
| miR.609 + miR.520f + miR.494 | 0,71 | 0,067113092 |
| miR.558 + miR.520f + miR.130a | 0,71 | 0,032963824 |
| miR.609 + miR.362 + miR.494 | 0,71 | 0,132878392 |
| miR.609 + miR.520f + miR.220a | 0,71 | 0,044047133 |
| miR.609 + miR.603 + miR.362 | 0,71 | 0,081082555 |
| miR.558 + miR.520f + miR.26a | 0,71 | 0,037521396 |
| miR.609 + miR.494 + miR.639 | 0,71 | 0,073255244 |
| miR.609 + miR.220a + miR.494 | 0,71 | 0,17258061 |
| miR.609 + miR.220a + miR.369.3p | 0,71 | 0,105201478 |
| miR.558 + miR.520f + miR.338 | 0,71 | 0,064777816 |
| miR.609 + miR.520f + miR.362 | 0,71 | 0,035840094 |
| miR.558 + miR.520f + miR.660 | 0,71 | 0,043413436 |
| miR.558 + miR.362 + miR.369.3p | 0,71 | 0,037425892 |
| miR.558 + miR.603 + miR.362 | 0,71 | 0,043710943 |
| miR.609 + miR.603 + miR.376a* | 0,71 | 0,128251061 |
| miR.609 + miR.220a + miR.376a* | 0,71 | 0,080434388 |
| miR.558 + miR.603 + miR.29a | 0,71 | 0,050887026 |
| miR.609 + miR.130a + miR.369.3p | 0,71 | 0,097832454 |
| miR.609 + miR.130a + miR.494 | 0,71 | 0,07652331 |
| miR.609 + miR.603 + miR.660 | 0,71 | 0,069081805 |
| miR.609 + miR.603 + miR.26a | 0,71 | 0,119585833 |
| miR.609 + miR.220a + miR.362 | 0,71 | 0,050963096 |
| miR.609 + miR.603 + miR.519b | 0,71 | 0,070308772 |
| miR.558 + miR.603 + miR.130a | 0,71 | 0,028731659 |
| miR.558 + miR.603 + miR.369.3p | 0,71 | 0,051261759 |
| miR.558 + miR.603 + miR.639 | 0,71 | 0,034357969 |
| miR.558 + miR.520f + miR.494 | 0,71 | 0,048911277 |
| miR.603 + miR.520f + miR.29a | 0,71 | 0,216340461 |
| miR.558 + miR.603 + miR.660 | 0,71 | 0,027394144 |
| miR.609 + miR.520f + miR.519b | 0,71 | 0,033679188 |
| miR.609 + miR.520f + miR.338 | 0,7 | 0,052551862 |
| miR.603 + miR.362 + miR.369.3p | 0,7 | 0,198969083 |
| miR.558 + miR.520f + miR.220a | 0,7 | 0,044708648 |
| miR.603 + miR.520f + miR.639 | 0,7 | 0,167994772 |
| miR.609 + miR.603 + miR.338 | 0,7 | 0,084883885 |
| miR.603 + miR.520f + miR.130a | 0,7 | 0,141242618 |
| miR.609 + miR.520f + miR.660 | 0,7 | 0,016329201 |
| miR.609 + miR.369.3p + miR.494 | 0,7 | 0,049507017 |
| miR.558 + miR.520f + miR.369.3p | 0,7 | 0,128349981 |
| miR.609 + miR.520f + miR.369.3p | 0,7 | 0,056625869 |
| miR.362 + miR.369.3p + miR.518c | 0,7 | 0,240334825 |
| miR.558 + miR.603 + miR.494 | 0,7 | 0,021795267 |
| miR.519b + miR.518c + miR.639 | 0,7 | 0,007041137 |
| miR.603 + miR.520f + miR.26a | 0,7 | 0,181375394 |
| miR.609 + miR.519b + miR.362 | 0,7 | 0,017616618 |
| miR.609 + miR.26a + miR.362 | 0,7 | 0,049968858 |
| miR.609 + miR.220a + miR.519b | 0,7 | 0,027688308 |
| miR.558 + miR.603 + miR.220a | 0,7 | 0,04012403 |
| miR.519b + miR.362 + miR.518c | 0,7 | 0,026035969 |
| miR.603 + miR.520f + miR.494 | 0,7 | 0,119510911 |
| miR.519b + miR.376a* + miR.518c | 0,7 | 0,007101906 |
| miR.558 + miR.520f + miR.376a* | 0,7 | 0,148510611 |
| miR.519b + miR.369.3p + miR.518c | 0,7 | 0,006727401 |
| miR.609 + miR.362 + miR.376a* | 0,7 | 0,065078334 |
| miR.558 + miR.520f + miR.519b | 0,7 | 0,159181427 |
| miR.519b + miR.29a + miR.518c | 0,7 | 0,025523668 |
| miR.603 + miR.520f + miR.362 | 0,7 | 0,142734333 |
| miR.609 + miR.362 + miR.338 | 0,7 | 0,039368124 |
| miR.603 + miR.362 + miR.494 | 0,7 | 0,09934405 |
| miR.609 + miR.494 + miR.660 | 0,7 | 0,041673346 |
| miR.558 + miR.29a + miR.362 | 0,7 | 0,004731426 |
| miR.609 + miR.520f + miR.376a* | 0,7 | 0,020124337 |
| miR.558 + miR.362 + miR.494 | 0,7 | 0,003012638 |
| miR.520f + miR.29a + miR.362 | 0,7 | 0,089888022 |
| miR.558 + miR.603 + miR.338 | 0,7 | 0,015964208 |
| miR.519b + miR.130a + miR.518c | 0,7 | 0,00436048 |
| miR.362 + miR.518c + miR.639 | 0,7 | 0,074343301 |
| miR.558 + miR.362 + miR.639 | 0,7 | 0,003521047 |
| miR.520f + miR.362 + miR.639 | 0,7 | 0,089557122 |
| miR.519b + miR.518c + miR.660 | 0,7 | 0,004070438 |
| miR.519b + miR.494 + miR.518c | 0,7 | 0,002940958 |
| miR.558 + miR.603 + miR.26a | 0,7 | 0,014992291 |
| miR.519b + miR.518c + miR.338 | 0,7 | 0,00107145 |
| miR.558 + miR.603 + miR.519b | 0,7 | 0,03465954 |
| miR.520f + miR.26a + miR.639 | 0,7 | 0,07962049 |
| miR.558 + miR.603 + miR.376a* | 0,7 | 0,031039049 |
| miR.520f + miR.130a + miR.362 | 0,69 | 0,085235769 |
| miR.558 + miR.130a + miR.362 | 0,69 | 0,00601709 |
| miR.520f + miR.26a + miR.29a | 0,69 | 0,087651916 |
| miR.603 + miR.520f + miR.660 | 0,69 | 0,077140142 |
| miR.520f + miR.29a + miR.639 | 0,69 | 0,081746407 |
| miR.520f + miR.29a + miR.494 | 0,69 | 0,065088308 |
| miR.520f + miR.220a + miR.29a | 0,69 | 0,071074473 |
| miR.520f + miR.519b + miR.29a | 0,69 | 0,052203253 |
| miR.603 + miR.520f + miR.220a | 0,69 | 0,13159188 |
| miR.603 + miR.29a + miR.494 | 0,69 | 0,057716574 |
| miR.603 + miR.369.3p + miR.494 | 0,69 | 0,063429906 |
| miR.558 + miR.26a + miR.29a | 0,69 | 0,016454956 |
| miR.520f + miR.26a + miR.494 | 0,69 | 0,037334593 |
| miR.520f + miR.519b + miR.639 | 0,69 | 0,032303599 |
| miR.520f + miR.29a + miR.376a* | 0,69 | 0,135732892 |
| miR.558 + miR.220a + miR.362 | 0,69 | 0,004264036 |
| miR.520f + miR.26a + miR.362 | 0,69 | 0,080410499 |
| miR.362 + miR.494 + miR.518c | 0,69 | 0,07811005 |
| miR.558 + miR.220a + miR.29a | 0,69 | 0,003817785 |
| miR.603 + miR.130a + miR.362 | 0,69 | 0,107693542 |
| miR.520f + miR.362 + miR.369.3p | 0,69 | 0,064838129 |
| miR.603 + miR.520f + miR.369.3p | 0,69 | 0,097355846 |
| miR.603 + miR.520f + miR.338 | 0,69 | 0,083688787 |
| miR.609 + miR.376a* + miR.660 | 0,69 | 0,030919011 |
| miR.558 + miR.29a + miR.369.3p | 0,69 | 0,008209665 |
| miR.603 + miR.220a + miR.494 | 0,69 | 0,09413129 |
| miR.603 + miR.362 + miR.639 | 0,69 | 0,092399431 |
| miR.603 + miR.494 + miR.639 | 0,69 | 0,037277169 |
| miR.603 + miR.29a + miR.362 | 0,69 | 0,097411564 |
| miR.558 + miR.362 + miR.338 | 0,69 | 0,001934736 |
| miR.558 + miR.29a + miR.494 | 0,69 | 0,002735141 |
| miR.558 + miR.29a + miR.639 | 0,69 | 0,00511974 |
| miR.558 + miR.362 + miR.376a* | 0,69 | 0,004511201 |
| miR.520f + miR.362 + miR.494 | 0,69 | 0,026077672 |
| miR.520f + miR.494 + miR.639 | 0,69 | 0,028291164 |
| miR.558 + miR.29a + miR.376a* | 0,69 | 0,022595613 |
| miR.520f + miR.130a + miR.26a | 0,69 | 0,032662435 |
| miR.558 + miR.26a + miR.362 | 0,69 | 0,001724111 |
| miR.520f + miR.26a + miR.376a* | 0,69 | 0,102288809 |
| miR.520f + miR.130a + miR.639 | 0,69 | 0,037949646 |
| miR.558 + miR.29a + miR.660 | 0,69 | 0,00269176 |
| miR.558 + miR.519b + miR.362 | 0,69 | 0,007170633 |
| miR.520f + miR.29a + miR.338 | 0,69 | 0,113544366 |
| miR.603 + miR.494 + miR.660 | 0,69 | 0,030503802 |
| miR.558 + miR.130a + miR.29a | 0,69 | 0,00294133 |
| miR.603 + miR.130a + miR.369.3p | 0,69 | 0,058256247 |
| miR.520f + miR.29a + miR.369.3p | 0,69 | 0,074005777 |
| miR.603 + miR.29a + miR.369.3p | 0,69 | 0,094414449 |
| miR.520f + miR.130a + miR.29a | 0,69 | 0,06100135 |
| miR.520f + miR.29a + miR.660 | 0,69 | 0,077631374 |
| miR.520f + miR.519b + miR.130a | 0,69 | 0,016231919 |
| miR.558 + miR.29a + miR.338 | 0,69 | 0,005031265 |
| miR.603 + miR.220a + miR.29a | 0,69 | 0,090210804 |
| miR.558 + miR.519b + miR.29a | 0,69 | 0,00734062 |
| miR.603 + miR.29a + miR.639 | 0,69 | 0,086681916 |
| miR.520f + miR.639 + miR.338 | 0,69 | 0,062844863 |
| miR.603 + miR.130a + miR.494 | 0,69 | 0,033425556 |
| miR.603 + miR.369.3p + miR.639 | 0,69 | 0,079970621 |
| miR.520f + miR.220a + miR.26a | 0,69 | 0,064168511 |
| miR.603 + miR.220a + miR.130a | 0,69 | 0,064383883 |
| miR.603 + miR.220a + miR.369.3p | 0,69 | 0,102676466 |
| miR.520f + miR.220a + miR.130a | 0,69 | 0,032168586 |
| miR.558 + miR.369.3p + miR.660 | 0,69 | 0,005397536 |
| miR.520f + miR.220a + miR.639 | 0,69 | 0,060100112 |
| miR.520f + miR.639 + miR.660 | 0,69 | 0,044269744 |
| miR.520f + miR.130a + miR.376a* | 0,69 | 0,054980261 |
| miR.558 + miR.369.3p + miR.639 | 0,69 | 0,016041004 |
| miR.603 + miR.376a* + miR.494 | 0,69 | 0,046186987 |
| miR.603 + miR.26a + miR.29a | 0,69 | 0,063535409 |
| miR.603 + miR.369.3p + miR.660 | 0,69 | 0,053000268 |
| miR.603 + miR.29a + miR.660 | 0,69 | 0,078229291 |
| miR.603 + miR.220a + miR.362 | 0,69 | 0,061173635 |
| miR.603 + miR.130a + miR.660 | 0,69 | 0,059182476 |
| miR.603 + miR.130a + miR.639 | 0,69 | 0,04451678 |
| miR.520f + miR.130a + miR.494 | 0,69 | 0,031419644 |
| miR.558 + miR.376a* + miR.660 | 0,68 | 0,008666668 |
| miR.220a + miR.362 + miR.369.3p | 0,68 | 0,044803856 |
| miR.603 + miR.29a + miR.338 | 0,68 | 0,061647642 |
| miR.558 + miR.220a + miR.369.3p | 0,68 | 0,006032894 |
| miR.603 + miR.130a + miR.29a | 0,68 | 0,065069428 |
| miR.603 + miR.29a + miR.376a* | 0,68 | 0,098148148 |
| miR.603 + miR.519b + miR.29a | 0,68 | 0,079745901 |
| miR.520f + miR.519b + miR.26a | 0,68 | 0,021950393 |
| miR.520f + miR.130a + miR.338 | 0,68 | 0,034120701 |
| miR.558 + miR.220a + miR.130a | 0,68 | 0,001355518 |
| miR.520f + miR.26a + miR.660 | 0,68 | 0,03716572 |
| miR.520f + miR.519b + miR.362 | 0,68 | 0,018306926 |
| miR.520f + miR.130a + miR.369.3p | 0,68 | 0,025465139 |
| miR.603 + miR.639 + miR.660 | 0,68 | 0,051673284 |
| miR.558 + miR.130a + miR.660 | 0,68 | 0,00227285 |
| miR.603 + miR.130a + miR.376a* | 0,68 | 0,087534658 |
| miR.520f + miR.369.3p + miR.639 | 0,68 | 0,031523393 |
| miR.520f + miR.26a + miR.338 | 0,68 | 0,03608043 |
| miR.603 + miR.220a + miR.639 | 0,68 | 0,074165337 |
| miR.558 + miR.220a + miR.494 | 0,68 | 0,00165927 |
| miR.603 + miR.519b + miR.494 | 0,68 | 0,033679942 |
| miR.603 + miR.494 + miR.338 | 0,68 | 0,032721745 |
| miR.520f + miR.220a + miR.494 | 0,68 | 0,020323857 |
| miR.603 + miR.26a + miR.494 | 0,68 | 0,032072768 |
| miR.558 + miR.130a + miR.369.3p | 0,68 | 0,007685614 |
| miR.29a + miR.518c + miR.639 | 0,68 | 0,023364438 |
| miR.494 + miR.518c + miR.639 | 0,68 | 0,008490205 |
| miR.520f + miR.130a + miR.660 | 0,68 | 0,029924621 |
| miR.603 + miR.220a + miR.660 | 0,68 | 0,082252214 |
| miR.520f + miR.26a + miR.369.3p | 0,68 | 0,026372464 |
| miR.369.3p + miR.518c + miR.639 | 0,68 | 0,02039752 |
| miR.362 + miR.369.3p + miR.494 | 0,68 | 0,026195497 |
| miR.603 + miR.519b + miR.130a | 0,68 | 0,030867499 |
| miR.603 + miR.130a + miR.338 | 0,68 | 0,028259552 |
| miR.603 + miR.130a + miR.26a | 0,68 | 0,021419132 |
| miR.603 + miR.519b + miR.639 | 0,68 | 0,036692848 |
| miR.558 + miR.130a + miR.639 | 0,68 | 0,00230415 |
| miR.603 + miR.519b + miR.362 | 0,68 | 0,062185979 |
| miR.603 + miR.26a + miR.362 | 0,68 | 0,058826941 |
| miR.603 + miR.362 + miR.338 | 0,68 | 0,063726262 |
| miR.558 + miR.130a + miR.376a* | 0,68 | 0,006780287 |
| miR.519b + miR.362 + miR.369.3p | 0,68 | 0,031917711 |
| miR.603 + miR.362 + miR.660 | 0,68 | 0,06167808 |
| miR.558 + miR.130a + miR.494 | 0,68 | 0,001593409 |
| miR.603 + miR.362 + miR.376a* | 0,68 | 0,063688629 |
| miR.558 + miR.130a + miR.26a | 0,68 | 0,003314831 |
| miR.603 + miR.376a* + miR.660 | 0,68 | 0,062752031 |
| miR.520f + miR.362 + miR.376a* | 0,68 | 0,037891073 |
| miR.29a + miR.369.3p + miR.518c | 0,68 | 0,026641203 |
| miR.603 + miR.376a* + miR.639 | 0,68 | 0,062767214 |
| miR.558 + miR.130a + miR.338 | 0,68 | 0,001298161 |
| miR.29a + miR.494 + miR.518c | 0,68 | 0,015993401 |
| miR.603 + miR.639 + miR.338 | 0,68 | 0,038471506 |
| miR.558 + miR.519b + miR.130a | 0,68 | 0,001359148 |
| miR.603 + miR.26a + miR.639 | 0,68 | 0,036012679 |
| miR.220a + miR.362 + miR.494 | 0,68 | 0,021666565 |
| miR.520f + miR.369.3p + miR.494 | 0,68 | 0,01293201 |
| miR.362 + miR.369.3p + miR.376a* | 0,68 | 0,047295371 |
| miR.603 + miR.26a + miR.369.3p | 0,68 | 0,041274316 |
| miR.603 + miR.519b + miR.660 | 0,68 | 0,042202556 |
| miR.603 + miR.220a + miR.26a | 0,67 | 0,050004442 |
| miR.362 + miR.369.3p + miR.639 | 0,67 | 0,042589232 |
| miR.603 + miR.369.3p + miR.338 | 0,67 | 0,037912775 |
| miR.603 + miR.369.3p + miR.376a* | 0,67 | 0,056124246 |
| miR.603 + miR.220a + miR.519b | 0,67 | 0,046039626 |
| miR.603 + miR.660 + miR.338 | 0,67 | 0,032875331 |
| miR.603 + miR.26a + miR.660 | 0,67 | 0,034332136 |
| miR.603 + miR.220a + miR.338 | 0,67 | 0,045592948 |
| miR.558 + miR.376a* + miR.494 | 0,67 | 0,01210817 |
| miR.369.3p + miR.494 + miR.518c | 0,67 | 0,00381182 |
| miR.558 + miR.369.3p + miR.338 | 0,67 | 0,012678172 |
| miR.29a + miR.362 + miR.369.3p | 0,67 | 0,051664943 |
| miR.130a + miR.362 + miR.369.3p | 0,67 | 0,041646443 |
| miR.29a + miR.518c + miR.660 | 0,67 | 0,014200948 |
| miR.603 + miR.220a + miR.376a* | 0,67 | 0,063739599 |
| miR.220a + miR.29a + miR.494 | 0,67 | 0,010435825 |
| miR.220a + miR.29a + miR.362 | 0,67 | 0,013267597 |
| miR.130a + miR.369.3p + miR.518c | 0,67 | 0,013797868 |
| miR.130a + miR.494 + miR.518c | 0,67 | 0,003426413 |
| miR.220a + miR.29a + miR.369.3p | 0,67 | 0,010805745 |
| miR.520f + miR.376a* + miR.660 | 0,67 | 0,023277242 |
| miR.220a + miR.130a + miR.362 | 0,67 | 0,015581923 |
| miR.220a + miR.362 + miR.639 | 0,67 | 0,008960577 |
| miR.220a + miR.26a + miR.29a | 0,67 | 0,015919194 |
| miR.519b + miR.29a + miR.362 | 0,66 | 0,007917993 |
| miR.220a + miR.29a + miR.338 | 0,66 | 0,006269301 |
| miR.220a + miR.29a + miR.639 | 0,66 | 0,013210616 |
| miR.220a + miR.519b + miR.29a | 0,66 | 0,006033479 |
| miR.603 + miR.519b + miR.26a | 0,66 | 0,016181215 |
| miR.519b + miR.130a + miR.362 | 0,66 | 0,0029988 |
| miR.220a + miR.130a + miR.369.3p | 0,66 | 0,004916638 |
| miR.220a + miR.29a + miR.660 | 0,66 | 0,009183359 |
| miR.26a + miR.369.3p + miR.518c | 0,66 | 0,009051473 |
| miR.220a + miR.130a + miR.29a | 0,66 | 0,007773035 |
| miR.220a + miR.29a + miR.376a* | 0,66 | 0,02391839 |
| miR.369.3p + miR.518c + miR.338 | 0,66 | 0,008593343 |
| miR.603 + miR.26a + miR.338 | 0,66 | 0,017783329 |
| miR.220a + miR.369.3p + miR.639 | 0,66 | 0,00521421 |
| miR.519b + miR.26a + miR.29a | 0,66 | 0,002257527 |
| miR.519b + miR.29a + miR.494 | 0,66 | 0,001399685 |
| miR.519b + miR.29a + miR.369.3p | 0,66 | 0,003631973 |
| miR.603 + miR.26a + miR.376a* | 0,66 | 0,022188973 |
| miR.220a + miR.26a + miR.362 | 0,66 | 0,004094142 |
| miR.220a + miR.362 + miR.338 | 0,66 | 0,004256069 |
| miR.519b + miR.29a + miR.639 | 0,66 | 0,001554 |
| miR.220a + miR.362 + miR.376a* | 0,66 | 0,009279042 |
| miR.603 + miR.376a* + miR.338 | 0,66 | 0,032502963 |
| miR.29a + miR.362 + miR.639 | 0,66 | 0,008171297 |
| miR.220a + miR.130a + miR.639 | 0,66 | 0,001492459 |
| miR.220a + miR.519b + miR.130a | 0,66 | 0,000761264 |
| miR.220a + miR.130a + miR.660 | 0,66 | 0,002601161 |
| miR.519b + miR.29a + miR.376a* | 0,66 | 0,007472073 |
| miR.519b + miR.130a + miR.29a | 0,65 | 0,001009144 |
| miR.519b + miR.29a + miR.660 | 0,65 | 0,001366399 |
| miR.220a + miR.130a + miR.376a* | 0,65 | 0,005951799 |
| miR.220a + miR.130a + miR.26a | 0,65 | 0,000508695 |
| miR.519b + miR.130a + miR.376a* | 0,65 | 0,000560668 |
| miR.220a + miR.130a + miR.338 | 0,65 | 0,000874004 |
| miR.130a + miR.29a + miR.362 | 0,65 | 0,005112882 |

**Table 4: clusters of 4 miRNAs**

| **Cluster of 4 miRs** | **c_tau level** | **pv compared to top signature miR.609 + miR.29a + miR.376a* + miR.518c** |
|---|---|---|
| miR.609 + miR.29a + miR.376a* + miR.518c | 0,77 | NaN |
| miR.558 + miR.609 + miR.29a + miR.518c | 0,77 | 0,095190878 |
| miR.558 + miR.609 + miR.29a + miR.376a* | 0,77 | 0,234133564 |
| miR.609 + miR.520f + miR.29a + miR.376a* | 0,77 | 0,398192778 |
| miR.558 + miR.609 + miR.603 + miR.518c | 0,77 | 0,152400827 |
| miR.609 + miR.520f + miR.29a + miR.518c | 0,77 | 0,273655288 |
| miR.558 + miR.609 + miR.520f + miR.518c | 0,77 | 0,120996525 |
| miR.558 + miR.609 + miR.518c + miR.639 | 0,76 | 0,083980327 |
| miR.558 + miR.609 + miR.220a + miR.518c | 0,76 | 0,090562295 |
| miR.558 + miR.609 + miR.376a* + miR.518c | 0,76 | 0,073563814 |
| miR.558 + miR.609 + miR.520f + miR.29a | 0,76 | 0,212166867 |
| miR.609 + miR.603 + miR.29a + miR.518c | 0,76 | 0,069410938 |
| miR.558 + miR.609 + miR.369.3p + miR.518c | 0,76 | 0,073717701 |
| miR.558 + miR.609 + miR.362 + miR.518c | 0,76 | 0,050881463 |
| miR.558 + miR.609 + miR.130a + miR.518c | 0,76 | 0,071838125 |
| miR.609 + miR.603 + miR.220a + miR.518c | 0,76 | 0,101227072 |
| miR.609 + miR.220a + miR.29a + miR.518c | 0,76 | 0,05942434 |
| miR.558 + miR.603 + miR.220a + miR.518c | 0,76 | 0,131622103 |
| miR.558 + miR.609 + miR.519b + miR.518c | 0,76 | 0,041357661 |
| miR.609 + miR.520f + miR.518c + miR.639 | 0,76 | 0,147820503 |
| miR.558 + miR.609 + miR.494 + miR.518c | 0,76 | 0,059097293 |
| miR.603 + miR.220a + miR.376a* + miR.518c | 0,76 | 0,251129292 |
| miR.558 + miR.609 + miR.26a + miR.518c | 0,76 | 0,061349034 |
| miR.558 + miR.603 + miR.520f + miR.518c | 0,76 | 0,195773827 |
| miR.609 + miR.520f + miR.130a + miR.518c | 0,76 | 0,116815227 |
| miR.558 + miR.609 + miR.518c + miR.338 | 0,76 | 0,05045062 |
| miR.558 + miR.609 + miR.518c + miR.660 | 0,76 | 0,039220441 |
| miR.609 + miR.603 + miR.518c + miR.639 | 0,76 | 0,071585762 |
| miR.558 + miR.603 + miR.376a* + miR.518c | 0,76 | 0,202745975 |
| miR.609 + miR.603 + miR.376a* + miR.518c | 0,76 | 0,084566847 |
| miR.609 + miR.29a + miR.518c + miR.660 | 0,76 | 0,006818281 |
| miR.609 + miR.26a + miR.29a + miR.518c | 0,76 | 0,033615265 |
| miR.609 + miR.603 + miR.130a + miR.518c | 0,76 | 0,064304505 |
| miR.609 + miR.603 + miR.520f + miR.518c | 0,76 | 0,073345843 |
| miR.558 + miR.609 + miR.26a + miR.29a | 0,75 | 0,07199124 |
| miR.609 + miR.29a + miR.518c + miR.338 | 0,75 | 0,043991625 |
| miR.609 + miR.520f + miR.130a + miR.376a* | 0,75 | 0,275240376 |
| miR.558 + miR.603 + miR.519b + miR.518c | 0,75 | 0,145532044 |
| miR.609 + miR.220a + miR.29a + miR.376a* | 0,75 | 0,092195252 |
| miR.609 + miR.29a + miR.376a* + miR.494 | 0,75 | 0,268412151 |
| miR.609 + miR.603 + miR.369.3p + miR.518c | 0,75 | 0,05151607 |
| miR.558 + miR.520f + miR.518c + miR.639 | 0,75 | 0,160078581 |
| miR.558 + miR.609 + miR.520f + miR.639 | 0,75 | 0,153716109 |
| miR.609 + miR.603 + miR.29a + miR.376a* | 0,75 | 0,121083107 |
| miR.603 + miR.220a + miR.519b + miR.518c | 0,75 | 0,122942194 |
| miR.603 + miR.520f + miR.220a + miR.518c | 0,75 | 0,182075349 |
| miR.558 + miR.609 + miR.520f + miR.130a | 0,75 | 0,219179588 |
| miR.558 + miR.603 + miR.26a + miR.518c | 0,75 | 0,15839003 |
| miR.609 + miR.29a + miR.369.3p + miR.376a* | 0,75 | 0,213298508 |
| miR.603 + miR.220a + miR.369.3p + miR.518c | 0,75 | 0,126725345 |
| miR.558 + miR.603 + miR.518c + miR.639 | 0,75 | 0,159516149 |
| miR.558 + miR.520f + miR.220a + miR.518c | 0,75 | 0,154099639 |
| miR.609 + miR.603 + miR.494 + miR.518c | 0,75 | 0,094774889 |
| miR.609 + miR.29a + miR.494 + miR.518c | 0,75 | 0,042714709 |
| miR.609 + miR.220a + miR.130a + miR.518c | 0,75 | 0,033957969 |
| miR.558 + miR.603 + miR.369.3p + miR.518c | 0,75 | 0,139052213 |
| miR.609 + miR.29a + miR.369.3p + miR.518c | 0,75 | 0,02790735 |
| miR.609 + miR.519b + miR.29a + miR.518c | 0,75 | 0,026540884 |
| miR.603 + miR.220a + miR.518c + miR.338 | 0,75 | 0,109761825 |
| miR.558 + miR.520f + miR.29a + miR.518c | 0,75 | 0,169971614 |
| miR.558 + miR.603 + miR.362 + miR.518c | 0,75 | 0,125790751 |
| miR.609 + miR.130a + miR.29a + miR.518c | 0,75 | 0,017242261 |
| miR.603 + miR.220a + miR.26a + miR.518c | 0,75 | 0,090332806 |
| miR.558 + miR.603 + miR.29a + miR.518c | 0,75 | 0,137256379 |
| miR.603 + miR.520f + miR.518c + miR.639 | 0,75 | 0,155894054 |
| miR.603 + miR.220a + miR.29a + miR.518c | 0,75 | 0,105622688 |
| miR.558 + miR.603 + miR.494 + miR.518c | 0,75 | 0,120489954 |
| miR.558 + miR.520f + miR.376a* + miR.518c | 0,75 | 0,193976453 |
| miR.558 + miR.603 + miR.518c + miR.338 | 0,75 | 0,125210123 |
| miR.609 + miR.603 + miR.362 + miR.518c | 0,75 | 0,051557056 |
| miR.609 + miR.29a + miR.518c + miR.639 | 0,75 | 0,025353784 |
| miR.609 + miR.520f + miR.220a + miR.518c | 0,75 | 0,034932887 |
| miR.558 + miR.603 + miR.518c + miR.660 | 0,75 | 0,120602125 |
| miR.603 + miR.220a + miR.518c + miR.639 | 0,75 | 0,089640403 |
| miR.609 + miR.29a + miR.362 + miR.518c | 0,75 | 0,022807144 |
| miR.609 + miR.220a + miR.376a* + miR.518c | 0,75 | 0,015135288 |
| miR.558 + miR.603 + miR.130a + miR.518c | 0,75 | 0,128050808 |
| miR.609 + miR.220a + miR.518c + miR.639 | 0,75 | 0,019525674 |
| miR.558 + miR.609 + miR.603 + miR.29a | 0,75 | 0,11995271 |
| miR.603 + miR.220a + miR.130a + miR.518c | 0,75 | 0,101475598 |
| miR.609 + miR.520f + miR.26a + miR.518c | 0,75 | 0,091970396 |
| miR.603 + miR.220a + miR.494 + miR.518c | 0,75 | 0,102280329 |
| miR.609 + miR.603 + miR.519b + miR.518c | 0,75 | 0,033387808 |
| miR.558 + miR.520f + miR.362 + miR.518c | 0,75 | 0,17757916 |
| miR.558 + miR.520f + miR.130a + miR.518c | 0,75 | 0,164603281 |
| miR.609 + miR.603 + miR.518c + miR.338 | 0,75 | 0,040306705 |
| miR.603 + miR.520f + miR.376a* + miR.518c | 0,75 | 0,178218301 |
| miR.609 + miR.520f + miR.29a + miR.660 | 0,75 | 0,108259038 |
| miR.609 + miR.603 + miR.26a + miR.518c | 0,75 | 0,049760274 |
| miR.603 + miR.220a + miR.362 + miR.518c | 0,75 | 0,084267929 |
| miR.603 + miR.376a* + miR.518c + miR.639 | 0,75 | 0,110692495 |
| miR.558 + miR.609 + miR.29a + miR.660 | 0,75 | 0,062339195 |
| miR.609 + miR.603 + miR.518c + miR.660 | 0,75 | 0,03753584 |
| miR.558 + miR.520f + miR.519b + miR.518c | 0,75 | 0,140569368 |
| miR.558 + miR.609 + miR.130a + miR.376a* | 0,75 | 0,155334179 |
| miR.603 + miR.220a + miR.518c + miR.660 | 0,75 | 0,086588446 |
| miR.609 + miR.519b + miR.29a + miR.376a* | 0,75 | 0,083277641 |
| miR.558 + miR.609 + miR.376a* + miR.639 | 0,75 | 0,130880406 |
| miR.558 + miR.220a + miR.376a* + miR.518c | 0,75 | 0,108015506 |
| miR.558 + miR.520f + miR.26a + miR.518c | 0,75 | 0,15060931 |
| miR.558 + miR.609 + miR.220a + miR.29a | 0,75 | 0,099431509 |
| miR.558 + miR.520f + miR.369.3p + miR.518c | 0,75 | 0,145736289 |
| miR.558 + miR.520f + miR.494 + miR.518c | 0,75 | 0,122357549 |
| miR.609 + miR.520f + miR.29a + miR.494 | 0,75 | 0,172325158 |
| miR.558 + miR.609 + miR.29a + miR.369.3p | 0,75 | 0,09238121 |
| miR.558 + miR.609 + miR.130a + miR.29a | 0,75 | 0,072519142 |
| miR.609 + miR.220a + miR.369.3p + miR.518c | 0,75 | 0,020967329 |
| miR.603 + miR.520f + miR.29a + miR.518c | 0,75 | 0,151377195 |
| miR.558 + miR.520f + miR.518c + miR.338 | 0,75 | 0,150058461 |
| miR.558 + miR.520f + miR.518c + miR.660 | 0,75 | 0,132520189 |
| miR.603 + miR.369.3p + miR.376a* + miR.518c | 0,75 | 0,179261881 |
| miR.609 + miR.130a + miR.376a* + miR.518c | 0,75 | 0,043347291 |
| miR.558 + miR.609 + miR.29a + miR.338 | 0,75 | 0,06854081 |
| miR.558 + miR.609 + miR.520f + miR.26a | 0,75 | 0,156943535 |
| miR.609 + miR.603 + miR.520f + miR.29a | 0,75 | 0,095775145 |
| miR.558 + miR.609 + miR.519b + miR.29a | 0,75 | 0,061975286 |
| miR.558 + miR.609 + miR.29a + miR.639 | 0,75 | 0,076366259 |
| miR.558 + miR.609 + miR.29a + miR.494 | 0,75 | 0,103217378 |
| miR.558 + miR.609 + miR.603 + miR.520f | 0,75 | 0,170065251 |
| miR.558 + miR.220a + miR.26a + miR.518c | 0,75 | 0,062062634 |
| miR.609 + miR.29a + miR.376a* + miR.639 | 0,75 | 0,045578904 |
| miR.609 + miR.220a + miR.494 + miR.518c | 0,75 | 0,026701873 |
| miR.609 + miR.29a + miR.362 + miR.376a* | 0,75 | 0,05758252 |
| miR.609 + miR.520f + miR.519b + miR.29a | 0,75 | 0,071806723 |
| miR.609 + miR.520f + miR.220a + miR.29a | 0,75 | 0,066879617 |
| miR.609 + miR.520f + miR.29a + miR.369.3p | 0,75 | 0,105980196 |
| miR.603 + miR.520f + miR.130a + miR.518c | 0,75 | 0,146050405 |
| miR.609 + miR.520f + miR.29a + miR.338 | 0,75 | 0,084787236 |
| miR.609 + miR.520f + miR.29a + miR.639 | 0,75 | 0,105287948 |
| miR.603 + miR.520f + miR.369.3p + miR.518c | 0,75 | 0,15502797 |
| miR.603 + miR.29a + miR.376a* + miR.518c | 0,74 | 0,095638325 |
| miR.609 + miR.376a* + miR.518c + miR.639 | 0,74 | 0,037647912 |
| miR.603 + miR.520f + miR.362 + miR.518c | 0,74 | 0,128652026 |
| miR.558 + miR.609 + miR.29a + miR.362 | 0,74 | 0,073016857 |
| miR.603 + miR.520f + miR.519b + miR.518c | 0,74 | 0,116485645 |
| miR.609 + miR.520f + miR.130a + miR.29a | 0,74 | 0,11856521 |
| miR.609 + miR.519b + miR.518c + miR.639 | 0,74 | 0,01994205 |
| miR.558 + miR.220a + miR.519b + miR.518c | 0,74 | 0,053231829 |
| miR.609 + miR.520f + miR.26a + miR.29a | 0,74 | 0,103809182 |
| miR.603 + miR.520f + miR.26a + miR.518c | 0,74 | 0,121981164 |
| miR.609 + miR.29a + miR.376a* + miR.660 | 0,74 | 0,05046512 |
| miR.609 + miR.520f + miR.29a + miR.362 | 0,74 | 0,092715547 |
| miR.603 + miR.376a* + miR.518c + miR.660 | 0,74 | 0,12721963 |
| miR.603 + miR.520f + miR.518c + miR.338 | 0,74 | 0,12431959 |
| miR.603 + miR.520f + miR.518c + miR.660 | 0,74 | 0,12431959 |
| miR.609 + miR.220a + miR.362 + miR.518c | 0,74 | 0,016326012 |
| miR.603 + miR.520f + miR.494 + miR.518c | 0,74 | 0,136298214 |
| miR.609 + miR.220a + miR.518c + miR.338 | 0,74 | 0,013512608 |
| miR.609 + miR.29a + miR.376a* + miR.338 | 0,74 | 0,050304492 |
| miR.609 + miR.220a + miR.519b + miR.518c | 0,74 | 0,018397 |
| miR.558 + miR.609 + miR.603 + miR.639 | 0,74 | 0,134932573 |
| miR.609 + miR.130a + miR.29a + miR.376a* | 0,74 | 0,078618906 |
| miR.609 + miR.220a + miR.26a + miR.518c | 0,74 | 0,013136055 |
| miR.609 + miR.26a + miR.29a + miR.376a* | 0,74 | 0,033752402 |
| miR.609 + miR.220a + miR.518c + miR.660 | 0,74 | 0,009578358 |
| miR.603 + miR.26a + miR.518c + miR.639 | 0,74 | 0,082856643 |
| miR.558 + miR.220a + miR.518c + miR.338 | 0,74 | 0,051067743 |
| miR.558 + miR.609 + miR.362 + miR.369.3p | 0,74 | 0,098473275 |
| miR.603 + miR.362 + miR.376a* + miR.518c | 0,74 | 0,119681093 |
| miR.520f + miR.220a + miR.518c + miR.639 | 0,74 | 0,066460344 |
| miR.603 + miR.26a + miR.29a + miR.518c | 0,74 | 0,081998228 |
| miR.603 + miR.130a + miR.376a* + miR.518c | 0,74 | 0,089401331 |
| miR.558 + miR.609 + miR.520f + miR.338 | 0,74 | 0,134256712 |
| miR.609 + miR.603 + miR.130a + miR.376a* | 0,74 | 0,104954096 |
| miR.520f + miR.220a + miR.29a + miR.518c | 0,74 | 0,085293212 |
| miR.558 + miR.609 + miR.520f + miR.494 | 0,74 | 0,135588189 |
| miR.609 + miR.369.3p + miR.518c + miR.639 | 0,74 | 0,030410317 |
| miR.609 + miR.519b + miR.130a + miR.518c | 0,74 | 0,030907058 |
| miR.558 + miR.609 + miR.520f + miR.376a* | 0,74 | 0,099852691 |
| miR.558 + miR.609 + miR.520f + miR.220a | 0,74 | 0,120841026 |
| miR.558 + miR.609 + miR.603 + miR.130a | 0,74 | 0,086267578 |
| miR.558 + miR.26a + miR.29a + miR.518c | 0,74 | 0,068441857 |
| miR.603 + miR.376a* + miR.494 + miR.518c | 0,74 | 0,116351712 |
| miR.558 + miR.609 + miR.520f + miR.362 | 0,74 | 0,106771094 |
| miR.558 + miR.220a + miR.369.3p + miR.518c | 0,74 | 0,041229229 |
| miR.609 + miR.130a + miR.518c + miR.639 | 0,74 | 0,013478854 |
| miR.558 + miR.609 + miR.520f + miR.369.3p | 0,74 | 0,115909957 |
| miR.558 + miR.220a + miR.518c + miR.639 | 0,74 | 0,042333948 |
| miR.558 + miR.376a* + miR.518c + miR.639 | 0,74 | 0,074901972 |
| miR.603 + miR.362 + miR.369.3p + miR.518c | 0,74 | 0,117819056 |
| miR.558 + miR.220a + miR.494 + miR.518c | 0,74 | 0,032449642 |
| miR.558 + miR.220a + miR.29a + miR.518c | 0,74 | 0,034843117 |
| miR.609 + miR.494 + miR.518c + miR.639 | 0,74 | 0,030646365 |
| miR.558 + miR.609 + miR.603 + miR.369.3p | 0,74 | 0,17745831 |
| miR.609 + miR.362 + miR.518c + miR.639 | 0,74 | 0,020828142 |
| miR.609 + miR.520f + miR.369.3p + miR.518c | 0,74 | 0,036347494 |
| miR.558 + miR.220a + miR.362 + miR.518c | 0,74 | 0,035052722 |
| miR.558 + miR.26a + miR.518c + miR.639 | 0,74 | 0,055224505 |
| miR.609 + miR.130a + miR.26a + miR.518c | 0,74 | 0,011711735 |
| miR.609 + miR.130a + miR.362 + miR.518c | 0,74 | 0,015579647 |
| miR.558 + miR.609 + miR.603 + miR.220a | 0,74 | 0,094494537 |
| miR.609 + miR.520f + miR.519b + miR.518c | 0,74 | 0,032444677 |
| miR.558 + miR.609 + miR.220a + miR.376a* | 0,74 | 0,077934955 |
| miR.603 + miR.26a + miR.369.3p + miR.518c | 0,74 | 0,078701326 |
| miR.558 + miR.220a + miR.518c + miR.660 | 0,74 | 0,051369854 |
| miR.603 + miR.369.3p + miR.518c + miR.639 | 0,74 | 0,063261821 |
| miR.609 + miR.362 + miR.369.3p + miR.518c | 0,74 | 0,034899386 |
| miR.609 + miR.520f + miR.494 + miR.518c | 0,74 | 0,07781653 |
| miR.558 + miR.29a + miR.376a* + miR.518c | 0,74 | 0,04969725 |
| miR.558 + miR.220a + miR.130a + miR.518c | 0,74 | 0,044650196 |
| miR.603 + miR.518c + miR.639 + miR.338 | 0,74 | 0,055957384 |
| miR.520f + miR.220a + miR.376a* + miR.518c | 0,74 | 0,094204525 |
| miR.558 + miR.609 + miR.369.3p + miR.376a* | 0,74 | 0,11312355 |
| miR.609 + miR.520f + miR.26a + miR.376a* | 0,74 | 0,114301319 |
| miR.609 + miR.603 + miR.520f + miR.130a | 0,74 | 0,103585291 |
| miR.558 + miR.362 + miR.376a* + miR.518c | 0,74 | 0,075181584 |
| miR.609 + miR.520f + miR.362 + miR.518c | 0,74 | 0,034998525 |
| miR.603 + miR.26a + miR.376a* + miR.518c | 0,74 | 0,075475153 |
| miR.603 + miR.376a* + miR.518c + miR.338 | 0,74 | 0,092567256 |
| miR.558 + miR.376a* + miR.518c + miR.660 | 0,74 | 0,058226264 |
| miR.603 + miR.362 + miR.518c + miR.639 | 0,74 | 0,074247111 |
| miR.558 + miR.369.3p + miR.376a* + miR.518c | 0,74 | 0,08001079 |
| miR.603 + miR.519b + miR.376a* + miR.518c | 0,74 | 0,084991001 |
| miR.603 + miR.130a + miR.26a + miR.518c | 0,74 | 0,069975625 |
| miR.520f + miR.220a + miR.130a + miR.518c | 0,74 | 0,064535284 |
| miR.558 + miR.609 + miR.603 + miR.362 | 0,74 | 0,09356382 |
| miR.558 + miR.609 + miR.603 + miR.376a* | 0,74 | 0,100172643 |
| miR.558 + miR.609 + miR.520f + miR.660 | 0,74 | 0,110938722 |
| miR.520f + miR.376a* + miR.518c + miR.639 | 0,74 | 0,098515677 |
| miR.558 + miR.609 + miR.520f + miR.519b | 0,74 | 0,125171619 |
| miR.609 + miR.603 + miR.29a + miR.494 | 0,74 | 0,101151086 |
| miR.609 + miR.518c + miR.639 + miR.338 | 0,74 | 0,01521563 |
| miR.520f + miR.220a + miR.26a + miR.518c | 0,74 | 0,070228502 |
| miR.609 + miR.130a + miR.369.3p + miR.518c | 0,74 | 0,02414883 |
| miR.558 + miR.609 + miR.362 + miR.639 | 0,74 | 0,076219252 |
| miR.520f + miR.220a + miR.369.3p + miR.518c | 0,74 | 0,066978308 |
| miR.558 + miR.609 + miR.362 + miR.376a* | 0,74 | 0,056518304 |
| miR.609 + miR.130a + miR.518c + miR.338 | 0,74 | 0,009388196 |
| miR.609 + miR.26a + miR.518c + miR.639 | 0,74 | 0,012827359 |
| miR.558 + miR.609 + miR.603 + miR.494 | 0,74 | 0,124215101 |
| miR.609 + miR.520f + miR.518c + miR.338 | 0,74 | 0,030672998 |
| miR.603 + miR.29a + miR.369.3p + miR.518c | 0,74 | 0,071151405 |
| miR.603 + miR.29a + miR.518c + miR.639 | 0,74 | 0,058594387 |
| miR.603 + miR.519b + miR.518c + miR.639 | 0,74 | 0,066748811 |
| miR.520f + miR.519b + miR.518c + miR.639 | 0,74 | 0,054222827 |
| miR.603 + miR.494 + miR.518c + miR.639 | 0,74 | 0,076089769 |
| miR.558 + miR.609 + miR.220a + miR.369.3p | 0,74 | 0,090077117 |
| miR.603 + miR.369.3p + miR.518c + miR.338 | 0,74 | 0,071015847 |
| miR.558 + miR.609 + miR.369.3p + miR.639 | 0,74 | 0,095032576 |
| miR.603 + miR.130a + miR.518c + miR.639 | 0,74 | 0,072705347 |
| miR.609 + miR.518c + miR.639 + miR.660 | 0,74 | 0,013137355 |
| miR.558 + miR.609 + miR.376a* + miR.494 | 0,74 | 0,159926065 |
| miR.520f + miR.220a + miR.362 + miR.518c | 0,74 | 0,055403772 |
| miR.558 + miR.609 + miR.130a + miR.362 | 0,74 | 0,05851125 |
| miR.603 + miR.518c + miR.639 + miR.660 | 0,74 | 0,064717331 |
| miR.609 + miR.520f + miR.130a + miR.639 | 0,74 | 0,083646158 |
| miR.609 + miR.520f + miR.518c + miR.660 | 0,74 | 0,026861186 |
| miR.603 + miR.26a + miR.362 + miR.518c | 0,74 | 0,063115644 |
| miR.520f + miR.220a + miR.518c + miR.338 | 0,74 | 0,047670481 |
| miR.603 + miR.519b + miR.369.3p + miR.518c | 0,74 | 0,073788459 |
| miR.520f + miR.220a + miR.519b + miR.518c | 0,74 | 0,052030294 |
| miR.609 + miR.603 + miR.520f + miR.639 | 0,74 | 0,094165599 |
| miR.609 + miR.520f + miR.376a* + miR.639 | 0,74 | 0,118141535 |
| miR.558 + miR.609 + miR.220a + miR.639 | 0,74 | 0,072866358 |
| miR.603 + miR.29a + miR.518c + miR.338 | 0,74 | 0,043282333 |
| miR.609 + miR.130a + miR.518c + miR.660 | 0,74 | 0,010119594 |
| miR.558 + miR.362 + miR.369.3p + miR.518c | 0,74 | 0,076227533 |
| miR.558 + miR.609 + miR.220a + miR.130a | 0,74 | 0,05483949 |
| miR.603 + miR.29a + miR.362 + miR.518c | 0,74 | 0,05582789 |
| miR.558 + miR.609 + miR.220a + miR.362 | 0,74 | 0,047620739 |
| miR.520f + miR.220a + miR.494 + miR.518c | 0,74 | 0,06173118 |
| miR.520f + miR.29a + miR.518c + miR.639 | 0,74 | 0,084401779 |
| miR.603 + miR.369.3p + miR.518c + miR.660 | 0,74 | 0,066259739 |
| miR.603 + miR.130a + miR.369.3p + miR.518c | 0,74 | 0,066259739 |
| miR.558 + miR.26a + miR.362 + miR.518c | 0,74 | 0,064332146 |
| miR.520f + miR.26a + miR.518c + miR.639 | 0,74 | 0,074182159 |
| miR.558 + miR.609 + miR.130a + miR.369.3p | 0,74 | 0,066572075 |
| miR.520f + miR.29a + miR.376a* + miR.518c | 0,74 | 0,128861379 |
| miR.520f + miR.362 + miR.518c + miR.639 | 0,74 | 0,05445848 |
| miR.558 + miR.609 + miR.362 + miR.494 | 0,74 | 0,09032679 |
| miR.558 + miR.609 + miR.603 + miR.660 | 0,74 | 0,101202769 |
| miR.609 + miR.520f + miR.26a + miR.639 | 0,74 | 0,087783637 |
| miR.558 + miR.609 + miR.130a + miR.639 | 0,74 | 0,063069995 |
| miR.520f + miR.220a + miR.518c + miR.660 | 0,74 | 0,065363815 |
| miR.603 + miR.369.3p + miR.494 + miR.518c | 0,74 | 0,070213824 |
| miR.603 + miR.519b + miR.362 + miR.518c | 0,74 | 0,045382411 |
| miR.603 + miR.26a + miR.518c + miR.660 | 0,74 | 0,060830051 |
| miR.609 + miR.130a + miR.494 + miR.518c | 0,74 | 0,024287975 |
| miR.558 + miR.130a + miR.29a + miR.518c | 0,74 | 0,045640866 |
| miR.558 + miR.609 + miR.494 + miR.639 | 0,74 | 0,099348006 |
| miR.603 + miR.130a + miR.29a + miR.518c | 0,74 | 0,045398461 |
| miR.603 + miR.362 + miR.518c + miR.338 | 0,74 | 0,069621505 |
| miR.603 + miR.519b + miR.29a + miR.518c | 0,74 | 0,055764584 |
| miR.558 + miR.609 + miR.603 + miR.26a | 0,74 | 0,109309598 |
| miR.558 + miR.609 + miR.603 + miR.338 | 0,74 | 0,107471247 |
| miR.609 + miR.519b + miR.369.3p + miR.518c | 0,74 | 0,024341477 |
| miR.603 + miR.130a + miR.362 + miR.518c | 0,73 | 0,055400192 |
| miR.609 + miR.362 + miR.494 + miR.518c | 0,73 | 0,043500436 |
| miR.520f + miR.369.3p + miR.518c + miR.639 | 0,73 | 0,066107433 |
| miR.558 + miR.609 + miR.603 + miR.519b | 0,73 | 0,101532949 |
| miR.603 + miR.362 + miR.494 + miR.518c | 0,73 | 0,080810581 |
| miR.609 + miR.603 + miR.220a + miR.29a | 0,73 | 0,030911624 |
| miR.558 + miR.26a + miR.376a* + miR.518c | 0,73 | 0,065170768 |
| miR.558 + miR.130a + miR.376a* + miR.518c | 0,73 | 0,062083572 |
| miR.558 + miR.26a + miR.369.3p + miR.518c | 0,73 | 0,073399404 |
| miR.520f + miR.518c + miR.639 + miR.660 | 0,73 | 0,075619421 |
| miR.609 + miR.520f + miR.130a + miR.26a | 0,73 | 0,097353957 |
| miR.603 + miR.26a + miR.494 + miR.518c | 0,73 | 0,077581338 |
| miR.558 + miR.362 + miR.518c + miR.639 | 0,73 | 0,033826398 |
| miR.603 + miR.29a + miR.494 + miR.518c | 0,73 | 0,060683834 |
| miR.609 + miR.603 + miR.29a + miR.369.3p | 0,73 | 0,065959119 |
| miR.520f + miR.494 + miR.518c + miR.639 | 0,73 | 0,074944414 |
| miR.520f + miR.26a + miR.376a* + miR.518c | 0,73 | 0,058205606 |
| miR.520f + miR.130a + miR.518c + miR.639 | 0,73 | 0,060947304 |
| miR.603 + miR.518c + miR.660 + miR.338 | 0,73 | 0,056606581 |
| miR.609 + miR.603 + miR.376a* + miR.639 | 0,73 | 0,115496164 |
| miR.520f + miR.518c + miR.639 + miR.338 | 0,73 | 0,063945903 |
| miR.603 + miR.362 + miR.518c + miR.660 | 0,73 | 0,05818279 |
| miR.558 + miR.519b + miR.376a* + miR.518c | 0,73 | 0,045554382 |
| miR.558 + miR.376a* + miR.518c + miR.338 | 0,73 | 0,055855034 |
| miR.558 + miR.130a + miR.26a + miR.518c | 0,73 | 0,042176299 |
| miR.558 + miR.609 + miR.376a* + miR.660 | 0,73 | 0,044024045 |
| miR.603 + miR.519b + miR.26a + miR.518c | 0,73 | 0,062257825 |
| miR.609 + miR.519b + miR.376a* + miR.518c | 0,73 | 0,015982589 |
| miR.609 + miR.520f + miR.130a + miR.660 | 0,73 | 0,101833469 |
| miR.609 + miR.520f + miR.130a + miR.369.3p | 0,73 | 0,109412444 |
| miR.609 + miR.519b + miR.362 + miR.518c | 0,73 | 0,013056845 |
| miR.603 + miR.29a + miR.518c + miR.660 | 0,73 | 0,059519308 |
| miR.609 + miR.520f + miR.130a + miR.362 | 0,73 | 0,068097694 |
| miR.558 + miR.609 + miR.130a + miR.26a | 0,73 | 0,057495804 |
| miR.609 + miR.26a + miR.29a + miR.494 | 0,73 | 0,042038841 |
| miR.603 + miR.26a + miR.518c + miR.338 | 0,73 | 0,051744542 |
| miR.609 + miR.130a + miR.362 + miR.376a* | 0,73 | 0,082310119 |
| miR.609 + miR.520f + miR.220a + miR.130a | 0,73 | 0,0878706 |
| miR.609 + miR.220a + miR.130a + miR.376a* | 0,73 | 0,044324572 |
| miR.609 + miR.520f + miR.519b + miR.130a | 0,73 | 0,090034683 |
| miR.609 + miR.520f + miR.130a + miR.494 | 0,73 | 0,096237724 |
| miR.220a + miR.26a + miR.29a + miR.518c | 0,73 | 0,028305867 |
| miR.558 + miR.376a* + miR.494 + miR.518c | 0,73 | 0,049466424 |
| miR.558 + miR.609 + miR.519b + miR.639 | 0,73 | 0,059730051 |
| miR.603 + miR.130a + miR.518c + miR.338 | 0,73 | 0,037054069 |
| miR.558 + miR.609 + miR.26a + miR.639 | 0,73 | 0,062921851 |
| miR.558 + miR.29a + miR.362 + miR.518c | 0,73 | 0,030396599 |
| miR.603 + miR.519b + miR.518c + miR.660 | 0,73 | 0,036802339 |
| miR.558 + miR.609 + miR.220a + miR.494 | 0,73 | 0,089461022 |
| miR.603 + miR.519b + miR.494 + miR.518c | 0,73 | 0,054177507 |
| miR.558 + miR.609 + miR.639 + miR.338 | 0,73 | 0,063619561 |
| miR.603 + miR.494 + miR.518c + miR.338 | 0,73 | 0,073653458 |
| miR.609 + miR.520f + miR.376a* + miR.518c | 0,73 | 0,022392935 |
| miR.609 + miR.520f + miR.130a + miR.338 | 0,73 | 0,102375209 |
| miR.609 + miR.29a + miR.362 + miR.660 | 0,73 | 0,005505747 |
| miR.558 + miR.26a + miR.518c + miR.660 | 0,73 | 0,049765564 |
| miR.609 + miR.520f + miR.494 + miR.639 | 0,73 | 0,085074805 |
| miR.558 + miR.609 + miR.130a + miR.494 | 0,73 | 0,054027886 |
| miR.603 + miR.130a + miR.518c + miR.660 | 0,73 | 0,051150114 |
| miR.558 + miR.519b + miR.362 + miR.518c | 0,73 | 0,037444677 |
| miR.603 + miR.519b + miR.518c + miR.338 | 0,73 | 0,047404685 |
| miR.558 + miR.609 + miR.639 + miR.660 | 0,73 | 0,067591843 |
| miR.603 + miR.519b + miR.130a + miR.518c | 0,73 | 0,050878399 |
| miR.558 + miR.609 + miR.362 + miR.660 | 0,73 | 0,048363097 |
| miR.603 + miR.494 + miR.518c + miR.660 | 0,73 | 0,070813234 |
| miR.609 + miR.603 + miR.29a + miR.660 | 0,73 | 0,017966954 |
| miR.558 + miR.518c + miR.639 + miR.338 | 0,73 | 0,031060775 |
| miR.609 + miR.603 + miR.376a* + miR.494 | 0,73 | 0,155790044 |
| miR.609 + miR.220a + miR.29a + miR.494 | 0,73 | 0,037062083 |
| miR.520f + miR.519b + miR.29a + miR.518c | 0,73 | 0,067754735 |
| miR.609 + miR.603 + miR.29a + miR.639 | 0,73 | 0,037384146 |
| miR.603 + miR.130a + miR.494 + miR.518c | 0,73 | 0,069744604 |
| miR.609 + miR.603 + miR.26a + miR.29a | 0,73 | 0,028652026 |
| miR.558 + miR.609 + miR.130a + miR.338 | 0,73 | 0,038234098 |
| miR.558 + miR.609 + miR.519b + miR.130a | 0,73 | 0,042415851 |
| miR.558 + miR.609 + miR.130a + miR.660 | 0,73 | 0,045579859 |
| miR.558 + miR.609 + miR.220a + miR.338 | 0,73 | 0,058298105 |
| miR.609 + miR.603 + miR.29a + miR.338 | 0,73 | 0,020377726 |
| miR.520f + miR.29a + miR.518c + miR.660 | 0,73 | 0,075863818 |
| miR.558 + miR.362 + miR.518c + miR.660 | 0,73 | 0,047026908 |
| miR.220a + miR.369.3p + miR.376a* + miR.518c | 0,73 | 0,068113783 |
| miR.558 + miR.609 + miR.362 + miR.338 | 0,73 | 0,042500706 |
| miR.520f + miR.29a + miR.362 + miR.518c | 0,73 | 0,09084255 |
| miR.558 + miR.362 + miR.494 + miR.518c | 0,73 | 0,030321617 |
| miR.558 + miR.609 + miR.220a + miR.26a | 0,73 | 0,058239393 |
| miR.558 + miR.362 + miR.518c + miR.338 | 0,73 | 0,038933989 |
| miR.609 + miR.519b + miR.494 + miR.518c | 0,73 | 0,017166781 |
| miR.558 + miR.609 + miR.220a + miR.519b | 0,73 | 0,05863021 |
| miR.558 + miR.519b + miR.26a + miR.518c | 0,73 | 0,05010813 |
| miR.609 + miR.603 + miR.520f + miR.26a | 0,73 | 0,09381255 |
| miR.558 + miR.609 + miR.220a + miR.660 | 0,73 | 0,058239393 |
| miR.609 + miR.130a + miR.29a + miR.494 | 0,73 | 0,037976088 |
| miR.220a + miR.29a + miR.376a* + miR.518c | 0,73 | 0,025191606 |
| miR.558 + miR.609 + miR.376a* + miR.338 | 0,73 | 0,057703944 |
| miR.609 + miR.29a + miR.494 + miR.660 | 0,73 | 0,035778842 |
| miR.220a + miR.29a + miR.518c + miR.338 | 0,73 | 0,027143718 |
| miR.558 + miR.130a + miR.518c + miR.639 | 0,73 | 0,036393513 |
| miR.609 + miR.520f + miR.26a + miR.494 | 0,73 | 0,097028554 |
| miR.558 + miR.369.3p + miR.518c + miR.639 | 0,73 | 0,049119107 |
| miR.558 + miR.609 + miR.26a + miR.362 | 0,73 | 0,038073012 |
| miR.558 + miR.29a + miR.518c + miR.639 | 0,73 | 0,038052152 |
| miR.558 + miR.519b + miR.518c + miR.639 | 0,73 | 0,032629944 |
| miR.609 + miR.520f + miR.519b + miR.639 | 0,73 | 0,069677371 |
| miR.558 + miR.29a + miR.518c + miR.338 | 0,73 | 0,023717845 |
| miR.558 + miR.130a + miR.362 + miR.518c | 0,73 | 0,033933391 |
| miR.558 + miR.609 + miR.369.3p + miR.494 | 0,73 | 0,09121593 |
| miR.609 + miR.603 + miR.130a + miR.29a | 0,73 | 0,03420256 |
| miR.520f + miR.26a + miR.29a + miR.518c | 0,73 | 0,067269802 |
| miR.520f + miR.29a + miR.518c + miR.338 | 0,73 | 0,076669475 |
| miR.609 + miR.603 + miR.519b + miR.29a | 0,73 | 0,028546438 |
| miR.609 + miR.369.3p + miR.494 + miR.518c | 0,73 | 0,028830831 |
| miR.558 + miR.609 + miR.519b + miR.362 | 0,73 | 0,038229586 |
| miR.558 + miR.494 + miR.518c + miR.639 | 0,73 | 0,032661435 |
| miR.558 + miR.519b + miR.29a + miR.518c | 0,73 | 0,033339954 |
| miR.609 + miR.29a + miR.369.3p + miR.494 | 0,73 | 0,056013402 |
| miR.520f + miR.29a + miR.369.3p + miR.518c | 0,73 | 0,08584984 |
| miR.558 + miR.609 + miR.26a + miR.376a* | 0,73 | 0,045281352 |
| miR.520f + miR.130a + miR.29a + miR.518c | 0,73 | 0,08778147 |
| miR.609 + miR.220a + miR.26a + miR.29a | 0,73 | 0,011664266 |
| miR.609 + miR.362 + miR.369.3p + miR.376a* | 0,73 | 0,148943316 |
| miR.609 + miR.220a + miR.29a + miR.369.3p | 0,73 | 0,024156115 |
| miR.558 + miR.26a + miR.494 + miR.518c | 0,73 | 0,054062325 |
| miR.558 + miR.518c + miR.639 + miR.660 | 0,73 | 0,038428075 |
| miR.609 + miR.29a + miR.362 + miR.369.3p | 0,73 | 0,040814635 |
| miR.609 + miR.603 + miR.29a + miR.362 | 0,73 | 0,034280118 |
| miR.558 + miR.609 + miR.369.3p + miR.660 | 0,73 | 0,068825487 |
| miR.609 + miR.29a + miR.494 + miR.338 | 0,73 | 0,026047031 |
| miR.558 + miR.26a + miR.518c + miR.338 | 0,73 | 0,055344824 |
| miR.558 + miR.29a + miR.494 + miR.518c | 0,73 | 0,022491443 |
| miR.609 + miR.519b + miR.518c + miR.660 | 0,73 | 0,00782303 |
| miR.520f + miR.29a + miR.494 + miR.518c | 0,73 | 0,078245802 |
| miR.609 + miR.220a + miR.29a + miR.660 | 0,73 | 0,006263507 |
| miR.558 + miR.29a + miR.369.3p + miR.518c | 0,73 | 0,046525542 |
| miR.558 + miR.609 + miR.369.3p + miR.338 | 0,73 | 0,086904488 |
| miR.609 + miR.29a + miR.362 + miR.494 | 0,73 | 0,054141582 |
| miR.609 + miR.603 + miR.520f + miR.494 | 0,73 | 0,104015054 |
| miR.609 + miR.520f + miR.362 + miR.639 | 0,73 | 0,072460936 |
| miR.609 + miR.520f + miR.639 + miR.338 | 0,73 | 0,074374157 |
| miR.609 + miR.29a + miR.494 + miR.639 | 0,73 | 0,042566365 |
| miR.609 + miR.519b + miR.26a + miR.518c | 0,73 | 0,006647307 |
| miR.520f + miR.130a + miR.376a* + miR.518c | 0,73 | 0,04859414 |
| miR.609 + miR.519b + miR.518c + miR.338 | 0,73 | 0,006807561 |
| miR.609 + miR.220a + miR.29a + miR.338 | 0,73 | 0,015999341 |
| miR.609 + miR.519b + miR.29a + miR.494 | 0,73 | 0,040745552 |
| miR.558 + miR.29a + miR.518c + miR.660 | 0,73 | 0,030602068 |
| miR.558 + miR.609 + miR.26a + miR.369.3p | 0,73 | 0,081066424 |
| miR.609 + miR.519b + miR.130a + miR.376a* | 0,73 | 0,060025821 |
| miR.558 + miR.609 + miR.519b + miR.369.3p | 0,73 | 0,077410062 |
| miR.558 + miR.519b + miR.369.3p + miR.518c | 0,73 | 0,053466843 |
| miR.609 + miR.26a + miR.362 + miR.518c | 0,73 | 0,009256496 |
| miR.609 + miR.369.3p + miR.518c + miR.660 | 0,73 | 0,010362334 |
| miR.558 + miR.369.3p + miR.494 + miR.518c | 0,73 | 0,042544066 |
| miR.558 + miR.519b + miR.518c + miR.660 | 0,73 | 0,033810946 |
| miR.609 + miR.520f + miR.220a + miR.639 | 0,73 | 0,057658231 |
| miR.520f + miR.519b + miR.130a + miR.518c | 0,73 | 0,043576783 |
| miR.609 + miR.362 + miR.376a* + miR.518c | 0,73 | 0,003926436 |
| miR.520f + miR.362 + miR.369.3p + miR.518c | 0,73 | 0,07167175 |
| miR.558 + miR.369.3p + miR.518c + miR.338 | 0,73 | 0,03659566 |
| miR.220a + miR.518c + miR.639 + miR.338 | 0,73 | 0,008625653 |
| miR.520f + miR.26a + miR.362 + miR.518c | 0,73 | 0,042129104 |
| miR.609 + miR.603 + miR.362 + miR.369.3p | 0,73 | 0,066110609 |
| miR.520f + miR.130a + miR.362 + miR.518c | 0,73 | 0,066757079 |
| miR.558 + miR.369.3p + miR.518c + miR.660 | 0,73 | 0,04576864 |
| miR.609 + miR.362 + miR.518c + miR.660 | 0,73 | 0,005445028 |
| miR.609 + miR.520f + miR.369.3p + miR.639 | 0,73 | 0,060200502 |
| miR.609 + miR.220a + miR.519b + miR.29a | 0,73 | 0,017829862 |
| miR.220a + miR.376a* + miR.518c + miR.639 | 0,73 | 0,024536373 |
| miR.609 + miR.220a + miR.29a + miR.639 | 0,73 | 0,017469826 |
| miR.609 + miR.220a + miR.130a + miR.29a | 0,73 | 0,016035157 |
| miR.558 + miR.518c + miR.660 + miR.338 | 0,73 | 0,026559768 |
| miR.220a + miR.26a + miR.369.3p + miR.518c | 0,73 | 0,023284351 |
| miR.520f + miR.130a + miR.26a + miR.518c | 0,73 | 0,049486625 |
| miR.609 + miR.519b + miR.376a* + miR.639 | 0,73 | 0,074001601 |
| miR.609 + miR.362 + miR.518c + miR.338 | 0,73 | 0,005669306 |
| miR.609 + miR.494 + miR.518c + miR.660 | 0,73 | 0,017339014 |
| miR.558 + miR.130a + miR.369.3p + miR.518c | 0,73 | 0,04801593 |
| miR.558 + miR.519b + miR.518c + miR.338 | 0,73 | 0,022642243 |
| miR.609 + miR.603 + miR.494 + miR.639 | 0,73 | 0,099375105 |
| miR.609 + miR.519b + miR.26a + miR.29a | 0,73 | 0,009115276 |
| miR.558 + miR.603 + miR.520f + miR.639 | 0,73 | 0,111635217 |
| miR.609 + miR.130a + miR.369.3p + miR.376a* | 0,73 | 0,094334817 |
| miR.520f + miR.519b + miR.26a + miR.518c | 0,73 | 0,036616858 |
| miR.558 + miR.494 + miR.518c + miR.660 | 0,73 | 0,0209133 |
| miR.558 + miR.519b + miR.494 + miR.518c | 0,73 | 0,029778652 |
| miR.220a + miR.369.3p + miR.518c + miR.338 | 0,73 | 0,024792375 |
| miR.609 + miR.220a + miR.29a + miR.362 | 0,73 | 0,017908865 |
| miR.220a + miR.130a + miR.26a + miR.518c | 0,73 | 0,020876153 |
| miR.609 + miR.520f + miR.639 + miR.660 | 0,73 | 0,055728073 |
| miR.609 + miR.26a + miR.29a + miR.362 | 0,73 | 0,006368033 |
| miR.558 + miR.519b + miR.130a + miR.518c | 0,73 | 0,027551883 |
| miR.558 + miR.609 + miR.494 + miR.660 | 0,73 | 0,065683851 |
| miR.609 + miR.603 + miR.130a + miR.369.3p | 0,73 | 0,058944345 |
| miR.220a + miR.26a + miR.518c + miR.639 | 0,73 | 0,01481234 |
| miR.609 + miR.369.3p + miR.376a* + miR.518c | 0,73 | 0,014701023 |
| miR.520f + miR.26a + miR.369.3p + miR.518c | 0,73 | 0,034621629 |
| miR.520f + miR.26a + miR.518c + miR.660 | 0,73 | 0,029220716 |
| miR.609 + miR.519b + miR.29a + miR.369.3p | 0,73 | 0,023972747 |
| miR.609 + miR.603 + miR.220a + miR.130a | 0,73 | 0,034829252 |
| miR.558 + miR.130a + miR.518c + miR.660 | 0,73 | 0,030952624 |
| miR.220a + miR.130a + miR.376a* + miR.518c | 0,73 | 0,028758485 |
| miR.609 + miR.26a + miR.29a + miR.639 | 0,73 | 0,007463068 |
| miR.609 + miR.26a + miR.29a + miR.369.3p | 0,73 | 0,015898228 |
| miR.558 + miR.609 + miR.26a + miR.494 | 0,73 | 0,068523648 |
| miR.520f + miR.26a + miR.494 + miR.518c | 0,73 | 0,044919939 |
| miR.520f + miR.519b + miR.362 + miR.518c | 0,73 | 0,048769905 |
| miR.520f + miR.26a + miR.518c + miR.338 | 0,73 | 0,029715495 |
| miR.609 + miR.603 + miR.369.3p + miR.376a* | 0,73 | 0,077237275 |
| miR.558 + miR.609 + miR.494 + miR.338 | 0,73 | 0,077101971 |
| miR.609 + miR.26a + miR.494 + miR.518c | 0,73 | 0,017895554 |
| miR.558 + miR.603 + miR.520f + miR.29a | 0,73 | 0,109254688 |
| miR.609 + miR.376a* + miR.494 + miR.639 | 0,73 | 0,136796764 |
| miR.520f + miR.130a + miR.369.3p + miR.518c | 0,73 | 0,054306405 |
| miR.609 + miR.603 + miR.130a + miR.494 | 0,73 | 0,066990008 |
| miR.609 + miR.29a + miR.369.3p + miR.338 | 0,73 | 0,012834623 |

**Table 5: clusters of 5 miRNAs**

| **Cluster of 5 miRs** | **c_tau level** | **pv compared to top signature miR.558 + miR.609 + miR.29a + miR.376a* + miR.518c** |
|---|---|---|
| miR.558 + miR.609 + miR.29a + miR.376a* + miR.518c | 0,79 | NaN |
| miR.609 + miR.603 + miR.29a + miR.376a* + miR.518c | 0,79 | 0,327442837 |
| miR.558 + miR.609 + miR.520f + miR.29a + miR.376a* | 0,79 | 0,327799124 |
| miR.609 + miR.220a + miR.29a + miR.376a* + miR.518c | 0,79 | 0,01115903 |
| miR.609 + miR.520f + miR.29a + miR.376a* + miR.518c | 0,79 | 0,450779905 |
| miR.558 + miR.609 + miR.520f + miR.29a + miR.518c | 0,78 | 0,059817433 |
| miR.558 + miR.609 + miR.26a + miR.29a + miR.518c | 0,78 | 0,043671541 |
| miR.558 + miR.609 + miR.376a* + miR.518c + miR.639 | 0,78 | 0,23022043 |
| miR.609 + miR.519b + miR.29a + miR.376a* + miR.518c | 0,78 | 0,188539884 |
| miR.558 + miR.609 + miR.29a + miR.518c + miR.660 | 0,78 | 0,002653657 |
| miR.609 + miR.520f + miR.29a + miR.376a* + miR.494 | 0,78 | 0,249066295 |
| miR.558 + miR.609 + miR.29a + miR.369.3p + miR.376a* | 0,78 | 0,158036688 |
| miR.558 + miR.609 + miR.520f + miR.518c + miR.639 | 0,78 | 0,063513262 |
| miR.609 + miR.29a + miR.369.3p + miR.376a* + miR.518c | 0,78 | 0,143839599 |
| miR.558 + miR.609 + miR.130a + miR.376a* + miR.518c | 0,78 | 0,110267568 |
| miR.558 + miR.609 + miR.603 + miR.376a* + miR.518c | 0,78 | 0,035625688 |
| miR.609 + miR.520f + miR.29a + miR.518c + miR.660 | 0,78 | 0,067617987 |
| miR.609 + miR.29a + miR.376a* + miR.494 + miR.518c | 0,77 | 0,350446224 |
| miR.558 + miR.609 + miR.603 + miR.29a + miR.518c | 0,77 | 0,016877074 |
| miR.609 + miR.603 + miR.130a + miR.376a* + miR.518c | 0,77 | 0,041540235 |
| miR.609 + miR.603 + miR.376a* + miR.518c + miR.639 | 0,77 | 0,026897842 |
| miR.558 + miR.609 + miR.220a + miR.29a + miR.376a* | 0,77 | 0,017879839 |
| miR.558 + miR.609 + miR.220a + miR.29a + miR.518c | 0,77 | 0,006280085 |
| miR.558 + miR.609 + miR.29a + miR.518c + miR.338 | 0,77 | 0,011101684 |
| miR.558 + miR.609 + miR.29a + miR.376a* + miR.494 | 0,77 | 0,062612995 |
| miR.558 + miR.609 + miR.603 + miR.520f + miR.518c | 0,77 | 0,03443809 |
| miR.558 + miR.609 + miR.220a + miR.376a* + miR.518c | 0,77 | 0,033997596 |
| miR.558 + miR.609 + miR.130a + miR.29a + miR.518c | 0,77 | 0,002887257 |
| miR.558 + miR.609 + miR.520f + miR.130a + miR.518c | 0,77 | 0,037877132 |
| miR.558 + miR.609 + miR.603 + miR.220a + miR.518c | 0,77 | 0,017976338 |
| miR.609 + miR.520f + miR.29a + miR.376a* + miR.338 | 0,77 | 0,407308292 |
| miR.609 + miR.29a + miR.376a* + miR.518c + miR.639 | 0,77 | 0,17469374 |
| miR.558 + miR.609 + miR.26a + miR.29a + miR.376a* | 0,77 | 0,008922532 |
| miR.558 + miR.609 + miR.520f + miR.130a + miR.376a* | 0,77 | 0,140289539 |
| miR.609 + miR.520f + miR.130a + miR.376a* + miR.518c | 0,77 | 0,059066796 |
| miR.558 + miR.609 + miR.603 + miR.518c + miR.639 | 0,77 | 0,027137149 |
| miR.609 + miR.520f + miR.29a + miR.369.3p + miR.376a* | 0,77 | 0,373692037 |
| miR.609 + miR.520f + miR.26a + miR.29a + miR.376a* | 0,77 | 0,366136004 |
| miR.558 + miR.609 + miR.29a + miR.376a* + miR.639 | 0,77 | 0,020223844 |
| miR.558 + miR.609 + miR.369.3p + miR.376a* + miR.518c | 0,77 | 0,167914542 |
| miR.609 + miR.29a + miR.376a* + miR.518c + miR.660 | 0,77 | 0,113571149 |
| miR.609 + miR.220a + miR.130a + miR.376a* + miR.518c | 0,77 | 0,028574731 |
| miR.609 + miR.26a + miR.29a + miR.376a* + miR.518c | 0,77 | 0,157501218 |
| miR.609 + miR.520f + miR.130a + miR.29a + miR.376a* | 0,77 | 0,231742772 |
| miR.609 + miR.29a + miR.362 + miR.376a* + miR.518c | 0,77 | 0,185874123 |
| miR.609 + miR.520f + miR.519b + miR.29a + miR.376a* | 0,77 | 0,192784408 |
| miR.609 + miR.29a + miR.376a* + miR.518c + miR.338 | 0,77 | 0,133790333 |
| miR.609 + miR.130a + miR.29a + miR.376a* + miR.518c | 0,77 | 0,174916686 |
| miR.558 + miR.609 + miR.603 + miR.29a + miR.376a* | 0,77 | 0,031799615 |
| miR.558 + miR.609 + miR.29a + miR.369.3p + miR.518c | 0,77 | 0,005160508 |
| miR.609 + miR.603 + miR.220a + miR.376a* + miR.518c | 0,77 | 0,04671063 |
| miR.609 + miR.603 + miR.520f + miR.29a + miR.518c | 0,77 | 0,099573877 |
| miR.609 + miR.603 + miR.520f + miR.29a + miR.376a* | 0,77 | 0,37830202 |
| miR.558 + miR.609 + miR.603 + miR.369.3p + miR.518c | 0,77 | 0,028220566 |
| miR.558 + miR.603 + miR.220a + miR.376a* + miR.518c | 0,77 | 0,03980927 |
| miR.558 + miR.609 + miR.519b + miR.29a + miR.518c | 0,77 | 0,007674366 |
| miR.558 + miR.609 + miR.29a + miR.362 + miR.518c | 0,77 | 0,004377824 |
| miR.558 + miR.609 + miR.29a + miR.518c + miR.639 | 0,77 | 0,005411378 |
| miR.558 + miR.609 + miR.29a + miR.494 + miR.518c | 0,77 | 0,004831169 |
| miR.558 + miR.609 + miR.603 + miR.130a + miR.518c | 0,77 | 0,017870286 |
| miR.558 + miR.609 + miR.603 + miR.519b + miR.518c | 0,77 | 0,027427599 |
| miR.609 + miR.520f + miR.220a + miR.29a + miR.518c | 0,77 | 0,048133374 |
| miR.558 + miR.609 + miR.520f + miR.220a + miR.518c | 0,77 | 0,026797673 |
| miR.558 + miR.609 + miR.520f + miR.376a* + miR.518c | 0,77 | 0,100903248 |
| miR.558 + miR.609 + miR.26a + miR.518c + miR.639 | 0,77 | 0,095109646 |
| miR.558 + miR.609 + miR.29a + miR.362 + miR.376a* | 0,77 | 0,013602017 |
| miR.558 + miR.609 + miR.29a + miR.376a* + miR.660 | 0,77 | 0,018468784 |
| miR.558 + miR.609 + miR.29a + miR.376a* + miR.338 | 0,77 | 0,0121737 |
| miR.558 + miR.609 + miR.520f + miR.29a + miR.660 | 0,77 | 0,066139496 |
| miR.609 + miR.520f + miR.220a + miR.29a + miR.376a* | 0,77 | 0,225976847 |
| miR.609 + miR.520f + miR.29a + miR.376a* + miR.639 | 0,77 | 0,28280271 |
| miR.558 + miR.609 + miR.520f + miR.26a + miR.518c | 0,77 | 0,024846111 |
| miR.558 + miR.609 + miR.520f + miR.369.3p + miR.518c | 0,77 | 0,041924518 |
| miR.609 + miR.520f + miR.29a + miR.518c + miR.639 | 0,77 | 0,033274671 |
| miR.609 + miR.520f + miR.29a + miR.376a* + miR.660 | 0,77 | 0,253849844 |
| miR.609 + miR.220a + miR.29a + miR.518c + miR.660 | 0,77 | 0,001466597 |
| miR.558 + miR.609 + miR.519b + miR.29a + miR.376a* | 0,77 | 0,021908997 |
| miR.558 + miR.609 + miR.130a + miR.29a + miR.376a* | 0,77 | 0,01948068 |
| miR.609 + miR.520f + miR.29a + miR.518c + miR.338 | 0,77 | 0,022546602 |
| miR.609 + miR.520f + miR.29a + miR.362 + miR.518c | 0,77 | 0,035845969 |
| miR.609 + miR.520f + miR.29a + miR.362 + miR.376a* | 0,77 | 0,235677599 |
| miR.609 + miR.603 + miR.220a + miR.29a + miR.518c | 0,77 | 0,02363119 |
| miR.558 + miR.609 + miR.603 + miR.26a + miR.518c | 0,77 | 0,029204349 |
| miR.609 + miR.520f + miR.29a + miR.494 + miR.518c | 0,77 | 0,034015223 |
| miR.558 + miR.609 + miR.603 + miR.518c + miR.338 | 0,77 | 0,023944369 |
| miR.558 + miR.603 + miR.220a + miR.519b + miR.518c | 0,77 | 0,046578651 |
| miR.609 + miR.603 + miR.369.3p + miR.376a* + miR.518c | 0,77 | 0,036178907 |
| miR.609 + miR.520f + miR.29a + miR.369.3p + miR.518c | 0,77 | 0,026716689 |
| miR.558 + miR.609 + miR.603 + miR.494 + miR.518c | 0,77 | 0,020979226 |
| miR.558 + miR.609 + miR.520f + miR.519b + miR.518c | 0,77 | 0,075018623 |
| miR.558 + miR.609 + miR.603 + miR.362 + miR.518c | 0,77 | 0,01311257 |
| miR.603 + miR.220a + miR.369.3p + miR.376a* + miR.518c | 0,77 | 0,090956912 |
| miR.609 + miR.520f + miR.130a + miR.29a + miR.518c | 0,77 | 0,030110896 |
| miR.609 + miR.520f + miR.26a + miR.29a + miR.518c | 0,77 | 0,033978099 |
| miR.558 + miR.609 + miR.220a + miR.518c + miR.639 | 0,77 | 0,010358514 |
| miR.609 + miR.520f + miR.519b + miR.29a + miR.518c | 0,77 | 0,029936391 |
| miR.609 + miR.520f + miR.376a* + miR.518c + miR.639 | 0,77 | 0,0426317 |
| miR.558 + miR.609 + miR.520f + miR.362 + miR.518c | 0,77 | 0,037379284 |
| miR.558 + miR.609 + miR.520f + miR.518c + miR.338 | 0,77 | 0,032694828 |
| miR.558 + miR.609 + miR.603 + miR.518c + miR.660 | 0,77 | 0,022460856 |
| miR.558 + miR.609 + miR.220a + miR.369.3p + miR.518c | 0,77 | 0,015591292 |
| miR.558 + miR.609 + miR.376a* + miR.518c + miR.338 | 0,77 | 0,072802745 |
| miR.609 + miR.603 + miR.520f + miR.518c + miR.639 | 0,77 | 0,058039953 |
| miR.558 + miR.609 + miR.220a + miR.519b + miR.518c | 0,77 | 0,008198503 |
| miR.609 + miR.603 + miR.29a + miR.518c + miR.660 | 0,77 | 0,004944248 |
| miR.558 + miR.609 + miR.520f + miR.494 + miR.518c | 0,77 | 0,046137579 |
| miR.558 + miR.609 + miR.518c + miR.639 + miR.338 | 0,77 | 0,035275149 |
| miR.558 + miR.609 + miR.376a* + miR.518c + miR.660 | 0,77 | 0,05650841 |
| miR.558 + miR.609 + miR.362 + miR.376a* + miR.518c | 0,77 | 0,064266389 |
| miR.609 + miR.603 + miR.26a + miR.29a + miR.518c | 0,77 | 0,0346868 |
| miR.609 + miR.603 + miR.29a + miR.376a* + miR.494 | 0,77 | 0,109005651 |
| miR.609 + miR.220a + miR.29a + miR.518c + miR.338 | 0,77 | 0,00542927 |
| miR.558 + miR.609 + miR.220a + miR.130a + miR.518c | 0,77 | 0,006191584 |
| miR.558 + miR.609 + miR.520f + miR.518c + miR.660 | 0,77 | 0,039493571 |
| miR.558 + miR.609 + miR.518c + miR.639 + miR.660 | 0,77 | 0,075839077 |
| miR.609 + miR.603 + miR.29a + miR.518c + miR.338 | 0,77 | 0,012245334 |
| miR.609 + miR.220a + miR.26a + miR.29a + miR.518c | 0,77 | 0,009385259 |
| miR.558 + miR.609 + miR.362 + miR.518c + miR.639 | 0,77 | 0,015266454 |
| miR.558 + miR.609 + miR.520f + miR.26a + miR.29a | 0,77 | 0,056607697 |
| miR.603 + miR.220a + miR.29a + miR.376a* + miR.518c | 0,77 | 0,068683646 |
| miR.558 + miR.609 + miR.220a + miR.26a + miR.518c | 0,77 | 0,011596267 |
| miR.558 + miR.609 + miR.369.3p + miR.518c + miR.639 | 0,77 | 0,039044417 |
| miR.609 + miR.520f + miR.130a + miR.376a* + miR.338 | 0,77 | 0,227383044 |
| miR.558 + miR.609 + miR.220a + miR.518c + miR.660 | 0,77 | 0,008650483 |
| miR.558 + miR.609 + miR.520f + miR.130a + miR.29a | 0,76 | 0,049657608 |
| miR.558 + miR.609 + miR.130a + miR.26a + miR.518c | 0,76 | 0,032765546 |
| miR.558 + miR.609 + miR.220a + miR.518c + miR.338 | 0,76 | 0,007071801 |
| miR.558 + miR.609 + miR.494 + miR.518c + miR.639 | 0,76 | 0,024604157 |
| miR.558 + miR.609 + miR.520f + miR.519b + miR.29a | 0,76 | 0,088466268 |
| miR.609 + miR.603 + miR.520f + miR.130a + miR.518c | 0,76 | 0,060820074 |
| miR.558 + miR.609 + miR.519b + miR.518c + miR.639 | 0,76 | 0,037860542 |
| miR.603 + miR.220a + miR.130a + miR.376a* + miR.518c | 0,76 | 0,059374762 |
| miR.558 + miR.603 + miR.520f + miR.376a* + miR.518c | 0,76 | 0,05220861 |
| miR.558 + miR.609 + miR.603 + miR.520f + miR.29a | 0,76 | 0,043855643 |
| miR.609 + miR.603 + miR.29a + miR.369.3p + miR.376a* | 0,76 | 0,181642701 |
| miR.558 + miR.609 + miR.220a + miR.362 + miR.518c | 0,76 | 0,007746776 |
| miR.558 + miR.609 + miR.130a + miR.518c + miR.639 | 0,76 | 0,023485054 |
| miR.558 + miR.609 + miR.520f + miR.29a + miR.639 | 0,76 | 0,062774075 |
| miR.558 + miR.609 + miR.220a + miR.494 + miR.518c | 0,76 | 0,007759013 |
| miR.558 + miR.609 + miR.26a + miR.376a* + miR.518c | 0,76 | 0,042891276 |
| miR.558 + miR.609 + miR.519b + miR.376a* + miR.518c | 0,76 | 0,044007041 |
| miR.558 + miR.609 + miR.520f + miR.376a* + miR.639 | 0,76 | 0,155435952 |
| miR.609 + miR.603 + miR.220a + miR.130a + miR.518c | 0,76 | 0,023795849 |
| miR.609 + miR.603 + miR.520f + miR.130a + miR.376a* | 0,76 | 0,144793046 |
| miR.558 + miR.609 + miR.376a* + miR.494 + miR.518c | 0,76 | 0,107352539 |
| miR.558 + miR.609 + miR.520f + miR.29a + miR.494 | 0,76 | 0,063864386 |
| miR.558 + miR.609 + miR.362 + miR.369.3p + miR.518c | 0,76 | 0,014633171 |
| miR.558 + miR.609 + miR.520f + miR.29a + miR.362 | 0,76 | 0,066015305 |
| miR.558 + miR.609 + miR.520f + miR.29a + miR.369.3p | 0,76 | 0,06352373 |
| miR.609 + miR.603 + miR.220a + miR.518c + miR.639 | 0,76 | 0,018261185 |
| miR.603 + miR.220a + miR.376a* + miR.518c + miR.639 | 0,76 | 0,054394079 |
| miR.558 + miR.609 + miR.520f + miR.29a + miR.338 | 0,76 | 0,057639817 |
| miR.558 + miR.609 + miR.520f + miR.220a + miR.29a | 0,76 | 0,068562148 |
| miR.609 + miR.220a + miR.29a + miR.369.3p + miR.376a* | 0,76 | 0,168648268 |
| miR.558 + miR.603 + miR.520f + miR.220a + miR.518c | 0,76 | 0,020501787 |
| miR.609 + miR.520f + miR.220a + miR.130a + miR.518c | 0,76 | 0,017413575 |
| miR.609 + miR.603 + miR.29a + miR.369.3p + miR.518c | 0,76 | 0,011994811 |
| miR.609 + miR.220a + miR.376a* + miR.518c + miR.639 | 0,76 | 0,002219588 |
| miR.558 + miR.603 + miR.376a* + miR.518c + miR.639 | 0,76 | 0,027563503 |
| miR.558 + miR.603 + miR.520f + miR.519b + miR.518c | 0,76 | 0,07252901 |
| miR.609 + miR.603 + miR.220a + miR.369.3p + miR.518c | 0,76 | 0,019700311 |
| miR.609 + miR.603 + miR.29a + miR.494 + miR.518c | 0,76 | 0,022769325 |
| miR.558 + miR.603 + miR.220a + miR.518c + miR.338 | 0,76 | 0,01179661 |
| miR.558 + miR.603 + miR.369.3p + miR.376a* + miR.518c | 0,76 | 0,065638767 |
| miR.558 + miR.603 + miR.220a + miR.26a + miR.518c | 0,76 | 0,033907502 |
| miR.609 + miR.603 + miR.520f + miR.220a + miR.518c | 0,76 | 0,01732095 |
| miR.609 + miR.603 + miR.376a* + miR.494 + miR.518c | 0,76 | 0,058385957 |
| miR.609 + miR.603 + miR.220a + miR.519b + miR.518c | 0,76 | 0,018677282 |
| miR.558 + miR.609 + miR.26a + miR.369.3p + miR.518c | 0,76 | 0,064077303 |
| miR.609 + miR.603 + miR.29a + miR.518c + miR.639 | 0,76 | 0,016265908 |
| miR.609 + miR.603 + miR.220a + miR.494 + miR.518c | 0,76 | 0,023047739 |
| miR.558 + miR.603 + miR.520f + miR.518c + miR.639 | 0,76 | 0,029622658 |
| miR.558 + miR.609 + miR.130a + miR.518c + miR.338 | 0,76 | 0,014299426 |
| miR.609 + miR.603 + miR.519b + miR.29a + miR.518c | 0,76 | 0,016726432 |
| miR.558 + miR.609 + miR.130a + miR.369.3p + miR.518c | 0,76 | 0,014416164 |
| miR.609 + miR.26a + miR.29a + miR.518c + miR.660 | 0,76 | 0,000646569 |
| miR.558 + miR.520f + miR.376a* + miR.518c + miR.639 | 0,76 | 0,089500075 |
| miR.603 + miR.520f + miR.376a* + miR.518c + miR.639 | 0,76 | 0,035843726 |
| miR.609 + miR.603 + miR.130a + miR.29a + miR.518c | 0,76 | 0,013363391 |
| miR.609 + miR.603 + miR.29a + miR.362 + miR.518c | 0,76 | 0,013168181 |
| miR.603 + miR.220a + miR.519b + miR.376a* + miR.518c | 0,76 | 0,076073782 |
| miR.609 + miR.520f + miR.220a + miR.518c + miR.639 | 0,76 | 0,019407372 |
| miR.609 + miR.220a + miR.29a + miR.376a* + miR.494 | 0,76 | 0,114865704 |
| miR.609 + miR.520f + miR.130a + miR.518c + miR.639 | 0,76 | 0,01746451 |
| miR.603 + miR.520f + miR.220a + miR.376a* + miR.518c | 0,76 | 0,071800437 |
| miR.609 + miR.603 + miR.220a + miR.518c + miR.338 | 0,76 | 0,016855614 |
| miR.558 + miR.603 + miR.29a + miR.376a* + miR.518c | 0,76 | 0,013240032 |
| miR.609 + miR.29a + miR.362 + miR.369.3p + miR.376a* | 0,76 | 0,220411833 |
| miR.558 + miR.603 + miR.220a + miR.369.3p + miR.518c | 0,76 | 0,011574552 |
| miR.609 + miR.520f + miR.518c + miR.639 + miR.660 | 0,76 | 0,026277595 |
| miR.558 + miR.609 + miR.519b + miR.369.3p + miR.518c | 0,76 | 0,048179323 |
| miR.558 + miR.609 + miR.130a + miR.362 + miR.518c | 0,76 | 0,009488054 |
| miR.609 + miR.520f + miR.130a + miR.518c + miR.660 | 0,76 | 0,011782281 |
| miR.558 + miR.609 + miR.362 + miR.518c + miR.660 | 0,76 | 0,021648874 |
| miR.609 + miR.603 + miR.130a + miR.26a + miR.518c | 0,76 | 0,011152616 |
| miR.558 + miR.609 + miR.520f + miR.26a + miR.376a* | 0,76 | 0,051931099 |
| miR.558 + miR.603 + miR.376a* + miR.518c + miR.660 | 0,76 | 0,035240698 |
| miR.558 + miR.609 + miR.519b + miR.362 + miR.518c | 0,76 | 0,025224668 |
| miR.609 + miR.220a + miR.29a + miR.369.3p + miR.518c | 0,76 | 0,003275441 |
| miR.558 + miR.609 + miR.369.3p + miR.518c + miR.338 | 0,76 | 0,035823594 |
| miR.609 + miR.520f + miR.26a + miR.518c + miR.639 | 0,76 | 0,032642896 |
| miR.558 + miR.609 + miR.369.3p + miR.494 + miR.518c | 0,76 | 0,024228242 |
| miR.558 + miR.609 + miR.26a + miR.362 + miR.518c | 0,76 | 0,0244115 |
| miR.609 + miR.603 + miR.220a + miR.518c + miR.660 | 0,76 | 0,025612318 |
| miR.609 + miR.603 + miR.220a + miR.26a + miR.518c | 0,76 | 0,022902934 |
| miR.609 + miR.220a + miR.29a + miR.494 + miR.518c | 0,76 | 0,003430325 |
| miR.558 + miR.609 + miR.130a + miR.518c + miR.660 | 0,76 | 0,023670606 |
| miR.609 + miR.29a + miR.369.3p + miR.376a* + miR.494 | 0,76 | 0,120811765 |
| miR.558 + miR.609 + miR.362 + miR.518c + miR.338 | 0,76 | 0,014571932 |
| miR.558 + miR.609 + miR.369.3p + miR.518c + miR.660 | 0,76 | 0,044847549 |
| miR.558 + miR.609 + miR.130a + miR.494 + miR.518c | 0,76 | 0,020151915 |
| miR.558 + miR.609 + miR.519b + miR.130a + miR.518c | 0,76 | 0,026257119 |
| miR.609 + miR.220a + miR.29a + miR.362 + miR.518c | 0,76 | 0,002018625 |
| miR.609 + miR.603 + miR.220a + miR.362 + miR.518c | 0,76 | 0,019735452 |
| miR.609 + miR.29a + miR.362 + miR.518c + miR.660 | 0,76 | 0,001531379 |
| miR.609 + miR.220a + miR.130a + miR.29a + miR.518c | 0,76 | 0,002969535 |
| miR.609 + miR.603 + miR.520f + miR.376a* + miR.518c | 0,76 | 0,021791801 |
| miR.558 + miR.603 + miR.220a + miR.518c + miR.660 | 0,76 | 0,013152501 |
| miR.558 + miR.603 + miR.220a + miR.29a + miR.518c | 0,76 | 0,007763354 |
| miR.558 + miR.609 + miR.362 + miR.494 + miR.518c | 0,76 | 0,014731694 |
| miR.609 + miR.520f + miR.130a + miR.26a + miR.376a* | 0,76 | 0,16200117 |
| miR.558 + miR.603 + miR.220a + miR.518c + miR.639 | 0,76 | 0,007508418 |
| miR.558 + miR.603 + miR.26a + miR.518c + miR.639 | 0,76 | 0,052154719 |
| miR.609 + miR.220a + miR.29a + miR.518c + miR.639 | 0,76 | 0,003091116 |
| miR.558 + miR.603 + miR.220a + miR.494 + miR.518c | 0,76 | 0,010178039 |
| miR.609 + miR.520f + miR.519b + miR.518c + miR.639 | 0,76 | 0,026238301 |
| miR.609 + miR.519b + miR.29a + miR.369.3p + miR.376a* | 0,76 | 0,107303707 |
| miR.609 + miR.220a + miR.519b + miR.29a + miR.518c | 0,76 | 0,003631923 |
| miR.609 + miR.220a + miR.369.3p + miR.376a* + miR.518c | 0,76 | 0,007208339 |
| miR.558 + miR.520f + miR.29a + miR.376a* + miR.518c | 0,76 | 0,049689549 |
| miR.558 + miR.603 + miR.520f + miR.29a + miR.518c | 0,76 | 0,021737337 |
| miR.609 + miR.26a + miR.29a + miR.494 + miR.518c | 0,76 | 0,002102546 |
| miR.603 + miR.220a + miR.376a* + miR.494 + miR.518c | 0,76 | 0,036759049 |
| miR.609 + miR.603 + miR.220a + miR.29a + miR.376a* | 0,76 | 0,053938304 |
| miR.558 + miR.603 + miR.519b + miR.376a* + miR.518c | 0,76 | 0,041720652 |
| miR.609 + miR.520f + miR.494 + miR.518c + miR.639 | 0,76 | 0,022405267 |
| miR.558 + miR.603 + miR.220a + miR.130a + miR.518c | 0,76 | 0,01231129 |
| miR.558 + miR.603 + miR.220a + miR.362 + miR.518c | 0,76 | 0,00903321 |
| miR.609 + miR.519b + miR.26a + miR.29a + miR.518c | 0,76 | 0,001598197 |
| miR.609 + miR.603 + miR.26a + miR.518c + miR.639 | 0,76 | 0,015889918 |
| miR.609 + miR.29a + miR.518c + miR.660 + miR.338 | 0,76 | 0,000161201 |
| miR.609 + miR.520f + miR.369.3p + miR.518c + miR.639 | 0,76 | 0,023386701 |
| miR.558 + miR.603 + miR.362 + miR.376a* + miR.518c | 0,76 | 0,027638813 |
| miR.609 + miR.603 + miR.369.3p + miR.518c + miR.639 | 0,76 | 0,022896138 |
| miR.609 + miR.520f + miR.130a + miR.376a* + miR.639 | 0,76 | 0,081231616 |
| miR.609 + miR.519b + miR.376a* + miR.518c + miR.639 | 0,76 | 0,008433744 |
| miR.558 + miR.609 + miR.519b + miR.518c + miR.338 | 0,76 | 0,041751735 |
| miR.558 + miR.603 + miR.26a + miR.29a + miR.518c | 0,76 | 0,014455142 |
| miR.609 + miR.603 + miR.518c + miR.639 + miR.338 | 0,76 | 0,005822029 |
| miR.609 + miR.603 + miR.130a + miR.518c + miR.639 | 0,76 | 0,010164618 |
| miR.609 + miR.603 + miR.376a* + miR.518c + miR.660 | 0,76 | 0,010253425 |
| miR.558 + miR.609 + miR.519b + miR.518c + miR.660 | 0,76 | 0,049471421 |
| miR.558 + miR.609 + miR.130a + miR.369.3p + miR.376a* | 0,76 | 0,149697262 |
| miR.558 + miR.609 + miR.26a + miR.494 + miR.518c | 0,76 | 0,046963722 |
| miR.603 + miR.220a + miR.376a* + miR.518c + miR.660 | 0,76 | 0,048900387 |
| miR.558 + miR.603 + miR.520f + miR.494 + miR.518c | 0,76 | 0,024856532 |
| miR.609 + miR.520f + miR.26a + miR.376a* + miR.518c | 0,76 | 0,021053337 |
| miR.609 + miR.520f + miR.518c + miR.639 + miR.338 | 0,76 | 0,019478405 |
| miR.609 + miR.519b + miR.29a + miR.518c + miR.660 | 0,76 | 0,000401115 |
| miR.609 + miR.29a + miR.494 + miR.518c + miR.660 | 0,76 | 0,002457844 |
| miR.609 + miR.520f + miR.130a + miR.369.3p + miR.376a* | 0,76 | 0,104284792 |
| miR.558 + miR.609 + miR.519b + miR.26a + miR.518c | 0,76 | 0,049598796 |
| miR.609 + miR.520f + miR.362 + miR.518c + miR.639 | 0,76 | 0,021826248 |
| miR.609 + miR.603 + miR.520f + miR.369.3p + miR.518c | 0,76 | 0,021265273 |
| miR.558 + miR.603 + miR.520f + miR.369.3p + miR.518c | 0,76 | 0,033445228 |
| miR.558 + miR.603 + miR.130a + miR.376a* + miR.518c | 0,76 | 0,028261204 |
| miR.609 + miR.603 + miR.376a* + miR.518c + miR.338 | 0,76 | 0,010570983 |
| miR.609 + miR.603 + miR.362 + miR.376a* + miR.518c | 0,76 | 0,008819616 |
| miR.609 + miR.26a + miR.29a + miR.369.3p + miR.518c | 0,76 | 0,0021862 |
| miR.558 + miR.603 + miR.520f + miR.130a + miR.518c | 0,76 | 0,027187735 |
| miR.609 + miR.520f + miR.130a + miR.369.3p + miR.518c | 0,76 | 0,009091034 |
| miR.609 + miR.29a + miR.362 + miR.376a* + miR.494 | 0,76 | 0,07611372 |
| miR.603 + miR.220a + miR.362 + miR.376a* + miR.518c | 0,76 | 0,048416338 |
| miR.558 + miR.603 + miR.520f + miR.362 + miR.518c | 0,76 | 0,032644745 |
| miR.603 + miR.220a + miR.376a* + miR.518c + miR.338 | 0,76 | 0,038486109 |
| miR.603 + miR.220a + miR.26a + miR.376a* + miR.518c | 0,76 | 0,046320209 |
| miR.558 + miR.520f + miR.220a + miR.376a* + miR.518c | 0,76 | 0,033038578 |
| miR.609 + miR.520f + miR.519b + miR.130a + miR.518c | 0,76 | 0,010550971 |
| miR.558 + miR.603 + miR.520f + miR.518c + miR.660 | 0,76 | 0,034114893 |
| miR.603 + miR.520f + miR.29a + miR.376a* + miR.518c | 0,76 | 0,023006145 |
| miR.558 + miR.603 + miR.520f + miR.518c + miR.338 | 0,76 | 0,036799544 |
| miR.558 + miR.609 + miR.519b + miR.494 + miR.518c | 0,76 | 0,06410699 |
| miR.609 + miR.220a + miR.130a + miR.26a + miR.518c | 0,76 | 0,002119052 |
| miR.558 + miR.609 + miR.26a + miR.518c + miR.338 | 0,76 | 0,042287284 |
| miR.609 + miR.519b + miR.130a + miR.376a* + miR.518c | 0,76 | 0,007989029 |
| miR.558 + miR.609 + miR.26a + miR.518c + miR.660 | 0,76 | 0,040582972 |
| miR.609 + miR.520f + miR.130a + miR.518c + miR.338 | 0,76 | 0,006938008 |
| miR.609 + miR.603 + miR.494 + miR.518c + miR.639 | 0,76 | 0,017767437 |
| miR.609 + miR.520f + miR.130a + miR.362 + miR.518c | 0,76 | 0,008980282 |
| miR.558 + miR.603 + miR.26a + miR.376a* + miR.518c | 0,76 | 0,042647738 |
| miR.609 + miR.520f + miR.130a + miR.26a + miR.518c | 0,76 | 0,010152129 |
| miR.609 + miR.520f + miR.130a + miR.494 + miR.518c | 0,76 | 0,010953139 |
| miR.609 + miR.603 + miR.130a + miR.518c + miR.338 | 0,76 | 0,006348197 |
| miR.558 + miR.603 + miR.520f + miR.26a + miR.518c | 0,76 | 0,035806694 |
| miR.609 + miR.603 + miR.518c + miR.639 + miR.660 | 0,76 | 0,010630384 |
| miR.558 + miR.609 + miR.369.3p + miR.376a* + miR.639 | 0,76 | 0,229021768 |
| miR.558 + miR.609 + miR.603 + miR.130a + miR.376a* | 0,76 | 0,03971776 |
| miR.609 + miR.603 + miR.26a + miR.376a* + miR.518c | 0,76 | 0,006742826 |
| miR.609 + miR.603 + miR.130a + miR.369.3p + miR.518c | 0,76 | 0,010159921 |
| miR.609 + miR.603 + miR.362 + miR.518c + miR.639 | 0,76 | 0,011672276 |
| miR.558 + miR.609 + miR.518c + miR.660 + miR.338 | 0,76 | 0,031801258 |
| miR.609 + miR.26a + miR.29a + miR.518c + miR.338 | 0,76 | 0,003025169 |
| miR.558 + miR.609 + miR.494 + miR.518c + miR.338 | 0,76 | 0,032582396 |
| miR.603 + miR.362 + miR.369.3p + miR.376a* + miR.518c | 0,76 | 0,067169531 |
| miR.609 + miR.519b + miR.29a + miR.376a* + miR.494 | 0,76 | 0,057015103 |
| miR.609 + miR.603 + miR.520f + miR.26a + miR.518c | 0,76 | 0,016606257 |
| miR.558 + miR.609 + miR.26a + miR.29a + miR.369.3p | 0,76 | 0,016964813 |
| miR.609 + miR.603 + miR.520f + miR.494 + miR.518c | 0,76 | 0,03216242 |
| miR.609 + miR.603 + miR.29a + miR.376a* + miR.639 | 0,76 | 0,030163877 |
| miR.558 + miR.603 + miR.376a* + miR.494 + miR.518c | 0,76 | 0,023924104 |
| miR.558 + miR.603 + miR.376a* + miR.518c + miR.338 | 0,76 | 0,026608271 |
| miR.609 + miR.603 + miR.519b + miR.518c + miR.639 | 0,76 | 0,01119512 |
| miR.558 + miR.609 + miR.29a + miR.362 + miR.660 | 0,76 | 0,009330873 |
| miR.558 + miR.609 + miR.494 + miR.518c + miR.660 | 0,76 | 0,047432705 |
| miR.558 + miR.520f + miR.220a + miR.519b + miR.518c | 0,76 | 0,039277549 |
| miR.609 + miR.29a + miR.369.3p + miR.518c + miR.660 | 0,76 | 0,00098718 |
| miR.609 + miR.520f + miR.519b + miR.130a + miR.376a* | 0,76 | 0,075797653 |
| miR.609 + miR.29a + miR.518c + miR.639 + miR.660 | 0,76 | 0,00073455 |
| miR.558 + miR.609 + miR.603 + miR.520f + miR.639 | 0,76 | 0,042887922 |
| miR.558 + miR.609 + miR.603 + miR.376a* + miR.639 | 0,76 | 0,058038116 |
| miR.558 + miR.609 + miR.26a + miR.29a + miR.660 | 0,76 | 0,011052131 |
| miR.609 + miR.26a + miR.29a + miR.518c + miR.639 | 0,76 | 0,001547257 |
| miR.558 + miR.609 + miR.220a + miR.26a + miR.29a | 0,76 | 0,013185857 |
| miR.609 + miR.130a + miR.29a + miR.376a* + miR.494 | 0,76 | 0,047466604 |
| miR.603 + miR.369.3p + miR.376a* + miR.518c + miR.639 | 0,76 | 0,020526146 |
| miR.609 + miR.603 + miR.519b + miR.376a* + miR.518c | 0,76 | 0,007223758 |
| miR.609 + miR.520f + miR.29a + miR.494 + miR.660 | 0,76 | 0,073589539 |
| miR.609 + miR.29a + miR.494 + miR.518c + miR.338 | 0,76 | 0,003462953 |
| miR.603 + miR.220a + miR.29a + miR.518c + miR.338 | 0,76 | 0,023023695 |
| miR.609 + miR.520f + miR.26a + miR.376a* + miR.639 | 0,76 | 0,117375859 |
| miR.603 + miR.520f + miR.220a + miR.518c + miR.639 | 0,76 | 0,025887464 |
| miR.603 + miR.520f + miR.220a + miR.519b + miR.518c | 0,76 | 0,025767911 |
| miR.558 + miR.603 + miR.519b + miR.362 + miR.518c | 0,76 | 0,024961269 |
| miR.609 + miR.130a + miR.29a + miR.518c + miR.660 | 0,76 | 0,000273592 |
| miR.609 + miR.603 + miR.130a + miR.518c + miR.660 | 0,76 | 0,011725317 |
| miR.609 + miR.603 + miR.130a + miR.362 + miR.518c | 0,76 | 0,010515123 |
| miR.609 + miR.29a + miR.369.3p + miR.518c + miR.338 | 0,76 | 0,003907214 |
| miR.558 + miR.603 + miR.26a + miR.369.3p + miR.518c | 0,76 | 0,050279892 |
| miR.603 + miR.220a + miR.519b + miR.518c + miR.338 | 0,76 | 0,01514577 |
| miR.609 + miR.603 + miR.520f + miR.519b + miR.518c | 0,76 | 0,014157426 |
| miR.558 + miR.609 + miR.520f + miR.130a + miR.639 | 0,76 | 0,045951688 |
| miR.558 + miR.609 + miR.603 + miR.520f + miR.130a | 0,76 | 0,036822881 |
| miR.609 + miR.519b + miR.29a + miR.518c + miR.338 | 0,76 | 0,002082521 |
| miR.609 + miR.26a + miR.29a + miR.362 + miR.518c | 0,76 | 0,000765601 |
| miR.558 + miR.609 + miR.362 + miR.369.3p + miR.376a* | 0,76 | 0,108386454 |
| miR.603 + miR.220a + miR.519b + miR.29a + miR.518c | 0,76 | 0,012790104 |
| miR.609 + miR.603 + miR.362 + miR.369.3p + miR.518c | 0,76 | 0,015538273 |
| miR.609 + miR.603 + miR.130a + miR.494 + miR.518c | 0,76 | 0,011597837 |
| miR.558 + miR.603 + miR.519b + miR.518c + miR.639 | 0,76 | 0,023795778 |
| miR.603 + miR.220a + miR.26a + miR.29a + miR.518c | 0,76 | 0,018423813 |
| miR.558 + miR.603 + miR.519b + miR.26a + miR.518c | 0,76 | 0,040091082 |
| miR.609 + miR.520f + miR.130a + miR.362 + miR.376a* | 0,76 | 0,073024904 |
| miR.609 + miR.603 + miR.519b + miR.130a + miR.518c | 0,76 | 0,008962594 |
| miR.609 + miR.220a + miR.130a + miR.518c + miR.338 | 0,76 | 0,002205947 |
| miR.609 + miR.130a + miR.26a + miR.29a + miR.518c | 0,76 | 0,000957283 |
| miR.609 + miR.520f + miR.220a + miR.130a + miR.376a* | 0,76 | 0,063686016 |
| miR.603 + miR.520f + miR.220a + miR.29a + miR.518c | 0,76 | 0,020043897 |
| miR.609 + miR.603 + miR.520f + miR.362 + miR.518c | 0,76 | 0,014416878 |
| miR.558 + miR.609 + miR.130a + miR.362 + miR.376a* | 0,76 | 0,024321151 |
| miR.558 + miR.609 + miR.26a + miR.29a + miR.639 | 0,76 | 0,009247032 |
| miR.558 + miR.609 + miR.603 + miR.26a + miR.29a | 0,76 | 0,009942144 |
| miR.609 + miR.130a + miR.376a* + miR.518c + miR.639 | 0,76 | 0,009279881 |
| miR.603 + miR.220a + miR.519b + miR.369.3p + miR.518c | 0,76 | 0,019144995 |
| miR.558 + miR.520f + miR.494 + miR.518c + miR.639 | 0,76 | 0,028787601 |
| miR.558 + miR.609 + miR.26a + miR.29a + miR.494 | 0,76 | 0,00790317 |
| miR.609 + miR.603 + miR.520f + miR.518c + miR.338 | 0,76 | 0,015771629 |
| miR.609 + miR.130a + miR.29a + miR.494 + miR.518c | 0,76 | 0,001342942 |
| miR.558 + miR.609 + miR.520f + miR.376a* + miR.338 | 0,76 | 0,109265275 |
| miR.558 + miR.603 + miR.519b + miR.518c + miR.660 | 0,76 | 0,02783213 |
| miR.603 + miR.220a + miR.369.3p + miR.518c + miR.338 | 0,76 | 0,011651358 |
| miR.558 + miR.520f + miR.130a + miR.376a* + miR.518c | 0,76 | 0,035536194 |
| miR.558 + miR.520f + miR.220a + miR.518c + miR.639 | 0,76 | 0,018069663 |
| miR.558 + miR.520f + miR.519b + miR.518c + miR.639 | 0,76 | 0,06482366 |
| miR.558 + miR.603 + miR.130a + miR.26a + miR.518c | 0,76 | 0,016575711 |
| miR.603 + miR.220a + miR.519b + miR.26a + miR.518c | 0,76 | 0,015828338 |
| miR.609 + miR.29a + miR.376a* + miR.494 + miR.639 | 0,76 | 0,037368818 |
| miR.558 + miR.609 + miR.520f + miR.26a + miR.639 | 0,76 | 0,032760971 |
| miR.558 + miR.603 + miR.519b + miR.369.3p + miR.518c | 0,76 | 0,042299566 |
| miR.603 + miR.520f + miR.130a + miR.376a* + miR.518c | 0,76 | 0,021525819 |
| miR.609 + miR.26a + miR.29a + miR.376a* + miR.494 | 0,76 | 0,043344291 |
| miR.609 + miR.520f + miR.130a + miR.376a* + miR.494 | 0,76 | 0,057857469 |
| miR.558 + miR.603 + miR.518c + miR.639 + miR.338 | 0,75 | 0,012562831 |
| miR.609 + miR.603 + miR.130a + miR.29a + miR.376a* | 0,75 | 0,038169185 |
| miR.558 + miR.520f + miR.518c + miR.639 + miR.660 | 0,75 | 0,032273928 |
| miR.558 + miR.603 + miR.519b + miR.29a + miR.518c | 0,75 | 0,015180046 |
| miR.558 + miR.609 + miR.220a + miR.130a + miR.376a* | 0,75 | 0,02221586 |
| miR.558 + miR.609 + miR.26a + miR.29a + miR.338 | 0,75 | 0,008220027 |
| miR.609 + miR.603 + miR.520f + miR.518c + miR.660 | 0,75 | 0,010092484 |
| miR.609 + miR.369.3p + miR.376a* + miR.518c + miR.639 | 0,75 | 0,029475112 |
| miR.558 + miR.609 + miR.26a + miR.29a + miR.362 | 0,75 | 0,007678726 |
| miR.558 + miR.609 + miR.520f + miR.639 + miR.660 | 0,75 | 0,089040477 |
| miR.603 + miR.520f + miR.220a + miR.369.3p + miR.518c | 0,75 | 0,022342134 |
| miR.609 + miR.603 + miR.369.3p + miR.494 + miR.518c | 0,75 | 0,016807895 |
| miR.609 + miR.603 + miR.26a + miR.369.3p + miR.518c | 0,75 | 0,009087212 |
| miR.603 + miR.220a + miR.518c + miR.639 + miR.338 | 0,75 | 0,0099053 |
| miR.609 + miR.29a + miR.518c + miR.639 + miR.338 | 0,75 | 0,001599317 |
| miR.558 + miR.603 + miR.362 + miR.369.3p + miR.518c | 0,75 | 0,010086123 |
| miR.558 + miR.609 + miR.519b + miR.26a + miR.29a | 0,75 | 0,008859121 |
| miR.603 + miR.220a + miR.26a + miR.369.3p + miR.518c | 0,75 | 0,018703057 |
| miR.558 + miR.520f + miR.220a + miR.29a + miR.518c | 0,75 | 0,017183184 |
| miR.609 + miR.220a + miR.376a* + miR.494 + miR.518c | 0,75 | 0,00489172 |
| miR.558 + miR.609 + miR.130a + miR.26a + miR.29a | 0,75 | 0,004334831 |
| miR.558 + miR.520f + miR.26a + miR.376a* + miR.518c | 0,75 | 0,028539114 |
| miR.558 + miR.609 + miR.520f + miR.639 + miR.338 | 0,75 | 0,050919535 |
| miR.609 + miR.29a + miR.362 + miR.518c + miR.338 | 0,75 | 0,001525708 |
| miR.609 + miR.29a + miR.369.3p + miR.376a* + miR.639 | 0,75 | 0,07072185 |
| miR.558 + miR.603 + miR.519b + miR.518c + miR.338 | 0,75 | 0,02210537 |
| miR.558 + miR.603 + miR.519b + miR.130a + miR.518c | 0,75 | 0,024636374 |
| miR.609 + miR.130a + miR.29a + miR.518c + miR.338 | 0,75 | 0,001253716 |
| miR.609 + miR.603 + miR.26a + miR.29a + miR.376a* | 0,75 | 0,019969573 |
| miR.609 + miR.220a + miR.519b + miR.29a + miR.376a* | 0,75 | 0,018820628 |
| miR.609 + miR.520f + miR.130a + miR.376a* + miR.660 | 0,75 | 0,04349445 |
| miR.558 + miR.609 + miR.130a + miR.376a* + miR.639 | 0,75 | 0,051369927 |
| miR.609 + miR.220a + miR.29a + miR.362 + miR.376a* | 0,75 | 0,02102776 |
| miR.603 + miR.520f + miR.369.3p + miR.376a* + miR.518c | 0,75 | 0,031199248 |
| miR.603 + miR.220a + miR.130a + miR.26a + miR.518c | 0,75 | 0,018175075 |
| miR.558 + miR.220a + miR.29a + miR.376a* + miR.518c | 0,75 | 0,011886225 |
| miR.603 + miR.220a + miR.130a + miR.518c + miR.338 | 0,75 | 0,011863166 |
| miR.609 + miR.220a + miR.26a + miR.29a + miR.376a* | 0,75 | 0,010887199 |
| miR.603 + miR.520f + miR.220a + miR.130a + miR.518c | 0,75 | 0,018897988 |
| miR.603 + miR.220a + miR.519b + miR.494 + miR.518c | 0,75 | 0,01581099 |
| miR.603 + miR.520f + miR.220a + miR.518c + miR.338 | 0,75 | 0,021372759 |
| miR.558 + miR.603 + miR.130a + miR.518c + miR.639 | 0,75 | 0,014618027 |
| miR.609 + miR.603 + miR.29a + miR.362 + miR.376a* | 0,75 | 0,050058787 |
| miR.609 + miR.603 + miR.369.3p + miR.518c + miR.338 | 0,75 | 0,010014277 |
| miR.558 + miR.609 + miR.520f + miR.369.3p + miR.639 | 0,75 | 0,038186301 |
| miR.558 + miR.609 + miR.362 + miR.376a* + miR.639 | 0,75 | 0,047674346 |
| miR.609 + miR.29a + miR.376a* + miR.494 + miR.338 | 0,75 | 0,053116412 |
| miR.558 + miR.520f + miR.130a + miR.518c + miR.639 | 0,75 | 0,022328503 |
| miR.558 + miR.520f + miR.519b + miR.29a + miR.518c | 0,75 | 0,052640607 |
| miR.558 + miR.603 + miR.26a + miR.362 + miR.518c | 0,75 | 0,021593994 |
| miR.609 + miR.220a + miR.29a + miR.376a* + miR.338 | 0,75 | 0,019694695 |
| miR.558 + miR.603 + miR.369.3p + miR.518c + miR.639 | 0,75 | 0,01007484 |
| miR.603 + miR.220a + miR.26a + miR.518c + miR.639 | 0,75 | 0,014638688 |
| miR.558 + miR.520f + miR.26a + miR.518c + miR.639 | 0,75 | 0,029269796 |
| miR.609 + miR.603 + miR.519b + miR.29a + miR.376a* | 0,75 | 0,04581019 |
| miR.558 + miR.520f + miR.362 + miR.518c + miR.639 | 0,75 | 0,017951532 |
| miR.609 + miR.220a + miR.29a + miR.376a* + miR.639 | 0,75 | 0,01913617 |
| miR.558 + miR.220a + miR.369.3p + miR.376a* + miR.518c | 0,75 | 0,049161847 |
| miR.558 + miR.603 + miR.130a + miR.29a + miR.518c | 0,75 | 0,007216532 |
| miR.603 + miR.220a + miR.519b + miR.130a + miR.518c | 0,75 | 0,01262932 |
| miR.558 + miR.609 + miR.520f + miR.130a + miR.26a | 0,75 | 0,043520581 |
| miR.558 + miR.603 + miR.362 + miR.518c + miR.639 | 0,75 | 0,006232439 |
| miR.558 + miR.603 + miR.519b + miR.494 + miR.518c | 0,75 | 0,022492489 |
| miR.609 + miR.520f + miR.220a + miR.376a* + miR.518c | 0,75 | 0,00384492 |
| miR.609 + miR.220a + miR.130a + miR.29a + miR.376a* | 0,75 | 0,028916521 |
| miR.558 + miR.520f + miR.518c + miR.639 + miR.338 | 0,75 | 0,01885151 |
| miR.609 + miR.130a + miR.362 + miR.376a* + miR.518c | 0,75 | 0,003891989 |
| miR.558 + miR.609 + miR.520f + miR.494 + miR.639 | 0,75 | 0,048260686 |
| miR.603 + miR.220a + miR.519b + miR.518c + miR.639 | 0,75 | 0,014390889 |
| miR.558 + miR.609 + miR.520f + miR.362 + miR.639 | 0,75 | 0,035567697 |
| miR.558 + miR.609 + miR.520f + miR.130a + miR.660 | 0,75 | 0,06507655 |
| miR.609 + miR.520f + miR.220a + miR.369.3p + miR.518c | 0,75 | 0,005772128 |
| miR.609 + miR.603 + miR.29a + miR.376a* + miR.338 | 0,75 | 0,03681333 |
| miR.558 + miR.520f + miR.376a* + miR.518c + miR.660 | 0,75 | 0,046044877 |
| miR.609 + miR.603 + miR.519b + miR.369.3p + miR.518c | 0,75 | 0,009001864 |
| miR.609 + miR.29a + miR.369.3p + miR.376a* + miR.338 | 0,75 | 0,087747794 |
| miR.609 + miR.220a + miR.29a + miR.376a* + miR.660 | 0,75 | 0,019498297 |
| miR.609 + miR.29a + miR.376a* + miR.494 + miR.660 | 0,75 | 0,036531086 |
| miR.558 + miR.520f + miR.376a* + miR.518c + miR.338 | 0,75 | 0,098259776 |
| miR.609 + miR.130a + miR.29a + miR.369.3p + miR.518c | 0,75 | 0,001741294 |
| miR.558 + miR.520f + miR.369.3p + miR.518c + miR.639 | 0,75 | 0,019447124 |
| miR.609 + miR.520f + miR.26a + miR.376a* + miR.494 | 0,75 | 0,076045797 |
| miR.603 + miR.220a + miR.519b + miR.518c + miR.660 | 0,75 | 0,017343411 |
| miR.609 + miR.220a + miR.130a + miR.518c + miR.660 | 0,75 | 0,001581982 |
| miR.558 + miR.603 + miR.26a + miR.518c + miR.660 | 0,75 | 0,027131535 |
| miR.558 + miR.520f + miR.29a + miR.518c + miR.639 | 0,75 | 0,019640508 |
| miR.609 + miR.130a + miR.29a + miR.518c + miR.639 | 0,75 | 0,001080999 |
| miR.558 + miR.609 + miR.520f + miR.130a + miR.369.3p | 0,75 | 0,053459123 |
| miR.558 + miR.609 + miR.520f + miR.519b + miR.639 | 0,75 | 0,053286342 |
| miR.603 + miR.520f + miR.220a + miR.518c + miR.660 | 0,75 | 0,022335483 |
| miR.558 + miR.609 + miR.520f + miR.519b + miR.130a | 0,75 | 0,064845424 |
| miR.558 + miR.520f + miR.362 + miR.376a* + miR.518c | 0,75 | 0,045030008 |
| miR.558 + miR.609 + miR.520f + miR.220a + miR.639 | 0,75 | 0,041778052 |
| miR.609 + miR.29a + miR.362 + miR.369.3p + miR.518c | 0,75 | 0,002486241 |
| miR.609 + miR.603 + miR.29a + miR.376a* + miR.660 | 0,75 | 0,044235947 |
| miR.603 + miR.220a + miR.369.3p + miR.518c + miR.639 | 0,75 | 0,011980221 |
| miR.558 + miR.609 + miR.520f + miR.130a + miR.362 | 0,75 | 0,027438706 |
| miR.603 + miR.220a + miR.29a + miR.369.3p + miR.518c | 0,75 | 0,010519744 |
| miR.609 + miR.519b + miR.29a + miR.369.3p + miR.518c | 0,75 | 0,00214254 |
| miR.558 + miR.520f + miR.220a + miR.494 + miR.518c | 0,75 | 0,012772203 |
| miR.609 + miR.519b + miR.29a + miR.376a* + miR.639 | 0,75 | 0,015007279 |
| miR.609 + miR.603 + miR.369.3p + miR.518c + miR.660 | 0,75 | 0,00793125 |
| miR.558 + miR.603 + miR.494 + miR.518c + miR.639 | 0,75 | 0,005636393 |
| miR.603 + miR.520f + miR.26a + miR.376a* + miR.518c | 0,75 | 0,024421876 |
| miR.609 + miR.520f + miR.220a + miR.26a + miR.518c | 0,75 | 0,010530365 |
| miR.558 + miR.609 + miR.520f + miR.130a + miR.338 | 0,75 | 0,046884652 |
| miR.558 + miR.520f + miR.29a + miR.494 + miR.518c | 0,75 | 0,017713046 |
| miR.603 + miR.369.3p + miR.376a* + miR.518c + miR.660 | 0,75 | 0,030292073 |
| miR.603 + miR.520f + miR.220a + miR.494 + miR.518c | 0,75 | 0,020658815 |
| miR.558 + miR.520f + miR.369.3p + miR.376a* + miR.518c | 0,75 | 0,088750924 |
| miR.609 + miR.220a + miR.130a + miR.369.3p + miR.518c | 0,75 | 0,002238117 |
| miR.558 + miR.603 + miR.369.3p + miR.494 + miR.518c | 0,75 | 0,01247287 |
| miR.609 + miR.603 + miR.362 + miR.494 + miR.518c | 0,75 | 0,014175895 |
| miR.603 + miR.220a + miR.519b + miR.362 + miR.518c | 0,75 | 0,013147374 |
| miR.609 + miR.29a + miR.494 + miR.518c + miR.639 | 0,75 | 0,001660365 |
| miR.603 + miR.520f + miR.220a + miR.26a + miR.518c | 0,75 | 0,019222776 |
| miR.520f + miR.220a + miR.29a + miR.376a* + miR.518c | 0,75 | 0,019991243 |
| miR.558 + miR.603 + miR.26a + miR.518c + miR.338 | 0,75 | 0,023494384 |
| miR.558 + miR.603 + miR.29a + miR.518c + miR.338 | 0,75 | 0,006133672 |

**Table 6 clusters of 6miRNAs**

| **Cluster of 6 miRs** | **c_tau level** | **pv compared to top signature miR.558 + miR.609 + miR.220a + miR.29a + miR.376a* + miR.518c** |
|---|---|---|
| miR.558 + miR.609 + miR.220a + miR.29a + miR.376a* + miR.518c | 0,8 | NaN |
| miR.558 + miR.609 + miR.520f + miR.29a + miR.376a* + miR.518c | 0,8 | 0,425262874 |
| miR.558 + miR.609 + miR.603 + miR.29a + miR.376a* + miR.518c | 0,8 | 0,000198569 |
| miR.558 + miR.609 + miR.29a + miR.369.3p + miR.376a* + miR.518c | 0,8 | 0,192669487 |
| miR.558 + miR.609 + miR.26a + miR.29a + miR.376a* + miR.518c | 0,8 | 0,357087852 |
| miR.558 + miR.609 + miR.29a + miR.376a* + miR.518c + miR.639 | 0,79 | 0,185758947 |
| miR.609 + miR.603 + miR.220a + miR.29a + miR.376a* + miR.518c | 0,79 | 0,462031556 |
| miR.558 + miR.609 + miR.29a + miR.376a* + miR.518c + miR.660 | 0,79 | 0,100722966 |
| miR.558 + miR.609 + miR.29a + miR.376a* + miR.518c + miR.338 | 0,79 | 0,176804634 |
| miR.558 + miR.609 + miR.130a + miR.29a + miR.376a* + miR.518c | 0,79 | 0,132965668 |
| miR.558 + miR.609 + miR.29a + miR.362 + miR.376a* + miR.518c | 0,79 | 0,112793246 |
| miR.558 + miR.609 + miR.519b + miR.29a + miR.376a* + miR.518c | 0,79 | 0,131995208 |
| miR.558 + miR.609 + miR.29a + miR.376a* + miR.494 + miR.518c | 0,79 | 0,292038462 |
| miR.609 + miR.603 + miR.29a + miR.369.3p + miR.376a* + miR.518c | 0,79 | 0,495842065 |
| miR.609 + miR.603 + miR.520f + miR.29a + miR.376a* + miR.518c | 0,79 | 0,164096863 |
| miR.609 + miR.220a + miR.29a + miR.369.3p + miR.376a* + miR.518c | 0,79 | 0,109937592 |
| miR.609 + miR.520f + miR.220a + miR.29a + miR.376a* + miR.518c | 0,79 | 0,465866529 |
| miR.558 + miR.609 + miR.520f + miR.29a + miR.376a* + miR.338 | 0,79 | 0,24024849 |
| miR.609 + miR.520f + miR.29a + miR.376a* + miR.494 + miR.518c | 0,79 | 0,362228462 |
| miR.558 + miR.609 + miR.520f + miR.29a + miR.518c + miR.660 | 0,79 | 0,061409925 |
| miR.558 + miR.609 + miR.520f + miR.29a + miR.376a* + miR.494 | 0,79 | 0,098769582 |
| miR.609 + miR.603 + miR.29a + miR.376a* + miR.494 + miR.518c | 0,79 | 0,062376144 |
| miR.558 + miR.609 + miR.520f + miR.29a + miR.369.3p + miR.376a* | 0,79 | 0,260005036 |
| miR.609 + miR.520f + miR.29a + miR.369.3p + miR.376a* + miR.518c | 0,79 | 0,496052539 |
| miR.558 + miR.609 + miR.520f + miR.376a* + miR.518c + miR.639 | 0,79 | 0,267576326 |
| miR.609 + miR.603 + miR.29a + miR.376a* + miR.518c + miR.639 | 0,79 | 0,011245226 |
| miR.558 + miR.609 + miR.26a + miR.29a + miR.518c + miR.660 | 0,79 | 0,04047064 |
| miR.558 + miR.609 + miR.603 + miR.376a* + miR.518c + miR.639 | 0,79 | 0,103974742 |
| miR.609 + miR.520f + miR.29a + miR.376a* + miR.518c + miR.660 | 0,79 | 0,266209089 |
| miR.609 + miR.220a + miR.519b + miR.29a + miR.376a* + miR.518c | 0,79 | 0,454399121 |
| miR.609 + miR.220a + miR.29a + miR.376a* + miR.494 + miR.518c | 0,79 | 0,422773766 |
| miR.609 + miR.520f + miR.29a + miR.376a* + miR.518c + miR.338 | 0,79 | 0,488487745 |
| miR.609 + miR.520f + miR.519b + miR.29a + miR.376a* + miR.518c | 0,79 | 0,221080827 |
| miR.558 + miR.609 + miR.603 + miR.520f + miR.29a + miR.376a* | 0,79 | 0,086973376 |
| miR.558 + miR.609 + miR.520f + miR.29a + miR.376a* + miR.660 | 0,79 | 0,15681019 |
| miR.609 + miR.603 + miR.26a + miR.29a + miR.376a* + miR.518c | 0,79 | 0,007221034 |
| miR.558 + miR.609 + miR.520f + miR.130a + miR.376a* + miR.518c | 0,79 | 0,111580627 |
| miR.609 + miR.220a + miR.29a + miR.362 + miR.376a* + miR.518c | 0,79 | 0,465457785 |
| miR.609 + miR.603 + miR.29a + miR.362 + miR.376a* + miR.518c | 0,79 | 0,002216649 |
| miR.609 + miR.220a + miR.26a + miR.29a + miR.376a* + miR.518c | 0,79 | 0,488707209 |
| miR.609 + miR.603 + miR.29a + miR.376a* + miR.518c + miR.660 | 0,79 | 0,002116363 |
| miR.609 + miR.603 + miR.130a + miR.29a + miR.376a* + miR.518c | 0,79 | 0,004761298 |
| miR.609 + miR.520f + miR.29a + miR.362 + miR.376a* + miR.518c | 0,79 | 0,309625965 |
| miR.609 + miR.520f + miR.26a + miR.29a + miR.376a* + miR.518c | 0,79 | 0,361650132 |
| miR.609 + miR.520f + miR.130a + miR.29a + miR.376a* + miR.518c | 0,79 | 0,26767295 |
| miR.609 + miR.603 + miR.29a + miR.376a* + miR.518c + miR.338 | 0,79 | 0,009226262 |
| miR.558 + miR.609 + miR.520f + miR.29a + miR.376a* + miR.639 | 0,79 | 0,120239833 |
| miR.609 + miR.220a + miR.29a + miR.376a* + miR.518c + miR.338 | 0,79 | 0,39600383 |
| miR.609 + miR.603 + miR.519b + miR.29a + miR.376a* + miR.518c | 0,79 | 0,003518071 |
| miR.558 + miR.609 + miR.520f + miR.519b + miR.29a + miR.376a* | 0,79 | 0,131613028 |
| miR.609 + miR.220a + miR.29a + miR.376a* + miR.518c + miR.660 | 0,79 | 0,293581541 |
| miR.609 + miR.520f + miR.29a + miR.376a* + miR.518c + miR.639 | 0,79 | 0,277735408 |
| miR.609 + miR.220a + miR.29a + miR.376a* + miR.518c + miR.639 | 0,79 | 0,409188251 |
| miR.558 + miR.609 + miR.520f + miR.130a + miR.29a + miR.376a* | 0,79 | 0,123312922 |
| miR.609 + miR.603 + miR.220a + miR.130a + miR.376a* + miR.518c | 0,79 | 0,093728469 |
| miR.558 + miR.609 + miR.520f + miR.26a + miR.29a + miR.376a* | 0,79 | 0,105948702 |
| miR.558 + miR.609 + miR.520f + miR.220a + miR.29a + miR.376a* | 0,78 | 0,115738585 |
| miR.609 + miR.220a + miR.130a + miR.29a + miR.376a* + miR.518c | 0,78 | 0,479361199 |
| miR.558 + miR.609 + miR.520f + miR.29a + miR.362 + miR.376a* | 0,78 | 0,129581995 |
| miR.558 + miR.609 + miR.603 + miR.130a + miR.376a* + miR.518c | 0,78 | 0,027048087 |
| miR.558 + miR.609 + miR.603 + miR.26a + miR.29a + miR.518c | 0,78 | 0,017201072 |
| miR.558 + miR.609 + miR.369.3p + miR.376a* + miR.518c + miR.639 | 0,78 | 0,268171173 |
| miR.558 + miR.609 + miR.220a + miR.130a + miR.376a* + miR.518c | 0,78 | 0,071004976 |
| miR.558 + miR.609 + miR.603 + miR.220a + miR.376a* + miR.518c | 0,78 | 0,037830085 |
| miR.558 + miR.609 + miR.220a + miR.26a + miR.29a + miR.518c | 0,78 | 0,018586641 |
| miR.558 + miR.609 + miR.520f + miR.26a + miR.29a + miR.518c | 0,78 | 0,023669412 |
| miR.558 + miR.609 + miR.220a + miR.376a* + miR.518c + miR.639 | 0,78 | 0,146858934 |
| miR.609 + miR.603 + miR.520f + miR.130a + miR.376a* + miR.518c | 0,78 | 0,029015602 |
| miR.558 + miR.609 + miR.26a + miR.29a + miR.362 + miR.518c | 0,78 | 0,018300028 |
| miR.609 + miR.519b + miR.29a + miR.369.3p + miR.376a* + miR.518c | 0,78 | 0,424917376 |
| miR.558 + miR.609 + miR.26a + miR.29a + miR.369.3p + miR.518c | 0,78 | 0,022564996 |
| miR.558 + miR.609 + miR.603 + miR.369.3p + miR.376a* + miR.518c | 0,78 | 0,088380038 |
| miR.558 + miR.609 + miR.520f + miR.29a + miR.518c + miR.338 | 0,78 | 0,015998239 |
| miR.558 + miR.609 + miR.26a + miR.29a + miR.518c + miR.338 | 0,78 | 0,023785496 |
| miR.558 + miR.609 + miR.130a + miR.29a + miR.518c + miR.660 | 0,78 | 0,001551724 |
| miR.558 + miR.609 + miR.130a + miR.376a* + miR.518c + miR.639 | 0,78 | 0,135766224 |
| miR.558 + miR.609 + miR.520f + miR.519b + miR.29a + miR.518c | 0,78 | 0,035621536 |
| miR.558 + miR.609 + miR.220a + miR.29a + miR.518c + miR.660 | 0,78 | 0,00600659 |
| miR.609 + miR.520f + miR.29a + miR.376a* + miR.494 + miR.338 | 0,78 | 0,218054449 |
| miR.558 + miR.609 + miR.520f + miR.29a + miR.362 + miR.518c | 0,78 | 0,017324965 |
| miR.609 + miR.603 + miR.369.3p + miR.376a* + miR.518c + miR.639 | 0,78 | 0,052726214 |
| miR.558 + miR.609 + miR.520f + miR.130a + miR.29a + miR.518c | 0,78 | 0,015419299 |
| miR.558 + miR.609 + miR.520f + miR.130a + miR.376a* + miR.338 | 0,78 | 0,303295878 |
| miR.558 + miR.609 + miR.26a + miR.29a + miR.518c + miR.639 | 0,78 | 0,013352756 |
| miR.558 + miR.609 + miR.520f + miR.29a + miR.518c + miR.639 | 0,78 | 0,019870772 |
| miR.558 + miR.609 + miR.130a + miR.26a + miR.29a + miR.518c | 0,78 | 0,019211323 |
| miR.558 + miR.609 + miR.520f + miR.518c + miR.639 + miR.660 | 0,78 | 0,141871505 |
| miR.558 + miR.609 + miR.520f + miR.220a + miR.29a + miR.518c | 0,78 | 0,015020769 |
| miR.558 + miR.609 + miR.520f + miR.29a + miR.369.3p + miR.518c | 0,78 | 0,010237864 |
| miR.558 + miR.609 + miR.520f + miR.29a + miR.494 + miR.518c | 0,78 | 0,009486224 |
| miR.558 + miR.609 + miR.603 + miR.29a + miR.518c + miR.660 | 0,78 | 0,006453536 |
| miR.558 + miR.609 + miR.29a + miR.362 + miR.369.3p + miR.376a* | 0,78 | 0,120744206 |
| miR.558 + miR.609 + miR.26a + miR.29a + miR.494 + miR.518c | 0,78 | 0,014994735 |
| miR.558 + miR.609 + miR.603 + miR.520f + miR.29a + miR.518c | 0,78 | 0,021035633 |
| miR.558 + miR.609 + miR.519b + miR.26a + miR.29a + miR.518c | 0,78 | 0,018119309 |
| miR.609 + miR.603 + miR.220a + miR.376a* + miR.518c + miR.639 | 0,78 | 0,028081925 |
| miR.609 + miR.603 + miR.130a + miR.369.3p + miR.376a* + miR.518c | 0,78 | 0,023330265 |
| miR.609 + miR.603 + miR.520f + miR.376a* + miR.518c + miR.639 | 0,78 | 0,039819944 |
| miR.609 + miR.520f + miR.220a + miR.130a + miR.376a* + miR.518c | 0,78 | 0,040364078 |
| miR.558 + miR.609 + miR.130a + miR.369.3p + miR.376a* + miR.518c | 0,78 | 0,147174396 |
| miR.609 + miR.29a + miR.369.3p + miR.376a* + miR.494 + miR.518c | 0,78 | 0,212674547 |
| miR.609 + miR.603 + miR.520f + miR.29a + miR.376a* + miR.494 | 0,78 | 0,095803005 |
| miR.558 + miR.609 + miR.29a + miR.362 + miR.518c + miR.660 | 0,78 | 0,005039364 |
| miR.609 + miR.603 + miR.130a + miR.376a* + miR.518c + miR.639 | 0,78 | 0,018369545 |
| miR.609 + miR.519b + miR.29a + miR.376a* + miR.518c + miR.639 | 0,78 | 0,019397867 |
| miR.558 + miR.609 + miR.220a + miR.29a + miR.369.3p + miR.376a* | 0,78 | 0,140691113 |
| miR.558 + miR.609 + miR.220a + miR.369.3p + miR.376a* + miR.518c | 0,78 | 0,124067642 |
| miR.558 + miR.609 + miR.603 + miR.520f + miR.518c + miR.639 | 0,78 | 0,043027803 |
| miR.558 + miR.609 + miR.29a + miR.369.3p + miR.376a* + miR.494 | 0,78 | 0,166394208 |
| miR.609 + miR.520f + miR.29a + miR.369.3p + miR.376a* + miR.494 | 0,78 | 0,225078086 |
| miR.609 + miR.520f + miR.26a + miR.29a + miR.376a* + miR.494 | 0,78 | 0,126890301 |
| miR.558 + miR.609 + miR.26a + miR.29a + miR.369.3p + miR.376a* | 0,78 | 0,142788727 |
| miR.558 + miR.609 + miR.29a + miR.518c + miR.660 + miR.338 | 0,78 | 0,00250342 |
| miR.558 + miR.609 + miR.603 + miR.29a + miR.369.3p + miR.376a* | 0,78 | 0,126575709 |
| miR.558 + miR.609 + miR.362 + miR.376a* + miR.518c + miR.639 | 0,78 | 0,133604741 |
| miR.609 + miR.520f + miR.220a + miR.29a + miR.518c + miR.660 | 0,78 | 0,033902659 |
| miR.609 + miR.519b + miR.29a + miR.376a* + miR.494 + miR.518c | 0,78 | 0,057426252 |
| miR.558 + miR.609 + miR.376a* + miR.518c + miR.639 + miR.338 | 0,78 | 0,119882578 |
| miR.609 + miR.29a + miR.376a* + miR.494 + miR.518c + miR.639 | 0,78 | 0,064820004 |
| miR.558 + miR.609 + miR.376a* + miR.494 + miR.518c + miR.639 | 0,78 | 0,235901663 |
| miR.609 + miR.603 + miR.220a + miR.369.3p + miR.376a* + miR.518c | 0,78 | 0,046852084 |
| miR.609 + miR.519b + miR.26a + miR.29a + miR.376a* + miR.518c | 0,78 | 0,010708884 |
| miR.558 + miR.609 + miR.26a + miR.376a* + miR.518c + miR.639 | 0,78 | 0,126690944 |
| miR.558 + miR.609 + miR.220a + miR.29a + miR.518c + miR.338 | 0,78 | 0,006155848 |
| miR.609 + miR.29a + miR.362 + miR.369.3p + miR.376a* + miR.518c | 0,78 | 0,303261509 |
| miR.609 + miR.520f + miR.130a + miR.376a* + miR.518c + miR.639 | 0,78 | 0,066060212 |
| miR.558 + miR.609 + miR.519b + miR.376a* + miR.518c + miR.639 | 0,78 | 0,128912262 |
| miR.558 + miR.609 + miR.603 + miR.220a + miR.519b + miR.518c | 0,78 | 0,052360911 |
| miR.558 + miR.609 + miR.376a* + miR.518c + miR.639 + miR.660 | 0,78 | 0,119939336 |
| miR.609 + miR.520f + miR.29a + miR.494 + miR.518c + miR.660 | 0,78 | 0,036862504 |
| miR.558 + miR.609 + miR.603 + miR.29a + miR.518c + miR.338 | 0,78 | 0,007693048 |
| miR.609 + miR.29a + miR.369.3p + miR.376a* + miR.518c + miR.639 | 0,78 | 0,328525056 |
| miR.609 + miR.520f + miR.29a + miR.376a* + miR.494 + miR.660 | 0,78 | 0,159619532 |
| miR.558 + miR.609 + miR.603 + miR.220a + miR.29a + miR.518c | 0,78 | 0,01005745 |
| miR.558 + miR.609 + miR.603 + miR.520f + miR.376a* + miR.518c | 0,78 | 0,043056915 |
| miR.609 + miR.520f + miR.29a + miR.376a* + miR.494 + miR.639 | 0,78 | 0,092755061 |
| miR.609 + miR.520f + miR.220a + miR.29a + miR.376a* + miR.494 | 0,78 | 0,088959415 |
| miR.609 + miR.520f + miR.29a + miR.518c + miR.639 + miR.660 | 0,78 | 0,03311913 |
| miR.558 + miR.609 + miR.130a + miR.362 + miR.376a* + miR.518c | 0,78 | 0,047174907 |
| miR.558 + miR.609 + miR.29a + miR.518c + miR.639 + miR.660 | 0,78 | 0,007003397 |
| miR.558 + miR.609 + miR.130a + miR.376a* + miR.518c + miR.338 | 0,78 | 0,054960555 |
| miR.609 + miR.519b + miR.130a + miR.29a + miR.376a* + miR.518c | 0,78 | 0,018045409 |
| miR.558 + miR.609 + miR.29a + miR.369.3p + miR.376a* + miR.338 | 0,78 | 0,105915537 |
| miR.609 + miR.519b + miR.29a + miR.362 + miR.376a* + miR.518c | 0,78 | 0,008708266 |
| miR.609 + miR.519b + miR.29a + miR.376a* + miR.518c + miR.338 | 0,78 | 0,008592583 |
| miR.609 + miR.520f + miR.519b + miR.29a + miR.376a* + miR.494 | 0,78 | 0,07554015 |
| miR.558 + miR.609 + miR.520f + miR.26a + miR.376a* + miR.518c | 0,78 | 0,033047054 |
| miR.558 + miR.609 + miR.519b + miR.29a + miR.518c + miR.660 | 0,78 | 0,0033052 |
| miR.609 + miR.519b + miR.29a + miR.376a* + miR.518c + miR.660 | 0,78 | 0,009079165 |
| miR.558 + miR.609 + miR.130a + miR.29a + miR.518c + miR.338 | 0,78 | 0,002325394 |
| miR.609 + miR.29a + miR.369.3p + miR.376a* + miR.518c + miR.660 | 0,78 | 0,275695159 |
| miR.609 + miR.603 + miR.376a* + miR.494 + miR.518c + miR.639 | 0,78 | 0,03123954 |
| miR.558 + miR.609 + miR.220a + miR.130a + miR.29a + miR.518c | 0,78 | 0,001458956 |
| miR.558 + miR.609 + miR.130a + miR.26a + miR.376a* + miR.518c | 0,78 | 0,067089746 |
| miR.609 + miR.520f + miR.130a + miR.376a* + miR.518c + miR.338 | 0,78 | 0,115491461 |
| miR.558 + miR.609 + miR.29a + miR.369.3p + miR.376a* + miR.639 | 0,78 | 0,168741776 |
| miR.609 + miR.520f + miR.130a + miR.29a + miR.376a* + miR.338 | 0,78 | 0,265488968 |
| miR.558 + miR.609 + miR.29a + miR.369.3p + miR.518c + miR.660 | 0,78 | 0,006670492 |
| miR.609 + miR.520f + miR.29a + miR.369.3p + miR.376a* + miR.338 | 0,78 | 0,370072661 |
| miR.609 + miR.520f + miR.29a + miR.362 + miR.376a* + miR.494 | 0,78 | 0,08150245 |
| miR.609 + miR.520f + miR.130a + miR.29a + miR.376a* + miR.494 | 0,78 | 0,089734587 |
| miR.558 + miR.609 + miR.603 + miR.376a* + miR.518c + miR.338 | 0,78 | 0,041849811 |
| miR.609 + miR.26a + miR.29a + miR.369.3p + miR.376a* + miR.518c | 0,78 | 0,263624916 |
| miR.558 + miR.609 + miR.603 + miR.519b + miR.29a + miR.518c | 0,78 | 0,012130373 |
| miR.609 + miR.520f + miR.29a + miR.362 + miR.518c + miR.660 | 0,78 | 0,025516226 |
| miR.558 + miR.609 + miR.603 + miR.519b + miR.376a* + miR.518c | 0,78 | 0,03013586 |
| miR.558 + miR.609 + miR.520f + miR.362 + miR.518c + miR.639 | 0,78 | 0,042176469 |
| miR.558 + miR.609 + miR.520f + miR.130a + miR.518c + miR.639 | 0,78 | 0,028306951 |
| miR.558 + miR.609 + miR.26a + miR.29a + miR.376a* + miR.494 | 0,78 | 0,046957121 |
| miR.609 + miR.220a + miR.130a + miR.369.3p + miR.376a* + miR.518c | 0,78 | 0,044898898 |
| miR.558 + miR.609 + miR.603 + miR.376a* + miR.518c + miR.660 | 0,78 | 0,030903885 |
| miR.558 + miR.609 + miR.362 + miR.369.3p + miR.376a* + miR.518c | 0,78 | 0,112556837 |
| miR.558 + miR.609 + miR.520f + miR.518c + miR.639 + miR.338 | 0,78 | 0,039709829 |
| miR.558 + miR.609 + miR.29a + miR.494 + miR.518c + miR.660 | 0,78 | 0,003625737 |
| miR.609 + miR.603 + miR.520f + miR.29a + miR.518c + miR.660 | 0,78 | 0,019161415 |
| miR.558 + miR.609 + miR.603 + miR.362 + miR.376a* + miR.518c | 0,78 | 0,028918491 |
| miR.558 + miR.609 + miR.603 + miR.130a + miR.29a + miR.518c | 0,78 | 0,004039676 |
| miR.558 + miR.609 + miR.603 + miR.376a* + miR.494 + miR.518c | 0,78 | 0,075790989 |
| miR.558 + miR.609 + miR.520f + miR.220a + miR.518c + miR.639 | 0,78 | 0,041798464 |
| miR.558 + miR.609 + miR.130a + miR.376a* + miR.518c + miR.660 | 0,78 | 0,037992011 |
| miR.558 + miR.609 + miR.520f + miR.369.3p + miR.518c + miR.639 | 0,78 | 0,042386029 |
| miR.558 + miR.609 + miR.520f + miR.494 + miR.518c + miR.639 | 0,78 | 0,047663269 |
| miR.558 + miR.609 + miR.520f + miR.26a + miR.518c + miR.639 | 0,78 | 0,040311664 |
| miR.558 + miR.609 + miR.603 + miR.520f + miR.130a + miR.376a* | 0,78 | 0,108630608 |
| miR.558 + miR.609 + miR.130a + miR.29a + miR.369.3p + miR.376a* | 0,78 | 0,121675959 |
| miR.558 + miR.609 + miR.519b + miR.29a + miR.369.3p + miR.376a* | 0,78 | 0,121675959 |
| miR.558 + miR.609 + miR.520f + miR.519b + miR.518c + miR.639 | 0,78 | 0,06245737 |
| miR.558 + miR.609 + miR.29a + miR.369.3p + miR.376a* + miR.660 | 0,78 | 0,120491127 |
| miR.609 + miR.603 + miR.130a + miR.362 + miR.376a* + miR.518c | 0,78 | 0,007456785 |
| miR.609 + miR.520f + miR.130a + miR.29a + miR.518c + miR.660 | 0,78 | 0,017846749 |
| miR.558 + miR.609 + miR.603 + miR.29a + miR.376a* + miR.494 | 0,78 | 0,017722258 |
| miR.558 + miR.609 + miR.220a + miR.29a + miR.376a* + miR.494 | 0,78 | 0,033479475 |
| miR.609 + miR.26a + miR.29a + miR.376a* + miR.494 + miR.518c | 0,78 | 0,03916501 |
| miR.609 + miR.603 + miR.130a + miR.376a* + miR.518c + miR.338 | 0,78 | 0,0151384 |
| miR.609 + miR.130a + miR.29a + miR.369.3p + miR.376a* + miR.518c | 0,78 | 0,237823936 |
| miR.609 + miR.603 + miR.130a + miR.26a + miR.376a* + miR.518c | 0,78 | 0,006029358 |
| miR.558 + miR.609 + miR.603 + miR.26a + miR.518c + miR.639 | 0,78 | 0,046808887 |
| miR.609 + miR.520f + miR.29a + miR.369.3p + miR.518c + miR.660 | 0,78 | 0,016655018 |
| miR.558 + miR.609 + miR.520f + miR.130a + miR.369.3p + miR.376a* | 0,78 | 0,219283343 |
| miR.609 + miR.29a + miR.362 + miR.376a* + miR.494 + miR.518c | 0,78 | 0,086495985 |
| miR.558 + miR.609 + miR.220a + miR.26a + miR.29a + miR.376a* | 0,78 | 0,009727333 |
| miR.558 + miR.609 + miR.603 + miR.29a + miR.369.3p + miR.518c | 0,78 | 0,006324878 |
| miR.609 + miR.603 + miR.130a + miR.376a* + miR.518c + miR.660 | 0,78 | 0,004947727 |
| miR.558 + miR.609 + miR.603 + miR.520f + miR.519b + miR.518c | 0,78 | 0,03236939 |
| miR.558 + miR.609 + miR.130a + miR.29a + miR.518c + miR.639 | 0,78 | 0,001807018 |
| miR.558 + miR.603 + miR.220a + miR.519b + miR.376a* + miR.518c | 0,78 | 0,071846662 |
| miR.609 + miR.603 + miR.220a + miR.29a + miR.518c + miR.660 | 0,78 | 0,011602529 |
| miR.558 + miR.603 + miR.220a + miR.369.3p + miR.376a* + miR.518c | 0,78 | 0,08367822 |
| miR.609 + miR.29a + miR.369.3p + miR.376a* + miR.518c + miR.338 | 0,78 | 0,314466464 |
| miR.609 + miR.603 + miR.520f + miR.29a + miR.376a* + miR.338 | 0,78 | 0,256073965 |
| miR.609 + miR.603 + miR.520f + miR.29a + miR.369.3p + miR.376a* | 0,78 | 0,199149406 |
| miR.558 + miR.609 + miR.130a + miR.376a* + miR.494 + miR.518c | 0,78 | 0,044155188 |
| miR.609 + miR.520f + miR.29a + miR.518c + miR.660 + miR.338 | 0,78 | 0,015927423 |
| miR.558 + miR.609 + miR.603 + miR.26a + miR.376a* + miR.518c | 0,78 | 0,033092463 |
| miR.609 + miR.130a + miR.29a + miR.376a* + miR.494 + miR.518c | 0,78 | 0,038978082 |
| miR.558 + miR.609 + miR.130a + miR.29a + miR.376a* + miR.494 | 0,78 | 0,024533495 |
| miR.558 + miR.609 + miR.603 + miR.520f + miR.130a + miR.518c | 0,78 | 0,038232842 |
| miR.558 + miR.609 + miR.519b + miR.130a + miR.29a + miR.518c | 0,78 | 0,002342319 |
| miR.558 + miR.609 + miR.519b + miR.130a + miR.376a* + miR.518c | 0,78 | 0,056517414 |
| miR.609 + miR.520f + miR.26a + miR.29a + miR.518c + miR.660 | 0,78 | 0,018204943 |
| miR.609 + miR.520f + miR.26a + miR.29a + miR.376a* + miR.338 | 0,78 | 0,258680925 |
| miR.609 + miR.29a + miR.376a* + miR.494 + miR.518c + miR.660 | 0,77 | 0,024560439 |
| miR.609 + miR.520f + miR.519b + miR.29a + miR.518c + miR.660 | 0,77 | 0,020408091 |
| miR.609 + miR.520f + miR.26a + miR.376a* + miR.518c + miR.639 | 0,77 | 0,064748083 |
| miR.558 + miR.609 + miR.603 + miR.520f + miR.220a + miR.518c | 0,77 | 0,021787736 |
| miR.558 + miR.609 + miR.220a + miR.519b + miR.376a* + miR.518c | 0,77 | 0,0447292 |
| miR.558 + miR.609 + miR.603 + miR.220a + miR.518c + miR.639 | 0,77 | 0,019775213 |
| miR.558 + miR.609 + miR.520f + miR.220a + miR.376a* + miR.518c | 0,77 | 0,056528715 |
| miR.558 + miR.609 + miR.220a + miR.519b + miR.29a + miR.518c | 0,77 | 0,00822999 |
| miR.558 + miR.609 + miR.520f + miR.130a + miR.26a + miR.376a* | 0,77 | 0,095906364 |
| miR.609 + miR.29a + miR.376a* + miR.494 + miR.518c + miR.338 | 0,77 | 0,047215942 |
| miR.558 + miR.609 + miR.603 + miR.220a + miR.518c + miR.660 | 0,77 | 0,018447378 |
| miR.609 + miR.520f + miR.130a + miR.26a + miR.376a* + miR.518c | 0,77 | 0,03724997 |
| miR.558 + miR.609 + miR.130a + miR.29a + miR.369.3p + miR.518c | 0,77 | 0,002453207 |
| miR.558 + miR.609 + miR.603 + miR.29a + miR.362 + miR.518c | 0,77 | 0,004641534 |
| miR.609 + miR.520f + miR.26a + miR.29a + miR.369.3p + miR.376a* | 0,77 | 0,388929625 |
| miR.609 + miR.603 + miR.520f + miR.130a + miR.29a + miR.376a* | 0,77 | 0,117512572 |
| miR.558 + miR.609 + miR.603 + miR.29a + miR.518c + miR.639 | 0,77 | 0,005046545 |
| miR.558 + miR.609 + miR.520f + miR.376a* + miR.518c + miR.338 | 0,77 | 0,161048175 |
| miR.558 + miR.609 + miR.520f + miR.369.3p + miR.376a* + miR.518c | 0,77 | 0,151625914 |
| miR.609 + miR.520f + miR.519b + miR.29a + miR.376a* + miR.338 | 0,77 | 0,20458255 |
| miR.558 + miR.609 + miR.520f + miR.376a* + miR.639 + miR.338 | 0,77 | 0,250373701 |
| miR.558 + miR.609 + miR.220a + miR.376a* + miR.518c + miR.338 | 0,77 | 0,083435977 |
| miR.609 + miR.520f + miR.519b + miR.29a + miR.369.3p + miR.376a* | 0,77 | 0,195417176 |
| miR.558 + miR.609 + miR.520f + miR.130a + miR.376a* + miR.639 | 0,77 | 0,134936459 |
| miR.609 + miR.603 + miR.519b + miR.376a* + miR.518c + miR.639 | 0,77 | 0,008328199 |
| miR.558 + miR.609 + miR.603 + miR.220a + miR.26a + miR.518c | 0,77 | 0,025277755 |
| miR.558 + miR.609 + miR.603 + miR.520f + miR.369.3p + miR.518c | 0,77 | 0,031808939 |
| miR.558 + miR.609 + miR.29a + miR.362 + miR.376a* + miR.494 | 0,77 | 0,030604567 |
| miR.558 + miR.609 + miR.603 + miR.220a + miR.518c + miR.338 | 0,77 | 0,022354317 |
| miR.558 + miR.609 + miR.603 + miR.220a + miR.29a + miR.376a* | 0,77 | 0,016896958 |
| miR.609 + miR.520f + miR.29a + miR.376a* + miR.639 + miR.338 | 0,77 | 0,19620365 |
| miR.609 + miR.603 + miR.362 + miR.376a* + miR.518c + miR.639 | 0,77 | 0,007184524 |
| miR.609 + miR.603 + miR.376a* + miR.518c + miR.639 + miR.338 | 0,77 | 0,007340145 |
| miR.558 + miR.609 + miR.603 + miR.29a + miR.494 + miR.518c | 0,77 | 0,003513241 |
| miR.609 + miR.603 + miR.519b + miR.130a + miR.376a* + miR.518c | 0,77 | 0,004945048 |
| miR.558 + miR.609 + miR.603 + miR.220a + miR.369.3p + miR.518c | 0,77 | 0,026723847 |
| miR.609 + miR.520f + miR.519b + miR.130a + miR.376a* + miR.518c | 0,77 | 0,017190898 |
| miR.609 + miR.603 + miR.130a + miR.376a* + miR.494 + miR.518c | 0,77 | 0,005030959 |
| miR.609 + miR.520f + miR.130a + miR.369.3p + miR.376a* + miR.518c | 0,77 | 0,037841858 |
| miR.609 + miR.520f + miR.130a + miR.29a + miR.369.3p + miR.376a* | 0,77 | 0,229369867 |
| miR.609 + miR.520f + miR.519b + miR.376a* + miR.518c + miR.639 | 0,77 | 0,033069795 |
| miR.558 + miR.609 + miR.29a + miR.376a* + miR.494 + miR.639 | 0,77 | 0,017693081 |
| miR.558 + miR.609 + miR.29a + miR.369.3p + miR.518c + miR.338 | 0,77 | 0,009476879 |
| miR.609 + miR.603 + miR.376a* + miR.518c + miR.639 + miR.660 | 0,77 | 0,004435959 |
| miR.558 + miR.609 + miR.520f + miR.130a + miR.518c + miR.660 | 0,77 | 0,029765067 |
| miR.558 + miR.609 + miR.369.3p + miR.376a* + miR.518c + miR.338 | 0,77 | 0,148815903 |
| miR.609 + miR.603 + miR.26a + miR.376a* + miR.518c + miR.639 | 0,77 | 0,00424272 |
| miR.558 + miR.609 + miR.130a + miR.29a + miR.494 + miR.518c | 0,77 | 0,001226394 |
| miR.558 + miR.609 + miR.29a + miR.376a* + miR.494 + miR.338 | 0,77 | 0,031320284 |
| miR.558 + miR.609 + miR.220a + miR.29a + miR.362 + miR.518c | 0,77 | 0,004050479 |
| miR.558 + miR.609 + miR.603 + miR.130a + miR.26a + miR.518c | 0,77 | 0,02497583 |
| miR.609 + miR.520f + miR.220a + miR.29a + miR.369.3p + miR.376a* | 0,77 | 0,152820685 |
| miR.609 + miR.520f + miR.29a + miR.376a* + miR.660 + miR.338 | 0,77 | 0,368688621 |
| miR.609 + miR.603 + miR.520f + miR.26a + miR.29a + miR.376a* | 0,77 | 0,127440633 |
| miR.609 + miR.520f + miR.220a + miR.29a + miR.376a* + miR.338 | 0,77 | 0,167521517 |
| miR.558 + miR.609 + miR.520f + miR.220a + miR.130a + miR.518c | 0,77 | 0,023300975 |
| miR.558 + miR.609 + miR.220a + miR.29a + miR.376a* + miR.338 | 0,77 | 0,009309358 |
| miR.558 + miR.609 + miR.520f + miR.519b + miR.130a + miR.518c | 0,77 | 0,020395383 |
| miR.558 + miR.609 + miR.29a + miR.362 + miR.518c + miR.338 | 0,77 | 0,008204675 |
| miR.558 + miR.609 + miR.220a + miR.29a + miR.376a* + miR.639 | 0,77 | 0,012364405 |
| miR.558 + miR.609 + miR.220a + miR.29a + miR.362 + miR.376a* | 0,77 | 0,015122956 |
| miR.558 + miR.609 + miR.220a + miR.29a + miR.518c + miR.639 | 0,77 | 0,002237474 |
| miR.558 + miR.609 + miR.220a + miR.29a + miR.494 + miR.518c | 0,77 | 0,001803869 |
| miR.558 + miR.609 + miR.220a + miR.376a* + miR.494 + miR.518c | 0,77 | 0,075716129 |
| miR.558 + miR.609 + miR.603 + miR.29a + miR.376a* + miR.639 | 0,77 | 0,010701564 |
| miR.558 + miR.609 + miR.29a + miR.518c + miR.639 + miR.338 | 0,77 | 0,008310265 |
| miR.558 + miR.609 + miR.220a + miR.130a + miR.29a + miR.376a* | 0,77 | 0,01373442 |
| miR.558 + miR.609 + miR.130a + miR.29a + miR.362 + miR.518c | 0,77 | 0,001299145 |
| miR.558 + miR.609 + miR.220a + miR.26a + miR.376a* + miR.518c | 0,77 | 0,033073663 |
| miR.558 + miR.609 + miR.520f + miR.130a + miR.362 + miR.518c | 0,77 | 0,01566809 |
| miR.558 + miR.609 + miR.220a + miR.29a + miR.376a* + miR.660 | 0,77 | 0,013804712 |
| miR.558 + miR.609 + miR.519b + miR.29a + miR.518c + miR.338 | 0,77 | 0,013321564 |
| miR.609 + miR.29a + miR.376a* + miR.518c + miR.639 + miR.660 | 0,77 | 0,024120709 |
| miR.558 + miR.609 + miR.29a + miR.494 + miR.518c + miR.338 | 0,77 | 0,006527235 |
| miR.558 + miR.609 + miR.603 + miR.520f + miR.26a + miR.518c | 0,77 | 0,023003976 |
| miR.558 + miR.609 + miR.220a + miR.29a + miR.369.3p + miR.518c | 0,77 | 0,001975563 |
| miR.609 + miR.603 + miR.220a + miR.376a* + miR.494 + miR.518c | 0,77 | 0,047933012 |
| miR.558 + miR.609 + miR.603 + miR.220a + miR.130a + miR.518c | 0,77 | 0,0140883 |
| miR.558 + miR.609 + miR.603 + miR.520f + miR.518c + miR.660 | 0,77 | 0,012731247 |
| miR.558 + miR.609 + miR.26a + miR.29a + miR.376a* + miR.639 | 0,77 | 0,015937405 |
| miR.558 + miR.609 + miR.220a + miR.362 + miR.376a* + miR.518c | 0,77 | 0,035003582 |
| miR.609 + miR.220a + miR.29a + miR.518c + miR.660 + miR.338 | 0,77 | 0,00097626 |
| miR.609 + miR.603 + miR.520f + miR.130a + miR.376a* + miR.338 | 0,77 | 0,218924232 |
| miR.609 + miR.220a + miR.26a + miR.29a + miR.518c + miR.660 | 0,77 | 0,001753314 |
| miR.558 + miR.609 + miR.220a + miR.376a* + miR.518c + miR.660 | 0,77 | 0,038367824 |
| miR.558 + miR.609 + miR.520f + miR.130a + miR.26a + miR.518c | 0,77 | 0,014789949 |
| miR.558 + miR.609 + miR.220a + miR.519b + miR.29a + miR.376a* | 0,77 | 0,021990482 |
| miR.558 + miR.609 + miR.603 + miR.220a + miR.362 + miR.518c | 0,77 | 0,025129012 |
| miR.558 + miR.609 + miR.603 + miR.520f + miR.518c + miR.338 | 0,77 | 0,02258038 |
| miR.558 + miR.609 + miR.603 + miR.520f + miR.494 + miR.518c | 0,77 | 0,016561973 |
| miR.558 + miR.609 + miR.603 + miR.520f + miR.362 + miR.518c | 0,77 | 0,021028373 |
| miR.558 + miR.609 + miR.29a + miR.376a* + miR.494 + miR.660 | 0,77 | 0,021775453 |
| miR.609 + miR.220a + miR.130a + miR.26a + miR.376a* + miR.518c | 0,77 | 0,007569947 |
| miR.558 + miR.603 + miR.220a + miR.29a + miR.376a* + miR.518c | 0,77 | 0,031755674 |
| miR.558 + miR.609 + miR.520f + miR.130a + miR.369.3p + miR.518c | 0,77 | 0,014593479 |
| miR.558 + miR.609 + miR.603 + miR.518c + miR.639 + miR.338 | 0,77 | 0,016413963 |
| miR.558 + miR.609 + miR.603 + miR.220a + miR.494 + miR.518c | 0,77 | 0,019203352 |
| miR.609 + miR.520f + miR.26a + miR.29a + miR.376a* + miR.639 | 0,77 | 0,110639251 |
| miR.558 + miR.609 + miR.520f + miR.130a + miR.494 + miR.518c | 0,77 | 0,017097309 |
| miR.558 + miR.609 + miR.603 + miR.369.3p + miR.518c + miR.639 | 0,77 | 0,020788841 |
| miR.609 + miR.520f + miR.29a + miR.362 + miR.376a* + miR.338 | 0,77 | 0,245277466 |
| miR.609 + miR.603 + miR.29a + miR.369.3p + miR.376a* + miR.494 | 0,77 | 0,101266137 |
| miR.609 + miR.520f + miR.130a + miR.26a + miR.29a + miR.376a* | 0,77 | 0,11994057 |
| miR.609 + miR.220a + miR.519b + miR.130a + miR.376a* + miR.518c | 0,77 | 0,008059565 |
| miR.609 + miR.520f + miR.29a + miR.369.3p + miR.376a* + miR.639 | 0,77 | 0,17954231 |
| miR.558 + miR.609 + miR.519b + miR.29a + miR.376a* + miR.494 | 0,77 | 0,028918961 |
| miR.609 + miR.603 + miR.220a + miR.29a + miR.518c + miR.338 | 0,77 | 0,013917486 |
| miR.558 + miR.609 + miR.603 + miR.518c + miR.639 + miR.660 | 0,77 | 0,028832708 |
| miR.558 + miR.609 + miR.603 + miR.26a + miR.29a + miR.376a* | 0,77 | 0,005596967 |
| miR.558 + miR.603 + miR.220a + miR.376a* + miR.518c + miR.639 | 0,77 | 0,044013585 |
| miR.609 + miR.520f + miR.519b + miR.130a + miR.29a + miR.376a* | 0,77 | 0,110347939 |
| miR.609 + miR.130a + miR.29a + miR.376a* + miR.518c + miR.639 | 0,77 | 0,03202496 |
| miR.558 + miR.609 + miR.603 + miR.519b + miR.518c + miR.639 | 0,77 | 0,025874765 |
| miR.609 + miR.603 + miR.362 + miR.369.3p + miR.376a* + miR.518c | 0,77 | 0,018929539 |
| miR.558 + miR.609 + miR.519b + miR.26a + miR.29a + miR.376a* | 0,77 | 0,006899444 |
| miR.558 + miR.603 + miR.520f + miR.376a* + miR.518c + miR.639 | 0,77 | 0,050784236 |
| miR.609 + miR.26a + miR.29a + miR.376a* + miR.518c + miR.639 | 0,77 | 0,031111936 |
| miR.603 + miR.220a + miR.29a + miR.369.3p + miR.376a* + miR.518c | 0,77 | 0,071892876 |
| miR.603 + miR.220a + miR.369.3p + miR.376a* + miR.518c + miR.639 | 0,77 | 0,056473226 |
| miR.609 + miR.220a + miR.369.3p + miR.376a* + miR.518c + miR.639 | 0,77 | 0,015191152 |
| miR.558 + miR.609 + miR.220a + miR.26a + miR.518c + miR.639 | 0,77 | 0,055363873 |
| miR.609 + miR.29a + miR.362 + miR.376a* + miR.518c + miR.639 | 0,77 | 0,032246525 |
| miR.609 + miR.29a + miR.376a* + miR.518c + miR.639 + miR.338 | 0,77 | 0,033895696 |
| miR.558 + miR.609 + miR.26a + miR.29a + miR.376a* + miR.660 | 0,77 | 0,009362703 |
| miR.609 + miR.603 + miR.520f + miR.220a + miR.29a + miR.518c | 0,77 | 0,027071432 |
| miR.558 + miR.609 + miR.520f + miR.130a + miR.362 + miR.376a* | 0,77 | 0,108536286 |
| miR.609 + miR.520f + miR.220a + miR.130a + miR.29a + miR.376a* | 0,77 | 0,099971942 |
| miR.609 + miR.520f + miR.26a + miR.29a + miR.376a* + miR.660 | 0,77 | 0,166622163 |
| miR.558 + miR.609 + miR.520f + miR.130a + miR.518c + miR.338 | 0,77 | 0,017531482 |
| miR.558 + miR.609 + miR.603 + miR.494 + miR.518c + miR.639 | 0,77 | 0,014891641 |
| miR.558 + miR.609 + miR.29a + miR.376a* + miR.639 + miR.338 | 0,77 | 0,013546542 |
| miR.609 + miR.26a + miR.29a + miR.376a* + miR.518c + miR.660 | 0,77 | 0,013272168 |
| miR.558 + miR.609 + miR.520f + miR.26a + miR.376a* + miR.639 | 0,77 | 0,094588654 |
| miR.609 + miR.520f + miR.220a + miR.26a + miR.29a + miR.376a* | 0,77 | 0,137256623 |
| miR.558 + miR.603 + miR.520f + miR.220a + miR.376a* + miR.518c | 0,77 | 0,041411027 |
| miR.609 + miR.520f + miR.519b + miR.26a + miR.29a + miR.376a* | 0,77 | 0,12140393 |
| miR.558 + miR.609 + miR.220a + miR.130a + miR.26a + miR.518c | 0,77 | 0,016521337 |
| miR.609 + miR.520f + miR.130a + miR.362 + miR.376a* + miR.518c | 0,77 | 0,012298954 |
| miR.558 + miR.609 + miR.369.3p + miR.376a* + miR.518c + miR.660 | 0,77 | 0,084244914 |
| miR.558 + miR.609 + miR.520f + miR.130a + miR.376a* + miR.494 | 0,77 | 0,113821271 |
| miR.558 + miR.609 + miR.29a + miR.362 + miR.376a* + miR.639 | 0,77 | 0,01408851 |
| miR.558 + miR.609 + miR.520f + miR.220a + miR.519b + miR.518c | 0,77 | 0,044301384 |
| miR.609 + miR.520f + miR.519b + miR.29a + miR.376a* + miR.639 | 0,77 | 0,078616685 |
| miR.609 + miR.520f + miR.29a + miR.362 + miR.369.3p + miR.376a* | 0,77 | 0,127243263 |
| miR.558 + miR.603 + miR.220a + miR.130a + miR.376a* + miR.518c | 0,77 | 0,032649417 |
| miR.609 + miR.520f + miR.29a + miR.369.3p + miR.376a* + miR.660 | 0,77 | 0,234423912 |
| miR.558 + miR.609 + miR.520f + miR.519b + miR.130a + miR.376a* | 0,77 | 0,107754432 |
| miR.558 + miR.609 + miR.520f + miR.130a + miR.376a* + miR.660 | 0,77 | 0,105306429 |
| miR.558 + miR.609 + miR.603 + miR.130a + miR.518c + miR.639 | 0,77 | 0,018065119 |
| miR.609 + miR.603 + miR.520f + miR.29a + miR.376a* + miR.639 | 0,77 | 0,083710321 |
| miR.609 + miR.220a + miR.130a + miR.362 + miR.376a* + miR.518c | 0,77 | 0,009445869 |
| miR.609 + miR.520f + miR.130a + miR.376a* + miR.518c + miR.660 | 0,77 | 0,010483661 |
| miR.558 + miR.609 + miR.603 + miR.362 + miR.518c + miR.639 | 0,77 | 0,018899261 |
| miR.609 + miR.520f + miR.130a + miR.376a* + miR.494 + miR.518c | 0,77 | 0,010472044 |
| miR.609 + miR.220a + miR.130a + miR.376a* + miR.518c + miR.639 | 0,77 | 0,00817285 |
| miR.558 + miR.609 + miR.130a + miR.29a + miR.376a* + miR.639 | 0,77 | 0,011349157 |
| miR.609 + miR.603 + miR.220a + miR.26a + miR.29a + miR.518c | 0,77 | 0,02045932 |
| miR.558 + miR.603 + miR.520f + miR.220a + miR.519b + miR.518c | 0,77 | 0,067945261 |
| miR.609 + miR.520f + miR.130a + miR.29a + miR.376a* + miR.660 | 0,77 | 0,090321044 |
| miR.609 + miR.603 + miR.520f + miR.29a + miR.518c + miR.338 | 0,77 | 0,01144112 |
| miR.609 + miR.603 + miR.520f + miR.220a + miR.29a + miR.376a* | 0,77 | 0,104546742 |
| miR.558 + miR.609 + miR.26a + miR.29a + miR.362 + miR.376a* | 0,77 | 0,00565928 |
| miR.609 + miR.520f + miR.220a + miR.376a* + miR.518c + miR.639 | 0,77 | 0,025482588 |
| miR.609 + miR.29a + miR.362 + miR.376a* + miR.518c + miR.660 | 0,77 | 0,028162606 |
| miR.558 + miR.609 + miR.520f + miR.220a + miR.130a + miR.376a* | 0,77 | 0,121940052 |
| miR.558 + miR.609 + miR.520f + miR.29a + miR.362 + miR.660 | 0,77 | 0,071950411 |
| miR.558 + miR.609 + miR.603 + miR.26a + miR.369.3p + miR.518c | 0,77 | 0,042242342 |
| miR.609 + miR.603 + miR.220a + miR.519b + miR.376a* + miR.518c | 0,77 | 0,00831626 |
| miR.609 + miR.520f + miR.130a + miR.29a + miR.376a* + miR.639 | 0,77 | 0,084943006 |
| miR.558 + miR.609 + miR.130a + miR.26a + miR.29a + miR.376a* | 0,77 | 0,010687046 |
| miR.609 + miR.603 + miR.520f + miR.29a + miR.376a* + miR.660 | 0,77 | 0,089051081 |
| miR.609 + miR.520f + miR.26a + miR.29a + miR.362 + miR.376a* | 0,77 | 0,125842842 |
| miR.558 + miR.609 + miR.26a + miR.29a + miR.376a* + miR.338 | 0,77 | 0,010653659 |
| miR.609 + miR.220a + miR.29a + miR.362 + miR.518c + miR.660 | 0,77 | 0,003228105 |
| miR.558 + miR.609 + miR.26a + miR.369.3p + miR.518c + miR.639 | 0,77 | 0,084756756 |
| miR.609 + miR.520f + miR.220a + miR.29a + miR.362 + miR.518c | 0,77 | 0,006958009 |
| miR.609 + miR.29a + miR.362 + miR.376a* + miR.518c + miR.338 | 0,77 | 0,011074393 |
| miR.609 + miR.603 + miR.520f + miR.29a + miR.518c + miR.639 | 0,77 | 0,017446179 |
| miR.558 + miR.609 + miR.520f + miR.29a + miR.639 + miR.660 | 0,77 | 0,064805339 |
| miR.558 + miR.609 + miR.519b + miR.29a + miR.376a* + miR.639 | 0,77 | 0,008827513 |
| miR.558 + miR.609 + miR.29a + miR.376a* + miR.639 + miR.660 | 0,77 | 0,009735835 |
| miR.609 + miR.220a + miR.130a + miR.376a* + miR.494 + miR.518c | 0,77 | 0,008170883 |
| miR.609 + miR.520f + miR.220a + miR.29a + miR.518c + miR.338 | 0,77 | 0,008962688 |
| miR.558 + miR.609 + miR.603 + miR.29a + miR.376a* + miR.660 | 0,77 | 0,002801981 |
| miR.609 + miR.29a + miR.376a* + miR.518c + miR.660 + miR.338 | 0,77 | 0,011595155 |
| miR.609 + miR.26a + miR.29a + miR.362 + miR.376a* + miR.518c | 0,77 | 0,011813893 |
| miR.609 + miR.220a + miR.130a + miR.376a* + miR.518c + miR.660 | 0,77 | 0,0051046 |
| miR.603 + miR.220a + miR.130a + miR.369.3p + miR.376a* + miR.518c | 0,77 | 0,07082274 |
| miR.609 + miR.26a + miR.29a + miR.376a* + miR.518c + miR.338 | 0,77 | 0,011217691 |
| miR.558 + miR.609 + miR.603 + miR.519b + miR.369.3p + miR.518c | 0,77 | 0,024556776 |
| miR.558 + miR.609 + miR.362 + miR.518c + miR.639 + miR.660 | 0,77 | 0,068240133 |
| miR.558 + miR.609 + miR.603 + miR.130a + miR.518c + miR.338 | 0,77 | 0,014304072 |
| miR.558 + miR.609 + miR.603 + miR.130a + miR.29a + miR.376a* | 0,77 | 0,004576634 |
| miR.609 + miR.520f + miR.130a + miR.29a + miR.362 + miR.376a* | 0,77 | 0,093750787 |
| miR.558 + miR.609 + miR.603 + miR.519b + miR.130a + miR.518c | 0,77 | 0,023536179 |
| miR.609 + miR.130a + miR.26a + miR.29a + miR.376a* + miR.518c | 0,77 | 0,021357078 |
| miR.609 + miR.603 + miR.26a + miR.29a + miR.518c + miR.660 | 0,77 | 0,002390266 |
| miR.609 + miR.130a + miR.29a + miR.362 + miR.376a* + miR.518c | 0,77 | 0,033894424 |
| miR.609 + miR.603 + miR.520f + miR.29a + miR.362 + miR.518c | 0,77 | 0,015426528 |
| miR.609 + miR.520f + miR.519b + miR.29a + miR.376a* + miR.660 | 0,77 | 0,098260698 |
| miR.609 + miR.603 + miR.520f + miR.519b + miR.29a + miR.376a* | 0,77 | 0,094345939 |
| miR.558 + miR.609 + miR.603 + miR.130a + miR.369.3p + miR.518c | 0,77 | 0,018185215 |
| miR.609 + miR.603 + miR.520f + miR.220a + miR.376a* + miR.518c | 0,77 | 0,018022844 |
| miR.609 + miR.603 + miR.520f + miR.519b + miR.29a + miR.518c | 0,77 | 0,016525501 |
| miR.558 + miR.603 + miR.220a + miR.519b + miR.26a + miR.518c | 0,77 | 0,045168349 |
| miR.558 + miR.609 + miR.520f + miR.220a + miR.518c + miR.660 | 0,77 | 0,010454011 |
| miR.558 + miR.609 + miR.130a + miR.26a + miR.518c + miR.639 | 0,77 | 0,04326088 |
| miR.558 + miR.609 + miR.603 + miR.29a + miR.362 + miR.376a* | 0,77 | 0,002518751 |
| miR.558 + miR.609 + miR.520f + miR.220a + miR.369.3p + miR.518c | 0,77 | 0,030428094 |
| miR.609 + miR.603 + miR.220a + miR.26a + miR.376a* + miR.518c | 0,77 | 0,011199454 |
| miR.558 + miR.609 + miR.603 + miR.29a + miR.376a* + miR.338 | 0,77 | 0,002040468 |
| miR.609 + miR.130a + miR.29a + miR.376a* + miR.518c + miR.338 | 0,77 | 0,017779127 |
| miR.558 + miR.609 + miR.520f + miR.376a* + miR.518c + miR.660 | 0,77 | 0,047455935 |
| miR.558 + miR.609 + miR.520f + miR.376a* + miR.494 + miR.518c | 0,77 | 0,07847662 |
| miR.609 + miR.603 + miR.520f + miR.26a + miR.376a* + miR.518c | 0,77 | 0,00854435 |
| miR.609 + miR.603 + miR.220a + miR.519b + miR.29a + miR.518c | 0,77 | 0,010617572 |
| miR.558 + miR.609 + miR.520f + miR.362 + miR.376a* + miR.518c | 0,77 | 0,053788243 |
| miR.609 + miR.603 + miR.220a + miR.376a* + miR.518c + miR.338 | 0,77 | 0,013059379 |
| miR.609 + miR.603 + miR.520f + miR.26a + miR.29a + miR.518c | 0,77 | 0,016119849 |
| miR.558 + miR.609 + miR.603 + miR.362 + miR.369.3p + miR.518c | 0,77 | 0,015781877 |
| miR.609 + miR.603 + miR.29a + miR.362 + miR.369.3p + miR.376a* | 0,77 | 0,079825069 |
| miR.558 + miR.609 + miR.519b + miR.29a + miR.369.3p + miR.518c | 0,77 | 0,003692251 |
| miR.609 + miR.603 + miR.369.3p + miR.376a* + miR.518c + miR.338 | 0,77 | 0,014175499 |
| miR.558 + miR.609 + miR.520f + miR.519b + miR.26a + miR.518c | 0,77 | 0,022487851 |
| miR.609 + miR.520f + miR.220a + miR.29a + miR.376a* + miR.660 | 0,77 | 0,093807333 |
| miR.558 + miR.609 + miR.26a + miR.369.3p + miR.376a* + miR.518c | 0,77 | 0,098169323 |
| miR.609 + miR.220a + miR.130a + miR.376a* + miR.518c + miR.338 | 0,77 | 0,007775701 |
| miR.609 + miR.520f + miR.369.3p + miR.376a* + miR.518c + miR.639 | 0,77 | 0,075449122 |
| miR.558 + miR.603 + miR.520f + miR.29a + miR.376a* + miR.518c | 0,77 | 0,01426733 |
| miR.558 + miR.609 + miR.29a + miR.369.3p + miR.518c + miR.639 | 0,77 | 0,002316902 |
| miR.609 + miR.603 + miR.520f + miR.518c + miR.639 + miR.660 | 0,77 | 0,037719953 |
| miR.558 + miR.609 + miR.603 + miR.130a + miR.518c + miR.660 | 0,77 | 0,024449524 |
| miR.558 + miR.609 + miR.29a + miR.369.3p + miR.494 + miR.518c | 0,77 | 0,0018993 |
| miR.558 + miR.609 + miR.603 + miR.369.3p + miR.518c + miR.338 | 0,77 | 0,021836721 |
| miR.558 + miR.609 + miR.29a + miR.362 + miR.369.3p + miR.518c | 0,77 | 0,002372279 |
| miR.558 + miR.609 + miR.520f + miR.519b + miR.376a* + miR.518c | 0,77 | 0,051807111 |
| miR.609 + miR.520f + miR.376a* + miR.494 + miR.518c + miR.639 | 0,77 | 0,043382601 |
| miR.609 + miR.603 + miR.220a + miR.376a* + miR.518c + miR.660 | 0,77 | 0,010569123 |
| miR.558 + miR.609 + miR.519b + miR.369.3p + miR.376a* + miR.518c | 0,77 | 0,12723108 |
| miR.558 + miR.609 + miR.520f + miR.29a + miR.494 + miR.660 | 0,77 | 0,052610624 |
| miR.558 + miR.609 + miR.220a + miR.518c + miR.639 + miR.660 | 0,77 | 0,023773521 |
| miR.558 + miR.603 + miR.220a + miR.519b + miR.518c + miR.338 | 0,77 | 0,04050716 |
| miR.609 + miR.603 + miR.220a + miR.362 + miR.376a* + miR.518c | 0,77 | 0,008313058 |
| miR.609 + miR.603 + miR.520f + miR.29a + miR.369.3p + miR.518c | 0,77 | 0,013175473 |
| miR.609 + miR.520f + miR.376a* + miR.518c + miR.639 + miR.338 | 0,77 | 0,060908541 |
| miR.558 + miR.609 + miR.130a + miR.29a + miR.362 + miR.376a* | 0,77 | 0,0053212 |
| miR.609 + miR.603 + miR.520f + miR.130a + miR.29a + miR.518c | 0,77 | 0,012872201 |
| miR.609 + miR.520f + miR.220a + miR.519b + miR.29a + miR.376a* | 0,77 | 0,078542643 |
| miR.558 + miR.609 + miR.603 + miR.520f + miR.29a + miR.660 | 0,77 | 0,047264387 |
| miR.609 + miR.603 + miR.520f + miR.29a + miR.494 + miR.518c | 0,77 | 0,017700054 |
| miR.609 + miR.520f + miR.220a + miR.519b + miR.29a + miR.518c | 0,77 | 0,007178292 |
| miR.558 + miR.609 + miR.603 + miR.369.3p + miR.518c + miR.660 | 0,77 | 0,030997545 |
| miR.609 + miR.130a + miR.29a + miR.376a* + miR.518c + miR.660 | 0,77 | 0,019251846 |
| miR.609 + miR.520f + miR.220a + miR.26a + miR.29a + miR.518c | 0,77 | 0,008605824 |
| miR.558 + miR.603 + miR.220a + miR.376a* + miR.518c + miR.660 | 0,77 | 0,029523797 |
| miR.609 + miR.603 + miR.520f + miR.29a + miR.362 + miR.376a* | 0,77 | 0,095268033 |
| miR.558 + miR.609 + miR.603 + miR.519b + miR.29a + miR.376a* | 0,77 | 0,001813366 |
| miR.558 + miR.609 + miR.520f + miR.220a + miR.362 + miR.518c | 0,77 | 0,019808849 |
| miR.558 + miR.609 + miR.369.3p + miR.376a* + miR.494 + miR.518c | 0,77 | 0,13727794 |
| miR.609 + miR.520f + miR.130a + miR.369.3p + miR.376a* + miR.338 | 0,77 | 0,136659491 |
| miR.558 + miR.609 + miR.603 + miR.519b + miR.362 + miR.518c | 0,77 | 0,020886818 |
| miR.558 + miR.609 + miR.29a + miR.362 + miR.376a* + miR.660 | 0,77 | 0,019395961 |
| miR.558 + miR.609 + miR.603 + miR.519b + miR.494 + miR.518c | 0,77 | 0,024375379 |
| miR.558 + miR.603 + miR.220a + miR.26a + miR.376a* + miR.518c | 0,77 | 0,038369815 |
| miR.609 + miR.520f + miR.220a + miR.29a + miR.518c + miR.639 | 0,77 | 0,004671713 |
| miR.558 + miR.603 + miR.220a + miR.376a* + miR.494 + miR.518c | 0,77 | 0,021836525 |
| miR.558 + miR.609 + miR.520f + miR.220a + miR.518c + miR.338 | 0,77 | 0,019232048 |
| miR.609 + miR.520f + miR.220a + miR.29a + miR.494 + miR.518c | 0,77 | 0,006824784 |
| miR.558 + miR.609 + miR.519b + miR.29a + miR.362 + miR.518c | 0,77 | 0,003863341 |
| miR.603 + miR.220a + miR.519b + miR.29a + miR.376a* + miR.518c | 0,77 | 0,039032708 |
| miR.558 + miR.603 + miR.220a + miR.376a* + miR.518c + miR.338 | 0,77 | 0,02199773 |
| miR.558 + miR.609 + miR.520f + miR.220a + miR.494 + miR.518c | 0,77 | 0,017215728 |
| miR.558 + miR.609 + miR.519b + miR.29a + miR.494 + miR.518c | 0,77 | 0,003871226 |
| miR.603 + miR.220a + miR.362 + miR.369.3p + miR.376a* + miR.518c | 0,77 | 0,066129138 |
| miR.558 + miR.609 + miR.520f + miR.220a + miR.26a + miR.518c | 0,77 | 0,018026819 |
| miR.609 + miR.520f + miR.29a + miR.362 + miR.518c + miR.639 | 0,77 | 0,008417738 |
| miR.558 + miR.609 + miR.29a + miR.362 + miR.494 + miR.518c | 0,77 | 0,001691111 |
| miR.558 + miR.609 + miR.29a + miR.362 + miR.518c + miR.639 | 0,77 | 0,002216628 |
| miR.558 + miR.609 + miR.603 + miR.519b + miR.518c + miR.338 | 0,77 | 0,018574053 |

**Table 7 clusters of 7 miRNAs**

| **Cluster of 7 miRs** | **c_tau level** | **pv compared to top signature miR.558 + miR.609** + **miR.220aa + miR.29a + miR.369.3p + miR.376a* + miR.518c** |
|---|---|---|
| miR.558 + miR.609 + miR.220aa + miR.29a + miR.369.3p + miR.376a* + miR.518c | 0,8 | NaN |
| miR.558 + miR.609 + miR.603 + miR.220aa + miR.29a + miR.376a* + miR.518c | 0,8 | 0,025002564 |
| miR.558 + miR.609 + miR.520f + miR.220aa + miR.29a + miR.376a* + miR.518c | 0,8 | 0,236514028 |
| miR.558 + miR.609 + miR.520f + miR.29a + miR.376a* + miR.518c + miR.660 | 0,8 | 0,234767626 |
| miR.558 + miR.609 + miR.520f + miR.29a + miR.369.3p + miR.376a* + miR.518c | 0,8 | 0,368604402 |
| miR.558 + miR.609 + miR.603 + miR.29a + miR.369.3p + miR.376a* + miR.518c | 0,8 | 4,66E-005 |
| miR.558 + miR.609 + miR.220aa + miR.26a + miR.29a + miR.376a* + miR.518c | 0,8 | 0,400443165 |
| miR.558 + miR.609 + miR.220aa + miR.29a + miR.362 + miR.376a* + miR.518c | 0,8 | 0,063808643 |
| miR.558 + miR.609 + miR.603 + miR.520f + miR.29a + miR.376a* + miR.518c | 0,8 | 0,061426174 |
| miR.558 + miR.609 + miR.220aa + miR.29a + miR.376a* + miR.494 + miR.518c | 0,8 | 0,009429917 |
| miR.558 + miR.609 + miR.520f + miR.29a + miR.376a* + miR.518c + miR.338 | 0,8 | 0,451228907 |
| miR.558 + miR.609 + miR.220aa + miR.29a + miR.376a* + miR.518c + miR.660 | 0,8 | 0,052632921 |
| miR.558 + miR.609 + miR.220aa + miR.130a + miR.29a + miR.376a* + miR.518c | 0,8 | 0,023756083 |
| miR.558 + miR.609 + miR.520f + miR.29a + miR.362 + miR.376a* + miR.518c | 0,8 | 0,362771469 |
| miR.558 + miR.609 + miR.520f + miR.29a + miR.376a* + miR.494 + miR.518c | 0,8 | 0,254988519 |
| miR.558 + miR.609 + miR.220aa + miR.519b + miR.29a + miR.376a* + miR.518c | 0,8 | 0,101848935 |
| miR.558 + miR.609 + miR.520f + miR.29a + miR.376a* + miR.518c + miR.639 | 0,8 | 0,173022543 |
| miR.558 + miR.609 + miR.220aa + miR.29a + miR.376a* + miR.518c + miR.338 | 0,8 | 0,022009342 |
| miR.558 + miR.609 + miR.220aa + miR.29a + miR.376a* + miR.518c + miR.639 | 0,8 | 0,014190264 |
| miR.558 + miR.609 + miR.26a + miR.29a + miR.369.3p + miR.376a* + miR.518c | 0,8 | 0,487971958 |
| miR.558 + miR.609 + miR.520f + miR.26a + miR.29a + miR.376a* + miR.518c | 0,8 | 0,263651262 |
| miR.558 + miR.609 + miR.520f + miR.130a + miR.29a + miR.376a* + miR.518c | 0,8 | 0,21029374 |
| miR.558 + miR.609 + miR.520f + miR.519b + miR.29a + miR.376a* + miR.518c | 0,8 | 0,262148524 |
| miR.609 + miR.603 + miR.220aa + miR.29a + miR.369.3p + miR.376a* + miR.518c | 0,8 | 0,287440685 |
| miR.558 + miR.609 + miR.603 + miR.26a + miR.29a + miR.376a* + miR.518c | 0,8 | 0,002876115 |
| miR.558 + miR.609 + miR.603 + miR.29a + miR.376a* + miR.518c + miR.639 | 0,8 | 0,001233164 |
| miR.558 + miR.609 + miR.26a + miR.29a + miR.376a* + miR.518c + miR.639 | 0,8 | 0,275744525 |
| miR.558 + miR.609 + miR.130a + miR.29a + miR.369.3p + miR.376a* + miR.518c | 0,8 | 0,255194458 |
| miR.558 + miR.609 + miR.29a + miR.369.3p + miR.376a* + miR.518c + miR.660 | 0,8 | 0,13651033 |
| miR.558 + miR.609 + miR.603 + miR.29a + miR.362 + miR.376a* + miR.518c | 0,8 | 0,000337044 |
| miR.558 + miR.609 + miR.603 + miR.29a + miR.376a* + miR.518c + miR.660 | 0,8 | 0,00089689 |
| miR.558 + miR.609 + miR.603 + miR.29a + miR.376a* + miR.518c + miR.338 | 0,8 | 0,000809225 |
| miR.558 + miR.609 + miR.603 + miR.519b + miR.29a + miR.376a* + miR.518c | 0,8 | 0,001161154 |
| miR.558 + miR.609 + miR.29a + miR.369.3p + miR.376a* + miR.518c + miR.639 | 0,8 | 0,171792923 |
| miR.558 + miR.609 + miR.603 + miR.130a + miR.29a + miR.376a* + miR.518c | 0,8 | 0,000360769 |
| miR.558 + miR.609 + miR.29a + miR.362 + miR.369.3p + miR.376a* + miR.518c | 0,8 | 0,24498342 |
| miR.558 + miR.609 + miR.29a + miR.369.3p + miR.376a* + miR.494 + miR.518c | 0,8 | 0,160522317 |
| miR.558 + miR.609 + miR.29a + miR.369.3p + miR.376a* + miR.518c + miR.338 | 0,8 | 0,3404612 |
| miR.558 + miR.609 + miR.603 + miR.29a + miR.376a* + miR.494 + miR.518c | 0,8 | 0,005187074 |
| miR.609 + miR.603 + miR.520f + miR.220aa + miR.29a + miR.376a* + miR.518c | 0,8 | 0,322541602 |
| miR.558 + miR.609 + miR.26a + miR.29a + miR.376a* + miR.494 + miR.518c | 0,8 | 0,156342193 |
| miR.558 + miR.609 + miR.519b + miR.29a + miR.369.3p + miR.376a* + miR.518c | 0,8 | 0,110020017 |
| miR.558 + miR.609 + miR.26a + miR.29a + miR.376a* + miR.518c + miR.338 | 0,8 | 0,261803986 |
| miR.609 + miR.603 + miR.220aa + miR.29a + miR.362 + miR.376a* + miR.518c | 0,8 | 0,244551383 |
| miR.609 + miR.603 + miR.220aa + miR.519b + miR.29a + miR.376a* + miR.518c | 0,8 | 0,198217239 |
| miR.609 + miR.603 + miR.220aa + miR.26a + miR.29a + miR.376a* + miR.518c | 0,8 | 0,155639038 |
| miR.609 + miR.603 + miR.520f + miR.29a + miR.369.3p + miR.376a* + miR.518c | 0,8 | 0,112953682 |
| miR.609 + miR.603 + miR.220aa + miR.29a + miR.376a* + miR.518c + miR.660 | 0,8 | 0,204298726 |
| miR.558 + miR.609 + miR.130a + miR.29a + miR.376a* + miR.494 + miR.518c | 0,8 | 0,150493528 |
| miR.558 + miR.609 + miR.130a + miR.29a + miR.376a* + miR.518c + miR.639 | 0,8 | 0,068254929 |
| miR.609 + miR.603 + miR.520f + miR.29a + miR.376a* + miR.494 + miR.518c | 0,8 | 0,094133593 |
| miR.558 + miR.609 + miR.26a + miR.29a + miR.376a* + miR.518c + miR.660 | 0,8 | 0,142031284 |
| miR.558 + miR.609 + miR.26a + miR.29a + miR.362 + miR.376a* + miR.518c | 0,8 | 0,210300462 |
| miR.558 + miR.609 + miR.519b + miR.26a + miR.29a + miR.376a* + miR.518c | 0,8 | 0,158773957 |
| miR.558 + miR.609 + miR.130a + miR.26a + miR.29a + miR.376a* + miR.518c | 0,8 | 0,141313551 |
| miR.558 + miR.609 + miR.29a + miR.376a* + miR.518c + miR.639 + miR.338 | 0,79 | 0,098937459 |
| miR.558 + miR.609 + miR.29a + miR.362 + miR.376a* + miR.518c + miR.639 | 0,79 | 0,114050461 |
| miR.609 + miR.520f + miR.220aa + miR.29a + miR.369.3p + miR.376a* + miR.518c | 0,79 | 0,386937705 |
| miR.609 + miR.603 + miR.220aa + miR.29a + miR.376a* + miR.494 + miR.518c | 0,79 | 0,095343307 |
| miR.609 + miR.520f + miR.220aa + miR.29a + miR.376a* + miR.494 + miR.518c | 0,79 | 0,34388918 |
| miR.609 + miR.603 + miR.220aa + miR.29a + miR.376a* + miR.518c + miR.639 | 0,79 | 0,080340683 |
| miR.558 + miR.609 + miR.519b + miR.29a + miR.376a* + miR.518c + miR.639 | 0,79 | 0,062136982 |
| miR.558 + miR.609 + miR.29a + miR.376a* + miR.494 + miR.518c + miR.639 | 0,79 | 0,059422498 |
| miR.609 + miR.603 + miR.220aa + miR.130a + miR.29a + miR.376a* + miR.518c | 0,79 | 0,12155247 |
| miR.609 + miR.603 + miR.220aa + miR.29a + miR.376a* + miR.518c + miR.338 | 0,79 | 0,092825736 |
| miR.558 + miR.609 + miR.29a + miR.362 + miR.376a* + miR.518c + miR.338 | 0,79 | 0,042112106 |
| miR.558 + miR.609 + miR.130a + miR.29a + miR.362 + miR.376a* + miR.518c | 0,79 | 0,057370027 |
| miR.558 + miR.609 + miR.29a + miR.376a* + miR.518c + miR.660 + miR.338 | 0,79 | 0,09140135 |
| miR.558 + miR.609 + miR.29a + miR.376a* + miR.518c + miR.639 + miR.660 | 0,79 | 0,14662121 |
| miR.558 + miR.609 + miR.130a + miR.29a + miR.376a* + miR.518c + miR.338 | 0,79 | 0,028655704 |
| miR.609 + miR.603 + miR.520f + miR.29a + miR.376a* + miR.518c + miR.639 | 0,79 | 0,01819747 |
| miR.558 + miR.609 + miR.519b + miR.29a + miR.376a* + miR.518c + miR.660 | 0,79 | 0,040305976 |
| miR.558 + miR.609 + miR.29a + miR.362 + miR.376a* + miR.518c + miR.660 | 0,79 | 0,023853552 |
| miR.558 + miR.609 + miR.130a + miR.29a + miR.376a* + miR.518c + miR.660 | 0,79 | 0,070099167 |
| miR.558 + miR.609 + miR.519b + miR.29a + miR.376a* + miR.518c + miR.338 | 0,79 | 0,047274439 |
| miR.558 + miR.609 + miR.29a + miR.376a* + miR.494 + miR.518c + miR.338 | 0,79 | 0,074655187 |
| miR.558 + miR.609 + miR.519b + miR.29a + miR.362 + miR.376a* + miR.518c | 0,79 | 0,051958259 |
| miR.558 + miR.609 + miR.603 + miR.369.3p + miR.376a* + miR.518c + miR.639 | 0,79 | 0,129365113 |
| miR.558 + miR.609 + miR.519b + miR.130a + miR.29a + miR.376a* + miR.518c | 0,79 | 0,062867155 |
| miR.609 + miR.603 + miR.29a + miR.369.3p + miR.376a* + miR.494 + miR.518c | 0,79 | 0,024020148 |
| miR.558 + miR.609 + miR.29a + miR.362 + miR.376a* + miR.494 + miR.518c | 0,79 | 0,075519808 |
| miR.609 + miR.603 + miR.29a + miR.369.3p + miR.376a* + miR.518c + miR.639 | 0,79 | 0,018578868 |
| miR.609 + miR.603 + miR.520f + miR.130a + miR.29a + miR.376a* + miR.518c | 0,79 | 0,030963519 |
| miR.609 + miR.603 + miR.26a + miR.29a + miR.369.3p + miR.376a* + miR.518c | 0,79 | 0,019120213 |
| miR.558 + miR.609 + miR.519b + miR.29a + miR.376a* + miR.494 + miR.518c | 0,79 | 0,161499486 |
| miR.609 + miR.220aa + miR.29a + miR.369.3p + miR.376a* + miR.518c + miR.660 | 0,79 | 0,347879436 |
| miR.558 + miR.609 + miR.520f + miR.29a + miR.369.3p + miR.376a* + miR.338 | 0,79 | 0,192029995 |
| miR.558 + miR.609 + miR.29a + miR.376a* + miR.494 + miR.518c + miR.660 | 0,79 | 0,056021885 |
| miR.609 + miR.603 + miR.520f + miR.29a + miR.376a* + miR.518c + miR.338 | 0,79 | 0,382763568 |
| miR.609 + miR.603 + miR.29a + miR.362 + miR.369.3p + miR.376a* + miR.518c | 0,79 | 0,110998575 |
| miR.609 + miR.520f + miR.220aa + miR.29a + miR.376a* + miR.518c + miR.660 | 0,79 | 0,341995375 |
| miR.609 + miR.520f + miR.220aa + miR.29a + miR.362 + miR.376a* + miR.518c | 0,79 | 0,475144603 |
| miR.609 + miR.603 + miR.29a + miR.369.3p + miR.376a* + miR.518c + miR.660 | 0,79 | 0,005214757 |
| miR.609 + miR.603 + miR.519b + miR.29a + miR.369.3p + miR.376a* + miR.518c | 0,79 | 0,006677565 |
| miR.609 + miR.603 + miR.520f + miR.29a + miR.362 + miR.376a* + miR.518c | 0,79 | 0,078638928 |
| miR.609 + miR.520f + miR.220aa + miR.130a + miR.29a + miR.376a* + miR.518c | 0,79 | 0,279815891 |
| miR.558 + miR.609 + miR.520f + miR.29a + miR.376a* + miR.494 + miR.338 | 0,79 | 0,086095333 |
| miR.609 + miR.220aa + miR.519b + miR.29a + miR.369.3p + miR.376a* + miR.518c | 0,79 | 0,053108906 |
| miR.609 + miR.220aa + miR.26a + miR.29a + miR.369.3p + miR.376a* + miR.518c | 0,79 | 0,231379998 |
| miR.609 + miR.603 + miR.29a + miR.369.3p + miR.376a* + miR.518c + miR.338 | 0,79 | 0,095546627 |
| miR.609 + miR.603 + miR.520f + miR.26a + miR.29a + miR.376a* + miR.518c | 0,79 | 0,077713891 |
| miR.609 + miR.603 + miR.520f + miR.519b + miR.29a + miR.376a* + miR.518c | 0,79 | 0,054308627 |
| miR.609 + miR.520f + miR.29a + miR.376a* + miR.494 + miR.518c + miR.338 | 0,79 | 0,264931684 |
| miR.609 + miR.603 + miR.130a + miR.29a + miR.369.3p + miR.376a* + miR.518c | 0,79 | 0,004061965 |
| miR.558 + miR.609 + miR.603 + miR.520f + miR.376a* + miR.518c + miR.639 | 0,79 | 0,164459406 |
| miR.609 + miR.603 + miR.520f + miR.29a + miR.376a* + miR.518c + miR.660 | 0,79 | 0,04984183 |
| miR.609 + miR.520f + miR.29a + miR.369.3p + miR.376a* + miR.494 + miR.518c | 0,79 | 0,2712076 |
| miR.609 + miR.220aa + miR.29a + miR.369.3p + miR.376a* + miR.518c + miR.338 | 0,79 | 0,118569941 |
| miR.558 + miR.609 + miR.603 + miR.520f + miR.130a + miR.376a* + miR.518c | 0,79 | 0,03928753 |
| miR.609 + miR.220aa + miR.29a + miR.369.3p + miR.376a* + miR.494 + miR.518c | 0,79 | 0,441436661 |
| miR.609 + miR.603 + miR.29a + miR.376a* + miR.494 + miR.518c + miR.639 | 0,79 | 0,005325103 |
| miR.558 + miR.609 + miR.520f + miR.130a + miR.376a* + miR.518c + miR.338 | 0,79 | 0,208365173 |
| miR.609 + miR.520f + miR.220aa + miR.26a + miR.29a + miR.376a* + miR.518c | 0,79 | 0,336945692 |
| miR.609 + miR.520f + miR.29a + miR.376a* + miR.494 + miR.518c + miR.660 | 0,79 | 0,215220026 |
| miR.609 + miR.520f + miR.29a + miR.376a* + miR.494 + miR.518c + miR.639 | 0,79 | 0,168490783 |
| miR.558 + miR.609 + miR.220aa + miR.26a + miR.29a + miR.518c + miR.660 | 0,79 | 0,024360855 |
| miR.558 + miR.609 + miR.603 + miR.220aa + miR.376a* + miR.518c + miR.639 | 0,79 | 0,042927216 |
| miR.609 + miR.520f + miR.220aa + miR.519b + miR.29a + miR.376a* + miR.518c | 0,79 | 0,376416943 |
| miR.558 + miR.609 + miR.520f + miR.376a* + miR.518c + miR.639 + miR.338 | 0,79 | 0,36433758 |
| miR.558 + miR.609 + miR.520f + miR.29a + miR.362 + miR.518c + miR.660 | 0,79 | 0,069346328 |
| miR.609 + miR.220aa + miR.29a + miR.369.3p + miR.376a* + miR.518c + miR.639 | 0,79 | 0,105590463 |
| miR.609 + miR.520f + miR.519b + miR.29a + miR.369.3p + miR.376a* + miR.518c | 0,79 | 0,180385142 |
| miR.558 + miR.609 + miR.520f + miR.29a + miR.369.3p + miR.376a* + miR.494 | 0,79 | 0,078706196 |
| miR.609 + miR.520f + miR.220aa + miR.29a + miR.376a* + miR.518c + miR.338 | 0,79 | 0,437108605 |
| miR.558 + miR.609 + miR.520f + miR.29a + miR.518c + miR.639 + miR.660 | 0,79 | 0,081885245 |
| miR.609 + miR.220aa + miR.29a + miR.362 + miR.369.3p + miR.376a* + miR.518c | 0,79 | 0,151705294 |
| miR.558 + miR.609 + miR.520f + miR.130a + miR.376a* + miR.518c + miR.639 | 0,79 | 0,176315207 |
| miR.609 + miR.520f + miR.220aa + miR.29a + miR.376a* + miR.518c + miR.639 | 0,79 | 0,32112685 |
| miR.558 + miR.609 + miR.520f + miR.26a + miR.29a + miR.518c + miR.660 | 0,79 | 0,059516306 |
| miR.558 + miR.609 + miR.520f + miR.29a + miR.376a* + miR.660 + miR.338 | 0,79 | 0,255670703 |
| miR.558 + miR.609 + miR.520f + miR.130a + miR.29a + miR.376a* + miR.338 | 0,79 | 0,106841277 |
| miR.609 + miR.603 + miR.220aa + miR.130a + miR.369.3p + miR.376a* + miR.518c | 0,79 | 0,051594779 |
| miR.558 + miR.609 + miR.520f + miR.369.3p + miR.376a* + miR.518c + miR.639 | 0,79 | 0,302834571 |
| miR.558 + miR.609 + miR.520f + miR.29a + miR.376a* + miR.639 + miR.338 | 0,79 | 0,107168484 |
| miR.558 + miR.609 + miR.603 + miR.26a + miR.29a + miR.518c + miR.660 | 0,79 | 0,018705037 |
| miR.558 + miR.609 + miR.603 + miR.220aa + miR.130a + miR.376a* + miR.518c | 0,79 | 0,041655174 |
| miR.558 + miR.609 + miR.520f + miR.519b + miR.29a + miR.376a* + miR.338 | 0,79 | 0,146035682 |
| miR.558 + miR.609 + miR.603 + miR.520f + miR.29a + miR.376a* + miR.338 | 0,79 | 0,092270512 |
| miR.609 + miR.520f + miR.26a + miR.29a + miR.376a* + miR.494 + miR.518c | 0,79 | 0,151560335 |
| miR.558 + miR.609 + miR.520f + miR.26a + miR.29a + miR.376a* + miR.338 | 0,79 | 0,084840869 |
| miR.609 + miR.520f + miR.29a + miR.369.3p + miR.376a* + miR.518c + miR.338 | 0,79 | 0,442036117 |
| miR.609 + miR.220aa + miR.130a + miR.29a + miR.369.3p + miR.376a* + miR.518c | 0,79 | 0,13732403 |
| miR.558 + miR.609 + miR.520f + miR.220aa + miR.130a + miR.376a* + miR.518c | 0,79 | 0,050995659 |
| miR.558 + miR.609 + miR.520f + miR.220aa + miR.29a + miR.376a* + miR.338 | 0,79 | 0,081447451 |
| miR.609 + miR.520f + miR.29a + miR.362 + miR.376a* + miR.494 + miR.518c | 0,79 | 0,179772063 |
| miR.558 + miR.609 + miR.603 + miR.130a + miR.369.3p + miR.376a* + miR.518c | 0,79 | 0,025022285 |
| miR.609 + miR.520f + miR.519b + miR.29a + miR.376a* + miR.494 + miR.518c | 0,79 | 0,157354426 |
| miR.609 + miR.603 + miR.26a + miR.29a + miR.376a* + miR.494 + miR.518c | 0,79 | 0,019309474 |
| miR.558 + miR.609 + miR.520f + miR.519b + miR.29a + miR.518c + miR.660 | 0,79 | 0,052011918 |
| miR.558 + miR.609 + miR.520f + miR.130a + miR.29a + miR.518c + miR.660 | 0,79 | 0,062591102 |
| miR.609 + miR.220aa + miR.26a + miR.29a + miR.376a* + miR.494 + miR.518c | 0,79 | 0,056334367 |
| miR.558 + miR.609 + miR.520f + miR.220aa + miR.29a + miR.518c + miR.660 | 0,79 | 0,059394226 |
| miR.558 + miR.609 + miR.220aa + miR.369.3p + miR.376a* + miR.518c + miR.639 | 0,79 | 0,14915253 |
| miR.558 + miR.609 + miR.603 + miR.130a + miR.376a* + miR.518c + miR.639 | 0,79 | 0,036435405 |
| miR.558 + miR.609 + miR.603 + miR.520f + miR.29a + miR.518c + miR.660 | 0,79 | 0,046421266 |
| miR.609 + miR.520f + miR.130a + miR.29a + miR.376a* + miR.494 + miR.518c | 0,79 | 0,159193429 |
| miR.558 + miR.609 + miR.520f + miR.519b + miR.29a + miR.376a* + miR.494 | 0,79 | 0,108376704 |
| miR.609 + miR.520f + miR.29a + miR.369.3p + miR.376a* + miR.518c + miR.639 | 0,79 | 0,223681012 |
| miR.558 + miR.609 + miR.520f + miR.29a + miR.518c + miR.660 + miR.338 | 0,79 | 0,046748659 |
| miR.558 + miR.609 + miR.603 + miR.520f + miR.29a + miR.369.3p + miR.376a* | 0,79 | 0,0678819 |
| miR.558 + miR.609 + miR.520f + miR.29a + miR.376a* + miR.494 + miR.660 | 0,79 | 0,125273034 |
| miR.558 + miR.609 + miR.520f + miR.29a + miR.494 + miR.518c + miR.660 | 0,79 | 0,054337348 |
| miR.558 + miR.609 + miR.520f + miR.29a + miR.376a* + miR.494 + miR.639 | 0,79 | 0,063699307 |
| miR.558 + miR.609 + miR.603 + miR.220aa + miR.369.3p + miR.376a* + miR.518c | 0,79 | 0,033066672 |
| miR.558 + miR.609 + miR.520f + miR.29a + miR.369.3p + miR.518c + miR.660 | 0,79 | 0,057263944 |
| miR.609 + miR.603 + miR.519b + miR.29a + miR.376a* + miR.494 + miR.518c | 0,79 | 0,014393844 |
| miR.609 + miR.220aa + miR.29a + miR.376a* + miR.494 + miR.518c + miR.660 | 0,79 | 0,05588982 |
| miR.558 + miR.609 + miR.520f + miR.29a + miR.362 + miR.376a* + miR.338 | 0,79 | 0,152086774 |
| miR.609 + miR.520f + miR.29a + miR.369.3p + miR.376a* + miR.518c + miR.660 | 0,79 | 0,220871924 |
| miR.558 + miR.609 + miR.520f + miR.29a + miR.369.3p + miR.376a* + miR.639 | 0,79 | 0,092882264 |
| miR.558 + miR.609 + miR.603 + miR.520f + miR.29a + miR.376a* + miR.494 | 0,79 | 0,046125093 |
| miR.609 + miR.520f + miR.26a + miR.29a + miR.369.3p + miR.376a* + miR.518c | 0,79 | 0,183771477 |
| miR.558 + miR.609 + miR.520f + miR.26a + miR.29a + miR.376a* + miR.494 | 0,79 | 0,058610704 |
| miR.609 + miR.603 + miR.29a + miR.376a* + miR.494 + miR.518c + miR.338 | 0,79 | 0,03003348 |
| miR.609 + miR.603 + miR.220aa + miR.369.3p + miR.376a* + miR.518c + miR.639 | 0,79 | 0,03181683 |
| miR.558 + miR.609 + miR.520f + miR.130a + miR.29a + miR.369.3p + miR.376a* | 0,79 | 0,070136984 |
| miR.558 + miR.609 + miR.520f + miR.29a + miR.362 + miR.376a* + miR.494 | 0,79 | 0,066075059 |
| miR.609 + miR.520f + miR.130a + miR.29a + miR.369.3p + miR.376a* + miR.518c | 0,79 | 0,180389967 |
| miR.609 + miR.603 + miR.520f + miR.220aa + miR.130a + miR.376a* + miR.518c | 0,79 | 0,079715485 |
| miR.558 + miR.609 + miR.520f + miR.130a + miR.29a + miR.376a* + miR.494 | 0,79 | 0,057588097 |
| miR.609 + miR.603 + miR.130a + miR.29a + miR.376a* + miR.494 + miR.518c | 0,79 | 0,020795419 |
| miR.558 + miR.609 + miR.520f + miR.29a + miR.369.3p + miR.376a* + miR.660 | 0,79 | 0,104980423 |
| miR.609 + miR.520f + miR.519b + miR.29a + miR.376a* + miR.518c + miR.639 | 0,79 | 0,04536668 |
| miR.558 + miR.609 + miR.520f + miR.220aa + miR.29a + miR.376a* + miR.494 | 0,79 | 0,064514507 |
| miR.609 + miR.603 + miR.29a + miR.376a* + miR.494 + miR.518c + miR.660 | 0,79 | 0,018013249 |
| miR.558 + miR.609 + miR.520f + miR.376a* + miR.518c + miR.639 + miR.660 | 0,79 | 0,286575086 |
| miR.609 + miR.603 + miR.29a + miR.362 + miR.376a* + miR.494 + miR.518c | 0,79 | 0,016458536 |
| miR.609 + miR.520f + miR.29a + miR.376a* + miR.518c + miR.660 + miR.338 | 0,79 | 0,386922586 |
| miR.558 + miR.609 + miR.520f + miR.26a + miR.29a + miR.369.3p + miR.376a* | 0,79 | 0,061480526 |
| miR.609 + miR.603 + miR.26a + miR.29a + miR.376a* + miR.518c + miR.639 | 0,79 | 0,009034589 |
| miR.558 + miR.609 + miR.520f + miR.130a + miR.369.3p + miR.376a* + miR.518c | 0,79 | 0,057548194 |
| miR.609 + miR.520f + miR.519b + miR.130a + miR.29a + miR.376a* + miR.518c | 0,79 | 0,087144576 |
| miR.558 + miR.609 + miR.520f + miR.220aa + miR.29a + miR.369.3p + miR.376a* | 0,79 | 0,063034652 |
| miR.558 + miR.609 + miR.520f + miR.220aa + miR.376a* + miR.518c + miR.639 | 0,79 | 0,216787991 |
| miR.609 + miR.603 + miR.520f + miR.130a + miR.376a* + miR.518c + miR.639 | 0,79 | 0,033871147 |
| miR.609 + miR.520f + miR.26a + miR.29a + miR.376a* + miR.518c + miR.338 | 0,79 | 0,351173259 |
| miR.558 + miR.609 + miR.130a + miR.26a + miR.29a + miR.518c + miR.660 | 0,79 | 0,013327785 |
| miR.558 + miR.609 + miR.520f + miR.26a + miR.376a* + miR.518c + miR.639 | 0,79 | 0,130137736 |
| miR.558 + miR.609 + miR.520f + miR.29a + miR.362 + miR.369.3p + miR.376a* | 0,79 | 0,061385059 |
| miR.558 + miR.609 + miR.220aa + miR.130a + miR.369.3p + miR.376a* + miR.518c | 0,79 | 0,056436539 |
| miR.609 + miR.520f + miR.26a + miR.29a + miR.376a* + miR.518c + miR.660 | 0,79 | 0,146738814 |
| miR.558 + miR.609 + miR.520f + miR.519b + miR.29a + miR.369.3p + miR.376a* | 0,79 | 0,080909233 |
| miR.558 + miR.609 + miR.26a + miR.29a + miR.369.3p + miR.518c + miR.660 | 0,79 | 0,02075549 |
| miR.609 + miR.520f + miR.29a + miR.376a* + miR.518c + miR.639 + miR.338 | 0,79 | 0,216249971 |
| miR.558 + miR.609 + miR.520f + miR.29a + miR.376a* + miR.639 + miR.660 | 0,79 | 0,082945161 |
| miR.609 + miR.520f + miR.519b + miR.29a + miR.376a* + miR.518c + miR.338 | 0,79 | 0,324638105 |
| miR.558 + miR.609 + miR.26a + miR.29a + miR.518c + miR.639 + miR.660 | 0,79 | 0,044353924 |
| miR.609 + miR.220aa + miR.519b + miR.26a + miR.29a + miR.376a* + miR.518c | 0,79 | 0,062424972 |
| miR.609 + miR.603 + miR.130a + miR.26a + miR.29a + miR.376a* + miR.518c | 0,79 | 0,004990594 |
| miR.558 + miR.609 + miR.603 + miR.220aa + miR.519b + miR.376a* + miR.518c | 0,79 | 0,03147335 |
| miR.609 + miR.520f + miR.519b + miR.29a + miR.376a* + miR.518c + miR.660 | 0,79 | 0,119888614 |
| miR.609 + miR.603 + miR.130a + miR.29a + miR.376a* + miR.518c + miR.639 | 0,79 | 0,00067983 |
| miR.609 + miR.520f + miR.29a + miR.376a* + miR.518c + miR.639 + miR.660 | 0,79 | 0,134394809 |
| miR.558 + miR.609 + miR.26a + miR.29a + miR.494 + miR.518c + miR.660 | 0,79 | 0,014052264 |
| miR.558 + miR.609 + miR.26a + miR.29a + miR.518c + miR.660 + miR.338 | 0,79 | 0,010675371 |
| miR.609 + miR.603 + miR.29a + miR.376a* + miR.518c + miR.639 + miR.338 | 0,79 | 0,007014287 |
| miR.609 + miR.520f + miR.519b + miR.26a + miR.29a + miR.376a* + miR.518c | 0,79 | 0,099139978 |
| miR.609 + miR.603 + miR.520f + miR.130a + miR.376a* + miR.518c + miR.338 | 0,79 | 0,103316352 |
| miR.558 + miR.609 + miR.520f + miR.362 + miR.376a* + miR.518c + miR.639 | 0,79 | 0,232377675 |
| miR.609 + miR.520f + miR.29a + miR.362 + miR.376a* + miR.518c + miR.338 | 0,79 | 0,345706741 |
| miR.609 + miR.220aa + miR.519b + miR.29a + miR.362 + miR.376a* + miR.518c | 0,79 | 0,018034377 |
| miR.558 + miR.609 + miR.520f + miR.376a* + miR.494 + miR.518c + miR.639 | 0,79 | 0,285149788 |
| miR.558 + miR.609 + miR.520f + miR.519b + miR.376a* + miR.518c + miR.639 | 0,79 | 0,232964205 |
| miR.558 + miR.609 + miR.519b + miR.26a + miR.29a + miR.518c + miR.660 | 0,79 | 0,007457376 |
| miR.609 + miR.603 + miR.220aa + miR.130a + miR.376a* + miR.518c + miR.639 | 0,79 | 0,040593982 |
| miR.609 + miR.520f + miR.26a + miR.29a + miR.376a* + miR.518c + miR.639 | 0,79 | 0,07329894 |
| miR.558 + miR.609 + miR.520f + miR.130a + miR.26a + miR.376a* + miR.518c | 0,79 | 0,030507676 |
| miR.609 + miR.520f + miR.519b + miR.29a + miR.362 + miR.376a* + miR.518c | 0,79 | 0,165907826 |
| miR.609 + miR.520f + miR.130a + miR.29a + miR.376a* + miR.518c + miR.660 | 0,79 | 0,104030103 |
| miR.558 + miR.609 + miR.520f + miR.26a + miR.29a + miR.376a* + miR.660 | 0,79 | 0,072464296 |
| miR.609 + miR.603 + miR.519b + miR.29a + miR.376a* + miR.518c + miR.639 | 0,79 | 0,004981793 |
| miR.609 + miR.520f + miR.130a + miR.29a + miR.376a* + miR.518c + miR.639 | 0,79 | 0,120914979 |
| miR.558 + miR.609 + miR.603 + miR.26a + miR.376a* + miR.518c + miR.639 | 0,79 | 0,089934804 |
| miR.609 + miR.220aa + miR.26a + miR.29a + miR.362 + miR.376a* + miR.518c | 0,79 | 0,063933855 |
| miR.609 + miR.220aa + miR.29a + miR.362 + miR.376a* + miR.494 + miR.518c | 0,79 | 0,027539531 |
| miR.558 + miR.609 + miR.603 + miR.376a* + miR.518c + miR.639 + miR.338 | 0,79 | 0,054331874 |
| miR.609 + miR.520f + miR.130a + miR.29a + miR.376a* + miR.518c + miR.338 | 0,79 | 0,270368469 |
| miR.609 + miR.603 + miR.29a + miR.362 + miR.376a* + miR.518c + miR.639 | 0,79 | 0,00552345 |
| miR.609 + miR.520f + miR.130a + miR.26a + miR.29a + miR.376a* + miR.518c | 0,79 | 0,107840654 |
| miR.609 + miR.520f + miR.26a + miR.29a + miR.362 + miR.376a* + miR.518c | 0,79 | 0,17426981 |
| miR.609 + miR.220aa + miR.29a + miR.376a* + miR.494 + miR.518c + miR.338 | 0,79 | 0,016790109 |
| miR.558 + miR.609 + miR.603 + miR.362 + miR.376a* + miR.518c + miR.639 | 0,79 | 0,053886075 |
| miR.558 + miR.609 + miR.26a + miR.29a + miR.362 + miR.518c + miR.660 | 0,79 | 0,016111562 |
| miR.609 + miR.220aa + miR.519b + miR.29a + miR.376a* + miR.518c + miR.338 | 0,79 | 0,008169744 |
| miR.558 + miR.609 + miR.603 + miR.376a* + miR.518c + miR.639 + miR.660 | 0,79 | 0,061209527 |
| miR.609 + miR.220aa + miR.519b + miR.29a + miR.376a* + miR.494 + miR.518c | 0,79 | 0,024368321 |
| miR.609 + miR.520f + miR.29a + miR.362 + miR.369.3p + miR.376a* + miR.518c | 0,79 | 0,197937421 |
| miR.558 + miR.609 + miR.603 + miR.519b + miR.376a* + miR.518c + miR.639 | 0,79 | 0,059897897 |
| miR.609 + miR.220aa + miR.29a + miR.362 + miR.376a* + miR.518c + miR.338 | 0,79 | 0,012360346 |
| miR.609 + miR.220aa + miR.519b + miR.29a + miR.376a* + miR.518c + miR.660 | 0,79 | 0,012017444 |
| miR.558 + miR.609 + miR.603 + miR.520f + miR.29a + miR.376a* + miR.660 | 0,79 | 0,05638436 |
| miR.609 + miR.603 + miR.29a + miR.376a* + miR.518c + miR.639 + miR.660 | 0,79 | 0,006887135 |
| miR.558 + miR.609 + miR.603 + miR.376a* + miR.494 + miR.518c + miR.639 | 0,79 | 0,091458863 |
| miR.558 + miR.609 + miR.220aa + miR.130a + miR.376a* + miR.518c + miR.639 | 0,79 | 0,048328988 |
| miR.609 + miR.520f + miR.29a + miR.362 + miR.376a* + miR.518c + miR.639 | 0,79 | 0,155225133 |
| miR.609 + miR.220aa + miR.519b + miR.29a + miR.376a* + miR.518c + miR.639 | 0,79 | 0,011852535 |
| miR.609 + miR.520f + miR.29a + miR.362 + miR.376a* + miR.518c + miR.660 | 0,79 | 0,095588241 |
| miR.609 + miR.220aa + miR.130a + miR.29a + miR.376a* + miR.494 + miR.518c | 0,79 | 0,023573084 |
| miR.558 + miR.609 + miR.520f + miR.220aa + miR.29a + miR.376a* + miR.660 | 0,79 | 0,061842619 |
| miR.609 + miR.520f + miR.130a + miR.29a + miR.362 + miR.376a* + miR.518c | 0,79 | 0,130868338 |
| miR.558 + miR.609 + miR.520f + miR.130a + miR.376a* + miR.494 + miR.518c | 0,79 | 0,031074593 |
| miR.609 + miR.603 + miR.26a + miR.29a + miR.376a* + miR.518c + miR.338 | 0,79 | 0,008422824 |
| miR.609 + miR.220aa + miR.29a + miR.376a* + miR.494 + miR.518c + miR.639 | 0,79 | 0,028170458 |
| miR.558 + miR.609 + miR.603 + miR.520f + miR.29a + miR.376a* + miR.639 | 0,79 | 0,037375968 |
| miR.558 + miR.609 + miR.520f + miR.29a + miR.362 + miR.376a* + miR.660 | 0,79 | 0,06263938 |
| miR.558 + miR.609 + miR.603 + miR.520f + miR.519b + miR.29a + miR.376a* | 0,79 | 0,034434776 |
| miR.609 + miR.603 + miR.220aa + miR.130a + miR.26a + miR.376a* + miR.518c | 0,79 | 0,036223839 |
| miR.558 + miR.609 + miR.520f + miR.26a + miR.29a + miR.376a* + miR.639 | 0,79 | 0,043744427 |
| miR.609 + miR.603 + miR.519b + miR.26a + miR.29a + miR.376a* + miR.518c | 0,79 | 0,001746115 |
| miR.609 + miR.603 + miR.26a + miR.29a + miR.362 + miR.376a* + miR.518c | 0,79 | 0,002496086 |
| miR.558 + miR.609 + miR.603 + miR.220aa + miR.29a + miR.518c + miR.660 | 0,79 | 0,008586363 |
| miR.609 + miR.603 + miR.220aa + miR.519b + miR.130a + miR.376a* + miR.518c | 0,79 | 0,036722839 |
| miR.558 + miR.609 + miR.520f + miR.130a + miR.362 + miR.376a* + miR.518c | 0,79 | 0,031754317 |
| miR.558 + miR.609 + miR.520f + miR.519b + miR.29a + miR.376a* + miR.660 | 0,79 | 0,055797438 |
| miR.609 + miR.603 + miR.520f + miR.130a + miR.369.3p + miR.376a* + miR.518c | 0,79 | 0,013538666 |
| miR.558 + miR.609 + miR.603 + miR.520f + miR.130a + miR.29a + miR.376a* | 0,79 | 0,035530556 |
| miR.558 + miR.609 + miR.369.3p + miR.376a* + miR.518c + miR.639 + miR.338 | 0,79 | 0,135959601 |
| miR.609 + miR.603 + miR.130a + miR.369.3p + miR.376a* + miR.518c + miR.639 | 0,79 | 0,010841931 |
| miR.609 + miR.603 + miR.130a + miR.29a + miR.376a* + miR.518c + miR.338 | 0,79 | 0,004726664 |
| miR.609 + miR.603 + miR.26a + miR.29a + miR.376a* + miR.518c + miR.660 | 0,79 | 0,001776638 |
| miR.558 + miR.609 + miR.220aa + miR.26a + miR.29a + miR.362 + miR.518c | 0,79 | 0,006241375 |
| miR.558 + miR.609 + miR.520f + miR.130a + miR.376a* + miR.518c + miR.660 | 0,79 | 0,037090034 |
| miR.609 + miR.220aa + miR.29a + miR.362 + miR.376a* + miR.518c + miR.639 | 0,79 | 0,031439295 |
| miR.609 + miR.220aa + miR.130a + miR.26a + miR.29a + miR.376a* + miR.518c | 0,79 | 0,055177943 |
| miR.609 + miR.220aa + miR.26a + miR.29a + miR.376a* + miR.518c + miR.338 | 0,79 | 0,046300412 |
| miR.609 + miR.220aa + miR.519b + miR.130a + miR.29a + miR.376a* + miR.518c | 0,79 | 0,007592753 |
| miR.558 + miR.609 + miR.520f + miR.130a + miR.29a + miR.376a* + miR.639 | 0,79 | 0,04211244 |
| miR.558 + miR.609 + miR.220aa + miR.130a + miR.29a + miR.518c + miR.660 | 0,79 | 0,001843614 |
| miR.558 + miR.609 + miR.520f + miR.29a + miR.362 + miR.376a* + miR.639 | 0,79 | 0,053291837 |
| miR.609 + miR.603 + miR.130a + miR.29a + miR.362 + miR.376a* + miR.518c | 0,79 | 0,000162055 |
| miR.609 + miR.603 + miR.519b + miR.29a + miR.362 + miR.376a* + miR.518c | 0,79 | 0,000823729 |
| miR.609 + miR.603 + miR.29a + miR.362 + miR.376a* + miR.518c + miR.338 | 0,79 | 0,003948422 |
| miR.609 + miR.603 + miR.130a + miR.29a + miR.376a* + miR.518c + miR.660 | 0,79 | 8,98E-005 |
| miR.609 + miR.603 + miR.29a + miR.362 + miR.376a* + miR.518c + miR.660 | 0,79 | 0,001086233 |
| miR.609 + miR.220aa + miR.26a + miR.29a + miR.376a* + miR.518c + miR.639 | 0,79 | 0,062331349 |
| miR.558 + miR.609 + miR.520f + miR.519b + miR.130a + miR.376a* + miR.518c | 0,79 | 0,041258478 |
| miR.558 + miR.609 + miR.603 + miR.220aa + miR.26a + miR.29a + miR.518c | 0,79 | 0,030884002 |
| miR.609 + miR.220aa + miR.130a + miR.29a + miR.376a* + miR.518c + miR.660 | 0,79 | 0,030204084 |
| miR.558 + miR.609 + miR.520f + miR.220aa + miR.29a + miR.376a* + miR.639 | 0,79 | 0,03510479 |
| miR.609 + miR.220aa + miR.26a + miR.29a + miR.376a* + miR.518c + miR.660 | 0,79 | 0,034474487 |
| miR.609 + miR.603 + miR.519b + miR.130a + miR.29a + miR.376a* + miR.518c | 0,79 | 0,000183613 |
| miR.609 + miR.603 + miR.29a + miR.376a* + miR.518c + miR.660 + miR.338 | 0,79 | 0,002734206 |
| miR.609 + miR.603 + miR.519b + miR.29a + miR.376a* + miR.518c + miR.660 | 0,79 | 0,001358362 |
| miR.558 + miR.609 + miR.603 + miR.26a + miR.29a + miR.369.3p + miR.518c | 0,79 | 0,01577491 |
| miR.609 + miR.220aa + miR.29a + miR.376a* + miR.518c + miR.660 + miR.338 | 0,79 | 0,005252008 |
| miR.558 + miR.609 + miR.520f + miR.130a + miR.26a + miR.29a + miR.376a* | 0,79 | 0,034781279 |
| miR.609 + miR.220aa + miR.29a + miR.376a* + miR.518c + miR.639 + miR.338 | 0,79 | 0,034145139 |
| miR.609 + miR.603 + miR.519b + miR.29a + miR.376a* + miR.518c + miR.338 | 0,79 | 0,002255017 |
| miR.558 + miR.609 + miR.520f + miR.519b + miR.29a + miR.362 + miR.376a* | 0,79 | 0,047064737 |
| miR.558 + miR.609 + miR.520f + miR.519b + miR.29a + miR.376a* + miR.639 | 0,79 | 0,035807403 |
| miR.558 + miR.609 + miR.362 + miR.369.3p + miR.376a* + miR.518c + miR.639 | 0,79 | 0,147161344 |
| miR.558 + miR.609 + miR.520f + miR.130a + miR.29a + miR.376a* + miR.660 | 0,79 | 0,058152187 |
| miR.558 + miR.609 + miR.130a + miR.369.3p + miR.376a* + miR.518c + miR.639 | 0,79 | 0,128514837 |
| miR.558 + miR.609 + miR.603 + miR.520f + miR.26a + miR.29a + miR.376a* | 0,79 | 0,028503537 |
| miR.558 + miR.609 + miR.603 + miR.130a + miR.26a + miR.376a* + miR.518c | 0,79 | 0,027580709 |
| miR.558 + miR.609 + miR.603 + miR.26a + miR.29a + miR.518c + miR.338 | 0,79 | 0,02047927 |
| miR.558 + miR.609 + miR.603 + miR.130a + miR.376a* + miR.518c + miR.338 | 0,79 | 0,032979385 |
| miR.609 + miR.220aa + miR.29a + miR.376a* + miR.518c + miR.639 + miR.660 | 0,79 | 0,016361163 |
| miR.558 + miR.609 + miR.220aa + miR.130a + miR.26a + miR.376a* + miR.518c | 0,79 | 0,036616618 |
| miR.558 + miR.609 + miR.26a + miR.369.3p + miR.376a* + miR.518c + miR.639 | 0,79 | 0,144178297 |
| miR.558 + miR.609 + miR.520f + miR.130a + miR.29a + miR.362 + miR.376a* | 0,79 | 0,0455553 |
| miR.558 + miR.609 + miR.520f + miR.220aa + miR.130a + miR.29a + miR.376a* | 0,79 | 0,031594159 |
| miR.609 + miR.519b + miR.26a + miR.29a + miR.369.3p + miR.376a* + miR.518c | 0,79 | 0,056505964 |
| miR.609 + miR.220aa + miR.130a + miR.29a + miR.362 + miR.376a* + miR.518c | 0,79 | 0,032180945 |
| miR.609 + miR.220aa + miR.29a + miR.362 + miR.376a* + miR.518c + miR.660 | 0,79 | 0,01185485 |
| miR.558 + miR.609 + miR.220aa + miR.29a + miR.518c + miR.660 + miR.338 | 0,79 | 0,002784308 |
| miR.558 + miR.609 + miR.520f + miR.519b + miR.130a + miR.29a + miR.376a* | 0,79 | 0,035266528 |
| miR.558 + miR.609 + miR.520f + miR.26a + miR.29a + miR.362 + miR.376a* | 0,79 | 0,041576634 |
| miR.558 + miR.609 + miR.603 + miR.520f + miR.29a + miR.362 + miR.376a* | 0,79 | 0,042506952 |
| miR.558 + miR.609 + miR.603 + miR.520f + miR.26a + miR.29a + miR.518c | 0,79 | 0,019690085 |
| miR.558 + miR.609 + miR.603 + miR.130a + miR.362 + miR.376a* + miR.518c | 0,79 | 0,009913306 |
| miR.609 + miR.603 + miR.520f + miR.369.3p + miR.376a* + miR.518c + miR.639 | 0,79 | 0,020460122 |
| miR.609 + miR.603 + miR.220aa + miR.130a + miR.376a* + miR.518c + miR.338 | 0,79 | 0,10654041 |
| miR.558 + miR.609 + miR.603 + miR.520f + miR.220aa + miR.29a + miR.376a* | 0,79 | 0,033882422 |
| miR.558 + miR.609 + miR.520f + miR.220aa + miR.26a + miR.29a + miR.376a* | 0,79 | 0,027325355 |
| miR.558 + miR.609 + miR.220aa + miR.29a + miR.362 + miR.518c + miR.660 | 0,79 | 0,003900385 |
| miR.609 + miR.220aa + miR.130a + miR.29a + miR.376a* + miR.518c + miR.338 | 0,79 | 0,012269227 |
| miR.558 + miR.609 + miR.520f + miR.220aa + miR.519b + miR.29a + miR.376a* | 0,79 | 0,032081583 |
| miR.558 + miR.609 + miR.520f + miR.130a + miR.369.3p + miR.376a* + miR.338 | 0,79 | 0,295223702 |
| miR.558 + miR.609 + miR.220aa + miR.26a + miR.29a + miR.518c + miR.338 | 0,79 | 0,012301699 |
| miR.609 + miR.603 + miR.220aa + miR.130a + miR.376a* + miR.494 + miR.518c | 0,79 | 0,046543256 |
| miR.609 + miR.603 + miR.220aa + miR.130a + miR.376a* + miR.518c + miR.660 | 0,79 | 0,075257701 |
| miR.558 + miR.609 + miR.603 + miR.130a + miR.376a* + miR.518c + miR.660 | 0,79 | 0,012535871 |
| miR.558 + miR.609 + miR.520f + miR.519b + miR.26a + miR.29a + miR.376a* | 0,79 | 0,032383598 |
| miR.609 + miR.603 + miR.220aa + miR.130a + miR.362 + miR.376a* + miR.518c | 0,79 | 0,053512435 |
| miR.609 + miR.519b + miR.29a + miR.369.3p + miR.376a* + miR.518c + miR.639 | 0,79 | 0,047045854 |
| miR.558 + miR.609 + miR.603 + miR.26a + miR.29a + miR.362 + miR.518c | 0,79 | 0,01624326 |
| miR.558 + miR.609 + miR.520f + miR.220aa + miR.29a + miR.362 + miR.376a* | 0,79 | 0,04227083 |
| miR.558 + miR.609 + miR.29a + miR.362 + miR.369.3p + miR.376a* + miR.660 | 0,78 | 0,170577351 |
| miR.558 + miR.609 + miR.603 + miR.26a + miR.29a + miR.518c + miR.639 | 0,78 | 0,017268238 |
| miR.558 + miR.609 + miR.603 + miR.519b + miR.26a + miR.29a + miR.518c | 0,78 | 0,02295382 |
| miR.609 + miR.220aa + miR.130a + miR.29a + miR.376a* + miR.518c + miR.639 | 0,78 | 0,045718265 |
| miR.558 + miR.609 + miR.603 + miR.519b + miR.130a + miR.376a* + miR.518c | 0,78 | 0,018681653 |
| miR.609 + miR.603 + miR.520f + miR.376a* + miR.518c + miR.639 + miR.338 | 0,78 | 0,079513918 |
| miR.558 + miR.609 + miR.220aa + miR.130a + miR.376a* + miR.518c + miR.338 | 0,78 | 0,04606652 |
| miR.558 + miR.609 + miR.603 + miR.362 + miR.369.3p + miR.376a* + miR.518c | 0,78 | 0,02760726 |
| miR.609 + miR.520f + miR.29a + miR.369.3p + miR.376a* + miR.494 + miR.338 | 0,78 | 0,108065437 |
| miR.609 + miR.519b + miR.29a + miR.362 + miR.369.3p + miR.376a* + miR.518c | 0,78 | 0,151827667 |
| miR.558 + miR.609 + miR.603 + miR.130a + miR.376a* + miR.494 + miR.518c | 0,78 | 0,017785064 |
| miR.558 + miR.609 + miR.519b + miR.369.3p + miR.376a* + miR.518c + miR.639 | 0,78 | 0,142879297 |
| miR.609 + miR.603 + miR.520f + miR.220aa + miR.376a* + miR.518c + miR.639 | 0,78 | 0,032965168 |
| miR.558 + miR.609 + miR.603 + miR.130a + miR.26a + miR.29a + miR.518c | 0,78 | 0,011673792 |
| miR.558 + miR.609 + miR.220aa + miR.130a + miR.376a* + miR.494 + miR.518c | 0,78 | 0,018551299 |
| miR.558 + miR.609 + miR.220aa + miR.130a + miR.362 + miR.376a* + miR.518c | 0,78 | 0,016995329 |
| miR.558 + miR.609 + miR.369.3p + miR.376a* + miR.518c + miR.639 + miR.660 | 0,78 | 0,128633443 |
| miR.558 + miR.609 + miR.603 + miR.26a + miR.29a + miR.494 + miR.518c | 0,78 | 0,017647124 |
| miR.558 + miR.609 + miR.603 + miR.369.3p + miR.376a* + miR.518c + miR.338 | 0,78 | 0,055527369 |
| miR.609 + miR.603 + miR.520f + miR.29a + miR.376a* + miR.494 + miR.338 | 0,78 | 0,110738092 |
| miR.609 + miR.29a + miR.362 + miR.369.3p + miR.376a* + miR.518c + miR.660 | 0,78 | 0,289420411 |
| miR.558 + miR.609 + miR.520f + miR.26a + miR.29a + miR.362 + miR.518c | 0,78 | 0,014683101 |
| miR.558 + miR.609 + miR.220aa + miR.519b + miR.130a + miR.376a* + miR.518c | 0,78 | 0,035167937 |
| miR.558 + miR.609 + miR.603 + miR.130a + miR.29a + miR.518c + miR.660 | 0,78 | 0,004039961 |
| miR.558 + miR.609 + miR.130a + miR.29a + miR.518c + miR.639 + miR.660 | 0,78 | 0,006978861 |
| miR.558 + miR.609 + miR.220aa + miR.130a + miR.376a* + miR.518c + miR.660 | 0,78 | 0,030526538 |
| miR.558 + miR.609 + miR.603 + miR.520f + miR.518c + miR.639 + miR.660 | 0,78 | 0,098028023 |
| miR.558 + miR.609 + miR.603 + miR.220aa + miR.376a* + miR.518c + miR.338 | 0,78 | 0,017517995 |
| miR.558 + miR.609 + miR.520f + miR.130a + miR.376a* + miR.639 + miR.338 | 0,78 | 0,232178322 |
| miR.609 + miR.519b + miR.29a + miR.369.3p + miR.376a* + miR.494 + miR.518c | 0,78 | 0,07723018 |
| miR.609 + miR.520f + miR.220aa + miR.130a + miR.369.3p + miR.376a* + miR.518c | 0,78 | 0,023212457 |
| miR.558 + miR.609 + miR.220aa + miR.130a + miR.26a + miR.29a + miR.518c | 0,78 | 0,007519246 |
| miR.558 + miR.609 + miR.520f + miR.26a + miR.29a + miR.518c + miR.338 | 0,78 | 0,014007881 |
| miR.609 + miR.603 + miR.362 + miR.369.3p + miR.376a* + miR.518c + miR.639 | 0,78 | 0,018224599 |
| miR.558 + miR.609 + miR.220aa + miR.26a + miR.29a + miR.369.3p + miR.518c | 0,78 | 0,010640788 |
| miR.609 + miR.603 + miR.520f + miR.26a + miR.376a* + miR.518c + miR.639 | 0,78 | 0,040581193 |
| miR.609 + miR.603 + miR.369.3p + miR.376a* + miR.518c + miR.639 + miR.338 | 0,78 | 0,014921049 |
| miR.558 + miR.609 + miR.220aa + miR.376a* + miR.518c + miR.639 + miR.338 | 0,78 | 0,078540457 |
| miR.558 + miR.609 + miR.26a + miR.29a + miR.362 + miR.518c + miR.338 | 0,78 | 0,017131629 |
| miR.558 + miR.609 + miR.130a + miR.29a + miR.362 + miR.518c + miR.660 | 0,78 | 0,000338646 |
| miR.558 + miR.609 + miR.520f + miR.220aa + miR.26a + miR.29a + miR.518c | 0,78 | 0,011161417 |
| miR.558 + miR.609 + miR.603 + miR.520f + miR.220aa + miR.376a* + miR.518c | 0,78 | 0,026649299 |
| miR.558 + miR.609 + miR.603 + miR.220aa + miR.362 + miR.376a* + miR.518c | 0,78 | 0,013961948 |
| miR.558 + miR.609 + miR.603 + miR.520f + miR.130a + miR.376a* + miR.338 | 0,78 | 0,246690869 |
| miR.558 + miR.609 + miR.603 + miR.220aa + miR.376a* + miR.518c + miR.660 | 0,78 | 0,013150272 |
| miR.558 + miR.609 + miR.220aa + miR.376a* + miR.494 + miR.518c + miR.639 | 0,78 | 0,092408184 |
| miR.558 + miR.609 + miR.220aa + miR.519b + miR.26a + miR.29a + miR.518c | 0,78 | 0,013614183 |
| miR.558 + miR.609 + miR.603 + miR.220aa + miR.26a + miR.376a* + miR.518c | 0,78 | 0,01128397 |
| miR.609 + miR.603 + miR.520f + miR.130a + miR.26a + miR.376a* + miR.518c | 0,78 | 0,010920085 |
| miR.558 + miR.609 + miR.130a + miR.29a + miR.518c + miR.660 + miR.338 | 0,78 | 0,000125877 |
| miR.558 + miR.609 + miR.520f + miR.26a + miR.29a + miR.518c + miR.639 | 0,78 | 0,018969429 |
| miR.558 + miR.609 + miR.520f + miR.130a + miR.26a + miR.29a + miR.518c | 0,78 | 0,011746875 |
| miR.558 + miR.609 + miR.130a + miR.362 + miR.369.3p + miR.376a* + miR.518c | 0,78 | 0,037309484 |
| miR.558 + miR.609 + miR.220aa + miR.26a + miR.29a + miR.494 + miR.518c | 0,78 | 0,007507676 |
| miR.558 + miR.609 + miR.520f + miR.26a + miR.29a + miR.369.3p + miR.518c | 0,78 | 0,014342151 |
| miR.558 + miR.609 + miR.603 + miR.220aa + miR.376a* + miR.494 + miR.518c | 0,78 | 0,017097182 |
| miR.609 + miR.603 + miR.220aa + miR.376a* + miR.494 + miR.518c + miR.639 | 0,78 | 0,03325381 |
| miR.609 + miR.603 + miR.130a + miR.362 + miR.369.3p + miR.376a* + miR.518c | 0,78 | 0,009392167 |
| miR.558 + miR.609 + miR.29a + miR.362 + miR.369.3p + miR.376a* + miR.494 | 0,78 | 0,048829978 |
| miR.558 + miR.609 + miR.29a + miR.362 + miR.369.3p + miR.518c + miR.660 | 0,78 | 0,004400146 |
| miR.558 + miR.609 + miR.369.3p + miR.376a* + miR.494 + miR.518c + miR.639 | 0,78 | 0,163352928 |
| miR.558 + miR.609 + miR.220aa + miR.376a* + miR.518c + miR.639 + miR.660 | 0,78 | 0,103008562 |
| miR.558 + miR.609 + miR.220aa + miR.26a + miR.29a + miR.518c + miR.639 | 0,78 | 0,006804615 |
| miR.558 + miR.609 + miR.603 + miR.29a + miR.518c + miR.660 + miR.338 | 0,78 | 0,00223336 |
| miR.558 + miR.609 + miR.520f + miR.26a + miR.29a + miR.494 + miR.518c | 0,78 | 0,010475226 |
| miR.558 + miR.609 + miR.603 + miR.519b + miR.369.3p + miR.376a* + miR.518c | 0,78 | 0,035598786 |
| miR.558 + miR.609 + miR.220aa + miR.26a + miR.376a* + miR.518c + miR.639 | 0,78 | 0,083481811 |
| miR.558 + miR.609 + miR.26a + miR.29a + miR.369.3p + miR.518c + miR.338 | 0,78 | 0,014576318 |
| miR.558 + miR.609 + miR.603 + miR.520f + miR.369.3p + miR.376a* + miR.518c | 0,78 | 0,03197051 |
| miR.558 + miR.609 + miR.130a + miR.26a + miR.29a + miR.362 + miR.518c | 0,78 | 0,010372529 |
| miR.609 + miR.520f + miR.220aa + miR.130a + miR.376a* + miR.518c + miR.639 | 0,78 | 0,02410423 |
| miR.609 + miR.520f + miR.26a + miR.29a + miR.376a* + miR.494 + miR.338 | 0,78 | 0,056627184 |
| miR.558 + miR.609 + miR.603 + miR.520f + miR.519b + miR.29a + miR.518c | 0,78 | 0,048193832 |
| miR.558 + miR.609 + miR.603 + miR.29a + miR.362 + miR.518c + miR.660 | 0,78 | 0,004829453 |
| miR.609 + miR.603 + miR.520f + miR.519b + miR.130a + miR.376a* + miR.518c | 0,78 | 0,006763588 |
| miR.609 + miR.603 + miR.520f + miR.130a + miR.362 + miR.376a* + miR.518c | 0,78 | 0,005724349 |
| miR.558 + miR.609 + miR.220aa + miR.362 + miR.376a* + miR.518c + miR.639 | 0,78 | 0,065406188 |
| miR.609 + miR.603 + miR.520f + miR.130a + miR.376a* + miR.494 + miR.518c | 0,78 | 0,006763588 |
| miR.609 + miR.603 + miR.369.3p + miR.376a* + miR.494 + miR.518c + miR.639 | 0,78 | 0,020815213 |
| miR.609 + miR.603 + miR.520f + miR.130a + miR.376a* + miR.518c + miR.660 | 0,78 | 0,00649936 |
| miR.558 + miR.609 + miR.130a + miR.26a + miR.376a* + miR.518c + miR.639 | 0,78 | 0,115075349 |
| miR.609 + miR.603 + miR.130a + miR.369.3p + miR.376a* + miR.518c + miR.338 | 0,78 | 0,008293336 |
| miR.558 + miR.609 + miR.220aa + miR.519b + miR.376a* + miR.518c + miR.639 | 0,78 | 0,066085896 |
| miR.609 + miR.519b + miR.130a + miR.29a + miR.369.3p + miR.376a* + miR.518c | 0,78 | 0,033545182 |
| miR.609 + miR.603 + miR.520f + miR.376a* + miR.494 + miR.518c + miR.639 | 0,78 | 0,043081915 |
| miR.609 + miR.520f + miR.29a + miR.376a* + miR.494 + miR.639 + miR.338 | 0,78 | 0,105883144 |
| miR.558 + miR.609 + miR.603 + miR.369.3p + miR.376a* + miR.518c + miR.660 | 0,78 | 0,030516934 |
| miR.558 + miR.609 + miR.520f + miR.519b + miR.26a + miR.29a + miR.518c | 0,78 | 0,02547074 |
| miR.609 + miR.520f + miR.29a + miR.376a* + miR.494 + miR.660 + miR.338 | 0,78 | 0,1747453 |
| miR.609 + miR.519b + miR.29a + miR.369.3p + miR.376a* + miR.518c + miR.660 | 0,78 | 0,04021298 |
| miR.558 + miR.609 + miR.130a + miR.369.3p + miR.376a* + miR.518c + miR.338 | 0,78 | 0,079708523 |
| miR.609 + miR.603 + miR.520f + miR.29a + miR.369.3p + miR.376a* + miR.494 | 0,78 | 0,077941718 |
| miR.558 + miR.609 + miR.603 + miR.29a + miR.362 + miR.369.3p + miR.376a* | 0,78 | 0,027716639 |
| miR.558 + miR.609 + miR.603 + miR.520f + miR.130a + miR.29a + miR.518c | 0,78 | 0,021815158 |
| miR.558 + miR.609 + miR.520f + miR.519b + miR.130a + miR.29a + miR.518c | 0,78 | 0,02772778 |
| miR.558 + miR.609 + miR.26a + miR.29a + miR.362 + miR.494 + miR.518c | 0,78 | 0,009084869 |
| miR.609 + miR.519b + miR.29a + miR.369.3p + miR.376a* + miR.518c + miR.338 | 0,78 | 0,071009407 |
| miR.558 + miR.609 + miR.130a + miR.26a + miR.29a + miR.369.3p + miR.518c | 0,78 | 0,006328575 |
| miR.558 + miR.609 + miR.520f + miR.29a + miR.362 + miR.518c + miR.338 | 0,78 | 0,014421096 |
| miR.558 + miR.609 + miR.130a + miR.376a* + miR.518c + miR.639 + miR.338 | 0,78 | 0,108959907 |
| miR.558 + miR.609 + miR.603 + miR.520f + miR.29a + miR.362 + miR.518c | 0,78 | 0,028510686 |
| miR.558 + miR.609 + miR.26a + miR.29a + miR.369.3p + miR.376a* + miR.494 | 0,78 | 0,066158718 |
| miR.558 + miR.609 + miR.519b + miR.26a + miR.29a + miR.362 + miR.518c | 0,78 | 0,008922316 |
| miR.558 + miR.609 + miR.520f + miR.130a + miR.29a + miR.362 + miR.518c | 0,78 | 0,016751707 |
| miR.558 + miR.609 + miR.603 + miR.520f + miR.26a + miR.376a* + miR.518c | 0,78 | 0,016754078 |
| miR.558 + miR.609 + miR.130a + miR.26a + miR.29a + miR.518c + miR.639 | 0,78 | 0,009978795 |
| miR.609 + miR.29a + miR.369.3p + miR.376a* + miR.494 + miR.518c + miR.639 | 0,78 | 0,025322603 |
| miR.558 + miR.609 + miR.520f + miR.220aa + miR.519b + miR.29a + miR.518c | 0,78 | 0,05345257 |
| miR.558 + miR.609 + miR.520f + miR.130a + miR.29a + miR.518c + miR.338 | 0,78 | 0,016788844 |
| miR.558 + miR.609 + miR.520f + miR.519b + miR.29a + miR.362 + miR.518c | 0,78 | 0,037161741 |
| miR.558 + miR.609 + miR.220aa + miR.29a + miR.369.3p + miR.518c + miR.660 | 0,78 | 0,002872986 |
| miR.558 + miR.609 + miR.26a + miR.29a + miR.369.3p + miR.518c + miR.639 | 0,78 | 0,012857981 |
| miR.558 + miR.609 + miR.520f + miR.130a + miR.29a + miR.518c + miR.639 | 0,78 | 0,020261972 |
| miR.558 + miR.609 + miR.26a + miR.29a + miR.362 + miR.369.3p + miR.376a* | 0,78 | 0,035205989 |
| miR.558 + miR.609 + miR.26a + miR.29a + miR.362 + miR.369.3p + miR.518c | 0,78 | 0,009225922 |
| miR.558 + miR.609 + miR.26a + miR.29a + miR.369.3p + miR.494 + miR.518c | 0,78 | 0,008130434 |
| miR.558 + miR.609 + miR.603 + miR.29a + miR.369.3p + miR.376a* + miR.494 | 0,78 | 0,012928909 |
| miR.609 + miR.603 + miR.519b + miR.369.3p + miR.376a* + miR.518c + miR.639 | 0,78 | 0,0083191 |
| miR.558 + miR.609 + miR.520f + miR.519b + miR.29a + miR.518c + miR.338 | 0,78 | 0,024292032 |
| miR.558 + miR.609 + miR.220aa + miR.26a + miR.29a + miR.369.3p + miR.376a* | 0,78 | 0,022371809 |
| miR.558 + miR.609 + miR.130a + miR.362 + miR.376a* + miR.518c + miR.639 | 0,78 | 0,034122028 |
| miR.558 + miR.609 + miR.130a + miR.26a + miR.29a + miR.518c + miR.338 | 0,78 | 0,01379703 |
| miR.558 + miR.609 + miR.603 + miR.520f + miR.220aa + miR.29a + miR.518c | 0,78 | 0,02836698 |
| miR.558 + miR.609 + miR.520f + miR.362 + miR.518c + miR.639 + miR.660 | 0,78 | 0,100024182 |
| miR.558 + miR.609 + miR.603 + miR.520f + miR.29a + miR.518c + miR.338 | 0,78 | 0,016907051 |
| miR.558 + miR.609 + miR.26a + miR.29a + miR.518c + miR.639 + miR.338 | 0,78 | 0,013084907 |
| miR.558 + miR.609 + miR.520f + miR.29a + miR.362 + miR.494 + miR.518c | 0,78 | 0,011779808 |
| miR.558 + miR.609 + miR.130a + miR.29a + miR.369.3p + miR.518c + miR.660 | 0,78 | 0,000728457 |
| miR.558 + miR.609 + miR.519b + miR.26a + miR.29a + miR.369.3p + miR.518c | 0,78 | 0,008975345 |
| miR.609 + miR.603 + miR.130a + miR.26a + miR.369.3p + miR.376a* + miR.518c | 0,78 | 0,006730499 |
| miR.558 + miR.609 + miR.520f + miR.220aa + miR.29a + miR.518c + miR.338 | 0,78 | 0,010603664 |
| miR.558 + miR.609 + miR.220aa + miR.29a + miR.362 + miR.369.3p + miR.376a* | 0,78 | 0,021540479 |
| miR.558 + miR.609 + miR.603 + miR.520f + miR.29a + miR.494 + miR.518c | 0,78 | 0,020102222 |
| miR.558 + miR.609 + miR.520f + miR.29a + miR.518c + miR.639 + miR.338 | 0,78 | 0,017021124 |
| miR.609 + miR.520f + miR.220aa + miR.130a + miR.376a* + miR.518c + miR.338 | 0,78 | 0,04349407 |
| miR.558 + miR.609 + miR.130a + miR.29a + miR.494 + miR.518c + miR.660 | 0,78 | 0,001064546 |
| miR.558 + miR.609 + miR.520f + miR.29a + miR.362 + miR.518c + miR.639 | 0,78 | 0,015233262 |
| miR.558 + miR.609 + miR.520f + miR.130a + miR.26a + miR.376a* + miR.338 | 0,78 | 0,243410022 |
| miR.609 + miR.520f + miR.29a + miR.362 + miR.376a* + miR.494 + miR.660 | 0,78 | 0,113681762 |
| miR.558 + miR.609 + miR.603 + miR.26a + miR.369.3p + miR.376a* + miR.518c | 0,78 | 0,04241887 |
| miR.558 + miR.609 + miR.519b + miR.130a + miR.29a + miR.518c + miR.660 | 0,78 | 0,000490446 |
| miR.558 + miR.609 + miR.130a + miR.26a + miR.369.3p + miR.376a* + miR.518c | 0,78 | 0,058994582 |
| miR.558 + miR.609 + miR.520f + miR.29a + miR.494 + miR.518c + miR.338 | 0,78 | 0,01513134 |
| miR.609 + miR.520f + miR.220aa + miR.29a + miR.376a* + miR.494 + miR.338 | 0,78 | 0,063917787 |
| miR.558 + miR.609 + miR.603 + miR.220aa + miR.29a + miR.518c + miR.338 | 0,78 | 0,007654893 |
| miR.558 + miR.609 + miR.603 + miR.520f + miR.376a* + miR.518c + miR.338 | 0,78 | 0,090850124 |
| miR.558 + miR.609 + miR.220aa + miR.519b + miR.29a + miR.518c + miR.660 | 0,78 | 0,004246216 |
| miR.558 + miR.609 + miR.519b + miR.130a + miR.376a* + miR.518c + miR.639 | 0,78 | 0,05677794 |
| miR.558 + miR.609 + miR.520f + miR.220aa + miR.29a + miR.362 + miR.518c | 0,78 | 0,018548067 |
| miR.609 + miR.29a + miR.362 + miR.369.3p + miR.376a* + miR.494 + miR.518c | 0,78 | 0,074783771 |
| miR.558 + miR.609 + miR.520f + miR.29a + miR.369.3p + miR.518c + miR.338 | 0,78 | 0,012831826 |
| miR.558 + miR.609 + miR.603 + miR.369.3p + miR.376a* + miR.494 + miR.518c | 0,78 | 0,053291295 |

**Table 8: clusters of 8 miRNAs**

| **Cluster of 8 miRs** | **c_tau level** | **pv compared to top signature miR.558 + miR.609 + miR.603 + miR.220a + miR.29a + miR.369.3 p + miR.376a* + miR.518c** |
|---|---|---|
| miR.558 + miR.609 + miR.603 + miR.220a + miR.29a + miR.369.3p + miR.376a* + miR.518c | 0,81 | NaN |
| miR.558 + miR.609 + miR.603 + miR.220a + miR.26a + miR.29a + miR.376a* + miR.518c | 0,81 | 0,268588527 |
| miR.558 + miR.609 + miR.220a + miR.26a + miR.29a + miR.369.3p + miR.376a* + miR.518c | 0,81 | 0,213114289 |
| miR.558 + miR.609 + miR.220a + miR.29a + miR.369.3p + miR.376a* + miR.518c + miR.660 | 0,81 | 0,182815818 |
| miR.558 + miR.609 + miR.603 + miR.220a + miR.519b + miR.29a + miR.376a* + miR.518c | 0,81 | 0,289192622 |
| miR.558 + miR.609 + miR.520f + miR.220a + miR.29a + miR.369.3p + miR.376a* + miR.518c | 0,81 | 0,361357455 |
| miR.558 + miR.609 + miR.603 + miR.220a + miR.29a + miR.362 + miR.376a* + miR.518c | 0,81 | 0,077393148 |
| miR.558 + miR.609 + miR.603 + miR.520f + miR.220a + miR.29a + miR.376a* + miR.518c | 0,81 | 0,122807573 |
| miR.558 + miR.609 + miR.603 + miR.520f + miR.29a + miR.369.3p + miR.376a* + miR.518c | 0,81 | 0,272458791 |
| miR.558 + miR.609 + miR.603 + miR.26a + miR.29a + miR.369.3p + miR.376a* + miR.518c | 0,81 | 0,431946111 |
| miR.558 + miR.609 + miR.520f + miR.29a + miR.369.3p + miR.376a* + miR.518c + miR.338 | 0,81 | 0,412120313 |
| miR.558 + miR.609 + miR.520f + miR.220a + miR.29a + miR.362 + miR.376a* + miR.518c | 0,8 | 0,44685223 |
| miR.558 + miR.609 + miR.603 + miR.220a + miR.29a + miR.376a* + miR.518c + miR.660 | 0,8 | 0,055136323 |
| miR.558 + miR.609 + miR.520f + miR.220a + miR.29a + miR.376a* + miR.518c + miR.660 | 0,8 | 0,313798592 |
| miR.558 + miR.609 + miR.520f + miR.29a + miR.376a* + miR.518c + miR.660 + miR.338 | 0,8 | 0,489039597 |
| miR.558 + miR.609 + miR.520f + miR.29a + miR.369.3p + miR.376a* + miR.518c + miR.660 | 0,8 | 0,393346155 |
| miR.558 + miR.609 + miR.520f + miR.220a + miR.29a + miR.376a* + miR.518c + miR.338 | 0,8 | 0,482514973 |
| miR.558 + miR.609 + miR.220a + miR.130a + miR.29a + miR.369.3p + miR.376a* + miR.518c | 0,8 | 0,228451591 |
| miR.558 + miR.609 + miR.220a + miR.26a + miR.29a + miR.362 + miR.376a* + miR.518c | 0,8 | 0,350771488 |
| miR.558 + miR.609 + miR.220a + miR.26a + miR.29a + miR.376a* + miR.494 + miR.518c | 0,8 | 0,323451407 |
| miR.558 + miR.609 + miR.220a + miR.29a + miR.362 + miR.369.3p + miR.376a* + miR.518c | 0,8 | 0,371229727 |
| miR.558 + miR.609 + miR.220a + miR.29a + miR.369.3p + miR.376a* + miR.494 + miR.518c | 0,8 | 0,406008638 |
| miR.558 + miR.609 + miR.220a + miR.519b + miR.29a + miR.369.3p + miR.376a* + miR.518c | 0,8 | 0,292171386 |
| miR.558 + miR.609 + miR.220a + miR.29a + miR.369.3p + miR.376a* + miR.518c + miR.639 | 0,8 | 0,367581417 |
| miR.558 + miR.609 + miR.220a + miR.29a + miR.369.3p + miR.376a* + miR.518c + miR.338 | 0,8 | 0,231792794 |
| miR.558 + miR.609 + miR.520f + miR.220a + miR.29a + miR.376a* + miR.494 + miR.518c | 0,8 | 0,29026386 |
| miR.558 + miR.609 + miR.520f + miR.29a + miR.369.3p + miR.376a* + miR.518c + miR.639 | 0,8 | 0,294789553 |
| miR.558 + miR.609 + miR.220a + miR.26a + miR.29a + miR.376a* + miR.518c + miR.660 | 0,8 | 0,293352698 |
| miR.558 + miR.609 + miR.520f + miR.220a + miR.26a + miR.29a + miR.376a* + miR.518c | 0,8 | 0,414989303 |
| miR.558 + miR.609 + miR.603 + miR.220a + miR.29a + miR.376a* + miR.518c + miR.639 | 0,8 | 0,018528237 |
| miR.558 + miR.609 + miR.603 + miR.520f + miR.29a + miR.376a* + miR.518c + miR.338 | 0,8 | 0,319545984 |
| miR.558 + miR.609 + miR.520f + miR.29a + miR.369.3p + miR.376a* + miR.494 + miR.518c | 0,8 | 0,286586866 |
| miR.558 + miR.609 + miR.603 + miR.220a + miR.130a + miR.29a + miR.376a* + miR.518c | 0,8 | 0,019571018 |
| miR.558 + miR.609 + miR.603 + miR.220a + miR.29a + miR.376a* + miR.518c + miR.338 | 0,8 | 0,031011931 |
| miR.558 + miR.609 + miR.603 + miR.520f + miR.29a + miR.376a* + miR.494 + miR.518c | 0,8 | 0,119310203 |
| miR.558 + miR.609 + miR.603 + miR.520f + miR.29a + miR.362 + miR.376a* + miR.518c | 0,8 | 0,220773607 |
| miR.558 + miR.609 + miR.603 + miR.520f + miR.29a + miR.376a* + miR.518c + miR.639 | 0,8 | 0,111018106 |
| miR.558 + miR.609 + miR.520f + miR.26a + miR.29a + miR.369.3p + miR.376a* + miR.518c | 0,8 | 0,323312997 |
| miR.558 + miR.609 + miR.520f + miR.220a + miR.519b + miR.29a + miR.376a* + miR.518c | 0,8 | 0,459439323 |
| miR.558 + miR.609 + miR.520f + miR.220a + miR.130a + miR.29a + miR.376a* + miR.518c | 0,8 | 0,317028691 |
| miR.558 + miR.609 + miR.520f + miR.220a + miR.29a + miR.376a* + miR.518c + miR.639 | 0,8 | 0,313525665 |
| miR.609 + miR.603 + miR.220a + miR.29a + miR.369.3p + miR.376a* + miR.518c + miR.660 | 0,8 | 0,150538532 |
| miR.558 + miR.609 + miR.520f + miR.29a + miR.376a* + miR.494 + miR.518c + miR.338 | 0,8 | 0,2666813 |
| miR.558 + miR.609 + miR.603 + miR.520f + miR.29a + miR.376a* + miR.518c + miR.660 | 0,8 | 0,166396868 |
| miR.558 + miR.609 + miR.520f + miR.26a + miR.29a + miR.376a* + miR.518c + miR.660 | 0,8 | 0,192635079 |
| miR.558 + miR.609 + miR.520f + miR.29a + miR.376a* + miR.518c + miR.639 + miR.660 | 0,8 | 0,326979926 |
| miR.558 + miR.609 + miR.603 + miR.519b + miR.29a + miR.369.3p + miR.376a* + miR.518c | 0,8 | 0,184541446 |
| miR.558 + miR.609 + miR.520f + miR.29a + miR.362 + miR.376a* + miR.518c + miR.660 | 0,8 | 0,20424416 |
| miR.558 + miR.609 + miR.520f + miR.29a + miR.376a* + miR.518c + miR.639 + miR.338 | 0,8 | 0,312699347 |
| miR.558 + miR.609 + miR.603 + miR.29a + miR.369.3p + miR.376a* + miR.518c + miR.639 | 0,8 | 0,18500349 |
| miR.558 + miR.609 + miR.603 + miR.29a + miR.369.3p + miR.376a* + miR.518c + miR.660 | 0,8 | 0,121068728 |
| miR.558 + miR.609 + miR.520f + miR.29a + miR.362 + miR.369.3p + miR.376a* + miR.518c | 0,8 | 0,289544573 |
| miR.558 + miR.609 + miR.520f + miR.29a + miR.362 + miR.376a* + miR.518c + miR.338 | 0,8 | 0,496530929 |
| miR.558 + miR.609 + miR.603 + miR.220a + miR.29a + miR.376a* + miR.494 + miR.518c | 0,8 | 0,00891388 |
| miR.558 + miR.609 + miR.603 + miR.29a + miR.362 + miR.369.3p + miR.376a* + miR.518c | 0,8 | 0,200931672 |
| miR.558 + miR.609 + miR.220a + miR.26a + miR.29a + miR.376a* + miR.518c + miR.338 | 0,8 | 0,361017321 |
| miR.558 + miR.609 + miR.520f + miR.519b + miR.29a + miR.376a* + miR.518c + miR.660 | 0,8 | 0,206909861 |
| miR.558 + miR.609 + miR.603 + miR.130a + miR.29a + miR.369.3p + miR.376a* + miR.518c | 0,8 | 0,148949042 |
| miR.558 + miR.609 + miR.520f + miR.519b + miR.29a + miR.369.3p + miR.376a* + miR.518c | 0,8 | 0,300806891 |
| miR.558 + miR.609 + miR.520f + miR.130a + miR.29a + miR.369.3p + miR.376a* + miR.518c | 0,8 | 0,262991213 |
| miR.558 + miR.609 + miR.520f + miR.29a + miR.362 + miR.376a* + miR.518c + miR.639 | 0,8 | 0,303472164 |
| miR.558 + miR.609 + miR.220a + miR.130a + miR.29a + miR.362 + miR.376a* + miR.518c | 0,8 | 0,263995365 |
| miR.558 + miR.609 + miR.520f + miR.29a + miR.376a* + miR.494 + miR.518c + miR.660 | 0,8 | 0,240538444 |
| miR.558 + miR.609 + miR.220a + miR.519b + miR.29a + miR.362 + miR.376a* + miR.518c | 0,8 | 0,287179997 |
| miR.558 + miR.609 + miR.220a + miR.130a + miR.26a + miR.29a + miR.376a* + miR.518c | 0,8 | 0,252584893 |
| miR.558 + miR.609 + miR.220a + miR.26a + miR.29a + miR.376a* + miR.518c + miR.639 | 0,8 | 0,2300802 |
| miR.558 + miR.609 + miR.220a + miR.519b + miR.26a + miR.29a + miR.376a* + miR.518c | 0,8 | 0,212034229 |
| miR.558 + miR.609 + miR.603 + miR.520f + miR.519b + miR.29a + miR.376a* + miR.518c | 0,8 | 0,150034774 |
| miR.558 + miR.609 + miR.220a + miR.29a + miR.362 + miR.376a* + miR.518c + miR.338 | 0,8 | 0,18806259 |
| miR.558 + miR.609 + miR.520f + miR.29a + miR.376a* + miR.494 + miR.518c + miR.639 | 0,8 | 0,16791359 |
| miR.558 + miR.609 + miR.220a + miR.29a + miR.362 + miR.376a* + miR.518c + miR.639 | 0,8 | 0,187103112 |
| miR.558 + miR.609 + miR.603 + miR.520f + miR.130a + miR.29a + miR.376a* + miR.518c | 0,8 | 0,078398975 |
| miR.558 + miR.609 + miR.603 + miR.520f + miR.26a + miR.29a + miR.376a* + miR.518c | 0,8 | 0,140471281 |
| miR.558 + miR.609 + miR.520f + miR.29a + miR.362 + miR.376a* + miR.494 + miR.518c | 0,8 | 0,329819355 |
| miR.558 + miR.609 + miR.26a + miR.29a + miR.369.3p + miR.376a* + miR.518c + miR.338 | 0,8 | 0,449625169 |
| miR.558 + miR.609 + miR.603 + miR.29a + miR.369.3p + miR.376a* + miR.518c + miR.338 | 0,8 | 0,335759964 |
| miR.558 + miR.609 + miR.220a + miR.29a + miR.376a* + miR.494 + miR.518c + miR.660 | 0,8 | 0,267344726 |
| miR.558 + miR.609 + miR.520f + miR.130a + miR.29a + miR.376a* + miR.518c + miR.338 | 0,8 | 0,308053158 |
| miR.558 + miR.609 + miR.26a + miR.29a + miR.369.3p + miR.376a* + miR.494 + miR.518c | 0,8 | 0,207927956 |
| miR.558 + miR.609 + miR.26a + miR.29a + miR.369.3p + miR.376a* + miR.518c + miR.639 | 0,8 | 0,186741599 |
| miR.558 + miR.609 + miR.220a + miR.519b + miR.29a + miR.376a* + miR.518c + miR.660 | 0,8 | 0,254116495 |
| miR.558 + miR.609 + miR.603 + miR.29a + miR.369.3p + miR.376a* + miR.494 + miR.518c | 0,8 | 0,171531707 |
| miR.558 + miR.609 + miR.220a + miR.29a + miR.376a* + miR.518c + miR.639 + miR.660 | 0,8 | 0,248205674 |
| miR.558 + miR.609 + miR.220a + miR.130a + miR.29a + miR.376a* + miR.518c + miR.660 | 0,8 | 0,2530454 |
| miR.558 + miR.609 + miR.520f + miR.26a + miR.29a + miR.376a* + miR.518c + miR.338 | 0,8 | 0,37924681 |
| miR.558 + miR.609 + miR.520f + miR.519b + miR.29a + miR.376a* + miR.518c + miR.338 | 0,8 | 0,454251801 |
| miR.558 + miR.609 + miR.220a + miR.29a + miR.362 + miR.376a* + miR.518c + miR.660 | 0,8 | 0,214624308 |
| miR.558 + miR.609 + miR.220a + miR.29a + miR.376a* + miR.518c + miR.660 + miR.338 | 0,8 | 0,245944937 |
| miR.558 + miR.609 + miR.220a + miR.130a + miR.29a + miR.376a* + miR.494 + miR.518c | 0,8 | 0,256722473 |
| miR.609 + miR.603 + miR.220a + miR.26a + miR.29a + miR.369.3p + miR.376a* + miR.518c | 0,8 | 0,185061761 |
| miR.558 + miR.609 + miR.603 + miR.26a + miR.29a + miR.376a* + miR.518c + miR.639 | 0,8 | 0,211280496 |
| miR.558 + miR.609 + miR.520f + miR.130a + miR.29a + miR.376a* + miR.518c + miR.639 | 0,8 | 0,176391392 |
| miR.609 + miR.603 + miR.220a + miR.29a + miR.369.3p + miR.376a* + miR.494 + miR.518c | 0,8 | 0,261711358 |
| miR.558 + miR.609 + miR.520f + miR.519b + miR.29a + miR.376a* + miR.494 + miR.518c | 0,8 | 0,239641338 |
| miR.558 + miR.609 + miR.220a + miR.519b + miR.130a + miR.29a + miR.376a* + miR.518c | 0,8 | 0,266135049 |
| miR.558 + miR.609 + miR.520f + miR.130a + miR.29a + miR.376a* + miR.518c + miR.660 | 0,8 | 0,198874815 |
| miR.558 + miR.609 + miR.220a + miR.130a + miR.29a + miR.376a* + miR.518c + miR.338 | 0,8 | 0,238065786 |
| miR.558 + miR.609 + miR.520f + miR.130a + miR.29a + miR.362 + miR.376a* + miR.518c | 0,8 | 0,295159139 |
| miR.558 + miR.609 + miR.603 + miR.26a + miR.29a + miR.376a* + miR.494 +miR.518c | 0,8 | 0,12140431 |
| miR.558 + miR.609 + miR.520f + miR.519b + miR.29a + miR.362 + miR.376a* + miR.518c | 0,8 | 0,320182911 |
| miR.558 + miR.609 + miR.220a + miR.519b + miR.29a + miR.376a* + miR.518c + miR.639 | 0,8 | 0,279758171 |
| miR.558 + miR.609 + miR.220a + miR.130a + miR.29a + miR.376a* + miR.518c + miR.639 | 0,8 | 0,249205161 |
| miR.558 + miR.609 + miR.520f + miR.26a + miR.29a + miR.362 + miR.376a* + miR.518c | 0,8 | 0,300314786 |
| miR.558 + miR.609 + miR.220a + miR.519b + miR.29a + miR.376a* + miR.518c + miR.338 | 0,8 | 0,340189429 |
| miR.558 + miR.609 + miR.520f + miR.26a + miR.29a + miR.376a* + miR.518c + miR.639 | 0,8 | 0,200668383 |
| miR.558 + miR.609 + miR.520f + miR.130a + miR.29a + miR.376a* + miR.494 + miR.518c | 0,8 | 0,225833391 |
| miR.558 + miR.609 + miR.26a + miR.29a + miR.369.3p + miR.376a* + miR.518c + miR.660 | 0,8 | 0,268694626 |
| miR.558 + miR.609 + miR.520f + miR.26a + miR.29a + miR.376a* + miR.494 + miR.518c | 0,8 | 0,221197278 |
| miR.558 + miR.609 + miR.519b + miR.26a + miR.29a + miR.369.3p + miR.376a* + miR.518c | 0,8 | 0,238847644 |
| miR.558 + miR.609 + miR.220a + miR.29a + miR.376a* + miR.518c + miR.639 + miR.338 | 0,8 | 0,196005317 |
| miR.558 + miR.609 + miR.520f + miR.519b + miR.29a + miR.376a* + miR.518c + miR.639 | 0,8 | 0,19084843 |
| miR.558 + miR.609 + miR.220a + miR.29a + miR.376a* + miR.494 + miR.518c + miR.639 | 0,8 | 0,203579685 |
| miR.609 + miR.603 + miR.520f + miR.220a + miR.29a + miR.369.3p + miR.376a* + miR.518c | 0,8 | 0,228865741 |
| miR.558 + miR.609 + miR.26a + miR.29a + miR.362 + miR.369.3p + miR.376a* + miR.518c | 0,8 | 0,202072601 |
| miR.558 + miR.609 + miR.220a + miR.29a + miR.376a* + miR.494 + miR.518c + miR.338 | 0,8 | 0,175352497 |
| miR.558 + miR.609 + miR.603 + miR.26a + miR.29a + miR.376a* + miR.518c + miR.338 | 0,8 | 0,199674652 |
| miR.558 + miR.609 + miR.29a + miR.362 + miR.369.3p + miR.376a* + miR.518c + miR.660 | 0,8 | 0,420367935 |
| miR.558 + miR.609 + miR.130a + miR.26a + miR.29a + miR.369.3p + miR.376a* + miR.518c | 0,8 | 0,328716887 |
| miR.609 + miR.603 + miR.220a + miR.519b + miR.29a + miR.369.3p + miR.376a* + miR.518c | 0,8 | 0,271684254 |
| miR.558 + miR.609 + miR.520f + miR.130a + miR.26a + miR.29a + miR.376a* + miR.518c | 0,8 | 0,237214954 |
| miR.609 + miR.603 + miR.220a + miR.29a + miR.362 + miR.369.3p + miR.376a* + miR.518c | 0,8 | 0,087793914 |
| miR.609 + miR.603 + miR.220a + miR.29a + miR.369.3p + miR.376a* + miR.518c + miR.639 | 0,8 | 0,177717277 |
| miR.558 + miR.609 + miR.130a + miR.29a + miR.369.3p + miR.376a* + miR.518c + miR.639 | 0,8 | 0,165100031 |
| miR.558 + miR.609 + miR.520f + miR.519b + miR.130a + miR.29a + miR.376a* + miR.518c | 0,8 | 0,236748396 |
| miR.558 + miR.609 + miR.520f + miR.519b + miR.26a + miR.29a + miR.376a* + miR.518c | 0,8 | 0,243362019 |
| miR.609 + miR.603 + miR.220a + miR.29a + miR.369.3p + miR.376a* + miR.518c + miR.338 | 0,8 | 0,056920249 |
| miR.558 + miR.609 + miR.130a + miR.29a + miR.369.3p + miR.376a* + miR.494 + miR.518c | 0,8 | 0,138873294 |
| miR.558 + miR.609 + miR.220a + miR.29a + miR.362 + miR.376a* + miR.494 + miR.518c | 0,8 | 0,261772468 |
| miR.558 + miR.609 + miR.220a + miR.519b + miR.29a + miR.376a* + miR.494 + miR.518c | 0,8 | 0,277820043 |
| miR.558 + miR.609 + miR.603 + miR.26a + miR.29a + miR.362 + miR.376a* + miR.518c | 0,8 | 0,20891331 |
| miR.558 + miR.609 + miR.603 + miR.29a + miR.376a* + miR.518c + miR.639 + miR.660 | 0,8 | 0,135181446 |
| miR.609 + miR.603 + miR.220a + miR.130a + miR.29a + miR.369.3p + miR.376a* + miR.518c | 0,8 | 0,128158679 |
| miR.558 + miR.609 + miR.603 + miR.130a + miR.29a + miR.376a* + miR.494 + miR.518c | 0,8 | 0,056880534 |
| miR.558 + miR.609 + miR.603 + miR.130a + miR.26a + miR.29a + miR.376a* + miR.518c | 0,8 | 0,123855549 |
| miR.558 + miR.609 + miR.130a + miR.29a + miR.376a* + miR.494 + miR.518c + miR.639 | 0,8 | 0,118227692 |
| miR.558 + miR.609 + miR.603 + miR.26a + miR.29a + miR.376a* + miR.518c + miR.660 | 0,8 | 0,154474098 |
| miR.558 + miR.609 + miR.603 + miR.130a + miR.29a + miR.376a* + miR.518c + miR.639 | 0,8 | 0,062202459 |
| miR.558 + miR.609 + miR.603 + miR.519b + miR.26a + miR.29a + miR.376a* + miR.518c | 0,8 | 0,174325893 |
| miR.558 + miR.609 + miR.603 + miR.29a + miR.376a* + miR.494 + miR.518c + miR.639 | 0,8 | 0,034840802 |
| miR.558 + miR.609 + miR.29a + miR.369.3p + miR.376a* + miR.518c + miR.639 + miR.338 | 0,8 | 0,280813829 |
| miR.558 + miR.609 + miR.26a + miR.29a + miR.376a* + miR.518c + miR.639 + miR.338 | 0,8 | 0,1616683 |
| miR.558 + miR.609 + miR.603 + miR.29a + miR.376a* + miR.518c + miR.639 + miR.338 | 0,8 | 0,056465371 |
| miR.558 + miR.609 + miR.603 + miR.29a + miR.362 + miR.376a* + miR.518c + miR.639 | 0,8 | 0,097576451 |
| miR.558 + miR.609 + miR.130a + miR.29a + miR.369.3p + miR.376a* + miR.518c + miR.338 | 0,8 | 0,195306685 |
| miR.609 + miR.603 + miR.520f + miR.220a + miR.29a + miR.362 + miR.376a* + miR.518c | 0,8 | 0,298103073 |
| miR.558 + miR.609 + miR.26a + miR.29a + miR.362 + miR.376a* + miR.518c + miR.639 | 0,8 | 0,11359095 |
| miR.558 + miR.609 + miR.29a + miR.369.3p + miR.376a* + miR.518c + miR.639 + miR.660 | 0,8 | 0,138043804 |
| miR.558 + miR.609 + miR.519b + miR.26a + miR.29a + miR.376a* + miR.518c + miR.639 | 0,8 | 0,097424408 |
| miR.558 + miR.609 + miR.29a + miR.369.3p + miR.376a* + miR.494 + miR.518c + miR.639 | 0,8 | 0,120044773 |
| miR.609 + miR.603 + miR.520f + miR.29a + miR.369.3p + miR.376a* + miR.518c + miR.338 | 0,8 | 0,465334345 |
| miR.558 + miR.609 + miR.603 + miR.519b + miR.29a + miR.376a* + miR.518c + miR.639 | 0,8 | 0,121362751 |
| miR.609 + miR.603 + miR.520f + miR.29a + miR.369.3p + miR.376a* + miR.494 + miR.518c | 0,8 | 0,137329361 |
| miR.558 + miR.609 + miR.603 + miR.29a + miR.376a* + miR.494 + miR.518c + miR.660 | 0,8 | 0,022947512 |
| miR.558 + miR.609 + miR.130a + miR.29a + miR.369.3p + miR.376a* + miR.518c + miR.660 | 0,8 | 0,127014266 |
| miR.558 + miR.609 + miR.603 + miR.519b + miR.29a + miR.376a* + miR.494 + miR.518c | 0,8 | 0,235217454 |
| miR.558 + miR.609 + miR.130a + miR.26a + miR.29a + miR.376a* + miR.518c + miR.639 | 0,8 | 0,068427243 |
| miR.558 + miR.609 + miR.603 + miR.519b + miR.29a + miR.362 + miR.376a* + miR.518c | 0,8 | 0,109812067 |
| miR.558 + miR.609 + miR.603 + miR.29a + miR.376a* + miR.518c + miR.660 + miR.338 | 0,8 | 0,028202923 |
| miR.558 + miR.609 + miR.519b + miR.29a + miR.369.3p + miR.376a* + miR.518c + miR.660 | 0,8 | 0,082284699 |
| miR.558 + miR.609 + miR.130a + miR.29a + miR.362 + miR.369.3p + miR.376a* + miR.518c | 0,8 | 0,120164496 |
| miR.558 + miR.609 + miR.29a + miR.362 + miR.369.3p + miR.376a* + miR.518c + miR.639 | 0,8 | 0,186922436 |
| miR.558 + miR.609 + miR.603 + miR.130a + miR.29a + miR.362 + miR.376a* + miR.518c | 0,8 | 0,029305309 |
| miR.558 + miR.609 + miR.603 + miR.29a + miR.362 + miR.376a* + miR.518c + miR.338 | 0,8 | 0,031276981 |
| miR.558 + miR.609 + miR.603 + miR.519b + miR.29a + miR.376a* + miR.518c + miR.338 | 0,8 | 0,140168957 |
| miR.558 + miR.609 + miR.519b + miR.29a + miR.369.3p + miR.376a* + miR.518c + miR.639 | 0,8 | 0,104607901 |
| miR.558 + miR.609 + miR.519b + miR.130a + miR.29a + miR.369.3p + miR.376a* + miR.518c | 0,8 | 0,069192656 |
| miR.558 + miR.609 + miR.603 + miR.519b + miR.29a + miR.376a* + miR.518c + miR.660 | 0,8 | 0,085445288 |
| miR.609 + miR.603 + miR.520f + miR.220a + miR.29a + miR.376a* + miR.494 + miR.518c | 0,8 | 0,178980602 |
| miR.558 + miR.609 + miR.603 + miR.130a + miR.29a + miR.376a* + miR.518c + miR.338 | 0,8 | 0,015359888 |
| miR.558 + miR.609 + miR.603 + miR.29a + miR.362 + miR.376a* + miR.518c + miR.660 | 0,8 | 0,015195308 |
| miR.609 + miR.520f + miR.220a + miR.29a + miR.369.3p + miR.376a* + miR.518c + miR.660 | 0,8 | 0,435898894 |
| miR.609 + miR.603 + miR.520f + miR.220a + miR.29a + miR.376a* + miR.518c + miR.660 | 0,8 | 0,25544504 |
| miR.609 + miR.603 + miR.220a + miR.519b + miR.29a + miR.376a* + miR.494 + miR.518c | 0,8 | 0,079935193 |
| miR.558 + miR.609 + miR.603 + miR.130a + miR.29a + miR.376a* + miR.518c + miR.660 | 0,8 | 0,026025415 |
| miR.558 + miR.609 + miR.603 + miR.29a + miR.376a* + miR.494 + miR.518c + miR.338 | 0,8 | 0,035055003 |
| miR.558 + miR.609 + miR.603 + miR.519b + miR.130a + miR.29a + miR.376a* + miR.518c | 0,8 | 0,089871509 |
| miR.558 + miR.609 + miR.519b + miR.29a + miR.362 + miR.369.3p + miR.376a* + miR.518c | 0,8 | 0,103014709 |
| miR.609 + miR.603 + miR.220a + miR.26a + miR.29a + miR.376a* + miR.494 + miR.518c | 0,8 | 0,067719009 |
| miR.558 + miR.609 + miR.130a + miR.29a + miR.376a* + miR.518c + miR.639 + miR.660 | 0,8 | 0,143256166 |
| miR.558 + miR.609 + miR.29a + miR.369.3p + miR.376a* + miR.518c + miR.660 + miR.338 | 0,8 | 0,180360693 |
| miR.558 + miR.609 + miR.29a + miR.369.3p + miR.376a* + miR.494 + miR.518c + miR.338 | 0,8 | 0,146286413 |
| miR.558 + miR.609 + miR.29a + miR.369.3p + miR.376a* + miR.494 + miR.518c + miR.660 | 0,8 | 0,094223238 |
| miR.609 + miR.603 + miR.220a + miR.519b + miR.29a + miR.362 + miR.376a* + miR.518c | 0,8 | 0,213858057 |
| miR.558 + miR.609 + miR.26a + miR.29a + miR.376a* + miR.518c + miR.639 + miR.660 | 0,8 | 0,086522612 |
| miR.609 + miR.603 + miR.220a + miR.29a + miR.376a* + miR.494 + miR.518c + miR.660 | 0,8 | 0,119327352 |
| miR.558 + miR.609 + miR.29a + miR.362 + miR.369.3p + miR.376a* + miR.518c + miR.338 | 0,8 | 0,266383734 |
| miR.558 + miR.609 + miR.26a + miR.29a + miR.376a* + miR.494 + miR.518c + miR.639 | 0,8 | 0,072177566 |
| miR.609 + miR.603 + miR.520f + miR.29a + miR.369.3p + miR.376a* + miR.518c + miR.639 | 0,8 | 0,172400609 |
| miR.558 + miR.609 + miR.130a + miR.26a + miR.29a + miR.376a* + miR.494 + miR.518c | 0,8 | 0,075112277 |
| miR.558 + miR.609 + miR.29a + miR.362 + miR.369.3p + miR.376a* + miR.494 + miR.518c | 0,8 | 0,094654013 |
| miR.609 + miR.603 + miR.520f + miR.220a + miR.130a + miR.29a + miR.376a* + miR.518c | 0,8 | 0,119123492 |
| miR.609 + miR.520f + miR.220a + miR.29a + miR.376a* + miR.494 + miR.518c + miR.660 | 0,8 | 0,264617006 |
| miR.558 + miR.609 + miR.26a + miR.29a + miR.376a* + miR.494 + miR.518c + miR.338 | 0,8 | 0,133540571 |
| miR.558 + miR.609 + miR.519b + miR.29a + miR.369.3p + miR.376a* + miR.494 + miR.518c | 0,8 | 0,080724013 |
| miR.558 + miR.609 + miR.519b + miR.29a + miR.369.3p + miR.376a* + miR.518c + miR.338 | 0,8 | 0,154390529 |
| miR.609 + miR.520f + miR.220a + miR.29a + miR.369.3p + miR.376a* + miR.494 + miR.518c | 0,8 | 0,396584072 |
| miR.609 + miR.603 + miR.520f + miR.29a + miR.376a* + miR.494 + miR.518c + miR.338 | 0,8 | 0,142937902 |
| miR.609 + miR.603 + miR.220a + miR.26a + miR.29a + miR.362 + miR.376a* + miR.518c | 0,8 | 0,1707397 |
| miR.558 + miR.609 + miR.26a + miR.29a + miR.376a* + miR.494 + miR.518c + miR.660 | 0,8 | 0,036014826 |
| miR.609 + miR.603 + miR.520f + miR.130a + miR.29a + miR.369.3p + miR.376a* + miR.518c | 0,8 | 0,104606059 |
| miR.609 + miR.603 + miR.520f + miR.220a + miR.519b + miR.29a + miR.376a* + miR.518c | 0,8 | 0,186283275 |
| miR.558 + miR.609 + miR.603 + miR.29a + miR.362 + miR.376a* + miR.494 + miR.518c | 0,8 | 0,046802641 |
| miR.609 + miR.603 + miR.520f + miR.29a + miR.376a* + miR.494 + miR.518c + miR.639 | 0,8 | 0,071257355 |
| miR.609 + miR.603 + miR.520f + miR.220a + miR.26a + miR.29a + miR.376a* + miR.518c | 0,8 | 0,232483239 |
| miR.558 + miR.609 + miR.26a + miR.29a + miR.362 + miR.376a* + miR.494 + miR.518c | 0,8 | 0,064134024 |
| miR.558 + miR.609 + miR.130a + miR.26a + miR.29a + miR.376a* + miR.518c + miR.338 | 0,8 | 0,110791155 |
| miR.558 + miR.609 + miR.519b + miR.26a + miR.29a + miR.376a* + miR.518c + miR.338 | 0,8 | 0,093287896 |
| miR.609 + miR.603 + miR.520f + miR.29a + miR.369.3p + miR.376a* + miR.518c + miR.660 | 0,8 | 0,134407093 |
| miR.558 + miR.609 + miR.603 + miR.220a + miR.369.3p + miR.376a* + miR.518c + miR.639 | 0,8 | 0,162259653 |
| miR.558 + miR.609 + miR.26a + miR.29a + miR.362 + miR.376a* + miR.518c + miR.338 | 0,8 | 0,133489174 |
| miR.558 + miR.609 + miR.519b + miR.26a + miR.29a + miR.376a* + miR.494 + miR.518c | 0,8 | 0,078055879 |
| miR.609 + miR.220a + miR.29a + miR.362 + miR.369.3p + miR.376a* + miR.518c + miR.660 | 0,8 | 0,328685687 |
| miR.609 + miR.603 + miR.220a + miR.29a + miR.362 + miR.376a* + miR.518c + miR.639 | 0,8 | 0,107395171 |
| miR.609 + miR.603 + miR.220a + miR.519b + miR.26a + miR.29a + miR.376a* + miR.518c | 0,8 | 0,094737666 |
| miR.609 + miR.603 + miR.220a + miR.519b + miR.29a + miR.376a* + miR.518c + miR.660 | 0,8 | 0,212758102 |
| miR.609 + miR.603 + miR.220a + miR.26a + miR.29a + miR.376a* + miR.518c + miR.639 | 0,8 | 0,053619139 |
| miR.558 + miR.609 + miR.130a + miR.29a + miR.362 + miR.376a* + miR.518c + miR.639 | 0,8 | 0,101029379 |
| miR.609 + miR.603 + miR.220a + miR.29a + miR.376a* + miR.518c + miR.639 + miR.660 | 0,8 | 0,074083874 |
| miR.609 + miR.603 + miR.220a + miR.29a + miR.362 + miR.376a* + miR.518c + miR.338 | 0,8 | 0,095069147 |
| miR.609 + miR.520f + miR.220a + miR.29a + miR.362 + miR.376a* + miR.494 + miR.518c | 0,8 | 0,303762525 |
| miR.609 + miR.603 + miR.520f + miR.29a + miR.376a* + miR.494 + miR.518c + miR.660 | 0,8 | 0,123670087 |
| miR.609 + miR.603 + miR.220a + miR.519b + miR.29a + miR.376a* + miR.518c + miR.639 | 0,8 | 0,094014764 |
| miR.558 + miR.609 + miR.130a + miR.29a + miR.376a* + miR.494 + miR.518c + miR.660 | 0,8 | 0,045727689 |
| miR.609 + miR.603 + miR.520f + miR.26a + miR.29a + miR.369.3p + miR.376a* + miR.518c | 0,8 | 0,197866833 |
| miR.609 + miR.603 + miR.220a + miR.29a + miR.362 + miR.376a* + miR.518c + miR.660 | 0,8 | 0,117066493 |
| miR.609 + miR.603 + miR.520f + miR.29a + miR.376a* + miR.518c + miR.639 + miR.338 | 0,8 | 0,166778331 |
| miR.609 + miR.603 + miR.520f + miR.519b + miR.29a + miR.369.3p + miR.376a* + miR.518c | 0,8 | 0,125139861 |
| miR.558 + miR.609 + miR.130a + miR.29a + miR.376a* + miR.518c + miR.639 + miR.338 | 0,8 | 0,089186105 |
| miR.609 + miR.603 + miR.520f + miR.220a + miR.29a + miR.376a* + miR.518c + miR.639 | 0,8 | 0,160389649 |
| miR.558 + miR.609 + miR.519b + miR.130a + miR.29a + miR.376a* + miR.494 + miR.518c | 0,8 | 0,084954918 |
| miR.609 + miR.603 + miR.220a + miR.26a + miR.29a + miR.376a* + miR.518c + miR.338 | 0,8 | 0,102695035 |
| miR.609 + miR.603 + miR.520f + miR.29a + miR.362 + miR.376a* + miR.494 + miR.518c | 0,8 | 0,110204651 |
| miR.558 + miR.609 + miR.603 + miR.520f + miR.376a* + miR.518c + miR.639 + miR.338 | 0,8 | 0,284136432 |
| miR.609 + miR.603 + miR.520f + miR.220a + miR.29a + miR.376a* + miR.518c + miR.338 | 0,8 | 0,463417504 |
| miR.558 + miR.609 + miR.130a + miR.29a + miR.376a* + miR.494 + miR.518c + miR.338 | 0,8 | 0,131382553 |
| miR.609 + miR.603 + miR.520f + miR.519b + miR.29a + miR.376a* + miR.494 + miR.518c | 0,8 | 0,079519627 |
| miR.558 + miR.609 + miR.130a + miR.29a + miR.362 + miR.376a* + miR.494 + miR.518c | 0,8 | 0,099330475 |
| miR.609 + miR.603 + miR.220a + miR.130a + miR.26a + miR.29a + miR.376a* + miR.518c | 0,8 | 0,140026047 |
| miR.609 + miR.603 + miR.220a + miR.26a + miR.29a + miR.376a* + miR.518c + miR.660 | 0,8 | 0,121181666 |
| miR.609 + miR.603 + miR.29a + miR.369.3p + miR.376a* + miR.494 + miR.518c + miR.639 | 0,8 | 0,045503448 |
| miR.609 + miR.603 + miR.220a + miR.130a + miR.29a + miR.376a* + miR.518c + miR.660 | 0,8 | 0,204975548 |
| miR.558 + miR.609 + miR.519b + miR.26a + miR.29a + miR.376a* + miR.518c + miR.660 | 0,8 | 0,047513606 |
| miR.558 + miR.609 + miR.26a + miR.29a + miR.376a* + miR.518c + miR.660 + miR.338 | 0,8 | 0,112203655 |
| miR.558 + miR.609 + miR.519b + miR.130a + miR.29a + miR.376a* + miR.518c + miR.639 | 0,8 | 0,079495498 |
| miR.609 + miR.603 + miR.520f + miR.29a + miR.362 + miR.369.3p + miR.376a* + miR.518c | 0,8 | 0,175008729 |
| miR.609 + miR.603 + miR.220a + miR.29a + miR.376a* + miR.518c + miR.660 + miR.338 | 0,8 | 0,12574749 |
| miR.609 + miR.603 + miR.220a + miR.519b + miR.130a + miR.29a + miR.376a* + miR.518c | 0,8 | 0,129863439 |
| miR.558 + miR.609 + miR.603 + miR.520f + miR.369.3p + miR.376a* + miR.518c + miR.639 | 0,8 | 0,262358671 |
| miR.609 + miR.603 + miR.520f + miR.26a + miR.29a + miR.376a* + miR.494 + miR.518c | 0,8 | 0,07637776 |
| miR.558 + miR.609 + miR.519b + miR.26a + miR.29a + miR.362 + miR.376a* + miR.518c | 0,8 | 0,072637868 |
| miR.609 + miR.603 + miR.520f + miR.130a + miR.29a + miR.376a* + miR.494 + miR.518c | 0,8 | 0,075484443 |
| miR.609 + miR.520f + miR.220a + miR.519b + miR.29a + miR.369.3p + miR.376a* + miR.518c | 0,8 | 0,449651468 |
| miR.609 + miR.603 + miR.26a + miR.29a + miR.369.3p + miR.376a* + miR.494 + miR.518c | 0,8 | 0,059350142 |
| miR.558 + miR.609 + miR.603 + miR.220a + miR.130a + miR.369.3p + miR.376a* + miR.518c | 0,8 | 0,047306499 |
| miR.609 + miR.520f + miR.220a + miR.130a + miR.29a + miR.369.3p + miR.376a* + miR.518c | 0,8 | 0,351618123 |
| miR.609 + miR.603 + miR.220a + miR.130a + miR.29a + miR.362 + miR.376a* + miR.518c | 0,8 | 0,1585677 97 |
| miR.558 + miR.609 + miR.519b + miR.130a + miR.26a + miR.29a + miR.376a* + miR.518c | 0,8 | 0,049965452 |
| miR.558 + miR.609 + miR.520f + miR.29a + miR.369.3p + miR.376a* + miR.494 + miR.338 | 0,8 | 0,187377223 |
| miR.558 + miR.609 + miR.130a + miR.26a + miR.29a + miR.362 + miR.376a* + miR.518c | 0,8 | 0,066922956 |
| miR.558 + miR.609 + miR.26a + miR.29a + miR.362 + miR.376a* + miR.518c + miR.660 | 0,8 | 0,029053191 |
| miR.558 + miR.609 + miR.130a + miR.26a + miR.29a + miR.376a* + miR.518c + miR.660 | 0,8 | 0,034497604 |
| miR.558 + miR.609 + miR.603 + miR.520f + miR.130a + miR.376a* + miR.518c + miR.338 | 0,8 | 0,16955606 |
| miR.558 + miR.609 + miR.29a + miR.362 + miR.376a* + miR.518c + miR.639 + miR.660 | 0,8 | 0,063517482 |
| miR.558 + miR.609 + miR.520f + miR.29a + miR.369.3p + miR.376a* + miR.660 + miR.338 | 0,8 | 0,386278992 |
| miR.609 + miR.603 + miR.29a + miR.369.3p + miR.376a* + miR.518c + miR.639 + miR.338 | 0,8 | 0,148250881 |
| miR.609 + miR.603 + miR.26a + miR.29a + miR.369.3p + miR.376a* + miR.518c + miR.639 | 0,8 | 0,040619957 |
| miR.558 + miR.609 + miR.603 + miR.520f + miR.130a + miR.376a* + miR.518c + miR.639 | 0,8 | 0,117617632 |
| miR.609 + miR.220a + miR.29a + miR.369.3p + miR.376a* + miR.494 + miR.518c + miR.660 | 0,8 | 0,227245906 |
| miR.609 + miR.603 + miR.520f + miR.130a + miR.29a + miR.376a* + miR.518c + miR.338 | 0,8 | 0,214616822 |
| miR.609 + miR.520f + miR.220a + miR.130a + miR.29a + miR.376a* + miR.518c + miR.660 | 0,8 | 0,217974393 |
| miR.609 + miR.603 + miR.220a + miR.519b + miR.29a + miR.376a* + miR.518c + miR.338 | 0,8 | 0,106536586 |
| miR.558 + miR.609 + miR.29a + miR.376a* + miR.494 + miR.518c + miR.639 + miR.660 | 0,8 | 0,047867864 |
| miR.558 + miR.609 + miR.29a + miR.362 + miR.376a* + miR.518c + miR.639 + miR.338 | 0,79 | 0,104359161 |
| miR.609 + miR.520f + miR.220a + miR.29a + miR.376a* + miR.494 + miR.518c + miR.338 | 0,79 | 0,279861289 |
| miR.609 + miR.520f + miR.220a + miR.29a + miR.362 + miR.369.3p + miR.376a* + miR.518c | 0,79 | 0,465378209 |
| miR.609 + miR.603 + miR.220a + miR.29a + miR.376a* + miR.494 + miR.518c + miR.639 | 0,79 | 0,014065978 |
| miR.609 + miR.520f + miR.220a + miR.29a + miR.369.3p + miR.376a* + miR.518c + miR.338 | 0,79 | 0,464336891 |
| miR.609 + miR.603 + miR.220a + miR.29a + miR.362 + miR.376a* + miR.494 + miR.518c | 0,79 | 0,070687312 |
| miR.609 + miR.520f + miR.220a + miR.130a + miR.29a + miR.376a* + miR.494 + miR.518c | 0,79 | 0,183461255 |
| miR.609 + miR.603 + miR.520f + miR.29a + miR.376a* + miR.518c + miR.639 + miR.660 | 0,79 | 0,057213186 |
| miR.558 + miR.609 + miR.29a + miR.362 + miR.376a* + miR.494 + miR.518c + miR.660 | 0,79 | 0,027088602 |
| miR.609 + miR.603 + miR.29a + miR.369.3p + miR.376a* + miR.494 + miR.518c + miR.338 | 0,79 | 0,102702923 |
| miR.558 + miR.609 + miR.603 + miR.520f + miR.220a + miR.376a* + miR.518c + miR.639 | 0,79 | 0,155884459 |
| miR.609 + miR.603 + miR.29a + miR.362 + miR.369.3p + miR.376a* + miR.518c + miR.660 | 0,79 | 0,239347088 |
| miR.609 + miR.603 + miR.220a + miR.130a + miR.29a + miR.376a* + miR.494 + miR.518c | 0,79 | 0,018685042 |
| miR.609 + miR.520f + miR.220a + miR.26a + miR.29a + miR.369.3p + miR.376a* + miR.518c | 0,79 | 0,411255671 |
| miR.558 + miR.609 + miR.519b + miR.29a + miR.376a* + miR.518c + miR.639 + miR.338 | 0,79 | 0,063912679 |
| miR.558 + miR.609 + miR.520f + miR.130a + miR.376a* + miR.518c + miR.639 + miR.338 | 0,79 | 0,256645515 |
| miR.609 + miR.603 + miR.520f + miR.130a + miR.29a + miR.376a* + miR.518c + miR.639 | 0,79 | 0,037498021 |
| miR.609 + miR.220a + miR.26a + miR.29a + miR.369.3p + miR.376a* + miR.494 + miR.518c | 0,79 | 0,316840421 |
| miR.558 + miR.609 + miR.29a + miR.376a* + miR.494 + miR.518c + miR.639 + miR.338 | 0,79 | 0,061686596 |
| miR.558 + miR.609 + miR.603 + miR.369.3p + miR.376a* + miR.518c + miR.639 + miR.338 | 0,79 | 0,218566902 |
| miR.558 + miR.609 + miR.519b + miR.29a + miR.362 + miR.376a* + miR.518c + miR.639 | 0,79 | 0,056849978 |
| miR.609 + miR.520f + miR.220a + miR.29a + miR.376a* + miR.494 + miR.518c + miR.639 | 0,79 | 0,170298444 |
| miR.609 + miR.520f + miR.220a + miR.29a + miR.369.3p + miR.376a* + miR.518c + miR.639 | 0,79 | 0,465899598 |
| miR.609 + miR.603 + miR.520f + miR.29a + miR.362 + miR.376a* + miR.518c + miR.639 | 0,79 | 0,070826007 |
| miR.609 + miR.520f + miR.220a + miR.519b + miR.29a + miR.376a* + miR.494 + miR.518c | 0,79 | 0,204368669 |
| miR.609 + miR.603 + miR.220a + miR.29a + miR.376a* + miR.494 + miR.518c + miR.338 | 0,79 | 0,034996557 |
| miR.609 + miR.520f + miR.220a + miR.26a + miR.29a + miR.376a* + miR.494 + miR.518c | 0,79 | 0,225911103 |
| miR.609 + miR.220a + miR.519b + miR.29a + miR.369.3p + miR.376a* + miR.518c + miR.660 | 0,79 | 0,328982051 |
| miR.558 + miR.609 + miR.520f + miR.130a + miR.369.3p + miR.376a* + miR.518c + miR.338 | 0,79 | 0,256583654 |
| miR.609 + miR.603 + miR.220a + miR.29a + miR.376a* + miR.518c + miR.639 + miR.338 | 0,79 | 0,025434948 |
| miR.609 + miR.520f + miR.220a + miR.130a + miR.29a + miR.362 + miR.376a* + miR.518c | 0,79 | 0,279953483 |
| miR.609 + miR.520f + miR.29a + miR.369.3p + miR.376a* + miR.494 + miR.518c + miR.338 | 0,79 | 0,189088526 |
| miR.558 + miR.609 + miR.603 + miR.520f + miR.130a + miR.369.3p + miR.376a* + miR.518c | 0,79 | 0,064198399 |
| miR.558 + miR.609 + miR.520f + miR.29a + miR.369.3p + miR.376a* + miR.639 + miR.338 | 0,79 | 0,278238064 |
| miR.609 + miR.603 + miR.26a + miR.29a + miR.369.3p + miR.376a* + miR.518c + miR.338 | 0,79 | 0,341314163 |
| miR.558 + miR.609 + miR.519b + miR.29a + miR.376a* + miR.494 + miR.518c + miR.639 | 0,79 | 0,040660192 |
| miR.609 + miR.603 + miR.220a + miR.130a + miR.29a + miR.376a* + miR.518c + miR.338 | 0,79 | 0,141246547 |
| miR.558 + miR.609 + miR.29a + miR.376a* + miR.518c + miR.639 + miR.660 + miR.338 | 0,79 | 0,134976955 |
| miR.558 + miR.609 + miR.29a + miR.362 + miR.376a* + miR.494 + miR.518c + miR.639 | 0,79 | 0,030368201 |
| miR.558 + miR.609 + miR.603 + miR.130a + miR.369.3p + miR.376a* + miR.518c + miR.639 | 0,79 | 0,163503941 |
| miR.609 + miR.603 + miR.220a + miR.130a + miR.29a + miR.376a* + miR.518c + miR.639 | 0,79 | 0,062054808 |
| miR.609 + miR.520f + miR.29a + miR.376a* + miR.494 + miR.518c + miR.660 + miR.338 | 0,79 | 0,231117069 |
| miR.558 + miR.609 + miR.603 + miR.26a + miR.369.3p + miR.376a* + miR.518c + miR.639 | 0,79 | 0,247509787 |
| miR.609 + miR.520f + miR.220a + miR.26a + miR.29a + miR.376a* + miR.518c + miR.660 | 0,79 | 0,246924086 |
| miR.558 + miR.609 + miR.520f + miR.369.3p + miR.376a* + miR.518c + miR.639 + miR.338 | 0,79 | 0,312469759 |
| miR.609 + miR.603 + miR.520f + miR.130a + miR.29a + miR.362 + miR.376a* + miR.518c | 0,79 | 0,048265991 |
| miR.558 + miR.609 + miR.519b + miR.29a + miR.376a* + miR.518c + miR.639 + miR.660 | 0,79 | 0,061297534 |
| miR.558 + miR.609 + miR.130a + miR.29a + miR.362 + miR.376a* + miR.518c + miR.338 | 0,79 | 0,0562700 86 |
| miR.558 + miR.609 + miR.520f + miR.130a + miR.29a + miR.369.3p + miR.376a* + miR.338 | 0,79 | 0,258763326 |
| miR.558 + miR.609 + miR.29a + miR.362 + miR.376a* + miR.518c + miR.660 + miR.338 | 0,79 | 0,025551434 |
| miR.609 + miR.603 + miR.29a + miR.362 + miR.369.3p + miR.376a* + miR.494 + miR.518c | 0,79 | 0,120634276 |
| miR.609 + miR.220a + miR.26a + miR.29a + miR.369.3p + miR.376a* + miR.518c + miR.660 | 0,79 | 0,457598987 |
| miR.609 + miR.603 + miR.520f + miR.130a + miR.29a + miR.376a* + miR.518c + miR.660 | 0,79 | 0,056117071 |
| miR.558 + miR.609 + miR.519b + miR.29a + miR.362 + miR.376a* + miR.518c + miR.660 | 0,79 | 0,019781247 |
| miR.609 + miR.603 + miR.520f + miR.519b + miR.29a + miR.376a* + miR.518c + miR.639 | 0,79 | 0,042751085 |
| miR.609 + miR.603 + miR.130a + miR.29a + miR.369.3p + miR.376a* + miR.494 + miR.518c | 0,79 | 0,042899006 |
| miR.558 + miR.609 + miR.603 + miR.520f + miR.220a + miR.130a + miR.376a* + miR.518c | 0,79 | 0,029829614 |
| miR.558 + miR.609 + miR.130a + miR.29a + miR.376a* + miR.518c + miR.660 + miR.338 | 0,79 | 0,042703307 |
| miR.609 + miR.603 + miR.520f + miR.29a + miR.362 + miR.376a* + miR.518c + miR.338 | 0,79 | 0,33965037 |
| miR.558 + miR.609 + miR.519b + miR.29a + miR.376a* + miR.518c + miR.660 + miR.338 | 0,79 | 0,043604576 |
| miR.609 + miR.603 + miR.519b + miR.29a + miR.369.3p + miR.376a* + miR.494 + miR.518c | 0,79 | 0,044786066 |
| miR.558 + miR.609 + miR.603 + miR.362 + miR.369.3p + miR.376a* + miR.518c + miR.639 | 0,79 | 0,139841484 |
| miR.558 + miR.609 + miR.520f + miR.519b + miR.29a + miR.376a* + miR.494 + miR.338 | 0,79 | 0,252724832 |
| miR.609 + miR.603 + miR.520f + miR.26a + miR.29a + miR.376a* + miR.518c + miR.338 | 0,79 | 0,250206184 |
| miR.558 + miR.609 + miR.519b + miR.29a + miR.362 + miR.376a* + miR.518c + miR.338 | 0,79 | 0,036084696 |
| miR.558 + miR.609 + miR.130a + miR.29a + miR.362 + miR.376a* + miR.518c + miR.660 | 0,79 | 0,027794919 |
| miR.609 + miR.603 + miR.519b + miR.29a + miR.369.3p + miR.376a* + miR.518c + miR.639 | 0,79 | 0,030735393 |
| miR.609 + miR.220a + miR.519b + miR.26a + miR.29a + miR.369.3p + miR.376a* + miR.518c | 0,79 | 0,471614629 |
| miR.558 + miR.609 + miR.519b + miR.130a + miR.29a + miR.376a* + miR.518c + miR.660 | 0,79 | 0,036027337 |
| miR.609 + miR.603 + miR.29a + miR.369.3p + miR.376a* + miR.518c + miR.639 + miR.660 | 0,79 | 0,047357012 |
| miR.609 + miR.603 + miR.29a + miR.362 + miR.369.3p + miR.376a* + miR.518c + miR.338 | 0,79 | 0,31970622 |
| miR.558 + miR.609 + miR.519b + miR.130a + miR.29a + miR.362 + miR.376a* + miR.518c | 0,79 | 0,043132144 |
| miR.609 + miR.603 + miR.520f + miR.26a + miR.29a + miR.376a* + miR.518c + miR.639 | 0,79 | 0,044595943 |
| miR.609 + miR.603 + miR.520f + miR.29a + miR.376a* + miR.518c + miR.660 + miR.338 | 0,79 | 0,281114861 |
| miR.558 + miR.609 + miR.519b + miR.130a + miR.29a + miR.376a* + miR.518c + miR.338 | 0,79 | 0,038646208 |
| miR.558 + miR.609 + miR.29a + miR.362 + miR.376a* + miR.494 + miR.518c + miR.338 | 0,79 | 0,083279685 |
| miR.558 + miR.609 + miR.603 + miR.369.3p + miR.376a* + miR.518c + miR.639 + miR.660 | 0,79 | 0,231557189 |
| miR.609 + miR.520f + miR.29a + miR.369.3p + miR.376a* + miR.494 + miR.518c + miR.660 | 0,79 | 0,114451385 |
| miR.558 + miR.609 + miR.603 + miR.220a + miR.26a + miR.29a + miR.518c + miR.660 | 0,79 | 0,031755053 |
| miR.609 + miR.603 + miR.130a + miR.29a + miR.369.3p + miR.376a* + miR.518c + miR.639 | 0,79 | 0,035254339 |
| miR.609 + miR.603 + miR.519b + miR.26a + miR.29a + miR.369.3p + miR.376a* + miR.518c | 0,79 | 0,056844182 |
| miR.609 + miR.603 + miR.520f + miR.130a + miR.26a + miR.29a + miR.376a* + miR.518c | 0,79 | 0,041982334 |
| miR.609 + miR.520f + miR.220a + miR.26a + miR.29a + miR.362 + miR.376a* + miR.518c | 0,79 | 0,40011922 |
| miR.558 + miR.609 + miR.519b + miR.29a + miR.376a* + miR.494 + miR.518c + miR.338 | 0,79 | 0,08561037 |
| miR.609 + miR.220a + miR.130a + miR.29a + miR.369.3p + miR.376a* + miR.518c + miR.660 | 0,79 | 0,29937593 |
| miR.609 + miR.603 + miR.26a + miR.29a + miR.362 + miR.369.3p + miR.376a* + miR.518c | 0,79 | 0,135631786 |
| miR.609 + miR.603 + miR.29a + miR.362 + miR.369.3p + miR.376a* + miR.518c + miR.639 | 0,79 | 0,090600075 |
| miR.558 + miR.609 + miR.603 + miR.520f + miR.29a + miR.369.3p + miR.376a* + miR.338 | 0,79 | 0,153718607 |
| miR.609 + miR.603 + miR.26a + miR.29a + miR.369.3p + miR.376a* + miR.518c + miR.660 | 0,79 | 0,043162093 |
| miR.558 + miR.609 + miR.520f + miR.29a + miR.376a* + miR.494 + miR.639 + miR.338 | 0,79 | 0,14820154 |
| miR.609 + miR.520f + miR.29a + miR.369.3p + miR.376a* + miR.494 + miR.518c + miR.639 | 0,79 | 0,095739415 |
| miR.609 + miR.603 + miR.29a + miR.369.3p + miR.376a* + miR.494 + miR.518c + miR.660 | 0,79 | 0,040154197 |
| miR.609 + miR.603 + miR.520f + miR.519b + miR.130a + miR.29a + miR.376a* + miR.518c | 0,79 | 0,044739482 |
| miR.609 + miR.520f + miR.29a + miR.376a* + miR.494 + miR.518c + miR.639 + miR.660 | 0,79 | 0,119345831 |
| miR.609 + miR.603 + miR.130a + miR.26a + miR.29a + miR.369.3p + miR.376a* + miR.518c | 0,79 | 0,031585691 |
| miR.558 + miR.609 + miR.519b + miR.29a + miR.362 + miR.376a* + miR.494 + miR.518c | 0,79 | 0,06162303 |
| miR.558 + miR.609 + miR.603 + miR.220a + miR.130a + miR.376a* + miR.518c + miR.639 | 0,79 | 0,047240347 |
| miR.558 + miR.609 + miR.603 + miR.519b + miR.369.3p + miR.376a* + miR.518c + miR.639 | 0,79 | 0,237565849 |
| miR.609 + miR.520f + miR.220a + miR.29a + miR.376a* + miR.518c + miR.660 + miR.338 | 0,79 | 0,340001773 |
| miR.609 + miR.603 + miR.520f + miR.26a + miR.29a + miR.362 + miR.376a* + miR.518c | 0,79 | 0,090374906 |
| miR.609 + miR.220a + miR.29a + miR.369.3p + miR.376a* + miR.518c + miR.639 + miR.660 | 0,79 | 0,397342617 |
| miR.558 + miR.609 + miR.520f + miR.519b + miR.29a + miR.369.3p + miR.376a* + miR.338 | 0,79 | 0,309152891 |
| miR.609 + miR.220a + miR.29a + miR.369.3p + miR.376a* + miR.518c + miR.660 + miR.338 | 0,79 | 0,380220712 |
| miR.609 + miR.520f + miR.220a + miR.519b + miR.29a + miR.376a* + miR.518c + miR.660 | 0,79 | 0,152635729 |
| miR.558 + miR.609 + miR.520f + miR.29a + miR.376a* + miR.494 + miR.660 + miR.338 | 0,79 | 0,301429516 |
| miR.609 + miR.520f + miR.220a + miR.29a + miR.376a* + miR.518c + miR.639 + miR.660 | 0,79 | 0,182807676 |
| miR.558 + miR.609 + miR.520f + miR.220a + miR.29a + miR.369.3p + miR.376a* + miR.338 | 0,79 | 0,185479846 |
| miR.558 + miR.609 + miR.520f + miR.26a + miR.29a + miR.369.3p + miR.376a* + miR.338 | 0,79 | 0,197426817 |
| miR.558 + miR.609 + miR.603 + miR.220a + miR.519b + miR.130a + miR.376a* + miR.518c | 0,79 | 0,07127935 |
| miR.609 + miR.603 + miR.29a + miR.369.3p + miR.376a* + miR.518c + miR.660 + miR.338 | 0,79 | 0,208196677 |
| miR.609 + miR.520f + miR.220a + miR.519b + miR.29a + miR.362 + miR.376a* + miR.518c | 0,79 | 0,359853699 |
| miR.558 + miR.609 + miR.29a + miR.376a* + miR.494 + miR.518c + miR.660 + miR.338 | 0,79 | 0,065888161 |
| miR.609 + miR.520f + miR.29a + miR.369.3p + miR.376a* + miR.518c + miR.660 + miR.338 | 0,79 | 0,340812349 |
| miR.609 + miR.520f + miR.519b + miR.29a + miR.369.3p + miR.376a* + miR.518c + miR.639 | 0,79 | 0,089042496 |
| miR.558 + miR.609 + miR.520f + miR.29a + miR.362 + miR.369.3p + miR.376a* + miR.660 | 0,79 | 0,294570752 |
| miR.558 + miR.609 + miR.603 + miR.520f + miR.26a + miR.376a* + miR.518c + miR.639 | 0,79 | 0,129760043 |
| miR.558 + miR.609 + miR.519b + miR.29a + miR.376a* + miR.494 + miR.518c + miR.660 | 0,79 | 0,042861313 |
| miR.609 + miR.603 + miR.519b + miR.29a + miR.362 + miR.369.3p + miR.376a* + miR.518c | 0,79 | 0,152183224 |
| miR.558 + miR.609 + miR.520f + miR.220a + miR.130a + miR.376a* + miR.518c + miR.338 | 0,79 | 0,201695924 |
| miR.609 + miR.520f + miR.220a + miR.29a + miR.362 + miR.376a* + miR.518c + miR.660 | 0,79 | 0,208199176 |
| miR.609 + miR.520f + miR.220a + miR.130a + miR.29a + miR.376a* + miR.518c + miR.338 | 0,79 | 0,280012662 |
| miR.609 + miR.603 + miR.520f + miR.220a + miR.130a + miR.369.3p + miR.376a* + miR.518c | 0,79 | 0,059917474 |
| miR.558 + miR.609 + miR.603 + miR.220a + miR.519b + miR.369.3p + miR.376a* + miR.518c | 0,79 | 0,066670565 |
| miR.558 + miR.609 + miR.520f + miR.29a + miR.362 + miR.369.3p + miR.376a* + miR.338 | 0,79 | 0,182667564 |
| miR.609 + miR.520f + miR.220a + miR.519b + miR.130a + miR.29a + miR.376a* + miR.518c | 0,79 | 0,18231478 |
| miR.609 + miR.520f + miR.220a + miR.29a + miR.362 + miR.376a* + miR.518c + miR.639 | 0,79 | 0,298952941 |
| miR.609 + miR.520f + miR.220a + miR.29a + miR.362 + miR.376a* + miR.518c + miR.338 | 0,79 | 0,379786737 |
| miR.609 + miR.520f + miR.26a + miR.29a + miR.376a* + miR.494 + miR.518c + miR.660 | 0,79 | 0,125015225 |
| miR.558 + miR.609 + miR.603 + miR.520f + miR.29a + miR.376a* + miR.494 + miR.338 | 0,79 | 0,088730013 |
| miR.558 + miR.609 + miR.520f + miR.130a + miR.369.3p + miR.376a* + miR.518c + miR.639 | 0,79 | 0,237171356 |
| miR.609 + miR.603 + miR.520f + miR.130a + miR.376a* + miR.518c + miR.639 + miR.338 | 0,79 | 0,09389186 |
| miR.609 + miR.603 + miR.520f + miR.519b + miR.29a + miR.376a* + miR.518c + miR.338 | 0,79 | 0,284157594 |
| miR.609 + miR.520f + miR.519b + miR.29a + miR.369.3p + miR.376a* + miR.518c + miR.338 | 0,79 | 0,252322389 |
| miR.609 + miR.520f + miR.519b + miR.29a + miR.369.3p + miR.376a* + miR.494 + miR.518c | 0,79 | 0,066610658 |
| miR.558 + miR.609 + miR.520f + miR.29a + miR.362 + miR.518c + miR.639 + miR.660 | 0,79 | 0,065060289 |
| miR.609 + miR.220a + miR.130a + miR.26a + miR.29a + miR.369.3p + miR.376a* + miR.518c | 0,79 | 0,367250572 |
| miR.609 + miR.603 + miR.519b + miR.29a + miR.369.3p + miR.376a* + miR.518c + miR.660 | 0,79 | 0,034723432 |
| miR.558 + miR.609 + miR.520f + miR.220a + miR.130a + miR.369.3p + miR.376a* + miR.518c | 0,79 | 0,090632124 |
| miR.609 + miR.603 + miR.220a + miR.130a + miR.369.3p + miR.376a* + miR.518c + miR.639 | 0,79 | 0,050903247 |
| miR.609 + miR.520f + miR.519b + miR.29a + miR.369.3p + miR.376a* + miR.518c + miR.660 | 0,79 | 0,057529682 |
| miR.609 + miR.520f + miR.220a + miR.130a + miR.26a + miR.29a + miR.376a* + miR.518c | 0,79 | 0,165732822 |
| miR.609 + miR.603 + miR.520f + miR.26a + miR.29a + miR.376a* + miR.518c + miR.660 | 0,79 | 0,059939163 |
| miR.558 + miR.609 + miR.520f + miR.29a + miR.362 + miR.376a* + miR.494 + miR.338 | 0,79 | 0,146847775 |
| miR.609 + miR.520f + miR.29a + miR.362 + miR.376a* + miR.494 + miR.518c + miR.338 | 0,79 | 0,183593874 |
| miR.609 + miR.220a + miR.519b + miR.29a + miR.369.3p + miR.376a* + miR.494 + miR.518c | 0,79 | 0,431925176 |
| miR.558 + miR.609 + miR.520f + miR.26a + miR.29a + miR.376a* + miR.494 + miR.338 | 0,79 | 0,111381504 |
| miR.609 + miR.603 + miR.520f + miR.519b + miR.29a + miR.362 + miR.376a* + miR.518c | 0,79 | 0,098953257 |
| miR.609 + miR.520f + miR.220a + miR.130a + miR.29a + miR.376a* + miR.518c + miR.639 | 0,79 | 0,174486634 |
| miR.558 + miR.609 + miR.520f + miR.220a + miR.130a + miR.376a* + miR.518c + miR.639 | 0,79 | 0,12723752 |
| miR.558 + miR.609 + miR.603 + miR.369.3p + miR.376a* + miR.494 + miR.518c + miR.639 | 0,79 | 0,25168833 |
| miR.558 + miR.609 + miR.520f + miR.130a + miR.29a + miR.376a* + miR.494 + miR.338 | 0,79 | 0,117744264 |
| miR.609 + miR.603 + miR.130a + miR.29a + miR.369.3p + miR.376a* + miR.518c + miR.338 | 0,79 | 0,117534401 |
| miR.558 + miR.609 + miR.520f + miR.376a* + miR.518c + miR.639 + miR.660 + miR.338 | 0,79 | 0,395905603 |
| miR.609 + miR.220a + miR.519b + miR.29a + miR.369.3p + miR.376a* + miR.518c + miR.639 | 0,79 | 0,496016522 |
| miR.558 + miR.609 + miR.520f + miR.220a + miR.29a + miR.376a* + miR.494 + miR.338 | 0,79 | 0,114259054 |
| miR.609 + miR.220a + miR.519b + miR.29a + miR.369.3p + miR.376a* + miR.518c + miR.338 | 0,79 | 0,383941349 |
| miR.609 + miR.220a + miR.26a + miR.29a + miR.369.3p + miR.376a* + miR.518c + miR.639 | 0,79 | 0,433126729 |
| miR.609 + miR.603 + miR.520f + miR.29a + miR.362 + miR.376a* + miR.518c + miR.660 | 0,79 | 0,060237243 |
| miR.609 + miR.603 + miR.220a + miR.130a + miR.26a + miR.369.3p + miR.376a* + miR.518c | 0,79 | 0,039528655 |
| miR.558 + miR.609 + miR.520f + miR.220a + miR.369.3p + miR.376a* + miR.518c + miR.639 | 0,79 | 0,166040329 |
| miR.609 + miR.603 + miR.130a + miR.29a + miR.362 + miR.369.3p + miR.376a* + miR.518c | 0,79 | 0,136175349 |
| miR.609 + miR.603 + miR.519b + miR.29a + miR.369.3p + miR.376a* + miR.518c + miR.338 | 0,79 | 0,197410181 |
| miR.609 + miR.220a + miR.26a + miR.29a + miR.369.3p + miR.376a* + miR.518c + miR.338 | 0,79 | 0,409586732 |
| miR.558 + miR.609 + miR.220a + miR.26a + miR.29a + miR.518c + miR.660 + miR.338 | 0,79 | 0,030944515 |
| miR.558 + miR.609 + miR.220a + miR.26a + miR.29a + miR.362 + miR.518c + miR.660 | 0,79 | 0,027952168 |
| miR.558 + miR.609 + miR.520f + miR.29a + miR.362 + miR.376a* + miR.494 + miR.660 | 0,79 | 0,124411527 |
| miR.609 + miR.520f + miR.26a + miR.29a + miR.369.3p + miR.376a* + miR.494 + miR.518c | 0,79 | 0,080840583 |
| miR.609 + miR.603 + miR.520f + miR.519b + miR.26a + miR.29a + miR.376a* + miR.518c | 0,79 | 0,058848822 |
| miR.609 + miR.220a + miR.519b + miR.29a + miR.362 + miR.369.3p + miR.376a* + miR.518c | 0,79 | 0,464343227 |
| miR.558 + miR.609 + miR.520f + miR.220a + miR.26a + miR.29a + miR.518c + miR.660 | 0,79 | 0,031137959 |
| miR.558 + miR.609 + miR.520f + miR.26a + miR.29a + miR.518c + miR.639 + miR.660 | 0,79 | 0,046148103 |
| miR.558 + miR.609 + miR.520f + miR.220a + miR.130a + miR.376a* + miR.518c + miR.660 | 0,79 | 0,054357431 |
| miR.609 + miR.520f + miR.26a + miR.29a + miR.376a* + miR.494 + miR.518c + miR.338 | 0,79 | 0,132907257 |
| miR.609 + miR.603 + miR.519b + miR.130a + miR.29a + miR.369.3p + miR.376a* + miR.518c | 0,79 | 0,034127363 |
| miR.558 + miR.609 + miR.520f + miR.29a + miR.369.3p + miR.376a* + miR.494 + miR.660 | 0,79 | 0,162971938 |
| miR.558 + miR.609 + miR.603 + miR.130a + miR.26a + miR.369.3p + miR.376a* + miR.518c | 0,79 | 0,100931545 |
| miR.558 + miR.609 + miR.520f + miR.220a + miR.29a + miR.362 + miR.518c + miR.660 | 0,79 | 0,056476805 |
| miR.558 + miR.609 + miR.603 + miR.130a + miR.369.3p + miR.376a* + miR.518c + miR.338 | 0,79 | 0,063090031 |
| miR.609 + miR.603 + miR.130a + miR.29a + miR.369.3p + miR.376a* + miR.518c + miR.660 | 0,79 | 0,033305231 |
| miR.609 + miR.603 + miR.26a + miR.29a + miR.376a* + miR.494 + miR.518c + miR.639 | 0,79 | 0,010451913 |
| miR.609 + miR.603 + miR.29a + miR.376a* + miR.494 + miR.518c + miR.639 + miR.338 | 0,79 | 0,02489557 |
| miR.609 + miR.603 + miR.130a + miR.29a + miR.376a* + miR.494 + miR.518c + miR.639 | 0,79 | 0,013238823 |
| miR.609 + miR.603 + miR.520f + miR.519b + miR.29a + miR.376a* + miR.518c + miR.660 | 0,79 | 0,056573042 |
| miR.558 + miR.609 + miR.520f + miR.29a + miR.362 + miR.369.3p + miR.518c + miR.660 | 0,79 | 0,032593838 |
| miR.609 + miR.520f + miR.130a + miR.29a + miR.369.3p + miR.376a* + miR.494 + miR.518c | 0,79 | 0,094907421 |
| miR.609 + miR.520f + miR.29a + miR.362 + miR.376a* + miR.494 + miR.518c + miR.660 | 0,79 | 0,071419167 |
| miR.558 + miR.609 + miR.603 + miR.520f + miR.29a + miR.369.3p + miR.376a* + miR.494 | 0,79 | 0,085192814 |
| miR.609 + miR.603 + miR.519b + miR.29a + miR.376a* + miR.494 + miR.518c + miR.639 | 0,79 | 0,007619869 |
| miR.609 + miR.520f + miR.519b + miR.29a + miR.376a* + miR.494 + miR.518c + miR.660 | 0,79 | 0,077672648 |
| miR.609 + miR.520f + miR.519b + miR.29a + miR.376a* + miR.494 + miR.518c + miR.639 | 0,79 | 0,051971571 |
| miR.558 + miR.609 + miR.603 + miR.520f + miR.519b + miR.130a + miR.376a* + miR.518c | 0,79 | 0,040154426 |
| miR.609 + miR.220a + miR.130a + miR.29a + miR.369.3p + miR.376a* + miR.494 + miR.518c | 0,79 | 0,330151427 |
| miR.558 + miR.609 + miR.603 + miR.130a + miR.362 + miR.369.3p + miR.376a* + miR.518c | 0,79 | 0,043811715 |
| miR.609 + miR.520f + miR.29a + miR.362 + miR.376a* + miR.494 + miR.518c + miR.639 | 0,79 | 0,114744713 |
| miR.558 + miR.609 + miR.520f + miR.130a + miR.29a + miR.518c + miR.639 + miR.660 | 0,79 | 0,078551074 |
| miR.558 + miR.609 + miR.603 + miR.520f + miR.376a* + miR.518c + miR.639 + miR.660 | 0,79 | 0,322852283 |
| miR.558 + miR.609 + miR.520f + miR.29a + miR.376a* + miR.639 + miR.660 + miR.338 | 0,79 | 0,387825763 |
| miR.609 + miR.520f + miR.519b + miR.29a + miR.376a* + miR.494 + miR.518c + miR.338 | 0,79 | 0,073800921 |
| miR.609 + miR.603 + miR.520f + miR.130a + miR.369.3p + miR.376a* + miR.518c + miR.639 | 0,79 | 0,077297966 |
| miR.609 + miR.603 + miR.29a + miR.376a* + miR.494 + miR.518c + miR.639 + miR.660 | 0,79 | 0,00710432 |
| miR.558 + miR.609 + miR.603 + miR.220a + miR.376a* + miR.518c + miR.639 + miR.338 | 0,79 | 0,070383796 |
| miR.609 + miR.520f + miR.519b + miR.130a + miR.29a + miR.369.3p + miR.376a* + miR.518c | 0,79 | 0,087421804 |
| miR.609 + miR.603 + miR.520f + miR.130a + miR.369.3p + miR.376a* + miR.518c + miR.338 | 0,79 | 0,184327837 |
| miR.558 + miR.609 + miR.520f + miR.519b + miR.29a + miR.369.3p + miR.376a* + miR.494 | 0,79 | 0,128429369 |
| miR.558 + miR.609 + miR.520f + miR.130a + miR.376a* + miR.518c + miR.660 + miR.338 | 0,79 | 0,181948423 |
| miR.558 + miR.609 + miR.220a + miR.130a + miR.26a + miR.29a + miR.518c + miR.660 | 0,79 | 0,017902511 |
| miR.558 + miR.609 + miR.603 + miR.220a + miR.519b + miR.376a* + miR.518c + miR.639 | 0,79 | 0,085472418 |
| miR.558 + miR.609 + miR.603 + miR.520f + miR.130a + miR.376a* + miR.494 + miR.518c | 0,79 | 0,022805475 |
| miR.558 + miR.609 + miR.520f + miR.130a + miR.376a* + miR.494 + miR.518c + miR.338 | 0,79 | 0,144650307 |
| miR.558 + miR.609 + miR.603 + miR.520f + miR.29a + miR.362 + miR.518c + miR.660 | 0,79 | 0,042956133 |
| miR.609 + miR.520f + miR.220a + miR.26a + miR.29a + miR.376a* + miR.518c + miR.338 | 0,79 | 0,283619468 |
| miR.558 + miR.609 + miR.603 + miR.520f + miR.26a + miR.29a + miR.518c + miR.660 | 0,79 | 0,031415816 |
| miR.609 + miR.220a + miR.29a + miR.369.3p + miR.376a* + miR.494 + miR.518c + miR.639 | 0,79 | 0,362071428 |
| miR.558 + miR.609 + miR.603 + miR.520f + miR.376a* + miR.494 + miR.518c + miR.639 | 0,79 | 0,213697254 |
| miR.558 + miR.609 + miR.603 + miR.520f + miR.130a + miR.376a* + miR.518c + miR.660 | 0,79 | 0,031045783 |
| miR.609 + miR.520f + miR.220a + miR.519b + miR.29a + miR.376a* + miR.518c + miR.338 | 0,79 | 0,258225028 |
| miR.558 + miR.609 + miR.603 + miR.520f + miR.130a + miR.362 + miR.376a* + miR.518c | 0,79 | 0,02212113 |
| miR.558 + miR.609 + miR.520f + miR.519b + miR.130a + miR.376a* + miR.518c + miR.338 | 0,79 | 0,170310922 |
| miR.558 + miR.609 + miR.603 + miR.520f + miR.130a + miR.26a + miR.376a* + miR.518c | 0,79 | 0,024719357 |
| miR.609 + miR.520f + miR.220a + miR.519b + miR.26a + miR.29a + miR.376a* + miR.518c | 0,79 | 0,204155936 |
| miR.558 + miR.609 + miR.603 + miR.520f + miR.29a + miR.518c + miR.639 + miR.660 | 0,79 | 0,071267072 |
| miR.609 + miR.520f + miR.26a + miR.29a + miR.376a* + miR.494 + miR.518c + miR.639 | 0,79 | 0,104625293 |
| miR.609 + miR.603 + miR.29a + miR.362 + miR.376a* + miR.494 + miR.518c + miR.639 | 0,79 | 0,009163906 |
| miR.609 + miR.520f + miR.29a + miR.362 + miR.369.3p + miR.376a* + miR.494 + miR.518c | 0,79 | 0,073238708 |
| miR.558 + miR.609 + miR.520f + miR.369.3p + miR.376a* + miR.518c + miR.639 + miR.660 | 0,79 | 0,223363007 |
| miR.558 + miR.609 + miR.520f + miR.130a + miR.26a + miR.376a* + miR.518c + miR.639 | 0,79 | 0,090110171 |
| miR.558 + miR.609 + miR.520f + miR.130a + miR.376a* + miR.518c + miR.639 + miR.660 | 0,79 | 0,208790148 |
| miR.609 + miR.220a + miR.29a + miR.369.3p + miR.376a* + miR.494 + miR.518c + miR.338 | 0,79 | 0,309376449 |

**Table 9: clusters of 9 miRNAs**

| **Cluster of 9 miRs** | **c_tau level** | **pv compared to top signature miR.558 + miR.609 + miR.603 + miR.220a + miR.26a + miR.29a + miR.369. 3p + miR.376a * + miR.518c** |
|---|---|---|
| miR.558 + miR.609 + miR.603 + miR.220a + miR.26a + miR.29a + miR.369.3p + miR.376a* + miR.518c | 0,81 | NaN |
| miR.558 + miR.609 + miR.603 + miR.220a + miR.29a + miR.369.3p + miR.376a* + miR.518c + miR.660 | 0,81 | 0,405184155 |
| miR.558 + miR.609 + miR.603 + miR.220a + miR.519b + miR.29a + miR.369.3p + miR.376a* + miR.518c | 0,81 | 0,246448719 |
| miR.558 + miR.609 + miR.603 + miR.520f + miR.220a + miR.29a + miR.369.3p + miR.376a* + miR.518c | 0,81 | 0,196840946 |
| miR.558 + miR.609 + miR.520f + miR.220a + miR.29a + miR.369.3p + miR.376a* + miR.518c + miR.660 | 0,81 | 0,329909732 |
| miR.558 + miR.609 + miR.220a + miR.26a + miR.29a + miR.369.3p + miR.376a* + miR.518c + miR.660 | 0,81 | 0,128466451 |
| miR.558 + miR.609 + miR.603 + miR.220a + miR.130a + miR.29a + miR.369.3p + miR.376a* + miR.518c | 0,81 | 0,116317947 |
| miR.558 + miR.609 + miR.603 + miR.220a + miR.519b + miR.29a + miR.362 + miR.376a* + miR.518c | 0,81 | 0,447554582 |
| miR.558 + miR.609 + miR.603 + miR.220a + miR.26a + miR.29a + miR.362 + miR.376a* + miR.518c | 0,81 | 0,059893333 |
| miR.558 + miR.609 + miR.603 + miR.520f + miR.29a + miR.369.3p + miR.376a* + miR.518c + miR.338 | 0,81 | 0,463029319 |
| miR.558 + miR.609 + miR.603 + miR.220a + miR.26a + miR.29a + miR.376a* + miR.518c + miR.660 | 0,81 | 0,039588187 |
| miR.558 + miR.609 + miR.603 + miR.220a + miR.26a + miR.29a + miR.376a* + miR.518c + miR.639 | 0,81 | 0,012535164 |
| miR.558 + miR.609 + miR.520f + miR.220a + miR.29a + miR.369.3p + miR.376a* + miR.518c + miR.338 | 0,81 | 0,400683559 |
| miR.558 + miR.609 + miR.220a + miR.29a + miR.362 + miR.369.3p + miR.376a* + miR.518c + miR.660 | 0,81 | 0,311774714 |
| miR.558 + miR.609 + miR.220a + miR.26a + miR.29a + miR.369.3p + miR.376a* + miR.518c + miR.338 | 0,81 | 0,110070757 |
| miR.558 + miR.609 + miR.603 + miR.220a + miR.519b + miR.26a + miR.29a + miR.376a* + miR.518c | 0,81 | 0,139809243 |
| miR.558 + miR.609 + miR.603 + miR.220a + miR.29a + miR.362 + miR.369.3p + miR.376a* + miR.518c | 0,81 | 0,190392203 |
| miR.558 + miR.609 + miR.603 + miR.220a + miR.29a + miR.369.3p + miR.376a* + miR.518c + miR.639 | 0,81 | 0,081427064 |
| miR.558 + miR.609 + miR.603 + miR.220a + miR.26a + miR.29a + miR.376a* + miR.518c + miR.338 | 0,81 | 0,048680066 |
| miR.558 + miR.609 + miR.603 + miR.220a + miR.29a + miR.369.3p + miR.376a* + miR.518c + miR.338 | 0,81 | 0,186226373 |
| miR.558 + miR.609 + miR.603 + miR.520f + miR.220a + miR.29a + miR.362 + miR.376a* + miR.518c | 0,81 | 0,09905613 |
| miR.558 + miR.609 + miR.603 + miR.220a + miR.29a + miR.369.3p + miR.376a* + miR.494 + miR.518c | 0,81 | 0,130611427 |
| miR.558 + miR.609 + miR.603 + miR.220a + miR.26a + miR.29a + miR.376a* + miR.494 + miR.518c | 0,81 | 0,005338207 |
| miR.558 + miR.609 + miR.220a + miR.26a + miR.29a + miR.369.3p + miR.376a* + miR.494 + miR.518c | 0,81 | 0,320717135 |
| miR.558 + miR.609 + miR.520f + miR.29a + miR.369.3p + miR.376a* + miR.518c + miR.660 + miR.338 | 0,81 | 0,42529961 |
| miR.558 + miR.609 + miR.520f + miR.220a + miR.26a + miR.29a + miR.369.3p + miR.376a* + miR.518c | 0,81 | 0,334919585 |
| miR.558 + miR.609 + miR.603 + miR.520f + miR.29a + miR.369.3p + miR.376a* + miR.518c + miR.639 | 0,81 | 0,067091161 |
| miR.558 + miR.609 + miR.603 + miR.220a + miR.130a + miR.26a + miR.29a + miR.376a* + miR.518c | 0,81 | 0,014626567 |
| miR.558 + miR.609 + miR.520f + miR.220a + miR.29a + miR.362 + miR.369.3p + miR.376a* + miR.518c | 0,81 | 0,495987113 |
| miR.558 + miR.609 + miR.220a + miR.26a + miR.29a + miR.362 + miR.369.3p + miR.376a* + miR.518c | 0,81 | 0,221787351 |
| miR.558 + miR.609 + miR.603 + miR.520f + miR.220a + miR.29a + miR.376a* + miR.518c + miR.660 | 0,81 | 0,099997395 |
| miR.558 + miR.609 + miR.220a + miR.26a + miR.29a + miR.369.3p + miR.376a* + miR.518c + miR.639 | 0,81 | 0,329506061 |
| miR.558 + miR.609 + miR.603 + miR.220a + miR.519b + miR.29a + miR.376a* + miR.494 + miR.518c | 0,81 | 0,324863522 |
| miR.558 + miR.609 + miR.603 + miR.520f + miR.220a + miR.29a + miR.376a* + miR.518c + miR.338 | 0,81 | 0,127330549 |
| miR.558 + miR.609 + miR.603 + miR.520f + miR.29a + miR.369.3p + miR.376a* + miR.518c + miR.660 | 0,81 | 0,204887351 |
| miR.558 + miR.609 + miR.220a + miR.29a + miR.369.3p + miR.376a* + miR.494 + miR.518c + miR.660 | 0,81 | 0,427310852 |
| miR.558 + miR.609 + miR.603 + miR.26a + miR.29a + miR.369.3p + miR.376a* + miR.518c + miR.639 | 0,81 | 0,232508147 |
| miR.558 + miR.609 + miR.603 + miR.26a + miR.29a + miR.369.3p + miR.376a* + miR.518c + miR.338 | 0,81 | 0,426798872 |
| miR.558 + miR.609 + miR.603 + miR.520f + miR.220a + miR.26a + miR.29a + miR.376a* + miR.518c | 0,81 | 0,015632232 |
| miR.558 + miR.609 + miR.220a + miR.29a + miR.369.3p + miR.376a* + miR.518c + miR.639 + miR.660 | 0,81 | 0,406477895 |
| miR.558 + miR.609 + miR.603 + miR.520f + miR.220a + miR.519b + miR.29a + miR.376a* + miR.518c | 0,81 | 0,107863762 |
| miR.558 + miR.609 + miR.220a + miR.519b + miR.29a + miR.369.3p + miR.376a* + miR.518c + miR.660 | 0,81 | 0,378455134 |
| miR.558 + miR.609 + miR.520f + miR.220a + miR.29a + miR.376a* + miR.518c + miR.660 + miR.338 | 0,81 | 0,354575192 |
| miR.558 + miR.609 + miR.220a + miR.29a + miR.369.3p + miR.376a* + miR.518c + miR.660 + miR.338 | 0,81 | 0,380394727 |
| miR.558 + miR.609 + miR.520f + miR.220a + miR.29a + miR.369.3p + miR.376a* + miR.494 + miR.518c | 0,81 | 0,399172056 |
| miR.558 + miR.609 + miR.520f + miR.220a + miR.29a + miR.376a* + miR.494 + miR.518c + miR.660 | 0,81 | 0,207190665 |
| miR.558 + miR.609 + miR.220a + miR.519b + miR.26a + miR.29a + miR.369.3p + miR.376a* + miR.518c | 0,81 | 0,296070608 |
| miR.558 + miR.609 + miR.520f + miR.220a + miR.29a + miR.369.3p + miR.376a* + miR.518c + miR.639 | 0,81 | 0,409887674 |
| miR.558 + miR.609 + miR.603 + miR.520f + miR.26a + miR.29a + miR.369.3p + miR.376a* + miR.518c | 0,81 | 0,065157593 |
| miR.558 + miR.609 + miR.603 + miR.220a + miR.519b + miR.29a + miR.376a* + miR.518c + miR.338 | 0,81 | 0,379025909 |
| miR.558 + miR.609 + miR.220a + miR.130a + miR.26a + miR.29a + miR.369.3p + miR.376a* + miR.518c | 0,81 | 0,237552871 |
| miR.558 + miR.609 + miR.603 + miR.520f + miR.220a + miR.29a + miR.376a* + miR.518c + miR.639 | 0,81 | 0,024425329 |
| miR.558 + miR.609 + miR.520f + miR.29a + miR.369.3p + miR.376a* + miR.518c + miR.639 + miR.338 | 0,81 | 0,413149685 |
| miR.558 + miR.609 + miR.220a + miR.130a + miR.29a + miR.369.3p + miR.376a* + miR.518c + miR.660 | 0,81 | 0,481305199 |
| miR.558 + miR.609 + miR.603 + miR.220a + miR.519b + miR.29a + miR.376a* + miR.518c + miR.639 | 0,81 | 0,259947051 |
| miR.558 + miR.609 + miR.520f + miR.29a + miR.376a* + miR.518c + miR.639 + miR.660 + miR.338 | 0,81 | 0,460534976 |
| miR.558 + miR.609 + miR.603 + miR.220a + miR.519b + miR.130a + miR.29a + miR.376a* + miR.518c | 0,81 | 0,24708209 |
| miR.558 + miR.609 + miR.603 + miR.26a + miR.29a + miR.369.3p + miR.376a* + miR.494 + miR.518c | 0,81 | 0,257662026 |
| miR.558 + miR.609 + miR.603 + miR.220a + miR.519b + miR.29a + miR.376a* + miR.518c + miR.660 | 0,81 | 0,424422464 |
| miR.558 + miR.609 + miR.520f + miR.220a + miR.130a + miR.29a + miR.369.3p + miR.376a* + miR.518c | 0,81 | 0,479425443 |
| miR.558 + miR.609 + miR.603 + miR.220a + miR.29a + miR.362 + miR.376a* + miR.518c + miR.639 | 0,81 | 0,13589269 |
| miR.558 + miR.609 + miR.520f + miR.29a + miR.376a* + miR.494 + miR.518c + miR.660 + miR.338 | 0,81 | 0,36967044 |
| miR.558 + miR.609 + miR.603 + miR.520f + miR.29a + miR.369.3p + miR.376a* + miR.494 + miR.518c | 0,81 | 0,089395994 |
| miR.558 + miR.609 + miR.520f + miR.220a + miR.519b + miR.29a + miR.369.3p + miR.376a* + miR.518c | 0,81 | 0,298435504 |
| miR.558 + miR.609 + miR.603 + miR.520f + miR.220a + miR.29a + miR.376a* + miR.494 + miR.518c | 0,81 | 0,010543759 |
| miR.558 + miR.609 + miR.520f + miR.220a + miR.29a + miR.362 + miR.376a* + miR.518c + miR.338 | 0,81 | 0,480715861 |
| miR.558 + miR.609 + miR.603 + miR.220a + miR.130a + miR.29a + miR.362 + miR.376a* + miR.518c | 0,81 | 0,081844216 |
| miR.558 + miR.609 + miR.520f + miR.29a + miR.369.3p + miR.376a* + miR.494 + miR.518c + miR.338 | 0,81 | 0,342503816 |
| miR.558 + miR.609 + miR.603 + miR.520f + miR.29a + miR.362 + miR.369.3p + miR.376a* + miR.518c | 0,81 | 0,098699779 |
| miR.558 + miR.609 + miR.603 + miR.220a + miR.29a + miR.362 + miR.376a* + miR.518c + miR.338 | 0,81 | 0,164711094 |
| miR.558 + miR.609 + miR.603 + miR.26a + miR.29a + miR.369.3p + miR.376a* + miR.518c + miR.660 | 0,81 | 0,218296942 |
| miR.558 + miR.609 + miR.603 + miR.520f + miR.130a + miR.29a + miR.369.3p + miR.376a* + miR.518c | 0,81 | 0,092864134 |
| miR.558 + miR.609 + miR.603 + miR.520f + miR.519b + miR.29a + miR.369.3p + miR.376a* + miR.518c | 0,81 | 0,114699091 |
| miR.558 + miR.609 + miR.520f + miR.220a + miR.29a + miR.376a* + miR.518c + miR.639 + miR.660 | 0,81 | 0,303649269 |
| miR.558 + miR.609 + miR.220a + miR.130a + miR.29a + miR.369.3p + miR.376a* + miR.518c + miR.639 | 0,81 | 0,297064743 |
| miR.558 + miR.609 + miR.603 + miR.520f + miR.29a + miR.376a* + miR.518c + miR.639 + miR.338 | 0,81 | 0,1815712 |
| miR.558 + miR.609 + miR.520f + miR.220a + miR.29a + miR.362 + miR.376a* + miR.494 + miR.518c | 0,81 | 0,21918321 |
| miR.558 + miR.609 + miR.520f + miR.220a + miR.29a + miR.362 + miR.376a* + miR.518c + miR.639 | 0,81 | 0,263170677 |
| miR.558 + miR.609 + miR.220a + miR.26a + miR.29a + miR.362 + miR.376a* + miR.494 + miR.518c | 0,81 | 0,249212767 |
| miR.558 + miR.609 + miR.520f + miR.26a + miR.29a + miR.369.3p + miR.376a* + miR.518c + miR.338 | 0,81 | 0,488093002 |
| miR.558 + miR.609 + miR.603 + miR.130a + miR.26a + miR.29a + miR.369.3p + miR.376a* + miR.518c | 0,81 | 0,174142711 |
| miR.558 + miR.609 + miR.520f + miR.220a + miR.519b + miR.29a + miR.362 + miR.376a* + miR.518c | 0,81 | 0,425943522 |
| miR.558 + miR.609 + miR.603 + miR.220a + miR.29a + miR.376a* + miR.518c + miR.639 + miR.660 | 0,81 | 0,08633331 |
| miR.558 + miR.609 + miR.603 + miR.519b + miR.26a + miR.29a + miR.369.3p + miR.376a* + miR.518c | 0,81 | 0,136399791 |
| miR.558 + miR.609 + miR.220a + miR.519b + miR.130a + miR.29a + miR.369.3p + miR.376a* + miR.518c | 0,81 | 0,368636506 |
| miR.558 + miR.609 + miR.603 + miR.26a + miR.29a + miR.362 + miR.369.3p + miR.376a* + miR.518c | 0,81 | 0,216977335 |
| miR.558 + miR.609 + miR.520f + miR.130a + miR.29a + miR.369.3p + miR.376a* + miR.518c + miR.338 | 0,81 | 0,35581821 |
| miR.558 + miR.609 + miR.520f + miR.29a + miR.369.3p + miR.376a* + miR.518c + miR.639 + miR.660 | 0,81 | 0,363334394 |
| miR.558 + miR.609 + miR.520f + miR.220a + miR.519b + miR.29a + miR.376a* + miR.518c + miR.660 | 0,81 | 0,264417081 |
| miR.558 + miR.609 + miR.603 + miR.130a + miR.29a + miR.369.3p + miR.376a* + miR.518c + miR.639 | 0,81 | 0,10961265 |
| miR.558 + miR.609 + miR.520f + miR.29a + miR.362 + miR.369.3p + miR.376a* + miR.518c + miR.338 | 0,81 | 0,348273131 |
| miR.558 + miR.609 + miR.603 + miR.520f + miR.220a + miR.130a + miR.29a + miR.376a* + miR.518c | 0,8 | 0,020094622 |
| miR.558 + miR.609 + miR.520f + miR.220a + miR.26a + miR.29a + miR.376a* + miR.518c + miR.660 | 0,8 | 0,162238347 |
| miR.558 + miR.609 + miR.520f + miR.29a + miR.369.3p + miR.376a* + miR.494 + miR.518c + miR.660 | 0,8 | 0,224746043 |
| miR.558 + miR.609 + miR.520f + miR.220a + miR.26a + miR.29a + miR.362 + miR.376a* + miR.518c | 0,8 | 0,232798065 |
| miR.558 + miR.609 + miR.603 + miR.220a + miR.130a + miR.29a + miR.376a* + miR.518c + miR.660 | 0,8 | 0,048798642 |
| miR.558 + miR.609 + miR.520f + miR.220a + miR.130a + miR.29a + miR.362 + miR.376a* + miR.518c | 0,8 | 0,264567693 |
| miR.558 + miR.609 + miR.603 + miR.220a + miR.29a + miR.376a* + miR.518c + miR.660 + miR.338 | 0,8 | 0,100789797 |
| miR.558 + miR.609 + miR.520f + miR.220a + miR.29a + miR.362 + miR.376a* + miR.518c + miR.660 | 0,8 | 0,204969048 |
| miR.558 + miR.609 + miR.520f + miR.220a + miR.29a + miR.376a* + miR.494 + miR.518c + miR.338 | 0,8 | 0,224810798 |
| miR.558 + miR.609 + miR.603 + miR.220a + miR.29a + miR.362 + miR.376a* + miR.518c + miR.660 | 0,8 | 0,075776502 |
| miR.558 + miR.609 + miR.603 + miR.520f + miR.29a + miR.362 + miR.376a* + miR.518c + miR.639 | 0,8 | 0,092813236 |
| miR.558 + miR.609 + miR.220a + miR.130a + miR.29a + miR.369.3p + miR.376a* + miR.494 + miR.518c | 0,8 | 0,296308912 |
| miR.558 + miR.609 + miR.520f + miR.220a + miR.130a + miR.29a + miR.376a* + miR.518c + miR.660 | 0,8 | 0,195599244 |
| miR.558 + miR.609 + miR.520f + miR.519b + miR.29a + miR.369.3p + miR.376a* + miR.518c + miR.338 | 0,8 | 0,479936725 |
| miR.558 + miR.609 + miR.603 + miR.520f + miR.29a + miR.376a* + miR.494 + miR.518c + miR.338 | 0,8 | 0,211200269 |
| miR.558 + miR.609 + miR.520f + miR.29a + miR.362 + miR.376a* + miR.518c + miR.660 + miR.338 | 0,8 | 0,327412429 |
| miR.558 + miR.609 + miR.603 + miR.520f + miR.29a + miR.362 + miR.376a* + miR.518c + miR.338 | 0,8 | 0,331718628 |
| miR.558 + miR.609 + miR.220a + miR.26a + miR.29a + miR.376a* + miR.494 + miR.518c + miR.660 | 0,8 | 0,215177023 |
| miR.558 + miR.609 + miR.520f + miR.26a + miR.29a + miR.376a* + miR.518c + miR.660 + miR.338 | 0,8 | 0,342947707 |
| miR.558 + miR.609 + miR.603 + miR.520f + miR.29a + miR.376a* + miR.518c + miR.639 + miR.660 | 0,8 | 0,215973331 |
| miR.558 + miR.609 + miR.603 + miR.520f + miR.29a + miR.376a* + miR.494 + miR.518c + miR.639 | 0,8 | 0,016870957 |
| miR.558 + miR.609 + miR.520f + miR.29a + miR.369.3p + miR.376a* + miR.494 + miR.518c + miR.639 | 0,8 | 0,115598303 |
| miR.558 + miR.609 + miR.520f + miR.220a + miR.519b + miR.29a + miR.376a* + miR.494 + miR.518c | 0,8 | 0,232468645 |
| miR.558 + miR.609 + miR.603 + miR.520f + miR.29a + miR.376a* + miR.518c + miR.660 + miR.338 | 0,8 | 0,373285911 |
| miR.558 + miR.609 + miR.603 + miR.220a + miR.29a + miR.376a* + miR.494 + miR.518c + miR.660 | 0,8 | 0,104374757 |
| miR.558 + miR.609 + miR.220a + miR.519b + miR.26a + miR.29a + miR.376a* + miR.494 + miR.518c | 0,8 | 0,279417062 |
| miR.558 + miR.609 + miR.603 + miR.520f + miR.519b + miR.29a + miR.376a* + miR.494 + miR.518c | 0,8 | 0,062938142 |
| miR.558 + miR.609 + miR.520f + miR.220a + miR.29a + miR.376a* + miR.518c + miR.639 + miR.338 | 0,8 | 0,236017156 |
| miR.558 + miR.609 + miR.220a + miR.26a + miR.29a + miR.376a* + miR.494 + miR.518c + miR.338 | 0,8 | 0,221173558 |
| miR.558 + miR.609 + miR.220a + miR.519b + miR.26a + miR.29a + miR.362 + miR.376a* + miR.518c | 0,8 | 0,252819696 |
| miR.558 + miR.609 + miR.520f + miR.29a + miR.362 + miR.369.3p + miR.376a* + miR.518c + miR.660 | 0,8 | 0,285434425 |
| miR.558 + miR.609 + miR.603 + miR.29a + miR.362 + miR.369.3p + miR.376a* + miR.518c + miR.660 | 0,8 | 0,259589577 |
| miR.558 + miR.609 + miR.603 + miR.220a + miR.130a + miR.29a + miR.376a* + miR.494 + miR.518c | 0,8 | 0,006847963 |
| miR.558 + miR.609 + miR.520f + miR.220a + miR.519b + miR.29a + miR.376a* + miR.518c + miR.338 | 0,8 | 0,390803298 |
| miR.558 + miR.609 + miR.520f + miR.220a + miR.130a + miR.29a + miR.376a* + miR.518c + miR.338 | 0,8 | 0,308429664 |
| miR.558 + miR.609 + miR.520f + miR.130a + miR.29a + miR.376a* + miR.518c + miR.639 + miR.660 | 0,8 | 0,172455922 |
| miR.558 + miR.609 + miR.520f + miR.519b + miR.29a + miR.376a* + miR.518c + miR.660 + miR.338 | 0,8 | 0,351658298 |
| miR.558 + miR.609 + miR.220a + miR.26a + miR.29a + miR.362 + miR.376a* + miR.518c + miR.338 | 0,8 | 0,35269588 |
| miR.558 + miR.609 + miR.220a + miR.26a + miR.29a + miR.362 + miR.376a* + miR.518c + miR.639 | 0,8 | 0,170694315 |
| miR.558 + miR.609 + miR.220a + miR.130a + miR.29a + miR.369.3p + miR.376a* + miR.518c + miR.338 | 0,8 | 0,373269862 |
| miR.558 + miR.609 + miR.220a + miR.130a + miR.26a + miR.29a + miR.376a* + miR.494 + miR.518c | 0,8 | 0,20149379 |
| miR.558 + miR.609 + miR.220a + miR.519b + miR.29a + miR.362 + miR.369.3p + miR.376a* + miR.518c | 0,8 | 0,394859734 |
| miR.558 + miR.609 + miR.603 + miR.520f + miR.29a + miR.362 + miR.376a* + miR.494 + miR.518c | 0,8 | 0,078778091 |
| miR.558 + miR.609 + miR.603 + miR.29a + miR.369.3p + miR.376a* + miR.518c + miR.639 + miR.338 | 0,8 | 0,166924142 |
| miR.558 + miR.609 + miR.220a + miR.130a + miR.29a + miR.362 + miR.369.3p + miR.376a* + miR.518c | 0,8 | 0,237585098 |
| miR.558 + miR.609 + miR.520f + miR.220a + miR.26a + miR.29a + miR.376a* + miR.518c + miR.338 | 0,8 | 0,306992027 |
| miR.558 + miR.609 + miR.520f + miR.26a + miR.29a + miR.369.3p + miR.376a* + miR.518c + miR.660 | 0,8 | 0,184596775 |
| miR.558 + miR.609 + miR.520f + miR.26a + miR.29a + miR.369.3p + miR.376a* + miR.518c + miR.639 | 0,8 | 0,127457221 |
| miR.558 + miR.609 + miR.520f + miR.29a + miR.362 + miR.376a* + miR.518c + miR.639 + miR.338 | 0,8 | 0,314682404 |
| miR.558 + miR.609 + miR.220a + miR.130a + miR.26a + miR.29a + miR.362 + miR.376a* + miR.518c | 0,8 | 0,196230158 |
| miR.558 + miR.609 + miR.220a + miR.26a + miR.29a + miR.376a* + miR.518c + miR.639 + miR.660 | 0,8 | 0,246176262 |
| miR.609 + miR.603 + miR.520f + miR.220a + miR.29a + miR.369.3p + miR.376a* + miR.518c + miR.660 | 0,8 | 0,221039373 |
| miR.558 + miR.609 + miR.603 + miR.520f + miR.130a + miR.29a + miR.376a* + miR.518c + miR.338 | 0,8 | 0,25365842 |
| miR.558 + miR.609 + miR.220a + miR.26a + miR.29a + miR.376a* + miR.494 + miR.518c + miR.639 | 0,8 | 0,170983241 |
| miR.609 + miR.603 + miR.220a + miR.29a + miR.369.3p + miR.376a* + miR.494 + miR.518c + miR.660 | 0,8 | 0,474796754 |
| miR.558 + miR.609 + miR.520f + miR.29a + miR.376a* + miR.494 + miR.518c + miR.639 + miR.338 | 0,8 | 0,157646369 |
| miR.558 + miR.609 + miR.220a + miR.29a + miR.362 + miR.369.3p + miR.376a* + miR.518c + miR.639 | 0,8 | 0,175504569 |
| miR.558 + miR.609 + miR.520f + miR.220a + miR.130a + miR.29a + miR.376a* + miR.494 + miR.518c | 0,8 | 0,078147535 |
| miR.558 + miR.609 + miR.220a + miR.29a + miR.362 + miR.369.3p + miR.376a* + miR.494 + miR.518c | 0,8 | 0,190649471 |
| miR.558 + miR.609 + miR.520f + miR.220a + miR.26a + miR.29a + miR.376a* + miR.494 + miR.518c | 0,8 | 0,117757749 |
| miR.558 + miR.609 + miR.520f + miR.220a + miR.29a + miR.376a* + miR.494 + miR.518c + miR.639 | 0,8 | 0,071425949 |
| miR.609 + miR.603 + miR.220a + miR.29a + miR.362 + miR.369.3p + miR.376a* + miR.518c + miR.660 | 0,8 | 0,48849136 |
| miR.558 + miR.609 + miR.520f + miR.29a + miR.362 + miR.369.3p + miR.376a* + miR.518c + miR.639 | 0,8 | 0,131590258 |
| miR.558 + miR.609 + miR.603 + miR.520f + miR.26a + miR.29a + miR.376a* + miR.518c + miR.639 | 0,8 | 0,013949842 |
| miR.558 + miR.609 + miR.603 + miR.520f + miR.29a + miR.376a* + miR.494 + miR.518c + miR.660 | 0,8 | 0,074032881 |
| miR.558 + miR.609 + miR.520f + miR.130a + miR.29a + miR.369.3p + miR.376a* + miR.518c + miR.660 | 0,8 | 0,184603818 |
| miR.558 + miR.609 + miR.520f + miR.29a + miR.376a* + miR.494 + miR.518c + miR.639 + miR.660 | 0,8 | 0,189111019 |
| miR.558 + miR.609 + miR.220a + miR.519b + miR.29a + miR.369.3p + miR.376a* + miR.518c + miR.639 | 0,8 | 0,365639771 |
| miR.558 + miR.609 + miR.520f + miR.130a + miR.29a + miR.376a* + miR.494 + miR.518c + miR.660 | 0,8 | 0,079497418 |
| miR.558 + miR.609 + miR.220a + miR.26a + miR.29a + miR.376a* + miR.518c + miR.660 + miR.338 | 0,8 | 0,288432683 |
| miR.558 + miR.609 + miR.220a + miR.519b + miR.29a + miR.369.3p + miR.376a* + miR.518c + miR.338 | 0,8 | 0,391496807 |
| miR.558 + miR.609 + miR.520f + miR.220a + miR.26a + miR.29a + miR.376a* + miR.518c + miR.639 | 0,8 | 0,061782416 |
| miR.558 + miR.609 + miR.603 + miR.220a + miR.29a + miR.362 + miR.376a* + miR.494 + miR.518c | 0,8 | 0,121198281 |
| miR.558 + miR.609 + miR.220a + miR.29a + miR.362 + miR.369.3p + miR.376a* + miR.518c + miR.338 | 0,8 | 0,222233599 |
| miR.558 + miR.609 + miR.220a + miR.26a + miR.29a + miR.362 + miR.376a* + miR.518c + miR.660 | 0,8 | 0,188586623 |
| miR.558 + miR.609 + miR.220a + miR.519b + miR.26a + miR.29a + miR.376a* + miR.518c + miR.660 | 0,8 | 0,226750233 |
| miR.558 + miR.609 + miR.520f + miR.220a + miR.130a + miR.26a + miR.29a + miR.376a* + miR.518c | 0,8 | 0,098943942 |
| miR.558 + miR.609 + miR.603 + miR.520f + miR.130a + miR.29a + miR.376a* + miR.518c + miR.639 | 0,8 | 0,017256413 |
| miR.558 + miR.609 + miR.220a + miR.130a + miR.26a + miR.29a + miR.376a* + miR.518c + miR.660 | 0,8 | 0,176732056 |
| miR.558 + miR.609 + miR.520f + miR.26a + miR.29a + miR.369.3p + miR.376a* + miR.494 + miR.518c | 0,8 | 0,153093676 |
| miR.558 + miR.609 + miR.603 + miR.130a + miR.29a + miR.369.3p + miR.376a* + miR.494 + miR.518c | 0,8 | 0,074838999 |
| miR.558 + miR.609 + miR.603 + miR.520f + miR.519b + miR.29a + miR.376a* + miR.518c + miR.639 | 0,8 | 0,017218361 |
| miR.558 + miR.609 + miR.520f + miR.220a + miR.519b + miR.26a + miR.29a + miR.376a* + miR.518c | 0,8 | 0,151358087 |
| miR.558 + miR.609 + miR.603 + miR.520f + miR.519b + miR.29a + miR.362 + miR.376a* + miR.518c | 0,8 | 0,080317105 |
| miR.558 + miR.609 + miR.520f + miR.130a + miR.29a + miR.369.3p + miR.376a* + miR.518c + miR.639 | 0,8 | 0,134521813 |
| miR.558 + miR.609 + miR.520f + miR.519b + miR.29a + miR.369.3p + miR.376a* + miR.518c + miR.639 | 0,8 | 0,144018839 |
| miR.558 + miR.609 + miR.520f + miR.29a + miR.362 + miR.376a* + miR.494 + miR.518c + miR.338 | 0,8 | 0,328546416 |
| miR.558 + miR.609 + miR.603 + miR.220a + miR.29a + miR.376a* + miR.494 + miR.518c + miR.639 | 0,8 | 0,019253644 |
| miR.558 + miR.609 + miR.520f + miR.29a + miR.362 + miR.369.3p + miR.376a* + miR.494 + miR.518c | 0,8 | 0,106348993 |
| miR.558 + miR.609 + miR.603 + miR.520f + miR.26a + miR.29a + miR.376a* + miR.518c + miR.338 | 0,8 | 0,151958651 |
| miR.558 + miR.609 + miR.520f + miR.519b + miR.29a + miR.369.3p + miR.376a* + miR.518c + miR.660 | 0,8 | 0,182822237 |
| miR.558 + miR.609 + miR.520f + miR.130a + miR.29a + miR.376a* + miR.494 + miR.518c + miR.639 | 0,8 | 0,055727295 |
| miR.558 + miR.609 + miR.520f + miR.220a + miR.130a + miR.29a + miR.376a* + miR.518c + miR.639 | 0,8 | 0,086087625 |
| miR.558 + miR.609 + miR.603 + miR.220a + miR.29a + miR.376a* + miR.518c + miR.639 + miR.338 | 0,8 | 0,012546125 |
| miR.558 + miR.609 + miR.220a + miR.26a + miR.29a + miR.376a* + miR.518c + miR.639 + miR.338 | 0,8 | 0,185309344 |
| miR.558 + miR.609 + miR.130a + miR.26a + miR.29a + miR.369.3p + miR.376a* + miR.518c + miR.639 | 0,8 | 0,122208698 |
| miR.609 + miR.603 + miR.220a + miR.26a + miR.29a + miR.369.3p + miR.376a* + miR.494 + miR.518c | 0,8 | 0,427593084 |
| miR.558 + miR.609 + miR.603 + miR.220a + miR.130a + miR.29a + miR.376a* + miR.518c + miR.639 | 0,8 | 0,011082239 |
| miR.558 + miR.609 + miR.520f + miR.220a + miR.519b + miR.130a + miR.29a + miR.376a* + miR.518c | 0,8 | 0,136140854 |
| miR.558 + miR.609 + miR.603 + miR.29a + miR.369.3p + miR.376a* + miR.518c + miR.660 + miR.338 | 0,8 | 0,16699956 |
| miR.558 + miR.609 + miR.26a + miR.29a + miR.369.3p + miR.376a* + miR.518c + miR.639 + miR.338 | 0,8 | 0,429224314 |
| miR.558 + miR.609 + miR.603 + miR.520f + miR.130a + miR.29a + miR.362 + miR.376a* + miR.518c | 0,8 | 0,060663582 |
| miR.558 + miR.609 + miR.603 + miR.520f + miR.519b + miR.29a + miR.376a* + miR.518c + miR.338 | 0,8 | 0,265640636 |
| miR.558 + miR.609 + miR.603 + miR.220a + miR.130a + miR.29a + miR.376a* + miR.518c + miR.338 | 0,8 | 0,018996692 |
| miR.558 + miR.609 + miR.520f + miR.29a + miR.362 + miR.376a* + miR.494 + miR.518c + miR.639 | 0,8 | 0,115205009 |
| miR.558 + miR.609 + miR.603 + miR.26a + miR.29a + miR.376a* + miR.518c + miR.639 + miR.338 | 0,8 | 0,198914508 |
| miR.558 + miR.609 + miR.603 + miR.520f + miR.130a + miR.29a + miR.376a* + miR.494 + miR.518c | 0,8 | 0,032853655 |
| miR.558 + miR.609 + miR.603 + miR.220a + miR.29a + miR.376a* + miR.494 + miR.518c + miR.338 | 0,8 | 0,020442168 |
| miR.558 + miR.609 + miR.603 + miR.29a + miR.369.3p + miR.376a* + miR.518c + miR.639 + miR.660 | 0,8 | 0,296530549 |
| miR.558 + miR.609 + miR.220a + miR.519b + miR.29a + miR.369.3p + miR.376a* + miR.494 + miR.518c | 0,8 | 0,389441123 |
| miR.558 + miR.609 + miR.520f + miR.130a + miR.29a + miR.369.3p + miR.376a* + miR.494 + miR.518c | 0,8 | 0,136097883 |
| miR.609 + miR.603 + miR.220a + miR.26a + miR.29a + miR.369.3p + miR.376a* + miR.518c + miR.660 | 0,8 | 0,253503881 |
| miR.558 + miR.609 + miR.603 + miR.520f + miR.26a + miR.29a + miR.376a* + miR.494 + miR.518c | 0,8 | 0,029060967 |
| miR.558 + miR.609 + miR.220a + miR.519b + miR.130a + miR.29a + miR.376a* + miR.494 + miR.518c | 0,8 | 0,15282261 |
| miR.558 + miR.609 + miR.220a + miR.130a + miR.29a + miR.376a* + miR.494 + miR.518c + miR.660 | 0,8 | 0,124333642 |
| miR.558 + miR.609 + miR.520f + miR.519b + miR.29a + miR.369.3p + miR.376a* + miR.494 + miR.518c | 0,8 | 0,191950259 |
| miR.558 + miR.609 + miR.130a + miR.26a + miR.29a + miR.369.3p + miR.376a* + miR.494 + miR.518c | 0,8 | 0,069718 |
| miR.558 + miR.609 + miR.603 + miR.29a + miR.362 + miR.369.3p + miR.376a* + miR.518c + miR.338 | 0,8 | 0,119114353 |
| miR.558 + miR.609 + miR.603 + miR.130a + miR.29a + miR.369.3p + miR.376a* + miR.518c + miR.338 | 0,8 | 0,119451203 |
| miR.558 + miR.609 + miR.603 + miR.29a + miR.369.3p + miR.376a* + miR.494 + miR.518c + miR.639 | 0,8 | 0,102353955 |
| miR.609 + miR.603 + miR.220a + miR.519b + miR.29a + miR.369.3p + miR.376a* + miR.518c + miR.660 | 0,8 | 0,271375564 |
| miR.558 + miR.609 + miR.520f + miR.519b + miR.29a + miR.376a* + miR.494 + miR.518c + miR.338 | 0,8 | 0,224800326 |
| miR.558 + miR.609 + miR.603 + miR.29a + miR.369.3p + miR.376a* + miR.494 + miR.518c + miR.660 | 0,8 | 0,08274244 |
| miR.558 + miR.609 + miR.520f + miR.130a + miR.29a + miR.376a* + miR.518c + miR.660 + miR.338 | 0,8 | 0,363852303 |
| miR.558 + miR.609 + miR.603 + miR.520f + miR.26a + miR.29a + miR.362 + miR.376a* + miR.518c | 0,8 | 0,050927502 |
| miR.558 + miR.609 + miR.603 + miR.520f + miR.130a + miR.29a + miR.376a* + miR.518c + miR.660 | 0,8 | 0,092661585 |
| miR.558 + miR.609 + miR.520f + miR.130a + miR.29a + miR.362 + miR.376a* + miR.518c + miR.338 | 0,8 | 0,35349821 |
| miR.558 + miR.609 + miR.26a + miR.29a + miR.369.3p + miR.376a* + miR.494 + miR.518c + miR.338 | 0,8 | 0,373724854 |
| miR.558 + miR.609 + miR.520f + miR.26a + miR.29a + miR.362 + miR.369.3p + miR.376a* + miR.518c | 0,8 | 0,103714601 |
| miR.558 + miR.609 + miR.520f + miR.220a + miR.519b + miR.29a + miR.376a* + miR.518c + miR.639 | 0,8 | 0,111859043 |
| miR.609 + miR.603 + miR.220a + miR.29a + miR.369.3p + miR.376a* + miR.518c + miR.639 + miR.660 | 0,8 | 0,472103206 |
| miR.558 + miR.609 + miR.603 + miR.130a + miR.29a + miR.369.3p + miR.376a* + miR.518c + miR.660 | 0,8 | 0,085614049 |
| miR.558 + miR.609 + miR.603 + miR.519b + miR.29a + miR.369.3p + miR.376a* + miR.518c + miR.660 | 0,8 | 0,203232586 |
| miR.558 + miR.609 + miR.603 + miR.520f + miR.519b + miR.29a + miR.376a* + miR.518c + miR.660 | 0,8 | 0,081620747 |
| miR.558 + miR.609 + miR.603 + miR.519b + miR.29a + miR.369.3p + miR.376a* + miR.518c + miR.639 | 0,8 | 0,130881195 |
| miR.558 + miR.609 + miR.603 + miR.519b + miR.29a + miR.362 + miR.369.3p + miR.376a* + miR.518c | 0,8 | 0,096326216 |
| miR.558 + miR.609 + miR.220a + miR.29a + miR.369.3p + miR.376a* + miR.494 + miR.518c + miR.639 | 0,8 | 0,172144231 |
| miR.558 + miR.609 + miR.220a + miR.519b + miR.130a + miR.29a + miR.362 + miR.376a* + miR.518c | 0,8 | 0,249366138 |
| miR.558 + miR.609 + miR.603 + miR.520f + miR.29a + miR.362 + miR.376a* + miR.518c + miR.660 | 0,8 | 0,095128794 |
| miR.558 + miR.609 + miR.520f + miR.130a + miR.29a + miR.376a* + miR.494 + miR.518c + miR.338 | 0,8 | 0,209393423 |
| miR.558 + miR.609 + miR.220a + miR.29a + miR.369.3p + miR.376a* + miR.494 + miR.518c + miR.338 | 0,8 | 0,227799703 |
| miR.558 + miR.609 + miR.603 + miR.29a + miR.362 + miR.369.3p + miR.376a* + miR.518c + miR.639 | 0,8 | 0,065582373 |
| miR.558 + miR.609 + miR.520f + miR.519b + miR.29a + miR.376a* + miR.494 + miR.518c + miR.660 | 0,8 | 0,078551699 |
| miR.558 + miR.609 + miR.520f + miR.26a + miR.29a + miR.376a* + miR.494 + miR.518c + miR.338 | 0,8 | 0,239015696 |
| miR.558 + miR.609 + miR.520f + miR.519b + miR.29a + miR.376a* + miR.518c + miR.639 + miR.338 | 0,8 | 0,213830836 |
| miR.558 + miR.609 + miR.603 + miR.520f + miR.26a + miR.29a + miR.376a* + miR.518c + miR.660 | 0,8 | 0,085213256 |
| miR.558 + miR.609 + miR.520f + miR.519b + miR.26a + miR.29a + miR.369.3p + miR.376a* + miR.518c | 0,8 | 0,133535929 |
| miR.558 + miR.609 + miR.520f + miR.519b + miR.130a + miR.29a + miR.376a* + miR.494 + miR.518c | 0,8 | 0,101755017 |
| miR.558 + miR.609 + miR.520f + miR.26a + miR.29a + miR.362 + miR.376a* + miR.518c + miR.660 | 0,8 | 0,067179551 |
| miR.558 + miR.609 + miR.520f + miR.130a + miR.26a + miR.29a + miR.369.3p + miR.376a* + miR.518c | 0,8 | 0,106466 987 |
| miR.558 + miR.609 + miR.520f + miR.519b + miR.29a + miR.376a* + miR.518c + miR.639 + miR.660 | 0,8 | 0,162912065 |
| miR.558 + miR.609 + miR.520f + miR.130a + miR.29a + miR.376a* + miR.518c + miR.639 + miR.338 | 0,8 | 0,180518269 |
| miR.558 + miR.609 + miR.220a + miR.29a + miR.369.3p + miR.376a* + miR.518c + miR.639 + miR.338 | 0,8 | 0,229237386 |
| miR.558 + miR.609 + miR.520f + miR.26a + miR.29a + miR.376a* + miR.518c + miR.639 + miR.338 | 0,8 | 0,17032701 |
| miR.558 + miR.609 + miR.520f + miR.29a + miR.362 + miR.376a* + miR.518c + miR.639 + miR.660 | 0,8 | 0,160777596 |
| miR.558 + miR.609 + miR.520f + miR.26a + miR.29a + miR.376a* + miR.518c + miR.639 + miR.660 | 0,8 | 0,171614654 |
| miR.558 + miR.609 + miR.520f + miR.519b + miR.29a + miR.362 + miR.376a* + miR.518c + miR.338 | 0,8 | 0,428704603 |
| miR.558 + miR.609 + miR.26a + miR.29a + miR.369.3p + miR.376a* + miR.494 + miR.518c + miR.639 | 0,8 | 0,104096385 |
| miR.558 + miR.609 + miR.220a + miR.130a + miR.29a + miR.362 + miR.376a* + miR.494 + miR.518c | 0,8 | 0,13332866 |
| miR.558 + miR.609 + miR.220a + miR.130a + miR.26a + miR.29a + miR.376a* + miR.518c + miR.338 | 0,8 | 0,273608191 |
| miR.558 + miR.609 + miR.603 + miR.130a + miR.29a + miR.376a* + miR.494 + miR.518c + miR.639 | 0,8 | 0,024497224 |
| miR.558 + miR.609 + miR.220a + miR.519b + miR.29a + miR.376a* + miR.494 + miR.518c + miR.660 | 0,8 | 0,206194156 |
| miR.558 + miR.609 + miR.603 + miR.130a + miR.29a + miR.362 + miR.369.3p + miR.376a* + miR.518c | 0,8 | 0,042217641 |
| miR.558 + miR.609 + miR.603 + miR.519b + miR.29a + miR.369.3p + miR.376a* + miR.494 + miR.518c | 0,8 | 0,196342391 |
| miR.558 + miR.609 + miR.220a + miR.130a + miR.29a + miR.362 + miR.376a* + miR.518c + miR.639 | 0,8 | 0,144684567 |
| miR.558 + miR.609 + miR.220a + miR.519b + miR.26a + miR.29a + miR.376a* + miR.518c + miR.338 | 0,8 | 0,26724475 |
| miR.558 + miR.609 + miR.26a + miR.29a + miR.369.3p + miR.376a* + miR.518c + miR.639 + miR.660 | 0,8 | 0,143259995 |
| miR.558 + miR.609 + miR.520f + miR.519b + miR.29a + miR.362 + miR.376a* + miR.518c + miR.660 | 0,8 | 0,07658739 |
| miR.558 + miR.609 + miR.520f + miR.519b + miR.130a + miR.29a + miR.376a* + miR.518c + miR.660 | 0,8 | 0,07674067 |
| miR.558 + miR.609 + miR.520f + miR.519b + miR.29a + miR.362 + miR.369.3p + miR.376a* + miR.518c | 0,8 | 0,099325768 |
| miR.558 + miR.609 + miR.520f + miR.26a + miR.29a + miR.362 + miR.376a* + miR.518c + miR.338 | 0,8 | 0,371461895 |
| miR.558 + miR.609 + miR.520f + miR.130a + miR.29a + miR.362 + miR.369.3p + miR.376a* + miR.518c | 0,8 | 0,074703987 |
| miR.558 + miR.609 + miR.603 + miR.26a + miR.29a + miR.376a* + miR.494 + miR.518c + miR.639 | 0,8 | 0,05069194 |
| miR.558 + miR.609 + miR.29a + miR.362 + miR.369.3p + miR.376a* + miR.518c + miR.660 + miR.338 | 0,8 | 0,365122071 |
| miR.558 + miR.609 + miR.603 + miR.519b + miR.130a + miR.29a + miR.369.3p + miR.376a* + miR.518c | 0,8 | 0,129465375 |
| miR.558 + miR.609 + miR.220a + miR.130a + miR.29a + miR.362 + miR.376a* + miR.518c + miR.338 | 0,8 | 0,150124142 |
| miR.558 + miR.609 + miR.603 + miR.520f + miR.519b + miR.130a + miR.29a + miR.376a* + miR.518c | 0,8 | 0,024607047 |
| miR.558 + miR.609 + miR.520f + miR.29a + miR.362 + miR.376a* + miR.494 + miR.518c + miR.660 | 0,8 | 0,063271826 |
| miR.558 + miR.609 + miR.520f + miR.26a + miR.29a + miR.376a* + miR.494 + miR.518c + miR.660 | 0,8 | 0,064058318 |
| miR.558 + miR.609 + miR.520f + miR.130a + miR.29a + miR.362 + miR.376a* + miR.518c + miR.639 | 0,8 | 0,126667018 |
| miR.609 + miR.603 + miR.220a + miR.29a + miR.369.3p + miR.376a* + miR.518c + miR.660 + miR.338 | 0,8 | 0,332766418 |
| miR.558 + miR.609 + miR.220a + miR.519b + miR.29a + miR.362 + miR.376a* + miR.518c + miR.639 | 0,8 | 0,280490895 |
| miR.558 + miR.609 + miR.520f + miR.26a + miR.29a + miR.362 + miR.376a* + miR.518c + miR.639 | 0,8 | 0,100263046 |
| miR.558 + miR.609 + miR.519b + miR.26a + miR.29a + miR.369.3p + miR.376a* + miR.518c + miR.639 | 0,8 | 0,07995757 |
| miR.558 + miR.609 + miR.220a + miR.519b + miR.29a + miR.362 + miR.376a* + miR.518c + miR.338 | 0,8 | 0,329238753 |
| miR.558 + miR.609 + miR.220a + miR.130a + miR.26a + miR.29a + miR.376a* + miR.518c + miR.639 | 0,8 | 0,129710832 |
| miR.558 + miR.609 + miR.520f + miR.519b + miR.130a + miR.29a + miR.369.3p + miR.376a* + miR.518c | 0,8 | 0,134355095 |
| miR.558 + miR.609 + miR.220a + miR.519b + miR.26a + miR.29a + miR.376a* + miR.518c + miR.639 | 0,8 | 0,251972101 |
| miR.558 + miR.609 + miR.220a + miR.519b + miR.130a + miR.26a + miR.29a + miR.376a* + miR.518c | 0,8 | 0,257965806 |
| miR.558 + miR.609 + miR.603 + miR.520f + miR.519b + miR.26a + miR.29a + miR.376a* + miR.518c | 0,8 | 0,021827111 |
| miR.558 + miR.609 + miR.520f + miR.519b + miR.29a + miR.362 + miR.376a* + miR.518c + miR.639 | 0,8 | 0,100596699 |
| miR.558 + miR.609 + miR.520f + miR.519b + miR.29a + miR.362 + miR.376a* + miR.494 + miR.518c | 0,8 | 0,165993763 |
| miR.558 + miR.609 + miR.603 + miR.519b + miR.29a + miR.369.3p + miR.376a* + miR.518c + miR.338 | 0,8 | 0,257926032 |
| miR.558 + miR.609 + miR.603 + miR.29a + miR.369.3p + miR.376a* + miR.494 + miR.518c + miR.338 | 0,8 | 0,143503023 |
| miR.609 + miR.603 + miR.520f + miR.220a + miR.29a + miR.362 + miR.369.3p + miR.376a* + miR.518c | 0,8 | 0,305273849 |
| miR.558 + miR.609 + miR.220a + miR.29a + miR.362 + miR.376a* + miR.494 + miR.518c + miR.660 | 0,8 | 0,116391653 |
| miR.558 + miR.609 + miR.26a + miR.29a + miR.369.3p + miR.376a* + miR.518c + miR.660 + miR.338 | 0,8 | 0,427902828 |
| miR.558 + miR.609 + miR.520f + miR.519b + miR.29a + miR.376a* + miR.494 + miR.518c + miR.639 | 0,8 | 0,064412715 |
| miR.558 + miR.609 + miR.26a + miR.29a + miR.369.3p + miR.376a* + miR.494 + miR.518c + miR.660 | 0,8 | 0,11552722 |
| miR.558 + miR.609 + miR.520f + miR.26a + miR.29a + miR.376a* + miR.494 + miR.518c + miR.639 | 0,8 | 0,041948152 |
| miR.558 + miR.609 + miR.520f + miR.130a + miR.29a + miR.362 + miR.376a* + miR.494 + miR.518c | 0,8 | 0,108599573 |
| miR.558 + miR.609 + miR.603 + miR.26a + miR.29a + miR.362 + miR.376a* + miR.518c + miR.639 | 0,8 | 0,18904734 |
| miR.558 + miR.609 + miR.603 + miR.520f + miR.130a + miR.26a + miR.29a + miR.376a* + miR.518c | 0,8 | 0,021148867 |
| miR.558 + miR.609 + miR.519b + miR.26a + miR.29a + miR.369.3p + miR.376a* + miR.518c + miR.338 | 0,8 | 0,38337346 |
| miR.558 + miR.609 + miR.220a + miR.29a + miR.362 + miR.376a* + miR.518c + miR.639 + miR.338 | 0,8 | 0,163203959 |
| miR.609 + miR.603 + miR.220a + miR.519b + miR.29a + miR.369.3p + miR.376a* + miR.494 + miR.518c | 0,8 | 0,463138608 |
| miR.609 + miR.603 + miR.220a + miR.130a + miR.29a + miR.369.3p + miR.376a* + miR.518c + miR.660 | 0,8 | 0,481612847 |
| miR.609 + miR.603 + miR.520f + miR.220a + miR.29a + miR.369.3p + miR.376a* + miR.494 + miR.518c | 0,8 | 0,411190909 |
| miR.558 + miR.609 + miR.220a + miR.29a + miR.376a* + miR.494 + miR.518c + miR.639 + miR.660 | 0,8 | 0,115285111 |
| miR.558 + miR.609 + miR.26a + miR.29a + miR.362 + miR.369.3p + miR.376a* + miR.518c + miR.660 | 0,8 | 0,377938106 |
| miR.558 + miR.609 + miR.520f + miR.26a + miR.29a + miR.362 + miR.376a* + miR.494 + miR.518c | 0,8 | 0,097758112 |
| miR.558 + miR.609 + miR.26a + miR.29a + miR.362 + miR.369.3p + miR.376a* + miR.518c + miR.338 | 0,8 | 0,429345055 |
| miR.558 + miR.609 + miR.603 + miR.26a + miR.29a + miR.376a* + miR.518c + miR.639 + miR.660 | 0,8 | 0,183860403 |
| miR.558 + miR.609 + miR.29a + miR.362 + miR.369.3p + miR.376a* + miR.518c + miR.639 + miR.660 | 0,8 | 0,34935503 |
| miR.558 + miR.609 + miR.603 + miR.26a + miR.29a + miR.376a* + miR.494 + miR.518c + miR.338 | 0,8 | 0,078425624 |
| miR.558 + miR.609 + miR.130a + miR.26a + miR.29a + miR.369.3p + miR.376a* + miR.518c + miR.338 | 0,8 | 0,398793295 |
| miR.558 + miR.609 + miR.220a + miR.130a + miR.29a + miR.376a* + miR.494 + miR.518c + miR.639 | 0,8 | 0,025222439 |
| miR.609 + miR.603 + miR.520f + miR.220a + miR.26a + miR.29a + miR.369.3p + miR.376a* + miR.518c | 0,8 | 0,268929606 |
| miR.558 + miR.609 + miR.220a + miR.130a + miR.29a + miR.376a* + miR.518c + miR.639 + miR.660 | 0,8 | 0,111832869 |
| miR.558 + miR.609 + miR.220a + miR.29a + miR.376a* + miR.494 + miR.518c + miR.660 + miR.338 | 0,8 | 0,175735685 |
| miR.558 + miR.609 + miR.520f + miR.130a + miR.26a + miR.29a + miR.376a* + miR.518c + miR.338 | 0,8 | 0,199409176 |
| miR.609 + miR.603 + miR.520f + miR.220a + miR.519b + miR.29a + miR.369.3p + miR.376a* + miR.518c | 0,8 | 0,442508019 |
| miR.609 + miR.603 + miR.220a + miR.130a + miR.26a + miR.29a + miR.369.3p + miR.376a* + miR.518c | 0,8 | 0,427623952 |
| miR.558 + miR.609 + miR.603 + miR.29a + miR.362 + miR.369.3p + miR.376a* + miR.494 + miR.518c | 0,8 | 0,053765849 |
| miR.558 + miR.609 + miR.519b + miR.26a + miR.29a + miR.369.3p + miR.376a* + miR.494 + miR.518c | 0,8 | 0,071596829 |
| miR.558 + miR.609 + miR.220a + miR.519b + miR.130a + miR.29a + miR.376a* + miR.518c + miR.660 | 0,8 | 0,155742136 |
| miR.558 + miR.609 + miR.220a + miR.519b + miR.29a + miR.376a* + miR.518c + miR.639 + miR.660 | 0,8 | 0,12950482 |
| miR.609 + miR.603 + miR.220a + miR.26a + miR.29a + miR.369.3p + miR.376a* + miR.518c + miR.639 | 0,8 | 0,471126345 |
| miR.609 + miR.603 + miR.220a + miR.26a + miR.29a + miR.369.3p + miR.376a* + miR.518c + miR.338 | 0,8 | 0,174744472 |
| miR.558 + miR.609 + miR.220a + miR.519b + miR.29a + miR.362 + miR.376a* + miR.518c + miR.660 | 0,8 | 0,136241522 |
| miR.558 + miR.609 + miR.26a + miR.29a + miR.362 + miR.369.3p + miR.376a* + miR.494 + miR.518c | 0,8 | 0,084003226 |
| miR.558 + miR.609 + miR.520f + miR.519b + miR.130a + miR.29a + miR.376a* + miR.518c + miR.338 | 0,8 | 0,269413661 |
| miR.609 + miR.603 + miR.520f + miR.220a + miR.519b + miR.29a + miR.376a* + miR.494 + miR.518c | 0,8 | 0,158062 |
| miR.609 + miR.603 + miR.220a + miR.519b + miR.26a + miR.29a + miR.369.3p + miR.376a* + miR.518c | 0,8 | 0,422983257 |
| miR.609 + miR.603 + miR.520f + miR.220a + miR.130a + miR.29a + miR.369.3p + miR.376a* + miR.518c | 0,8 | 0,426997797 |
| miR.609 + miR.603 + miR.220a + miR.26a + miR.29a + miR.362 + miR.369.3p + miR.376a* + miR.518c | 0,8 | 0,323308493 |
| miR.558 + miR.609 + miR.220a + miR.519b + miR.29a + miR.376a* + miR.518c + miR.660 + miR.338 | 0,8 | 0,17076 026 |
| miR.558 + miR.609 + miR.520f + miR.519b + miR.26a + miR.29a + miR.376a* + miR.518c + miR.338 | 0,8 | 0,28683872 |
| miR.558 + miR.609 + miR.220a + miR.130a + miR.29a + miR.362 + miR.376a* + miR.518c + miR.660 | 0,8 | 0,077463871 |
| miR.558 + miR.609 + miR.220a + miR.29a + miR.362 + miR.376a* + miR.518c + miR.639 + miR.660 | 0,8 | 0,084212395 |
| miR.558 + miR.609 + miR.220a + miR.29a + miR.362 + miR.376a* + miR.518c + miR.660 + miR.338 | 0,8 | 0,110346752 |
| miR.558 + miR.609 + miR.220a + miR.29a + miR.376a* + miR.518c + miR.639 + miR.660 + miR.338 | 0,8 | 0,123021301 |
| miR.558 + miR.609 + miR.26a + miR.29a + miR.362 + miR.369.3p + miR.376a* + miR.518c + miR.639 | 0,8 | 0,141080519 |
| miR.558 + miR.609 + miR.220a + miR.130a + miR.29a + miR.376a* + miR.494 + miR.518c + miR.338 | 0,8 | 0,020979497 |
| miR.558 + miR.609 + miR.519b + miR.26a + miR.29a + miR.369.3p + miR.376a* + miR.518c + miR.660 | 0,8 | 0,12421106 |
| miR.558 + miR.609 + miR.603 + miR.519b + miR.26a + miR.29a + miR.376a* + miR.518c + miR.639 | 0,8 | 0,103141448 |
| miR.558 + miR.609 + miR.130a + miR.29a + miR.369.3p + miR.376a* + miR.518c + miR.639 + miR.338 | 0,8 | 0,113194072 |
| miR.558 + miR.609 + miR.220a + miR.130a + miR.29a + miR.376a* + miR.518c + miR.660 + miR.338 | 0,8 | 0,100544208 |
| miR.558 + miR.609 + miR.603 + miR.130a + miR.26a + miR.29a + miR.376a* + miR.494 + miR.518c | 0,8 | 0,052344062 |
| miR.609 + miR.603 + miR.220a + miR.29a + miR.362 + miR.369.3p + miR.376a* + miR.494 + miR.518c | 0,8 | 0,350274623 |
| miR.558 + miR.609 + miR.130a + miR.29a + miR.369.3p + miR.376a* + miR.494 + miR.518c + miR.639 | 0,8 | 0,13106931 |
| miR.558 + miR.609 + miR.603 + miR.130a + miR.26a + miR.29a + miR.376a* + miR.518c + miR.639 | 0,8 | 0,049287003 |
| miR.558 + miR.609 + miR.520f + miR.130a + miR.26a + miR.29a + miR.376a* + miR.518c + miR.639 | 0,8 | 0,058279819 |
| miR.558 + miR.609 + miR.520f + miR.519b + miR.26a + miR.29a + miR.376a* + miR.494 + miR.518c | 0,8 | 0,066098363 |
| miR.558 + miR.609 + miR.130a + miR.26a + miR.29a + miR.376a* + miR.494 + miR.518c + miR.639 | 0,8 | 0,065283681 |
| miR.558 + miR.609 + miR.603 + miR.519b + miR.26a + miR.29a + miR.376a* + miR.494 + miR.518c | 0,8 | 0,184657667 |
| miR.558 + miR.609 + miR.520f + miR.519b + miR.130a + miR.29a + miR.376a* + miR.518c + miR.639 | 0,8 | 0,043721789 |
| miR.558 + miR.609 + miR.220a + miR.519b + miR.130a + miR.29a + miR.376a* + miR.518c + miR.639 | 0,8 | 0,079817418 |
| miR.558 + miR.609 + miR.220a + miR.519b + miR.130a + miR.29a + miR.376a* + miR.518c + miR.338 | 0,8 | 0,089405155 |
| miR.558 + miR.609 + miR.520f + miR.130a + miR.29a + miR.362 + miR.376a* + miR.518c + miR.660 | 0,8 | 0,080171246 |
| miR.558 + miR.609 + miR.520f + miR.130a + miR.26a + miR.29a + miR.376a* + miR.518c + miR.660 | 0,8 | 0,068256211 |
| miR.558 + miR.609 + miR.29a + miR.362 + miR.369.3p + miR.376a* + miR.494 + miR.518c + miR.660 | 0,8 | 0,196601967 |
| miR.558 + miR.609 + miR.520f + miR.519b + miR.26a + miR.29a + miR.362 + miR.376a* + miR.518c | 0,8 | 0,088997057 |
| miR.558 + miR.609 + miR.220a + miR.519b + miR.29a + miR.376a* + miR.518c + miR.639 + miR.338 | 0,8 | 0,105010951 |
| miR.558 + miR.609 + miR.603 + miR.519b + miR.130a + miR.29a + miR.376a* + miR.494 + miR.518c | 0,8 | 0,097981396 |
| miR.558 + miR.609 + miR.220a + miR.130a + miR.29a + miR.376a* + miR.518c + miR.639 + miR.338 | 0,8 | 0,022940199 |
| miR.558 + miR.609 + miR.520f + miR.519b + miR.26a + miR.29a + miR.376a* + miR.518c + miR.660 | 0,8 | 0,084782852 |
| miR.558 + miR.609 + miR.520f + miR.130a + miR.26a + miR.29a + miR.362 + miR.376a* + miR.518c | 0,8 | 0,076049991 |
| miR.558 + miR.609 + miR.520f + miR.519b + miR.130a + miR.29a + miR.362 + miR.376a* + miR.518c | 0,8 | 0,099497198 |
| miR.558 + miR.609 + miR.520f + miR.130a + miR.26a + miR.29a + miR.376a* + miR.494 + miR.518c | 0,8 | 0,060483414 |
| miR.558 + miR.609 + miR.220a + miR.519b + miR.29a + miR.376a* + miR.494 + miR.518c + miR.639 | 0,8 | 0,12618368 |
| miR.609 + miR.603 + miR.220a + miR.519b + miR.130a + miR.29a + miR.369.3p + miR.376a* + miR.518c | 0,8 | 0,487950583 |
| miR.609 + miR.603 + miR.520f + miR.220a + miR.29a + miR.369.3p + miR.376a* + miR.518c + miR.338 | 0,8 | 0,213794953 |
| miR.609 + miR.603 + miR.520f + miR.220a + miR.29a + miR.376a* + miR.494 + miR.518c + miR.660 | 0,8 | 0,138062234 |
| miR.558 + miR.609 + miR.520f + miR.519b + miR.26a + miR.29a + miR.376a* + miR.518c + miR.639 | 0,8 | 0,034901673 |
| miR.609 + miR.603 + miR.520f + miR.220a + miR.29a + miR.369.3p + miR.376a* + miR.518c + miR.639 | 0,8 | 0,478297632 |
| miR.558 + miR.609 + miR.603 + miR.26a + miR.29a + miR.362 + miR.376a* + miR.518c + miR.338 | 0,8 | 0,150637982 |
| miR.558 + miR.609 + miR.519b + miR.29a + miR.362 + miR.369.3p + miR.376a* + miR.518c + miR.660 | 0,8 | 0,212509268 |
| miR.558 + miR.609 + miR.519b + miR.26a + miR.29a + miR.362 + miR.369.3p + miR.376a* + miR.518c | 0,8 | 0,119732414 |
| miR.609 + miR.603 + miR.220a + miR.519b + miR.29a + miR.376a* + miR.494 + miR.518c + miR.660 | 0,8 | 0,26408102 |
| miR.609 + miR.520f + miR.220a + miR.29a + miR.369.3p + miR.376a* + miR.494 + miR.518c + miR.660 | 0,8 | 0,382526012 |
| miR.558 + miR.609 + miR.220a + miR.519b + miR.29a + miR.362 + miR.376a* + miR.494 + miR.518c | 0,8 | 0,267305514 |
| miR.558 + miR.609 + miR.603 + miR.130a + miR.26a + miR.29a + miR.376a* + miR.518c + miR.338 | 0,8 | 0,077106553 |
| miR.558 + miR.609 + miR.603 + miR.26a + miR.29a + miR.362 + miR.376a* + miR.494 + miR.518c | 0,8 | 0,063217109 |
| miR.558 + miR.609 + miR.130a + miR.29a + miR.369.3p + miR.376a* + miR.518c + miR.639 + miR.660 | 0,8 | 0,227075835 |
| miR.558 + miR.609 + miR.603 + miR.26a + miR.29a + miR.376a* + miR.494 + miR.518c + miR.660 | 0,8 | 0,041314211 |
| miR.609 + miR.603 + miR.520f + miR.29a + miR.369.3p + miR.376a* + miR.494 + miR.518c + miR.338 | 0,8 | 0,345531422 |
| miR.558 + miR.609 + miR.130a + miR.26a + miR.29a + miR.369.3p + miR.376a* + miR.518c + miR.660 | 0,8 | 0,114196672 |
| miR.558 + miR.609 + miR.220a + miR.29a + miR.362 + miR.376a* + miR.494 + miR.518c + miR.639 | 0,8 | 0,141067233 |
| miR.609 + miR.603 + miR.220a + miR.29a + miR.369.3p + miR.376a* + miR.494 + miR.518c + miR.639 | 0,8 | 0,315892469 |
| miR.558 + miR.609 + miR.220a + miR.29a + miR.362 + miR.376a* + miR.494 + miR.518c + miR.338 | 0,8 | 0,164020547 |
| miR.558 + miR.609 + miR.603 + miR.130a + miR.29a + miR.376a* + miR.518c + miR.639 + miR.338 | 0,8 | 0,006479141 |
| miR.558 + miR.609 + miR.519b + miR.130a + miR.26a + miR.29a + miR.369.3p + miR.376a* + miR.518c | 0,8 | 0,077117054 |
| miR.558 + miR.609 + miR.220a + miR.29a + miR.376a* + miR.494 + miR.518c + miR.639 + miR.338 | 0,8 | 0,035534543 |
| miR.609 + miR.603 + miR.220a + miR.29a + miR.369.3p + miR.376a* + miR.494 + miR.518c + miR.338 | 0,8 | 0,415251922 |
| miR.609 + miR.603 + miR.520f + miR.29a + miR.369.3p + miR.376a* + miR.518c + miR.639 + miR.338 | 0,8 | 0,319667202 |
| miR.558 + miR.609 + miR.130a + miR.29a + miR.362 + miR.369.3p + miR.376a* + miR.518c + miR.660 | 0,8 | 0,264912863 |
| miR.609 + miR.603 + miR.520f + miR.220a + miR.29a + miR.362 + miR.376a* + miR.494 + miR.518c | 0,8 | 0,087304781 |
| miR.609 + miR.603 + miR.220a + miR.519b + miR.26a + miR.29a + miR.376a* + miR.494 + miR.518c | 0,8 | 0,034587309 |
| miR.609 + miR.603 + miR.220a + miR.519b + miR.29a + miR.362 + miR.369.3p + miR.376a* + miR.518c | 0,8 | 0,480285293 |
| miR.609 + miR.603 + miR.220a + miR.519b + miR.29a + miR.369.3p + miR.376a* + miR.518c + miR.338 | 0,8 | 0,433362225 |
| miR.558 + miR.609 + miR.520f + miR.29a + miR.362 + miR.369.3p + miR.376a* + miR.660 + miR.338 | 0,8 | 0,290697116 |
| miR.558 + miR.609 + miR.603 + miR.519b + miR.26a + miR.29a + miR.376a* + miR.518c + miR.338 | 0,8 | 0,139822779 |
| miR.609 + miR.603 + miR.220a + miR.130a + miR.29a + miR.369.3p + miR.376a* + miR.494 + miR.518c | 0,8 | 0,306145794 |
| miR.609 + miR.603 + miR.220a + miR.519b + miR.29a + miR.369.3p + miR.376a* + miR.518c + miR.639 | 0,8 | 0,457628132 |
| miR.558 + miR.609 + miR.130a + miR.26a + miR.29a + miR.362 + miR.369.3p + miR.376a* + miR.518c | 0,8 | 0,103179358 |
| miR.609 + miR.603 + miR.220a + miR.29a + miR.369.3p + miR.376a* + miR.518c + miR.639 + miR.338 | 0,8 | 0,495998135 |
| miR.558 + miR.609 + miR.603 + miR.519b + miR.29a + miR.376a* + miR.494 + miR.518c + miR.639 | 0,8 | 0,034868758 |
| miR.558 + miR.609 + miR.130a + miR.29a + miR.376a* + miR.494 + miR.518c + miR.639 + miR.660 | 0,8 | 0,158089758 |
| miR.558 + miR.609 + miR.130a + miR.29a + miR.369.3p + miR.376a* + miR.494 + miR.518c + miR.660 | 0,8 | 0,114688875 |
| miR.558 + miR.609 + miR.130a + miR.29a + miR.369.3p + miR.376a* + miR.494 + miR.518c + miR.338 | 0,8 | 0,171828811 |
| miR.558 + miR.609 + miR.130a + miR.29a + miR.362 + miR.369.3p + miR.376a* + miR.518c + miR.639 | 0,8 | 0,090864947 |
| miR.609 + miR.603 + miR.220a + miR.519b + miR.29a + miR.362 + miR.376a* + miR.494 + miR.518c | 0,8 | 0,181494289 |
| miR.609 + miR.603 + miR.220a + miR.519b + miR.130a + miR.29a + miR.376a* + miR.494 + miR.518c | 0,8 | 0,051961949 |
| miR.609 + miR.603 + miR.220a + miR.29a + miR.362 + miR.369.3p + miR.376a* + miR.518c + miR.639 | 0,8 | 0,414565334 |
| miR.609 + miR.603 + miR.520f + miR.220a + miR.130a + miR.29a + miR.376a* + miR.518c + miR.660 | 0,8 | 0,127139137 |
| miR.558 + miR.609 + miR.519b + miR.130a + miR.29a + miR.369.3p + miR.376a* + miR.518c + miR.639 | 0,8 | 0,073997354 |
| miR.558 + miR.609 + miR.130a + miR.26a + miR.29a + miR.362 + miR.376a* + miR.518c + miR.639 | 0,8 | 0,106245043 |
| miR.558 + miR.609 + miR.520f + miR.519b + miR.130a + miR.26a + miR.29a + miR.376a* + miR.518c | 0,8 | 0,020564717 |
| miR.609 + miR.603 + miR.220a + miR.29a + miR.362 + miR.369.3p + miR.376a* + miR.518c + miR.338 | 0,8 | 0,457160688 |
| miR.558 + miR.609 + miR.603 + miR.130a + miR.26a + miR.29a + miR.362 + miR.376a* + miR.518c | 0,8 | 0,028720054 |
| miR.558 + miR.609 + miR.220a + miR.519b + miR.29a + miR.376a* + miR.494 + miR.518c + miR.338 | 0,8 | 0,135275975 |
| miR.558 + miR.609 + miR.603 + miR.130a + miR.29a + miR.362 + miR.376a* + miR.518c + miR.639 | 0,8 | 0,035358736 |
| miR.558 + miR.609 + miR.130a + miR.26a + miR.29a + miR.376a* + miR.518c + miR.639 + miR.660 | 0,8 | 0,117954732 |
| miR.558 + miR.609 + miR.130a + miR.29a + miR.362 + miR.376a* + miR.494 + miR.518c + miR.639 | 0,8 | 0,095810638 |
| miR.558 + miR.609 + miR.130a + miR.29a + miR.362 + miR.369.3p + miR.376a* + miR.494 + miR.518c | 0,8 | 0,129757872 |
| miR.558 + miR.609 + miR.603 + miR.519b + miR.26a + miR.29a + miR.362 + miR.376a* + miR.518c | 0,8 | 0,094782244 |
| miR.558 + miR.609 + miR.603 + miR.130a + miR.29a + miR.376a* + miR.518c + miR.639 + miR.660 | 0,8 | 0,066510885 |
| miR.558 + miR.609 + miR.519b + miR.130a + miR.29a + miR.369.3p + miR.376a* + miR.494 + miR.518c | 0,8 | 0,068273345 |
| miR.558 + miR.609 + miR.603 + miR.26a + miR.29a + miR.376a* + miR.518c + miR.660 + miR.338 | 0,8 | 0,101713117 |
| miR.558 + miR.609 + miR.603 + miR.519b + miR.130a + miR.26a + miR.29a + miR.376a* + miR.518c | 0,8 | 0,035453664 |
| miR.609 + miR.603 + miR.220a + miR.130a + miR.29a + miR.362 + miR.369.3p + miR.376a* + miR.518c | 0,8 | 0,37009407 |
| miR.558 + miR.609 + miR.603 + miR.130a + miR.26a + miR.29a + miR.376a* + miR.518c + miR.660 | 0,8 | 0,021782704 |
| miR.558 + miR.609 + miR.603 + miR.519b + miR.29a + miR.376a* + miR.518c + miR.639 + miR.660 | 0,8 | 0,039816049 |
| miR.558 + miR.609 + miR.603 + miR.130a + miR.29a + miR.362 + miR.376a* + miR.494 + miR.518c | 0,8 | 0,011144508 |
| miR.609 + miR.603 + miR.220a + miR.130a + miR.29a + miR.369.3p + miR.376a* + miR.518c + miR.338 | 0,8 | 0,44092978 |
| miR.609 + miR.603 + miR.520f + miR.29a + miR.369.3p + miR.376a* + miR.494 + miR.518c + miR.660 | 0,8 | 0,137581827 |
| miR.558 + miR.609 + miR.603 + miR.130a + miR.29a + miR.376a* + miR.494 + miR.518c + miR.338 | 0,8 | 0,009263095 |
| miR.558 + miR.609 + miR.603 + miR.130a + miR.29a + miR.376a* + miR.494 + miR.518c + miR.660 | 0,8 | 0,010895069 |
| miR.558 + miR.609 + miR.603 + miR.29a + miR.376a* + miR.494 + miR.518c + miR.639+miR.338 | 0,8 | 0,017277545 |
| miR.558 + miR.609 + miR.603 + miR.29a + miR.362 + miR.376a* + miR.518c + miR.639 + miR.660 | 0,8 | 0,032467834 |
| miR.609 + miR.520f + miR.220a + miR.29a + miR.362 + miR.369.3p + miR.376a* + miR.518c + miR.660 | 0,8 | 0,392375462 |
| miR.558 + miR.609 + miR.603 + miR.29a + miR.376a* + miR.494 + miR.518c + miR.639 + miR.660 | 0,8 | 0,038240115 |
| miR.558 + miR.609 + miR.130a + miR.29a + miR.376a* + miR.494 + miR.518c + miR.639 + miR.338 | 0,8 | 0,09606346 |
| miR.558 + miR.609 + miR.603 + miR.26a + miR.29a + miR.362 + miR.376a* + miR.518c + miR.660 | 0,8 | 0,022539566 |
| miR.609 + miR.603 + miR.220a + miR.130a + miR.29a + miR.369.3p + miR.376a* + miR.518c + miR.639 | 0,8 | 0,399140134 |
| miR.609 + miR.603 + miR.520f + miR.220a + miR.29a + miR.362 + miR.376a* + miR.518c + miR.639 | 0,8 | 0,144676791 |
| miR.609 + miR.603 + miR.520f + miR.29a + miR.369.3p + miR.376a* + miR.518c + miR.660 + miR.338 | 0,8 | 0,393283621 |
| miR.558 + miR.609 + miR.519b + miR.26a + miR.29a + miR.376a* + miR.518c + miR.639+miR.338 | 0,8 | 0,108718229 |
| miR.558 + miR.609 + miR.603 + miR.519b + miR.26a + miR.29a + miR.376a* + miR.518c + miR.660 | 0,8 | 0,078605663 |
| miR.558 + miR.609 + miR.26a + miR.29a + miR.376a* + miR.518c + miR.639 + miR.660 + miR.338 | 0,8 | 0,20368957 |
| miR.558 + miR.609 + miR.26a + miR.29a + miR.362 + miR.376a* + miR.518c + miR.639+miR.338 | 0,8 | 0,213832161 |
| miR.558 + miR.609 + miR.29a + miR.369.3p + miR.376a* + miR.518c + miR.639 + miR.660 + miR.338 | 0,8 | 0,244691 095 |
| miR.609 + miR.520f + miR.220a + miR.130a + miR.29a + miR.369.3p + miR.376a* + miR.518c + miR.660 | 0,8 | 0,360236492 |
| miR.558 + miR.609 + miR.603 + miR.520f + miR.130a + miR.376a* + miR.518c + miR.639 + miR.338 | 0,8 | 0,142353629 |
| miR.558 + miR.609 + miR.519b + miR.130a + miR.29a + miR.376a* + miR.494 + miR.518c + miR.639 | 0,8 | 0,052844987 |
| miR.558 + miR.609 + miR.603 + miR.519b + miR.130a + miR.29a + miR.376a* + miR.518c + miR.639 | 0,8 | 0,029623938 |
| miR.558 + miR.609 + miR.603 + miR.29a + miR.362 + miR.376a* + miR.494 + miR.518c + miR.639 | 0,8 | 0,017294487 |
| miR.558 + miR.609 + miR.603 + miR.29a + miR.362 + miR.376a* + miR.518c + miR.639+miR.338 | 0,8 | 0,024834852 |
| miR.558 + miR.609 + miR.29a + miR.369.3p + miR.376a* + miR.494 + miR.518c + miR.639 + miR.660 | 0,8 | 0,155807164 |
| miR.609 + miR.603 + miR.520f + miR.220a + miR.519b + miR.29a + miR.362 + miR.376a* + miR.518c | 0,8 | 0,238139694 |
| miR.558 + miR.609 + miR.603 + miR.520f + miR.369.3p + miR.376a* + miR.518c + miR.639 + miR.338 | 0,8 | 0,207790313 |
| miR.609 + miR.603 + miR.520f + miR.29a + miR.369.3p + miR.376a* + miR.494 + miR.518c + miR.639 | 0,8 | 0,116866676 |
| miR.558 + miR.609 + miR.519b + miR.29a + miR.369.3p + miR.376a* + miR.518c + miR.639 + miR.338 | 0,8 | 0,140009465 |
| miR.558 + miR.609 + miR.130a + miR.29a + miR.369.3p + miR.376a* + miR.518c + miR.660 + miR.338 | 0,8 | 0,133472241 |
| miR.558 + miR.609 + miR.130a + miR.26a + miR.29a + miR.376a* + miR.518c + miR.639+miR.338 | 0,8 | 0,115608628 |
| miR.558 + miR.609 + miR.29a + miR.369.3p + miR.376a* + miR.494 + miR.518c + miR.639+miR.338 | 0,8 | 0,126671162 |
| miR.558 + miR.609 + miR.603 + miR.29a + miR.376a* + miR.518c + miR.639 + miR.660 + miR.338 | 0,8 | 0,057914406 |
| miR.558 + miR.609 + miR.130a + miR.29a + miR.362 + miR.369.3p + miR.376a* + miR.518c + miR.338 | 0,8 | 0,132734107 |
| miR.609 + miR.603 + miR.520f + miR.29a + miR.369.3p + miR.376a* + miR.518c + miR.639 + miR.660 | 0,8 | 0,166407828 |
| miR.558 + miR.609 + miR.519b + miR.26a + miR.29a + miR.362 + miR.376a* + miR.518c + miR.639 | 0,8 | 0,066227121 |
| miR.558 + miR.609 + miR.519b + miR.29a + miR.369.3p + miR.376a* + miR.518c + miR.639 + miR.660 | 0,8 | 0,171844452 |
| miR.609 + miR.603 + miR.520f + miR.220a + miR.29a + miR.376a* + miR.518c + miR.639 + miR.660 | 0,8 | 0,091869834 |
| miR.558 + miR.609 + miR.603 + miR.519b + miR.29a + miR.376a* + miR.494 + miR.518c + miR.338 | 0,8 | 0,086101572 |
| miR.609 + miR.220a + miR.29a + miR.362 + miR.369.3p + miR.376a* + miR.494 + miR.518c + miR.660 | 0,8 | 0,349722804 |
| miR.609 + miR.603 + miR.520f + miR.130a + miR.29a + miR.369.3p + miR.376a* + miR.518c + miR.338 | 0,8 | 0,432397172 |
| miR.609 + miR.603 + miR.520f + miR.220a + miR.130a + miR.29a + miR.362 + miR.376a* + miR.518c | 0,8 | 0,128742377 |
| miR.558 + miR.609 + miR.603 + miR.519b + miR.29a + miR.376a* + miR.518c + miR.639 + miR.338 | 0,8 | 0,022446774 |
| miR.558 + miR.609 + miR.29a + miR.362 + miR.369.3p + miR.376a* + miR.518c + miR.639 + miR.338 | 0,8 | 0,149018493 |
| miR.609 + miR.603 + miR.220a + miR.26a + miR.29a + miR.376a* + miR.494 + miR.518c + miR.660 | 0,8 | 0,104536552 |
| miR.609 + miR.603 + miR.520f + miR.519b + miR.29a + miR.369.3p + miR.376a* + miR.494 + miR.518c | 0,8 | 0,110908764 |
| miR.558 + miR.609 + miR.519b + miR.130a + miR.26a + miR.29a + miR.376a* + miR.494 + miR.518c | 0,8 | 0,064289596 |
| miR.558 + miR.609 + miR.603 + miR.519b + miR.29a + miR.362 + miR.376a* + miR.518c + miR.639 | 0,8 | 0,04786143 |
| miR.609 + miR.603 + miR.520f + miR.220a + miR.29a + miR.376a* + miR.494 + miR.518c + miR.639 | 0,8 | 0,026086832 |
| miR.558 + miR.609 + miR.603 + miR.520f + miR.130a + miR.369.3p + miR.376a* + miR.518c + miR.338 | 0,8 | 0,202312429 |
| miR.609 + miR.520f + miR.220a + miR.519b + miR.29a + miR.369.3p + miR.376a* + miR.518c + miR.660 | 0,8 | 0,432735808 |
| miR.558 + miR.609 + miR.603 + miR.520f + miR.130a + miR.369.3p + miR.376a* + miR.518c + miR.639 | 0,8 | 0,15793484 |
| miR.558 + miR.609 + miR.519b + miR.26a + miR.29a + miR.376a* + miR.518c + miR.639 + miR.660 | 0,8 | 0,08087559 |
| miR.558 + miR.609 + miR.29a + miR.362 + miR.369.3p + miR.376a* + miR.494 + miR.518c + miR.639 | 0,8 | 0,092391206 |
| miR.609 + miR.603 + miR.520f + miR.26a + miR.29a + miR.369.3p + miR.376a* + miR.518c + miR.338 | 0,8 | 0,479385389 |
| miR.558 + miR.609 + miR.26a + miR.29a + miR.376a* + miR.494 + miR.518c + miR.639 + miR.660 | 0,8 | 0,063910988 |
| miR.558 + miR.609 + miR.519b + miR.130a + miR.26a + miR.29a + miR.376a* + miR.518c + miR.639 | 0,8 | 0,03309098 |
| miR.558 + miR.609 + miR.519b + miR.29a + miR.369.3p + miR.376a* + miR.494 + miR.518c + miR.639 | 0,8 | 0,082814397 |
| miR.609 + miR.603 + miR.520f + miR.220a + miR.29a + miR.376a* + miR.494 + miR.518c + miR.338 | 0,8 | 0,085588816 |
| miR.558 + miR.609 + miR.603 + miR.29a + miR.362 + miR.376a* + miR.494 + miR.518c + miR.660 | 0,8 | 0,018293817 |
| miR.609 + miR.603 + miR.520f + miR.220a + miR.130a + miR.29a + miR.376a* + miR.494 + miR.518c | 0,8 | 0,042337528 |
| miR.558 + miR.609 + miR.130a + miR.26a + miR.29a + miR.376a* + miR.494 + miR.518c + miR.660 | 0,8 | 0,058870396 |
| miR.609 + miR.603 + miR.520f + miR.130a + miR.29a + miR.369.3p + miR.376a* + miR.494 + miR.518c | 0,8 | 0,13154698 |
| miR.609 + miR.603 + miR.520f + miR.519b + miR.29a + miR.369.3p + miR.376a* + miR.518c + miR.338 | 0,8 | 0,430628151 |
| miR.609 + miR.603 + miR.220a + miR.26a + miR.29a + miR.362 + miR.376a* + miR.494 + miR.518c | 0,8 | 0,049252717 |
| miR.558 + miR.609 + miR.519b + miR.130a + miR.29a + miR.369.3p + miR.376a* + miR.518c + miR.338 | 0,8 | 0,120103833 |
| miR.609 + miR.603 + miR.520f + miR.29a + miR.376a* + miR.494 + miR.518c + miR.639 + miR.660 | 0,8 | 0,067806989 |
| miR.609 + miR.603 + miR.520f + miR.220a + miR.519b + miR.29a + miR.376a* + miR.518c + miR.660 | 0,8 | 0,221127196 |
| miR.609 + miR.603 + miR.520f + miR.220a + miR.29a + miR.376a* + miR.518c + miR.660 + miR.338 | 0,8 | 0,302752216 |
| miR.609 + miR.603 + miR.520f + miR.26a + miR.29a + miR.369.3p + miR.376a* + miR.494 + miR.518c | 0,8 | 0,132855449 |
| miR.609 + miR.520f + miR.220a + miR.29a + miR.369.3p + miR.376a* + miR.518c + miR.639 + miR.660 | 0,8 | 0,450491072 |
| miR.609 + miR.603 + miR.520f + miR.130a + miR.29a + miR.369.3p + miR.376a* + miR.518c + miR.639 | 0,8 | 0,084150772 |
| miR.558 + miR.609 + miR.29a + miR.369.3p + miR.376a* + miR.494 + miR.518c + miR.660 + miR.338 | 0,8 | 0,165261001 |
| miR.558 + miR.609 + miR.603 + miR.519b + miR.29a + miR.376a* + miR.518c + miR.660 + miR.338 | 0,8 | 0,030694635 |

**Table 10: clusters of 10 miRNAs**

| **Cluster of 10 miRs** | **c_tau level** | **pv compared to top signatur e miR.558 + miR.609 + miR.603 + miR.220 a+ miR.26a + miR.29a + miR.369 .3p + miR.376 a* + miR.518 c+ miR.660** |
|---|---|---|
| miR.558 + miR.609 + miR.603 + miR.220a + miR.26a + miR.29a + miR.369.3p + miR.376a* + miR.518c + miR.660 | 0,81 | NaN |
| miR.558 + miR.609 + miR.603 + miR.220a + miR.29a + miR.362 + miR.369.3p + miR.376a* + miR.518c + miR.660 | 0,81 | 0,242090457 |
| miR.558 + miR.609 + miR.603 + miR.520f + miR.220a + miR.29a + miR.369.3p + miR.376a* + miR.518c + miR.660 | 0,81 | 0,305884533 |
| miR.558 + miR.609 + miR.603 + miR.220a + miR.26a + miR.29a + miR.369.3p + miR.376a* + miR.518c + miR.338 | 0,81 | 0,496600392 |
| miR.558 + miR.609 + miR.603 + miR.220a + miR.26a + miR.29a + miR.362 + miR.369.3p + miR.376a* + miR.518c | 0,81 | 0,176882281 |
| miR.558 + miR.609 + miR.603 + miR.220a + miR.26a + miR.29a + miR.369.3p + miR.376a* + miR.518c + miR.639 | 0,81 | 0,209905826 |
| miR.558 + miR.609 + miR.603 + miR.220a + miR.26a + miR.29a + miR.369.3p + miR.376a* + miR.494 + miR.518c | 0,81 | 0,177508558 |
| miR.558 + miR.609 + miR.603 + miR.220a + miR.29a + miR.369.3p + miR.376a* + miR.518c + miR.639 + miR.660 | 0,81 | 0,13465496 |
| miR.558 + miR.609 + miR.603 + miR.220a + miR.519b + miR.29a + miR.369.3p + miR.376a* + miR.518c + miR.660 | 0,81 | 0,297307498 |
| miR.558 + miR.609 + miR.603 + miR.220a + miR.130a + miR.26a + miR.29a + miR.369.3p + miR.376a* + miR.518c | 0,81 | 0,223947224 |
| miR.558 + miR.609 + miR.603 + miR.520f + miR.220a + miR.26a + miR.29a + miR.369.3p + miR.376a* + miR.518c | 0,81 | 0,21718402 |
| miR.558 + miR.609 + miR.603 + miR.220a + miR.29a + miR.369.3p + miR.376a* + miR.494 + miR.518c + miR.660 | 0,81 | 0,157448882 |
| miR.558 + miR.609 + miR.603 + miR.220a + miR.29a + miR.369.3p + miR.376a* + miR.518c + miR.660 + miR.338 | 0,81 | 0,269392482 |
| miR.558 + miR.609 + miR.520f + miR.220a + miR.29a + miR.369.3p + miR.376a* + miR.518c + miR.660 + miR.338 | 0,81 | 0,382825458 |
| miR.558 + miR.609 + miR.603 + miR.220a + miR.130a + miR.29a + miR.369.3p + miR.376a* + miR.518c + miR.660 | 0,81 | 0,141825603 |
| miR.558 + miR.609 + miR.603 + miR.520f + miR.220a + miR.29a + miR.362 + miR.369.3p + miR.376a* + miR.518c | 0,81 | 0,070542282 |
| miR.558 + miR.609 + miR.520f + miR.29a + miR.362 + miR.369.3p + miR.376a* + miR.518c + miR.660 + miR.338 | 0,81 | 0,292740124 |
| miR.558 + miR.609 + miR.603 + miR.220a + miR.519b + miR.26a + miR.29a + miR.369.3p + miR.376a* + miR.518c | 0,81 | 0,384087979 |
| miR.558 + miR.609 + miR.603 + miR.220a + miR.519b + miR.29a + miR.362 + miR.369.3p + miR.376a* + miR.518c | 0,81 | 0,308180061 |
| miR.558 + miR.609 + miR.520f + miR.220a + miR.29a + miR.369.3p + miR.376a* + miR.518c + miR.639 + miR.660 | 0,81 | 0,37505175 |
| miR.558 + miR.609 + miR.603 + miR.220a + miR.519b + miR.130a + miR.29a + miR.369.3p + miR.376a* + miR.518c | 0,81 | 0,277532588 |
| miR.558 + miR.609 + miR.603 + miR.520f + miR.29a + miR.369.3p + miR.376a* + miR.518c + miR.639 + miR.338 | 0,81 | 0,146358228 |
| miR.558 + miR.609 + miR.520f + miR.220a + miR.29a + miR.369.3p + miR.376a* + miR.494 + miR.518c + miR.660 | 0,81 | 0,472431482 |
| miR.558 + miR.609 + miR.520f + miR.220a + miR.26a + miR.29a + miR.369.3p + miR.376a* + miR.518c + miR.660 | 0,81 | 0,295395022 |
| miR.558 + miR.609 + miR.603 + miR.520f + miR.220a + miR.519b + miR.29a + miR.369.3p + miR.376a* + miR.518c | 0,81 | 0,256455357 |
| miR.558 + miR.609 + miR.220a + miR.26a + miR.29a + miR.369.3p + miR.376a* + miR.494 + miR.518c + miR.660 | 0,81 | 0,307836339 |
| miR.558 + miR.609 + miR.520f + miR.29a + miR.369.3p + miR.376a* + miR.518c + miR.639 + miR.660 + miR.338 | 0,81 | 0,458941312 |
| miR.558 + miR.609 + miR.220a + miR.26a + miR.29a + miR.369.3p + miR.376a* + miR.518c + miR.660 + miR.338 | 0,81 | 0,08413771 |
| miR.558 + miR.609 + miR.603 + miR.220a + miR.519b + miR.29a + miR.369.3p + miR.376a* + miR.494 + miR.518c | 0,81 | 0,425031749 |
| miR.558 + miR.609 + miR.603 + miR.520f + miR.220a + miR.130a + miR.29a + miR.369.3p + miR.376a* + miR.518c | 0,81 | 0,073664811 |
| miR.558 + miR.609 + miR.520f + miR.220a + miR.29a + miR.362 + miR.369.3p + miR.376a* + miR.518c + miR.660 | 0,81 | 0,327162696 |
| miR.558 + miR.609 + miR.603 + miR.520f + miR.29a + miR.369.3p + miR.376a* + miR.518c + miR.660 + miR.338 | 0,81 | 0,415701525 |
| miR.558 + miR.609 + miR.220a + miR.26a + miR.29a + miR.369.3p + miR.376a* + miR.518c + miR.639 + miR.660 | 0,81 | 0,351475224 |
| miR.558 + miR.609 + miR.603 + miR.220a + miR.519b + miR.130a + miR.29a + miR.376a* + miR.494 + miR.518c | 0,81 | 0,072059664 |
| miR.558 + miR.609 + miR.520f + miR.220a + miR.130a + miR.29a + miR.369.3p + miR.376a* + miR.518c + miR.660 | 0,81 | 0,440507773 |
| miR.558 + miR.609 + miR.603 + miR.520f + miR.220a + miR.29a + miR.369.3p + miR.376a* + miR.518c + miR.338 | 0,81 | 0,269521529 |
| miR.558 + miR.609 + miR.520f + miR.220a + miR.519b + miR.29a + miR.369.3p + miR.376a* + miR.518c + miR.660 | 0,81 | 0,298687934 |
| miR.558 + miR.609 + miR.603 + miR.220a + miR.26a + miR.29a + miR.362 + miR.376a* + miR.494 + miR.518c | 0,81 | 0,004441999 |
| miR.558 + miR.609 + miR.220a + miR.26a + miR.29a + miR.362 + miR.369.3p + miR.376a* + miR.518c + miR.660 | 0,81 | 0,141098979 |
| miR.558 + miR.609 + miR.603 + miR.520f + miR.29a + miR.369.3p + miR.376a* + miR.518c + miR.639 + miR.660 | 0,81 | 0,063444211 |
| miR.558 + miR.609 + miR.603 + miR.220a + miR.130a + miR.29a + miR.369.3p + miR.376a* + miR.494 + miR.518c | 0,81 | 0,054972939 |
| miR.558 + miR.609 + miR.603 + miR.220a + miR.519b + miR.26a + miR.29a + miR.362 + miR.376a* + miR.518c | 0,81 | 0,064183216 |
| miR.558 + miR.609 + miR.603 + miR.220a + miR.519b + miR.29a + miR.369.3p + miR.376a* + miR.518c + miR.639 | 0,81 | 0,292553142 |
| miR.558 + miR.609 + miR.603 + miR.26a + miR.29a + miR.369.3p + miR.376a* + miR.518c + miR.639 + miR.338 | 0,81 | 0,378239996 |
| miR.558 + miR.609 + miR.603 + miR.520f + miR.220a + miR.519b + miR.29a + miR.376a* + miR.494 + miR.518c | 0,81 | 0,037056936 |
| miR.558 + miR.609 + miR.603 + miR.220a + miR.130a + miR.29a + miR.362 + miR.369.3p + miR.376a* + miR.518c | 0,81 | 0,050284986 |
| miR.558 + miR.609 + miR.220a + miR.29a + miR.362 + miR.369.3p + miR.376a* + miR.518c + miR.660 + miR.338 | 0,81 | 0,476759421 |
| miR.558 + miR.609 + miR.603 + miR.220a + miR.130a + miR.29a + miR.369.3p + miR.376a* + miR.518c + miR.639 | 0,81 | 0,017873439 |
| miR.558 + miR.609 + miR.603 + miR.520f + miR.29a + miR.369.3p + miR.376a* + miR.494 + miR.518c + miR.338 | 0,81 | 0,23623766 |
| miR.558 + miR.609 + miR.603 + miR.520f + miR.220a + miR.519b + miR.29a + miR.362 + miR.376a* + miR.518c | 0,81 | 0,114412749 |
| miR.558 + miR.609 + miR.603 + miR.520f + miR.26a + miR.29a + miR.369.3p + miR.376a* + miR.518c + miR.338 | 0,81 | 0,315070621 |
| miR.558 + miR.609 + miR.220a + miR.519b + miR.26a + miR.29a + miR.369.3p + miR.376a* + miR.518c + miR.660 | 0,81 | 0,228951442 |
| miR.558 + miR.609 + miR.520f + miR.220a + miR.26a + miR.29a + miR.369.3p + miR.376a* + miR.518c + miR.338 | 0,81 | 0,426409297 |
| miR.558 + miR.609 + miR.603 + miR.220a + miR.519b + miR.26a + miR.29a + miR.376a* + miR.494 + miR.518c | 0,81 | 0,04004517 |
| miR.558 + miR.609 + miR.603 + miR.220a + miR.26a + miR.29a + miR.376a* + miR.494 + miR.518c + miR.660 | 0,81 | 0,001870538 |
| miR.558 + miR.609 + miR.603 + miR.220a + miR.26a + miR.29a + miR.362 + miR.376a* + miR.518c + miR.639 | 0,81 | 0,006986126 |
| miR.558 + miR.609 + miR.603 + miR.520f + miR.130a + miR.29a + miR.369.3p + miR.376a* + miR.518c + miR.338 | 0,81 | 0,304421744 |
| miR.558 + miR.609 + miR.220a + miR.130a + miR.26a + miR.29a + miR.369.3p + miR.376a* + miR.518c + miR.660 | 0,81 | 0,216822953 |
| miR.558 + miR.609 + miR.603 + miR.220a + miR.519b + miR.29a + miR.362 + miR.376a* + miR.494 + miR.518c | 0,81 | 0,108357368 |
| miR.558 + miR.609 + miR.603 + miR.520f + miR.220a + miR.29a + miR.369.3p + miR.376a* + miR.518c + miR.639 | 0,81 | 0,03215356 |
| miR.558 + miR.609 + miR.603 + miR.520f + miR.519b + miR.29a + miR.369.3p + miR.376a* + miR.518c + miR.338 | 0,81 | 0,391842764 |
| miR.558 + miR.609 + miR.603 + miR.220a + miR.130a + miR.26a + miR.29a + miR.362 + miR.376a* + miR.518c | 0,81 | 0,003073458 |
| miR.558 + miR.609 + miR.520f + miR.220a + miR.29a + miR.362 + miR.369.3p + miR.376a* + miR.518c + miR.338 | 0,81 | 0,487671446 |
| miR.558 + miR.609 + miR.603 + miR.520f + miR.220a + miR.26a + miR.29a + miR.362 + miR.376a* + miR.518c | 0,81 | 0,005624385 |
| miR.558 + miR.609 + miR.220a + miR.130a + miR.29a + miR.362 + miR.369.3p + miR.376a* + miR.518c + miR.660 | 0,81 | 0,489205609 |
| miR.558 + miR.609 + miR.603 + miR.220a + miR.26a + miR.29a + miR.362 + miR.376a* + miR.518c + miR.338 | 0,81 | 0,032725563 |
| miR.558 + miR.609 + miR.603 + miR.520f + miR.220a + miR.29a + miR.369.3p + miR.376a* + miR.494 + miR.518c | 0,81 | 0,051081681 |
| miR.558 + miR.609 + miR.603 + miR.520f + miR.220a + miR.519b + miR.29a + miR.376a* + miR.518c + miR.660 | 0,81 | 0,063867955 |
| miR.558 + miR.609 + miR.520f + miR.29a + miR.369.3p + miR.376a* + miR.494 + miR.518c + miR.660 + miR.338 | 0,81 | 0,352231088 |
| miR.558 + miR.609 + miR.603 + miR.220a + miR.519b + miR.130a + miR.29a + miR.362 + miR.376a* + miR.518c | 0,81 | 0,091683373 |
| miR.558 + miR.609 + miR.603 + miR.520f + miR.220a + miR.29a + miR.376a* + miR.494 + miR.518c + miR.660 | 0,81 | 0,019374327 |
| miR.558 + miR.609 + miR.603 + miR.220a + miR.26a + miR.29a + miR.376a* + miR.494 + miR.518c + miR.639 | 0,81 | 0,000472352 |
| miR.558 + miR.609 + miR.220a + miR.130a + miR.29a + miR.369.3p + miR.376a* + miR.518c + miR.639 + miR.660 | 0,81 | 0,337869581 |
| miR.558 + miR.609 + miR.603 + miR.220a + miR.26a + miR.29a + miR.376a* + miR.494 + miR.518c + miR.338 | 0,81 | 0,007843322 |
| miR.558 + miR.609 + miR.603 + miR.220a + miR.519b + miR.26a + miR.29a + miR.376a* + miR.518c + miR.660 | 0,81 | 0,066267481 |
| miR.558 + miR.609 + miR.220a + miR.26a + miR.29a + miR.369.3p + miR.376a* + miR.494 + miR.518c + miR.338 | 0,81 | 0,333854732 |
| miR.558 + miR.609 + miR.603 + miR.220a + miR.519b + miR.29a + miR.362 + miR.376a* + miR.518c + miR.338 | 0,81 | 0,156363682 |
| miR.558 + miR.609 + miR.603 + miR.220a + miR.26a + miR.29a + miR.376a* + miR.518c + miR.639 + miR.660 | 0,81 | 0,001727364 |
| miR.558 + miR.609 + miR.603 + miR.220a + miR.519b + miR.26a + miR.29a + miR.376a* + miR.518c + miR.338 | 0,81 | 0,01944327 |
| miR.558 + miR.609 + miR.603 + miR.520f + miR.220a + miR.26a + miR.29a + miR.376a* + miR.518c + miR.660 | 0,81 | 0,013873658 |
| miR.558 + miR.609 + miR.603 + miR.220a + miR.519b + miR.29a + miR.376a* + miR.494 + miR.518c + miR.660 | 0,81 | 0,099460898 |
| miR.558 + miR.609 + miR.603 + miR.220a + miR.519b + miR.29a + miR.362 + miR.376a* + miR.518c + miR.639 | 0,81 | 0,131557674 |
| miR.558 + miR.609 + miR.603 + miR.520f + miR.29a + miR.362 + miR.369.3p + miR.376a* + miR.518c + miR.338 | 0,81 | 0,304698544 |
| miR.558 + miR.609 + miR.220a + miR.29a + miR.362 + miR.369.3p + miR.376a* + miR.494 + miR.518c + miR.660 | 0,81 | 0,464408364 |
| miR.558 + miR.609 + miR.520f + miR.220a + miR.29a + miR.369.3p + miR.376a* + miR.494 + miR.518c + miR.338 | 0,81 | 0,448232041 |
| miR.558 + miR.609 + miR.520f + miR.29a + miR.376a* + miR.494 + miR.518c + miR.639 + miR.660 + miR.338 | 0,81 | 0,230587013 |
| miR.558 + miR.609 + miR.603 + miR.520f + miR.220a + miR.29a + miR.376a* + miR.518c + miR.660 + miR.338 | 0,81 | 0,193908206 |
| miR.558 + miR.609 + miR.220a + miR.29a + miR.362 + miR.369.3p + miR.376a* + miR.518c + miR.639 + miR.660 | 0,81 | 0,475902061 |
| miR.558 + miR.609 + miR.603 + miR.520f + miR.220a + miR.29a + miR.362 + miR.376a* + miR.518c + miR.639 | 0,81 | 0,008531382 |
| miR.558 + miR.609 + miR.520f + miR.220a + miR.29a + miR.369.3p + miR.376a* + miR.518c + miR.639 + miR.338 | 0,81 | 0,276900513 |
| miR.558 + miR.609 + miR.603 + miR.220a + miR.26a + miR.29a + miR.362 + miR.376a* + miR.518c + miR.660 | 0,81 | 0,002224319 |
| miR.558 + miR.609 + miR.603 + miR.220a + miR.26a + miR.29a + miR.376a* + miR.518c + miR.639 + miR.338 | 0,81 | 0,002954162 |
| miR.558 + miR.609 + miR.603 + miR.220a + miR.130a + miR.26a + miR.29a + miR.376a* + miR.518c + miR.338 | 0,81 | 0,006050105 |
| miR.558 + miR.609 + miR.220a + miR.26a + miR.29a + miR.369.3p + miR.376a* + miR.518c + miR.639 + miR.338 | 0,81 | 0,458263005 |
| miR.558 + miR.609 + miR.603 + miR.520f + miR.220a + miR.29a + miR.362 + miR.376a* + miR.518c + miR.338 | 0,81 | 0,099135563 |
| miR.558 + miR.609 + miR.603 + miR.220a + miR.519b + miR.29a + miR.369.3p + miR.376a* + miR.518c + miR.338 | 0,81 | 0,364096223 |
| miR.558 + miR.609 + miR.520f + miR.220a + miR.29a + miR.376a* + miR.518c + miR.639 + miR.660 + miR.338 | 0,81 | 0,359569056 |
| miR.558 + miR.609 + miR.603 + miR.520f + miR.220a + miR.29a + miR.362 + miR.376a* + miR.494 + miR.518c | 0,81 | 0,00430911 |
| miR.558 + miR.609 + miR.603 + miR.220a + miR.130a + miR.26a + miR.29a + miR.376a* + miR.518c + miR.660 | 0,81 | 0,00188 |
| miR.558 + miR.609 + miR.603 + miR.520f + miR.26a + miR.29a + miR.369.3p + miR.376a* + miR.518c + miR.639 | 0,81 | 0,073848885 |
| miR.558 + miR.609 + miR.603 + miR.520f + miR.220a + miR.29a + miR.376a* + miR.518c + miR.639 + miR.660 | 0,81 | 0,051720828 |
| miR.558 + miR.609 + miR.603 + miR.220a + miR.29a + miR.362 + miR.369.3p + miR.376a* + miR.518c + miR.639 | 0,81 | 0,016477914 |
| miR.558 + miR.609 + miR.220a + miR.519b + miR.130a + miR.29a + miR.369.3p + miR.376a* + miR.518c + miR.660 | 0,81 | 0,286223061 |
| miR.558 + miR.609 + miR.520f + miR.220a + miR.26a + miR.29a + miR.369.3p + miR.376a* + miR.494 + miR.518c | 0,81 | 0,419018364 |
| miR.558 + miR.609 + miR.520f + miR.220a + miR.130a + miR.29a + miR.369.3p + miR.376a* + miR.518c + miR.338 | 0,81 | 0,393139492 |
| miR.558 + miR.609 + miR.220a + miR.26a + miR.29a + miR.362 + miR.369.3p + miR.376a* + miR.518c + miR.338 | 0,81 | 0,355492006 |
| miR.558 + miR.609 + miR.603 + miR.220a + miR.29a + miR.362 + miR.369.3p + miR.376a* + miR.494 + miR.518c | 0,81 | 0,04568601 |
| miR.558 + miR.609 + miR.520f + miR.220a + miR.26a + miR.29a + miR.362 + miR.369.3p + miR.376a* + miR.518c | 0,81 | 0,32893203 |
| miR.558 + miR.609 + miR.603 + miR.220a + miR.26a + miR.29a + miR.376a* + miR.518c + miR.660 + miR.338 | 0,81 | 0,038842008 |
| miR.558 + miR.609 + miR.603 + miR.220a + miR.519b + miR.29a + miR.362 + miR.376a* + miR.518c + miR.660 | 0,81 | 0,149408518 |
| miR.558 + miR.609 + miR.603 + miR.220a + miR.519b + miR.130a + miR.26a + miR.29a + miR.376a* + miR.518c | 0,81 | 0,010456894 |
| miR.558 + miR.609 + miR.603 + miR.220a + miR.29a + miR.362 + miR.369.3p + miR.376a* + miR.518c + miR.338 | 0,81 | 0,095445321 |
| miR.558 + miR.609 + miR.603 + miR.220a + miR.29a + miR.369.3p + miR.376a* + miR.494 + miR.518c + miR.338 | 0,81 | 0,076569129 |
| miR.558 + miR.609 + miR.603 + miR.220a + miR.29a + miR.369.3p + miR.376a* + miR.494 + miR.518c + miR.639 | 0,81 | 0,015134717 |
| miR.558 + miR.609 + miR.603 + miR.220a + miR.29a + miR.369.3p + miR.376a* + miR.518c + miR.639 + miR.338 | 0,81 | 0,058817566 |
| miR.558 + miR.609 + miR.220a + miR.26a + miR.29a + miR.362 + miR.369.3p + miR.376a* + miR.494 + miR.518c | 0,81 | 0,487975487 |
| miR.558 + miR.609 + miR.520f + miR.220a + miR.29a + miR.376a* + miR.494 + miR.518c + miR.660 + miR.338 | 0,81 | 0,257463392 |
| miR.558 + miR.609 + miR.220a + miR.130a + miR.29a + miR.369.3p + miR.376a* + miR.494 + miR.518c + miR.660 | 0,81 | 0,308349348 |
| miR.558 + miR.609 + miR.220a + miR.26a + miR.29a + miR.369.3p + miR.376a* + miR.494 + miR.518c + miR.639 | 0,81 | 0,471868104 |
| miR.558 + miR.609 + miR.220a + miR.130a + miR.26a + miR.29a + miR.369.3p + miR.376a* + miR.518c + miR.338 | 0,81 | 0,36201 6475 |
| miR.558 + miR.609 + miR.520f + miR.130a + miR.29a + miR.369.3p + miR.376a* + miR.518c + miR.660 + miR.338 | 0,81 | 0,313361348 |
| miR.558 + miR.609 + miR.603 + miR.520f + miR.29a + miR.369.3p + miR.376a* + miR.494 + miR.518c + miR.660 | 0,81 | 0,071084446 |
| miR.558 + miR.609 + miR.603 + miR.520f + miR.220a + miR.519b + miR.29a + miR.376a* + miR.518c + miR.338 | 0,81 | 0,089838817 |
| miR.558 + miR.609 + miR.520f + miR.130a + miR.29a + miR.369.3p + miR.376a* + miR.518c + miR.639 + miR.660 | 0,81 | 0,116329817 |
| miR.558 + miR.609 + miR.520f + miR.220a + miR.29a + miR.362 + miR.369.3p + miR.376a* + miR.494 + miR.518c | 0,81 | 0,252782252 |
| miR.558 + miR.609 + miR.603 + miR.26a + miR.29a + miR.369.3p + miR.376a* + miR.494 + miR.518c + miR.338 | 0,81 | 0,450726322 |
| miR.558 + miR.609 + miR.520f + miR.220a + miR.26a + miR.29a + miR.369.3p + miR.376a* + miR.518c + miR.639 | 0,81 | 0,283689117 |
| miR.558 + miR.609 + miR.603 + miR.26a + miR.29a + miR.369.3p + miR.376a* + miR.494 + miR.518c + miR.639 | 0,81 | 0,212059129 |
| miR.558 + miR.609 + miR.603 + miR.520f + miR.220a + miR.130a + miR.29a + miR.362 + miR.376a* + miR.518c | 0,81 | 0,007645967 |
| miR.558 + miR.609 + miR.520f + miR.26a + miR.29a + miR.369.3p + miR.376a* + miR.518c + miR.660 + miR.338 | 0,81 | 0,402354453 |
| miR.558 + miR.609 + miR.220a + miR.519b + miR.29a + miR.362 + miR.369.3p + miR.376a* + miR.518c + miR.660 | 0,81 | 0,469190746 |
| miR.558 + miR.609 + miR.520f + miR.220a + miR.29a + miR.362 + miR.369.3p + miR.376a* + miR.518c + miR.639 | 0,81 | 0,240452421 |
| miR.558 + miR.609 + miR.603 + miR.26a + miR.29a + miR.369.3p + miR.376a* + miR.518c + miR.639 + miR.660 | 0,81 | 0,231762576 |
| miR.558 + miR.609 + miR.220a + miR.519b + miR.26a + miR.29a + miR.369.3p + miR.376a* + miR.518c + miR.338 | 0,81 | 0,384843321 |
| miR.558 + miR.609 + miR.220a + miR.519b + miR.26a + miR.29a + miR.369.3p + miR.376a* + miR.494 + miR.518c | 0,81 | 0,449405178 |
| miR.558 + miR.609 + miR.603 + miR.130a + miR.26a + miR.29a + miR.369.3p + miR.376a* + miR.518c + miR.639 | 0,81 | 0,199361251 |
| miR.558 + miR.609 + miR.520f + miR.220a + miR.519b + miR.29a + miR.376a* + miR.494 + miR.518c + miR.660 | 0,81 | 0,210817077 |
| miR.558 + miR.609 + miR.603 + miR.220a + miR.130a + miR.26a + miR.29a + miR.376a* + miR.494 + miR.518c | 0,81 | 0,001027791 |
| miR.558 + miR.609 + miR.603 + miR.520f + miR.29a + miR.369.3p + miR.376a* + miR.494 + miR.518c + miR.639 | 0,81 | 0,044394297 |
| miR.558 + miR.609 + miR.603 + miR.520f + miR.29a + miR.362 + miR.369.3p + miR.376a* + miR.518c + miR.639 | 0,81 | 0,039881187 |
| miR.558 + miR.609 + miR.220a + miR.519b + miR.29a + miR.369.3p + miR.376a* + miR.494 + miR.518c + miR.660 | 0,81 | 0,326020377 |
| miR.558 + miR.609 + miR.520f + miR.519b + miR.29a + miR.369.3p + miR.376a* + miR.518c + miR.660 + miR.338 | 0,81 | 0,42508147 |
| miR.558 + miR.609 + miR.603 + miR.520f + miR.29a + miR.362 + miR.376a* + miR.518c + miR.639 + miR.338 | 0,81 | 0,094439382 |
| miR.558 + miR.609 + miR.220a + miR.26a + miR.29a + miR.362 + miR.369.3p + miR.376a* + miR.518c + miR.639 | 0,81 | 0,394201952 |
| miR.558 + miR.609 + miR.603 + miR.520f + miR.29a + miR.376a* + miR.518c + miR.639 + miR.660 + miR.338 | 0,81 | 0,204337264 |
| miR.558 + miR.609 + miR.603 + miR.220a + miR.130a + miR.29a + miR.369.3p + miR.376a* + miR.518c + miR.338 | 0,81 | 0,104079509 |
| miR.558 + miR.609 + miR.220a + miR.130a + miR.26a + miR.29a + miR.369.3p + miR.376a* + miR.518c + miR.639 | 0,81 | 0,322168393 |
| miR.558 + miR.609 + miR.603 + miR.520f + miR.220a + miR.29a + miR.362 + miR.376a* + miR.518c + miR.660 | 0,81 | 0,020820914 |
| miR.558 + miR.609 + miR.220a + miR.130a + miR.26a + miR.29a + miR.369.3p + miR.376a* + miR.494 + miR.518c | 0,81 | 0,471599647 |
| miR.558 + miR.609 + miR.520f + miR.220a + miR.519b + miR.29a + miR.369.3p + miR.376a* + miR.518c + miR.338 | 0,81 | 0,496153956 |
| miR.558 + miR.609 + miR.520f + miR.220a + miR.130a + miR.29a + miR.362 + miR.369.3p + miR.376a* + miR.518c | 0,81 | 0,263908247 |
| miR.558 + miR.609 + miR.603 + miR.520f + miR.220a + miR.130a + miR.29a + miR.376a* + miR.518c + miR.660 | 0,81 | 0,022160449 |
| miR.558 + miR.609 + miR.603 + miR.220a + miR.130a + miR.26a + miR.29a + miR.376a* + miR.518c + miR.639 | 0,81 | 0,00070945 |
| miR.558 + miR.609 + miR.520f + miR.220a + miR.29a + miR.376a* + miR.494 + miR.518c + miR.639 + miR.660 | 0,81 | 0,15161367 |
| miR.558 + miR.609 + miR.603 + miR.520f + miR.519b + miR.29a + miR.369.3p + miR.376a* + miR.518c + miR.639 | 0,81 | 0,069421065 |
| miR.558 + miR.609 + miR.603 + miR.26a + miR.29a + miR.369.3p + miR.376a* + miR.518c + miR.660 + miR.338 | 0,81 | 0,480125378 |
| miR.558 + miR.609 + miR.520f + miR.220a + miR.519b + miR.29a + miR.362 + miR.369.3p + miR.376a* + miR.518c | 0,81 | 0,306260109 |
| miR.558 + miR.609 + miR.520f + miR.26a + miR.29a + miR.369.3p + miR.376a* + miR.518c + miR.639 + miR.338 | 0,81 | 0,303184497 |
| miR.558 + miR.609 + miR.603 + miR.520f + miR.220a + miR.26a + miR.29a + miR.376a* + miR.518c + miR.639 | 0,81 | 0,001824984 |
| miR.558 + miR.609 + miR.603 + miR.220a + miR.519b + miR.29a + miR.376a* + miR.518c + miR.660 + miR.338 | 0,81 | 0,189955258 |
| miR.558 + miR.609 + miR.603 + miR.520f + miR.220a + miR.29a + miR.376a* + miR.494 + miR.518c + miR.338 | 0,81 | 0,035803136 |
| miR.558 + miR.609 + miR.603 + miR.520f + miR.220a + miR.26a + miR.29a + miR.376a* + miR.494 + miR.518c | 0,81 | 0,001336434 |
| miR.558 + miR.609 + miR.603 + miR.520f + miR.220a + miR.26a + miR.29a + miR.376a* + miR.518c + miR.338 | 0,81 | 0,025600957 |
| miR.558 + miR.609 + miR.520f + miR.29a + miR.369.3p + miR.376a* + miR.494 + miR.518c + miR.639 + miR.338 | 0,81 | 0,202237208 |
| miR.558 + miR.609 + miR.520f + miR.220a + miR.130a + miR.26a + miR.29a + miR.369.3p + miR.376a* + miR.518c | 0,81 | 0,347964318 |
| miR.558 + miR.609 + miR.603 + miR.520f + miR.519b + miR.29a + miR.369.3p + miR.376a* + miR.494 + miR.518c | 0,81 | 0,108055295 |
| miR.558 + miR.609 + miR.603 + miR.520f + miR.26a + miR.29a + miR.369.3p + miR.376a* + miR.518c + miR.660 | 0,81 | 0,041141359 |
| miR.558 + miR.609 + miR.603 + miR.130a + miR.29a + miR.369.3p + miR.376a* + miR.518c + miR.639 + miR.338 | 0,81 | 0,111738164 |
| miR.558 + miR.609 + miR.603 + miR.520f + miR.130a + miR.29a + miR.369.3p + miR.376a* + miR.518c + miR.639 | 0,81 | 0,044132835 |
| miR.558 + miR.609 + miR.603 + miR.520f + miR.26a + miR.29a + miR.369.3p + miR.376a* + miR.494 + miR.518c | 0,81 | 0,096065859 |
| miR.558 + miR.609 + miR.520f + miR.130a + miR.29a + miR.376a* + miR.518c + miR.639 + miR.660 + miR.338 | 0,81 | 0,234279085 |
| miR.558 + miR.609 + miR.603 + miR.220a + miR.519b + miR.29a + miR.376a* + miR.494 + miR.518c + miR.639 | 0,81 | 0,033482371 |
| miR.558 + miR.609 + miR.520f + miR.130a + miR.29a + miR.369.3p + miR.376a* + miR.494 + miR.518c + miR.660 | 0,81 | 0,080162974 |
| miR.558 + miR.609 + miR.520f + miR.220a + miR.519b + miR.29a + miR.362 + miR.376a* + miR.494 + miR.518c | 0,81 | 0,287359831 |
| miR.558 + miR.609 + miR.520f + miR.519b + miR.29a + miR.376a* + miR.494 + miR.518c + miR.660 + miR.338 | 0,81 | 0,186070849 |
| miR.558 + miR.609 + miR.220a + miR.29a + miR.369.3p + miR.376a* + miR.494 + miR.518c + miR.660 + miR.338 | 0,81 | 0,252498532 |
| miR.558 + miR.609 + miR.603 + miR.520f + miR.220a + miR.519b + miR.26a + miR.29a + miR.376a* + miR.518c | 0,81 | 0,008098556 |
| miR.558 + miR.609 + miR.520f + miR.220a + miR.519b + miR.26a + miR.29a + miR.369.3p + miR.376a* + miR.518c | 0,81 | 0,385959252 |
| miR.558 + miR.609 + miR.603 + miR.520f + miR.220a + miR.130a + miR.26a + miR.29a + miR.376a* + miR.518c | 0,81 | 0,002504812 |
| miR.558 + miR.609 + miR.220a + miR.519b + miR.26a + miR.29a + miR.362 + miR.369.3p + miR.376a* + miR.518c | 0,81 | 0,382855731 |
| miR.558 + miR.609 + miR.520f + miR.220a + miR.130a + miR.29a + miR.369.3p + miR.376a* + miR.494 + miR.518c | 0,81 | 0,246307193 |
| miR.558 + miR.609 + miR.220a + miR.519b + miR.130a + miR.26a + miR.29a + miR.369.3p + miR.376a* + miR.518c | 0,81 | 0,359670745 |
| miR.558 + miR.609 + miR.520f + miR.130a + miR.29a + miR.376a* + miR.494 + miR.518c + miR.639 + miR.660 | 0,81 | 0,082870159 |
| miR.558 + miR.609 + miR.220a + miR.29a + miR.369.3p + miR.376a* + miR.494 + miR.518c + miR.639 + miR.660 | 0,81 | 0,23614188 |
| miR.558 + miR.609 + miR.603 + miR.520f + miR.29a + miR.362 + miR.369.3p + miR.376a* + miR.518c + miR.660 | 0,81 | 0,215991569 |
| miR.558 + miR.609 + miR.603 + miR.520f + miR.130a + miR.29a + miR.369.3p + miR.376a* + miR.518c + miR.660 | 0,81 | 0,060077038 |
| miR.558 + miR.609 + miR.520f + miR.220a + miR.29a + miR.369.3p + miR.376a* + miR.494 + miR.518c + miR.639 | 0,81 | 0,171419098 |
| miR.558 + miR.609 + miR.603 + miR.520f + miR.29a + miR.376a* + miR.494 + miR.518c + miR.639 + miR.338 | 0,81 | 0,031423091 |
| miR.558 + miR.609 + miR.603 + miR.26a + miR.29a + miR.362 + miR.369.3p + miR.376a* + miR.518c + miR.338 | 0,81 | 0,473666173 |
| miR.558 + miR.609 + miR.603 + miR.130a + miR.26a + miR.29a + miR.369.3p + miR.376a* + miR.518c + miR.338 | 0,81 | 0,450664408 |
| miR.558 + miR.609 + miR.220a + miR.29a + miR.369.3p + miR.376a* + miR.518c + miR.639 + miR.660 + miR.338 | 0,81 | 0,316633041 |
| miR.558 + miR.609 + miR.220a + miR.519b + miR.29a + miR.369.3p + miR.376a* + miR.518c + miR.639 + miR.660 | 0,81 | 0,31322339 |
| miR.558 + miR.609 + miR.220a + miR.519b + miR.29a + miR.369.3p + miR.376a* + miR.518c + miR.660 + miR.338 | 0,81 | 0,407468907 |
| miR.558 + miR.609 + miR.603 + miR.26a + miR.29a + miR.369.3p + miR.376a* + miR.494 + miR.518c + miR.660 | 0,81 | 0,254326465 |
| miR.558 + miR.609 + miR.603 + miR.520f + miR.220a + miR.519b + miR.29a + miR.376a* + miR.518c + miR.639 | 0,81 | 0,01008574 |
| miR.558 + miR.609 + miR.220a + miR.130a + miR.26a + miR.29a + miR.362 + miR.369.3p + miR.376a* + miR.518c | 0,81 | 0,358881729 |
| miR.558 + miR.609 + miR.520f + miR.29a + miR.362 + miR.369.3p + miR.376a* + miR.518c + miR.639 + miR.338 | 0,81 | 0,229324595 |
| miR.558 + miR.609 + miR.603 + miR.520f + miR.519b + miR.29a + miR.376a* + miR.494 + miR.518c + miR.338 | 0,81 | 0,158335392 |
| miR.558 + miR.609 + miR.603 + miR.520f + miR.220a + miR.519b + miR.130a + miR.29a + miR.376a* + miR.518c | 0,81 | 0,011335269 |
| miR.558 + miR.609 + miR.603 + miR.519b + miR.26a + miR.29a + miR.369.3p + miR.376a* + miR.518c + miR.338 | 0,81 | 0,480661116 |
| miR.558 + miR.609 + miR.520f + miR.220a + miR.519b + miR.29a + miR.369.3p + miR.376a* + miR.494 + miR.518c | 0,81 | 0,356384924 |
| miR.558 + miR.609 + miR.520f + miR.220a + miR.26a + miR.29a + miR.376a* + miR.518c + miR.660 + miR.338 | 0,81 | 0,279741056 |
| miR.558 + miR.609 + miR.603 + miR.220a + miR.519b + miR.26a + miR.29a + miR.376a* + miR.518c + miR.639 | 0,81 | 0,009120381 |
| miR.558 + miR.609 + miR.520f + miR.220a + miR.130a + miR.29a + miR.376a* + miR.518c + miR.660 + miR.338 | 0,81 | 0,27193668 |
| miR.558 + miR.609 + miR.603 + miR.519b + miR.26a + miR.29a + miR.369.3p + miR.376a* + miR.518c + miR.639 | 0,81 | 0,205050527 |
| miR.558 + miR.609 + miR.603 + miR.220a + miR.130a + miR.29a + miR.376a* + miR.494 + miR.518c + miR.639 | 0,81 | 0,000348129 |
| miR.558 + miR.609 + miR.220a + miR.519b + miR.26a + miR.29a + miR.369.3p + miR.376a* + miR.518c + miR.639 | 0,81 | 0,388486849 |
| miR.558 + miR.609 + miR.520f + miR.220a + miR.29a + miR.362 + miR.376a* + miR.518c + miR.660 + miR.338 | 0,81 | 0,320250759 |
| miR.558 + miR.609 + miR.220a + miR.130a + miR.29a + miR.369.3p + miR.376a* + miR.518c + miR.660 + miR.338 | 0,81 | 0,480642513 |
| miR.558 + miR.609 + miR.520f + miR.220a + miR.519b + miR.29a + miR.362 + miR.376a* + miR.518c + miR.338 | 0,81 | 0,376650107 |
| miR.558 + miR.609 + miR.520f + miR.220a + miR.29a + miR.362 + miR.376a* + miR.494 + miR.518c + miR.338 | 0,81 | 0,281795082 |
| miR.558 + miR.609 + miR.603 + miR.29a + miR.362 + miR.369.3p + miR.376a* + miR.518c + miR.660 + miR.338 | 0,81 | 0,273430203 |
| miR.558 + miR.609 + miR.520f + miR.220a + miR.519b + miR.29a + miR.376a* + miR.518c + miR.660 + miR.338 | 0,81 | 0,456961795 |
| miR.558 + miR.609 + miR.603 + miR.26a + miR.29a + miR.362 + miR.369.3p + miR.376a* + miR.518c + miR.639 | 0,81 | 0,200255509 |
| miR.558 + miR.609 + miR.520f + miR.220a + miR.29a + miR.362 + miR.376a* + miR.494 + miR.518c + miR.660 | 0,81 | 0,08991085 |
| miR.558 + miR.609 + miR.603 + miR.520f + miR.130a + miR.26a + miR.29a + miR.369.3p + miR.376a* + miR.518c | 0,81 | 0,059566171 |
| miR.558 + miR.609 + miR.603 + miR.29a + miR.362 + miR.369.3p + miR.376a* + miR.518c + miR.639 + miR.660 | 0,81 | 0,264461638 |
| miR.558 + miR.609 + miR.603 + miR.220a + miR.519b + miR.29a + miR.376a* + miR.518c + miR.639 + miR.338 | 0,81 | 0,023753572 |
| miR.558 + miR.609 + miR.603 + miR.130a + miR.29a + miR.369.3p + miR.376a* + miR.494 + miR.518c + miR.639 | 0,81 | 0,073592704 |
| miR.558 + miR.609 + miR.520f + miR.220a + miR.26a + miR.29a + miR.376a* + miR.494 + miR.518c + miR.660 | 0,81 | 0,085052412 |
| miR.558 + miR.609 + miR.520f + miR.220a + miR.29a + miR.362 + miR.376a* + miR.518c + miR.639 + miR.338 | 0,81 | 0,185195833 |
| miR.558 + miR.609 + miR.603 + miR.220a + miR.519b + miR.130a + miR.29a + miR.376a* + miR.518c + miR.338 | 0,81 | 0,020120546 |
| miR.558 + miR.609 + miR.603 + miR.520f + miR.220a + miR.130a + miR.29a + miR.376a* + miR.518c + miR.639 | 0,81 | 0,000981724 |
| miR.558 + miR.609 + miR.603 + miR.220a + miR.130a + miR.29a + miR.362 + miR.376a* + miR.518c + miR.639 | 0,81 | 0,00504257 |
| miR.558 + miR.609 + miR.520f + miR.220a + miR.130a + miR.29a + miR.376a* + miR.494 + miR.518c + miR.660 | 0,81 | 0,073850466 |
| miR.558 + miR.609 + miR.520f + miR.29a + miR.362 + miR.376a* + miR.518c + miR.639 + miR.660 + miR.338 | 0,81 | 0,263320303 |
| miR.558 + miR.609 + miR.520f + miR.130a + miR.29a + miR.369.3p + miR.376a* + miR.494 + miR.518c + miR.639 | 0,81 | 0,108101911 |
| miR.558 + miR.609 + miR.603 + miR.220a + miR.519b + miR.130a + miR.29a + miR.376a* + miR.518c + miR.639 | 0,81 | 0,011210467 |
| miR.558 + miR.609 + miR.520f + miR.26a + miR.29a + miR.376a* + miR.518c + miR.639 + miR.660 + miR.338 | 0,81 | 0,222430836 |
| miR.558 + miR.609 + miR.520f + miR.519b + miR.29a + miR.376a* + miR.518c + miR.639 + miR.660 + miR.338 | 0,81 | 0,244405577 |
| miR.558 + miR.609 + miR.520f + miR.29a + miR.362 + miR.369.3p + miR.376a* + miR.518c + miR.639 + miR.660 | 0,81 | 0,248454565 |
| miR.558 + miR.609 + miR.603 + miR.26a + miR.29a + miR.362 + miR.369.3p + miR.376a* + miR.518c + miR.660 | 0,81 | 0,382633637 |
| miR.558 + miR.609 + miR.520f + miR.130a + miR.29a + miR.369.3p + miR.376a* + miR.518c + miR.639 + miR.338 | 0,81 | 0,236366899 |
| miR.558 + miR.609 + miR.603 + miR.520f + miR.519b + miR.29a + miR.369.3p + miR.376a* + miR.518c + miR.660 | 0,81 | 0,053852408 |
| miR.558 + miR.609 + miR.603 + miR.520f + miR.26a + miR.29a + miR.362 + miR.369.3p + miR.376a* + miR.518c | 0,81 | 0,079708776 |
| miR.558 + miR.609 + miR.603 + miR.520f + miR.130a + miR.29a + miR.369.3p + miR.376a* + miR.494 + miR.518c | 0,81 | 0,065575769 |
| miR.558 + miR.609 + miR.603 + miR.220a + miR.130a + miR.29a + miR.362 + miR.376a* + miR.494 + miR.518c | 0,81 | 0,001381817 |
| miR.558 + miR.609 + miR.520f + miR.220a + miR.130a + miR.29a + miR.369.3p + miR.376a* + miR.518c + miR.639 | 0,81 | 0,137192282 |
| miR.558 + miR.609 + miR.603 + miR.220a + miR.519b + miR.130a + miR.29a + miR.376a* + miR.518c + miR.660 | 0,81 | 0,107219978 |
| miR.558 + miR.609 + miR.220a + miR.519b + miR.130a + miR.29a + miR.369.3p + miR.376a* + miR.494 + miR.518c | 0,81 | 0,324848373 |
| miR.558 + miR.609 + miR.603 + miR.220a + miR.29a + miR.376a* + miR.518c + miR.639 + miR.660 + miR.338 | 0,81 | 0,015660171 |
| miR.558 + miR.609 + miR.520f + miR.26a + miR.29a + miR.369.3p + miR.376a* + miR.494 + miR.518c + miR.338 | 0,81 | 0,322097373 |
| miR.558 + miR.609 + miR.603 + miR.26a + miR.29a + miR.362 + miR.369.3p + miR.376a* + miR.494 + miR.518c | 0,81 | 0,307177464 |
| miR.558 + miR.609 + miR.520f + miR.220a + miR.29a + miR.362 + miR.376a* + miR.494 + miR.518c + miR.639 | 0,81 | 0,065097 |
| miR.558 + miR.609 + miR.520f + miR.26a + miR.29a + miR.376a* + miR.494 + miR.518c + miR.660 + miR.338 | 0,81 | 0,232021177 |
| miR.558 + miR.609 + miR.603 + miR.220a + miR.519b + miR.29a + miR.376a* + miR.518c + miR.639 + miR.660 | 0,81 | 0,133199324 |
| miR.558 + miR.609 + miR.603 + miR.130a + miR.26a + miR.29a + miR.369.3p + miR.376a* + miR.494 + miR.518c | 0,81 | 0,292852361 |
| miR.558 + miR.609 + miR.603 + miR.220a + miR.29a + miR.362 + miR.376a* + miR.518c + miR.639 + miR.338 | 0,81 | 0,005024632 |
| miR.558 + miR.609 + miR.603 + miR.520f + miR.29a + miR.362 + miR.369.3p + miR.376a* + miR.494 + miR.518c | 0,81 | 0,060906889 |
| miR.558 + miR.609 + miR.520f + miR.29a + miR.362 + miR.376a* + miR.494 + miR.518c + miR.660 + miR.338 | 0,81 | 0,159914432 |
| miR.558 + miR.609 + miR.520f + miR.130a + miR.29a + miR.376a* + miR.494 + miR.518c + miR.660 + miR.338 | 0,81 | 0,209755519 |
| miR.558 + miR.609 + miR.603 + miR.520f + miR.220a + miR.130a + miR.29a + miR.376a* + miR.494 + miR.518c | 0,81 | 0,002621169 |
| miR.558 + miR.609 + miR.220a + miR.26a + miR.29a + miR.376a* + miR.494 + miR.518c + miR.639 + miR.660 | 0,81 | 0,097978868 |
| miR.558 + miR.609 + miR.520f + miR.220a + miR.519b + miR.29a + miR.369.3p + miR.376a* + miR.518c + miR.639 | 0,81 | 0,263790625 |
| miR.609 + miR.603 + miR.520f + miR.220a + miR.29a + miR.369.3p + miR.376a* + miR.494 + miR.518c + miR.660 | 0,81 | 0,40479337 |
| miR.558 + miR.609 + miR.520f + miR.519b + miR.29a + miR.369.3p + miR.376a* + miR.494 + miR.518c + miR.338 | 0,81 | 0,35591504 |
| miR.558 + miR.609 + miR.603 + miR.520f + miR.29a + miR.376a* + miR.494 + miR.518c + miR.660 + miR.338 | 0,81 | 0,235368241 |
| miR.558 + miR.609 + miR.520f + miR.519b + miR.29a + miR.369.3p + miR.376a* + miR.518c + miR.639 + miR.660 | 0,81 | 0,080957183 |
| miR.558 + miR.609 + miR.520f + miR.29a + miR.369.3p + miR.376a* + miR.494 + miR.518c + miR.639 + miR.660 | 0,81 | 0,099097856 |
| miR.609 + miR.603 + miR.220a + miR.519b + miR.29a + miR.369.3p + miR.376a* + miR.494 + miR.518c + miR.660 | 0,81 | 0,39848711 |
| miR.558 + miR.609 + miR.603 + miR.519b + miR.26a + miR.29a + miR.369.3p + miR.376a* + miR.494 + miR.518c | 0,81 | 0,275215499 |
| miR.558 + miR.609 + miR.603 + miR.520f + miR.29a + miR.376a* + miR.494 + miR.518c + miR.639 + miR.660 | 0,81 | 0,02392215 |
| miR.558 + miR.609 + miR.520f + miR.220a + miR.26a + miR.29a + miR.362 + miR.376a* + miR.518c + miR.338 | 0,81 | 0,262963178 |
| miR.558 + miR.609 + miR.603 + miR.520f + miR.29a + miR.362 + miR.376a* + miR.494 + miR.518c + miR.338 | 0,81 | 0,155122084 |
| miR.558 + miR.609 + miR.520f + miR.29a + miR.362 + miR.369.3p + miR.376a* + miR.494 + miR.518c + miR.338 | 0,81 | 0,25689938 |
| miR.558 + miR.609 + miR.520f + miR.220a + miR.130a + miR.29a + miR.362 + miR.376a* + miR.518c + miR.338 | 0,81 | 0,334384744 |
| miR.558 + miR.609 + miR.603 + miR.220a + miR.29a + miR.376a* + miR.494 + miR.518c + miR.639 + miR.660 | 0,81 | 0,015683767 |
| miR.558 + miR.609 + miR.603 + miR.520f + miR.519b + miR.26a + miR.29a + miR.369.3p + miR.376a* + miR.518c | 0,81 | 0,124969108 |
| miR.558 + miR.609 + miR.520f + miR.130a + miR.29a + miR.369.3p + miR.376a* + miR.494 + miR.518c + miR.338 | 0,81 | 0,28283847 |
| miR.558 + miR.609 + miR.26a + miR.29a + miR.362 + miR.369.3p + miR.376a* + miR.518c + miR.660 + miR.338 | 0,81 | 0,329265895 |
| miR.558 + miR.609 + miR.520f + miR.220a + miR.519b + miR.130a + miR.29a + miR.369.3p + miR.376a* + miR.518c | 0,81 | 0,287853034 |
| miR.558 + miR.609 + miR.220a + miR.130a + miR.29a + miR.369.3p + miR.376a* + miR.518c + miR.639 + miR.338 | 0,81 | 0,20598416 |
| miR.558 + miR.609 + miR.603 + miR.220a + miR.130a + miR.29a + miR.376a* + miR.494 + miR.518c + miR.660 | 0,81 | 0,002888413 |
| miR.558 + miR.609 + miR.603 + miR.130a + miR.29a + miR.369.3p + miR.376a* + miR.518c + miR.639 + miR.660 | 0,81 | 0,073738899 |
| miR.558 + miR.609 + miR.220a + miR.130a + miR.26a + miR.29a + miR.376a* + miR.494 + miR.518c + miR.660 | 0,81 | 0,138336111 |
| miR.558 + miR.609 + miR.520f + miR.519b + miR.29a + miR.369.3p + miR.376a* + miR.518c + miR.639 + miR.338 | 0,81 | 0,263983098 |
| miR.558 + miR.609 + miR.520f + miR.220a + miR.29a + miR.362 + miR.376a* + miR.518c + miR.639 + miR.660 | 0,81 | 0,1472095 |
| miR.558 + miR.609 + miR.603 + miR.520f + miR.130a + miR.29a + miR.362 + miR.369.3p + miR.376a* + miR.518c | 0,81 | 0,05517958 |
| miR.558 + miR.609 + miR.603 + miR.519b + miR.26a + miR.29a + miR.369.3p + miR.376a* + miR.518c + miR.660 | 0,81 | 0,175306949 |
| miR.558 + miR.609 + miR.603 + miR.520f + miR.519b + miR.29a + miR.362 + miR.369.3p + miR.376a* + miR.518c | 0,81 | 0,064756459 |
| miR.558 + miR.609 + miR.603 + miR.220a + miR.130a + miR.29a + miR.362 + miR.376a* + miR.518c + miR.338 | 0,81 | 0,006001262 |
| miR.558 + miR.609 + miR.130a + miR.29a + miR.362 + miR.369.3p + miR.376a* + miR.518c + miR.639 + miR.660 | 0,81 | 0,252405915 |
| miR.558 + miR.609 + miR.220a + miR.130a + miR.29a + miR.362 + miR.369.3p + miR.376a* + miR.518c + miR.639 | 0,81 | 0,147640619 |
| miR.558 + miR.609 + miR.520f + miR.130a + miR.29a + miR.362 + miR.369.3p + miR.376a* + miR.518c + miR.660 | 0,81 | 0,160650467 |
| miR.558 + miR.609 + miR.603 + miR.520f + miR.29a + miR.362 + miR.376a* + miR.494 + miR.518c + miR.639 | 0,81 | 0,018157199 |
| miR.558 + miR.609 + miR.603 + miR.520f + miR.519b + miR.29a + miR.376a* + miR.518c + miR.639 + miR.338 | 0,81 | 0,0746739 |
| miR.558 + miR.609 + miR.603 + miR.130a + miR.26a + miR.29a + miR.369.3p + miR.376a* + miR.518c + miR.660 | 0,81 | 0,176148762 |
| miR.558 + miR.609 + miR.603 + miR.520f + miR.130a + miR.29a + miR.376a* + miR.518c + miR.639 + miR.338 | 0,81 | 0,027896681 |
| miR.558 + miR.609 + miR.520f + miR.220a + miR.519b + miR.29a + miR.376a* + miR.518c + miR.639 + miR.660 | 0,81 | 0,110719455 |
| miR.609 + miR.603 + miR.220a + miR.29a + miR.362 + miR.369.3p + miR.376a* + miR.494 + miR.518c + miR.660 | 0,81 | 0,447289899 |
| miR.558 + miR.609 + miR.220a + miR.26a + miR.29a + miR.362 + miR.376a* + miR.494 + miR.518c + miR.639 | 0,81 | 0,130242993 |
| miR.558 + miR.609 + miR.520f + miR.220a + miR.130a + miR.29a + miR.362 + miR.376a* + miR.494 + miR.518c | 0,81 | 0,141157911 |
| miR.558 + miR.609 + miR.520f + miR.220a + miR.26a + miR.29a + miR.376a* + miR.518c + miR.639 + miR.660 | 0,81 | 0,122398943 |
| miR.558 + miR.609 + miR.520f + miR.220a + miR.26a + miR.29a + miR.362 + miR.376a* + miR.494 + miR.518c | 0,81 | 0,092904362 |
| miR.558 + miR.609 + miR.220a + miR.519b + miR.130a + miR.29a + miR.369.3p + miR.376a* + miR.518c + miR.338 | 0,81 | 0,414797687 |
| miR.558 + miR.609 + miR.603 + miR.520f + miR.519b + miR.130a + miR.29a + miR.369.3p + miR.376a* + miR.518c | 0,81 | 0,074887432 |
| miR.558 + miR.609 + miR.520f + miR.130a + miR.26a + miR.29a + miR.369.3p + miR.376a* + miR.518c + miR.660 | 0,81 | 0,062741407 |
| miR.558 + miR.609 + miR.520f + miR.220a + miR.519b + miR.29a + miR.362 + miR.376a* + miR.518c + miR.639 | 0,81 | 0,175724939 |
| miR.558 + miR.609 + miR.603 + miR.520f + miR.26a + miR.29a + miR.376a* + miR.518c + miR.639 + miR.338 | 0,81 | 0,023148698 |
| miR.558 + miR.609 + miR.520f + miR.130a + miR.29a + miR.376a* + miR.494 + miR.518c + miR.639 + miR.338 | 0,81 | 0,050371527 |
| miR.558 + miR.609 + miR.220a + miR.519b + miR.130a + miR.29a + miR.362 + miR.369.3p + miR.376a* + miR.518c | 0,81 | 0,34228867 |
| miR.558 + miR.609 + miR.520f + miR.220a + miR.519b + miR.26a + miR.29a + miR.362 + miR.376a* + miR.518c | 0,81 | 0,211983257 |
| miR.558 + miR.609 + miR.520f + miR.220a + miR.29a + miR.376a* + miR.494 + miR.518c + miR.639 + miR.338 | 0,81 | 0,068578966 |
| miR.558 + miR.609 + miR.520f + miR.26a + miR.29a + miR.369.3p + miR.376a* + miR.518c + miR.639 + miR.660 | 0,81 | 0,095372662 |
| miR.558 + miR.609 + miR.520f + miR.220a + miR.130a + miR.29a + miR.376a* + miR.518c + miR.639 + miR.660 | 0,81 | 0,137704734 |
| miR.558 + miR.609 + miR.220a + miR.26a + miR.29a + miR.362 + miR.376a* + miR.494 + miR.518c + miR.338 | 0,81 | 0,235173704 |
| miR.558 + miR.609 + miR.520f + miR.220a + miR.519b + miR.29a + miR.362 + miR.376a* + miR.518c + miR.660 | 0,81 | 0,128233558 |
| miR.558 + miR.609 + miR.520f + miR.26a + miR.29a + miR.362 + miR.369.3p + miR.376a* + miR.518c + miR.338 | 0,81 | 0,337022388 |
| miR.558 + miR.609 + miR.520f + miR.220a + miR.519b + miR.130a + miR.29a + miR.362 + miR.376a* + miR.518c | 0,81 | 0,208433596 |
| miR.609 + miR.603 + miR.220a + miR.26a + miR.29a + miR.369.3p + miR.376a* + miR.494 + miR.518c + miR.660 | 0,81 | 0,4191519 |
| miR.558 + miR.609 + miR.603 + miR.520f + miR.220a + miR.29a + miR.376a* + miR.518c + miR.639 + miR.338 | 0,81 | 0,028388447 |
| miR.558 + miR.609 + miR.220a + miR.130a + miR.26a + miR.29a + miR.362 + miR.376a* + miR.494 + miR.518c | 0,81 | 0,210834278 |
| miR.558 + miR.609 + miR.220a + miR.519b + miR.130a + miR.29a + miR.369.3p + miR.376a* + miR.518c + miR.639 | 0,81 | 0,212381828 |
| miR.558 + miR.609 + miR.520f + miR.130a + miR.29a + miR.362 + miR.376a* + miR.494 + miR.518c + miR.639 | 0,81 | 0,050364553 |
| miR.558 + miR.609 + miR.520f + miR.220a + miR.26a + miR.29a + miR.362 + miR.376a* + miR.518c + miR.639 | 0,81 | 0,059177851 |
| miR.558 + miR.609 + miR.130a + miR.26a + miR.29a + miR.369.3p + miR.376a* + miR.494 + miR.518c + miR.639 | 0,81 | 0,12648103 |
| miR.558 + miR.609 + miR.603 + miR.220a + miR.29a + miR.362 + miR.376a* + miR.518c + miR.639 + miR.660 | 0,81 | 0,008465347 |
| miR.609 + miR.603 + miR.520f + miR.220a + miR.130a + miR.29a + miR.369.3p + miR.376a* + miR.518c + miR.660 | 0,81 | 0,420460443 |
| miR.558 + miR.609 + miR.520f + miR.29a + miR.362 + miR.369.3p + miR.376a* + miR.494 + miR.518c + miR.660 | 0,81 | 0,163184669 |
| miR.558 + miR.609 + miR.603 + miR.520f + miR.519b + miR.29a + miR.362 + miR.376a* + miR.494 + miR.518c | 0,81 | 0,039497864 |
| miR.558 + miR.609 + miR.520f + miR.220a + miR.519b + miR.29a + miR.376a* + miR.494 + miR.518c + miR.338 | 0,81 | 0,18456671 |
| miR.558 + miR.609 + miR.520f + miR.220a + miR.519b + miR.26a + miR.29a + miR.376a* + miR.518c + miR.660 | 0,81 | 0,082198902 |
| miR.558 + miR.609 + miR.520f + miR.130a + miR.29a + miR.362 + miR.369.3p + miR.376a* + miR.518c + miR.338 | 0,81 | 0,290246432 |
| miR.558 + miR.609 + miR.520f + miR.220a + miR.130a + miR.29a + miR.362 + miR.376a* + miR.518c + miR.639 | 0,81 | 0,103543937 |
| miR.558 + miR.609 + miR.603 + miR.519b + miR.130a + miR.29a + miR.369.3p + miR.376a* + miR.494 + miR.518c | 0,81 | 0,289155068 |
| miR.558 + miR.609 + miR.603 + miR.520f + miR.130a + miR.29a + miR.362 + miR.376a* + miR.518c + miR.338 | 0,81 | 0,195929136 |
| miR.558 + miR.609 + miR.520f + miR.220a + miR.519b + miR.130a + miR.29a + miR.376a* + miR.518c + miR.660 | 0,81 | 0,087787187 |
| miR.558 + miR.609 + miR.603 + miR.519b + miR.130a + miR.26a + miR.29a + miR.369.3p + miR.376a* + miR.518c | 0,81 | 0,229457517 |
| miR.558 + miR.609 + miR.603 + miR.220a + miR.130a + miR.29a + miR.376a* + miR.518c + miR.639 + miR.660 | 0,81 | 0,007679135 |
| miR.558 + miR.609 + miR.520f + miR.220a + miR.26a + miR.29a + miR.376a* + miR.494 + miR.518c + miR.338 | 0,81 | 0,111046985 |
| miR.558 + miR.609 + miR.520f + miR.130a + miR.26a + miR.29a + miR.369.3p + miR.376a* + miR.518c + miR.338 | 0,81 | 0,331151321 |
| miR.558 + miR.609 + miR.603 + miR.130a + miR.26a + miR.29a + miR.362 + miR.369.3p + miR.376a* + miR.518c | 0,81 | 0,280057814 |
| miR.558 + miR.609 + miR.520f + miR.130a + miR.26a + miR.29a + miR.369.3p + miR.376a* + miR.518c + miR.639 | 0,81 | 0,123952603 |
| miR.558 + miR.609 + miR.603 + miR.130a + miR.29a + miR.362 + miR.369.3p + miR.376a* + miR.518c + miR.639 | 0,8 | 0,05897204 |
| miR.558 + miR.609 + miR.603 + miR.220a + miR.29a + miR.376a* + miR.494 + miR.518c + miR.660 + miR.338 | 0,8 | 0,031541686 |
| miR.558 + miR.609 + miR.603 + miR.520f + miR.220a + miR.29a + miR.376a* + miR.494 + miR.518c + miR.639 | 0,8 | 0,001099932 |
| miR.558 + miR.609 + miR.220a + miR.519b + miR.26a + miR.29a + miR.362 + miR.376a* + miR.494 + miR.518c | 0,8 | 0,190198659 |
| miR.558 + miR.609 + miR.220a + miR.130a + miR.29a + miR.362 + miR.369.3p + miR.376a* + miR.494 + miR.518c | 0,8 | 0,171463275 |
| miR.558 + miR.609 + miR.220a + miR.130a + miR.29a + miR.369.3p + miR.376a* + miR.494 + miR.518c + miR.639 | 0,8 | 0,202808687 |
| miR.558 + miR.609 + miR.603 + miR.519b + miR.26a + miR.29a + miR.362 + miR.369.3p + miR.376a* + miR.518c | 0,8 | 0,261471368 |
| miR.558 + miR.609 + miR.220a + miR.519b + miR.26a + miR.29a + miR.376a* + miR.494 + miR.518c + miR.660 | 0,8 | 0,214274152 |
| miR.558 + miR.609 + miR.520f + miR.220a + miR.26a + miR.29a + miR.362 + miR.376a* + miR.518c + miR.660 | 0,8 | 0,088071967 |
| miR.558 + miR.609 + miR.603 + miR.520f + miR.519b + miR.29a + miR.376a* + miR.494 + miR.518c + miR.639 | 0,8 | 0,021161889 |
| miR.558 + miR.609 + miR.603 + miR.520f + miR.130a + miR.29a + miR.376a* + miR.518c + miR.660 + miR.338 | 0,8 | 0,261272665 |
| miR.558 + miR.609 + miR.520f + miR.220a + miR.130a + miR.26a + miR.29a + miR.376a* + miR.518c + miR.660 | 0,8 | 0,062487763 |
| miR.558 + miR.609 + miR.220a + miR.130a + miR.29a + miR.362 + miR.369.3p + miR.376a* + miR.518c + miR.338 | 0,8 | 0,234837862 |
| miR.558 + miR.609 + miR.520f + miR.29a + miR.362 + miR.376a* + miR.494 + miR.518c + miR.639 + miR.338 | 0,8 | 0,09822688 |
| miR.609 + miR.603 + miR.520f + miR.220a + miR.29a + miR.362 + miR.369.3p + miR.376a* + miR.518c + miR.660 | 0,8 | 0,292431465 |
| miR.558 + miR.609 + miR.220a + miR.130a + miR.29a + miR.369.3p + miR.376a* + miR.494 + miR.518c + miR.338 | 0,8 | 0,252903768 |
| miR.558 + miR.609 + miR.520f + miR.220a + miR.130a + miR.26a + miR.29a + miR.362 + miR.376a* + miR.518c | 0,8 | 0,099774745 |
| miR.558 + miR.609 + miR.603 + miR.520f + miR.26a + miR.29a + miR.376a* + miR.494 + miR.518c + miR.338 | 0,8 | 0,041986961 |
| miR.558 + miR.609 + miR.520f + miR.220a + miR.130a + miR.29a + miR.376a* + miR.494 + miR.518c + miR.338 | 0,8 | 0,170273859 |
| miR.558 + miR.609 + miR.603 + miR.520f + miR.220a + miR.130a + miR.29a + miR.376a* + miR.518c + miR.338 | 0,8 | 0,053892411 |
| miR.558 + miR.609 + miR.220a + miR.519b + miR.130a + miR.26a + miR.29a + miR.376a* + miR.494 + miR.518c | 0,8 | 0,089278645 |
| miR.609 + miR.603 + miR.220a + miR.130a + miR.26a + miR.29a + miR.369.3p + miR.376a* + miR.518c + miR.660 | 0,8 | 0,478869728 |
| miR.558 + miR.609 + miR.520f + miR.519b + miR.130a + miR.29a + miR.369.3p + miR.376a* + miR.518c + miR.338 | 0,8 | 0,322922403 |
| miR.558 + miR.609 + miR.520f + miR.26a + miR.29a + miR.369.3p + miR.376a* + miR.494 + miR.518c + miR.639 | 0,8 | 0,121240501 |
| miR.558 + miR.609 + miR.520f + miR.220a + miR.130a + miR.29a + miR.362 + miR.376a* + miR.518c + miR.660 | 0,8 | 0,078987383 |
| miR.558 + miR.609 + miR.603 + miR.220a + miR.29a + miR.362 + miR.376a* + miR.518c + miR.660 + miR.338 | 0,8 | 0,024503351 |
| miR.558 + miR.609 + miR.520f + miR.130a + miR.26a + miR.29a + miR.369.3p + miR.376a* + miR.494 + miR.518c | 0,8 | 0,085308895 |
| miR.558 + miR.609 + miR.603 + miR.520f + miR.130a + miR.29a + miR.376a* + miR.494 + miR.518c + miR.338 | 0,8 | 0,059234491 |
| miR.558 + miR.609 + miR.603 + miR.519b + miR.130a + miR.29a + miR.369.3p + miR.376a* + miR.518c + miR.639 | 0,8 | 0,104002014 |
| miR.558 + miR.609 + miR.520f + miR.519b + miR.130a + miR.29a + miR.369.3p + miR.376a* + miR.494 + miR.518c | 0,8 | 0,147864848 |
| miR.558 + miR.609 + miR.603 + miR.220a + miR.130a + miR.29a + miR.362 + miR.376a* + miR.518c + miR.660 | 0,8 | 0,008460968 |
| miR.558 + miR.609 + miR.603 + miR.520f + miR.26a + miR.29a + miR.362 + miR.376a* + miR.518c + miR.639 | 0,8 | 0,011457893 |
| miR.558 + miR.609 + miR.220a + miR.519b + miR.130a + miR.29a + miR.376a* + miR.494 + miR.518c + miR.660 | 0,8 | 0,112733405 |
| miR.558 + miR.609 + miR.220a + miR.26a + miR.29a + miR.376a* + miR.494 + miR.518c + miR.660 + miR.338 | 0,8 | 0,252848414 |
| miR.609 + miR.603 + miR.220a + miR.29a + miR.362 + miR.369.3p + miR.376a* + miR.518c + miR.639 + miR.660 | 0,8 | 0,480306281 |
| miR.558 + miR.609 + miR.520f + miR.519b + miR.29a + miR.362 + miR.369.3p + miR.376a* + miR.518c + miR.338 | 0,8 | 0,343418956 |
| miR.558 + miR.609 + miR.603 + miR.220a + miR.130a + miR.29a + miR.376a* + miR.518c + miR.660 + miR.338 | 0,8 | 0,020350316 |
| miR.558 + miR.609 + miR.520f + miR.519b + miR.26a + miR.29a + miR.369.3p + miR.376a* + miR.518c + miR.338 | 0,8 | 0,396120627 |
| miR.558 + miR.609 + miR.603 + miR.520f + miR.519b + miR.29a + miR.362 + miR.376a* + miR.518c + miR.338 | 0,8 | 0,183284615 |
| miR.558 + miR.609 + miR.220a + miR.519b + miR.130a + miR.29a + miR.362 + miR.376a* + miR.494 + miR.518c | 0,8 | 0,095177228 |
| miR.558 + miR.609 + miR.520f + miR.26a + miR.29a + miR.369.3p + miR.376a* + miR.494 + miR.518c + miR.660 | 0,8 | 0,077009346 |
| miR.609 + miR.603 + miR.520f + miR.220a + miR.29a + miR.369.3p + miR.376a* + miR.518c + miR.660 + miR.338 | 0,8 | 0,230976952 |
| miR.558 + miR.609 + miR.603 + miR.520f + miR.130a + miR.29a + miR.362 + miR.376a* + miR.518c + miR.639 | 0,8 | 0,013924943 |
| miR.558 + miR.609 + miR.520f + miR.220a + miR.519b + miR.130a + miR.29a + miR.376a* + miR.494 + miR.518c | 0,8 | 0,142359538 |
| miR.558 + miR.609 + miR.603 + miR.520f + miR.519b + miR.29a + miR.362 + miR.376a* + miR.518c + miR.639 | 0,8 | 0,015721883 |
| miR.558 + miR.609 + miR.130a + miR.29a + miR.369.3p + miR.376a* + miR.494 + miR.518c + miR.639 + miR.660 | 0,8 | 0,124896992 |
| miR.558 + miR.609 + miR.603 + miR.29a + miR.362 + miR.369.3p + miR.376a* + miR.494 + miR.518c + miR.660 | 0,8 | 0,134276714 |
| miR.609 + miR.603 + miR.220a + miR.519b + miR.26a + miR.29a + miR.369.3p + miR.376a* + miR.494 + miR.518c | 0,8 | 0,47236884 |
| miR.558 + miR.609 + miR.220a + miR.519b + miR.26a + miR.29a + miR.376a* + miR.494 + miR.518c + miR.338 | 0,8 | 0,119641425 |
| miR.558 + miR.609 + miR.520f + miR.26a + miR.29a + miR.362 + miR.376a* + miR.518c + miR.660 + miR.338 | 0,8 | 0,257438553 |
| miR.609 + miR.603 + miR.520f + miR.220a + miR.29a + miR.369.3p + miR.376a* + miR.518c + miR.639 + miR.660 | 0,8 | 0,328956297 |
| miR.558 + miR.609 + miR.603 + miR.520f + miR.26a + miR.29a + miR.362 + miR.376a* + miR.518c + miR.338 | 0,8 | 0,136991518 |
| miR.609 + miR.603 + miR.220a + miR.29a + miR.369.3p + miR.376a* + miR.494 + miR.518c + miR.639 + miR.660 | 0,8 | 0,313470786 |
| miR.558 + miR.609 + miR.603 + miR.520f + miR.130a + miR.29a + miR.376a* + miR.494 + miR.518c + miR.639 | 0,8 | 0,008372877 |
| miR.558 + miR.609 + miR.520f + miR.220a + miR.26a + miR.29a + miR.376a* + miR.518c + miR.639 + miR.338 | 0,8 | 0,059321615 |
| miR.558 + miR.609 + miR.603 + miR.520f + miR.29a + miR.362 + miR.376a* + miR.518c + miR.639 + miR.660 | 0,8 | 0,030320104 |
| miR.558 + miR.609 + miR.220a + miR.519b + miR.29a + miR.369.3p + miR.376a* + miR.494 + miR.518c + miR.639 | 0,8 | 0,321233184 |
| miR.558 + miR.609 + miR.220a + miR.26a + miR.29a + miR.362 + miR.376a* + miR.494 + miR.518c + miR.660 | 0,8 | 0,130349807 |
| miR.558 + miR.609 + miR.130a + miR.26a + miR.29a + miR.369.3p + miR.376a* + miR.518c + miR.639 + miR.660 | 0,8 | 0,09332473 |
| miR.558 + miR.609 + miR.603 + miR.520f + miR.26a + miR.29a + miR.376a* + miR.518c + miR.639 + miR.660 | 0,8 | 0,018658566 |
| miR.558 + miR.609 + miR.603 + miR.220a + miR.29a + miR.362 + miR.376a* + miR.494 + miR.518c + miR.660 | 0,8 | 0,010783662 |
| miR.558 + miR.609 + miR.520f + miR.29a + miR.362 + miR.369.3p + miR.376a* + miR.494 + miR.518c + miR.639 | 0,8 | 0,064170212 |
| miR.558 + miR.609 + miR.603 + miR.520f + miR.26a + miR.29a + miR.376a* + miR.494 + miR.518c + miR.639 | 0,8 | 0,005965356 |
| miR.558 + miR.609 + miR.520f + miR.519b + miR.29a + miR.369.3p + miR.376a* + miR.494 + miR.518c + miR.660 | 0,8 | 0,101150324 |
| miR.558 + miR.609 + miR.520f + miR.220a + miR.519b + miR.29a + miR.376a* + miR.494 + miR.518c + miR.639 | 0,8 | 0,047708118 |
| miR.558 + miR.609 + miR.520f + miR.220a + miR.519b + miR.26a + miR.29a + miR.376a* + miR.494 + miR.518c | 0,8 | 0,067717943 |
| miR.558 + miR.609 + miR.603 + miR.520f + miR.519b + miR.130a + miR.29a + miR.376a* + miR.494 + miR.518c | 0,8 | 0,037239584 |
| miR.558 + miR.609 + miR.603 + miR.520f + miR.519b + miR.29a + miR.376a* + miR.518c + miR.660 + miR.338 | 0,8 | 0,250456953 |
| miR.558 + miR.609 + miR.603 + miR.520f + miR.130a + miR.29a + miR.376a* + miR.518c + miR.639 + miR.660 | 0,8 | 0,027696717 |
| miR.558 + miR.609 + miR.603 + miR.520f + miR.29a + miR.362 + miR.376a* + miR.518c + miR.660 + miR.338 | 0,8 | 0,297239077 |
| miR.558 + miR.609 + miR.603 + miR.520f + miR.26a + miR.29a + miR.376a* + miR.518c + miR.660 + miR.338 | 0,8 | 0,296430343 |
| miR.558 + miR.609 + miR.220a + miR.519b + miR.26a + miR.29a + miR.376a* + miR.494 + miR.518c + miR.639 | 0,8 | 0,081806724 |
| miR.558 + miR.609 + miR.603 + miR.520f + miR.519b + miR.26a + miR.29a + miR.376a* + miR.494 + miR.518c | 0,8 | 0,021534962 |
| miR.558 + miR.609 + miR.520f + miR.519b + miR.26a + miR.29a + miR.376a* + miR.518c + miR.660 + miR.338 | 0,8 | 0,314668653 |
| miR.558 + miR.609 + miR.603 + miR.520f + miR.519b + miR.29a + miR.376a* + miR.518c + miR.639 + miR.660 | 0,8 | 0,024442943 |
| miR.558 + miR.609 + miR.520f + miR.26a + miR.29a + miR.362 + miR.369.3p + miR.376a* + miR.518c + miR.660 | 0,8 | 0,161361511 |
| miR.558 + miR.609 + miR.603 + miR.520f + miR.519b + miR.29a + miR.376a* + miR.494 + miR.518c + miR.660 | 0,8 | 0,022472817 |
| miR.609 + miR.603 + miR.220a + miR.29a + miR.362 + miR.369.3p + miR.376a* + miR.518c + miR.660 + miR.338 | 0,8 | 0,418900132 |
| miR.558 + miR.609 + miR.220a + miR.130a + miR.26a + miR.29a + miR.376a* + miR.494 + miR.518c + miR.338 | 0,8 | 0,079389399 |
| miR.558 + miR.609 + miR.220a + miR.519b + miR.26a + miR.29a + miR.362 + miR.376a* + miR.518c + miR.639 | 0,8 | 0,182886545 |
| miR.558 + miR.609 + miR.220a + miR.130a + miR.26a + miR.29a + miR.376a* + miR.494 + miR.518c + miR.639 | 0,8 | 0,048278412 |
| miR.558 + miR.609 + miR.220a + miR.26a + miR.29a + miR.376a* + miR.494 + miR.518c + miR.639 + miR.338 | 0,8 | 0,096115174 |
| miR.558 + miR.609 + miR.520f + miR.519b + miR.29a + miR.362 + miR.369.3p + miR.376a* + miR.518c + miR.660 | 0,8 | 0,106707357 |
| miR.609 + miR.603 + miR.220a + miR.519b + miR.130a + miR.29a + miR.369.3p + miR.376a* + miR.494 + miR.518c | 0,8 | 0,361619292 |
| miR.558 + miR.609 + miR.603 + miR.220a + miR.519b + miR.29a + miR.376a* + miR.494 + miR.518c + miR.338 | 0,8 | 0,040619312 |
| miR.558 + miR.609 + miR.220a + miR.519b + miR.26a + miR.29a + miR.362 + miR.376a* + miR.518c + miR.338 | 0,8 | 0,225550488 |
| miR.558 + miR.609 + miR.520f + miR.220a + miR.519b + miR.29a + miR.376a* + miR.518c + miR.639 + miR.338 | 0,8 | 0,104143064 |
| miR.558 + miR.609 + miR.520f + miR.519b + miR.29a + miR.369.3p + miR.376a* + miR.494 + miR.518c + miR.639 | 0,8 | 0,084569552 |
| miR.558 + miR.609 + miR.220a + miR.26a + miR.29a + miR.362 + miR.376a* + miR.518c + miR.639 + miR.338 | 0,8 | 0,226878972 |
| miR.558 + miR.609 + miR.520f + miR.220a + miR.130a + miR.29a + miR.376a* + miR.518c + miR.639 + miR.338 | 0,8 | 0,076866019 |
| miR.558 + miR.609 + miR.520f + miR.220a + miR.519b + miR.130a + miR.29a + miR.376a* + miR.518c + miR.338 | 0,8 | 0,128513794 |
| miR.558 + miR.609 + miR.520f + miR.220a + miR.26a + miR.29a + miR.376a* + miR.494 + miR.518c + miR.639 | 0,8 | 0,019413714 |
| miR.558 + miR.609 + miR.603 + miR.130a + miR.29a + miR.369.3p + miR.376a* + miR.494 + miR.518c + miR.338 | 0,8 | 0,094248495 |
| miR.558 + miR.609 + miR.520f + miR.29a + miR.362 + miR.369.3p + miR.376a* + miR.494 + miR.660 + miR.338 | 0,8 | 0,454067465 |
| miR.558 + miR.609 + miR.520f + miR.220a + miR.130a + miR.26a + miR.29a + miR.376a* + miR.518c + miR.338 | 0,8 | 0,11857397 |
| miR.558 + miR.609 + miR.520f + miR.519b + miR.29a + miR.362 + miR.376a* + miR.518c + miR.660 + miR.338 | 0,8 | 0,267168221 |
| miR.558 + miR.609 + miR.603 + miR.519b + miR.29a + miR.369.3p + miR.376a* + miR.518c + miR.639 + miR.338 | 0,8 | 0,135096255 |
| miR.609 + miR.603 + miR.220a + miR.26a + miR.29a + miR.362 + miR.369.3p + miR.376a* + miR.518c + miR.660 | 0,8 | 0,449112306 |
| miR.558 + miR.609 + miR.520f + miR.519b + miR.130a + miR.29a + miR.376a* + miR.518c + miR.639 + miR.660 | 0,8 | 0,035307844 |
| miR.558 + miR.609 + miR.520f + miR.130a + miR.26a + miR.29a + miR.376a* + miR.518c + miR.639 + miR.660 | 0,8 | 0,035307844 |
| miR.558 + miR.609 + miR.603 + miR.519b + miR.29a + miR.362 + miR.369.3p + miR.376a* + miR.518c + miR.660 | 0,8 | 0,113283862 |
| miR.558 + miR.609 + miR.520f + miR.130a + miR.29a + miR.362 + miR.376a* + miR.518c + miR.639 + miR.660 | 0,8 | 0,035307844 |
| miR.558 + miR.609 + miR.220a + miR.26a + miR.29a + miR.376a* + miR.518c + miR.639 + miR.660 + miR.338 | 0,8 | 0,178404706 |
| miR.558 + miR.609 + miR.220a + miR.130a + miR.29a + miR.376a* + miR.494 + miR.518c + miR.639 + miR.660 | 0,8 | 0,061816912 |
| miR.558 + miR.609 + miR.520f + miR.519b + miR.130a + miR.29a + miR.376a* + miR.494 + miR.518c + miR.338 | 0,8 | 0,119518264 |
| miR.558 + miR.609 + miR.520f + miR.26a + miR.29a + miR.362 + miR.376a* + miR.518c + miR.639 + miR.338 | 0,8 | 0,118487619 |
| miR.558 + miR.609 + miR.520f + miR.220a + miR.519b + miR.26a + miR.29a + miR.376a* + miR.518c + miR.338 | 0,8 | 0,105949407 |
| miR.558 + miR.609 + miR.520f + miR.26a + miR.29a + miR.362 + miR.369.3p + miR.376a* + miR.518c + miR.639 | 0,8 | 0,117792797 |
| miR.558 + miR.609 + miR.603 + miR.520f + miR.130a + miR.26a + miR.29a + miR.376a* + miR.518c + miR.338 | 0,8 | 0,042590319 |
| miR.558 + miR.609 + miR.603 + miR.220a + miR.130a + miR.29a + miR.376a* + miR.494 + miR.518c + miR.338 | 0,8 | 0,000354017 |
| miR.558 + miR.609 + miR.519b + miR.130a + miR.26a + miR.29a + miR.369.3p + miR.376a* + miR.518c + miR.639 | 0,8 | 0,102098744 |
| miR.558 + miR.609 + miR.520f + miR.220a + miR.130a + miR.29a + miR.376a* + miR.494 + miR.518c + miR.639 | 0,8 | 0,030243424 |
| miR.558 + miR.609 + miR.220a + miR.130a + miR.26a + miR.29a + miR.362 + miR.376a* + miR.518c + miR.338 | 0,8 | 0,234142517 |
| miR.558 + miR.609 + miR.220a + miR.26a + miR.29a + miR.362 + miR.376a* + miR.518c + miR.639 + miR.660 | 0,8 | 0,101833869 |
| miR.558 + miR.609 + miR.220a + miR.519b + miR.29a + miR.362 + miR.369.3p + miR.376a* + miR.518c + miR.639 | 0,8 | 0,338633389 |
| miR.558 + miR.609 + miR.130a + miR.26a + miR.29a + miR.369.3p + miR.376a* + miR.518c + miR.639 + miR.338 | 0,8 | 0,165168227 |
| miR.558 + miR.609 + miR.603 + miR.29a + miR.369.3p + miR.376a* + miR.518c + miR.639 + miR.660 + miR.338 | 0,8 | 0,124131098 |
| miR.558 + miR.609 + miR.520f + miR.519b + miR.26a + miR.29a + miR.369.3p + miR.376a* + miR.518c + miR.639 | 0,8 | 0,119651463 |
| miR.558 + miR.609 + miR.603 + miR.130a + miR.29a + miR.369.3p + miR.376a* + miR.494 + miR.518c + miR.660 | 0,8 | 0,044916097 |
| miR.558 + miR.609 + miR.520f + miR.26a + miR.29a + miR.376a* + miR.494 + miR.518c + miR.639 + miR.338 | 0,8 | 0,050187896 |
| miR.558 + miR.609 + miR.220a + miR.130a + miR.26a + miR.29a + miR.362 + miR.376a* + miR.518c + miR.639 | 0,8 | 0,176800046 |
| miR.558 + miR.609 + miR.220a + miR.519b + miR.130a + miR.26a + miR.29a + miR.362 + miR.376a* + miR.518c | 0,8 | 0,182652059 |
| miR.558 + miR.609 + miR.520f + miR.519b + miR.130a + miR.29a + miR.376a* + miR.494 + miR.518c + miR.660 | 0,8 | 0,043934059 |
| miR.558 + miR.609 + miR.603 + miR.130a + miR.29a + miR.362 + miR.369.3p + miR.376a* + miR.518c + miR.660 | 0,8 | 0,17812419 |
| miR.609 + miR.603 + miR.520f + miR.220a + miR.519b + miR.29a + miR.369.3p + miR.376a* + miR.518c + miR.660 | 0,8 | 0,388360738 |
| miR.558 + miR.609 + miR.520f + miR.29a + miR.362 + miR.376a* + miR.494 + miR.518c + miR.639 + miR.660 | 0,8 | 0,031409716 |
| miR.558 + miR.609 + miR.520f + miR.519b + miR.29a + miR.376a* + miR.494 + miR.518c + miR.639 + miR.338 | 0,8 | 0,08377831 |
| miR.558 + miR.609 + miR.520f + miR.220a + miR.130a + miR.26a + miR.29a + miR.376a* + miR.494 + miR.518c | 0,8 | 0,033215535 |
| miR.558 + miR.609 + miR.603 + miR.520f + miR.130a + miR.29a + miR.362 + miR.376a* + miR.494 + miR.518c | 0,8 | 0,012219591 |
| miR.558 + miR.609 + miR.520f + miR.130a + miR.29a + miR.362 + miR.376a* + miR.518c + miR.639 + miR.338 | 0,8 | 0,107574466 |
| miR.558 + miR.609 + miR.520f + miR.519b + miR.29a + miR.362 + miR.376a* + miR.518c + miR.639 + miR.338 | 0,8 | 0,17090723 |
| miR.609 + miR.603 + miR.520f + miR.220a + miR.26a + miR.29a + miR.369.3p + miR.376a* + miR.518c + miR.660 | 0,8 | 0,323412626 |
| miR.558 + miR.609 + miR.603 + miR.520f + miR.519b + miR.130a + miR.29a + miR.376a* + miR.518c + miR.338 | 0,8 | 0,097680191 |
| miR.558 + miR.609 + miR.220a + miR.519b + miR.26a + miR.29a + miR.376a* + miR.518c + miR.639 + miR.660 | 0,8 | 0,096384101 |
| miR.558 + miR.609 + miR.519b + miR.26a + miR.29a + miR.369.3p + miR.376a* + miR.518c + miR.639 + miR.338 | 0,8 | 0,262497855 |
| miR.558 + miR.609 + miR.603 + miR.520f + miR.26a + miR.29a + miR.362 + miR.376a* + miR.494 + miR.518c | 0,8 | 0,010450007 |
| miR.558 + miR.609 + miR.520f + miR.130a + miR.29a + miR.362 + miR.376a* + miR.494 + miR.518c + miR.660 | 0,8 | 0,029097308 |
| miR.558 + miR.609 + miR.603 + miR.29a + miR.369.3p + miR.376a* + miR.494 + miR.518c + miR.639 + miR.338 | 0,8 | 0,06745849 |
| miR.609 + miR.603 + miR.220a + miR.130a + miR.29a + miR.369.3p + miR.376a* + miR.494 + miR.518c + miR.660 | 0,8 | 0,283883726 |
| miR.558 + miR.609 + miR.603 + miR.520f + miR.29a + miR.362 + miR.376a* + miR.494 + miR.518c + miR.660 | 0,8 | 0,017728004 |
| miR.558 + miR.609 + miR.26a + miR.29a + miR.362 + miR.369.3p + miR.376a* + miR.518c + miR.639 + miR.660 | 0,8 | 0,273769934 |
| miR.558 + miR.609 + miR.603 + miR.520f + miR.130a + miR.26a + miR.29a + miR.376a* + miR.518c + miR.639 | 0,8 | 0,002350801 |
| miR.558 + miR.609 + miR.220a + miR.519b + miR.26a + miR.29a + miR.376a* + miR.518c + miR.660 + miR.338 | 0,8 | 0,246318434 |
| miR.558 + miR.609 + miR.520f + miR.519b + miR.130a + miR.29a + miR.376a* + miR.494 + miR.518c + miR.639 | 0,8 | 0,056916651 |
| miR.558 + miR.609 + miR.520f + miR.26a + miR.29a + miR.376a* + miR.494 + miR.518c + miR.639 + miR.660 | 0,8 | 0,040606025 |
| miR.558 + miR.609 + miR.220a + miR.26a + miR.29a + miR.362 + miR.376a* + miR.518c + miR.660 + miR.338 | 0,8 | 0,255488044 |
| miR.558 + miR.609 + miR.220a + miR.519b + miR.26a + miR.29a + miR.362 + miR.376a* + miR.518c + miR.660 | 0,8 | 0,233109969 |
| miR.558 + miR.609 + miR.220a + miR.130a + miR.26a + miR.29a + miR.376a* + miR.518c + miR.639 + miR.660 | 0,8 | 0,110347189 |
| miR.558 + miR.609 + miR.520f + miR.130a + miR.26a + miR.29a + miR.376a* + miR.494 + miR.518c + miR.660 | 0,8 | 0,027243459 |
| miR.558 + miR.609 + miR.220a + miR.130a + miR.26a + miR.29a + miR.376a* + miR.518c + miR.660 + miR.338 | 0,8 | 0,241113713 |
| miR.558 + miR.609 + miR.603 + miR.520f + miR.130a + miR.29a + miR.376a* + miR.494 + miR.518c + miR.660 | 0,8 | 0,014144873 |
| miR.558 + miR.609 + miR.220a + miR.519b + miR.29a + miR.362 + miR.369.3p + miR.376a* + miR.518c + miR.338 | 0,8 | 0,296740788 |
| miR.558 + miR.609 + miR.130a + miR.29a + miR.362 + miR.369.3p + miR.376a* + miR.494 + miR.518c + miR.660 | 0,8 | 0,194761578 |
| miR.558 + miR.609 + miR.520f + miR.220a + miR.130a + miR.26a + miR.29a + miR.376a* + miR.518c + miR.639 | 0,8 | 0,01661889 |
| miR.558 + miR.609 + miR.220a + miR.519b + miR.29a + miR.369.3p + miR.376a* + miR.518c + miR.639 + miR.338 | 0,8 | 0,30150057 |
| miR.558 + miR.609 + miR.520f + miR.519b + miR.130a + miR.29a + miR.362 + miR.376a* + miR.494 + miR.518c | 0,8 | 0,075560534 |
| miR.558 + miR.609 + miR.603 + miR.520f + miR.26a + miR.29a + miR.376a* + miR.494 + miR.518c + miR.660 | 0,8 | 0,015720296 |
| miR.558 + miR.609 + miR.520f + miR.130a + miR.29a + miR.362 + miR.369.3p + miR.376a* + miR.518c + miR.639 | 0,8 | 0,067265192 |
| miR.558 + miR.609 + miR.520f + miR.519b + miR.29a + miR.362 + miR.376a* + miR.494 + miR.518c + miR.338 | 0,8 | 0,262933746 |
| miR.558 + miR.609 + miR.520f + miR.519b + miR.29a + miR.362 + miR.369.3p + miR.376a* + miR.518c + miR.639 | 0,8 | 0,072646365 |
| miR.558 + miR.609 + miR.26a + miR.29a + miR.362 + miR.369.3p + miR.376a* + miR.494 + miR.518c + miR.660 | 0,8 | 0,233858196 |
| miR.609 + miR.603 + miR.220a + miR.26a + miR.29a + miR.369.3p + miR.376a* + miR.518c + miR.639 + miR.660 | 0,8 | 0,351462549 |
| miR.609 + miR.603 + miR.220a + miR.519b + miR.29a + miR.362 + miR.369.3p + miR.376a* + miR.518c + miR.660 | 0,8 | 0,408604619 |
| miR.558 + miR.609 + miR.603 + miR.520f + miR.519b + miR.26a + miR.29a + miR.376a* + miR.518c + miR.639 | 0,8 | 0,007457304 |
| miR.558 + miR.609 + miR.220a + miR.130a + miR.26a + miR.29a + miR.362 + miR.376a* + miR.518c + miR.660 | 0,8 | 0,176968562 |
| miR.558 + miR.609 + miR.520f + miR.220a + miR.519b + miR.26a + miR.29a + miR.376a* + miR.518c + miR.639 | 0,8 | 0,041621787 |
| miR.558 + miR.609 + miR.520f + miR.130a + miR.26a + miR.29a + miR.376a* + miR.494 + miR.518c + miR.639 | 0,8 | 0,036583587 |

**Table 11: clusters of 11 miRNAs**

| **Cluster of 11 miRs** | **c_tau level** | **pv compared to top signatu re miR.55 8+ miR.60 9+ miR.52 0f + miR.29 a+ miR.36 2+ miR.36 9.3p + miR.37 6a* + miR.51 8c + miR.63 9+ miR.66 0+ miR.33 8** |
|---|---|---|
| miR.558 + miR.609 + miR.520f + miR.29a + miR.362 + miR.369.3p + miR.376a* + miR.518c + miR.639 + miR.660 + miR.338 | 0,81 | NaN |
| miR.558 + miR.609 + miR.603 + miR.220a + miR.26a + miR.29a + miR.369.3p + miR.376a* + miR.518c + miR.660 + miR.338 | 0,81 | 0,157246016 |
| miR.558 + miR.609 + miR.603 + miR.220a + miR.26a + miR.29a + miR.369.3p + miR.376a* + miR.518c + miR.639 + miR.660 | 0,81 | 0,171856579 |
| miR.558 + miR.609 + miR.520f + miR.220a + miR.29a + miR.362 + miR.369.3p + miR.376a* + miR.518c + miR.660 + miR.338 | 0,81 | 0,496362599 |
| miR.558 + miR.609 + miR.603 + miR.220a + miR.26a + miR.29a + miR.369.3p + miR.376a* + miR.494 + miR.518c + miR.660 | 0,81 | 0,146004635 |
| miR.558 + miR.609 + miR.603 + miR.520f + miR.220a + miR.29a + miR.369.3p + miR.376a* + miR.518c + miR.660 + miR.338 | 0,81 | 0,091948512 |
| miR.558 + miR.609 + miR.603 + miR.520f + miR.220a + miR.26a + miR.29a + miR.369.3p + miR.376a* + miR.518c + miR.660 | 0,81 | 0,181963139 |
| miR.558 + miR.609 + miR.603 + miR.220a + miR.130a + miR.26a + miR.29a + miR.369.3p + miR.376a* + miR.518c + miR.660 | 0,81 | 0,165405632 |
| miR.558 + miR.609 + miR.603 + miR.520f + miR.220a + miR.29a + miR.369.3p + miR.376a* + miR.518c + miR.639 + miR.660 | 0,81 | 0,092406785 |
| miR.558 + miR.609 + miR.603 + miR.220a + miR.26a + miR.29a + miR.362 + miR.369.3p + miR.376a* + miR.518c + miR.660 | 0,81 | 0,329353441 |
| miR.558 + miR.609 + miR.603 + miR.220a + miR.519b + miR.26a + miR.29a + miR.369.3p + miR.376a* + miR.518c + miR.660 | 0,81 | 0,213883456 |
| miR.558 + miR.609 + miR.603 + miR.520f + miR.220a + miR.29a + miR.362 + miR.369.3p + miR.376a* + miR.518c + miR.660 | 0,81 | 0,150894928 |
| miR.558 + miR.609 + miR.603 + miR.220a + miR.519b + miR.29a + miR.369.3p + miR.376a* + miR.494 + miR.518c + miR.660 | 0,81 | 0,16646344 |
| miR.558 + miR.609 + miR.603 + miR.220a + miR.26a + miR.29a + miR.369.3p + miR.376a* + miR.518c + miR.639 + miR.338 | 0,81 | 0,055438935 |
| miR.558 + miR.609 + miR.603 + miR.220a + miR.26a + miR.29a + miR.369.3p + miR.376a* + miR.494 + miR.518c + miR.338 | 0,81 | 0,067433109 |
| miR.558 + miR.609 + miR.603 + miR.220a + miR.29a + miR.362 + miR.369.3p + miR.376a* + miR.494 + miR.518c + miR.660 | 0,81 | 0,208951906 |
| miR.558 + miR.609 + miR.603 + miR.520f + miR.220a + miR.29a + miR.369.3p + miR.376a* + miR.494 + miR.518c + miR.660 | 0,81 | 0,094187974 |
| miR.558 + miR.609 + miR.603 + miR.220a + miR.519b + miR.26a + miR.29a + miR.369.3p + miR.376a* + miR.494 + miR.518c | 0,81 | 0,106889517 |
| miR.558 + miR.609 + miR.603 + miR.520f + miR.220a + miR.519b + miR.29a + miR.369.3p + miR.376a* + miR.518c + miR.660 | 0,81 | 0,167163633 |
| miR.558 + miR.609 + miR.520f + miR.26a + miR.29a + miR.362 + miR.369.3p + miR.376a* + miR.518c + miR.660 + miR.338 | 0,81 | 0,274816579 |
| miR.558 + miR.609 + miR.603 + miR.220a + miR.26a + miR.29a + miR.362 + miR.369.3p + miR.376a* + miR.518c + miR.338 | 0,81 | 0,085174637 |
| miR.558 + miR.609 + miR.603 + miR.520f + miR.29a + miR.362 + miR.369.3p + miR.376a* + miR.518c + miR.660 + miR.338 | 0,81 | 0,199200667 |
| miR.558 + miR.609 + miR.520f + miR.220a + miR.29a + miR.369.3p + miR.376a* + miR.518c + miR.639 + miR.660 + miR.338 | 0,81 | 0,121073665 |
| miR.558 + miR.609 + miR.603 + miR.520f + miR.29a + miR.369.3p + miR.376a* + miR.518c + miR.639 + miR.660 + miR.338 | 0,81 | 0,010508988 |
| miR.558 + miR.609 + miR.603 + miR.220a + miR.130a + miR.29a + miR.362 + miR.369.3p + miR.376a* + miR.518c + miR.660 | 0,81 | 0,254433993 |
| miR.558 + miR.609 + miR.603 + miR.220a + miR.29a + miR.362 + miR.369.3p + miR.376a* + miR.518c + miR.639 + miR.660 | 0,81 | 0,191974648 |
| miR.558 + miR.609 + miR.603 + miR.520f + miR.220a + miR.26a + miR.29a + miR.369.3p + miR.376a* + miR.518c + miR.338 | 0,81 | 0,059765494 |
| miR.558 + miR.609 + miR.603 + miR.220a + miR.29a + miR.362 + miR.369.3p + miR.376a* + miR.518c + miR.660 + miR.338 | 0,81 | 0,411235756 |
| miR.558 + miR.609 + miR.603 + miR.220a + miR.130a + miR.26a + miR.29a + miR.369.3p + miR.376a* + miR.518c + miR.338 | 0,81 | 0,07159897 |
| miR.558 + miR.609 + miR.603 + miR.520f + miR.220a + miR.130a + miR.29a + miR.369.3p + miR.376a* + miR.518c + miR.660 | 0,81 | 0,098482424 |
| miR.558 + miR.609 + miR.603 + miR.220a + miR.519b + miR.130a + miR.29a + miR.369.3p + miR.376a* + miR.494 + miR.518c | 0,81 | 0,128718078 |
| miR.558 + miR.609 + miR.603 + miR.220a + miR.26a + miR.29a + miR.362 + miR.369.3p + miR.376a* + miR.518c + miR.639 | 0,81 | 0,080417628 |
| miR.558 + miR.609 + miR.603 + miR.220a + miR.26a + miR.29a + miR.362 + miR.369.3p + miR.376a* + miR.494 + miR.518c | 0,81 | 0,05140153 |
| miR.558 + miR.609 + miR.603 + miR.220a + miR.519b + miR.29a + miR.362 + miR.369.3p + miR.376a* + miR.518c + miR.660 | 0,81 | 0,227723131 |
| miR.558 + miR.609 + miR.603 + miR.220a + miR.519b + miR.130a + miR.29a + miR.369.3p + miR.376a* + miR.518c + miR.660 | 0,81 | 0,178842795 |
| miR.558 + miR.609 + miR.603 + miR.220a + miR.519b + miR.26a + miR.29a + miR.362 + miR.369.3p + miR.376a* + miR.518c | 0,81 | 0,13587915 |
| miR.558 + miR.609 + miR.603 + miR.220a + miR.519b + miR.130a + miR.29a + miR.362 + miR.376a* + miR.494 + miR.518c | 0,81 | 0,090644921 |
| miR.558 + miR.609 + miR.603 + miR.520f + miR.220a + miR.26a + miR.29a + miR.362 + miR.369.3p + miR.376a* + miR.518c | 0,81 | 0,066839667 |
| miR.558 + miR.609 + miR.520f + miR.220a + miR.29a + miR.369.3p + miR.376a* + miR.494 + miR.518c + miR.660 + miR.338 | 0,81 | 0,105045113 |
| miR.558 + miR.609 + miR.603 + miR.220a + miR.130a + miR.26a + miR.29a + miR.362 + miR.369.3p + miR.376a* + miR.518c | 0,81 | 0,066839667 |
| miR.558 + miR.609 + miR.603 + miR.220a + miR.26a + miR.29a + miR.369.3p + miR.376a* + miR.494 + miR.518c + miR.639 | 0,81 | 0,049902204 |
| miR.558 + miR.609 + miR.603 + miR.520f + miR.220a + miR.519b + miR.29a + miR.369.3p + miR.376a* + miR.494 + miR.518c | 0,81 | 0,089387076 |
| miR.558 + miR.609 + miR.603 + miR.220a + miR.130a + miR.26a + miR.29a + miR.369.3p + miR.376a* + miR.518c + miR.639 | 0,81 | 0,066355389 |
| miR.558 + miR.609 + miR.603 + miR.220a + miR.130a + miR.26a + miR.29a + miR.369.3p + miR.376a* + miR.494 + miR.518c | 0,81 | 0,045991693 |
| miR.558 + miR.609 + miR.520f + miR.29a + miR.362 + miR.369.3p + miR.376a* + miR.494 + miR.518c + miR.660 + miR.338 | 0,81 | 0,272054339 |
| miR.558 + miR.609 + miR.603 + miR.220a + miR.29a + miR.369.3p + miR.376a* + miR.494 + miR.518c + miR.639 + miR.660 | 0,81 | 0,131669372 |
| miR.558 + miR.609 + miR.603 + miR.520f + miR.220a + miR.26a + miR.29a + miR.369.3p + miR.376a* + miR.518c + miR.639 | 0,81 | 0,0583122 |
| miR.558 + miR.609 + miR.603 + miR.220a + miR.29a + miR.369.3p + miR.376a* + miR.518c + miR.639 + miR.660 + miR.338 | 0,81 | 0,18247775 |
| miR.558 + miR.609 + miR.603 + miR.220a + miR.519b + miR.26a + miR.29a + miR.369.3p + miR.376a* + miR.518c + miR.338 | 0,81 | 0,109488784 |
| miR.558 + miR.609 + miR.603 + miR.220a + miR.519b + miR.29a + miR.362 + miR.369.3p + miR.376a* + miR.494 + miR.518c | 0,81 | 0,079074586 |
| miR.558 + miR.609 + miR.603 + miR.520f + miR.220a + miR.29a + miR.362 + miR.369.3p + miR.376a* + miR.518c + miR.338 | 0,81 | 0,028631288 |
| miR.558 + miR.609 + miR.603 + miR.220a + miR.29a + miR.369.3p + miR.376a* + miR.494 + miR.518c + miR.660 + miR.338 | 0,81 | 0,235907465 |
| miR.558 + miR.609 + miR.603 + miR.220a + miR.130a + miR.29a + miR.369.3p + miR.376a* + miR.494 + miR.518c + miR.660 | 0,81 | 0,131461341 |
| miR.558 + miR.609 + miR.603 + miR.220a + miR.130a + miR.29a + miR.369.3p + miR.376a* + miR.518c + miR.639 + miR.660 | 0,81 | 0,147748075 |
| miR.558 + miR.609 + miR.603 + miR.520f + miR.220a + miR.26a + miR.29a + miR.369.3p + miR.376a* + miR.494 + miR.518c | 0,81 | 0,045216308 |
| miR.558 + miR.609 + miR.603 + miR.220a + miR.519b + miR.29a + miR.369.3p + miR.376a* + miR.518c + miR.639 + miR.660 | 0,81 | 0,220170147 |
| miR.558 + miR.609 + miR.603 + miR.220a + miR.519b + miR.29a + miR.369.3p + miR.376a* + miR.518c + miR.660 + miR.338 | 0,81 | 0,169875898 |
| miR.558 + miR.609 + miR.520f + miR.130a + miR.29a + miR.369.3p + miR.376a* + miR.518c + miR.639 + miR.660 + miR.338 | 0,81 | 0,128601523 |
| miR.558 + miR.609 + miR.220a + miR.26a + miR.29a + miR.362 + miR.369.3p + miR.376a* + miR.518c + miR.660 + miR.338 | 0,81 | 0,311444877 |
| miR.558 + miR.609 + miR.603 + miR.520f + miR.220a + miR.130a + miR.26a + miR.29a + miR.369.3p + miR.376a* + miR.518c | 0,81 | 0,066414344 |
| miR.558 + miR.609 + miR.520f + miR.220a + miR.26a + miR.29a + miR.369.3p + miR.376a* + miR.518c + miR.660 + miR.338 | 0,81 | 0,155513204 |
| miR.558 + miR.609 + miR.520f + miR.220a + miR.519b + miR.29a + miR.369.3p + miR.376a* + miR.518c + miR.660 + miR.338 | 0,81 | 0,214374038 |
| miR.558 + miR.609 + miR.520f + miR.220a + miR.130a + miR.29a + miR.369.3p + miR.376a* + miR.518c + miR.660 + miR.338 | 0,81 | 0,106900261 |
| miR.558 + miR.609 + miR.603 + miR.220a + miR.519b + miR.130a + miR.26a + miR.29a + miR.369.3p + miR.376a* + miR.518c | 0,81 | 0,119541359 |
| miR.558 + miR.609 + miR.603 + miR.520f + miR.220a + miR.519b + miR.29a + miR.369.3p + miR.376a* + miR.518c + miR.338 | 0,81 | 0,044448471 |
| miR.558 + miR.609 + miR.603 + miR.220a + miR.519b + miR.29a + miR.369.3p + miR.376a* + miR.494 + miR.518c + miR.639 | 0,81 | 0,080955667 |
| miR.558 + miR.609 + miR.603 + miR.520f + miR.220a + miR.519b + miR.29a + miR.362 + miR.376a* + miR.494 + miR.518c | 0,81 | 0,088147267 |
| miR.558 + miR.609 + miR.603 + miR.220a + miR.130a + miR.29a + miR.369.3p + miR.376a* + miR.518c + miR.660 + miR.338 | 0,81 | 0,210223613 |
| miR.558 + miR.609 + miR.603 + miR.220a + miR.519b + miR.130a + miR.29a + miR.362 + miR.369.3p + miR.376a* + miR.518c | 0,81 | 0,075058128 |
| miR.558 + miR.609 + miR.603 + miR.520f + miR.220a + miR.519b + miR.29a + miR.362 + miR.369.3p + miR.376a* + miR.518c | 0,81 | 0,116764211 |
| miR.558 + miR.609 + miR.603 + miR.520f + miR.220a + miR.29a + miR.362 + miR.369.3p + miR.376a* + miR.518c + miR.639 | 0,81 | 0,031559751 |
| miR.558 + miR.609 + miR.520f + miR.130a + miR.29a + miR.362 + miR.369.3p + miR.376a* + miR.518c + miR.660 + miR.338 | 0,81 | 0,300762925 |
| miR.558 + miR.609 + miR.520f + miR.220a + miR.29a + miR.369.3p + miR.376a* + miR.494 + miR.518c + miR.639 + miR.660 | 0,81 | 0,054662395 |
| miR.558 + miR.609 + miR.603 + miR.520f + miR.26a + miR.29a + miR.369.3p + miR.376a* + miR.518c + miR.639 + miR.338 | 0,81 | 0,029028283 |
| miR.558 + miR.609 + miR.520f + miR.220a + miR.26a + miR.29a + miR.369.3p + miR.376a* + miR.518c + miR.639 + miR.660 | 0,81 | 0,107383644 |
| miR.558 + miR.609 + miR.520f + miR.29a + miR.369.3p + miR.376a* + miR.494 + miR.518c + miR.639 + miR.660 + miR.338 | 0,81 | 0,081054444 |
| miR.558 + miR.609 + miR.603 + miR.520f + miR.220a + miR.29a + miR.362 + miR.369.3p + miR.376a* + miR.494 + miR.518c | 0,81 | 0,029649922 |
| miR.558 + miR.609 + miR.603 + miR.220a + miR.519b + miR.130a + miR.29a + miR.376a* + miR.494 + miR.518c + miR.660 | 0,81 | 0,080293447 |
| miR.558 + miR.609 + miR.220a + miR.26a + miR.29a + miR.369.3p + miR.376a* + miR.494 + miR.518c + miR.660 + miR.338 | 0,81 | 0,203556658 |
| miR.558 + miR.609 + miR.520f + miR.220a + miR.29a + miR.362 + miR.369.3p + miR.376a* + miR.518c + miR.639 + miR.660 | 0,81 | 0,119155844 |
| miR.558 + miR.609 + miR.603 + miR.520f + miR.220a + miR.130a + miR.29a + miR.362 + miR.369.3p + miR.376a* + miR.518c | 0,81 | 0,03916983 |
| miR.558 + miR.609 + miR.603 + miR.220a + miR.519b + miR.26a + miR.29a + miR.369.3p + miR.376a* + miR.518c + miR.639 | 0,81 | 0,118944722 |
| miR.558 + miR.609 + miR.603 + miR.520f + miR.220a + miR.519b + miR.26a + miR.29a + miR.369.3p + miR.376a* + miR.518c | 0,81 | 0,131184379 |
| miR.558 + miR.609 + miR.520f + miR.220a + miR.26a + miR.29a + miR.369.3p + miR.376a* + miR.494 + miR.518c + miR.660 | 0,81 | 0,152524201 |
| miR.558 + miR.609 + miR.603 + miR.220a + miR.519b + miR.130a + miR.29a + miR.369.3p + miR.376a* + miR.518c + miR.338 | 0,81 | 0,05479335 |
| miR.558 + miR.609 + miR.603 + miR.520f + miR.29a + miR.362 + miR.369.3p + miR.376a* + miR.518c + miR.639 + miR.338 | 0,81 | 0,023807176 |
| miR.558 + miR.609 + miR.520f + miR.519b + miR.29a + miR.362 + miR.369.3p + miR.376a* + miR.518c + miR.660 + miR.338 | 0,81 | 0,287009844 |
| miR.558 + miR.609 + miR.603 + miR.520f + miR.29a + miR.369.3p + miR.376a* + miR.494 + miR.518c + miR.660 + miR.338 | 0,81 | 0,022241593 |
| miR.558 + miR.609 + miR.603 + miR.220a + miR.519b + miR.29a + miR.362 + miR.369.3p + miR.376a* + miR.518c + miR.338 | 0,81 | 0,06891838 |
| miR.558 + miR.609 + miR.220a + miR.26a + miR.29a + miR.362 + miR.369.3p + miR.376a* + miR.494 + miR.518c + miR.660 | 0,81 | 0,202559842 |
| miR.558 + miR.609 + miR.603 + miR.26a + miR.29a + miR.362 + miR.369.3p + miR.376a* + miR.518c + miR.660 + miR.338 | 0,81 | 0,129538185 |
| miR.558 + miR.609 + miR.220a + miR.26a + miR.29a + miR.369.3p + miR.376a* + miR.518c + miR.639 + miR.660 + miR.338 | 0,81 | 0,206612529 |
| miR.558 + miR.609 + miR.520f + miR.220a + miR.29a + miR.362 + miR.369.3p + miR.376a* + miR.494 + miR.518c + miR.660 | 0,81 | 0,176200458 |
| miR.558 + miR.609 + miR.603 + miR.220a + miR.519b + miR.29a + miR.362 + miR.369.3p + miR.376a* + miR.518c + miR.639 | 0,81 | 0,078489254 |
| miR.558 + miR.609 + miR.520f + miR.220a + miR.26a + miR.29a + miR.362 + miR.369.3p + miR.376a* + miR.518c + miR.660 | 0,81 | 0,301062138 |
| miR.558 + miR.609 + miR.603 + miR.220a + miR.519b + miR.130a + miR.29a + miR.369.3p + miR.376a* + miR.518c + miR.639 | 0,81 | 0,067902456 |
| miR.558 + miR.609 + miR.603 + miR.220a + miR.519b + miR.130a + miR.26a + miR.29a + miR.376a* + miR.494 + miR.518c | 0,81 | 0,062014197 |
| miR.558 + miR.609 + miR.220a + miR.26a + miR.29a + miR.369.3p + miR.376a* + miR.494 + miR.518c + miR.639 + miR.660 | 0,81 | 0,21512478 |
| miR.558 + miR.609 + miR.520f + miR.220a + miR.130a + miR.29a + miR.369.3p + miR.376a* + miR.518c + miR.639 + miR.660 | 0,81 | 0,09004539 |
| miR.558 + miR.609 + miR.603 + miR.520f + miR.220a + miR.29a + miR.369.3p + miR.376a* + miR.518c + miR.639 + miR.338 | 0,81 | 0,015083939 |
| miR.558 + miR.609 + miR.520f + miR.220a + miR.130a + miR.29a + miR.369.3p + miR.376a* + miR.494 + miR.518c + miR.660 | 0,81 | 0,110624948 |
| miR.558 + miR.609 + miR.603 + miR.520f + miR.220a + miR.519b + miR.29a + miR.376a* + miR.494 + miR.518c + miR.660 | 0,81 | 0,095230232 |
| miR.558 + miR.609 + miR.603 + miR.220a + miR.519b + miR.26a + miR.29a + miR.362 + miR.376a* + miR.494 + miR.518c | 0,81 | 0,101333194 |
| miR.558 + miR.609 + miR.603 + miR.520f + miR.519b + miR.29a + miR.369.3p + miR.376a* + miR.494 + miR.518c + miR.338 | 0,81 | 0,031978532 |
| miR.558 + miR.609 + miR.603 + miR.220a + miR.130a + miR.29a + miR.362 + miR.369.3p + miR.376a* + miR.518c + miR.639 | 0,81 | 0,022672081 |
| miR.558 + miR.609 + miR.220a + miR.519b + miR.26a + miR.29a + miR.369.3p + miR.376a* + miR.494 + miR.518c + miR.660 | 0,81 | 0,294181385 |
| miR.558 + miR.609 + miR.603 + miR.520f + miR.519b + miR.29a + miR.369.3p + miR.376a* + miR.518c + miR.660 + miR.338 | 0,81 | 0,042711014 |
| miR.558 + miR.609 + miR.603 + miR.220a + miR.26a + miR.29a + miR.362 + miR.376a* + miR.518c + miR.639 + miR.338 | 0,81 | 0,061977272 |
| miR.558 + miR.609 + miR.603 + miR.520f + miR.130a + miR.29a + miR.369.3p + miR.376a* + miR.518c + miR.660 + miR.338 | 0,81 | 0,02204482 |
| miR.558 + miR.609 + miR.603 + miR.520f + miR.220a + miR.130a + miR.29a + miR.369.3p + miR.376a* + miR.518c + miR.639 | 0,81 | 0,023116393 |
| miR.558 + miR.609 + miR.603 + miR.520f + miR.130a + miR.29a + miR.369.3p + miR.376a* + miR.518c + miR.639 + miR.338 | 0,81 | 0,027568859 |
| miR.558 + miR.609 + miR.603 + miR.520f + miR.220a + miR.130a + miR.29a + miR.369.3p + miR.376a* + miR.494 + miR.518c | 0,81 | 0,024877954 |
| miR.558 + miR.609 + miR.520f + miR.26a + miR.29a + miR.369.3p + miR.376a* + miR.518c + miR.639 + miR.660 + miR.338 | 0,81 | 0,120221476 |
| miR.558 + miR.609 + miR.603 + miR.520f + miR.220a + miR.519b + miR.29a + miR.369.3p + miR.376a* + miR.518c + miR.639 | 0,81 | 0,074303321 |
| miR.558 + miR.609 + miR.603 + miR.520f + miR.29a + miR.369.3p + miR.376a* + miR.494 + miR.518c + miR.639 + miR.660 | 0,81 | 0,004682371 |
| miR.558 + miR.609 + miR.520f + miR.220a + miR.130a + miR.29a + miR.362 + miR.369.3p + miR.376a* + miR.518c + miR.660 | 0,81 | 0,235944522 |
| miR.558 + miR.609 + miR.520f + miR.130a + miR.29a + miR.369.3p + miR.376a* + miR.494 + miR.518c + miR.639 + miR.660 | 0,81 | 0,04833469 |
| miR.558 + miR.609 + miR.220a + miR.130a + miR.29a + miR.362 + miR.369.3p + miR.376a* + miR.494 + miR.518c + miR.660 | 0,81 | 0,172749789 |
| miR.558 + miR.609 + miR.520f + miR.519b + miR.29a + miR.369.3p + miR.376a* + miR.518c + miR.639 + miR.660 + miR.338 | 0,81 | 0,082595715 |
| miR.558 + miR.609 + miR.130a + miR.29a + miR.362 + miR.369.3p + miR.376a* + miR.494 + miR.518c + miR.639 + miR.660 | 0,81 | 0,116592883 |
| miR.558 + miR.609 + miR.603 + miR.26a + miR.29a + miR.369.3p + miR.376a* + miR.518c + miR.639 + miR.660 + miR.338 | 0,81 | 0,128889488 |
| miR.558 + miR.609 + miR.603 + miR.520f + miR.519b + miR.29a + miR.369.3p + miR.376a* + miR.518c + miR.639 + miR.338 | 0,81 | 0,039806387 |
| miR.558 + miR.609 + miR.520f + miR.220a + miR.519b + miR.29a + miR.369.3p + miR.376a* + miR.494 + miR.518c + miR.660 | 0,81 | 0,179763739 |
| miR.558 + miR.609 + miR.520f + miR.220a + miR.130a + miR.26a + miR.29a + miR.369.3p + miR.376a* + miR.518c + miR.660 | 0,81 | 0,191779006 |
| miR.558 + miR.609 + miR.603 + miR.220a + miR.519b + miR.26a + miR.29a + miR.376a* + miR.494 + miR.518c + miR.660 | 0,81 | 0,084003019 |
| miR.558 + miR.609 + miR.603 + miR.520f + miR.220a + miR.519b + miR.29a + miR.376a* + miR.494 + miR.518c + miR.338 | 0,81 | 0,027080309 |
| miR.558 + miR.609 + miR.603 + miR.130a + miR.26a + miR.29a + miR.369.3p + miR.376a* + miR.518c + miR.639 + miR.338 | 0,81 | 0,103986664 |
| miR.558 + miR.609 + miR.603 + miR.520f + miR.220a + miR.519b + miR.26a + miR.29a + miR.362 + miR.376a* + miR.518c | 0,81 | 0,107758422 |
| miR.558 + miR.609 + miR.220a + miR.519b + miR.26a + miR.29a + miR.369.3p + miR.376a* + miR.518c + miR.660 + miR.338 | 0,81 | 0,236412998 |
| miR.558 + miR.609 + miR.603 + miR.220a + miR.26a + miR.29a + miR.362 + miR.376a* + miR.494 + miR.518c + miR.639 | 0,81 | 0,046229114 |
| miR.558 + miR.609 + miR.603 + miR.220a + miR.130a + miR.29a + miR.369.3p + miR.376a* + miR.494 + miR.518c + miR.639 | 0,81 | 0,018181423 |
| miR.558 + miR.609 + miR.220a + miR.130a + miR.26a + miR.29a + miR.369.3p + miR.376a* + miR.518c + miR.660 + miR.338 | 0,81 | 0,180331179 |
| miR.558 + miR.609 + miR.603 + miR.520f + miR.26a + miR.29a + miR.369.3p + miR.376a* + miR.518c + miR.660 + miR.338 | 0,81 | 0,029370985 |
| miR.558 + miR.609 + miR.220a + miR.26a + miR.29a + miR.362 + miR.369.3p + miR.376a* + miR.518c + miR.639 + miR.660 | 0,81 | 0,287302613 |
| miR.558 + miR.609 + miR.603 + miR.520f + miR.26a + miR.29a + miR.369.3p + miR.376a* + miR.494 + miR.518c + miR.338 | 0,81 | 0,034711155 |
| miR.558 + miR.609 + miR.520f + miR.220a + miR.519b + miR.29a + miR.362 + miR.369.3p + miR.376a* + miR.518c + miR.660 | 0,81 | 0,277262363 |
| miR.558 + miR.609 + miR.220a + miR.130a + miR.26a + miR.29a + miR.369.3p + miR.376a* + miR.494 + miR.518c + miR.660 | 0,81 | 0,167285229 |
| miR.558 + miR.609 + miR.603 + miR.520f + miR.220a + miR.519b + miR.29a + miR.362 + miR.376a* + miR.518c + miR.338 | 0,81 | 0,061056014 |
| miR.558 + miR.609 + miR.520f + miR.220a + miR.519b + miR.29a + miR.369.3p + miR.376a* + miR.518c + miR.639 + miR.660 | 0,81 | 0,119596033 |
| miR.558 + miR.609 + miR.220a + miR.29a + miR.362 + miR.369.3p + miR.376a* + miR.518c + miR.639 + miR.660 + miR.338 | 0,81 | 0,260947722 |
| miR.558 + miR.609 + miR.603 + miR.520f + miR.220a + miR.29a + miR.369.3p + miR.376a* + miR.494 + miR.518c + miR.338 | 0,81 | 0,016688397 |
| miR.558 + miR.609 + miR.603 + miR.220a + miR.519b + miR.130a + miR.29a + miR.376a* + miR.494 + miR.518c + miR.338 | 0,81 | 0,046262483 |
| miR.558 + miR.609 + miR.603 + miR.520f + miR.220a + miR.519b + miR.130a + miR.29a + miR.369.3p + miR.376a* + miR.518c | 0,81 | 0,084016227 |
| miR.558 + miR.609 + miR.603 + miR.520f + miR.220a + miR.519b + miR.130a + miR.29a + miR.362 + miR.376a* + miR.518c | 0,81 | 0,115517656 |
| miR.558 + miR.609 + miR.603 + miR.220a + miR.130a + miR.29a + miR.362 + miR.369.3p + miR.376a* + miR.494 + miR.518c | 0,81 | 0,021317512 |
| miR.558 + miR.609 + miR.603 + miR.29a + miR.362 + miR.369.3p + miR.376a* + miR.518c + miR.639 + miR.660 + miR.338 | 0,81 | 0,061171041 |
| miR.558 + miR.609 + miR.603 + miR.220a + miR.519b + miR.26a + miR.29a + miR.362 + miR.376a* + miR.518c + miR.338 | 0,81 | 0,069080501 |
| miR.558 + miR.609 + miR.603 + miR.220a + miR.519b + miR.130a + miR.29a + miR.376a* + miR.494 + miR.518c + miR.639 | 0,81 | 0,052200567 |
| miR.558 + miR.609 + miR.603 + miR.520f + miR.220a + miR.519b + miR.26a + miR.29a + miR.376a* + miR.494 + miR.518c | 0,81 | 0,048373812 |
| miR.558 + miR.609 + miR.603 + miR.520f + miR.220a + miR.130a + miR.29a + miR.369.3p + miR.376a* + miR.518c + miR.338 | 0,81 | 0,020099179 |
| miR.558 + miR.609 + miR.603 + miR.520f + miR.220a + miR.29a + miR.376a* + miR.518c + miR.639 + miR.660 + miR.338 | 0,81 | 0,031295958 |
| miR.558 + miR.609 + miR.603 + miR.220a + miR.130a + miR.26a + miR.29a + miR.362 + miR.376a* + miR.494 + miR.518c | 0,81 | 0,052787732 |
| miR.558 + miR.609 + miR.220a + miR.519b + miR.26a + miR.29a + miR.369.3p + miR.376a* + miR.518c + miR.639 + miR.660 | 0,81 | 0,263389576 |
| miR.558 + miR.609 + miR.603 + miR.220a + miR.26a + miR.29a + miR.362 + miR.376a* + miR.494 + miR.518c + miR.338 | 0,81 | 0,04631865 |
| miR.558 + miR.609 + miR.520f + miR.220a + miR.519b + miR.26a + miR.29a + miR.369.3p + miR.376a* + miR.518c + miR.660 | 0,81 | 0,24123 2516 |
| miR.558 + miR.609 + miR.520f + miR.220a + miR.26a + miR.29a + miR.362 + miR.369.3p + miR.376a* + miR.518c + miR.338 | 0,81 | 0,048374898 |
| miR.558 + miR.609 + miR.220a + miR.130a + miR.29a + miR.362 + miR.369.3p + miR.376a* + miR.518c + miR.660 + miR.338 | 0,81 | 0,273236304 |
| miR.558 + miR.609 + miR.603 + miR.520f + miR.220a + miR.29a + miR.362 + miR.376a* + miR.518c + miR.639 + miR.338 | 0,81 | 0,020061474 |
| miR.558 + miR.609 + miR.603 + miR.520f + miR.220a + miR.519b + miR.130a + miR.29a + miR.376a* + miR.494 + miR.518c | 0,81 | 0,05988177 |
| miR.558 + miR.609 + miR.520f + miR.220a + miR.29a + miR.376a* + miR.494 + miR.518c + miR.639 + miR.660 + miR.338 | 0,81 | 0,028607081 |
| miR.558 + miR.609 + miR.603 + miR.520f + miR.220a + miR.29a + miR.376a* + miR.494 + miR.518c + miR.639 + miR.660 | 0,81 | 0,071271615 |
| miR.558 + miR.609 + miR.603 + miR.520f + miR.29a + miR.369.3p + miR.376a* + miR.494 + miR.518c + miR.639 + miR.338 | 0,81 | 0,014376236 |
| miR.558 + miR.609 + miR.220a + miR.29a + miR.362 + miR.369.3p + miR.376a* + miR.494 + miR.518c + miR.660 + miR.338 | 0,81 | 0,235462188 |
| miR.558 + miR.609 + miR.603 + miR.520f + miR.26a + miR.29a + miR.369.3p + miR.376a* + miR.518c + miR.639 + miR.660 | 0,81 | 0,012140255 |
| miR.558 + miR.609 + miR.520f + miR.220a + miR.26a + miR.29a + miR.369.3p + miR.376a* + miR.494 + miR.518c + miR.338 | 0,81 | 0,043902695 |
| miR.558 + miR.609 + miR.603 + miR.220a + miR.130a + miR.29a + miR.369.3p + miR.376a* + miR.494 + miR.518c + miR.338 | 0,81 | 0,025637666 |
| miR.558 + miR.609 + miR.603 + miR.520f + miR.220a + miR.26a + miR.29a + miR.362 + miR.376a* + miR.518c + miR.639 | 0,81 | 0,046912851 |
| miR.558 + miR.609 + miR.603 + miR.520f + miR.220a + miR.519b + miR.29a + miR.362 + miR.376a* + miR.518c + miR.660 | 0,81 | 0,121982862 |
| miR.558 + miR.609 + miR.603 + miR.220a + miR.26a + miR.29a + miR.376a* + miR.494 + miR.518c + miR.639 + miR.660 | 0,81 | 0,050429994 |
| miR.558 + miR.609 + miR.520f + miR.130a + miR.29a + miR.376a* + miR.494 + miR.518c + miR.639 + miR.660 + miR.338 | 0,81 | 0,031247547 |
| miR.558 + miR.609 + miR.603 + miR.520f + miR.220a + miR.29a + miR.376a* + miR.494 + miR.518c + miR.660 + miR.338 | 0,81 | 0,03715542 |
| miR.558 + miR.609 + miR.603 + miR.520f + miR.29a + miR.362 + miR.369.3p + miR.376a* + miR.518c + miR.639 + miR.660 | 0,81 | 0,002566694 |
| miR.558 + miR.609 + miR.220a + miR.519b + miR.26a + miR.29a + miR.362 + miR.369.3p + miR.376a* + miR.518c + miR.660 | 0,81 | 0,285636001 |
| miR.558 + miR.609 + miR.603 + miR.220a + miR.519b + miR.130a + miR.26a + miR.29a + miR.362 + miR.376a* + miR.518c | 0,81 | 0,090123865 |
| miR.558 + miR.609 + miR.603 + miR.520f + miR.130a + miR.26a + miR.29a + miR.369.3p + miR.376a* + miR.518c + miR.338 | 0,81 | 0,029075047 |
| miR.558 + miR.609 + miR.520f + miR.220a + miR.26a + miR.29a + miR.369.3p + miR.376a* + miR.518c + miR.639 + miR.338 | 0,81 | 0,038639888 |
| miR.558 + miR.609 + miR.603 + miR.220a + miR.519b + miR.26a + miR.29a + miR.376a* + miR.494 + miR.518c + miR.338 | 0,81 | 0,056499614 |
| miR.558 + miR.609 + miR.603 + miR.220a + miR.519b + miR.29a + miR.369.3p + miR.376a* + miR.494 + miR.518c + miR.338 | 0,81 | 0,063066277 |
| miR.558 + miR.609 + miR.603 + miR.220a + miR.519b + miR.26a + miR.29a + miR.362 + miR.376a* + miR.518c + miR.639 | 0,81 | 0,095366934 |
| miR.558 + miR.609 + miR.603 + miR.220a + miR.130a + miR.29a + miR.362 + miR.369.3p + miR.376a* + miR.518c + miR.338 | 0,81 | 0,021725703 |
| miR.558 + miR.609 + miR.520f + miR.519b + miR.29a + miR.369.3p + miR.376a* + miR.494 + miR.518c + miR.660 + miR.338 | 0,81 | 0,222321539 |
| miR.558 + miR.609 + miR.520f + miR.220a + miR.29a + miR.362 + miR.369.3p + miR.376a* + miR.518c + miR.639 + miR.338 | 0,81 | 0,016462533 |
| miR.558 + miR.609 + miR.603 + miR.520f + miR.130a + miR.29a + miR.369.3p + miR.376a* + miR.494 + miR.518c + miR.338 | 0,81 | 0,019295261 |
| miR.558 + miR.609 + miR.220a + miR.130a + miR.26a + miR.29a + miR.362 + miR.369.3p + miR.376a* + miR.518c + miR.660 | 0,81 | 0,304747856 |
| miR.558 + miR.609 + miR.603 + miR.520f + miR.220a + miR.519b + miR.29a + miR.376a* + miR.494 + miR.518c + miR.639 | 0,81 | 0,042315388 |
| miR.558 + miR.609 + miR.520f + miR.220a + miR.519b + miR.29a + miR.376a* + miR.494 + miR.518c + miR.660 + miR.338 | 0,81 | 0,165603354 |
| miR.558 + miR.609 + miR.603 + miR.520f + miR.29a + miR.362 + miR.369.3p + miR.376a* + miR.494 + miR.518c + miR.338 | 0,81 | 0,017937353 |
| miR.558 + miR.609 + miR.220a + miR.519b + miR.130a + miR.29a + miR.369.3p + miR.376a* + miR.494 + miR.518c + miR.660 | 0,81 | 0,227912696 |
| miR.558 + miR.609 + miR.520f + miR.29a + miR.362 + miR.376a* + miR.494 + miR.518c + miR.639 + miR.660 + miR.338 | 0,81 | 0,013107368 |
| miR.558 + miR.609 + miR.220a + miR.130a + miR.29a + miR.362 + miR.369.3p + miR.376a* + miR.518c + miR.639 + miR.660 | 0,81 | 0,277978719 |
| miR.558 + miR.609 + miR.603 + miR.520f + miR.220a + miR.519b + miR.29a + miR.362 + miR.376a* + miR.518c + miR.639 | 0,81 | 0,098132347 |
| miR.558 + miR.609 + miR.220a + miR.130a + miR.29a + miR.369.3p + miR.376a* + miR.518c + miR.639 + miR.660 + miR.338 | 0,81 | 0,165580534 |
| miR.558 + miR.609 + miR.603 + miR.26a + miR.29a + miR.362 + miR.369.3p + miR.376a* + miR.518c + miR.639 + miR.338 | 0,81 | 0,09756 0492 |
| miR.558 + miR.609 + miR.220a + miR.130a + miR.26a + miR.29a + miR.369.3p + miR.376a* + miR.518c + miR.639 + miR.660 | 0,81 | 0,269457271 |
| miR.558 + miR.609 + miR.520f + miR.220a + miR.29a + miR.362 + miR.369.3p + miR.376a* + miR.494 + miR.518c + miR.338 | 0,81 | 0,018148135 |
| miR.558 + miR.609 + miR.603 + miR.519b + miR.26a + miR.29a + miR.369.3p + miR.376a* + miR.518c + miR.639 + miR.338 | 0,81 | 0,077165642 |
| miR.558 + miR.609 + miR.603 + miR.26a + miR.29a + miR.369.3p + miR.376a* + miR.494 + miR.518c + miR.639 + miR.338 | 0,81 | 0,071608284 |
| miR.558 + miR.609 + miR.220a + miR.519b + miR.29a + miR.362 + miR.369.3p + miR.376a* + miR.518c + miR.660 + miR.338 | 0,81 | 0,269155184 |
| miR.558 + miR.609 + miR.520f + miR.220a + miR.519b + miR.130a + miR.29a + miR.369.3p + miR.376a* + miR.518c + miR.660 | 0,81 | 0,216868617 |
| miR.558 + miR.609 + miR.603 + miR.220a + miR.26a + miR.29a + miR.376a* + miR.494 + miR.518c + miR.660 + miR.338 | 0,81 | 0,060593535 |
| miR.558 + miR.609 + miR.603 + miR.520f + miR.220a + miR.26a + miR.29a + miR.376a* + miR.518c + miR.639 + miR.660 | 0,81 | 0,074448852 |
| miR.558 + miR.609 + miR.603 + miR.520f + miR.130a + miR.29a + miR.369.3p + miR.376a* + miR.518c + miR.639 + miR.660 | 0,81 | 0,019785352 |
| miR.558 + miR.609 + miR.603 + miR.520f + miR.220a + miR.519b + miR.29a + miR.376a* + miR.518c + miR.639 + miR.660 | 0,81 | 0,112686787 |
| miR.558 + miR.609 + miR.603 + miR.26a + miR.29a + miR.362 + miR.369.3p + miR.376a* + miR.518c + miR.639 + miR.660 | 0,81 | 0,039634756 |
| miR.558 + miR.609 + miR.603 + miR.520f + miR.26a + miR.29a + miR.362 + miR.369.3p + miR.376a* + miR.518c + miR.338 | 0,81 | 0,026101501 |
| miR.558 + miR.609 + miR.603 + miR.220a + miR.519b + miR.26a + miR.29a + miR.362 + miR.376a* + miR.518c + miR.660 | 0,81 | 0,113390363 |
| miR.558 + miR.609 + miR.603 + miR.520f + miR.29a + miR.376a* + miR.494 + miR.518c + miR.639 + miR.660 + miR.338 | 0,81 | 0,012312031 |
| miR.558 + miR.609 + miR.603 + miR.520f + miR.220a + miR.29a + miR.362 + miR.376a* + miR.494 + miR.518c + miR.639 | 0,81 | 0,018411513 |
| miR.558 + miR.609 + miR.520f + miR.220a + miR.29a + miR.369.3p + miR.376a* + miR.494 + miR.518c + miR.639 + miR.338 | 0,81 | 0,019031544 |
| miR.558 + miR.609 + miR.603 + miR.520f + miR.220a + miR.29a + miR.369.3p + miR.376a* + miR.494 + miR.518c + miR.639 | 0,81 | 0,009341948 |
| miR.558 + miR.609 + miR.603 + miR.220a + miR.519b + miR.26a + miR.29a + miR.376a* + miR.518c + miR.660 + miR.338 | 0,81 | 0,09326183 |
| miR.558 + miR.609 + miR.603 + miR.220a + miR.26a + miR.29a + miR.376a* + miR.494 + miR.518c + miR.639 + miR.338 | 0,81 | 0,017499963 |
| miR.558 + miR.609 + miR.603 + miR.520f + miR.130a + miR.29a + miR.362 + miR.369.3p + miR.376a* + miR.518c + miR.338 | 0,81 | 0,012000363 |
| miR.558 + miR.609 + miR.603 + miR.520f + miR.220a + miR.519b + miR.26a + miR.29a + miR.376a* + miR.518c + miR.660 | 0,81 | 0,137890968 |
| miR.558 + miR.609 + miR.603 + miR.520f + miR.519b + miR.130a + miR.29a + miR.369.3p + miR.376a* + miR.518c + miR.338 | 0,81 | 0,03062986 |
| miR.558 + miR.609 + miR.220a + miR.26a + miR.29a + miR.369.3p + miR.376a* + miR.494 + miR.518c + miR.639 + miR.338 | 0,81 | 0,057323871 |
| miR.558 + miR.609 + miR.603 + miR.220a + miR.130a + miR.26a + miR.29a + miR.376a* + miR.494 + miR.518c + miR.639 | 0,81 | 0,011173161 |
| miR.558 + miR.609 + miR.603 + miR.520f + miR.220a + miR.26a + miR.29a + miR.376a* + miR.494 + miR.518c + miR.660 | 0,81 | 0,051301049 |
| miR.558 + miR.609 + miR.603 + miR.520f + miR.220a + miR.130a + miR.29a + miR.376a* + miR.518c + miR.639 + miR.660 | 0,81 | 0,056009001 |
| miR.558 + miR.609 + miR.603 + miR.220a + miR.130a + miR.26a + miR.29a + miR.376a* + miR.494 + miR.518c + miR.660 | 0,81 | 0,05768124 |
| miR.558 + miR.609 + miR.520f + miR.220a + miR.130a + miR.26a + miR.29a + miR.369.3p + miR.376a* + miR.518c + miR.338 | 0,81 | 0,043360833 |
| miR.558 + miR.609 + miR.603 + miR.520f + miR.220a + miR.29a + miR.362 + miR.376a* + miR.494 + miR.518c + miR.338 | 0,81 | 0,016421895 |
| miR.558 + miR.609 + miR.603 + miR.220a + miR.26a + miR.29a + miR.362 + miR.376a* + miR.494 + miR.518c + miR.660 | 0,81 | 0,072490939 |
| miR.558 + miR.609 + miR.220a + miR.130a + miR.26a + miR.29a + miR.369.3p + miR.376a* + miR.518c + miR.639 + miR.338 | 0,81 | 0,065646277 |
| miR.558 + miR.609 + miR.603 + miR.220a + miR.519b + miR.26a + miR.29a + miR.376a* + miR.494 + miR.518c + miR.639 | 0,81 | 0,046523365 |
| miR.558 + miR.609 + miR.220a + miR.130a + miR.29a + miR.369.3p + miR.376a* + miR.494 + miR.518c + miR.639 + miR.660 | 0,81 | 0,111273576 |
| miR.558 + miR.609 + miR.603 + miR.220a + miR.519b + miR.29a + miR.362 + miR.376a* + miR.494 + miR.518c + miR.639 | 0,81 | 0,097485823 |
| miR.558 + miR.609 + miR.603 + miR.220a + miR.130a + miR.26a + miR.29a + miR.362 + miR.376a* + miR.518c + miR.639 | 0,81 | 0,052467669 |
| miR.558 + miR.609 + miR.603 + miR.520f + miR.519b + miR.26a + miR.29a + miR.369.3p + miR.376a* + miR.518c + miR.338 | 0,81 | 0,0378875 |
| miR.558 + miR.609 + miR.520f + miR.220a + miR.519b + miR.29a + miR.369.3p + miR.376a* + miR.494 + miR.518c + miR.338 | 0,81 | 0,089804686 |
| miR.558 + miR.609 + miR.603 + miR.220a + miR.130a + miR.26a + miR.29a + miR.362 + miR.376a* + miR.518c + miR.338 | 0,81 | 0,056385248 |
| miR.558 + miR.609 + miR.603 + miR.520f + miR.220a + miR.519b + miR.130a + miR.29a + miR.376a* + miR.518c + miR.660 | 0,81 | 0,132753572 |
| miR.558 + miR.609 + miR.603 + miR.130a + miR.26a + miR.29a + miR.369.3p + miR.376a* + miR.494 + miR.518c + miR.639 | 0,81 | 0,054725285 |
| miR.558 + miR.609 + miR.220a + miR.29a + miR.362 + miR.369.3p + miR.376a* + miR.494 + miR.518c + miR.639 + miR.660 | 0,81 | 0,153042349 |
| miR.558 + miR.609 + miR.220a + miR.519b + miR.26a + miR.29a + miR.369.3p + miR.376a* + miR.494 + miR.518c + miR.338 | 0,81 | 0,080942299 |
| miR.558 + miR.609 + miR.603 + miR.520f + miR.220a + miR.519b + miR.29a + miR.376a* + miR.518c + miR.660 + miR.338 | 0,81 | 0,060178915 |
| miR.558 + miR.609 + miR.603 + miR.26a + miR.29a + miR.369.3p + miR.376a* + miR.494 + miR.518c + miR.639 + miR.660 | 0,81 | 0,062862694 |
| miR.558 + miR.609 + miR.603 + miR.520f + miR.519b + miR.29a + miR.362 + miR.369.3p + miR.376a* + miR.518c + miR.338 | 0,81 | 0,038116876 |
| miR.558 + miR.609 + miR.520f + miR.220a + miR.130a + miR.29a + miR.362 + miR.369.3p + miR.376a* + miR.518c + miR.338 | 0,81 | 0,01928441 |
| miR.558 + miR.609 + miR.220a + miR.130a + miR.26a + miR.29a + miR.369.3p + miR.376a* + miR.494 + miR.518c + miR.338 | 0,81 | 0,069434014 |
| miR.558 + miR.609 + miR.520f + miR.26a + miR.29a + miR.369.3p + miR.376a* + miR.494 + miR.518c + miR.660 + miR.338 | 0,81 | 0,093282086 |
| miR.558 + miR.609 + miR.603 + miR.520f + miR.220a + miR.26a + miR.29a + miR.362 + miR.376a* + miR.518c + miR.660 | 0,81 | 0,075718044 |
| miR.558 + miR.609 + miR.603 + miR.520f + miR.220a + miR.26a + miR.29a + miR.362 + miR.376a* + miR.518c + miR.338 | 0,81 | 0,029713564 |
| miR.558 + miR.609 + miR.603 + miR.520f + miR.220a + miR.130a + miR.29a + miR.376a* + miR.518c + miR.660 + miR.338 | 0,81 | 0,041172667 |
| miR.558 + miR.609 + miR.520f + miR.220a + miR.26a + miR.29a + miR.362 + miR.369.3p + miR.376a* + miR.494 + miR.518c | 0,81 | 0,051887657 |
| miR.558 + miR.609 + miR.603 + miR.520f + miR.26a + miR.29a + miR.369.3p + miR.376a* + miR.494 + miR.518c + miR.639 | 0,81 | 0,019119189 |
| miR.558 + miR.609 + miR.520f + miR.130a + miR.29a + miR.369.3p + miR.376a* + miR.494 + miR.518c + miR.660 + miR.338 | 0,81 | 0,092761698 |
| miR.558 + miR.609 + miR.603 + miR.130a + miR.29a + miR.362 + miR.369.3p + miR.376a* + miR.518c + miR.639 + miR.660 | 0,81 | 0,107461489 |
| miR.558 + miR.609 + miR.603 + miR.220a + miR.26a + miR.29a + miR.376a* + miR.518c + miR.639 + miR.660 + miR.338 | 0,81 | 0,079419293 |
| miR.558 + miR.609 + miR.603 + miR.220a + miR.519b + miR.130a + miR.26a + miR.29a + miR.376a* + miR.518c + miR.338 | 0,81 | 0,03135775 |
| miR.558 + miR.609 + miR.603 + miR.220a + miR.519b + miR.29a + miR.362 + miR.376a* + miR.494 + miR.518c + miR.660 | 0,81 | 0,100913603 |
| miR.558 + miR.609 + miR.603 + miR.520f + miR.220a + miR.130a + miR.29a + miR.376a* + miR.494 + miR.518c + miR.660 | 0,81 | 0,049787804 |
| miR.558 + miR.609 + miR.603 + miR.220a + miR.130a + miR.29a + miR.369.3p + miR.376a* + miR.518c + miR.639 + miR.338 | 0,81 | 0,027100903 |
| miR.558 + miR.609 + miR.220a + miR.26a + miR.29a + miR.362 + miR.369.3p + miR.376a* + miR.494 + miR.518c + miR.338 | 0,81 | 0,048225115 |
| miR.558 + miR.609 + miR.603 + miR.220a + miR.130a + miR.26a + miR.29a + miR.376a* + miR.494 + miR.518c + miR.338 | 0,81 | 0,019214948 |
| miR.558 + miR.609 + miR.603 + miR.220a + miR.519b + miR.130a + miR.26a + miR.29a + miR.376a* + miR.518c + miR.660 | 0,81 | 0,118081012 |
| miR.558 + miR.609 + miR.603 + miR.520f + miR.220a + miR.29a + miR.362 + miR.376a* + miR.494 + miR.518c + miR.660 | 0,81 | 0,042256704 |
| miR.558 + miR.609 + miR.603 + miR.220a + miR.519b + miR.26a + miR.29a + miR.376a* + miR.518c + miR.639 + miR.660 | 0,81 | 0,11382439 |
| miR.558 + miR.609 + miR.603 + miR.220a + miR.130a + miR.26a + miR.29a + miR.376a* + miR.518c + miR.639 + miR.660 | 0,81 | 0,086310468 |
| miR.558 + miR.609 + miR.220a + miR.519b + miR.130a + miR.26a + miR.29a + miR.369.3p + miR.376a* + miR.518c + miR.660 | 0,81 | 0,294569844 |
| miR.558 + miR.609 + miR.603 + miR.220a + miR.519b + miR.26a + miR.29a + miR.376a* + miR.518c + miR.639 + miR.338 | 0,81 | 0,03381292 |
| miR.558 + miR.609 + miR.603 + miR.520f + miR.220a + miR.26a + miR.29a + miR.376a* + miR.518c + miR.660 + miR.338 | 0,81 | 0,045403754 |
| miR.558 + miR.609 + miR.603 + miR.520f + miR.220a + miR.130a + miR.29a + miR.362 + miR.376a* + miR.518c + miR.338 | 0,81 | 0,013739647 |
| miR.558 + miR.609 + miR.603 + miR.220a + miR.519b + miR.130a + miR.29a + miR.362 + miR.376a* + miR.518c + miR.639 | 0,81 | 0,075407389 |
| miR.558 + miR.609 + miR.520f + miR.220a + miR.26a + miR.29a + miR.362 + miR.369.3p + miR.376a* + miR.518c + miR.639 | 0,81 | 0,053593204 |
| miR.558 + miR.609 + miR.603 + miR.220a + miR.519b + miR.29a + miR.376a* + miR.494 + miR.518c + miR.639 + miR.660 | 0,81 | 0,085050933 |
| miR.558 + miR.609 + miR.603 + miR.220a + miR.519b + miR.130a + miR.29a + miR.362 + miR.376a* + miR.518c + miR.338 | 0,81 | 0,051576947 |
| miR.558 + miR.609 + miR.220a + miR.519b + miR.29a + miR.362 + miR.369.3p + miR.376a* + miR.494 + miR.518c + miR.660 | 0,81 | 0,151790369 |
| miR.558 + miR.609 + miR.603 + miR.520f + miR.519b + miR.29a + miR.369.3p + miR.376a* + miR.518c + miR.639 + miR.660 | 0,81 | 0,014192635 |
| miR.558 + miR.609 + miR.520f + miR.220a + miR.130a + miR.29a + miR.369.3p + miR.376a* + miR.494 + miR.518c + miR.338 | 0,81 | 0,012472117 |
| miR.558 + miR.609 + miR.603 + miR.220a + miR.130a + miR.26a + miR.29a + miR.376a* + miR.518c + miR.660 + miR.338 | 0,81 | 0,076540686 |
| miR.558 + miR.609 + miR.220a + miR.130a + miR.29a + miR.369.3p + miR.376a* + miR.494 + miR.518c + miR.660 + miR.338 | 0,81 | 0,067014402 |
| miR.558 + miR.609 + miR.603 + miR.130a + miR.29a + miR.369.3p + miR.376a* + miR.494 + miR.518c + miR.639 + miR.660 | 0,81 | 0,086421408 |
| miR.558 + miR.609 + miR.603 + miR.520f + miR.220a + miR.29a + miR.362 + miR.376a* + miR.518c + miR.660 + miR.338 | 0,81 | 0,041981926 |
| miR.558 + miR.609 + miR.603 + miR.220a + miR.519b + miR.29a + miR.376a* + miR.494 + miR.518c + miR.660 + miR.338 | 0,81 | 0,083692371 |
| miR.558 + miR.609 + miR.603 + miR.220a + miR.519b + miR.29a + miR.362 + miR.376a* + miR.518c + miR.639 + miR.338 | 0,81 | 0,04976216 |
| miR.558 + miR.609 + miR.603 + miR.520f + miR.220a + miR.130a + miR.29a + miR.362 + miR.376a* + miR.518c + miR.639 | 0,81 | 0,037063648 |
| miR.558 + miR.609 + miR.520f + miR.220a + miR.26a + miR.29a + miR.369.3p + miR.376a* + miR.494 + miR.518c + miR.639 | 0,81 | 0,039214085 |
| miR.558 + miR.609 + miR.603 + miR.220a + miR.26a + miR.29a + miR.362 + miR.376a* + miR.518c + miR.639 + miR.660 | 0,81 | 0,087085895 |
| miR.558 + miR.609 + miR.603 + miR.130a + miR.26a + miR.29a + miR.369.3p + miR.376a* + miR.518c + miR.639 + miR.660 | 0,81 | 0,081616764 |
| miR.558 + miR.609 + miR.220a + miR.519b + miR.130a + miR.26a + miR.29a + miR.369.3p + miR.376a* + miR.518c + miR.338 | 0,81 | 0,085988815 |
| miR.558 + miR.609 + miR.603 + miR.519b + miR.130a + miR.29a + miR.369.3p + miR.376a* + miR.494 + miR.518c + miR.639 | 0,81 | 0,092592731 |
| miR.558 + miR.609 + miR.220a + miR.519b + miR.130a + miR.29a + miR.369.3p + miR.376a* + miR.518c + miR.639 + miR.660 | 0,81 | 0,201261968 |
| miR.558 + miR.609 + miR.603 + miR.220a + miR.130a + miR.26a + miR.29a + miR.376a* + miR.518c + miR.639 + miR.338 | 0,81 | 0,022175169 |
| miR.558 + miR.609 + miR.603 + miR.520f + miR.220a + miR.26a + miR.29a + miR.362 + miR.376a* + miR.494 + miR.518c | 0,81 | 0,035996796 |
| miR.558 + miR.609 + miR.220a + miR.519b + miR.29a + miR.362 + miR.369.3p + miR.376a* + miR.518c + miR.639 + miR.660 | 0,81 | 0,186763379 |
| miR.558 + miR.609 + miR.603 + miR.520f + miR.220a + miR.26a + miR.29a + miR.376a* + miR.518c + miR.639 + miR.338 | 0,81 | 0,01721356 |
| miR.558 + miR.609 + miR.220a + miR.26a + miR.29a + miR.362 + miR.369.3p + miR.376a* + miR.518c + miR.639 + miR.338 | 0,81 | 0,064255573 |
| miR.558 + miR.609 + miR.603 + miR.26a + miR.29a + miR.369.3p + miR.376a* + miR.494 + miR.518c + miR.660 + miR.338 | 0,81 | 0,04049375 |
| miR.558 + miR.609 + miR.520f + miR.220a + miR.29a + miR.362 + miR.376a* + miR.518c + miR.639 + miR.660 + miR.338 | 0,81 | 0,022946312 |
| miR.558 + miR.609 + miR.603 + miR.220a + miR.29a + miR.362 + miR.369.3p + miR.376a* + miR.494 + miR.518c + miR.639 | 0,81 | 0,015549792 |
| miR.558 + miR.609 + miR.603 + miR.220a + miR.26a + miR.29a + miR.362 + miR.376a* + miR.518c + miR.660 + miR.338 | 0,81 | 0,077055176 |
| miR.558 + miR.609 + miR.603 + miR.220a + miR.130a + miR.26a + miR.29a + miR.362 + miR.376a* + miR.518c + miR.660 | 0,81 | 0,095416401 |
| miR.558 + miR.609 + miR.520f + miR.220a + miR.29a + miR.362 + miR.376a* + miR.494 + miR.518c + miR.660 + miR.338 | 0,81 | 0,025050582 |
| miR.558 + miR.609 + miR.603 + miR.520f + miR.220a + miR.29a + miR.362 + miR.376a* + miR.518c + miR.639 + miR.660 | 0,81 | 0,07243504 |
| miR.558 + miR.609 + miR.603 + miR.520f + miR.220a + miR.130a + miR.29a + miR.362 + miR.376a* + miR.494 + miR.518c | 0,81 | 0,019927203 |
| miR.558 + miR.609 + miR.603 + miR.520f + miR.29a + miR.362 + miR.369.3p + miR.376a* + miR.494 + miR.518c + miR.660 | 0,81 | 0,012455695 |
| miR.558 + miR.609 + miR.603 + miR.220a + miR.519b + miR.130a + miR.29a + miR.362 + miR.376a* + miR.518c + miR.660 | 0,81 | 0,075968748 |
| miR.558 + miR.609 + miR.520f + miR.220a + miR.26a + miR.29a + miR.376a* + miR.518c + miR.639 + miR.660 + miR.338 | 0,81 | 0,03433846 |
| miR.558 + miR.609 + miR.603 + miR.220a + miR.130a + miR.29a + miR.376a* + miR.494 + miR.518c + miR.639 + miR.660 | 0,81 | 0,062226162 |
| miR.558 + miR.609 + miR.520f + miR.220a + miR.519b + miR.26a + miR.29a + miR.369.3p + miR.376a* + miR.518c + miR.338 | 0,81 | 0,093529858 |
| miR.558 + miR.609 + miR.220a + miR.519b + miR.130a + miR.29a + miR.369.3p + miR.376a* + miR.518c + miR.660 + miR.338 | 0,81 | 0,198581619 |
| miR.558 + miR.609 + miR.603 + miR.26a + miR.29a + miR.362 + miR.369.3p + miR.376a* + miR.494 + miR.518c + miR.660 | 0,81 | 0,049827665 |
| miR.558 + miR.609 + miR.603 + miR.520f + miR.29a + miR.362 + miR.376a* + miR.494 + miR.518c + miR.639 + miR.338 | 0,81 | 0,007905548 |
| miR.558 + miR.609 + miR.603 + miR.220a + miR.29a + miR.362 + miR.369.3p + miR.376a* + miR.494 + miR.518c + miR.338 | 0,81 | 0,022692148 |
| miR.558 + miR.609 + miR.603 + miR.220a + miR.519b + miR.29a + miR.369.3p + miR.376a* + miR.518c + miR.639 + miR.338 | 0,81 | 0,067244444 |
| miR.558 + miR.609 + miR.220a + miR.519b + miR.26a + miR.29a + miR.362 + miR.369.3p + miR.376a* + miR.494 + miR.518c | 0,81 | 0,117542114 |
| miR.558 + miR.609 + miR.130a + miR.29a + miR.362 + miR.369.3p + miR.376a* + miR.518c + miR.639 + miR.660 + miR.338 | 0,81 | 0,220689648 |
| miR.558 + miR.609 + miR.603 + miR.520f + miR.26a + miR.29a + miR.369.3p + miR.376a* + miR.494 + miR.518c + miR.660 | 0,81 | 0,016007002 |
| miR.558 + miR.609 + miR.603 + miR.520f + miR.26a + miR.29a + miR.362 + miR.369.3p + miR.376a* + miR.518c + miR.639 | 0,81 | 0,022021555 |
| miR.558 + miR.609 + miR.130a + miR.26a + miR.29a + miR.369.3p + miR.376a* + miR.494 + miR.518c + miR.639 + miR.660 | 0,81 | 0,132901754 |
| miR.558 + miR.609 + miR.220a + miR.26a + miR.29a + miR.362 + miR.369.3p + miR.376a* + miR.494 + miR.518c + miR.639 | 0,81 | 0,069800398 |
| miR.558 + miR.609 + miR.603 + miR.520f + miR.130a + miR.29a + miR.369.3p + miR.376a* + miR.494 + miR.518c + miR.639 | 0,81 | 0,012340108 |
| miR.558 + miR.609 + miR.520f + miR.220a + miR.29a + miR.362 + miR.369.3p + miR.376a* + miR.494 + miR.518c + miR.639 | 0,81 | 0,015361333 |
| miR.558 + miR.609 + miR.520f + miR.220a + miR.130a + miR.29a + miR.369.3p + miR.376a* + miR.518c + miR.639 + miR.338 | 0,81 | 0,025699046 |
| miR.558 + miR.609 + miR.520f + miR.220a + miR.130a + miR.29a + miR.376a* + miR.518c + miR.639 + miR.660 + miR.338 | 0,81 | 0,030405254 |
| miR.558 + miR.609 + miR.603 + miR.520f + miR.519b + miR.29a + miR.376a* + miR.494 + miR.518c + miR.639 + miR.338 | 0,81 | 0,020540108 |
| miR.558 + miR.609 + miR.520f + miR.220a + miR.26a + miR.29a + miR.376a* + miR.494 + miR.518c + miR.660 + miR.338 | 0,81 | 0,042497847 |
| miR.558 + miR.609 + miR.603 + miR.220a + miR.29a + miR.362 + miR.369.3p + miR.376a* + miR.518c + miR.639 + miR.338 | 0,81 | 0,016978118 |
| miR.558 + miR.609 + miR.603 + miR.520f + miR.220a + miR.519b + miR.29a + miR.376a* + miR.518c + miR.639 + miR.338 | 0,81 | 0,01481517 |
| miR.558 + miR.609 + miR.520f + miR.130a + miR.26a + miR.29a + miR.369.3p + miR.376a* + miR.518c + miR.660 + miR.338 | 0,81 | 0,124051098 |
| miR.558 + miR.609 + miR.520f + miR.220a + miR.519b + miR.29a + miR.362 + miR.369.3p + miR.376a* + miR.518c + miR.338 | 0,81 | 0,078314353 |
| miR.558 + miR.609 + miR.603 + miR.520f + miR.220a + miR.519b + miR.130a + miR.29a + miR.376a* + miR.518c + miR.338 | 0,81 | 0,021390948 |
| miR.558 + miR.609 + miR.603 + miR.520f + miR.519b + miR.29a + miR.362 + miR.376a* + miR.518c + miR.639 + miR.338 | 0,81 | 0,025591113 |
| miR.558 + miR.609 + miR.603 + miR.220a + miR.519b + miR.29a + miR.362 + miR.376a* + miR.518c + miR.660 + miR.338 | 0,81 | 0,06634884 |
| miR.558 + miR.609 + miR.603 + miR.520f + miR.220a + miR.519b + miR.26a + miR.29a + miR.376a* + miR.518c + miR.338 | 0,81 | 0,024426349 |
| miR.558 + miR.609 + miR.520f + miR.519b + miR.130a + miR.29a + miR.369.3p + miR.376a* + miR.518c + miR.639 + miR.660 | 0,81 | 0,050286249 |
| miR.558 + miR.609 + miR.520f + miR.220a + miR.519b + miR.29a + miR.362 + miR.369.3p + miR.376a* + miR.494 + miR.518c | 0,81 | 0,112318628 |
| miR.558 + miR.609 + miR.603 + miR.220a + miR.519b + miR.29a + miR.362 + miR.376a* + miR.518c + miR.639 + miR.660 | 0,81 | 0,075811398 |
| miR.558 + miR.609 + miR.519b + miR.130a + miR.29a + miR.362 + miR.369.3p + miR.376a* + miR.518c + miR.639 + miR.660 | 0,81 | 0,09718383 |
| miR.558 + miR.609 + miR.520f + miR.26a + miR.29a + miR.369.3p + miR.376a* + miR.494 + miR.518c + miR.639 + miR.338 | 0,81 | 0,098814329 |
| miR.558 + miR.609 + miR.603 + miR.520f + miR.130a + miR.26a + miR.29a + miR.369.3p + miR.376a* + miR.518c + miR.639 | 0,81 | 0,035025936 |
| miR.558 + miR.609 + miR.603 + miR.520f + miR.519b + miR.29a + miR.376a* + miR.494 + miR.518c + miR.660 + miR.338 | 0,81 | 0,022883571 |
| miR.558 + miR.609 + miR.603 + miR.26a + miR.29a + miR.362 + miR.369.3p + miR.376a* + miR.494 + miR.518c + miR.338 | 0,81 | 0,040336229 |
| miR.558 + miR.609 + miR.603 + miR.519b + miR.26a + miR.29a + miR.369.3p + miR.376a* + miR.494 + miR.518c + miR.338 | 0,81 | 0,078455313 |
| miR.558 + miR.609 + miR.220a + miR.519b + miR.26a + miR.29a + miR.369.3p + miR.376a* + miR.518c + miR.639 + miR.338 | 0,81 | 0,106678031 |
| miR.558 + miR.609 + miR.603 + miR.519b + miR.26a + miR.29a + miR.369.3p + miR.376a* + miR.494 + miR.518c + miR.639 | 0,81 | 0,047800307 |
| miR.558 + miR.609 + miR.220a + miR.130a + miR.26a + miR.29a + miR.369.3p + miR.376a* + miR.494 + miR.518c + miR.639 | 0,81 | 0,056661572 |
| miR.558 + miR.609 + miR.220a + miR.130a + miR.26a + miR.29a + miR.362 + miR.369.3p + miR.376a* + miR.518c + miR.338 | 0,81 | 0,06114084 |
| miR.558 + miR.609 + miR.520f + miR.220a + miR.519b + miR.26a + miR.29a + miR.362 + miR.369.3p + miR.376a* + miR.518c | 0,81 | 0,104305147 |
| miR.558 + miR.609 + miR.603 + miR.520f + miR.29a + miR.362 + miR.369.3p + miR.376a* + miR.494 + miR.518c + miR.639 | 0,81 | 0,006938993 |
| miR.558 + miR.609 + miR.520f + miR.220a + miR.519b + miR.29a + miR.376a* + miR.494 + miR.518c + miR.639 + miR.660 | 0,81 | 0,075863803 |
| miR.558 + miR.609 + miR.520f + miR.130a + miR.29a + miR.362 + miR.369.3p + miR.376a* + miR.518c + miR.639 + miR.660 | 0,81 | 0,042787163 |
| miR.558 + miR.609 + miR.520f + miR.220a + miR.130a + miR.29a + miR.362 + miR.369.3p + miR.376a* + miR.494 + miR.518c | 0,81 | 0,026105257 |
| miR.558 + miR.609 + miR.220a + miR.130a + miR.26a + miR.29a + miR.362 + miR.369.3p + miR.376a* + miR.518c + miR.639 | 0,81 | 0,073602386 |
| miR.558 + miR.609 + miR.603 + miR.520f + miR.220a + miR.130a + miR.26a + miR.29a + miR.376a* + miR.518c + miR.660 | 0,81 | 0,057573822 |
| miR.558 + miR.609 + miR.520f + miR.220a + miR.519b + miR.29a + miR.362 + miR.376a* + miR.494 + miR.518c + miR.338 | 0,81 | 0,156587474 |
| miR.558 + miR.609 + miR.603 + miR.520f + miR.220a + miR.130a + miR.26a + miR.29a + miR.362 + miR.376a* + miR.518c | 0,81 | 0,041293271 |
| miR.558 + miR.609 + miR.520f + miR.220a + miR.130a + miR.29a + miR.376a* + miR.494 + miR.518c + miR.660 + miR.338 | 0,81 | 0,035990165 |
| miR.558 + miR.609 + miR.520f + miR.220a + miR.130a + miR.26a + miR.29a + miR.362 + miR.369.3p + miR.376a* + miR.518c | 0,81 | 0,061490013 |
| miR.558 + miR.609 + miR.520f + miR.519b + miR.130a + miR.29a + miR.369.3p + miR.376a* + miR.518c + miR.660 + miR.338 | 0,81 | 0,161684369 |
| miR.558 + miR.609 + miR.220a + miR.519b + miR.26a + miR.29a + miR.362 + miR.369.3p + miR.376a* + miR.518c + miR.338 | 0,81 | 0,086573213 |
| miR.558 + miR.609 + miR.520f + miR.29a + miR.362 + miR.369.3p + miR.376a* + miR.494 + miR.518c + miR.639 + miR.660 | 0,81 | 0,005060017 |
| miR.558 + miR.609 + miR.220a + miR.519b + miR.130a + miR.26a + miR.29a + miR.369.3p + miR.376a* + miR.494 + miR.518c | 0,81 | 0,129172239 |
| miR.558 + miR.609 + miR.603 + miR.520f + miR.220a + miR.26a + miR.29a + miR.376a* + miR.494 + miR.518c + miR.338 | 0,81 | 0,009723753 |
| miR.558 + miR.609 + miR.520f + miR.220a + miR.130a + miR.26a + miR.29a + miR.369.3p + miR.376a* + miR.494 + miR.518c | 0,81 | 0,0467 3094 |
| miR.558 + miR.609 + miR.26a + miR.29a + miR.362 + miR.369.3p + miR.376a* + miR.494 + miR.518c + miR.660 + miR.338 | 0,81 | 0,296286447 |
| miR.558 + miR.609 + miR.603 + miR.520f + miR.519b + miR.29a + miR.362 + miR.376a* + miR.494 + miR.518c + miR.338 | 0,81 | 0,036308954 |
| miR.558 + miR.609 + miR.603 + miR.520f + miR.519b + miR.26a + miR.29a + miR.369.3p + miR.376a* + miR.518c + miR.639 | 0,81 | 0,02993632 |
| miR.558 + miR.609 + miR.520f + miR.220a + miR.519b + miR.130a + miR.29a + miR.376a* + miR.494 + miR.518c + miR.660 | 0,81 | 0,099339586 |
| miR.558 + miR.609 + miR.520f + miR.220a + miR.519b + miR.26a + miR.29a + miR.376a* + miR.494 + miR.518c + miR.660 | 0,81 | 0,104036996 |
| miR.558 + miR.609 + miR.520f + miR.220a + miR.519b + miR.29a + miR.376a* + miR.518c + miR.639 + miR.660 + miR.338 | 0,81 | 0,052831722 |
| miR.558 + miR.609 + miR.603 + miR.519b + miR.26a + miR.29a + miR.369.3p + miR.376a* + miR.518c + miR.639 + miR.660 | 0,81 | 0,049674536 |
| miR.558 + miR.609 + miR.220a + miR.519b + miR.26a + miR.29a + miR.369.3p + miR.376a* + miR.494 + miR.518c + miR.639 | 0,81 | 0,11045933 |
| miR.558 + miR.609 + miR.220a + miR.130a + miR.26a + miR.29a + miR.362 + miR.369.3p + miR.376a* + miR.494 + miR.518c | 0,81 | 0,056192034 |
| miR.558 + miR.609 + miR.520f + miR.220a + miR.519b + miR.29a + miR.362 + miR.376a* + miR.494 + miR.518c + miR.660 | 0,81 | 0,112225925 |
| miR.558 + miR.609 + miR.603 + miR.520f + miR.519b + miR.29a + miR.369.3p + miR.376a* + miR.494 + miR.518c + miR.639 | 0,81 | 0,023455678 |
| miR.558 + miR.609 + miR.603 + miR.130a + miR.29a + miR.369.3p + miR.376a* + miR.494 + miR.518c + miR.639 + miR.338 | 0,81 | 0,063600125 |
| miR.558 + miR.609 + miR.603 + miR.520f + miR.519b + miR.29a + miR.369.3p + miR.376a* + miR.494 + miR.518c + miR.660 | 0,81 | 0,025545841 |
| miR.558 + miR.609 + miR.603 + miR.520f + miR.130a + miR.29a + miR.369.3p + miR.376a* + miR.494 + miR.518c + miR.660 | 0,81 | 0,011931387 |
| miR.558 + miR.609 + miR.520f + miR.29a + miR.362 + miR.369.3p + miR.376a* + miR.494 + miR.639 + miR.660 + miR.338 | 0,81 | 0,416173783 |
| miR.558 + miR.609 + miR.520f + miR.220a + miR.130a + miR.29a + miR.376a* + miR.494 + miR.518c + miR.639 + miR.660 | 0,81 | 0,040495396 |
| miR.558 + miR.609 + miR.603 + miR.26a + miR.29a + miR.362 + miR.369.3p + miR.376a* + miR.494 + miR.518c + miR.639 | 0,81 | 0,038626744 |
| miR.558 + miR.609 + miR.603 + miR.520f + miR.26a + miR.29a + miR.362 + miR.369.3p + miR.376a* + miR.518c + miR.660 | 0,81 | 0,023366486 |
| miR.558 + miR.609 + miR.520f + miR.130a + miR.29a + miR.362 + miR.369.3p + miR.376a* + miR.494 + miR.518c + miR.660 | 0,81 | 0,040028487 |
| miR.558 + miR.609 + miR.603 + miR.130a + miR.26a + miR.29a + miR.369.3p + miR.376a* + miR.494 + miR.518c + miR.338 | 0,81 | 0,029667186 |
| miR.558 + miR.609 + miR.520f + miR.130a + miR.26a + miR.29a + miR.369.3p + miR.376a* + miR.518c + miR.639 + miR.660 | 0,81 | 0,088773445 |
| miR.558 + miR.609 + miR.220a + miR.519b + miR.29a + miR.369.3p + miR.376a* + miR.494 + miR.518c + miR.660 + miR.338 | 0,81 | 0,143729223 |
| miR.558 + miR.609 + miR.220a + miR.519b + miR.29a + miR.369.3p + miR.376a* + miR.494 + miR.518c + miR.639 + miR.660 | 0,81 | 0,144697773 |
| miR.558 + miR.609 + miR.520f + miR.130a + miR.29a + miR.369.3p + miR.376a* + miR.494 + miR.518c + miR.639 + miR.338 | 0,81 | 0,027816587 |
| miR.558 + miR.609 + miR.520f + miR.519b + miR.26a + miR.29a + miR.369.3p + miR.376a* + miR.518c + miR.660 + miR.338 | 0,81 | 0,154113792 |
| miR.558 + miR.609 + miR.520f + miR.220a + miR.519b + miR.130a + miR.29a + miR.362 + miR.376a* + miR.494 + miR.518c | 0,81 | 0,153084048 |
| miR.558 + miR.609 + miR.520f + miR.220a + miR.519b + miR.29a + miR.369.3p + miR.376a* + miR.518c + miR.639 + miR.338 | 0,81 | 0,063432761 |
| miR.558 + miR.609 + miR.603 + miR.520f + miR.26a + miR.29a + miR.362 + miR.376a* + miR.518c + miR.639 + miR.338 | 0,81 | 0,016097375 |
| miR.558 + miR.609 + miR.220a + miR.519b + miR.130a + miR.29a + miR.362 + miR.369.3p + miR.376a* + miR.518c + miR.660 | 0,81 | 0,18482 0007 |
| miR.558 + miR.609 + miR.603 + miR.130a + miR.26a + miR.29a + miR.369.3p + miR.376a* + miR.518c + miR.660 + miR.338 | 0,81 | 0,057914614 |
| miR.558 + miR.609 + miR.520f + miR.220a + miR.519b + miR.130a + miR.29a + miR.369.3p + miR.376a* + miR.494 + miR.518c | 0,81 | 0,095816035 |
| miR.558 + miR.609 + miR.603 + miR.519b + miR.130a + miR.26a + miR.29a + miR.369.3p + miR.376a* + miR.518c + miR.639 | 0,81 | 0,060645707 |
| miR.609 + miR.603 + miR.520f + miR.220a + miR.519b + miR.29a + miR.369.3p + miR.376a* + miR.494 + miR.518c + miR.660 | 0,81 | 0,099724155 |
| miR.558 + miR.609 + miR.520f + miR.220a + miR.519b + miR.26a + miR.29a + miR.369.3p + miR.376a* + miR.494 + miR.518c | 0,81 | 0,102782071 |
| miR.558 + miR.609 + miR.220a + miR.519b + miR.130a + miR.26a + miR.29a + miR.362 + miR.369.3p + miR.376a* + miR.518c | 0,81 | 0,123426492 |
| miR.558 + miR.609 + miR.603 + miR.520f + miR.220a + miR.130a + miR.29a + miR.362 + miR.376a* + miR.518c + miR.660 | 0,81 | 0,051362561 |
| miR.558 + miR.609 + miR.520f + miR.220a + miR.130a + miR.29a + miR.362 + miR.369.3p + miR.376a* + miR.518c + miR.639 | 0,81 | 0,02196976 |
| miR.558 + miR.609 + miR.603 + miR.520f + miR.519b + miR.29a + miR.362 + miR.369.3p + miR.376a* + miR.518c + miR.639 | 0,81 | 0,023890343 |
| miR.558 + miR.609 + miR.520f + miR.220a + miR.29a + miR.362 + miR.376a* + miR.494 + miR.518c + miR.639 + miR.338 | 0,81 | 0,018338552 |
| miR.558 + miR.609 + miR.520f + miR.29a + miR.362 + miR.369.3p + miR.376a* + miR.494 + miR.518c + miR.639 + miR.338 | 0,81 | 0,019993663 |
| miR.558 + miR.609 + miR.603 + miR.130a + miR.26a + miR.29a + miR.362 + miR.369.3p + miR.376a* + miR.518c + miR.639 | 0,81 | 0,059898262 |
| miR.558 + miR.609 + miR.603 + miR.520f + miR.26a + miR.29a + miR.376a* + miR.518c + miR.639 + miR.660 + miR.338 | 0,81 | 0,014163526 |
| miR.558 + miR.609 + miR.603 + miR.520f + miR.519b + miR.26a + miR.29a + miR.369.3p + miR.376a* + miR.494 + miR.518c | 0,81 | 0,038010751 |
| miR.609 + miR.603 + miR.520f + miR.220a + miR.29a + miR.369.3p + miR.376a* + miR.494 + miR.518c + miR.639 + miR.660 | 0,81 | 0,038549994 |
| miR.558 + miR.609 + miR.520f + miR.220a + miR.26a + miR.29a + miR.376a* + miR.494 + miR.518c + miR.639 + miR.660 | 0,81 | 0,044172366 |
| miR.558 + miR.609 + miR.520f + miR.220a + miR.29a + miR.362 + miR.376a* + miR.494 + miR.518c + miR.639 + miR.660 | 0,81 | 0,032531163 |
| miR.558 + miR.609 + miR.603 + miR.520f + miR.29a + miR.362 + miR.376a* + miR.518c + miR.639 + miR.660 + miR.338 | 0,81 | 0,014691323 |
| miR.558 + miR.609 + miR.520f + miR.519b + miR.29a + miR.362 + miR.369.3p + miR.376a* + miR.518c + miR.639 + miR.660 | 0,81 | 0,001373045 |
| miR.558 + miR.609 + miR.520f + miR.220a + miR.130a + miR.26a + miR.29a + miR.369.3p + miR.376a* + miR.518c + miR.639 | 0,81 | 0,042499172 |
| miR.558 + miR.609 + miR.603 + miR.520f + miR.220a + miR.26a + miR.29a + miR.376a* + miR.494 + miR.518c + miR.639 | 0,81 | 0,015092493 |
| miR.558 + miR.609 + miR.603 + miR.220a + miR.519b + miR.130a + miR.29a + miR.376a* + miR.518c + miR.660 + miR.338 | 0,81 | 0,069813694 |
| miR.558 + miR.609 + miR.520f + miR.220a + miR.130a + miR.29a + miR.369.3p + miR.376a* + miR.494 + miR.518c + miR.639 | 0,81 | 0,015873606 |
| miR.558 + miR.609 + miR.520f + miR.26a + miR.29a + miR.362 + miR.369.3p + miR.376a* + miR.518c + miR.639 + miR.338 | 0,81 | 0,070490723 |
| miR.558 + miR.609 + miR.603 + miR.29a + miR.362 + miR.369.3p + miR.376a* + miR.494 + miR.518c + miR.660 + miR.338 | 0,81 | 0,094213424 |
| miR.558 + miR.609 + miR.603 + miR.520f + miR.220a + miR.130a + miR.26a + miR.29a + miR.376a* + miR.518c + miR.338 | 0,81 | 0,011896494 |
| miR.558 + miR.609 + miR.520f + miR.519b + miR.29a + miR.362 + miR.376a* + miR.494 + miR.518c + miR.660 + miR.338 | 0,81 | 0,108902057 |
| miR.558 + miR.609 + miR.603 + miR.520f + miR.519b + miR.130a + miR.29a + miR.369.3p + miR.376a* + miR.494 + miR.518c | 0,81 | 0,043412609 |
| miR.558 + miR.609 + miR.603 + miR.520f + miR.130a + miR.29a + miR.362 + miR.369.3p + miR.376a* + miR.518c + miR.639 | 0,81 | 0,017273326 |
| miR.558 + miR.609 + miR.603 + miR.520f + miR.130a + miR.29a + miR.362 + miR.376a* + miR.518c + miR.639 + miR.338 | 0,81 | 0,017606101 |
| miR.558 + miR.609 + miR.520f + miR.220a + miR.519b + miR.29a + miR.362 + miR.369.3p + miR.376a* + miR.518c + miR.639 | 0,81 | 0,088455078 |
| miR.558 + miR.609 + miR.220a + miR.29a + miR.369.3p + miR.376a* + miR.494 + miR.518c + miR.639 + miR.660 + miR.338 | 0,81 | 0,106187002 |
| miR.558 + miR.609 + miR.520f + miR.220a + miR.519b + miR.29a + miR.362 + miR.376a* + miR.494 + miR.518c + miR.639 | 0,81 | 0,114745014 |
| miR.558 + miR.609 + miR.603 + miR.220a + miR.519b + miR.29a + miR.376a* + miR.518c + miR.639 + miR.660 + miR.338 | 0,81 | 0,061048784 |
| miR.558 + miR.609 + miR.603 + miR.520f + miR.130a + miR.29a + miR.376a* + miR.518c + miR.639 + miR.660 + miR.338 | 0,81 | 0,014998606 |
| miR.558 + miR.609 + miR.603 + miR.520f + miR.220a + miR.130a + miR.26a + miR.29a + miR.376a* + miR.518c + miR.639 | 0,81 | 0,018647205 |
| miR.558 + miR.609 + miR.603 + miR.520f + miR.519b + miR.29a + miR.376a* + miR.518c + miR.639 + miR.660 + miR.338 | 0,81 | 0,021039622 |
| miR.558 + miR.609 + miR.520f + miR.220a + miR.519b + miR.130a + miR.29a + miR.369.3p + miR.376a* + miR.518c + miR.338 | 0,81 | 0,059254397 |
| miR.558 + miR.609 + miR.26a + miR.29a + miR.362 + miR.369.3p + miR.376a* + miR.518c + miR.639 + miR.660 + miR.338 | 0,81 | 0,260696485 |
| miR.609 + miR.603 + miR.220a + miR.26a + miR.29a + miR.362 + miR.369.3p + miR.376a* + miR.494 + miR.518c + miR.660 | 0,81 | 0,182700703 |
| miR.558 + miR.609 + miR.520f + miR.220a + miR.519b + miR.26a + miR.29a + miR.369.3p + miR.376a* + miR.518c + miR.639 | 0,81 | 0,077060389 |
| miR.558 + miR.609 + miR.520f + miR.519b + miR.29a + miR.369.3p + miR.376a* + miR.494 + miR.518c + miR.639 + miR.338 | 0,81 | 0,066005251 |
| miR.558 + miR.609 + miR.603 + miR.520f + miR.220a + miR.519b + miR.130a + miR.26a + miR.29a + miR.376a* + miR.518c | 0,81 | 0,056802355 |
| miR.558 + miR.609 + miR.603 + miR.130a + miR.29a + miR.369.3p + miR.376a* + miR.518c + miR.639 + miR.660 + miR.338 | 0,81 | 0,130785723 |
| miR.558 + miR.609 + miR.603 + miR.220a + miR.130a + miR.29a + miR.362 + miR.376a* + miR.494 + miR.518c + miR.639 | 0,81 | 0,02207973 |
| miR.558 + miR.609 + miR.603 + miR.519b + miR.26a + miR.29a + miR.369.3p + miR.376a* + miR.518c + miR.660 + miR.338 | 0,81 | 0,05751366 |
| miR.558 + miR.609 + miR.520f + miR.130a + miR.29a + miR.362 + miR.376a* + miR.494 + miR.518c + miR.639 + miR.338 | 0,81 | 0,035704573 |
| miR.558 + miR.609 + miR.603 + miR.520f + miR.26a + miR.29a + miR.376a* + miR.494 + miR.518c + miR.639 + miR.338 | 0,81 | 0,007665705 |
| miR.558 + miR.609 + miR.220a + miR.519b + miR.130a + miR.26a + miR.29a + miR.369.3p + miR.376a* + miR.518c + miR.639 | 0,81 | 0,10393488 |
| miR.609 + miR.603 + miR.520f + miR.220a + miR.29a + miR.362 + miR.369.3p + miR.376a* + miR.494 + miR.518c + miR.660 | 0,81 | 0,169286523 |
| miR.558 + miR.609 + miR.603 + miR.520f + miR.519b + miR.29a + miR.362 + miR.369.3p + miR.376a* + miR.494 + miR.518c | 0,81 | 0,038996754 |
| miR.558 + miR.609 + miR.520f + miR.130a + miR.26a + miR.29a + miR.376a* + miR.518c + miR.639 + miR.660 + miR.338 | 0,81 | 0,023260381 |
| miR.558 + miR.609 + miR.603 + miR.520f + miR.130a + miR.26a + miR.29a + miR.369.3p + miR.376a* + miR.518c + miR.660 | 0,81 | 0,018767456 |
| miR.558 + miR.609 + miR.603 + miR.220a + miR.519b + miR.29a + miR.362 + miR.376a* + miR.494 + miR.518c + miR.338 | 0,81 | 0,090567038 |
| miR.558 + miR.609 + miR.220a + miR.519b + miR.26a + miR.29a + miR.362 + miR.369.3p + miR.376a* + miR.518c + miR.639 | 0,81 | 0,107298349 |
| miR.558 + miR.609 + miR.603 + miR.520f + miR.130a + miR.29a + miR.376a* + miR.494 + miR.518c + miR.639 + miR.338 | 0,81 | 0,00823629 |
| miR.558 + miR.609 + miR.130a + miR.26a + miR.29a + miR.369.3p + miR.376a* + miR.494 + miR.518c + miR.639 + miR.338 | 0,81 | 0,151347347 |
| miR.558 + miR.609 + miR.520f + miR.130a + miR.29a + miR.362 + miR.376a* + miR.494 + miR.518c + miR.639 + miR.660 | 0,81 | 0,012970067 |
| miR.558 + miR.609 + miR.603 + miR.520f + miR.220a + miR.130a + miR.26a + miR.29a + miR.376a* + miR.494 + miR.518c | 0,81 | 0,010543674 |
| miR.558 + miR.609 + miR.520f + miR.220a + miR.519b + miR.26a + miR.29a + miR.362 + miR.376a* + miR.494 + miR.518c | 0,81 | 0,121473899 |
| miR.609 + miR.603 + miR.220a + miR.519b + miR.26a + miR.29a + miR.369.3p + miR.376a* + miR.494 + miR.518c + miR.660 | 0,81 | 0,095758197 |
| miR.558 + miR.609 + miR.519b + miR.26a + miR.29a + miR.362 + miR.369.3p + miR.376a* + miR.518c + miR.660 + miR.338 | 0,81 | 0,193220916 |
| miR.558 + miR.609 + miR.520f + miR.130a + miR.26a + miR.29a + miR.369.3p + miR.376a* + miR.494 + miR.518c + miR.660 | 0,81 | 0,046186278 |
| miR.558 + miR.609 + miR.520f + miR.519b + miR.130a + miR.29a + miR.376a* + miR.494 + miR.518c + miR.660 + miR.338 | 0,81 | 0,182429686 |
| miR.558 + miR.609 + miR.520f + miR.130a + miR.26a + miR.29a + miR.369.3p + miR.376a* + miR.494 + miR.518c + miR.639 | 0,81 | 0,058565236 |
| miR.609 + miR.603 + miR.220a + miR.29a + miR.362 + miR.369.3p + miR.376a* + miR.494 + miR.518c + miR.639 + miR.660 | 0,81 | 0,25083681 |
| miR.558 + miR.609 + miR.603 + miR.130a + miR.26a + miR.29a + miR.362 + miR.369.3p + miR.376a* + miR.518c + miR.338 | 0,81 | 0,035361638 |
| miR.558 + miR.609 + miR.520f + miR.519b + miR.29a + miR.376a* + miR.494 + miR.518c + miR.639 + miR.660 + miR.338 | 0,81 | 0,050680595 |
| miR.558 + miR.609 + miR.603 + miR.520f + miR.130a + miR.26a + miR.29a + miR.369.3p + miR.376a* + miR.494 + miR.518c | 0,81 | 0,021069965 |
| miR.558 + miR.609 + miR.520f + miR.519b + miR.130a + miR.29a + miR.376a* + miR.494 + miR.518c + miR.639 + miR.660 | 0,81 | 0,031941045 |
| miR.558 + miR.609 + miR.603 + miR.520f + miR.220a + miR.519b + miR.26a + miR.29a + miR.376a* + miR.518c + miR.639 | 0,81 | 0,049963026 |
| miR.558 + miR.609 + miR.220a + miR.519b + miR.29a + miR.369.3p + miR.376a* + miR.518c + miR.639 + miR.660 + miR.338 | 0,81 | 0,141750039 |
| miR.558 + miR.609 + miR.520f + miR.220a + miR.519b + miR.130a + miR.29a + miR.362 + miR.369.3p + miR.376a* + miR.518c | 0,81 | 0,096622842 |
| miR.558 + miR.609 + miR.603 + miR.29a + miR.362 + miR.369.3p + miR.376a* + miR.494 + miR.518c + miR.639 + miR.660 | 0,81 | 0,034245163 |
| miR.558 + miR.609 + miR.603 + miR.520f + miR.26a + miR.29a + miR.362 + miR.369.3p + miR.376a* + miR.494 + miR.518c | 0,81 | 0,014757351 |
| miR.558 + miR.609 + miR.603 + miR.519b + miR.130a + miR.29a + miR.369.3p + miR.376a* + miR.518c + miR.639 + miR.338 | 0,81 | 0,083844351 |
| miR.558 + miR.609 + miR.520f + miR.220a + miR.26a + miR.29a + miR.362 + miR.376a* + miR.494 + miR.518c + miR.338 | 0,81 | 0,028961257 |
| miR.558 + miR.609 + miR.603 + miR.519b + miR.130a + miR.26a + miR.29a + miR.369.3p + miR.376a* + miR.494 + miR.518c | 0,81 | 0,049473938 |
| miR.558 + miR.609 + miR.603 + miR.520f + miR.519b + miR.130a + miR.29a + miR.369.3p + miR.376a* + miR.518c + miR.639 | 0,81 | 0,045356413 |
| miR.558 + miR.609 + miR.520f + miR.130a + miR.26a + miR.29a + miR.376a* + miR.494 + miR.518c + miR.639 + miR.660 | 0,81 | 0,01361162 |
| miR.609 + miR.603 + miR.520f + miR.220a + miR.29a + miR.362 + miR.369.3p + miR.376a* + miR.518c + miR.660 + miR.338 | 0,81 | 0,365700497 |
| miR.558 + miR.609 + miR.603 + miR.520f + miR.519b + miR.29a + miR.362 + miR.369.3p + miR.376a* + miR.518c + miR.660 | 0,81 | 0,026497458 |
| miR.558 + miR.609 + miR.520f + miR.26a + miR.29a + miR.369.3p + miR.376a* + miR.494 + miR.518c + miR.639 + miR.660 | 0,81 | 0,040835548 |
| miR.558 + miR.609 + miR.520f + miR.220a + miR.130a + miR.26a + miR.29a + miR.376a* + miR.518c + miR.660 + miR.338 | 0,81 | 0,037096068 |
| miR.558 + miR.609 + miR.603 + miR.220a + miR.519b + miR.130a + miR.26a + miR.29a + miR.376a* + miR.518c + miR.639 | 0,81 | 0,044256523 |
| miR.558 + miR.609 + miR.520f + miR.220a + miR.26a + miR.29a + miR.362 + miR.376a* + miR.518c + miR.639 + miR.338 | 0,81 | 0,040345533 |
| miR.558 + miR.609 + miR.603 + miR.519b + miR.130a + miR.26a + miR.29a + miR.369.3p + miR.376a* + miR.518c + miR.338 | 0,81 | 0,0479813 |
| miR.609 + miR.603 + miR.220a + miR.519b + miR.29a + miR.362 + miR.369.3p + miR.376a* + miR.494 + miR.518c + miR.660 | 0,81 | 0,178950875 |
| miR.558 + miR.609 + miR.603 + miR.130a + miR.29a + miR.362 + miR.369.3p + miR.376a* + miR.518c + miR.639 + miR.338 | 0,81 | 0,067925379 |
| miR.558 + miR.609 + miR.520f + miR.220a + miR.519b + miR.29a + miR.362 + miR.376a* + miR.518c + miR.639 + miR.338 | 0,81 | 0,066226981 |
| miR.558 + miR.609 + miR.603 + miR.520f + miR.519b + miR.26a + miR.29a + miR.376a* + miR.494 + miR.518c + miR.338 | 0,81 | 0,027930109 |
| miR.558 + miR.609 + miR.520f + miR.130a + miR.26a + miR.29a + miR.369.3p + miR.376a* + miR.518c + miR.639 + miR.338 | 0,81 | 0,083554906 |
| miR.609 + miR.603 + miR.520f + miR.220a + miR.29a + miR.369.3p + miR.376a* + miR.494 + miR.518c + miR.660 + miR.338 | 0,81 | 0,120543505 |
| miR.558 + miR.609 + miR.603 + miR.520f + miR.362 + miR.369.3p + miR.376a* + miR.518c + miR.639 + miR.660 + miR.338 | 0,81 | 0,158481203 |
| miR.558 + miR.609 + miR.520f + miR.220a + miR.519b + miR.130a + miR.26a + miR.29a + miR.369.3p + miR.376a* + miR.518c | 0,81 | 0,092280989 |
| miR.558 + miR.609 + miR.603 + miR.520f + miR.220a + miR.519b + miR.130a + miR.29a + miR.376a* + miR.518c + miR.639 | 0,81 | 0,051565527 |
| miR.558 + miR.609 + miR.520f + miR.220a + miR.519b + miR.130a + miR.29a + miR.376a* + miR.518c + miR.660 + miR.338 | 0,81 | 0,068063887 |
| miR.558 + miR.609 + miR.603 + miR.130a + miR.26a + miR.29a + miR.369.3p + miR.376a* + miR.494 + miR.518c + miR.660 | 0,81 | 0,04079765 |
| miR.558 + miR.609 + miR.520f + miR.220a + miR.519b + miR.29a + miR.369.3p + miR.376a* + miR.494 + miR.518c + miR.639 | 0,81 | 0,067714172 |
| miR.558 + miR.609 + miR.520f + miR.220a + miR.26a + miR.29a + miR.362 + miR.376a* + miR.518c + miR.660 + miR.338 | 0,81 | 0,034211345 |
| miR.558 + miR.609 + miR.220a + miR.519b + miR.130a + miR.26a + miR.29a + miR.376a* + miR.494 + miR.518c + miR.660 | 0,81 | 0,118927367 |
| miR.558 + miR.609 + miR.520f + miR.220a + miR.130a + miR.29a + miR.362 + miR.376a* + miR.518c + miR.660 + miR.338 | 0,81 | 0,033067311 |
| miR.558 + miR.609 + miR.603 + miR.519b + miR.29a + miR.362 + miR.369.3p + miR.376a* + miR.518c + miR.660 + miR.338 | 0,81 | 0,070848434 |
| miR.558 + miR.609 + miR.603 + miR.520f + miR.130a + miR.29a + miR.362 + miR.369.3p + miR.376a* + miR.518c + miR.660 | 0,81 | 0,022320448 |
| miR.558 + miR.609 + miR.130a + miR.26a + miR.29a + miR.362 + miR.369.3p + miR.376a* + miR.518c + miR.639 + miR.660 | 0,81 | 0,12240663 |
| miR.558 + miR.609 + miR.130a + miR.26a + miR.29a + miR.369.3p + miR.376a* + miR.518c + miR.639 + miR.660 + miR.338 | 0,81 | 0,190655218 |
| miR.558 + miR.609 + miR.603 + miR.520f + miR.519b + miR.130a + miR.29a + miR.376a* + miR.494 + miR.518c + miR.338 | 0,81 | 0,033349431 |
| miR.558 + miR.609 + miR.520f + miR.220a + miR.130a + miR.29a + miR.362 + miR.376a* + miR.494 + miR.518c + miR.338 | 0,81 | 0,01826168 |
| miR.558 + miR.609 + miR.220a + miR.519b + miR.130a + miR.26a + miR.29a + miR.362 + miR.376a* + miR.494 + miR.518c | 0,81 | 0,146018166 |
| miR.558 + miR.609 + miR.603 + miR.520f + miR.519b + miR.26a + miR.29a + miR.369.3p + miR.376a* + miR.518c + miR.660 | 0,81 | 0,035714228 |
| miR.558 + miR.609 + miR.603 + miR.220a + miR.130a + miR.29a + miR.376a* + miR.494 + miR.518c + miR.639 + miR.338 | 0,81 | 0,008513863 |
| miR.558 + miR.609 + miR.603 + miR.519b + miR.29a + miR.362 + miR.369.3p + miR.376a* + miR.518c + miR.639 + miR.660 | 0,81 | 0,03394552 |
| miR.558 + miR.609 + miR.603 + miR.130a + miR.29a + miR.362 + miR.369.3p + miR.376a* + miR.518c + miR.660 + miR.338 | 0,81 | 0,078037577 |
| miR.558 + miR.609 + miR.603 + miR.520f + miR.220a + miR.29a + miR.376a* + miR.494 + miR.518c + miR.639 + miR.338 | 0,81 | 0,004743588 |
| miR.558 + miR.609 + miR.520f + miR.220a + miR.519b + miR.26a + miR.29a + miR.376a* + miR.518c + miR.660 + miR.338 | 0,81 | 0,059889168 |

**Table 12: clusters of 12 miRNAs**

| **Cluster of 12 miRs** | **c_tau level** | **pv compared to top signatu re miR.55 8+ miR.60 9+ miR.52 Of + miR.22 0a + miR.29a+ miR.36 2+ miR.36 9.3p + miR.37 6a* + miR.51 8c + miR.63 9+ miR.66 0+ miR.33 8** |
|---|---|---|
| miR.558 + miR.609 + miR.520f + miR.220a + miR.29a + miR.362 + miR.369.3p + miR.376a* + miR.518c + miR.639 + miR.660 + miR.338 | 0,81 | NaN |
| miR.558 + miR.609 + miR.603 + miR.520f + miR.29a + miR.362 + miR.369.3p + miR.376a* + miR.518c + miR.639 + miR.660 + miR.338 | 0,81 | 0,002592812 |
| miR.558 + miR.609 + miR.520f + miR.130a + miR.29a + miR.362 + miR.369.3p + miR.376a* + miR.518c + miR.639 + miR.660 + miR.338 | 0,81 | 0,488531084 |
| miR.558 + miR.609 + miR.520f + miR.29a + miR.362 + miR.369.3p + miR.376a* + miR.494 + miR.518c + miR.639 + miR.660 + miR.338 | 0,81 | 0,336902282 |
| miR.558 + miR.609 + miR.603 + miR.520f + miR.220a + miR.29a + miR.369.3p + miR.376a* + miR.518c + miR.639 + miR.660 + miR.338 | 0,81 | 0,02007668 |
| miR.558 + miR.609 + miR.603 + miR.220a + miR.26a + miR.29a + miR.362 + miR.369.3p + miR.376a* + miR.518c + miR.660 + miR.338 | 0,81 | 0,177098203 |
| miR.558 + miR.609 + miR.603 + miR.520f + miR.220a + miR.29a + miR.362 + miR.369.3p + miR.376a* + miR.518c + miR.660 + miR.338 | 0,81 | 0,189132192 |
| miR.558 + miR.609 + miR.520f + miR.519b + miR.29a + miR.362 + miR.369.3p + miR.376a* + miR.518c + miR.639 + miR.660 + miR.338 | 0,81 | 0,326944202 |
| miR.558 + miR.609 + miR.603 + miR.220a + miR.26a + miR.29a + miR.369.3p + miR.376a* + miR.518c + miR.639 + miR.660 + miR.338 | 0,81 | 0,124009861 |
| miR.558 + miR.609 + miR.603 + miR.220a + miR.519b + miR.26a + miR.29a + miR.369.3p + miR.376a* + miR.494 + miR.518c + miR.660 | 0,81 | 0,141408293 |
| miR.558 + miR.609 + miR.603 + miR.220a + miR.26a + miR.29a + miR.362 + miR.369.3p + miR.376a* + miR.518c + miR.639 + miR.660 | 0,81 | 0,126183915 |
| miR.558 + miR.609 + miR.603 + miR.520f + miR.220a + miR.26a + miR.29a + miR.369.3p + miR.376a* + miR.518c + miR.660 + miR.338 | 0,81 | 0,050609288 |
| miR.558 + miR.609 + miR.603 + miR.220a + miR.26a + miR.29a + miR.369.3p + miR.376a* + miR.494 + miR.518c + miR.660 + miR.338 | 0,81 | 0,065026461 |
| miR.558 + miR.609 + miR.603 + miR.220a + miR.26a + miR.29a + miR.369.3p + miR.376a* + miR.494 + miR.518c + miR.639 + miR.660 | 0,81 | 0,058482956 |
| miR.558 + miR.609 + miR.520f + miR.26a + miR.29a + miR.362 + miR.369.3p + miR.376a* + miR.518c + miR.639 + miR.660 + miR.338 | 0,81 | 0,412377362 |
| miR.558 + miR.609 + miR.603 + miR.220a + miR.26a + miR.29a + miR.362 + miR.369.3p + miR.376a* + miR.494 + miR.518c + miR.660 | 0,81 | 0,107094527 |
| miR.558 + miR.609 + miR.603 + miR.520f + miR.220a + miR.519b + miR.29a + miR.369.3p + miR.376a* + miR.518c + miR.660 + miR.338 | 0,81 | 0,06586111 |
| miR.558 + miR.609 + miR.520f + miR.220a + miR.29a + miR.362 + miR.369.3p + miR.376a* + miR.494 + miR.518c + miR.660 + miR.338 | 0,81 | 0,242438064 |
| miR.558 + miR.609 + miR.603 + miR.520f + miR.220a + miR.26a + miR.29a + miR.369.3p + miR.376a* + miR.518c + miR.639 + miR.660 | 0,81 | 0,081782138 |
| miR.558 + miR.609 + miR.603 + miR.520f + miR.220a + miR.29a + miR.369.3p + miR.376a* + miR.494 + miR.518c + miR.660 + miR.338 | 0,81 | 0,027361624 |
| miR.558 + miR.609 + miR.603 + miR.220a + miR.519b + miR.130a + miR.29a + miR.369.3p + miR.376a* + miR.494 + miR.518c + miR.660 | 0,81 | 0,15418724 |
| miR.558 + miR.609 + miR.603 + miR.520f + miR.220a + miR.519b + miR.29a + miR.369.3p + miR.376a* + miR.494 + miR.518c + miR.660 | 0,81 | 0,083490286 |
| miR.558 + miR.609 + miR.603 + miR.220a + miR.130a + miR.26a + miR.29a + miR.369.3p + miR.376a* + miR.518c + miR.660 + miR.338 | 0,81 | 0,06569385 |
| miR.558 + miR.609 + miR.520f + miR.220a + miR.130a + miR.29a + miR.362 + miR.369.3p + miR.376a* + miR.518c + miR.660 + miR.338 | 0,81 | 0,201067691 |
| miR.558 + miR.609 + miR.603 + miR.220a + miR.130a + miR.26a + miR.29a + miR.369.3p + miR.376a* + miR.494 + miR.518c + miR.660 | 0,81 | 0,078405114 |
| miR.558 + miR.609 + miR.603 + miR.520f + miR.220a + miR.26a + miR.29a + miR.369.3p + miR.376a* + miR.494 + miR.518c + miR.660 | 0,81 | 0,061031771 |
| miR.558 + miR.609 + miR.520f + miR.220a + miR.519b + miR.29a + miR.362 + miR.369.3p + miR.376a* + miR.518c + miR.660 + miR.338 | 0,81 | 0,215506409 |
| miR.558 + miR.609 + miR.603 + miR.520f + miR.220a + miR.130a + miR.29a + miR.369.3p + miR.376a* + miR.518c + miR.660 + miR.338 | 0,81 | 0,032147086 |
| miR.558 + miR.609 + miR.520f + miR.220a + miR.26a + miR.29a + miR.362 + miR.369.3p + miR.376a* + miR.518c + miR.660 + miR.338 | 0,81 | 0,210865186 |
| miR.558 + miR.609 + miR.603 + miR.220a + miR.519b + miR.26a + miR.29a + miR.362 + miR.369.3p + miR.376a* + miR.518c + miR.660 | 0,81 | 0,182910244 |
| miR.558 + miR.609 + miR.603 + miR.220a + miR.130a + miR.26a + miR.29a + miR.369.3p + miR.376a* + miR.518c + miR.639 + miR.660 | 0,81 | 0,114604304 |
| miR.558 + miR.609 + miR.130a + miR.29a + miR.362 + miR.369.3p + miR.376a* + miR.494 + miR.518c + miR.639 + miR.660 + miR.338 | 0,81 | 0,146794034 |
| miR.558 + miR.609 + miR.603 + miR.520f + miR.220a + miR.130a + miR.26a + miR.29a + miR.369.3p + miR.376a* + miR.518c + miR.660 | 0,81 | 0,076886713 |
| miR.558 + miR.609 + miR.603 + miR.520f + miR.220a + miR.29a + miR.369.3p + miR.376a* + miR.494 + miR.518c + miR.639 + miR.660 | 0,81 | 0,015969629 |
| miR.558 + miR.609 + miR.603 + miR.220a + miR.519b + miR.26a + miR.29a + miR.369.3p + miR.376a* + miR.494 + miR.518c + miR.338 | 0,81 | 0,09854142 |
| miR.558 + miR.609 + miR.603 + miR.220a + miR.519b + miR.26a + miR.29a + miR.369.3p + miR.376a* + miR.518c + miR.660 + miR.338 | 0,81 | 0,079364664 |
| miR.558 + miR.609 + miR.603 + miR.520f + miR.220a + miR.29a + miR.362 + miR.369.3p + miR.376a* + miR.518c + miR.639 + miR.660 | 0,81 | 0,034275923 |
| miR.558 + miR.609 + miR.603 + miR.220a + miR.29a + miR.362 + miR.369.3p + miR.376a* + miR.518c + miR.639 + miR.660 + miR.338 | 0,81 | 0,202746634 |
| miR.558 + miR.609 + miR.603 + miR.26a + miR.29a + miR.362 + miR.369.3p + miR.376a* + miR.518c + miR.639 + miR.660 + miR.338 | 0,81 | 0,155445223 |
| miR.558 + miR.609 + miR.603 + miR.520f + miR.220a + miR.26a + miR.29a + miR.362 + miR.369.3p + miR.376a* + miR.518c + miR.660 | 0,81 | 0,101826296 |
| miR.558 + miR.609 + miR.520f + miR.130a + miR.29a + miR.362 + miR.369.3p + miR.376a* + miR.494 + miR.518c + miR.660 + miR.338 | 0,81 | 0,268917868 |
| miR.558 + miR.609 + miR.520f + miR.26a + miR.29a + miR.362 + miR.369.3p + miR.376a* + miR.494 + miR.518c + miR.660 + miR.338 | 0,81 | 0,326230383 |
| miR.558 + miR.609 + miR.603 + miR.220a + miR.130a + miR.26a + miR.29a + miR.362 + miR.369.3p + miR.376a* + miR.518c + miR.660 | 0,81 | 0,112631263 |
| miR.558 + miR.609 + miR.603 + miR.220a + miR.519b + miR.29a + miR.362 + miR.369.3p + miR.376a* + miR.494 + miR.518c + miR.660 | 0,81 | 0,108348697 |
| miR.558 + miR.609 + miR.603 + miR.220a + miR.519b + miR.26a + miR.29a + miR.362 + miR.369.3p + miR.376a* + miR.494 + miR.518c | 0,81 | 0,108883642 |
| miR.558 + miR.609 + miR.603 + miR.220a + miR.130a + miR.29a + miR.362 + miR.369.3p + miR.376a* + miR.494 + miR.518c + miR.660 | 0,81 | 0,062616733 |
| miR.558 + miR.609 + miR.603 + miR.520f + miR.220a + miR.29a + miR.362 + miR.369.3p + miR.376a* + miR.494 + miR.518c + miR.660 | 0,81 | 0,029376529 |
| miR.558 + miR.609 + miR.603 + miR.220a + miR.519b + miR.130a + miR.26a + miR.29a + miR.369.3p + miR.376a* + miR.494 + miR.518c | 0,81 | 0,109712201 |
| miR.558 + miR.609 + miR.520f + miR.220a + miR.29a + miR.369.3p + miR.376a* + miR.494 + miR.518c + miR.639 + miR.660 + miR.338 | 0,81 | 0,057529773 |
| miR.558 + miR.609 + miR.603 + miR.220a + miR.519b + miR.26a + miR.29a + miR.369.3p + miR.376a* + miR.518c + miR.639 + miR.660 | 0,81 | 0,156489391 |
| miR.558 + miR.609 + miR.603 + miR.220a + miR.519b + miR.29a + miR.369.3p + miR.376a* + miR.494 + miR.518c + miR.660 + miR.338 | 0,81 | 0,129743304 |
| miR.558 + miR.609 + miR.603 + miR.220a + miR.26a + miR.29a + miR.362 + miR.369.3p + miR.376a* + miR.518c + miR.639 + miR.338 | 0,81 | 0,055914072 |
| miR.558 + miR.609 + miR.603 + miR.520f + miR.29a + miR.369.3p + miR.376a* + miR.494 + miR.518c + miR.639 + miR.660 + miR.338 | 0,81 | 0,015640054 |
| miR.558 + miR.609 + miR.603 + miR.520f + miR.220a + miR.519b + miR.26a + miR.29a + miR.369.3p + miR.376a* + miR.518c + miR.660 | 0,81 | 0,126250646 |
| miR.558 + miR.609 + miR.603 + miR.220a + miR.26a + miR.29a + miR.369.3p + miR.376a* + miR.494 + miR.518c + miR.639 + miR.338 | 0,81 | 0,0552 7903 |
| miR.558 + miR.609 + miR.220a + miR.26a + miR.29a + miR.362 + miR.369.3p + miR.376a* + miR.518c + miR.639 + miR.660 + miR.338 | 0,81 | 0,371855165 |
| miR.558 + miR.609 + miR.603 + miR.520f + miR.220a + miR.26a + miR.29a + miR.369.3p + miR.376a* + miR.518c + miR.639 + miR.338 | 0,81 | 0,038508599 |
| miR.558 + miR.609 + miR.603 + miR.220a + miR.26a + miR.29a + miR.362 + miR.369.3p + miR.376a* + miR.494 + miR.518c + miR.338 | 0,81 | 0,039809222 |
| miR.558 + miR.609 + miR.603 + miR.520f + miR.220a + miR.519b + miR.29a + miR.369.3p + miR.376a* + miR.518c + miR.639 + miR.660 | 0,81 | 0,100539927 |
| miR.558 + miR.609 + miR.603 + miR.520f + miR.220a + miR.130a + miR.29a + miR.369.3p + miR.376a* + miR.518c + miR.639 + miR.660 | 0,81 | 0,078439518 |
| miR.558 + miR.609 + miR.603 + miR.220a + miR.29a + miR.362 + miR.369.3p + miR.376a* + miR.494 + miR.518c + miR.639 + miR.660 | 0,81 | 0,103874716 |
| miR.558 + miR.609 + miR.603 + miR.220a + miR.519b + miR.130a + miR.26a + miR.29a + miR.369.3p + miR.376a* + miR.518c + miR.660 | 0,81 | 0,139958843 |
| miR.558 + miR.609 + miR.603 + miR.520f + miR.220a + miR.26a + miR.29a + miR.362 + miR.369.3p + miR.376a* + miR.518c + miR.338 | 0,81 | 0,035585025 |
| miR.558 + miR.609 + miR.603 + miR.220a + miR.519b + miR.29a + miR.369.3p + miR.376a* + miR.494 + miR.518c + miR.639 + miR.660 | 0,81 | 0,083709205 |
| miR.558 + miR.609 + miR.603 + miR.520f + miR.130a + miR.29a + miR.362 + miR.369.3p + miR.376a* + miR.518c + miR.660 + miR.338 | 0,81 | 0,178470673 |
| miR.558 + miR.609 + miR.603 + miR.520f + miR.220a + miR.519b + miR.29a + miR.362 + miR.369.3p + miR.376a* + miR.494 + miR.518c | 0,81 | 0,078231414 |
| miR.558 + miR.609 + miR.603 + miR.520f + miR.29a + miR.362 + miR.369.3p + miR.376a* + miR.494 + miR.518c + miR.660 + miR.338 | 0,81 | 0,148540156 |
| miR.558 + miR.609 + miR.603 + miR.220a + miR.130a + miR.26a + miR.29a + miR.369.3p + miR.376a* + miR.518c + miR.639 + miR.338 | 0,81 | 0,052870822 |
| miR.558 + miR.609 + miR.603 + miR.220a + miR.519b + miR.130a + miR.29a + miR.369.3p + miR.376a* + miR.494 + miR.518c + miR.338 | 0,81 | 0,102197194 |
| miR.558 + miR.609 + miR.603 + miR.220a + miR.519b + miR.130a + miR.26a + miR.29a + miR.362 + miR.376a* + miR.494 + miR.518c | 0,81 | 0,083184758 |
| miR.558 + miR.609 + miR.603 + miR.220a + miR.130a + miR.26a + miR.29a + miR.369.3p + miR.376a* + miR.494 + miR.518c + miR.338 | 0,81 | 0,039965868 |
| miR.558 + miR.609 + miR.603 + miR.220a + miR.519b + miR.130a + miR.29a + miR.362 + miR.369.3p + miR.376a* + miR.494 + miR.518c | 0,81 | 0,087852848 |
| miR.558 + miR.609 + miR.603 + miR.520f + miR.220a + miR.130a + miR.29a + miR.362 + miR.369.3p + miR.376a* + miR.518c + miR.660 | 0,81 | 0,048687321 |
| miR.558 + miR.609 + miR.603 + miR.220a + miR.130a + miR.29a + miR.362 + miR.369.3p + miR.376a* + miR.518c + miR.639 + miR.660 | 0,81 | 0,166015604 |
| miR.558 + miR.609 + miR.520f + miR.220a + miR.519b + miR.29a + miR.369.3p + miR.376a* + miR.494 + miR.518c + miR.660 + miR.338 | 0,81 | 0,198580988 |
| miR.558 + miR.609 + miR.603 + miR.520f + miR.220a + miR.26a + miR.29a + miR.369.3p + miR.376a* + miR.494 + miR.518c + miR.338 | 0,81 | 0,030327668 |
| miR.558 + miR.609 + miR.603 + miR.220a + miR.519b + miR.29a + miR.362 + miR.369.3p + miR.376a* + miR.518c + miR.660 + miR.338 | 0,81 | 0,202028658 |
| miR.558 + miR.609 + miR.603 + miR.520f + miR.26a + miR.29a + miR.369.3p + miR.376a* + miR.518c + miR.639 + miR.660 + miR.338 | 0,81 | 0,057304326 |
| miR.558 + miR.609 + miR.603 + miR.520f + miR.220a + miR.130a + miR.29a + miR.369.3p + miR.376a* + miR.494 + miR.518c + miR.660 | 0,81 | 0,026982462 |
| miR.558 + miR.609 + miR.603 + miR.520f + miR.26a + miR.29a + miR.362 + miR.369.3p + miR.376a* + miR.518c + miR.660 + miR.338 | 0,81 | 0,116492472 |
| miR.558 + miR.609 + miR.603 + miR.220a + miR.130a + miR.26a + miR.29a + miR.362 + miR.369.3p + miR.376a* + miR.518c + miR.338 | 0,81 | 0,046219772 |
| miR.558 + miR.609 + miR.603 + miR.220a + miR.519b + miR.26a + miR.29a + miR.369.3p + miR.376a* + miR.494 + miR.518c + miR.639 | 0,81 | 0,104033211 |
| miR.558 + miR.609 + miR.520f + miR.220a + miR.26a + miR.29a + miR.369.3p + miR.376a* + miR.518c + miR.639 + miR.660 + miR.338 | 0,81 | 0,170976258 |
| miR.558 + miR.609 + miR.603 + miR.520f + miR.220a + miR.519b + miR.26a + miR.29a + miR.369.3p + miR.376a* + miR.494 + miR.518c | 0,81 | 0,10934352 |
| miR.558 + miR.609 + miR.603 + miR.220a + miR.29a + miR.362 + miR.369.3p + miR.376a* + miR.494 + miR.518c + miR.660 + miR.338 | 0,81 | 0,127306475 |
| miR.558 + miR.609 + miR.603 + miR.220a + miR.130a + miR.29a + miR.369.3p + miR.376a* + miR.494 + miR.518c + miR.639 + miR.660 | 0,81 | 0,059034471 |
| miR.558 + miR.609 + miR.603 + miR.520f + miR.220a + miR.519b + miR.29a + miR.362 + miR.369.3p + miR.376a* + miR.518c + miR.660 | 0,81 | 0,08903709 |
| miR.558 + miR.609 + miR.603 + miR.520f + miR.519b + miR.29a + miR.369.3p + miR.376a* + miR.518c + miR.639 + miR.660 + miR.338 | 0,81 | 0,031648071 |
| miR.558 + miR.609 + miR.603 + miR.220a + miR.130a + miR.29a + miR.362 + miR.369.3p + miR.376a* + miR.518c + miR.660 + miR.338 | 0,81 | 0,199677494 |
| miR.558 + miR.609 + miR.603 + miR.220a + miR.130a + miR.29a + miR.369.3p + miR.376a* + miR.518c + miR.639 + miR.660 + miR.338 | 0,81 | 0,138829141 |
| miR.558 + miR.609 + miR.520f + miR.220a + miR.519b + miR.29a + miR.369.3p + miR.376a* + miR.518c + miR.639 + miR.660 + miR.338 | 0,81 | 0,117717175 |
| miR.558 + miR.609 + miR.603 + miR.220a + miR.26a + miR.29a + miR.362 + miR.369.3p + miR.376a* + miR.494 + miR.518c + miR.639 | 0,81 | 0,039564709 |
| miR.558 + miR.609 + miR.603 + miR.520f + miR.220a + miR.130a + miR.26a + miR.29a + miR.369.3p + miR.376a* + miR.518c + miR.338 | 0,81 | 0,036582714 |
| miR.558 + miR.609 + miR.520f + miR.130a + miR.29a + miR.369.3p + miR.376a* + miR.494 + miR.518c + miR.639 + miR.660 + miR.338 | 0,81 | 0,154022559 |
| miR.558 + miR.609 + miR.603 + miR.520f + miR.220a + miR.519b + miR.130a + miR.29a + miR.369.3p + miR.376a* + miR.518c + miR.660 | 0,81 | 0,104834008 |
| miR.558 + miR.609 + miR.520f + miR.519b + miR.26a + miR.29a + miR.362 + miR.369.3p + miR.376a* + miR.518c + miR.660 + miR.338 | 0,81 | 0,357884375 |
| miR.558 + miR.609 + miR.603 + miR.520f + miR.220a + miR.519b + miR.130a + miR.29a + miR.369.3p + miR.376a* + miR.494 + miR.518c | 0,81 | 0,096898134 |
| miR.558 + miR.609 + miR.220a + miR.26a + miR.29a + miR.362 + miR.369.3p + miR.376a* + miR.494 + miR.518c + miR.660 + miR.338 | 0,81 | 0,220933014 |
| miR.558 + miR.609 + miR.603 + miR.520f + miR.519b + miR.29a + miR.362 + miR.369.3p + miR.376a* + miR.518c + miR.660 + miR.338 | 0,81 | 0,125736984 |
| miR.558 + miR.609 + miR.603 + miR.220a + miR.519b + miR.29a + miR.362 + miR.369.3p + miR.376a* + miR.518c + miR.639 + miR.660 | 0,81 | 0,106875564 |
| miR.558 + miR.609 + miR.603 + miR.130a + miR.29a + miR.362 + miR.369.3p + miR.376a* + miR.518c + miR.639 + miR.660 + miR.338 | 0,81 | 0,133554228 |
| miR.558 + miR.609 + miR.603 + miR.220a + miR.130a + miR.26a + miR.29a + miR.362 + miR.369.3p + miR.376a* + miR.518c + miR.639 | 0,81 | 0,039682886 |
| miR.558 + miR.609 + miR.603 + miR.220a + miR.130a + miR.26a + miR.29a + miR.362 + miR.369.3p + miR.376a* + miR.494 + miR.518c | 0,81 | 0,041285397 |
| miR.558 + miR.609 + miR.603 + miR.220a + miR.519b + miR.130a + miR.29a + miR.369.3p + miR.376a* + miR.494 + miR.518c + miR.639 | 0,81 | 0,084614591 |
| miR.558 + miR.609 + miR.603 + miR.520f + miR.220a + miR.26a + miR.29a + miR.362 + miR.369.3p + miR.376a* + miR.518c + miR.639 | 0,81 | 0,0293337 |
| miR.558 + miR.609 + miR.603 + miR.220a + miR.519b + miR.130a + miR.29a + miR.369.3p + miR.376a* + miR.518c + miR.639 + miR.660 | 0,81 | 0,087459624 |
| miR.558 + miR.609 + miR.603 + miR.520f + miR.130a + miR.29a + miR.369.3p + miR.376a* + miR.518c + miR.639 + miR.660 + miR.338 | 0,81 | 0,021747995 |
| miR.558 + miR.609 + miR.603 + miR.520f + miR.220a + miR.519b + miR.26a + miR.29a + miR.362 + miR.369.3p + miR.376a* + miR.518c | 0,81 | 0,094564237 |
| miR.558 + miR.609 + miR.603 + miR.520f + miR.220a + miR.26a + miR.29a + miR.362 + miR.369.3p + miR.376a* + miR.494 + miR.518c | 0,81 | 0,037494818 |
| miR.558 + miR.609 + miR.520f + miR.220a + miR.130a + miR.29a + miR.369.3p + miR.376a* + miR.518c + miR.639 + miR.660 + miR.338 | 0,81 | 0,068668665 |
| miR.558 + miR.609 + miR.603 + miR.220a + miR.130a + miR.26a + miR.29a + miR.369.3p + miR.376a* + miR.494 + miR.518c + miR.639 | 0,81 | 0,041958545 |
| miR.558 + miR.609 + miR.603 + miR.220a + miR.519b + miR.26a + miR.29a + miR.362 + miR.369.3p + miR.376a* + miR.518c + miR.639 | 0,81 | 0,086356836 |
| miR.558 + miR.609 + miR.603 + miR.520f + miR.220a + miR.519b + miR.26a + miR.29a + miR.369.3p + miR.376a* + miR.518c + miR.338 | 0,81 | 0,082559044 |
| miR.558 + miR.609 + miR.603 + miR.520f + miR.220a + miR.519b + miR.29a + miR.362 + miR.369.3p + miR.376a* + miR.518c + miR.338 | 0,81 | 0,039798152 |
| miR.558 + miR.609 + miR.603 + miR.220a + miR.519b + miR.130a + miR.29a + miR.362 + miR.376a* + miR.494 + miR.518c + miR.639 | 0,81 | 0,104217685 |
| miR.558 + miR.609 + miR.520f + miR.220a + miR.26a + miR.29a + miR.369.3p + miR.376a* + miR.494 + miR.518c + miR.660 + miR.338 | 0,81 | 0,069197434 |
| miR.558 + miR.609 + miR.220a + miR.130a + miR.29a + miR.362 + miR.369.3p + miR.376a* + miR.518c + miR.639 + miR.660 + miR.338 | 0,81 | 0,258902111 |
| miR.558 + miR.609 + miR.603 + miR.520f + miR.519b + miR.29a + miR.369.3p + miR.376a* + miR.494 + miR.518c + miR.660 + miR.338 | 0,81 | 0,065111946 |
| miR.558 + miR.609 + miR.603 + miR.520f + miR.220a + miR.130a + miR.26a + miR.29a + miR.362 + miR.369.3p + miR.376a* + miR.518c | 0,81 | 0,045420644 |
| miR.558 + miR.609 + miR.603 + miR.220a + miR.519b + miR.130a + miR.29a + miR.362 + miR.376a* + miR.494 + miR.518c + miR.338 | 0,81 | 0,066336168 |
| miR.558 + miR.609 + miR.603 + miR.520f + miR.220a + miR.519b + miR.29a + miR.369.3p + miR.376a* + miR.494 + miR.518c + miR.338 | 0,81 | 0,053178985 |
| miR.558 + miR.609 + miR.603 + miR.520f + miR.220a + miR.519b + miR.29a + miR.376a* + miR.494 + miR.518c + miR.660 + miR.338 | 0,81 | 0,01952814 |
| miR.558 + miR.609 + miR.603 + miR.26a + miR.29a + miR.362 + miR.369.3p + miR.376a* + miR.494 + miR.518c + miR.660 + miR.338 | 0,81 | 0,129352779 |
| miR.558 + miR.609 + miR.603 + miR.220a + miR.29a + miR.369.3p + miR.376a* + miR.494 + miR.518c + miR.639 + miR.660 + miR.338 | 0,81 | 0,082167223 |
| miR.558 + miR.609 + miR.603 + miR.220a + miR.130a + miR.29a + miR.369.3p + miR.376a* + miR.494 + miR.518c + miR.660 + miR.338 | 0,81 | 0,063721406 |
| miR.558 + miR.609 + miR.603 + miR.220a + miR.519b + miR.130a + miR.26a + miR.29a + miR.362 + miR.369.3p + miR.376a* + miR.518c | 0,81 | 0,090634553 |
| miR.558 + miR.609 + miR.520f + miR.519b + miR.29a + miR.362 + miR.369.3p + miR.376a* + miR.494 + miR.518c + miR.660 + miR.338 | 0,81 | 0,355684696 |
| miR.558 + miR.609 + miR.603 + miR.220a + miR.519b + miR.130a + miR.29a + miR.369.3p + miR.376a* + miR.518c + miR.660 + miR.338 | 0,81 | 0,0740 9765 |
| miR.558 + miR.609 + miR.603 + miR.220a + miR.519b + miR.26a + miR.29a + miR.362 + miR.369.3p + miR.376a* + miR.518c + miR.338 | 0,81 | 0,076790974 |
| miR.558 + miR.609 + miR.603 + miR.220a + miR.519b + miR.130a + miR.29a + miR.362 + miR.369.3p + miR.376a* + miR.518c + miR.660 | 0,81 | 0,116971097 |
| miR.558 + miR.609 + miR.603 + miR.520f + miR.220a + miR.26a + miR.29a + miR.369.3p + miR.376a* + miR.494 + miR.518c + miR.639 | 0,81 | 0,04606939 |
| miR.558 + miR.609 + miR.603 + miR.520f + miR.220a + miR.519b + miR.130a + miR.29a + miR.362 + miR.376a* + miR.494 + miR.518c | 0,81 | 0,050118311 |
| miR.558 + miR.609 + miR.603 + miR.520f + miR.220a + miR.29a + miR.362 + miR.369.3p + miR.376a* + miR.494 + miR.518c + miR.338 | 0,81 | 0,019786653 |
| miR.558 + miR.609 + miR.603 + miR.520f + miR.220a + miR.519b + miR.29a + miR.362 + miR.376a* + miR.494 + miR.518c + miR.338 | 0,81 | 0,02708335 |
| miR.558 + miR.609 + miR.603 + miR.520f + miR.220a + miR.519b + miR.29a + miR.369.3p + miR.376a* + miR.494 + miR.518c + miR.639 | 0,81 | 0,071022896 |
| miR.558 + miR.609 + miR.520f + miR.220a + miR.130a + miR.29a + miR.369.3p + miR.376a* + miR.494 + miR.518c + miR.660 + miR.338 | 0,81 | 0,053347364 |
| miR.558 + miR.609 + miR.603 + miR.520f + miR.220a + miR.130a + miR.26a + miR.29a + miR.369.3p + miR.376a* + miR.518c + miR.639 | 0,81 | 0,045323492 |
| miR.558 + miR.609 + miR.603 + miR.220a + miR.519b + miR.130a + miR.26a + miR.29a + miR.376a* + miR.494 + miR.518c + miR.660 | 0,81 | 0,080182822 |
| miR.558 + miR.609 + miR.130a + miR.26a + miR.29a + miR.362 + miR.369.3p + miR.376a* + miR.518c + miR.639 + miR.660 + miR.338 | 0,81 | 0,283076839 |
| miR.558 + miR.609 + miR.603 + miR.220a + miR.519b + miR.26a + miR.29a + miR.369.3p + miR.376a* + miR.518c + miR.639 + miR.338 | 0,81 | 0,082612745 |
| miR.558 + miR.609 + miR.603 + miR.520f + miR.26a + miR.29a + miR.369.3p + miR.376a* + miR.494 + miR.518c + miR.639 + miR.338 | 0,81 | 0,051257339 |
| miR.558 + miR.609 + miR.603 + miR.520f + miR.220a + miR.130a + miR.26a + miR.29a + miR.369.3p + miR.376a* + miR.494 + miR.518c | 0,81 | 0,041750078 |
| miR.558 + miR.609 + miR.519b + miR.26a + miR.29a + miR.362 + miR.369.3p + miR.376a* + miR.518c + miR.639 + miR.660 + miR.338 | 0,81 | 0,209219184 |
| miR.558 + miR.609 + miR.603 + miR.220a + miR.519b + miR.29a + miR.362 + miR.369.3p + miR.376a* + miR.494 + miR.518c + miR.639 | 0,81 | 0,054616283 |
| miR.558 + miR.609 + miR.603 + miR.220a + miR.519b + miR.130a + miR.26a + miR.29a + miR.369.3p + miR.376a* + miR.518c + miR.338 | 0,81 | 0,077981211 |
| miR.558 + miR.609 + miR.520f + miR.220a + miR.130a + miR.29a + miR.369.3p + miR.376a* + miR.494 + miR.518c + miR.639 + miR.660 | 0,81 | 0,027596958 |
| miR.558 + miR.609 + miR.520f + miR.220a + miR.130a + miR.26a + miR.29a + miR.369.3p + miR.376a* + miR.518c + miR.660 + miR.338 | 0,81 | 0,09511014 |
| miR.558 + miR.609 + miR.220a + miR.130a + miR.29a + miR.362 + miR.369.3p + miR.376a* + miR.494 + miR.518c + miR.639 + miR.660 | 0,81 | 0,117596861 |
| miR.558 + miR.609 + miR.520f + miR.220a + miR.29a + miR.362 + miR.369.3p + miR.376a* + miR.494 + miR.518c + miR.639 + miR.660 | 0,81 | 0,00942296 |
| miR.558 + miR.609 + miR.603 + miR.220a + miR.519b + miR.130a + miR.26a + miR.29a + miR.376a* + miR.494 + miR.518c + miR.338 | 0,81 | 0,044567153 |
| miR.558 + miR.609 + miR.603 + miR.220a + miR.519b + miR.29a + miR.369.3p + miR.376a* + miR.518c + miR.639 + miR.660 + miR.338 | 0,81 | 0,059754431 |
| miR.558 + miR.609 + miR.220a + miR.26a + miR.29a + miR.369.3p + miR.376a* + miR.494 + miR.518c + miR.639 + miR.660 + miR.338 | 0,81 | 0,177500681 |
| miR.558 + miR.609 + miR.520f + miR.130a + miR.26a + miR.29a + miR.369.3p + miR.376a* + miR.518c + miR.639 + miR.660 + miR.338 | 0,81 | 0,20128664 |
| miR.558 + miR.609 + miR.603 + miR.220a + miR.519b + miR.26a + miR.29a + miR.362 + miR.376a* + miR.494 + miR.518c + miR.338 | 0,81 | 0,04197064 |
| miR.558 + miR.609 + miR.519b + miR.130a + miR.29a + miR.362 + miR.369.3p + miR.376a* + miR.518c + miR.639 + miR.660 + miR.338 | 0,81 | 0,106046306 |
| miR.558 + miR.609 + miR.220a + miR.130a + miR.29a + miR.362 + miR.369.3p + miR.376a* + miR.494 + miR.518c + miR.660 + miR.338 | 0,81 | 0,139816579 |
| miR.558 + miR.609 + miR.520f + miR.220a + miR.26a + miR.29a + miR.362 + miR.369.3p + miR.376a* + miR.518c + miR.639 + miR.660 | 0,81 | 0,056852964 |
| miR.558 + miR.609 + miR.520f + miR.519b + miR.130a + miR.29a + miR.369.3p + miR.376a* + miR.518c + miR.639 + miR.660 + miR.338 | 0,81 | 0,175255101 |
| miR.558 + miR.609 + miR.603 + miR.520f + miR.220a + miR.519b + miR.130a + miR.29a + miR.369.3p + miR.376a* + miR.518c + miR.338 | 0,81 | 0,050875026 |
| miR.558 + miR.609 + miR.603 + miR.26a + miR.29a + miR.369.3p + miR.376a* + miR.494 + miR.518c + miR.639 + miR.660 + miR.338 | 0,81 | 0,106355626 |
| miR.558 + miR.609 + miR.520f + miR.220a + miR.26a + miR.29a + miR.369.3p + miR.376a* + miR.494 + miR.518c + miR.639 + miR.660 | 0,81 | 0,080751308 |
| miR.558 + miR.609 + miR.603 + miR.520f + miR.220a + miR.519b + miR.130a + miR.29a + miR.362 + miR.369.3p + miR.376a* + miR.518c | 0,81 | 0,06798211 |
| miR.558 + miR.609 + miR.220a + miR.519b + miR.26a + miR.29a + miR.362 + miR.369.3p + miR.376a* + miR.518c + miR.660 + miR.338 | 0,81 | 0,259415946 |
| miR.558 + miR.609 + miR.520f + miR.130a + miR.29a + miR.362 + miR.369.3p + miR.376a* + miR.494 + miR.518c + miR.639 + miR.660 | 0,81 | 0,039528118 |
| miR.558 + miR.609 + miR.520f + miR.220a + miR.519b + miR.26a + miR.29a + miR.369.3p + miR.376a* + miR.518c + miR.660 + miR.338 | 0,81 | 0,186351378 |
| miR.558 + miR.609 + miR.603 + miR.520f + miR.220a + miR.519b + miR.26a + miR.29a + miR.362 + miR.376a* + miR.494 + miR.518c | 0,81 | 0,049974354 |
| miR.558 + miR.609 + miR.603 + miR.220a + miR.519b + miR.130a + miR.26a + miR.29a + miR.369.3p + miR.376a* + miR.518c + miR.639 | 0,81 | 0,092144157 |
| miR.558 + miR.609 + miR.603 + miR.220a + miR.519b + miR.130a + miR.26a + miR.29a + miR.376a* + miR.494 + miR.518c + miR.639 | 0,81 | 0,045242646 |
| miR.558 + miR.609 + miR.520f + miR.220a + miR.26a + miR.29a + miR.362 + miR.369.3p + miR.376a* + miR.494 + miR.518c + miR.660 | 0,81 | 0,08969417 |
| miR.558 + miR.609 + miR.130a + miR.26a + miR.29a + miR.362 + miR.369.3p + miR.376a* + miR.494 + miR.518c + miR.639 + miR.660 | 0,81 | 0,060981205 |
| miR.558 + miR.609 + miR.603 + miR.220a + miR.519b + miR.130a + miR.29a + miR.362 + miR.376a* + miR.494 + miR.518c + miR.660 | 0,81 | 0,080916323 |
| miR.558 + miR.609 + miR.520f + miR.220a + miR.519b + miR.130a + miR.29a + miR.369.3p + miR.376a* + miR.518c + miR.660 + miR.338 | 0,81 | 0,14154825 |
| miR.558 + miR.609 + miR.603 + miR.220a + miR.519b + miR.130a + miR.29a + miR.362 + miR.369.3p + miR.376a* + miR.518c + miR.338 | 0,81 | 0,03597143 |
| miR.558 + miR.609 + miR.603 + miR.520f + miR.220a + miR.519b + miR.29a + miR.362 + miR.376a* + miR.494 + miR.518c + miR.639 | 0,81 | 0,040098116 |
| miR.558 + miR.609 + miR.603 + miR.520f + miR.220a + miR.29a + miR.376a* + miR.494 + miR.518c + miR.639 + miR.660 + miR.338 | 0,81 | 0,005981727 |
| miR.558 + miR.609 + miR.603 + miR.520f + miR.220a + miR.519b + miR.29a + miR.362 + miR.369.3p + miR.376a* + miR.518c + miR.639 | 0,81 | 0,057462334 |
| miR.558 + miR.609 + miR.220a + miR.26a + miR.29a + miR.362 + miR.369.3p + miR.376a* + miR.494 + miR.518c + miR.639 + miR.660 | 0,81 | 0,105476242 |
| miR.558 + miR.609 + miR.603 + miR.220a + miR.519b + miR.130a + miR.29a + miR.362 + miR.369.3p + miR.376a* + miR.518c + miR.639 | 0,81 | 0,053864735 |
| miR.558 + miR.609 + miR.603 + miR.220a + miR.519b + miR.130a + miR.29a + miR.376a* + miR.494 + miR.518c + miR.660 + miR.338 | 0,81 | 0,054775303 |
| miR.558 + miR.609 + miR.603 + miR.520f + miR.220a + miR.130a + miR.29a + miR.362 + miR.369.3p + miR.376a* + miR.518c + miR.639 | 0,81 | 0,022055344 |
| miR.558 + miR.609 + miR.220a + miR.130a + miR.26a + miR.29a + miR.362 + miR.369.3p + miR.376a* + miR.518c + miR.660 + miR.338 | 0,81 | 0,334796332 |
| miR.558 + miR.609 + miR.220a + miR.130a + miR.26a + miR.29a + miR.369.3p + miR.376a* + miR.518c + miR.639 + miR.660 + miR.338 | 0,81 | 0,17540391 |
| miR.558 + miR.609 + miR.603 + miR.130a + miR.29a + miR.362 + miR.369.3p + miR.376a* + miR.494 + miR.518c + miR.639 + miR.660 | 0,81 | 0,08334602 |
| miR.558 + miR.609 + miR.603 + miR.520f + miR.220a + miR.29a + miR.362 + miR.369.3p + miR.376a* + miR.518c + miR.639 + miR.338 | 0,81 | 0,015001384 |
| miR.558 + miR.609 + miR.520f + miR.519b + miR.29a + miR.369.3p + miR.376a* + miR.494 + miR.518c + miR.639 + miR.660 + miR.338 | 0,81 | 0,123206228 |
| miR.558 + miR.609 + miR.603 + miR.520f + miR.220a + miR.130a + miR.29a + miR.362 + miR.369.3p + miR.376a* + miR.494 + miR.518c | 0,81 | 0,022472817 |
| miR.558 + miR.609 + miR.603 + miR.220a + miR.519b + miR.130a + miR.29a + miR.376a* + miR.494 + miR.518c + miR.639 + miR.660 | 0,81 | 0,049741064 |
| miR.558 + miR.609 + miR.603 + miR.520f + miR.26a + miR.29a + miR.362 + miR.369.3p + miR.376a* + miR.518c + miR.639 + miR.338 | 0,81 | 0,063997541 |
| miR.558 + miR.609 + miR.520f + miR.220a + miR.519b + miR.29a + miR.362 + miR.369.3p + miR.376a* + miR.494 + miR.518c + miR.660 | 0,81 | 0,102740231 |
| miR.558 + miR.609 + miR.520f + miR.130a + miR.26a + miR.29a + miR.362 + miR.369.3p + miR.376a* + miR.518c + miR.660 + miR.338 | 0,81 | 0,389791642 |
| miR.558 + miR.609 + miR.603 + miR.520f + miR.130a + miR.26a + miR.29a + miR.369.3p + miR.376a* + miR.518c + miR.639 + miR.338 | 0,81 | 0,060724377 |
| miR.558 + miR.609 + miR.603 + miR.130a + miR.26a + miR.29a + miR.369.3p + miR.376a* + miR.494 + miR.518c + miR.639 + miR.338 | 0,81 | 0,129006594 |
| miR.558 + miR.609 + miR.520f + miR.519b + miR.130a + miR.29a + miR.362 + miR.369.3p + miR.376a* + miR.518c + miR.660 + miR.338 | 0,81 | 0,358017579 |
| miR.558 + miR.609 + miR.520f + miR.26a + miR.29a + miR.369.3p + miR.376a* + miR.494 + miR.518c + miR.639 + miR.660 + miR.338 | 0,81 | 0,159221004 |
| miR.558 + miR.609 + miR.603 + miR.520f + miR.220a + miR.519b + miR.130a + miR.29a + miR.376a* + miR.494 + miR.518c + miR.660 | 0,81 | 0,054894571 |
| miR.558 + miR.609 + miR.520f + miR.519b + miR.130a + miR.29a + miR.369.3p + miR.376a* + miR.494 + miR.518c + miR.660 + miR.338 | 0,81 | 0,210899702 |
| miR.558 + miR.609 + miR.220a + miR.130a + miR.26a + miR.29a + miR.369.3p + miR.376a* + miR.494 + miR.518c + miR.660 + miR.338 | 0,81 | 0,106786642 |
| miR.558 + miR.609 + miR.603 + miR.520f + miR.130a + miR.29a + miR.369.3p + miR.376a* + miR.494 + miR.518c + miR.639 + miR.338 | 0,81 | 0,021848275 |
| miR.558 + miR.609 + miR.603 + miR.130a + miR.26a + miR.29a + miR.369.3p + miR.376a* + miR.518c + miR.639 + miR.660 + miR.338 | 0,81 | 0,163430507 |
| miR.558 + miR.609 + miR.220a + miR.130a + miR.26a + miR.29a + miR.369.3p + miR.376a* + miR.494 + miR.518c + miR.639 + miR.660 | 0,81 | 0,136259581 |
| miR.558 + miR.609 + miR.603 + miR.520f + miR.220a + miR.519b + miR.26a + miR.29a + miR.369.3p + miR.376a* + miR.518c + miR.639 | 0,81 | 0,088088935 |
| miR.558 + miR.609 + miR.603 + miR.520f + miR.130a + miR.29a + miR.369.3p + miR.376a* + miR.494 + miR.518c + miR.660 + miR.338 | 0,81 | 0,0240627 |
| miR.558 + miR.609 + miR.603 + miR.520f + miR.26a + miR.29a + miR.369.3p + miR.376a* + miR.494 + miR.518c + miR.660 + miR.338 | 0,81 | 0,034936273 |
| miR.558 + miR.609 + miR.603 + miR.220a + miR.519b + miR.130a + miR.29a + miR.369.3p + miR.376a* + miR.518c + miR.639 + miR.338 | 0,81 | 0,037656318 |
| miR.558 + miR.609 + miR.520f + miR.220a + miR.519b + miR.26a + miR.29a + miR.369.3p + miR.376a* + miR.494 + miR.518c + miR.660 | 0,81 | 0,172233835 |
| miR.558 + miR.609 + miR.603 + miR.220a + miR.26a + miR.29a + miR.376a* + miR.494 + miR.518c + miR.639 + miR.660 + miR.338 | 0,81 | 0,022139774 |
| miR.558 + miR.609 + miR.520f + miR.220a + miR.519b + miR.29a + miR.362 + miR.369.3p + miR.376a* + miR.518c + miR.639 + miR.660 | 0,81 | 0,02836117 |
| miR.558 + miR.609 + miR.26a + miR.29a + miR.362 + miR.369.3p + miR.376a* + miR.494 + miR.518c + miR.639 + miR.660 + miR.338 | 0,81 | 0,153774809 |
| miR.558 + miR.609 + miR.603 + miR.520f + miR.220a + miR.130a + miR.29a + miR.369.3p + miR.376a* + miR.494 + miR.518c + miR.639 | 0,81 | 0,017899036 |
| miR.558 + miR.609 + miR.603 + miR.520f + miR.220a + miR.519b + miR.130a + miR.26a + miR.29a + miR.369.3p + miR.376a* + miR.518c | 0,81 | 0,096257059 |
| miR.558 + miR.609 + miR.603 + miR.520f + miR.130a + miR.29a + miR.369.3p + miR.376a* + miR.494 + miR.518c + miR.639 + miR.660 | 0,81 | 0,017465393 |
| miR.558 + miR.609 + miR.603 + miR.520f + miR.220a + miR.29a + miR.369.3p + miR.376a* + miR.494 + miR.518c + miR.639 + miR.338 | 0,81 | 0,015137463 |
| miR.558 + miR.609 + miR.603 + miR.520f + miR.220a + miR.519b + miR.29a + miR.362 + miR.376a* + miR.494 + miR.518c + miR.660 | 0,81 | 0,055639673 |
| miR.558 + miR.609 + miR.603 + miR.520f + miR.519b + miR.130a + miR.29a + miR.369.3p + miR.376a* + miR.518c + miR.660 + miR.338 | 0,81 | 0,071805459 |
| miR.558 + miR.609 + miR.603 + miR.520f + miR.220a + miR.519b + miR.29a + miR.376a* + miR.494 + miR.518c + miR.639 + miR.660 | 0,81 | 0,034546593 |
| miR.558 + miR.609 + miR.603 + miR.519b + miR.26a + miR.29a + miR.362 + miR.369.3p + miR.376a* + miR.518c + miR.660 + miR.338 | 0,81 | 0,130017303 |
| miR.558 + miR.609 + miR.520f + miR.220a + miR.130a + miR.26a + miR.29a + miR.369.3p + miR.376a* + miR.494 + miR.518c + miR.660 | 0,81 | 0,087415784 |
| miR.558 + miR.609 + miR.603 + miR.220a + miR.130a + miR.26a + miR.29a + miR.376a* + miR.494 + miR.518c + miR.639 + miR.660 | 0,81 | 0,018542927 |
| miR.558 + miR.609 + miR.603 + miR.520f + miR.519b + miR.130a + miR.29a + miR.369.3p + miR.376a* + miR.494 + miR.518c + miR.338 | 0,81 | 0,067861605 |
| miR.558 + miR.609 + miR.520f + miR.220a + miR.130a + miR.26a + miR.29a + miR.369.3p + miR.376a* + miR.518c + miR.639 + miR.660 | 0,81 | 0,120120739 |
| miR.558 + miR.609 + miR.220a + miR.519b + miR.26a + miR.29a + miR.369.3p + miR.376a* + miR.518c + miR.639 + miR.660 + miR.338 | 0,81 | 0,194489944 |
| miR.558 + miR.609 + miR.603 + miR.220a + miR.519b + miR.29a + miR.362 + miR.369.3p + miR.376a* + miR.518c + miR.639 + miR.338 | 0,81 | 0,033896627 |
| miR.558 + miR.609 + miR.603 + miR.520f + miR.220a + miR.519b + miR.26a + miR.29a + miR.376a* + miR.494 + miR.518c + miR.660 | 0,81 | 0,043033016 |
| miR.558 + miR.609 + miR.603 + miR.130a + miR.26a + miR.29a + miR.362 + miR.369.3p + miR.376a* + miR.518c + miR.660 + miR.338 | 0,81 | 0,12068531 |
| miR.558 + miR.609 + miR.603 + miR.26a + miR.29a + miR.362 + miR.369.3p + miR.376a* + miR.494 + miR.518c + miR.639 + miR.660 | 0,81 | 0,031281313 |
| miR.558 + miR.609 + miR.520f + miR.220a + miR.519b + miR.130a + miR.29a + miR.369.3p + miR.376a* + miR.494 + miR.518c + miR.660 | 0,81 | 0,168682121 |
| miR.558 + miR.609 + miR.603 + miR.520f + miR.130a + miR.29a + miR.362 + miR.369.3p + miR.376a* + miR.518c + miR.639 + miR.338 | 0,81 | 0,025570568 |
| miR.558 + miR.609 + miR.603 + miR.520f + miR.519b + miR.29a + miR.369.3p + miR.376a* + miR.494 + miR.518c + miR.639 + miR.338 | 0,81 | 0,076088828 |
| miR.558 + miR.609 + miR.520f + miR.220a + miR.130a + miR.29a + miR.362 + miR.369.3p + miR.376a* + miR.494 + miR.518c + miR.660 | 0,81 | 0,059145395 |
| miR.558 + miR.609 + miR.220a + miR.130a + miR.26a + miR.29a + miR.362 + miR.369.3p + miR.376a* + miR.494 + miR.518c + miR.660 | 0,81 | 0,162066088 |
| miR.558 + miR.609 + miR.220a + miR.519b + miR.26a + miR.29a + miR.362 + miR.369.3p + miR.376a* + miR.494 + miR.518c + miR.660 | 0,81 | 0,232841234 |
| miR.558 + miR.609 + miR.220a + miR.519b + miR.26a + miR.29a + miR.369.3p + miR.376a* + miR.494 + miR.518c + miR.639 + miR.660 | 0,81 | 0,231961563 |
| miR.558 + miR.609 + miR.220a + miR.519b + miR.130a + miR.26a + miR.29a + miR.369.3p + miR.376a* + miR.494 + miR.518c + miR.660 | 0,81 | 0,169081995 |
| miR.558 + miR.609 + miR.603 + miR.520f + miR.519b + miR.26a + miR.29a + miR.369.3p + miR.376a* + miR.494 + miR.518c + miR.338 | 0,81 | 0,081746985 |
| miR.558 + miR.609 + miR.603 + miR.520f + miR.519b + miR.26a + miR.29a + miR.369.3p + miR.376a* + miR.518c + miR.639 + miR.338 | 0,81 | 0,06810275 |
| miR.558 + miR.609 + miR.603 + miR.520f + miR.130a + miR.26a + miR.29a + miR.369.3p + miR.376a* + miR.518c + miR.660 + miR.338 | 0,81 | 0,046108142 |
| miR.558 + miR.609 + miR.520f + miR.220a + miR.130a + miR.26a + miR.29a + miR.362 + miR.369.3p + miR.376a* + miR.518c + miR.660 | 0,81 | 0,152570284 |
| miR.558 + miR.609 + miR.220a + miR.519b + miR.26a + miR.29a + miR.369.3p + miR.376a* + miR.494 + miR.518c + miR.660 + miR.338 | 0,81 | 0,179884667 |
| miR.558 + miR.609 + miR.520f + miR.220a + miR.519b + miR.26a + miR.29a + miR.362 + miR.369.3p + miR.376a* + miR.518c + miR.660 | 0,81 | 0,193116825 |
| miR.558 + miR.609 + miR.603 + miR.220a + miR.519b + miR.29a + miR.369.3p + miR.376a* + miR.494 + miR.518c + miR.639 + miR.338 | 0,81 | 0,066058661 |
| miR.558 + miR.609 + miR.603 + miR.520f + miR.519b + miR.29a + miR.362 + miR.369.3p + miR.376a* + miR.494 + miR.518c + miR.338 | 0,81 | 0,059933189 |
| miR.558 + miR.609 + miR.603 + miR.220a + miR.519b + miR.26a + miR.29a + miR.376a* + miR.494 + miR.518c + miR.660 + miR.338 | 0,81 | 0,051471896 |
| miR.558 + miR.609 + miR.603 + miR.520f + miR.220a + miR.130a + miR.29a + miR.369.3p + miR.376a* + miR.518c + miR.639 + miR.338 | 0,81 | 0,014365012 |
| miR.558 + miR.609 + miR.603 + miR.220a + miR.519b + miR.26a + miR.29a + miR.362 + miR.376a* + miR.494 + miR.518c + miR.639 | 0,81 | 0,04706569 |
| miR.558 + miR.609 + miR.603 + miR.520f + miR.519b + miR.26a + miR.29a + miR.369.3p + miR.376a* + miR.518c + miR.660 + miR.338 | 0,81 | 0,06573151 |
| miR.558 + miR.609 + miR.520f + miR.220a + miR.519b + miR.29a + miR.369.3p + miR.376a* + miR.494 + miR.518c + miR.639 + miR.660 | 0,81 | 0,119343204 |
| miR.558 + miR.609 + miR.220a + miR.519b + miR.26a + miR.29a + miR.362 + miR.369.3p + miR.376a* + miR.518c + miR.639 + miR.660 | 0,81 | 0,109932735 |
| miR.558 + miR.609 + miR.603 + miR.220a + miR.519b + miR.26a + miR.29a + miR.362 + miR.376a* + miR.494 + miR.518c + miR.660 | 0,81 | 0,053887773 |
| miR.558 + miR.609 + miR.603 + miR.220a + miR.130a + miR.29a + miR.362 + miR.369.3p + miR.376a* + miR.494 + miR.518c + miR.639 | 0,81 | 0,013894687 |
| miR.558 + miR.609 + miR.520f + miR.220a + miR.130a + miR.29a + miR.362 + miR.369.3p + miR.376a* + miR.518c + miR.639 + miR.660 | 0,81 | 0,062249234 |
| miR.558 + miR.609 + miR.603 + miR.520f + miR.220a + miR.130a + miR.29a + miR.362 + miR.369.3p + miR.376a* + miR.518c + miR.338 | 0,81 | 0,022192148 |
| miR.558 + miR.609 + miR.603 + miR.220a + miR.26a + miR.29a + miR.362 + miR.376a* + miR.494 + miR.518c + miR.639 + miR.338 | 0,81 | 0,021787739 |
| miR.558 + miR.609 + miR.603 + miR.520f + miR.519b + miR.29a + miR.362 + miR.369.3p + miR.376a* + miR.518c + miR.639 + miR.338 | 0,81 | 0,058085464 |
| miR.558 + miR.609 + miR.603 + miR.520f + miR.220a + miR.519b + miR.29a + miR.362 + miR.376a* + miR.518c + miR.639 + miR.338 | 0,81 | 0,021492634 |
| miR.558 + miR.609 + miR.220a + miR.519b + miR.130a + miR.29a + miR.362 + miR.369.3p + miR.376a* + miR.494 + miR.518c + miR.660 | 0,81 | 0,166324239 |
| miR.558 + miR.609 + miR.603 + miR.220a + miR.130a + miR.26a + miR.29a + miR.362 + miR.376a* + miR.518c + miR.639 + miR.338 | 0,81 | 0,024702824 |
| miR.558 + miR.609 + miR.603 + miR.29a + miR.362 + miR.369.3p + miR.376a* + miR.494 + miR.518c + miR.639 + miR.660 + miR.338 | 0,81 | 0,047390942 |
| miR.558 + miR.609 + miR.603 + miR.130a + miR.26a + miR.29a + miR.369.3p + miR.376a* + miR.494 + miR.518c + miR.639 + miR.660 | 0,81 | 0,093783858 |
| miR.558 + miR.609 + miR.520f + miR.519b + miR.26a + miR.29a + miR.369.3p + miR.376a* + miR.518c + miR.639 + miR.660 + miR.338 | 0,81 | 0,18947222 |
| miR.558 + miR.609 + miR.603 + miR.520f + miR.29a + miR.362 + miR.369.3p + miR.376a* + miR.494 + miR.518c + miR.639 + miR.660 | 0,81 | 0,002840781 |
| miR.558 + miR.609 + miR.603 + miR.519b + miR.26a + miR.29a + miR.369.3p + miR.376a* + miR.518c + miR.639 + miR.660 + miR.338 | 0,81 | 0,191880906 |
| miR.558 + miR.609 + miR.603 + miR.520f + miR.26a + miR.29a + miR.369.3p + miR.376a* + miR.494 + miR.518c + miR.639 + miR.660 | 0,81 | 0,019834271 |
| miR.558 + miR.609 + miR.603 + miR.520f + miR.26a + miR.29a + miR.362 + miR.369.3p + miR.376a* + miR.518c + miR.639 + miR.660 | 0,81 | 0,006494839 |
| miR.558 + miR.609 + miR.603 + miR.520f + miR.220a + miR.519b + miR.29a + miR.369.3p + miR.376a* + miR.518c + miR.639 + miR.338 | 0,81 | 0,04153001 |
| miR.558 + miR.609 + miR.220a + miR.29a + miR.362 + miR.369.3p + miR.376a* + miR.494 + miR.518c + miR.639 + miR.660 + miR.338 | 0,81 | 0,106905315 |
| miR.558 + miR.609 + miR.603 + miR.220a + miR.130a + miR.26a + miR.29a + miR.362 + miR.376a* + miR.494 + miR.518c + miR.639 | 0,81 | 0,021948764 |
| miR.558 + miR.609 + miR.603 + miR.520f + miR.29a + miR.362 + miR.369.3p + miR.376a* + miR.494 + miR.518c + miR.639 + miR.338 | 0,81 | 0,014711722 |
| miR.558 + miR.609 + miR.603 + miR.520f + miR.220a + miR.519b + miR.26a + miR.29a + miR.362 + miR.376a* + miR.518c + miR.639 | 0,81 | 0,063714707 |
| miR.558 + miR.609 + miR.520f + miR.130a + miR.29a + miR.362 + miR.376a* + miR.494 + miR.518c + miR.639 + miR.660 + miR.338 | 0,81 | 0,017104441 |
| miR.558 + miR.609 + miR.520f + miR.130a + miR.26a + miR.29a + miR.369.3p + miR.376a* + miR.494 + miR.518c + miR.639 + miR.660 | 0,81 | 0,141167705 |
| miR.558 + miR.609 + miR.519b + miR.130a + miR.29a + miR.362 + miR.369.3p + miR.376a* + miR.494 + miR.518c + miR.639 + miR.660 | 0,81 | 0,0742 41908 |
| miR.558 + miR.609 + miR.603 + miR.520f + miR.220a + miR.519b + miR.130a + miR.29a + miR.362 + miR.376a* + miR.518c + miR.338 | 0,81 | 0,025818186 |
| miR.558 + miR.609 + miR.130a + miR.26a + miR.29a + miR.362 + miR.369.3p + miR.376a* + miR.494 + miR.518c + miR.660 + miR.338 | 0,81 | 0,290922579 |
| miR.558 + miR.609 + miR.603 + miR.520f + miR.220a + miR.519b + miR.130a + miR.29a + miR.376a* + miR.494 + miR.518c + miR.338 | 0,81 | 0,030305215 |
| miR.558 + miR.609 + miR.520f + miR.220a + miR.519b + miR.130a + miR.29a + miR.369.3p + miR.376a* + miR.518c + miR.639 + miR.660 | 0,81 | 0,111648116 |
| miR.558 + miR.609 + miR.603 + miR.220a + miR.519b + miR.26a + miR.29a + miR.376a* + miR.494 + miR.518c + miR.639 + miR.660 | 0,81 | 0,042901913 |
| miR.558 + miR.609 + miR.220a + miR.519b + miR.29a + miR.362 + miR.369.3p + miR.376a* + miR.518c + miR.639 + miR.660 + miR.338 | 0,81 | 0,112734324 |
| miR.558 + miR.609 + miR.520f + miR.220a + miR.26a + miR.29a + miR.362 + miR.369.3p + miR.376a* + miR.494 + miR.518c + miR.338 | 0,81 | 0,06594461 |
| miR.558 + miR.609 + miR.520f + miR.220a + miR.519b + miR.26a + miR.29a + miR.369.3p + miR.376a* + miR.518c + miR.639 + miR.660 | 0,81 | 0,120222111 |
| miR.558 + miR.609 + miR.520f + miR.220a + miR.29a + miR.362 + miR.376a* + miR.494 + miR.518c + miR.639 + miR.660 + miR.338 | 0,81 | 0,006393723 |
| miR.558 + miR.609 + miR.520f + miR.220a + miR.130a + miR.29a + miR.376a* + miR.494 + miR.518c + miR.639 + miR.660 + miR.338 | 0,81 | 0,018470788 |
| miR.558 + miR.609 + miR.603 + miR.520f + miR.220a + miR.519b + miR.29a + miR.376a* + miR.494 + miR.518c + miR.639 + miR.338 | 0,81 | 0,019428291 |
| miR.558 + miR.609 + miR.603 + miR.520f + miR.220a + miR.130a + miR.29a + miR.376a* + miR.494 + miR.518c + miR.639 + miR.660 | 0,81 | 0,01301409 |
| miR.558 + miR.609 + miR.603 + miR.520f + miR.220a + miR.26a + miR.29a + miR.362 + miR.376a* + miR.518c + miR.639 + miR.338 | 0,81 | 0,013746926 |
| miR.558 + miR.609 + miR.603 + miR.520f + miR.220a + miR.519b + miR.130a + miR.26a + miR.29a + miR.362 + miR.376a* + miR.518c | 0,81 | 0,074476753 |
| miR.558 + miR.609 + miR.603 + miR.520f + miR.220a + miR.519b + miR.26a + miR.29a + miR.362 + miR.376a* + miR.518c + miR.338 | 0,81 | 0,032386546 |
| miR.558 + miR.609 + miR.603 + miR.520f + miR.220a + miR.519b + miR.26a + miR.29a + miR.376a* + miR.494 + miR.518c + miR.338 | 0,81 | 0,030468916 |
| miR.558 + miR.609 + miR.520f + miR.220a + miR.26a + miR.29a + miR.369.3p + miR.376a* + miR.494 + miR.518c + miR.639 + miR.338 | 0,81 | 0,079528316 |
| miR.558 + miR.609 + miR.603 + miR.520f + miR.220a + miR.519b + miR.130a + miR.29a + miR.362 + miR.376a* + miR.518c + miR.639 | 0,81 | 0,063065268 |
| miR.558 + miR.609 + miR.220a + miR.519b + miR.130a + miR.29a + miR.362 + miR.369.3p + miR.376a* + miR.518c + miR.639 + miR.660 | 0,81 | 0,18351129 |
| miR.609 + miR.603 + miR.520f + miR.29a + miR.362 + miR.369.3p + miR.376a* + miR.494 + miR.518c + miR.639 + miR.660 + miR.338 | 0,81 | 0,265674832 |
| miR.558 + miR.609 + miR.603 + miR.520f + miR.220a + miR.26a + miR.29a + miR.376a* + miR.494 + miR.518c + miR.639 + miR.660 | 0,81 | 0,01544211 |
| miR.558 + miR.609 + miR.603 + miR.519b + miR.130a + miR.26a + miR.29a + miR.369.3p + miR.376a* + miR.518c + miR.639 + miR.338 | 0,81 | 0,124208435 |
| miR.558 + miR.609 + miR.603 + miR.519b + miR.26a + miR.29a + miR.362 + miR.369.3p + miR.376a* + miR.518c + miR.639 + miR.660 | 0,81 | 0,045304156 |
| miR.558 + miR.609 + miR.603 + miR.520f + miR.220a + miR.26a + miR.29a + miR.376a* + miR.494 + miR.518c + miR.660 + miR.338 | 0,81 | 0,026751435 |
| miR.558 + miR.609 + miR.603 + miR.520f + miR.519b + miR.130a + miR.29a + miR.369.3p + miR.376a* + miR.518c + miR.639 + miR.338 | 0,81 | 0,055350253 |
| miR.558 + miR.609 + miR.520f + miR.220a + miR.519b + miR.29a + miR.376a* + miR.494 + miR.518c + miR.639 + miR.660 + miR.338 | 0,81 | 0,082900428 |
| miR.558 + miR.609 + miR.603 + miR.520f + miR.220a + miR.29a + miR.362 + miR.369.3p + miR.376a* + miR.494 + miR.518c + miR.639 | 0,81 | 0,012420108 |
| miR.558 + miR.609 + miR.603 + miR.220a + miR.519b + miR.130a + miR.29a + miR.376a* + miR.494 + miR.518c + miR.639 + miR.338 | 0,81 | 0,028845391 |
| miR.558 + miR.609 + miR.603 + miR.520f + miR.220a + miR.130a + miR.29a + miR.369.3p + miR.376a* + miR.494 + miR.518c + miR.338 | 0,81 | 0,021174594 |
| miR.558 + miR.609 + miR.220a + miR.519b + miR.130a + miR.26a + miR.29a + miR.369.3p + miR.376a* + miR.518c + miR.639 + miR.660 | 0,81 | 0,176861305 |
| miR.558 + miR.609 + miR.603 + miR.519b + miR.130a + miR.29a + miR.369.3p + miR.376a* + miR.494 + miR.518c + miR.639 + miR.338 | 0,81 | 0,124043279 |
| miR.558 + miR.609 + miR.603 + miR.520f + miR.130a + miR.26a + miR.29a + miR.369.3p + miR.376a* + miR.494 + miR.518c + miR.338 | 0,81 | 0,029378212 |
| miR.558 + miR.609 + miR.603 + miR.520f + miR.220a + miR.26a + miR.29a + miR.376a* + miR.518c + miR.639 + miR.660 + miR.338 | 0,81 | 0,011053221 |
| miR.558 + miR.609 + miR.603 + miR.520f + miR.220a + miR.519b + miR.130a + miR.26a + miR.29a + miR.376a* + miR.494 + miR.518c | 0,81 | 0,043274351 |
| miR.558 + miR.609 + miR.519b + miR.130a + miR.26a + miR.29a + miR.362 + miR.369.3p + miR.376a* + miR.518c + miR.639 + miR.660 | 0,81 | 0,074341835 |
| miR.558 + miR.609 + miR.603 + miR.220a + miR.130a + miR.29a + miR.362 + miR.369.3p + miR.376a* + miR.494 + miR.518c + miR.338 | 0,81 | 0,016252215 |
| miR.558 + miR.609 + miR.603 + miR.520f + miR.220a + miR.519b + miR.130a + miR.29a + miR.369.3p + miR.376a* + miR.518c + miR.639 | 0,81 | 0,058328568 |
| miR.558 + miR.609 + miR.520f + miR.220a + miR.26a + miR.29a + miR.362 + miR.369.3p + miR.376a* + miR.518c + miR.639 + miR.338 | 0,81 | 0,071939318 |
| miR.558 + miR.609 + miR.603 + miR.519b + miR.29a + miR.362 + miR.369.3p + miR.376a* + miR.518c + miR.639 + miR.660 + miR.338 | 0,81 | 0,051451185 |
| miR.558 + miR.609 + miR.603 + miR.520f + miR.26a + miR.29a + miR.362 + miR.369.3p + miR.376a* + miR.494 + miR.518c + miR.338 | 0,81 | 0,025888854 |
| miR.558 + miR.609 + miR.603 + miR.520f + miR.220a + miR.519b + miR.26a + miR.29a + miR.362 + miR.376a* + miR.518c + miR.660 | 0,81 | 0,069083284 |
| miR.558 + miR.609 + miR.603 + miR.220a + miR.519b + miR.26a + miR.29a + miR.362 + miR.376a* + miR.518c + miR.639 + miR.338 | 0,81 | 0,042037969 |
| miR.558 + miR.609 + miR.220a + miR.519b + miR.130a + miR.29a + miR.362 + miR.369.3p + miR.376a* + miR.518c + miR.660 + miR.338 | 0,81 | 0,174025913 |
| miR.558 + miR.609 + miR.603 + miR.520f + miR.519b + miR.29a + miR.376a* + miR.494 + miR.518c + miR.639 + miR.660 + miR.338 | 0,81 | 0,015928597 |
| miR.558 + miR.609 + miR.603 + miR.220a + miR.519b + miR.130a + miR.26a + miR.29a + miR.362 + miR.376a* + miR.518c + miR.338 | 0,81 | 0,042037969 |
| miR.558 + miR.609 + miR.603 + miR.130a + miR.29a + miR.369.3p + miR.376a* + miR.494 + miR.518c + miR.639 + miR.660 + miR.338 | 0,81 | 0,080313155 |
| miR.558 + miR.609 + miR.220a + miR.130a + miR.26a + miR.29a + miR.362 + miR.369.3p + miR.376a* + miR.518c + miR.639 + miR.660 | 0,81 | 0,229477513 |
| miR.558 + miR.609 + miR.603 + miR.520f + miR.220a + miR.130a + miR.29a + miR.376a* + miR.518c + miR.639 + miR.660 + miR.338 | 0,81 | 0,008487668 |
| miR.558 + miR.609 + miR.520f + miR.220a + miR.29a + miR.362 + miR.369.3p + miR.376a* + miR.494 + miR.518c + miR.639 + miR.338 | 0,81 | 0,017076293 |
| miR.558 + miR.609 + miR.603 + miR.520f + miR.220a + miR.519b + miR.130a + miR.29a + miR.376a* + miR.494 + miR.518c + miR.639 | 0,81 | 0,039708588 |
| miR.558 + miR.609 + miR.220a + miR.519b + miR.130a + miR.26a + miR.29a + miR.369.3p + miR.376a* + miR.518c + miR.660 + miR.338 | 0,81 | 0,13385948 |
| miR.558 + miR.609 + miR.220a + miR.519b + miR.130a + miR.29a + miR.369.3p + miR.376a* + miR.494 + miR.518c + miR.639 + miR.660 | 0,81 | 0,17654863 |
| miR.558 + miR.609 + miR.520f + miR.220a + miR.26a + miR.29a + miR.376a* + miR.494 + miR.518c + miR.639 + miR.660 + miR.338 | 0,81 | 0,01373943 |
| miR.558 + miR.609 + miR.130a + miR.26a + miR.29a + miR.369.3p + miR.376a* + miR.494 + miR.518c + miR.639 + miR.660 + miR.338 | 0,81 | 0,136137339 |
| miR.558 + miR.609 + miR.520f + miR.220a + miR.130a + miR.26a + miR.29a + miR.362 + miR.369.3p + miR.376a* + miR.518c + miR.338 | 0,81 | 0,059078533 |
| miR.558 + miR.609 + miR.520f + miR.130a + miR.26a + miR.29a + miR.369.3p + miR.376a* + miR.494 + miR.518c + miR.639 + miR.338 | 0,81 | 0,150656465 |
| miR.558 + miR.609 + miR.520f + miR.220a + miR.519b + miR.130a + miR.29a + miR.362 + miR.369.3p + miR.376a* + miR.518c + miR.660 | 0,81 | 0,194891454 |
| miR.558 + miR.609 + miR.603 + miR.220a + miR.130a + miR.26a + miR.29a + miR.362 + miR.376a* + miR.494 + miR.518c + miR.338 | 0,81 | 0,036858595 |
| miR.609 + miR.603 + miR.520f + miR.220a + miR.29a + miR.362 + miR.369.3p + miR.376a* + miR.518c + miR.639 + miR.660 + miR.338 | 0,81 | 0,28878047 |
| miR.558 + miR.609 + miR.603 + miR.520f + miR.220a + miR.519b + miR.29a + miR.362 + miR.376a* + miR.518c + miR.639 + miR.660 | 0,81 | 0,063699233 |
| miR.558 + miR.609 + miR.603 + miR.520f + miR.220a + miR.29a + miR.362 + miR.376a* + miR.518c + miR.639 + miR.660 + miR.338 | 0,81 | 0,01008843 |
| miR.558 + miR.609 + miR.603 + miR.520f + miR.220a + miR.130a + miR.29a + miR.362 + miR.376a* + miR.518c + miR.639 + miR.338 | 0,81 | 0,011132006 |
| miR.558 + miR.609 + miR.520f + miR.220a + miR.519b + miR.26a + miR.29a + miR.369.3p + miR.376a* + miR.494 + miR.518c + miR.338 | 0,81 | 0,108702048 |
| miR.558 + miR.609 + miR.220a + miR.519b + miR.29a + miR.362 + miR.369.3p + miR.376a* + miR.494 + miR.518c + miR.660 + miR.338 | 0,81 | 0,135216814 |
| miR.558 + miR.609 + miR.603 + miR.130a + miR.26a + miR.29a + miR.362 + miR.369.3p + miR.376a* + miR.518c + miR.639 + miR.660 | 0,81 | 0,060174887 |
| miR.558 + miR.609 + miR.603 + miR.520f + miR.220a + miR.130a + miR.29a + miR.376a* + miR.494 + miR.518c + miR.660 + miR.338 | 0,81 | 0,012907975 |
| miR.558 + miR.609 + miR.603 + miR.520f + miR.220a + miR.519b + miR.26a + miR.29a + miR.376a* + miR.494 + miR.518c + miR.639 | 0,81 | 0,035766004 |
| miR.558 + miR.609 + miR.603 + miR.519b + miR.130a + miR.26a + miR.29a + miR.369.3p + miR.376a* + miR.494 + miR.518c + miR.639 | 0,81 | 0,12980033 |
| miR.558 + miR.609 + miR.520f + miR.220a + miR.519b + miR.29a + miR.362 + miR.369.3p + miR.376a* + miR.494 + miR.518c + miR.338 | 0,81 | 0,110800804 |
| miR.558 + miR.609 + miR.603 + miR.520f + miR.220a + miR.29a + miR.362 + miR.376a* + miR.494 + miR.518c + miR.639 + miR.660 | 0,81 | 0,012330843 |
| miR.558 + miR.609 + miR.603 + miR.520f + miR.220a + miR.29a + miR.362 + miR.376a* + miR.494 + miR.518c + miR.660 + miR.338 | 0,81 | 0,019484589 |
| miR.558 + miR.609 + miR.603 + miR.520f + miR.220a + miR.519b + miR.26a + miR.29a + miR.376a* + miR.518c + miR.660 + miR.338 | 0,81 | 0,0316 36068 |
| miR.558 + miR.609 + miR.520f + miR.130a + miR.26a + miR.29a + miR.376a* + miR.494 + miR.518c + miR.639 + miR.660 + miR.338 | 0,81 | 0,03241325 |
| miR.558 + miR.609 + miR.220a + miR.130a + miR.29a + miR.369.3p + miR.376a* + miR.494 + miR.518c + miR.639 + miR.660 + miR.338 | 0,81 | 0,142320328 |
| miR.558 + miR.609 + miR.520f + miR.519b + miR.29a + miR.362 + miR.376a* + miR.494 + miR.518c + miR.639 + miR.660 + miR.338 | 0,81 | 0,029351233 |
| miR.558 + miR.609 + miR.603 + miR.520f + miR.220a + miR.130a + miR.26a + miR.29a + miR.362 + miR.376a* + miR.518c + miR.639 | 0,81 | 0,015167093 |
| miR.558 + miR.609 + miR.603 + miR.520f + miR.220a + miR.26a + miR.29a + miR.362 + miR.376a* + miR.494 + miR.518c + miR.338 | 0,81 | 0,016209377 |
| miR.558 + miR.609 + miR.603 + miR.520f + miR.519b + miR.29a + miR.362 + miR.369.3p + miR.376a* + miR.518c + miR.639 + miR.660 | 0,81 | 0,001469839 |
| miR.558 + miR.609 + miR.220a + miR.519b + miR.130a + miR.29a + miR.369.3p + miR.376a* + miR.494 + miR.518c + miR.660 + miR.338 | 0,81 | 0,121695094 |
| miR.558 + miR.609 + miR.603 + miR.520f + miR.220a + miR.362 + miR.369.3p + miR.376a* + miR.518c + miR.639 + miR.660 + miR.338 | 0,81 | 0,137494535 |
| miR.558 + miR.609 + miR.603 + miR.520f + miR.220a + miR.519b + miR.130a + miR.29a + miR.362 + miR.376a* + miR.518c + miR.660 | 0,81 | 0,065356793 |
| miR.558 + miR.609 + miR.603 + miR.520f + miR.220a + miR.26a + miR.29a + miR.362 + miR.376a* + miR.494 + miR.518c + miR.639 | 0,81 | 0,011836102 |
| miR.558 + miR.609 + miR.603 + miR.519b + miR.130a + miR.29a + miR.362 + miR.369.3p + miR.376a* + miR.518c + miR.639 + miR.660 | 0,81 | 0,068256135 |
| miR.558 + miR.609 + miR.520f + miR.519b + miR.130a + miR.29a + miR.369.3p + miR.376a* + miR.494 + miR.518c + miR.639 + miR.660 | 0,81 | 0,145298664 |
| miR.558 + miR.609 + miR.603 + miR.220a + miR.26a + miR.29a + miR.362 + miR.376a* + miR.494 + miR.518c + miR.639 + miR.660 | 0,81 | 0,022531502 |
| miR.558 + miR.609 + miR.520f + miR.519b + miR.130a + miR.29a + miR.376a* + miR.494 + miR.518c + miR.639 + miR.660 + miR.338 | 0,81 | 0,092287149 |
| miR.558 + miR.609 + miR.520f + miR.220a + miR.130a + miR.26a + miR.29a + miR.369.3p + miR.376a* + miR.494 + miR.518c + miR.338 | 0,81 | 0,062383315 |
| miR.558 + miR.609 + miR.520f + miR.220a + miR.130a + miR.29a + miR.369.3p + miR.376a* + miR.494 + miR.518c + miR.639 + miR.338 | 0,81 | 0,018346144 |
| miR.558 + miR.609 + miR.603 + miR.220a + miR.519b + miR.26a + miR.29a + miR.362 + miR.376a* + miR.518c + miR.660 + miR.338 | 0,81 | 0,046966269 |
| miR.609 + miR.603 + miR.520f + miR.220a + miR.29a + miR.362 + miR.369.3p + miR.376a* + miR.494 + miR.518c + miR.660 + miR.338 | 0,81 | 0,459369718 |
| miR.558 + miR.609 + miR.603 + miR.520f + miR.29a + miR.362 + miR.376a* + miR.494 + miR.518c + miR.639 + miR.660 + miR.338 | 0,81 | 0,00453373 |
| miR.558 + miR.609 + miR.603 + miR.220a + miR.519b + miR.130a + miR.26a + miR.29a + miR.362 + miR.376a* + miR.518c + miR.639 | 0,81 | 0,059953662 |
| miR.558 + miR.609 + miR.603 + miR.520f + miR.519b + miR.130a + miR.26a + miR.29a + miR.369.3p + miR.376a* + miR.518c + miR.338 | 0,81 | 0,046046847 |
| miR.558 + miR.609 + miR.603 + miR.220a + miR.519b + miR.29a + miR.362 + miR.369.3p + miR.376a* + miR.494 + miR.518c + miR.338 | 0,81 | 0,060805084 |
| miR.558 + miR.609 + miR.603 + miR.519b + miR.130a + miR.29a + miR.369.3p + miR.376a* + miR.494 + miR.518c + miR.639 + miR.660 | 0,81 | 0,084343115 |
| miR.558 + miR.609 + miR.603 + miR.520f + miR.519b + miR.130a + miR.29a + miR.369.3p + miR.376a* + miR.494 + miR.518c + miR.639 | 0,81 | 0,055157305 |
| miR.558 + miR.609 + miR.603 + miR.220a + miR.519b + miR.26a + miR.29a + miR.376a* + miR.494 + miR.518c + miR.639 + miR.338 | 0,81 | 0,041939507 |
| miR.558 + miR.609 + miR.520f + miR.220a + miR.519b + miR.29a + miR.362 + miR.376a* + miR.494 + miR.518c + miR.660 + miR.338 | 0,81 | 0,131488636 |
| miR.558 + miR.609 + miR.603 + miR.520f + miR.130a + miR.29a + miR.362 + miR.369.3p + miR.376a* + miR.494 + miR.518c + miR.338 | 0,81 | 0,015459265 |
| miR.558 + miR.609 + miR.603 + miR.520f + miR.130a + miR.26a + miR.29a + miR.369.3p + miR.376a* + miR.518c + miR.639 + miR.660 | 0,81 | 0,042859862 |
| miR.558 + miR.609 + miR.520f + miR.220a + miR.130a + miR.26a + miR.29a + miR.369.3p + miR.376a* + miR.518c + miR.639 + miR.338 | 0,81 | 0,065284809 |
| miR.558 + miR.609 + miR.520f + miR.26a + miR.29a + miR.362 + miR.376a* + miR.494 + miR.518c + miR.639 + miR.660 + miR.338 | 0,81 | 0,008497078 |
| miR.558 + miR.609 + miR.520f + miR.519b + miR.26a + miR.29a + miR.369.3p + miR.376a* + miR.494 + miR.518c + miR.660 + miR.338 | 0,81 | 0,197398853 |
| miR.558 + miR.609 + miR.603 + miR.520f + miR.220a + miR.519b + miR.29a + miR.376a* + miR.518c + miR.639 + miR.660 + miR.338 | 0,81 | 0,022481601 |
| miR.558 + miR.609 + miR.603 + miR.520f + miR.130a + miR.26a + miR.29a + miR.362 + miR.369.3p + miR.376a* + miR.518c + miR.338 | 0,81 | 0,02748033 |
| miR.558 + miR.609 + miR.603 + miR.520f + miR.220a + miR.130a + miR.29a + miR.362 + miR.376a* + miR.494 + miR.518c + miR.639 | 0,81 | 0,014566377 |
| miR.558 + miR.609 + miR.220a + miR.130a + miR.26a + miR.29a + miR.369.3p + miR.376a* + miR.494 + miR.518c + miR.639 + miR.338 | 0,81 | 0,160453303 |
| miR.609 + miR.603 + miR.520f + miR.220a + miR.519b + miR.29a + miR.369.3p + miR.376a* + miR.494 + miR.518c + miR.660 + miR.338 | 0,81 | 0,073323357 |
| miR.558 + miR.609 + miR.603 + miR.220a + miR.519b + miR.130a + miR.26a + miR.29a + miR.376a* + miR.518c + miR.660 + miR.338 | 0,81 | 0,033961652 |
| miR.558 + miR.609 + miR.520f + miR.220a + miR.519b + miR.130a + miR.29a + miR.376a* + miR.494 + miR.518c + miR.660 + miR.338 | 0,81 | 0,151794101 |
| miR.558 + miR.609 + miR.603 + miR.220a + miR.130a + miR.26a + miR.29a + miR.376a* + miR.494 + miR.518c + miR.639 + miR.338 | 0,81 | 0,011945282 |
| miR.558 + miR.609 + miR.520f + miR.220a + miR.519b + miR.26a + miR.29a + miR.376a* + miR.494 + miR.518c + miR.660 + miR.338 | 0,81 | 0,122443803 |
| miR.558 + miR.609 + miR.603 + miR.520f + miR.220a + miR.26a + miR.29a + miR.362 + miR.376a* + miR.518c + miR.639 + miR.660 | 0,81 | 0,02084666 |
| miR.558 + miR.609 + miR.603 + miR.26a + miR.29a + miR.362 + miR.369.3p + miR.376a* + miR.494 + miR.518c + miR.639 + miR.338 | 0,81 | 0,1059354 |
| miR.558 + miR.609 + miR.603 + miR.520f + miR.519b + miR.29a + miR.369.3p + miR.376a* + miR.494 + miR.518c + miR.639 + miR.660 | 0,81 | 0,017721516 |
| miR.558 + miR.609 + miR.520f + miR.220a + miR.519b + miR.130a + miR.26a + miR.29a + miR.369.3p + miR.376a* + miR.518c + miR.660 | 0,81 | 0,145828771 |
| miR.558 + miR.609 + miR.603 + miR.220a + miR.130a + miR.26a + miR.29a + miR.376a* + miR.494 + miR.518c + miR.660 + miR.338 | 0,81 | 0,025582821 |
| miR.558 + miR.609 + miR.603 + miR.520f + miR.220a + miR.130a + miR.26a + miR.29a + miR.362 + miR.376a* + miR.518c + miR.338 | 0,81 | 0,019232568 |
| miR.558 + miR.609 + miR.520f + miR.220a + miR.130a + miR.29a + miR.362 + miR.369.3p + miR.376a* + miR.494 + miR.518c + miR.338 | 0,81 | 0,012884269 |
| miR.558 + miR.609 + miR.220a + miR.519b + miR.130a + miR.26a + miR.29a + miR.362 + miR.369.3p + miR.376a* + miR.518c + miR.660 | 0,81 | 0,208274507 |
| miR.558 + miR.609 + miR.603 + miR.520f + miR.130a + miR.26a + miR.29a + miR.369.3p + miR.376a* + miR.494 + miR.518c + miR.639 | 0,81 | 0,046115075 |
| miR.558 + miR.609 + miR.603 + miR.220a + miR.519b + miR.26a + miR.29a + miR.376a* + miR.518c + miR.639 + miR.660 + miR.338 | 0,81 | 0,031320253 |
| miR.609 + miR.603 + miR.520f + miR.220a + miR.29a + miR.362 + miR.369.3p + miR.376a* + miR.494 + miR.518c + miR.639 + miR.660 | 0,81 | 0,050804025 |
| miR.558 + miR.609 + miR.603 + miR.520f + miR.130a + miR.29a + miR.376a* + miR.494 + miR.518c + miR.639 + miR.660 + miR.338 | 0,81 | 0,010407148 |
| miR.558 + miR.609 + miR.603 + miR.130a + miR.26a + miR.29a + miR.362 + miR.369.3p + miR.376a* + miR.518c + miR.639 + miR.338 | 0,81 | 0,169599323 |
| miR.558 + miR.609 + miR.603 + miR.520f + miR.26a + miR.29a + miR.376a* + miR.494 + miR.518c + miR.639 + miR.660 + miR.338 | 0,81 | 0,007266293 |
| miR.558 + miR.609 + miR.220a + miR.519b + miR.130a + miR.26a + miR.29a + miR.369.3p + miR.376a* + miR.494 + miR.518c + miR.338 | 0,81 | 0,102180906 |
| miR.558 + miR.609 + miR.603 + miR.520f + miR.220a + miR.519b + miR.26a + miR.29a + miR.376a* + miR.518c + miR.639 + miR.660 | 0,81 | 0,056788287 |
| miR.558 + miR.609 + miR.520f + miR.220a + miR.519b + miR.26a + miR.29a + miR.362 + miR.369.3p + miR.376a* + miR.494 + miR.518c | 0,81 | 0,091913791 |
| miR.558 + miR.609 + miR.603 + miR.220a + miR.519b + miR.26a + miR.29a + miR.362 + miR.376a* + miR.518c + miR.639 + miR.660 | 0,81 | 0,065859447 |
| miR.558 + miR.609 + miR.520f + miR.220a + miR.26a + miR.29a + miR.362 + miR.369.3p + miR.376a* + miR.494 + miR.518c + miR.639 | 0,81 | 0,037019274 |
| miR.558 + miR.609 + miR.603 + miR.520f + miR.220a + miR.130a + miR.26a + miR.29a + miR.376a* + miR.518c + miR.660 + miR.338 | 0,81 | 0,012673271 |
| miR.558 + miR.609 + miR.520f + miR.220a + miR.519b + miR.130a + miR.29a + miR.369.3p + miR.376a* + miR.494 + miR.518c + miR.338 | 0,81 | 0,084575545 |
| miR.558 + miR.609 + miR.603 + miR.220a + miR.26a + miR.29a + miR.362 + miR.376a* + miR.494 + miR.518c + miR.660 + miR.338 | 0,81 | 0,024314979 |
| miR.558 + miR.609 + miR.603 + miR.520f + miR.220a + miR.519b + miR.130a + miR.29a + miR.376a* + miR.518c + miR.639 + miR.660 | 0,81 | 0,053034712 |
| miR.558 + miR.609 + miR.520f + miR.220a + miR.519b + miR.130a + miR.29a + miR.362 + miR.376a* + miR.494 + miR.518c + miR.338 | 0,81 | 0,105246147 |
| miR.558 + miR.609 + miR.603 + miR.520f + miR.220a + miR.519b + miR.130a + miR.26a + miR.29a + miR.376a* + miR.518c + miR.660 | 0,81 | 0,055184389 |
| miR.558 + miR.609 + miR.603 + miR.520f + miR.220a + miR.26a + miR.29a + miR.362 + miR.376a* + miR.494 + miR.518c + miR.660 | 0,81 | 0,021839451 |
| miR.558 + miR.609 + miR.603 + miR.520f + miR.220a + miR.130a + miR.26a + miR.29a + miR.376a* + miR.518c + miR.639 + miR.660 | 0,81 | 0,013455972 |
| miR.558 + miR.609 + miR.220a + miR.519b + miR.26a + miR.29a + miR.369.3p + miR.376a* + miR.494 + miR.518c + miR.639 + miR.338 | 0,81 | 0,107983349 |
| miR.558 + miR.609 + miR.603 + miR.520f + miR.519b + miR.130a + miR.29a + miR.362 + miR.369.3p + miR.376a* + miR.518c + miR.338 | 0,81 | 0,036866114 |
| miR.558 + miR.609 + miR.603 + miR.520f + miR.220a + miR.130a + miR.26a + miR.29a + miR.376a* + miR.494 + miR.518c + miR.660 | 0,81 | 0,018768361 |
| miR.558 + miR.609 + miR.520f + miR.220a + miR.519b + miR.29a + miR.369.3p + miR.376a* + miR.494 + miR.518c + miR.639 + miR.338 | 0,81 | 0,06726191 |
| miR.558 + miR.609 + miR.603 + miR.220a + miR.519b + miR.130a + miR.26a + miR.29a + miR.362 + miR.376a* + miR.518c + miR.660 | 0,81 | 0,065863144 |
| miR.558 + miR.609 + miR.220a + miR.130a + miR.26a + miR.29a + miR.362 + miR.369.3p + miR.376a* + miR.518c + miR.639 + miR.338 | 0,81 | 0,073562011 |
| miR.558 + miR.609 + miR.520f + miR.220a + miR.519b + miR.29a + miR.362 + miR.376a* + miR.494 + miR.518c + miR.639 + miR.338 | 0,81 | 0,082843943 |
| miR.558 + miR.609 + miR.220a + miR.519b + miR.29a + miR.362 + miR.369.3p + miR.376a* + miR.494 + miR.518c + miR.639 + miR.660 | 0,81 | 0,096588718 |
| miR.558 + miR.609 + miR.220a + miR.26a + miR.29a + miR.362 + miR.369.3p + miR.376a* + miR.494 + miR.518c + miR.639 + miR.338 | 0,81 | 0,08833119 |
| miR.558 + miR.609 + miR.603 + miR.520f + miR.220a + miR.519b + miR.130a + miR.29a + miR.376a* + miR.518c + miR.660 + miR.338 | 0,81 | 0,020873253 |
| miR.558 + miR.609 + miR.603 + miR.520f + miR.519b + miR.26a + miR.29a + miR.362 + miR.369.3p + miR.376a* + miR.518c + miR.338 | 0,81 | 0,05918306 |
| miR.609 + miR.603 + miR.220a + miR.26a + miR.29a + miR.362 + miR.369.3p + miR.376a* + miR.494 + miR.518c + miR.639 + miR.660 | 0,81 | 0,088589459 |
| miR.558 + miR.609 + miR.603 + miR.520f + miR.26a + miR.29a + miR.362 + miR.369.3p + miR.376a* + miR.494 + miR.518c + miR.660 | 0,81 | 0,015296836 |
| miR.558 + miR.609 + miR.603 + miR.220a + miR.130a + miR.29a + miR.369.3p + miR.376a* + miR.494 + miR.518c + miR.639 + miR.338 | 0,81 | 0,029482207 |
| miR.558 + miR.609 + miR.603 + miR.519b + miR.26a + miR.29a + miR.362 + miR.369.3p + miR.376a* + miR.518c + miR.639 + miR.338 | 0,81 | 0,150802321 |
| miR.558 + miR.609 + miR.603 + miR.220a + miR.130a + miR.29a + miR.362 + miR.369.3p + miR.376a* + miR.518c + miR.639 + miR.338 | 0,81 | 0,018974883 |
| miR.558 + miR.609 + miR.520f + miR.220a + miR.130a + miR.29a + miR.362 + miR.369.3p + miR.376a* + miR.518c + miR.639 + miR.338 | 0,81 | 0,023453589 |
| miR.558 + miR.609 + miR.520f + miR.220a + miR.519b + miR.26a + miR.29a + miR.362 + miR.369.3p + miR.376a* + miR.518c + miR.338 | 0,81 | 0,091880685 |
| miR.558 + miR.609 + miR.603 + miR.520f + miR.220a + miR.130a + miR.29a + miR.362 + miR.376a* + miR.494 + miR.518c + miR.338 | 0,81 | 0,008155178 |
| miR.558 + miR.609 + miR.603 + miR.220a + miR.130a + miR.26a + miR.29a + miR.362 + miR.376a* + miR.494 + miR.518c + miR.660 | 0,81 | 0,028393429 |
| miR.558 + miR.609 + miR.220a + miR.519b + miR.26a + miR.29a + miR.362 + miR.369.3p + miR.376a* + miR.494 + miR.518c + miR.338 | 0,81 | 0,106742132 |
| miR.558 + miR.609 + miR.603 + miR.220a + miR.130a + miR.26a + miR.29a + miR.376a* + miR.518c + miR.639 + miR.660 + miR.338 | 0,81 | 0,023293723 |
| miR.558 + miR.609 + miR.220a + miR.519b + miR.130a + miR.29a + miR.369.3p + miR.376a* + miR.518c + miR.639 + miR.660 + miR.338 | 0,81 | 0,160898556 |
| miR.558 + miR.609 + miR.603 + miR.519b + miR.26a + miR.29a + miR.369.3p + miR.376a* + miR.494 + miR.518c + miR.639 + miR.660 | 0,81 | 0,104359824 |
| miR.558 + miR.609 + miR.520f + miR.519b + miR.130a + miR.29a + miR.369.3p + miR.376a* + miR.494 + miR.518c + miR.639 + miR.338 | 0,81 | 0,150080424 |
| miR.558 + miR.609 + miR.603 + miR.220a + miR.519b + miR.29a + miR.362 + miR.376a* + miR.494 + miR.518c + miR.639 + miR.660 | 0,81 | 0,040242063 |
| miR.558 + miR.609 + miR.603 + miR.520f + miR.220a + miR.519b + miR.29a + miR.362 + miR.376a* + miR.518c + miR.660 + miR.338 | 0,81 | 0,033843126 |
| miR.558 + miR.609 + miR.220a + miR.130a + miR.26a + miR.29a + miR.362 + miR.369.3p + miR.376a* + miR.494 + miR.518c + miR.338 | 0,81 | 0,08263367 |
| miR.558 + miR.609 + miR.603 + miR.520f + miR.130a + miR.29a + miR.362 + miR.369.3p + miR.376a* + miR.518c + miR.639 + miR.660 | 0,81 | 0,009075302 |
| miR.558 + miR.609 + miR.603 + miR.130a + miR.26a + miR.29a + miR.362 + miR.369.3p + miR.376a* + miR.494 + miR.518c + miR.639 | 0,81 | 0,077297432 |
| miR.558 + miR.609 + miR.603 + miR.220a + miR.26a + miR.29a + miR.362 + miR.376a* + miR.518c + miR.639 + miR.660 + miR.338 | 0,81 | 0,026020114 |
| miR.558 + miR.609 + miR.520f + miR.130a + miR.26a + miR.29a + miR.369.3p + miR.376a* + miR.494 + miR.518c + miR.660 + miR.338 | 0,81 | 0,139163048 |
| miR.558 + miR.609 + miR.603 + miR.519b + miR.26a + miR.29a + miR.369.3p + miR.376a* + miR.494 + miR.518c + miR.639 + miR.338 | 0,81 | 0,107080588 |
| miR.558 + miR.609 + miR.520f + miR.519b + miR.130a + miR.29a + miR.362 + miR.369.3p + miR.376a* + miR.518c + miR.639 + miR.660 | 0,81 | 0,011583671 |
| miR.558 + miR.609 + miR.603 + miR.520f + miR.220a + miR.130a + miR.29a + miR.362 + miR.376a* + miR.518c + miR.660 + miR.338 | 0,81 | 0,008695689 |
| miR.558 + miR.609 + miR.603 + miR.220a + miR.519b + miR.29a + miR.362 + miR.376a* + miR.494 + miR.518c + miR.639 + miR.338 | 0,81 | 0,034361831 |
| miR.558 + miR.609 + miR.603 + miR.520f + miR.26a + miR.29a + miR.362 + miR.369.3p + miR.376a* + miR.494 + miR.518c + miR.639 | 0,81 | 0,058897126 |
| miR.558 + miR.609 + miR.220a + miR.519b + miR.130a + miR.26a + miR.29a + miR.369.3p + miR.376a* + miR.518c + miR.639 + miR.338 | 0,81 | 0,068326703 |
| miR.558 + miR.609 + miR.603 + miR.220a + miR.519b + miR.130a + miR.26a + miR.29a + miR.376a* + miR.518c + miR.639 + miR.338 | 0,81 | 0,024465061 |
| miR.558 + miR.609 + miR.603 + miR.520f + miR.519b + miR.26a + miR.29a + miR.369.3p + miR.376a* + miR.518c + miR.639 + miR.660 | 0,81 | 0,034232877 |
| miR.558 + miR.609 + miR.603 + miR.220a + miR.130a + miR.26a + miR.29a + miR.362 + miR.376a* + miR.518c + miR.660 + miR.338 | 0,81 | 0,028182389 |
| miR.558 + miR.609 + miR.603 + miR.520f + miR.220a + miR.26a + miR.29a + miR.362 + miR.376a* + miR.518c + miR.660 + miR.338 | 0,81 | 0,032196505 |
| miR.558 + miR.609 + miR.603 + miR.520f + miR.220a + miR.29a + miR.362 + miR.376a* + miR.494 + miR.518c + miR.639 + miR.338 | 0,81 | 0,010431776 |
| miR.558 + miR.609 + miR.520f + miR.220a + miR.130a + miR.26a + miR.29a + miR.362 + miR.369.3p + miR.376a* + miR.494 + miR.518c | 0,81 | 0,044108403 |
| miR.558 + miR.609 + miR.603 + miR.520f + miR.220a + miR.130a + miR.29a + miR.362 + miR.376a* + miR.494 + miR.518c + miR.660 | 0,81 | 0,01425374 |
| miR.558 + miR.609 + miR.603 + miR.519b + miR.130a + miR.26a + miR.29a + miR.369.3p + miR.376a* + miR.518c + miR.639 + miR.660 | 0,81 | 0,099730158 |
| miR.558 + miR.609 + miR.603 + miR.220a + miR.519b + miR.130a + miR.26a + miR.29a + miR.376a* + miR.518c + miR.639 + miR.660 | 0,81 | 0,051801339 |
| miR.558 + miR.609 + miR.520f + miR.220a + miR.519b + miR.130a + miR.29a + miR.362 + miR.369.3p + miR.376a* + miR.494 + miR.518c | 0,81 | 0,114057659 |
| miR.558 + miR.609 + miR.603 + miR.520f + miR.519b + miR.130a + miR.29a + miR.369.3p + miR.376a* + miR.518c + miR.639 + miR.660 | 0,81 | 0,036275635 |
| miR.558 + miR.609 + miR.603 + miR.520f + miR.519b + miR.29a + miR.362 + miR.369.3p + miR.376a* + miR.494 + miR.518c + miR.660 | 0,81 | 0,015514056 |
| miR.558 + miR.609 + miR.603 + miR.520f + miR.130a + miR.29a + miR.362 + miR.376a* + miR.494 + miR.518c + miR.639 + miR.338 | 0,81 | 0,013000463 |
| miR.558 + miR.609 + miR.220a + miR.519b + miR.130a + miR.26a + miR.29a + miR.362 + miR.369.3p + miR.376a* + miR.518c + miR.338 | 0,81 | 0,097029557 |
| miR.558 + miR.609 + miR.520f + miR.519b + miR.26a + miR.29a + miR.362 + miR.369.3p + miR.376a* + miR.518c + miR.639 + miR.660 | 0,81 | 0,009252758 |
| miR.558 + miR.609 + miR.603 + miR.520f + miR.519b + miR.130a + miR.29a + miR.376a* + miR.494 + miR.518c + miR.639 + miR.338 | 0,81 | 0,049176377 |
| miR.558 + miR.609 + miR.603 + miR.220a + miR.519b + miR.29a + miR.362 + miR.376a* + miR.494 + miR.518c + miR.660 + miR.338 | 0,81 | 0,028930724 |
| miR.558 + miR.609 + miR.603 + miR.520f + miR.220a + miR.130a + miR.26a + miR.29a + miR.362 + miR.376a* + miR.494 + miR.518c | 0,81 | 0,016122044 |
| miR.609 + miR.603 + miR.520f + miR.220a + miR.519b + miR.29a + miR.362 + miR.369.3p + miR.376a* + miR.494 + miR.518c + miR.660 | 0,81 | 0,081627784 |
| miR.558 + miR.609 + miR.520f + miR.26a + miR.29a + miR.362 + miR.369.3p + miR.376a* + miR.494 + miR.518c + miR.639 + miR.660 | 0,81 | 0,028677451 |
| miR.558 + miR.609 + miR.520f + miR.220a + miR.519b + miR.130a + miR.29a + miR.376a* + miR.494 + miR.518c + miR.639 + miR.660 | 0,81 | 0,046268632 |
| miR.558 + miR.609 + miR.520f + miR.220a + miR.519b + miR.26a + miR.29a + miR.369.3p + miR.376a* + miR.518c + miR.639 + miR.338 | 0,81 | 0,083714545 |
| miR.558 + miR.609 + miR.603 + miR.520f + miR.26a + miR.29a + miR.362 + miR.376a* + miR.494 + miR.518c + miR.639 + miR.338 | 0,81 | 0,014706726 |
| miR.558 + miR.609 + miR.603 + miR.520f + miR.220a + miR.519b + miR.130a + miR.26a + miR.29a + miR.376a* + miR.518c + miR.338 | 0,81 | 0,016802873 |
| miR.558 + miR.609 + miR.520f + miR.130a + miR.29a + miR.362 + miR.369.3p + miR.376a* + miR.494 + miR.639 + miR.660 + miR.338 | 0,81 | 0,326811823 |
| miR.558 + miR.609 + miR.603 + miR.520f + miR.130a + miR.29a + miR.362 + miR.369.3p + miR.376a* + miR.494 + miR.518c + miR.639 | 0,81 | 0,017844597 |
| miR.558 + miR.609 + miR.603 + miR.220a + miR.519b + miR.29a + miR.376a* + miR.494 + miR.518c + miR.639 + miR.660 + miR.338 | 0,81 | 0,022385025 |
| miR.558 + miR.609 + miR.520f + miR.220a + miR.130a + miR.26a + miR.29a + miR.369.3p + miR.376a* + miR.494 + miR.518c + miR.639 | 0,81 | 0,040945691 |
| miR.558 + miR.609 + miR.603 + miR.220a + miR.519b + miR.130a + miR.29a + miR.362 + miR.376a* + miR.518c + miR.639 + miR.338 | 0,81 | 0,028113842 |
| miR.558 + miR.609 + miR.603 + miR.220a + miR.130a + miR.26a + miR.29a + miR.362 + miR.376a* + miR.518c + miR.639 + miR.660 | 0,81 | 0,025666095 |
| miR.558 + miR.609 + miR.520f + miR.220a + miR.130a + miR.29a + miR.362 + miR.369.3p + miR.376a* + miR.494 + miR.518c + miR.639 | 0,81 | 0,015069342 |
| miR.558 + miR.609 + miR.603 + miR.519b + miR.130a + miR.26a + miR.29a + miR.369.3p + miR.376a* + miR.494 + miR.518c + miR.338 | 0,81 | 0,134016351 |
| miR.558 + miR.609 + miR.519b + miR.130a + miR.26a + miR.29a + miR.369.3p + miR.376a* + miR.518c + miR.639 + miR.660 + miR.338 | 0,81 | 0,264229673 |
| miR.558 + miR.609 + miR.603 + miR.519b + miR.26a + miR.29a + miR.362 + miR.369.3p + miR.376a* + miR.494 + miR.518c + miR.660 | 0,81 | 0,048621112 |
| miR.558 + miR.609 + miR.520f + miR.220a + miR.26a + miR.29a + miR.362 + miR.376a* + miR.494 + miR.518c + miR.660 + miR.338 | 0,81 | 0,024900079 |
| miR.558 + miR.609 + miR.603 + miR.520f + miR.220a + miR.519b + miR.130a + miR.29a + miR.376a* + miR.518c + miR.639 + miR.338 | 0,81 | 0,012540071 |
| miR.558 + miR.609 + miR.520f + miR.220a + miR.26a + miR.29a + miR.362 + miR.376a* + miR.518c + miR.639 + miR.660 + miR.338 | 0,81 | 0,014852528 |
| miR.609 + miR.603 + miR.220a + miR.519b + miR.26a + miR.29a + miR.362 + miR.369.3p + miR.376a* + miR.494 + miR.518c + miR.660 | 0,81 | 0,117838095 |
| miR.558 + miR.609 + miR.603 + miR.520f + miR.220a + miR.519b + miR.26a + miR.29a + miR.376a* + miR.518c + miR.639 + miR.338 | 0,81 | 0,019448369 |
| miR.558 + miR.609 + miR.520f + miR.220a + miR.519b + miR.29a + miR.362 + miR.369.3p + miR.376a* + miR.518c + miR.639 + miR.338 | 0,81 | 0,056690145 |
| miR.558 + miR.609 + miR.603 + miR.520f + miR.519b + miR.26a + miR.29a + miR.369.3p + miR.376a* + miR.494 + miR.518c + miR.660 | 0,81 | 0,057877174 |
| miR.558 + miR.609 + miR.603 + miR.220a + miR.130a + miR.29a + miR.362 + miR.376a* + miR.494 + miR.518c + miR.639 + miR.660 | 0,81 | 0,016410248 |
| miR.558 + miR.609 + miR.520f + miR.220a + miR.130a + miR.29a + miR.362 + miR.376a* + miR.494 + miR.518c + miR.660 + miR.338 | 0,81 | 0,026981964 |
| miR.558 + miR.609 + miR.603 + miR.520f + miR.130a + miR.362 + miR.369.3p + miR.376a* + miR.518c + miR.639 + miR.660 + miR.338 | 0,81 | 0,099572501 |
| miR.558 + miR.609 + miR.220a + miR.519b + miR.130a + miR.26a + miR.29a + miR.362 + miR.369.3p + miR.376a* + miR.494 + miR.518c | 0,81 | 0,113619614 |
| miR.558 + miR.609 + miR.520f + miR.220a + miR.130a + miR.29a + miR.362 + miR.376a* + miR.518c + miR.639 + miR.660 + miR.338 | 0,81 | 0,013267938 |
| miR.558 + miR.609 + miR.603 + miR.520f + miR.220a + miR.130a + miR.26a + miR.29a + miR.376a* + miR.494 + miR.518c + miR.639 | 0,81 | 0,012488873 |
| miR.558 + miR.609 + miR.603 + miR.130a + miR.26a + miR.29a + miR.369.3p + miR.376a* + miR.494 + miR.518c + miR.660 + miR.338 | 0,81 | 0,082804278 |
| miR.558 + miR.609 + miR.603 + miR.519b + miR.26a + miR.29a + miR.369.3p + miR.376a* + miR.494 + miR.518c + miR.660 + miR.338 | 0,81 | 0,107391331 |
| miR.558 + miR.609 + miR.220a + miR.130a + miR.26a + miR.29a + miR.362 + miR.369.3p + miR.376a* + miR.494 + miR.518c + miR.639 | 0,81 | 0,070220752 |
| miR.558 + miR.609 + miR.603 + miR.519b + miR.26a + miR.29a + miR.362 + miR.369.3p + miR.376a* + miR.494 + miR.518c + miR.338 | 0,81 | 0,112781487 |
| miR.558 + miR.609 + miR.519b + miR.26a + miR.29a + miR.362 + miR.369.3p + miR.376a* + miR.494 + miR.518c + miR.660 + miR.338 | 0,81 | 0,285336004 |

**Table 13: clusters of 13 miRNAs**

| **Cluster of 13 miRs** | **c_tau level** | **pv compared to top signat ure miR.5 58 + miR.6 09 + miR.5 20f + miR.1 30a + miR.2 9a + miR.3 62 + miR.3 69.3p + miR.3 76a* + miR.4 94 + miR.5 18c + miR.6 39 + miR.6 60 + miR.3 38** |
|---|---|---|
| miR.558 + miR.609 + miR.520f + miR.130a + miR.29a + miR.362 + miR.369.3p + miR.376a* + miR.494 + miR.518c + miR.639 + miR.660 + miR.338 | 0,82 | NaN |
| miR.558 + miR.609 + miR.603 + miR.520f + miR.220a + miR.29a + miR.362 + miR.369.3p + miR.376a* + miR.518c + miR.639 + miR.660 + miR.338 | 0,81 | 0,472941439 |
| miR.558 + miR.609 + miR.520f + miR.519b + miR.26a + miR.29a + miR.362 + miR.369.3p + miR.376a* + miR.518c + miR.639 + miR.660 + miR.338 | 0,81 | 0,445059025 |
| miR.558 + miR.609 + miR.520f + miR.220a + miR.29a + miR.362 + miR.369.3p + miR.376a* + miR.494 + miR.518c + miR.639 + miR.660 + miR.338 | 0,81 | 0,464030389 |
| miR.558 + miR.609 + miR.603 + miR.520f + miR.29a + miR.362 + miR.369.3p + miR.376a* + miR.494 + miR.518c + miR.639 + miR.660 + miR.338 | 0,81 | 0,272060897 |
| miR.558 + miR.609 + miR.603 + miR.220a + miR.26a + miR.29a + miR.362 + miR.369.3p + miR.376a* + miR.518c + miR.639 + miR.660 + miR.338 | 0,81 | 0,363975285 |
| miR.558 + miR.609 + miR.520f + miR.220a + miR.26a + miR.29a + miR.362 + miR.369.3p + miR.376a* + miR.518c + miR.639 + miR.660 + miR.338 | 0,81 | 0,447323743 |
| miR.558 + miR.609 + miR.520f + miR.519b + miR.130a + miR.29a + miR.362 + miR.369.3p + miR.376a* + miR.518c + miR.639 + miR.660 + miR.338 | 0,81 | 0,366518399 |
| miR.558 + miR.609 + miR.520f + miR.220a + miR.519b + miR.29a + miR.362 + miR.369.3p + miR.376a* + miR.518c + miR.639 + miR.660 + miR.338 | 0,81 | 0,314675009 |
| miR.558 + miR.609 + miR.603 + miR.520f + miR.26a + miR.29a + miR.362 + miR.369.3p + miR.376a* + miR.518c + miR.639 + miR.660 + miR.338 | 0,81 | 0,20443385 |
| miR.558 + miR.609 + miR.520f + miR.220a + miR.130a + miR.29a + miR.362 + miR.369.3p + miR.376a* + miR.518c + miR.639 + miR.660 + miR.338 | 0,81 | 0,424259484 |
| miR.558 + miR.609 + miR.603 + miR.520f + miR.130a + miR.29a + miR.362 + miR.369.3p + miR.376a* + miR.518c + miR.639 + miR.660 + miR.338 | 0,81 | 0,268558591 |
| miR.558 + miR.609 + miR.603 + miR.520f + miR.220a + miR.519b + miR.29a + miR.369.3p + miR.376a* + miR.494 + miR.518c + miR.660 + miR.338 | 0,81 | 0,193645248 |
| miR.558 + miR.609 + miR.603 + miR.520f + miR.519b + miR.29a + miR.362 + miR.369.3p + miR.376a* + miR.518c + miR.639 + miR.660 + miR.338 | 0,81 | 0,408551927 |
| miR.558 + miR.609 + miR.603 + miR.520f + miR.220a + miR.26a + miR.29a + miR.369.3p + miR.376a* + miR.518c + miR.639 + miR.660 + miR.338 | 0,81 | 0,164812906 |
| miR.558 + miR.609 + miR.520f + miR.130a + miR.26a + miR.29a + miR.362 + miR.369.3p + miR.376a* + miR.518c + miR.639 + miR.660 + miR.338 | 0,81 | 0,28390477 |
| miR.558 + miR.609 + miR.130a + miR.26a + miR.29a + miR.362 + miR.369.3p + miR.376a* + miR.494 + miR.518c + miR.639 + miR.660 + miR.338 | 0,81 | 0,287064031 |
| miR.558 + miR.609 + miR.603 + miR.220a + miR.26a + miR.29a + miR.362 + miR.369.3p + miR.376a* + miR.494 + miR.518c + miR.660 + miR.338 | 0,81 | 0,377718286 |
| miR.558 + miR.609 + miR.520f + miR.26a + miR.29a + miR.362 + miR.369.3p + miR.376a* + miR.494 + miR.518c + miR.639 + miR.660 + miR.338 | 0,81 | 0,495493491 |
| miR.558 + miR.609 + miR.603 + miR.220a + miR.26a + miR.29a + miR.362 + miR.369.3p + miR.376a* + miR.494 + miR.518c + miR.639 + miR.660 | 0,81 | 0,299726626 |
| miR.558 + miR.609 + miR.603 + miR.220a + miR.26a + miR.29a + miR.369.3p + miR.376a* + miR.494 + miR.518c + miR.639 + miR.660 + miR.338 | 0,81 | 0,129683152 |
| miR.558 + miR.609 + miR.603 + miR.520f + miR.220a + miR.519b + miR.29a + miR.362 + miR.369.3p + miR.376a* + miR.518c + miR.660 + miR.338 | 0,81 | 0,393237863 |
| miR.558 + miR.609 + miR.603 + miR.520f + miR.220a + miR.29a + miR.369.3p + miR.376a* + miR.494 + miR.518c + miR.639 + miR.660 + miR.338 | 0,81 | 0,09061259 |
| miR.558 + miR.609 + miR.603 + miR.520f + miR.220a + miR.29a + miR.362 + miR.369.3p + miR.376a* + miR.494 + miR.518c + miR.660 + miR.338 | 0,81 | 0,374257772 |
| miR.558 + miR.609 + miR.520f + miR.519b + miR.29a + miR.362 + miR.369.3p + miR.376a* + miR.494 + miR.518c + miR.639 + miR.660 + miR.338 | 0,81 | 0,428506317 |
| miR.558 + miR.609 + miR.603 + miR.520f + miR.220a + miR.26a + miR.29a + miR.362 + miR.369.3p + miR.376a* + miR.518c + miR.660 + miR.338 | 0,81 | 0,378482633 |
| miR.558 + miR.609 + miR.603 + miR.520f + miR.220a + miR.130a + miR.29a + miR.369.3p + miR.376a* + miR.518c + miR.639 + miR.660 + miR.338 | 0,81 | 0,154696043 |
| miR.558 + miR.609 + miR.603 + miR.220a + miR.130a + miR.29a + miR.362 + miR.369.3p + miR.376a* + miR.518c + miR.639 + miR.660 + miR.338 | 0,81 | 0,461459422 |
| miR.558 + miR.609 + miR.603 + miR.220a + miR.519b + miR.26a + miR.29a + miR.369.3p + miR.376a* + miR.494 + miR.518c + miR.660 + miR.338 | 0,81 | 0,131097854 |
| miR.558 + miR.609 + miR.603 + miR.520f + miR.220a + miR.26a + miR.29a + miR.369.3p + miR.376a* + miR.494 + miR.518c + miR.660 + miR.338 | 0,81 | 0,1205 40301 |
| miR.558 + miR.609 + miR.603 + miR.220a + miR.519b + miR.26a + miR.29a + miR.362 + miR.369.3p + miR.376a* + miR.494 + miR.518c + miR.660 | 0,81 | 0,23188802 |
| miR.558 + miR.609 + miR.603 + miR.220a + miR.519b + miR.26a + miR.29a + miR.362 + miR.369.3p + miR.376a* + miR.518c + miR.660 + miR.338 | 0,81 | 0,338325475 |
| miR.558 + miR.609 + miR.603 + miR.220a + miR.519b + miR.130a + miR.29a + miR.369.3p + miR.376a* + miR.494 + miR.518c + miR.660 + miR.338 | 0,81 | 0,115593707 |
| miR.558 + miR.609 + miR.603 + miR.220a + miR.130a + miR.26a + miR.29a + miR.362 + miR.369.3p + miR.376a* + miR.518c + miR.660 + miR.338 | 0,81 | 0,357504945 |
| miR.558 + miR.609 + miR.603 + miR.520f + miR.220a + miR.130a + miR.29a + miR.362 + miR.369.3p + miR.376a* + miR.518c + miR.660 + miR.338 | 0,81 | 0,404005572 |
| miR.558 + miR.609 + miR.603 + miR.26a + miR.29a + miR.362 + miR.369.3p + miR.376a* + miR.494 + miR.518c + miR.639 + miR.660 + miR.338 | 0,81 | 0,096770394 |
| miR.558 + miR.609 + miR.520f + miR.220a + miR.26a + miR.29a + miR.362 + miR.369.3p + miR.376a* + miR.494 + miR.518c + miR.660 + miR.338 | 0,81 | 0,447201342 |
| miR.558 + miR.609 + miR.603 + miR.520f + miR.220a + miR.519b + miR.29a + miR.369.3p + miR.376a* + miR.518c + miR.639 + miR.660 + miR.338 | 0,81 | 0,214286829 |
| miR.558 + miR.609 + miR.603 + miR.130a + miR.29a + miR.362 + miR.369.3p + miR.376a* + miR.494 + miR.518c + miR.639 + miR.660 + miR.338 | 0,81 | 0,298233807 |
| miR.558 + miR.609 + miR.603 + miR.520f + miR.220a + miR.519b + miR.26a + miR.29a + miR.369.3p + miR.376a* + miR.494 + miR.518c + miR.660 | 0,81 | 0,180517781 |
| miR.558 + miR.609 + miR.603 + miR.520f + miR.220a + miR.26a + miR.29a + miR.362 + miR.369.3p + miR.376a* + miR.518c + miR.639 + miR.660 | 0,81 | 0,280699357 |
| miR.558 + miR.609 + miR.603 + miR.220a + miR.519b + miR.26a + miR.29a + miR.369.3p + miR.376a* + miR.494 + miR.518c + miR.639 + miR.660 | 0,81 | 0,182193834 |
| miR.558 + miR.609 + miR.603 + miR.520f + miR.220a + miR.130a + miR.26a + miR.29a + miR.369.3p + miR.376a* + miR.518c + miR.660 + miR.338 | 0,81 | 0,137041633 |
| miR.558 + miR.609 + miR.520f + miR.220a + miR.130a + miR.29a + miR.362 + miR.369.3p + miR.376a* + miR.494 + miR.518c + miR.660 + miR.338 | 0,81 | 0,417142274 |
| miR.558 + miR.609 + miR.603 + miR.220a + miR.130a + miR.26a + miR.29a + miR.369.3p + miR.376a* + miR.518c + miR.639 + miR.660 + miR.338 | 0,81 | 0,199437025 |
| miR.558 + miR.609 + miR.603 + miR.220a + miR.130a + miR.26a + miR.29a + miR.369.3p + miR.376a* + miR.494 + miR.518c + miR.639 + miR.660 | 0,81 | 0,181981186 |
| miR.558 + miR.609 + miR.603 + miR.220a + miR.130a + miR.26a + miR.29a + miR.369.3p + miR.376a* + miR.494 + miR.518c + miR.660 + miR.338 | 0,81 | 0,113204377 |
| miR.558 + miR.609 + miR.603 + miR.220a + miR.519b + miR.130a + miR.26a + miR.29a + miR.369.3p + miR.376a* + miR.494 + miR.518c + miR.660 | 0,81 | 0,191134215 |
| miR.558 + miR.609 + miR.603 + miR.520f + miR.220a + miR.519b + miR.26a + miR.29a + miR.369.3p + miR.376a* + miR.518c + miR.660 + miR.338 | 0,81 | 0,204627596 |
| miR.558 + miR.609 + miR.603 + miR.220a + miR.130a + miR.29a + miR.362 + miR.369.3p + miR.376a* + miR.494 + miR.518c + miR.639 + miR.660 | 0,81 | 0,197575929 |
| miR.558 + miR.609 + miR.603 + miR.220a + miR.519b + miR.26a + miR.29a + miR.362 + miR.369.3p + miR.376a* + miR.518c + miR.639 + miR.660 | 0,81 | 0,21718402 |
| miR.558 + miR.609 + miR.520f + miR.220a + miR.519b + miR.29a + miR.362 + miR.369.3p + miR.376a* + miR.494 + miR.518c + miR.660 + miR.338 | 0,81 | 0,409045122 |
| miR.558 + miR.609 + miR.603 + miR.520f + miR.220a + miR.26a + miR.29a + miR.369.3p + miR.376a* + miR.494 + miR.518c + miR.639 + miR.660 | 0,81 | 0,119776607 |
| miR.558 + miR.609 + miR.603 + miR.220a + miR.519b + miR.130a + miR.29a + miR.369.3p + miR.376a* + miR.494 + miR.518c + miR.639 + miR.660 | 0,81 | 0,151514703 |
| miR.558 + miR.609 + miR.603 + miR.519b + miR.26a + miR.29a + miR.362 + miR.369.3p + miR.376a* + miR.518c + miR.639 + miR.660 + miR.338 | 0,81 | 0,172712142 |
| miR.558 + miR.609 + miR.603 + miR.130a + miR.26a + miR.29a + miR.362 + miR.369.3p + miR.376a* + miR.518c + miR.639 + miR.660 + miR.338 | 0,81 | 0,186993533 |
| miR.558 + miR.609 + miR.603 + miR.520f + miR.220a + miR.26a + miR.29a + miR.362 + miR.369.3p + miR.376a* + miR.494 + miR.518c + miR.660 | 0,81 | 0,190463659 |
| miR.558 + miR.609 + miR.603 + miR.520f + miR.220a + miR.519b + miR.130a + miR.29a + miR.369.3p + miR.376a* + miR.518c + miR.660 + miR.338 | 0,81 | 0,233359488 |
| miR.558 + miR.609 + miR.603 + miR.520f + miR.220a + miR.519b + miR.29a + miR.362 + miR.369.3p + miR.376a* + miR.494 + miR.518c + miR.660 | 0,81 | 0,234529745 |
| miR.558 + miR.609 + miR.603 + miR.220a + miR.519b + miR.130a + miR.29a + miR.362 + miR.369.3p + miR.376a* + miR.494 + miR.518c + miR.660 | 0,81 | 0,175576547 |
| miR.558 + miR.609 + miR.603 + miR.520f + miR.220a + miR.519b + miR.29a + miR.369.3p + miR.376a* + miR.494 + miR.518c + miR.639 + miR.660 | 0,81 | 0,196246439 |
| miR.558 + miR.609 + miR.220a + miR.130a + miR.29a + miR.362 + miR.369.3p + miR.376a* + miR.494 + miR.518c + miR.639 + miR.660 + miR.338 | 0,81 | 0,387358587 |
| miR.558 + miR.609 + miR.603 + miR.220a + miR.519b + miR.130a + miR.26a + miR.29a + miR.369.3p + miR.376a* + miR.494 + miR.518c + miR.338 | 0,81 | 0,097397814 |
| miR.558 + miR.609 + miR.603 + miR.520f + miR.220a + miR.29a + miR.362 + miR.369.3p + miR.376a* + miR.494 + miR.518c + miR.639 + miR.660 | 0,81 | 0,128333944 |
| miR.558 + miR.609 + miR.603 + miR.220a + miR.130a + miR.26a + miR.29a + miR.362 + miR.369.3p + miR.376a* + miR.494 + miR.518c + miR.660 | 0,81 | 0,232043214 |
| miR.558 + miR.609 + miR.603 + miR.520f + miR.220a + miR.519b + miR.130a + miR.29a + miR.369.3p + miR.376a* + miR.494 + miR.518c + miR.660 | 0,81 | 0,162771652 |
| miR.558 + miR.609 + miR.220a + miR.26a + miR.29a + miR.362 + miR.369.3p + miR.376a* + miR.494 + miR.518c + miR.639 + miR.660 + miR.338 | 0,81 | 0,444413996 |
| miR.558 + miR.609 + miR.603 + miR.520f + miR.220a + miR.519b + miR.26a + miR.29a + miR.369.3p + miR.376a* + miR.494 + miR.518c + miR.338 | 0,81 | 0,099259954 |
| miR.558 + miR.609 + miR.603 + miR.520f + miR.220a + miR.130a + miR.29a + miR.369.3p + miR.376a* + miR.494 + miR.518c + miR.660 + miR.338 | 0,81 | 0,117335604 |
| miR.558 + miR.609 + miR.603 + miR.520f + miR.220a + miR.130a + miR.29a + miR.369.3p + miR.376a* + miR.494 + miR.518c + miR.639 + miR.660 | 0,81 | 0,090046396 |
| miR.558 + miR.609 + miR.603 + miR.220a + miR.130a + miR.26a + miR.29a + miR.362 + miR.369.3p + miR.376a* + miR.518c + miR.639 + miR.660 | 0,81 | 0,353748036 |
| miR.558 + miR.609 + miR.603 + miR.220a + miR.519b + miR.26a + miR.29a + miR.362 + miR.369.3p + miR.376a* + miR.494 + miR.518c + miR.338 | 0,81 | 0,114205406 |
| miR.558 + miR.609 + miR.603 + miR.520f + miR.220a + miR.519b + miR.26a + miR.29a + miR.362 + miR.369.3p + miR.376a* + miR.518c + miR.660 | 0,81 | 0,246124567 |
| miR.558 + miR.609 + miR.519b + miR.130a + miR.26a + miR.29a + miR.362 + miR.369.3p + miR.376a* + miR.518c + miR.639 + miR.660 + miR.338 | 0,81 | 0,388923604 |
| miR.558 + miR.609 + miR.603 + miR.520f + miR.220a + miR.519b + miR.29a + miR.362 + miR.369.3p + miR.376a* + miR.494 + miR.518c + miR.338 | 0,81 | 0,10603463 |
| miR.558 + miR.609 + miR.603 + miR.520f + miR.220a + miR.130a + miR.26a + miR.29a + miR.369.3p + miR.376a* + miR.494 + miR.518c + miR.660 | 0,81 | 0,15167349 |
| miR.558 + miR.609 + miR.520f + miR.220a + miR.519b + miR.130a + miR.29a + miR.362 + miR.369.3p + miR.376a* + miR.518c + miR.660 + miR.338 | 0,81 | 0,483032004 |
| miR.558 + miR.609 + miR.520f + miR.220a + miR.519b + miR.26a + miR.29a + miR.362 + miR.369.3p + miR.376a* + miR.518c + miR.660 + miR.338 | 0,81 | 0,483412918 |
| miR.558 + miR.609 + miR.603 + miR.220a + miR.519b + miR.26a + miR.29a + miR.369.3p + miR.376a* + miR.518c + miR.639 + miR.660 + miR.338 | 0,81 | 0,178112812 |
| miR.558 + miR.609 + miR.603 + miR.220a + miR.519b + miR.130a + miR.26a + miR.29a + miR.362 + miR.369.3p + miR.376a* + miR.494 + miR.518c | 0,81 | 0,182035102 |
| miR.558 + miR.609 + miR.520f + miR.220a + miR.130a + miR.26a + miR.29a + miR.362 + miR.369.3p + miR.376a* + miR.518c + miR.660 + miR.338 | 0,81 | 0,496622557 |
| miR.558 + miR.609 + miR.603 + miR.520f + miR.220a + miR.130a + miR.26a + miR.29a + miR.369.3p + miR.376a* + miR.518c + miR.639 + miR.660 | 0,81 | 0,172344989 |
| miR.558 + miR.609 + miR.220a + miR.519b + miR.26a + miR.29a + miR.362 + miR.369.3p + miR.376a* + miR.518c + miR.639 + miR.660 + miR.338 | 0,81 | 0,42999729 |
| miR.558 + miR.609 + miR.603 + miR.520f + miR.519b + miR.29a + miR.369.3p + miR.376a* + miR.494 + miR.518c + miR.639 + miR.660 + miR.338 | 0,81 | 0,063892553 |
| miR.558 + miR.609 + miR.603 + miR.220a + miR.130a + miR.29a + miR.362 + miR.369.3p + miR.376a* + miR.494 + miR.518c + miR.660 + miR.338 | 0,81 | 0,348489444 |
| miR.558 + miR.609 + miR.603 + miR.220a + miR.519b + miR.29a + miR.362 + miR.369.3p + miR.376a* + miR.494 + miR.518c + miR.660 + miR.338 | 0,81 | 0,22534063 |
| miR.558 + miR.609 + miR.519b + miR.130a + miR.29a + miR.362 + miR.369.3p + miR.376a* + miR.494 + miR.518c + miR.639 + miR.660 + miR.338 | 0,81 | 0,172555113 |
| miR.558 + miR.609 + miR.603 + miR.520f + miR.220a + miR.519b + miR.130a + miR.29a + miR.369.3p + miR.376a* + miR.494 + miR.518c + miR.338 | 0,81 | 0,06035927 |
| miR.558 + miR.609 + miR.603 + miR.220a + miR.519b + miR.26a + miR.29a + miR.369.3p + miR.376a* + miR.494 + miR.518c + miR.639 + miR.338 | 0,81 | 0,10391966 |
| miR.558 + miR.609 + miR.603 + miR.520f + miR.130a + miR.29a + miR.369.3p + miR.376a* + miR.494 + miR.518c + miR.639 + miR.660 + miR.338 | 0,81 | 0,021685409 |
| miR.558 + miR.609 + miR.520f + miR.519b + miR.130a + miR.29a + miR.362 + miR.369.3p + miR.376a* + miR.494 + miR.518c + miR.660 + miR.338 | 0,81 | 0,436072031 |
| miR.558 + miR.609 + miR.520f + miR.220a + miR.130a + miR.29a + miR.369.3p + miR.376a* + miR.494 + miR.518c + miR.639 + miR.660 + miR.338 | 0,81 | 0,090304402 |
| miR.558 + miR.609 + miR.520f + miR.220a + miR.519b + miR.29a + miR.369.3p + miR.376a* + miR.494 + miR.518c + miR.639 + miR.660 + miR.338 | 0,81 | 0,262140064 |
| miR.558 + miR.609 + miR.603 + miR.220a + miR.519b + miR.130a + miR.26a + miR.29a + miR.369.3p + miR.376a* + miR.518c + miR.660 + miR.338 | 0,81 | 0,16719454 |
| miR.558 + miR.609 + miR.603 + miR.220a + miR.26a + miR.29a + miR.362 + miR.369.3p + miR.376a* + miR.494 + miR.518c + miR.639 + miR.338 | 0,81 | 0,051418316 |
| miR.558 + miR.609 + miR.603 + miR.520f + miR.220a + miR.26a + miR.29a + miR.362 + miR.369.3p + miR.376a* + miR.518c + miR.639 + miR.338 | 0,81 | 0,051492307 |
| miR.558 + miR.609 + miR.603 + miR.220a + miR.519b + miR.130a + miR.26a + miR.29a + miR.362 + miR.369.3p + miR.376a* + miR.518c + miR.660 | 0,81 | 0,236074713 |
| miR.558 + miR.609 + miR.603 + miR.520f + miR.220a + miR.130a + miR.26a + miR.29a + miR.362 + miR.369.3p + miR.376a* + miR.518c + miR.660 | 0,81 | 0,236869513 |
| miR.558 + miR.609 + miR.603 + miR.220a + miR.519b + miR.29a + miR.362 + miR.369.3p + miR.376a* + miR.494 + miR.518c + miR.639 + miR.660 | 0,81 | 0,190449602 |
| miR.558 + miR.609 + miR.603 + miR.520f + miR.26a + miR.29a + miR.369.3p + miR.376a* + miR.494 + miR.518c + miR.639 + miR.660 + miR.338 | 0,81 | 0,022397978 |
| miR.558 + miR.609 + miR.603 + miR.520f + miR.220a + miR.519b + miR.26a + miR.29a + miR.362 + miR.369.3p + miR.376a* + miR.494 + miR.518c | 0,81 | 0,123919493 |
| miR.558 + miR.609 + miR.520f + miR.130a + miR.26a + miR.29a + miR.362 + miR.369.3p + miR.376a* + miR.494 + miR.518c + miR.660 + miR.338 | 0,81 | 0,356299555 |
| miR.558 + miR.609 + miR.603 + miR.520f + miR.220a + miR.519b + miR.26a + miR.29a + miR.369.3p + miR.376a* + miR.518c + miR.639 + miR.660 | 0,81 | 0,207873921 |
| miR.558 + miR.609 + miR.603 + miR.520f + miR.220a + miR.130a + miR.29a + miR.362 + miR.369.3p + miR.376a* + miR.494 + miR.518c + miR.660 | 0,81 | 0,178581156 |
| miR.558 + miR.609 + miR.603 + miR.520f + miR.26a + miR.29a + miR.362 + miR.369.3p + miR.376a* + miR.494 + miR.518c + miR.660 + miR.338 | 0,81 | 0,238459179 |
| miR.558 + miR.609 + miR.603 + miR.520f + miR.519b + miR.29a + miR.362 + miR.369.3p + miR.376a* + miR.494 + miR.518c + miR.660 + miR.338 | 0,81 | 0,377482029 |
| miR.558 + miR.609 + miR.603 + miR.520f + miR.220a + miR.26a + miR.29a + miR.369.3p + miR.376a* + miR.494 + miR.518c + miR.639 + miR.338 | 0,81 | 0,052528182 |
| miR.558 + miR.609 + miR.603 + miR.220a + miR.519b + miR.29a + miR.362 + miR.369.3p + miR.376a* + miR.518c + miR.639 + miR.660 + miR.338 | 0,81 | 0,305502078 |
| miR.558 + miR.609 + miR.520f + miR.220a + miR.26a + miR.29a + miR.369.3p + miR.376a* + miR.494 + miR.518c + miR.639 + miR.660 + miR.338 | 0,81 | 0,158073054 |
| miR.558 + miR.609 + miR.603 + miR.220a + miR.130a + miR.26a + miR.29a + miR.369.3p + miR.376a* + miR.494 + miR.518c + miR.639 + miR.338 | 0,81 | 0,042969607 |
| miR.558 + miR.609 + miR.603 + miR.520f + miR.220a + miR.519b + miR.130a + miR.29a + miR.362 + miR.369.3p + miR.376a* + miR.494 + miR.518c | 0,81 | 0,135163353 |
| miR.558 + miR.609 + miR.520f + miR.519b + miR.26a + miR.29a + miR.362 + miR.369.3p + miR.376a* + miR.494 + miR.518c + miR.660 + miR.338 | 0,81 | 0,326826099 |
| miR.558 + miR.609 + miR.603 + miR.220a + miR.519b + miR.26a + miR.29a + miR.362 + miR.369.3p + miR.376a* + miR.494 + miR.518c + miR.639 | 0,81 | 0,125139629 |
| miR.558 + miR.609 + miR.603 + miR.220a + miR.519b + miR.130a + miR.26a + miR.29a + miR.369.3p + miR.376a* + miR.494 + miR.518c + miR.639 | 0,81 | 0,156284326 |
| miR.558 + miR.609 + miR.220a + miR.130a + miR.26a + miR.29a + miR.362 + miR.369.3p + miR.376a* + miR.518c + miR.639 + miR.660 + miR.338 | 0,81 | 0,450514876 |
| miR.558 + miR.609 + miR.603 + miR.520f + miR.220a + miR.519b + miR.29a + miR.362 + miR.369.3p + miR.376a* + miR.518c + miR.639 + miR.660 | 0,81 | 0,180818629 |
| miR.558 + miR.609 + miR.603 + miR.520f + miR.220a + miR.26a + miR.29a + miR.362 + miR.369.3p + miR.376a* + miR.494 + miR.518c + miR.338 | 0,81 | 0,035595123 |
| miR.558 + miR.609 + miR.603 + miR.220a + miR.519b + miR.130a + miR.29a + miR.362 + miR.369.3p + miR.376a* + miR.494 + miR.518c + miR.338 | 0,81 | 0,088341056 |
| miR.558 + miR.609 + miR.603 + miR.220a + miR.519b + miR.29a + miR.369.3p + miR.376a* + miR.494 + miR.518c + miR.639 + miR.660 + miR.338 | 0,81 | 0,104019261 |
| miR.558 + miR.609 + miR.603 + miR.520f + miR.220a + miR.519b + miR.130a + miR.26a + miR.29a + miR.369.3p + miR.376a* + miR.494 + miR.518c | 0,81 | 0,136023326 |
| miR.558 + miR.609 + miR.603 + miR.220a + miR.519b + miR.130a + miR.26a + miR.29a + miR.362 + miR.376a* + miR.494 + miR.518c + miR.338 | 0,81 | 0,060958369 |
| miR.558 + miR.609 + miR.603 + miR.130a + miR.26a + miR.29a + miR.362 + miR.369.3p + miR.376a* + miR.494 + miR.518c + miR.639 + miR.660 | 0,81 | 0,041496487 |
| miR.558 + miR.609 + miR.603 + miR.220a + miR.130a + miR.26a + miR.29a + miR.362 + miR.369.3p + miR.376a* + miR.518c + miR.639 + miR.338 | 0,81 | 0,053206862 |
| miR.558 + miR.609 + miR.603 + miR.220a + miR.130a + miR.29a + miR.369.3p + miR.376a* + miR.494 + miR.518c + miR.639 + miR.660 + miR.338 | 0,81 | 0,132410273 |
| miR.558 + miR.609 + miR.603 + miR.520f + miR.130a + miR.26a + miR.29a + miR.362 + miR.369.3p + miR.376a* + miR.518c + miR.660 + miR.338 | 0,81 | 0,168300313 |
| miR.558 + miR.609 + miR.603 + miR.220a + miR.130a + miR.26a + miR.29a + miR.362 + miR.369.3p + miR.376a* + miR.494 + miR.518c + miR.338 | 0,81 | 0,042876004 |
| miR.558 + miR.609 + miR.603 + miR.520f + miR.220a + miR.130a + miR.26a + miR.29a + miR.369.3p + miR.376a* + miR.518c + miR.639 + miR.338 | 0,81 | 0,049783643 |
| miR.558 + miR.609 + miR.603 + miR.520f + miR.220a + miR.130a + miR.26a + miR.29a + miR.362 + miR.369.3p + miR.376a* + miR.518c + miR.338 | 0,81 | 0,039514334 |
| miR.558 + miR.609 + miR.603 + miR.220a + miR.519b + miR.130a + miR.26a + miR.29a + miR.369.3p + miR.376a* + miR.518c + miR.639 + miR.660 | 0,81 | 0,217189728 |
| miR.558 + miR.609 + miR.603 + miR.520f + miR.130a + miR.29a + miR.362 + miR.369.3p + miR.376a* + miR.494 + miR.518c + miR.639 + miR.660 | 0,81 | 0,006068116 |
| miR.558 + miR.609 + miR.520f + miR.519b + miR.130a + miR.29a + miR.369.3p + miR.376a* + miR.494 + miR.518c + miR.639 + miR.660 + miR.338 | 0,81 | 0,120770018 |
| miR.558 + miR.609 + miR.603 + miR.520f + miR.220a + miR.130a + miR.26a + miR.29a + miR.369.3p + miR.376a* + miR.494 + miR.518c + miR.338 | 0,81 | 0,037361085 |
| miR.558 + miR.609 + miR.603 + miR.220a + miR.519b + miR.130a + miR.29a + miR.369.3p + miR.376a* + miR.494 + miR.518c + miR.639 + miR.338 | 0,81 | 0,063650582 |
| miR.558 + miR.609 + miR.603 + miR.220a + miR.519b + miR.130a + miR.26a + miR.29a + miR.362 + miR.376a* + miR.494 + miR.518c + miR.639 | 0,81 | 0,082538779 |
| miR.558 + miR.609 + miR.603 + miR.220a + miR.519b + miR.130a + miR.26a + miR.29a + miR.362 + miR.376a* + miR.494 + miR.518c + miR.660 | 0,81 | 0,111258667 |
| miR.558 + miR.609 + miR.603 + miR.520f + miR.220a + miR.130a + miR.29a + miR.362 + miR.369.3p + miR.376a* + miR.518c + miR.639 + miR.660 | 0,81 | 0,146308564 |
| miR.558 + miR.609 + miR.603 + miR.520f + miR.220a + miR.519b + miR.130a + miR.26a + miR.29a + miR.369.3p + miR.376a* + miR.518c + miR.660 | 0,81 | 0,217635541 |
| miR.558 + miR.609 + miR.603 + miR.520f + miR.220a + miR.519b + miR.29a + miR.362 + miR.369.3p + miR.376a* + miR.494 + miR.518c + miR.639 | 0,81 | 0,124531345 |
| miR.558 + miR.609 + miR.603 + miR.220a + miR.130a + miR.26a + miR.29a + miR.362 + miR.369.3p + miR.376a* + miR.494 + miR.518c + miR.639 | 0,81 | 0,070738674 |
| miR.558 + miR.609 + miR.603 + miR.220a + miR.519b + miR.130a + miR.29a + miR.362 + miR.369.3p + miR.376a* + miR.494 + miR.518c + miR.639 | 0,81 | 0,104649693 |
| miR.558 + miR.609 + miR.603 + miR.520f + miR.130a + miR.29a + miR.362 + miR.369.3p + miR.376a* + miR.494 + miR.518c + miR.660 + miR.338 | 0,81 | 0,32638794 |
| miR.558 + miR.609 + miR.603 + miR.220a + miR.519b + miR.130a + miR.29a + miR.362 + miR.369.3p + miR.376a* + miR.518c + miR.639 + miR.660 | 0,81 | 0,237519424 |
| miR.558 + miR.609 + miR.603 + miR.220a + miR.519b + miR.130a + miR.29a + miR.369.3p + miR.376a* + miR.518c + miR.639 + miR.660 + miR.338 | 0,81 | 0,16109912 |
| miR.558 + miR.609 + miR.603 + miR.520f + miR.519b + miR.26a + miR.29a + miR.362 + miR.369.3p + miR.376a* + miR.518c + miR.660 + miR.338 | 0,81 | 0,144140479 |
| miR.558 + miR.609 + miR.220a + miR.130a + miR.26a + miR.29a + miR.362 + miR.369.3p + miR.376a* + miR.494 + miR.518c + miR.660 + miR.338 | 0,81 | 0,373888812 |
| miR.558 + miR.609 + miR.520f + miR.220a + miR.519b + miR.26a + miR.29a + miR.369.3p + miR.376a* + miR.494 + miR.518c + miR.660 + miR.338 | 0,81 | 0,22144321 |
| miR.558 + miR.609 + miR.603 + miR.520f + miR.519b + miR.26a + miR.29a + miR.369.3p + miR.376a* + miR.518c + miR.639 + miR.660 + miR.338 | 0,81 | 0,050223589 |
| miR.558 + miR.609 + miR.520f + miR.220a + miR.519b + miR.130a + miR.29a + miR.369.3p + miR.376a* + miR.494 + miR.518c + miR.660 + miR.338 | 0,81 | 0,228984452 |
| miR.558 + miR.609 + miR.520f + miR.220a + miR.519b + miR.26a + miR.29a + miR.369.3p + miR.376a* + miR.518c + miR.639 + miR.660 + miR.338 | 0,81 | 0,202590909 |
| miR.558 + miR.609 + miR.603 + miR.520f + miR.220a + miR.519b + miR.130a + miR.29a + miR.369.3p + miR.376a* + miR.518c + miR.639 + miR.660 | 0,81 | 0,198405441 |
| miR.558 + miR.609 + miR.603 + miR.520f + miR.220a + miR.519b + miR.26a + miR.29a + miR.369.3p + miR.376a* + miR.494 + miR.518c + miR.639 | 0,81 | 0,107884424 |
| miR.558 + miR.609 + miR.603 + miR.220a + miR.519b + miR.130a + miR.29a + miR.362 + miR.369.3p + miR.376a* + miR.518c + miR.660 + miR.338 | 0,81 | 0,399603495 |
| miR.558 + miR.609 + miR.520f + miR.130a + miR.26a + miR.29a + miR.369.3p + miR.376a* + miR.494 + miR.518c + miR.639 + miR.660 + miR.338 | 0,81 | 0,151648953 |
| miR.558 + miR.609 + miR.603 + miR.519b + miR.130a + miR.29a + miR.362 + miR.369.3p + miR.376a* + miR.518c + miR.639 + miR.660 + miR.338 | 0,81 | 0,146726442 |
| miR.558 + miR.609 + miR.603 + miR.520f + miR.220a + miR.519b + miR.26a + miR.29a + miR.362 + miR.369.3p + miR.376a* + miR.518c + miR.338 | 0,81 | 0,116312649 |
| miR.558 + miR.609 + miR.603 + miR.220a + miR.519b + miR.130a + miR.26a + miR.29a + miR.362 + miR.369.3p + miR.376a* + miR.518c + miR.639 | 0,81 | 0,111737823 |
| miR.558 + miR.609 + miR.603 + miR.520f + miR.519b + miR.130a + miR.29a + miR.362 + miR.369.3p + miR.376a* + miR.518c + miR.660 + miR.338 | 0,81 | 0,217527688 |
| miR.558 + miR.609 + miR.603 + miR.130a + miR.26a + miR.29a + miR.369.3p + miR.376a* + miR.494 + miR.518c + miR.639 + miR.660 + miR.338 | 0,81 | 0,137020658 |
| miR.558 + miR.609 + miR.603 + miR.220a + miR.29a + miR.362 + miR.369.3p + miR.376a* + miR.494 + miR.518c + miR.639 + miR.660 + miR.338 | 0,81 | 0,287344575 |
| miR.558 + miR.609 + miR.603 + miR.520f + miR.220a + miR.26a + miR.29a + miR.362 + miR.369.3p + miR.376a* + miR.494 + miR.518c + miR.639 | 0,81 | 0,076865744 |
| miR.558 + miR.609 + miR.603 + miR.520f + miR.220a + miR.519b + miR.130a + miR.29a + miR.369.3p + miR.376a* + miR.494 + miR.518c + miR.639 | 0,81 | 0,095765593 |
| miR.558 + miR.609 + miR.603 + miR.520f + miR.220a + miR.519b + miR.130a + miR.29a + miR.362 + miR.369.3p + miR.376a* + miR.518c + miR.660 | 0,81 | 0,233473801 |
| miR.558 + miR.609 + miR.220a + miR.519b + miR.26a + miR.29a + miR.362 + miR.369.3p + miR.376a* + miR.494 + miR.518c + miR.660 + miR.338 | 0,81 | 0,439970485 |
| miR.558 + miR.609 + miR.603 + miR.520f + miR.130a + miR.26a + miR.29a + miR.369.3p + miR.376a* + miR.518c + miR.639 + miR.660 + miR.338 | 0,81 | 0,0322276 |
| miR.558 + miR.609 + miR.603 + miR.520f + miR.220a + miR.130a + miR.26a + miR.29a + miR.362 + miR.369.3p + miR.376a* + miR.518c + miR.639 | 0,81 | 0,091894888 |
| miR.558 + miR.609 + miR.520f + miR.220a + miR.130a + miR.26a + miR.29a + miR.369.3p + miR.376a* + miR.518c + miR.639 + miR.660 + miR.338 | 0,81 | 0,168935619 |
| miR.558 + miR.609 + miR.603 + miR.520f + miR.220a + miR.130a + miR.26a + miR.29a + miR.362 + miR.369.3p + miR.376a* + miR.494 + miR.518c | 0,81 | 0,062361826 |
| miR.558 + miR.609 + miR.603 + miR.520f + miR.519b + miR.130a + miR.29a + miR.369.3p + miR.376a* + miR.518c + miR.639 + miR.660 + miR.338 | 0,81 | 0,057639073 |
| miR.558 + miR.609 + miR.220a + miR.519b + miR.130a + miR.29a + miR.362 + miR.369.3p + miR.376a* + miR.518c + miR.639 + miR.660 + miR.338 | 0,81 | 0,442563861 |
| miR.558 + miR.609 + miR.603 + miR.520f + miR.220a + miR.519b + miR.26a + miR.29a + miR.362 + miR.369.3p + miR.376a* + miR.518c + miR.639 | 0,81 | 0,142333583 |
| miR.558 + miR.609 + miR.603 + miR.520f + miR.220a + miR.519b + miR.26a + miR.29a + miR.369.3p + miR.376a* + miR.518c + miR.639 + miR.338 | 0,81 | 0,083427866 |
| miR.558 + miR.609 + miR.519b + miR.26a + miR.29a + miR.362 + miR.369.3p + miR.376a* + miR.494 + miR.518c + miR.639 + miR.660 + miR.338 | 0,81 | 0,225943814 |
| miR.558 + miR.609 + miR.220a + miR.130a + miR.26a + miR.29a + miR.362 + miR.369.3p + miR.376a* + miR.494 + miR.518c + miR.639 + miR.660 | 0,81 | 0,220355412 |
| miR.558 + miR.609 + miR.603 + miR.520f + miR.220a + miR.519b + miR.29a + miR.376a* + miR.494 + miR.518c + miR.639 + miR.660 + miR.338 | 0,81 | 0,062678291 |
| miR.558 + miR.609 + miR.603 + miR.520f + miR.220a + miR.519b + miR.29a + miR.362 + miR.369.3p + miR.376a* + miR.518c + miR.639 + miR.338 | 0,81 | 0,096458624 |
| miR.558 + miR.609 + miR.520f + miR.220a + miR.26a + miR.29a + miR.362 + miR.369.3p + miR.376a* + miR.494 + miR.518c + miR.639 + miR.660 | 0,81 | 0,133522478 |
| miR.558 + miR.609 + miR.603 + miR.520f + miR.220a + miR.130a + miR.26a + miR.29a + miR.369.3p + miR.376a* + miR.494 + miR.518c + miR.639 | 0,81 | 0,057173785 |
| miR.558 + miR.609 + miR.603 + miR.520f + miR.220a + miR.519b + miR.130a + miR.26a + miR.29a + miR.369.3p + miR.376a* + miR.518c + miR.338 | 0,81 | 0,091936433 |
| miR.558 + miR.609 + miR.520f + miR.220a + miR.130a + miR.29a + miR.362 + miR.369.3p + miR.376a* + miR.494 + miR.518c + miR.639 + miR.660 | 0,81 | 0,137126068 |
| miR.558 + miR.609 + miR.520f + miR.220a + miR.519b + miR.130a + miR.29a + miR.369.3p + miR.376a* + miR.518c + miR.639 + miR.660 + miR.338 | 0,81 | 0,170485549 |
| miR.558 + miR.609 + miR.220a + miR.130a + miR.26a + miR.29a + miR.369.3p + miR.376a* + miR.494 + miR.518c + miR.639 + miR.660 + miR.338 | 0,81 | 0,209177085 |
| miR.558 + miR.609 + miR.603 + miR.520f + miR.220a + miR.519b + miR.130a + miR.26a + miR.29a + miR.362 + miR.369.3p + miR.376a* + miR.518c | 0,81 | 0,140365434 |
| miR.558 + miR.609 + miR.603 + miR.520f + miR.220a + miR.519b + miR.130a + miR.29a + miR.362 + miR.376a* + miR.494 + miR.518c + miR.338 | 0,81 | 0,053398377 |
| miR.558 + miR.609 + miR.603 + miR.520f + miR.220a + miR.519b + miR.130a + miR.29a + miR.376a* + miR.494 + miR.518c + miR.660 + miR.338 | 0,81 | 0,084042868 |
| miR.558 + miR.609 + miR.603 + miR.520f + miR.220a + miR.519b + miR.29a + miR.369.3p + miR.376a* + miR.494 + miR.518c + miR.639 + miR.338 | 0,81 | 0,083550012 |
| miR.558 + miR.609 + miR.603 + miR.520f + miR.220a + miR.519b + miR.130a + miR.29a + miR.362 + miR.369.3p + miR.376a* + miR.518c + miR.338 | 0,81 | 0,0905644 |
| miR.558 + miR.609 + miR.603 + miR.520f + miR.519b + miR.26a + miR.29a + miR.369.3p + miR.376a* + miR.494 + miR.518c + miR.660 + miR.338 | 0,81 | 0,095416778 |
| miR.558 + miR.609 + miR.603 + miR.220a + miR.519b + miR.130a + miR.29a + miR.362 + miR.376a* + miR.494 + miR.518c + miR.639 + miR.338 | 0,81 | 0,056415696 |
| miR.558 + miR.609 + miR.603 + miR.520f + miR.519b + miR.130a + miR.29a + miR.369.3p + miR.376a* + miR.494 + miR.518c + miR.660 + miR.338 | 0,81 | 0,075606555 |
| miR.558 + miR.609 + miR.603 + miR.519b + miR.26a + miR.29a + miR.362 + miR.369.3p + miR.376a* + miR.494 + miR.518c + miR.660 + miR.338 | 0,81 | 0,149873902 |
| miR.558 + miR.609 + miR.603 + miR.220a + miR.519b + miR.130a + miR.29a + miR.362 + miR.376a* + miR.494 + miR.518c + miR.639 + miR.660 | 0,81 | 0,10234866 |
| miR.558 + miR.609 + miR.603 + miR.220a + miR.519b + miR.26a + miR.29a + miR.362 + miR.369.3p + miR.376a* + miR.518c + miR.639 + miR.338 | 0,81 | 0,1095917 |
| miR.558 + miR.609 + miR.603 + miR.520f + miR.220a + miR.519b + miR.29a + miR.362 + miR.376a* + miR.494 + miR.518c + miR.660 + miR.338 | 0,81 | 0,105958858 |
| miR.558 + miR.609 + miR.603 + miR.520f + miR.220a + miR.519b + miR.130a + miR.29a + miR.362 + miR.376a* + miR.494 + miR.518c + miR.639 | 0,81 | 0,097183864 |
| miR.558 + miR.609 + miR.603 + miR.130a + miR.26a + miR.29a + miR.362 + miR.369.3p + miR.376a* + miR.494 + miR.518c + miR.660 + miR.338 | 0,81 | 0,094975348 |
| miR.558 + miR.609 + miR.603 + miR.220a + miR.519b + miR.130a + miR.26a + miR.29a + miR.376a* + miR.494 + miR.518c + miR.660 + miR.338 | 0,81 | 0,067932326 |
| miR.558 + miR.609 + miR.603 + miR.520f + miR.220a + miR.519b + miR.26a + miR.29a + miR.376a* + miR.494 + miR.518c + miR.660 + miR.338 | 0,81 | 0,10344806 |
| miR.558 + miR.609 + miR.603 + miR.520f + miR.220a + miR.519b + miR.130a + miR.26a + miR.29a + miR.362 + miR.376a* + miR.494 + miR.518c | 0,81 | 0,097108796 |
| miR.558 + miR.609 + miR.603 + miR.520f + miR.130a + miR.26a + miR.29a + miR.369.3p + miR.376a* + miR.494 + miR.518c + miR.639 + miR.338 | 0,81 | 0,034401585 |
| miR.558 + miR.609 + miR.603 + miR.520f + miR.519b + miR.130a + miR.29a + miR.369.3p + miR.376a* + miR.494 + miR.518c + miR.639 + miR.338 | 0,81 | 0,054079815 |
| miR.558 + miR.609 + miR.520f + miR.220a + miR.130a + miR.26a + miR.29a + miR.369.3p + miR.376a* + miR.494 + miR.518c + miR.660 + miR.338 | 0,81 | 0,106293041 |
| miR.558 + miR.609 + miR.603 + miR.520f + miR.220a + miR.519b + miR.29a + miR.362 + miR.376a* + miR.494 + miR.518c + miR.639 + miR.338 | 0,81 | 0,068104962 |
| miR.558 + miR.609 + miR.603 + miR.220a + miR.519b + miR.130a + miR.26a + miR.29a + miR.376a* + miR.494 + miR.518c + miR.639 + miR.660 | 0,81 | 0,109350789 |
| miR.558 + miR.609 + miR.603 + miR.220a + miR.519b + miR.130a + miR.26a + miR.29a + miR.362 + miR.369.3p + miR.376a* + miR.518c + miR.338 | 0,81 | 0,115746824 |
| miR.558 + miR.609 + miR.603 + miR.520f + miR.220a + miR.130a + miR.29a + miR.362 + miR.369.3p + miR.376a* + miR.494 + miR.518c + miR.338 | 0,81 | 0,042546222 |
| miR.558 + miR.609 + miR.603 + miR.520f + miR.220a + miR.519b + miR.26a + miR.29a + miR.362 + miR.376a* + miR.494 + miR.518c + miR.338 | 0,81 | 0,077476199 |
| miR.558 + miR.609 + miR.603 + miR.519b + miR.130a + miR.26a + miR.29a + miR.369.3p + miR.376a* + miR.494 + miR.518c + miR.639 + miR.338 | 0,81 | 0,093186559 |
| miR.558 + miR.609 + miR.603 + miR.520f + miR.220a + miR.519b + miR.130a + miR.29a + miR.362 + miR.376a* + miR.494 + miR.518c + miR.660 | 0,81 | 0,151147002 |
| miR.558 + miR.609 + miR.220a + miR.519b + miR.130a + miR.29a + miR.362 + miR.369.3p + miR.376a* + miR.494 + miR.518c + miR.660 + miR.338 | 0,81 | 0,27313387 |
| miR.558 + miR.609 + miR.603 + miR.220a + miR.519b + miR.130a + miR.26a + miR.29a + miR.376a* + miR.494 + miR.518c + miR.639 + miR.338 | 0,81 | 0,031402705 |
| miR.558 + miR.609 + miR.603 + miR.520f + miR.220a + miR.519b + miR.130a + miR.29a + miR.376a* + miR.494 + miR.518c + miR.639 + miR.660 | 0,81 | 0,108309166 |
| miR.558 + miR.609 + miR.520f + miR.220a + miR.130a + miR.26a + miR.29a + miR.369.3p + miR.376a* + miR.494 + miR.518c + miR.639 + miR.660 | 0,81 | 0,108296254 |
| miR.558 + miR.609 + miR.603 + miR.520f + miR.26a + miR.29a + miR.362 + miR.369.3p + miR.376a* + miR.494 + miR.518c + miR.639 + miR.338 | 0,81 | 0,026737371 |
| miR.558 + miR.609 + miR.603 + miR.220a + miR.519b + miR.130a + miR.26a + miR.29a + miR.369.3p + miR.376a* + miR.518c + miR.639 + miR.338 | 0,81 | 0,122408948 |
| miR.558 + miR.609 + miR.520f + miR.519b + miR.130a + miR.26a + miR.29a + miR.369.3p + miR.376a* + miR.518c + miR.639 + miR.660 + miR.338 | 0,81 | 0,10651772 |
| miR.558 + miR.609 + miR.520f + miR.130a + miR.26a + miR.29a + miR.362 + miR.369.3p + miR.376a* + miR.494 + miR.518c + miR.639 + miR.660 | 0,81 | 0,034075449 |
| miR.558 + miR.609 + miR.520f + miR.220a + miR.519b + miR.26a + miR.29a + miR.362 + miR.369.3p + miR.376a* + miR.518c + miR.639 + miR.660 | 0,81 | 0,204425213 |
| miR.558 + miR.609 + miR.603 + miR.220a + miR.519b + miR.26a + miR.29a + miR.362 + miR.376a* + miR.494 + miR.518c + miR.639 + miR.338 | 0,81 | 0,092015336 |
| miR.558 + miR.609 + miR.603 + miR.520f + miR.220a + miR.519b + miR.130a + miR.29a + miR.369.3p + miR.376a* + miR.518c + miR.639 + miR.338 | 0,81 | 0,064440424 |
| miR.558 + miR.609 + miR.603 + miR.520f + miR.220a + miR.130a + miR.29a + miR.362 + miR.369.3p + miR.376a* + miR.494 + miR.518c + miR.639 | 0,81 | 0,046519555 |
| miR.558 + miR.609 + miR.520f + miR.220a + miR.519b + miR.29a + miR.362 + miR.369.3p + miR.376a* + miR.494 + miR.518c + miR.639 + miR.660 | 0,81 | 0,187256281 |
| miR.558 + miR.609 + miR.603 + miR.520f + miR.220a + miR.519b + miR.130a + miR.29a + miR.362 + miR.369.3p + miR.376a* + miR.518c + miR.639 | 0,81 | 0,100998799 |
| miR.558 + miR.609 + miR.520f + miR.220a + miR.519b + miR.26a + miR.29a + miR.362 + miR.369.3p + miR.376a* + miR.494 + miR.518c + miR.660 | 0,81 | 0,226326011 |
| miR.558 + miR.609 + miR.520f + miR.519b + miR.130a + miR.29a + miR.362 + miR.369.3p + miR.376a* + miR.494 + miR.518c + miR.639 + miR.660 | 0,81 | 0,009030937 |
| miR.558 + miR.609 + miR.603 + miR.520f + miR.220a + miR.29a + miR.362 + miR.369.3p + miR.376a* + miR.494 + miR.518c + miR.639 + miR.338 | 0,81 | 0,049864978 |
| miR.558 + miR.609 + miR.519b + miR.130a + miR.26a + miR.29a + miR.362 + miR.369.3p + miR.376a* + miR.494 + miR.518c + miR.639 + miR.660 | 0,81 | 0,066783621 |
| miR.558 + miR.609 + miR.603 + miR.520f + miR.220a + miR.519b + miR.130a + miR.26a + miR.29a + miR.369.3p + miR.376a* + miR.518c + miR.639 | 0,81 | 0,118813626 |
| miR.558 + miR.609 + miR.603 + miR.520f + miR.220a + miR.29a + miR.362 + miR.376a* + miR.494 + miR.518c + miR.639 + miR.660 + miR.338 | 0,81 | 0,030176993 |
| miR.558 + miR.609 + miR.603 + miR.520f + miR.220a + miR.519b + miR.130a + miR.26a + miR.29a + miR.376a* + miR.494 + miR.518c + miR.660 | 0,81 | 0,123637498 |
| miR.558 + miR.609 + miR.520f + miR.220a + miR.519b + miR.130a + miR.26a + miR.29a + miR.369.3p + miR.376a* + miR.518c + miR.660 + miR.338 | 0,81 | 0,192757297 |
| miR.558 + miR.609 + miR.603 + miR.220a + miR.519b + miR.130a + miR.29a + miR.362 + miR.376a* + miR.494 + miR.518c + miR.660 + miR.338 | 0,81 | 0,062264897 |
| miR.558 + miR.609 + miR.520f + miR.519b + miR.130a + miR.26a + miR.29a + miR.362 + miR.369.3p + miR.376a* + miR.518c + miR.660 + miR.338 | 0,81 | 0,31004701 |
| miR.558 + miR.609 + miR.603 + miR.520f + miR.220a + miR.519b + miR.26a + miR.29a + miR.362 + miR.376a* + miR.494 + miR.518c + miR.639 | 0,81 | 0,130242102 |
| miR.558 + miR.609 + miR.603 + miR.520f + miR.220a + miR.26a + miR.29a + miR.376a* + miR.494 + miR.518c + miR.639 + miR.660 + miR.338 | 0,81 | 0,029732961 |
| miR.558 + miR.609 + miR.520f + miR.220a + miR.130a + miR.26a + miR.29a + miR.362 + miR.369.3p + miR.376a* + miR.494 + miR.518c + miR.660 | 0,81 | 0,241293383 |
| miR.558 + miR.609 + miR.603 + miR.520f + miR.220a + miR.130a + miR.29a + miR.376a* + miR.494 + miR.518c + miR.639 + miR.660 + miR.338 | 0,81 | 0,026158188 |
| miR.558 + miR.609 + miR.603 + miR.220a + miR.519b + miR.130a + miR.29a + miR.376a* + miR.494 + miR.518c + miR.639 + miR.660 + miR.338 | 0,81 | 0,054322724 |
| miR.558 + miR.609 + miR.603 + miR.220a + miR.519b + miR.130a + miR.29a + miR.362 + miR.369.3p + miR.376a* + miR.518c + miR.639 + miR.338 | 0,81 | 0,097413097 |
| miR.558 + miR.609 + miR.603 + miR.520f + miR.130a + miR.29a + miR.362 + miR.369.3p + miR.376a* + miR.494 + miR.518c + miR.639 + miR.338 | 0,81 | 0,023024307 |
| miR.558 + miR.609 + miR.220a + miR.519b + miR.26a + miR.29a + miR.362 + miR.369.3p + miR.376a* + miR.494 + miR.518c + miR.639 + miR.660 | 0,81 | 0,200981447 |
| miR.558 + miR.609 + miR.220a + miR.519b + miR.130a + miR.29a + miR.362 + miR.369.3p + miR.376a* + miR.494 + miR.518c + miR.639 + miR.660 | 0,81 | 0,189218997 |
| miR.558 + miR.609 + miR.220a + miR.519b + miR.130a + miR.26a + miR.29a + miR.362 + miR.369.3p + miR.376a* + miR.518c + miR.660 + miR.338 | 0,81 | 0,371816229 |
| miR.558 + miR.609 + miR.220a + miR.519b + miR.130a + miR.26a + miR.29a + miR.369.3p + miR.376a* + miR.494 + miR.518c + miR.639 + miR.660 | 0,81 | 0,257892045 |
| miR.558 + miR.609 + miR.603 + miR.220a + miR.130a + miR.26a + miR.29a + miR.376a* + miR.494 + miR.518c + miR.639 + miR.660 + miR.338 | 0,81 | 0,048311512 |
| miR.558 + miR.609 + miR.220a + miR.519b + miR.130a + miR.26a + miR.29a + miR.369.3p + miR.376a* + miR.518c + miR.639 + miR.660 + miR.338 | 0,81 | 0,223974146 |
| miR.558 + miR.609 + miR.603 + miR.519b + miR.130a + miR.29a + miR.369.3p + miR.376a* + miR.494 + miR.518c + miR.639 + miR.660 + miR.338 | 0,81 | 0,104633759 |
| miR.558 + miR.609 + miR.520f + miR.220a + miR.519b + miR.130a + miR.29a + miR.362 + miR.369.3p + miR.376a* + miR.494 + miR.518c + miR.660 | 0,81 | 0,31524463 |
| miR.558 + miR.609 + miR.520f + miR.519b + miR.26a + miR.29a + miR.369.3p + miR.376a* + miR.494 + miR.518c + miR.639 + miR.660 + miR.338 | 0,81 | 0,169572806 |
| miR.558 + miR.609 + miR.520f + miR.220a + miR.519b + miR.26a + miR.29a + miR.369.3p + miR.376a* + miR.494 + miR.518c + miR.639 + miR.660 | 0,81 | 0,150095078 |
| miR.558 + miR.609 + miR.603 + miR.520f + miR.220a + miR.130a + miR.29a + miR.362 + miR.369.3p + miR.376a* + miR.518c + miR.639 + miR.338 | 0,81 | 0,052605777 |
| miR.558 + miR.609 + miR.603 + miR.520f + miR.130a + miR.26a + miR.29a + miR.362 + miR.369.3p + miR.376a* + miR.518c + miR.639 + miR.338 | 0,81 | 0,031102589 |
| miR.558 + miR.609 + miR.603 + miR.519b + miR.130a + miR.26a + miR.29a + miR.369.3p + miR.376a* + miR.518c + miR.639 + miR.660 + miR.338 | 0,81 | 0,071281412 |
| miR.558 + miR.609 + miR.220a + miR.519b + miR.26a + miR.29a + miR.369.3p + miR.376a* + miR.494 + miR.518c + miR.639 + miR.660 + miR.338 | 0,81 | 0,232704663 |
| miR.558 + miR.609 + miR.603 + miR.130a + miR.26a + miR.29a + miR.362 + miR.369.3p + miR.376a* + miR.494 + miR.518c + miR.639 + miR.338 | 0,81 | 0,088718228 |
| miR.558 + miR.609 + miR.603 + miR.520f + miR.220a + miR.130a + miR.29a + miR.369.3p + miR.376a* + miR.494 + miR.518c + miR.639 + miR.338 | 0,81 | 0,033031201 |
| miR.609 + miR.603 + miR.520f + miR.220a + miR.29a + miR.362 + miR.369.3p + miR.376a* + miR.494 + miR.518c + miR.639 + miR.660 + miR.338 | 0,81 | 0,386426673 |
| miR.558 + miR.609 + miR.520f + miR.519b + miR.130a + miR.26a + miR.29a + miR.369.3p + miR.376a* + miR.494 + miR.518c + miR.660 + miR.338 | 0,81 | 0,25269539 |
| miR.558 + miR.609 + miR.603 + miR.520f + miR.130a + miR.26a + miR.29a + miR.369.3p + miR.376a* + miR.494 + miR.518c + miR.660 + miR.338 | 0,81 | 0,072920527 |
| miR.558 + miR.609 + miR.603 + miR.520f + miR.220a + miR.519b + miR.29a + miR.362 + miR.376a* + miR.494 + miR.518c + miR.639 + miR.660 | 0,81 | 0,121094502 |
| miR.558 + miR.609 + miR.520f + miR.220a + miR.130a + miR.26a + miR.29a + miR.362 + miR.369.3p + miR.376a* + miR.518c + miR.639 + miR.660 | 0,81 | 0,242402407 |
| miR.558 + miR.609 + miR.603 + miR.520f + miR.26a + miR.29a + miR.362 + miR.369.3p + miR.376a* + miR.494 + miR.518c + miR.639 + miR.660 | 0,81 | 0,015891226 |
| miR.558 + miR.609 + miR.603 + miR.220a + miR.26a + miR.29a + miR.362 + miR.376a* + miR.494 + miR.518c + miR.639 + miR.660 + miR.338 | 0,81 | 0,054315882 |
| miR.558 + miR.609 + miR.603 + miR.520f + miR.130a + miR.26a + miR.29a + miR.369.3p + miR.376a* + miR.494 + miR.518c + miR.639 + miR.660 | 0,81 | 0,039648106 |
| miR.558 + miR.609 + miR.603 + miR.519b + miR.130a + miR.26a + miR.29a + miR.362 + miR.369.3p + miR.376a* + miR.518c + miR.660 + miR.338 | 0,81 | 0,09555985 |
| miR.558 + miR.609 + miR.603 + miR.519b + miR.26a + miR.29a + miR.362 + miR.369.3p + miR.376a* + miR.494 + miR.518c + miR.639 + miR.660 | 0,81 | 0,022074144 |
| miR.558 + miR.609 + miR.220a + miR.519b + miR.130a + miR.26a + miR.29a + miR.369.3p + miR.376a* + miR.494 + miR.518c + miR.660 + miR.338 | 0,81 | 0,254826732 |
| miR.558 + miR.609 + miR.603 + miR.520f + miR.519b + miR.26a + miR.29a + miR.369.3p + miR.376a* + miR.494 + miR.518c + miR.639 + miR.338 | 0,81 | 0,056003775 |
| miR.558 + miR.609 + miR.220a + miR.519b + miR.130a + miR.26a + miR.29a + miR.362 + miR.369.3p + miR.376a* + miR.494 + miR.518c + miR.660 | 0,81 | 0,345123625 |
| miR.558 + miR.609 + miR.520f + miR.220a + miR.519b + miR.130a + miR.26a + miR.29a + miR.369.3p + miR.376a* + miR.494 + miR.518c + miR.660 | 0,81 | 0,244119723 |
| miR.558 + miR.609 + miR.603 + miR.520f + miR.220a + miR.519b + miR.26a + miR.29a + miR.376a* + miR.494 + miR.518c + miR.639 + miR.660 | 0,81 | 0,117388442 |
| miR.558 + miR.609 + miR.603 + miR.520f + miR.220a + miR.519b + miR.26a + miR.29a + miR.362 + miR.376a* + miR.494 + miR.518c + miR.660 | 0,81 | 0,144644235 |
| miR.558 + miR.609 + miR.603 + miR.519b + miR.130a + miR.26a + miR.29a + miR.369.3p + miR.376a* + miR.494 + miR.518c + miR.639 + miR.660 | 0,81 | 0,097466046 |
| miR.558 + miR.609 + miR.603 + miR.520f + miR.519b + miR.130a + miR.26a + miR.29a + miR.369.3p + miR.376a* + miR.518c + miR.660 + miR.338 | 0,81 | 0,120253234 |
| miR.558 + miR.609 + miR.603 + miR.520f + miR.519b + miR.130a + miR.26a + miR.29a + miR.369.3p + miR.376a* + miR.494 + miR.518c + miR.338 | 0,81 | 0,049964592 |
| miR.558 + miR.609 + miR.520f + miR.220a + miR.519b + miR.130a + miR.29a + miR.369.3p + miR.376a* + miR.494 + miR.518c + miR.639 + miR.660 | 0,81 | 0,175789263 |
| miR.558 + miR.609 + miR.603 + miR.220a + miR.519b + miR.26a + miR.29a + miR.362 + miR.376a* + miR.494 + miR.518c + miR.660 + miR.338 | 0,81 | 0,102398037 |
| miR.558 + miR.609 + miR.603 + miR.520f + miR.220a + miR.26a + miR.29a + miR.362 + miR.376a* + miR.494 + miR.518c + miR.639 + miR.338 | 0,81 | 0,015203128 |
| miR.558 + miR.609 + miR.603 + miR.520f + miR.519b + miR.29a + miR.362 + miR.369.3p + miR.376a* + miR.494 + miR.518c + miR.639 + miR.338 | 0,81 | 0,045984998 |
| miR.558 + miR.609 + miR.603 + miR.520f + miR.519b + miR.130a + miR.29a + miR.362 + miR.369.3p + miR.376a* + miR.494 + miR.518c + miR.338 | 0,81 | 0,045712288 |
| miR.558 + miR.609 + miR.603 + miR.220a + miR.130a + miR.26a + miR.29a + miR.362 + miR.376a* + miR.494 + miR.518c + miR.639 + miR.660 | 0,81 | 0,046429079 |
| miR.558 + miR.609 + miR.603 + miR.220a + miR.519b + miR.29a + miR.362 + miR.369.3p + miR.376a* + miR.494 + miR.518c + miR.639 + miR.338 | 0,81 | 0,090865721 |
| miR.558 + miR.609 + miR.603 + miR.220a + miR.130a + miR.29a + miR.362 + miR.369.3p + miR.376a* + miR.494 + miR.518c + miR.639 + miR.338 | 0,81 | 0,0269 8096 |
| miR.558 + miR.609 + miR.603 + miR.520f + miR.519b + miR.130a + miR.29a + miR.369.3p + miR.376a* + miR.494 + miR.518c + miR.639 + miR.660 | 0,81 | 0,05172435 |
| miR.558 + miR.609 + miR.603 + miR.220a + miR.130a + miR.26a + miR.29a + miR.362 + miR.376a* + miR.494 + miR.518c + miR.639 + miR.338 | 0,81 | 0,033364716 |
| miR.558 + miR.609 + miR.603 + miR.520f + miR.519b + miR.26a + miR.29a + miR.362 + miR.369.3p + miR.376a* + miR.518c + miR.639 + miR.338 | 0,81 | 0,04386086 |
| miR.558 + miR.609 + miR.603 + miR.520f + miR.519b + miR.26a + miR.29a + miR.362 + miR.369.3p + miR.376a* + miR.494 + miR.518c + miR.338 | 0,81 | 0,057183959 |
| miR.558 + miR.609 + miR.520f + miR.220a + miR.26a + miR.29a + miR.362 + miR.369.3p + miR.376a* + miR.494 + miR.518c + miR.639 + miR.338 | 0,81 | 0,045132674 |
| miR.558 + miR.609 + miR.603 + miR.520f + miR.519b + miR.26a + miR.29a + miR.362 + miR.369.3p + miR.376a* + miR.518c + miR.639 + miR.660 | 0,81 | 0,018395196 |
| miR.609 + miR.603 + miR.520f + miR.220a + miR.519b + miR.29a + miR.362 + miR.369.3p + miR.376a* + miR.494 + miR.518c + miR.660 + miR.338 | 0,81 | 0,459736758 |
| miR.558 + miR.609 + miR.603 + miR.220a + miR.519b + miR.26a + miR.29a + miR.376a* + miR.494 + miR.518c + miR.639 + miR.660 + miR.338 | 0,81 | 0,088404933 |
| miR.558 + miR.609 + miR.603 + miR.520f + miR.220a + miR.519b + miR.26a + miR.29a + miR.362 + miR.376a* + miR.518c + miR.639 + miR.338 | 0,81 | 0,080411041 |
| miR.558 + miR.609 + miR.520f + miR.220a + miR.130a + miR.26a + miR.29a + miR.369.3p + miR.376a* + miR.494 + miR.518c + miR.639 + miR.338 | 0,81 | 0,045221056 |
| miR.558 + miR.609 + miR.603 + miR.520f + miR.519b + miR.130a + miR.26a + miR.29a + miR.369.3p + miR.376a* + miR.518c + miR.639 + miR.338 | 0,81 | 0,042179126 |
| miR.558 + miR.609 + miR.520f + miR.220a + miR.130a + miR.29a + miR.362 + miR.376a* + miR.494 + miR.518c + miR.639 + miR.660 + miR.338 | 0,81 | 0,010997913 |
| miR.558 + miR.609 + miR.603 + miR.520f + miR.220a + miR.519b + miR.130a + miR.29a + miR.376a* + miR.494 + miR.518c + miR.639 + miR.338 | 0,81 | 0,018417286 |
| miR.609 + miR.603 + miR.220a + miR.26a + miR.29a + miR.362 + miR.369.3p + miR.376a* + miR.494 + miR.518c + miR.639 + miR.660 + miR.338 | 0,81 | 0,361025124 |
| miR.558 + miR.609 + miR.603 + miR.220a + miR.519b + miR.26a + miR.29a + miR.362 + miR.376a* + miR.494 + miR.518c + miR.639 + miR.660 | 0,81 | 0,117927725 |
| miR.558 + miR.609 + miR.603 + miR.520f + miR.220a + miR.519b + miR.130a + miR.29a + miR.362 + miR.376a* + miR.518c + miR.639 + miR.338 | 0,81 | 0,067829062 |
| miR.558 + miR.609 + miR.603 + miR.520f + miR.220a + miR.519b + miR.130a + miR.26a + miR.29a + miR.362 + miR.376a* + miR.518c + miR.338 | 0,81 | 0,092831003 |
| miR.558 + miR.609 + miR.520f + miR.220a + miR.519b + miR.130a + miR.26a + miR.29a + miR.369.3p + miR.376a* + miR.518c + miR.639 + miR.660 | 0,81 | 0,18155177 |
| miR.558 + miR.609 + miR.520f + miR.220a + miR.519b + miR.130a + miR.29a + miR.362 + miR.369.3p + miR.376a* + miR.518c + miR.639 + miR.660 | 0,81 | 0,241729871 |
| miR.558 + miR.609 + miR.603 + miR.520f + miR.220a + miR.130a + miR.26a + miR.29a + miR.376a* + miR.494 + miR.518c + miR.639 + miR.660 | 0,81 | 0,050900165 |
| miR.558 + miR.609 + miR.520f + miR.220a + miR.519b + miR.130a + miR.26a + miR.29a + miR.362 + miR.369.3p + miR.376a* + miR.518c + miR.660 | 0,81 | 0,262366457 |
| miR.558 + miR.609 + miR.603 + miR.519b + miR.130a + miR.29a + miR.362 + miR.369.3p + miR.376a* + miR.494 + miR.518c + miR.639 + miR.660 | 0,81 | 0,079200578 |
| miR.558 + miR.609 + miR.220a + miR.519b + miR.29a + miR.362 + miR.369.3p + miR.376a* + miR.494 + miR.518c + miR.639 + miR.660 + miR.338 | 0,81 | 0,225050342 |
| miR.558 + miR.609 + miR.603 + miR.520f + miR.220a + miR.130a + miR.29a + miR.362 + miR.376a* + miR.494 + miR.518c + miR.639 + miR.338 | 0,81 | 0,013924727 |
| miR.558 + miR.609 + miR.603 + miR.520f + miR.220a + miR.519b + miR.130a + miR.26a + miR.29a + miR.376a* + miR.494 + miR.518c + miR.338 | 0,81 | 0,026721962 |
| miR.558 + miR.609 + miR.603 + miR.519b + miR.29a + miR.362 + miR.369.3p + miR.376a* + miR.494 + miR.518c + miR.639 + miR.660 + miR.338 | 0,81 | 0,04508522 |
| miR.558 + miR.609 + miR.520f + miR.519b + miR.130a + miR.26a + miR.29a + miR.369.3p + miR.376a* + miR.494 + miR.518c + miR.639 + miR.660 | 0,81 | 0,088505592 |
| miR.558 + miR.609 + miR.603 + miR.520f + miR.220a + miR.519b + miR.26a + miR.29a + miR.376a* + miR.494 + miR.518c + miR.639 + miR.338 | 0,81 | 0,031154613 |
| miR.558 + miR.609 + miR.603 + miR.520f + miR.519b + miR.29a + miR.362 + miR.369.3p + miR.376a* + miR.494 + miR.518c + miR.639 + miR.660 | 0,81 | 0,005118879 |
| miR.558 + miR.609 + miR.520f + miR.220a + miR.519b + miR.130a + miR.29a + miR.376a* + miR.494 + miR.518c + miR.639 + miR.660 + miR.338 | 0,81 | 0,069586412 |
| miR.558 + miR.609 + miR.520f + miR.220a + miR.519b + miR.29a + miR.362 + miR.376a* + miR.494 + miR.518c + miR.639 + miR.660 + miR.338 | 0,81 | 0,067533792 |
| miR.558 + miR.609 + miR.603 + miR.520f + miR.220a + miR.519b + miR.130a + miR.26a + miR.29a + miR.362 + miR.376a* + miR.518c + miR.639 | 0,81 | 0,095777108 |
| miR.558 + miR.609 + miR.220a + miR.519b + miR.130a + miR.26a + miR.29a + miR.362 + miR.369.3p + miR.376a* + miR.518c + miR.639 + miR.660 | 0,81 | 0,300787646 |
| miR.609 + miR.603 + miR.520f + miR.130a + miR.29a + miR.362 + miR.369.3p + miR.376a* + miR.494 + miR.518c + miR.639 + miR.660 + miR.338 | 0,81 | 0,40673594 |
| miR.558 + miR.609 + miR.603 + miR.520f + miR.519b + miR.130a + miR.29a + miR.362 + miR.369.3p + miR.376a* + miR.518c + miR.639 + miR.338 | 0,81 | 0,048782077 |
| miR.558 + miR.609 + miR.520f + miR.130a + miR.26a + miR.29a + miR.362 + miR.376a* + miR.494 + miR.518c + miR.639 + miR.660 + miR.338 | 0,81 | 0,010597746 |
| miR.558 + miR.609 + miR.520f + miR.220a + miR.130a + miR.26a + miR.29a + miR.362 + miR.369.3p + miR.376a* + miR.494 + miR.518c + miR.338 | 0,81 | 0,034718922 |
| miR.558 + miR.609 + miR.603 + miR.520f + miR.220a + miR.130a + miR.26a + miR.29a + miR.362 + miR.376a* + miR.518c + miR.639 + miR.338 | 0,81 | 0,018119977 |
| miR.609 + miR.603 + miR.520f + miR.519b + miR.29a + miR.362 + miR.369.3p + miR.376a* + miR.494 + miR.518c + miR.639 + miR.660 + miR.338 | 0,81 | 0,385849057 |
| miR.558 + miR.609 + miR.520f + miR.220a + miR.26a + miR.29a + miR.362 + miR.376a* + miR.494 + miR.518c + miR.639 + miR.660 + miR.338 | 0,81 | 0,02249848 |
| miR.558 + miR.609 + miR.520f + miR.220a + miR.130a + miR.26a + miR.29a + miR.376a* + miR.494 + miR.518c + miR.639 + miR.660 + miR.338 | 0,81 | 0,016477681 |
| miR.558 + miR.609 + miR.220a + miR.519b + miR.130a + miR.29a + miR.369.3p + miR.376a* + miR.494 + miR.518c + miR.639 + miR.660 + miR.338 | 0,81 | 0,244670126 |
| miR.558 + miR.609 + miR.603 + miR.520f + miR.220a + miR.130a + miR.29a + miR.362 + miR.376a* + miR.494 + miR.518c + miR.639 + miR.660 | 0,81 | 0,046165598 |
| miR.558 + miR.609 + miR.603 + miR.520f + miR.220a + miR.130a + miR.26a + miR.29a + miR.376a* + miR.494 + miR.518c + miR.660 + miR.338 | 0,81 | 0,05165049 |
| miR.558 + miR.609 + miR.520f + miR.220a + miR.519b + miR.26a + miR.29a + miR.362 + miR.369.3p + miR.376a* + miR.494 + miR.518c + miR.338 | 0,81 | 0,075443189 |
| miR.558 + miR.609 + miR.519b + miR.130a + miR.26a + miR.29a + miR.362 + miR.369.3p + miR.376a* + miR.494 + miR.518c + miR.660 + miR.338 | 0,81 | 0,30874575 |
| miR.558 + miR.609 + miR.520f + miR.220a + miR.519b + miR.26a + miR.29a + miR.376a* + miR.494 + miR.518c + miR.639 + miR.660 + miR.338 | 0,81 | 0,073319372 |
| miR.558 + miR.609 + miR.520f + miR.519b + miR.130a + miR.29a + miR.362 + miR.376a* + miR.494 + miR.518c + miR.639 + miR.660 + miR.338 | 0,81 | 0,042740219 |
| miR.609 + miR.603 + miR.520f + miR.26a + miR.29a + miR.362 + miR.369.3p + miR.376a* + miR.494 + miR.518c + miR.639 + miR.660 + miR.338 | 0,81 | 0,471600557 |
| miR.558 + miR.609 + miR.603 + miR.520f + miR.220a + miR.26a + miR.29a + miR.362 + miR.376a* + miR.494 + miR.518c + miR.660 + miR.338 | 0,81 | 0,050276267 |
| miR.558 + miR.609 + miR.603 + miR.520f + miR.220a + miR.519b + miR.130a + miR.26a + miR.29a + miR.376a* + miR.494 + miR.518c + miR.639 | 0,81 | 0,055150859 |
| miR.558 + miR.609 + miR.603 + miR.520f + miR.220a + miR.130a + miR.26a + miR.29a + miR.376a* + miR.518c + miR.639 + miR.660 + miR.338 | 0,81 | 0,033222739 |
| miR.609 + miR.603 + miR.520f + miR.220a + miR.519b + miR.29a + miR.362 + miR.369.3p + miR.376a* + miR.518c + miR.639 + miR.660 + miR.338 | 0,81 | 0,38615574 |
| miR.558 + miR.609 + miR.603 + miR.520f + miR.519b + miR.130a + miR.362 + miR.369.3p + miR.376a* + miR.518c + miR.639 + miR.660 + miR.338 | 0,81 | 0,132875689 |
| miR.558 + miR.609 + miR.520f + miR.220a + miR.130a + miR.29a + miR.362 + miR.369.3p + miR.376a* + miR.494 + miR.518c + miR.639 + miR.338 | 0,81 | 0,032927583 |
| miR.558 + miR.609 + miR.603 + miR.520f + miR.220a + miR.26a + miR.29a + miR.362 + miR.376a* + miR.494 + miR.518c + miR.639 + miR.660 | 0,81 | 0,048454383 |
| miR.558 + miR.609 + miR.603 + miR.520f + miR.519b + miR.29a + miR.362 + miR.376a* + miR.494 + miR.518c + miR.639 + miR.660 + miR.338 | 0,81 | 0,01610058 |
| miR.558 + miR.609 + miR.603 + miR.520f + miR.220a + miR.519b + miR.26a + miR.29a + miR.362 + miR.376a* + miR.518c + miR.639 + miR.660 | 0,81 | 0,128991474 |
| miR.558 + miR.609 + miR.603 + miR.520f + miR.519b + miR.130a + miR.29a + miR.376a* + miR.494 + miR.518c + miR.639 + miR.660 + miR.338 | 0,81 | 0,023807371 |
| miR.558 + miR.609 + miR.603 + miR.520f + miR.220a + miR.130a + miR.362 + miR.369.3p + miR.376a* + miR.518c + miR.639 + miR.660 + miR.338 | 0,81 | 0,192196845 |
| miR.609 + miR.603 + miR.520f + miR.220a + miR.519b + miR.29a + miR.369.3p + miR.376a* + miR.494 + miR.518c + miR.639 + miR.660 + miR.338 | 0,81 | 0,128030782 |
| miR.558 + miR.609 + miR.603 + miR.520f + miR.220a + miR.26a + miR.29a + miR.362 + miR.376a* + miR.518c + miR.639 + miR.660 + miR.338 | 0,81 | 0,03202411 |
| miR.558 + miR.609 + miR.603 + miR.520f + miR.519b + miR.130a + miR.29a + miR.362 + miR.376a* + miR.494 + miR.518c + miR.639 + miR.338 | 0,81 | 0,034099937 |
| miR.558 + miR.609 + miR.520f + miR.220a + miR.519b + miR.26a + miR.29a + miR.369.3p + miR.376a* + miR.494 + miR.518c + miR.639 + miR.338 | 0,81 | 0,079452257 |
| miR.558 + miR.609 + miR.603 + miR.520f + miR.519b + miR.26a + miR.29a + miR.369.3p + miR.376a* + miR.494 + miR.518c + miR.639 + miR.660 | 0,81 | 0,027326002 |
| miR.558 + miR.609 + miR.603 + miR.520f + miR.220a + miR.362 + miR.369.3p + miR.376a* + miR.494 + miR.518c + miR.639 + miR.660 + miR.338 | 0,81 | 0,114463692 |
| miR.558 + miR.609 + miR.603 + miR.519b + miR.130a + miR.26a + miR.29a + miR.362 + miR.369.3p + miR.376a* + miR.518c + miR.639 + miR.660 | 0,81 | 0,059308071 |
| miR.558 + miR.609 + miR.603 + miR.220a + miR.519b + miR.130a + miR.26a + miR.29a + miR.362 + miR.376a* + miR.518c + miR.639 + miR.338 | 0,81 | 0,089768465 |
| miR.558 + miR.609 + miR.603 + miR.520f + miR.519b + miR.130a + miR.26a + miR.29a + miR.369.3p + miR.376a* + miR.494 + miR.518c + miR.639 | 0,81 | 0,052541728 |
| miR.558 + miR.609 + miR.520f + miR.130a + miR.26a + miR.29a + miR.362 + miR.369.3p + miR.376a* + miR.494 + miR.518c + miR.639 + miR.338 | 0,81 | 0,075210281 |
| miR.558 + miR.609 + miR.520f + miR.220a + miR.130a + miR.26a + miR.29a + miR.362 + miR.369.3p + miR.376a* + miR.518c + miR.639 + miR.338 | 0,81 | 0,061056171 |
| miR.558 + miR.609 + miR.603 + miR.519b + miR.130a + miR.29a + miR.362 + miR.369.3p + miR.376a* + miR.494 + miR.518c + miR.660 + miR.338 | 0,81 | 0,11034628 |
| miR.558 + miR.609 + miR.603 + miR.520f + miR.220a + miR.130a + miR.29a + miR.362 + miR.376a* + miR.518c + miR.639 + miR.660 + miR.338 | 0,81 | 0,036030433 |
| miR.558 + miR.609 + miR.603 + miR.520f + miR.220a + miR.519b + miR.130a + miR.29a + miR.362 + miR.376a* + miR.518c + miR.639 + miR.660 | 0,81 | 0,10661972 |
| miR.558 + miR.609 + miR.603 + miR.519b + miR.130a + miR.26a + miR.29a + miR.362 + miR.369.3p + miR.376a* + miR.518c + miR.639 + miR.338 | 0,81 | 0,081410858 |
| miR.558 + miR.609 + miR.603 + miR.520f + miR.220a + miR.519b + miR.130a + miR.26a + miR.29a + miR.362 + miR.376a* + miR.518c + miR.660 | 0,81 | 0,134906596 |
| miR.558 + miR.609 + miR.520f + miR.220a + miR.519b + miR.29a + miR.362 + miR.369.3p + miR.376a* + miR.494 + miR.518c + miR.639 + miR.338 | 0,81 | 0,104596229 |
| miR.558 + miR.609 + miR.603 + miR.520f + miR.220a + miR.130a + miR.29a + miR.362 + miR.376a* + miR.494 + miR.518c + miR.660 + miR.338 | 0,81 | 0,049421668 |
| miR.558 + miR.609 + miR.519b + miR.130a + miR.26a + miR.29a + miR.369.3p + miR.376a* + miR.494 + miR.518c + miR.639 + miR.660 + miR.338 | 0,81 | 0,212933566 |
| miR.558 + miR.609 + miR.603 + miR.520f + miR.130a + miR.26a + miR.362 + miR.369.3p + miR.376a* + miR.518c + miR.639 + miR.660 + miR.338 | 0,81 | 0,197008094 |
| miR.558 + miR.609 + miR.603 + miR.520f + miR.130a + miR.26a + miR.29a + miR.362 + miR.369.3p + miR.376a* + miR.494 + miR.518c + miR.338 | 0,81 | 0,031984849 |
| miR.609 + miR.603 + miR.520f + miR.220a + miR.130a + miR.29a + miR.362 + miR.369.3p + miR.376a* + miR.494 + miR.518c + miR.660 + miR.338 | 0,81 | 0,403062744 |
| miR.609 + miR.520f + miR.220a + miR.519b + miR.29a + miR.362 + miR.369.3p + miR.376a* + miR.494 + miR.518c + miR.639 + miR.660 + miR.338 | 0,81 | 0,320775769 |
| miR.558 + miR.609 + miR.520f + miR.519b + miR.130a + miR.26a + miR.29a + miR.362 + miR.369.3p + miR.376a* + miR.518c + miR.639 + miR.660 | 0,81 | 0,020618254 |
| miR.558 + miR.609 + miR.520f + miR.220a + miR.519b + miR.130a + miR.29a + miR.362 + miR.369.3p + miR.376a* + miR.494 + miR.518c + miR.338 | 0,81 | 0,094797523 |
| miR.558 + miR.609 + miR.603 + miR.519b + miR.130a + miR.29a + miR.362 + miR.369.3p + miR.376a* + miR.494 + miR.518c + miR.639 + miR.338 | 0,81 | 0,076381383 |
| miR.558 + miR.609 + miR.220a + miR.519b + miR.130a + miR.26a + miR.29a + miR.369.3p + miR.376a* + miR.494 + miR.518c + miR.639 + miR.338 | 0,81 | 0,066389273 |
| miR.558 + miR.609 + miR.520f + miR.220a + miR.519b + miR.130a + miR.26a + miR.29a + miR.369.3p + miR.376a* + miR.494 + miR.518c + miR.338 | 0,81 | 0,066096791 |
| miR.558 + miR.609 + miR.603 + miR.519b + miR.26a + miR.29a + miR.369.3p + miR.376a* + miR.494 + miR.518c + miR.639 + miR.660 + miR.338 | 0,81 | 0,067564173 |
| miR.558 + miR.609 + miR.603 + miR.220a + miR.519b + miR.26a + miR.29a + miR.362 + miR.376a* + miR.518c + miR.639 + miR.660 + miR.338 | 0,81 | 0,083413289 |
| miR.558 + miR.609 + miR.603 + miR.520f + miR.220a + miR.26a + miR.362 + miR.369.3p + miR.376a* + miR.518c + miR.639 + miR.660 + miR.338 | 0,81 | 0,155299782 |
| miR.558 + miR.609 + miR.603 + miR.519b + miR.130a + miR.26a + miR.29a + miR.362 + miR.369.3p + miR.376a* + miR.494 + miR.518c + miR.639 | 0,81 | 0,077741167 |
| miR.558 + miR.609 + miR.603 + miR.520f + miR.220a + miR.130a + miR.26a + miR.29a + miR.362 + miR.376a* + miR.494 + miR.518c + miR.338 | 0,81 | 0,021219669 |
| miR.558 + miR.609 + miR.603 + miR.520f + miR.130a + miR.26a + miR.29a + miR.362 + miR.369.3p + miR.376a* + miR.518c + miR.639 + miR.660 | 0,81 | 0,053951448 |
| miR.609 + miR.603 + miR.520f + miR.220a + miR.26a + miR.29a + miR.362 + miR.369.3p + miR.376a* + miR.494 + miR.518c + miR.660 + miR.338 | 0,81 | 0,443982258 |
| miR.558 + miR.609 + miR.603 + miR.520f + miR.220a + miR.519b + miR.362 + miR.369.3p + miR.376a* + miR.518c + miR.639 + miR.660 + miR.338 | 0,81 | 0,11173385 |
| miR.558 + miR.609 + miR.603 + miR.220a + miR.519b + miR.130a + miR.26a + miR.29a + miR.362 + miR.376a* + miR.518c + miR.660 + miR.338 | 0,81 | 0,0724 04804 |
| miR.558 + miR.609 + miR.603 + miR.520f + miR.220a + miR.130a + miR.26a + miR.29a + miR.362 + miR.376a* + miR.494 + miR.518c + miR.639 | 0,81 | 0,026378889 |
| miR.558 + miR.609 + miR.603 + miR.520f + miR.220a + miR.519b + miR.29a + miR.362 + miR.376a* + miR.518c + miR.639 + miR.660 + miR.338 | 0,81 | 0,077778662 |
| miR.558 + miR.609 + miR.603 + miR.520f + miR.220a + miR.519b + miR.130a + miR.29a + miR.376a* + miR.518c + miR.639 + miR.660 + miR.338 | 0,81 | 0,103228828 |
| miR.558 + miR.609 + miR.603 + miR.520f + miR.519b + miR.130a + miR.29a + miR.362 + miR.369.3p + miR.376a* + miR.494 + miR.518c + miR.639 | 0,81 | 0,05694836 |
| miR.558 + miR.609 + miR.520f + miR.220a + miR.519b + miR.26a + miR.29a + miR.362 + miR.376a* + miR.494 + miR.518c + miR.660 + miR.338 | 0,81 | 0,114359219 |
| miR.558 + miR.609 + miR.603 + miR.520f + miR.130a + miR.29a + miR.362 + miR.376a* + miR.494 + miR.518c + miR.639 + miR.660 + miR.338 | 0,81 | 0,010084958 |
| miR.558 + miR.609 + miR.603 + miR.520f + miR.26a + miR.29a + miR.362 + miR.376a* + miR.494 + miR.518c + miR.639 + miR.660 + miR.338 | 0,81 | 0,009887988 |
| miR.558 + miR.609 + miR.220a + miR.519b + miR.130a + miR.26a + miR.29a + miR.362 + miR.369.3p + miR.376a* + miR.494 + miR.518c + miR.338 | 0,81 | 0,089903015 |
| miR.558 + miR.609 + miR.520f + miR.220a + miR.519b + miR.130a + miR.29a + miR.362 + miR.376a* + miR.494 + miR.518c + miR.660 + miR.338 | 0,81 | 0,150029262 |
| miR.558 + miR.609 + miR.520f + miR.519b + miR.130a + miR.26a + miR.29a + miR.369.3p + miR.376a* + miR.494 + miR.518c + miR.639 + miR.338 | 0,81 | 0,088836809 |
| miR.558 + miR.609 + miR.603 + miR.520f + miR.519b + miR.130a + miR.26a + miR.29a + miR.362 + miR.369.3p + miR.376a* + miR.518c + miR.338 | 0,81 | 0,059390274 |
| miR.609 + miR.603 + miR.520f + miR.220a + miR.519b + miR.29a + miR.362 + miR.369.3p + miR.376a* + miR.494 + miR.518c + miR.639 + miR.660 | 0,81 | 0,13064767 |
| miR.558 + miR.609 + miR.603 + miR.520f + miR.519b + miR.26a + miR.29a + miR.376a* + miR.494 + miR.518c + miR.639 + miR.660 + miR.338 | 0,81 | 0,023953942 |
| miR.558 + miR.609 + miR.520f + miR.220a + miR.519b + miR.130a + miR.26a + miR.29a + miR.376a* + miR.494 + miR.518c + miR.660 + miR.338 | 0,81 | 0,157618381 |
| miR.558 + miR.609 + miR.603 + miR.220a + miR.519b + miR.130a + miR.26a + miR.29a + miR.376a* + miR.518c + miR.639 + miR.660 + miR.338 | 0,81 | 0,069866539 |
| miR.558 + miR.609 + miR.603 + miR.520f + miR.220a + miR.519b + miR.26a + miR.29a + miR.376a* + miR.518c + miR.639 + miR.660 + miR.338 | 0,81 | 0,090434072 |
| miR.558 + miR.609 + miR.220a + miR.130a + miR.26a + miR.29a + miR.362 + miR.369.3p + miR.376a* + miR.494 + miR.518c + miR.639 + miR.338 | 0,81 | 0,058237304 |
| miR.609 + miR.603 + miR.520f + miR.220a + miR.26a + miR.29a + miR.362 + miR.369.3p + miR.376a* + miR.494 + miR.518c + miR.639 + miR.660 | 0,81 | 0,099984841 |
| miR.558 + miR.609 + miR.520f + miR.220a + miR.519b + miR.130a + miR.29a + miR.362 + miR.376a* + miR.494 + miR.518c + miR.639 + miR.338 | 0,81 | 0,092166906 |
| miR.558 + miR.609 + miR.603 + miR.520f + miR.220a + miR.130a + miR.26a + miR.29a + miR.362 + miR.376a* + miR.518c + miR.639 + miR.660 | 0,81 | 0,07279787 |
| miR.609 + miR.603 + miR.520f + miR.220a + miR.519b + miR.26a + miR.29a + miR.369.3p + miR.376a* + miR.494 + miR.518c + miR.660 + miR.338 | 0,81 | 0,12642831 |
| miR.558 + miR.609 + miR.520f + miR.220a + miR.519b + miR.130a + miR.29a + miR.369.3p + miR.376a* + miR.494 + miR.518c + miR.639 + miR.338 | 0,81 | 0,064322743 |
| miR.558 + miR.609 + miR.603 + miR.520f + miR.130a + miR.26a + miR.29a + miR.362 + miR.369.3p + miR.376a* + miR.494 + miR.518c + miR.639 | 0,81 | 0,033837657 |
| miR.609 + miR.603 + miR.520f + miR.220a + miR.519b + miR.130a + miR.29a + miR.369.3p + miR.376a* + miR.494 + miR.518c + miR.660 + miR.338 | 0,81 | 0,148315589 |
| miR.558 + miR.609 + miR.603 + miR.520f + miR.130a + miR.26a + miR.29a + miR.376a* + miR.494 + miR.518c + miR.639 + miR.660 + miR.338 | 0,81 | 0,014773319 |
| miR.558 + miR.609 + miR.520f + miR.220a + miR.519b + miR.26a + miR.29a + miR.362 + miR.369.3p + miR.376a* + miR.494 + miR.518c + miR.639 | 0,81 | 0,132856533 |
| miR.558 + miR.609 + miR.603 + miR.220a + miR.130a + miR.26a + miR.29a + miR.362 + miR.376a* + miR.494 + miR.518c + miR.660 + miR.338 | 0,81 | 0,044475068 |
| miR.558 + miR.609 + miR.520f + miR.220a + miR.130a + miR.26a + miR.29a + miR.362 + miR.369.3p + miR.376a* + miR.494 + miR.518c + miR.639 | 0,81 | 0,054075693 |
| miR.558 + miR.609 + miR.603 + miR.520f + miR.220a + miR.130a + miR.26a + miR.29a + miR.362 + miR.376a* + miR.518c + miR.660 + miR.338 | 0,81 | 0,043628889 |
| miR.609 + miR.603 + miR.520f + miR.220a + miR.130a + miR.29a + miR.362 + miR.369.3p + miR.376a* + miR.494 + miR.518c + miR.639 + miR.660 | 0,81 | 0,069759092 |
| miR.609 + miR.603 + miR.220a + miR.519b + miR.26a + miR.29a + miR.362 + miR.369.3p + miR.376a* + miR.494 + miR.518c + miR.639 + miR.660 | 0,81 | 0,194638112 |
| miR.558 + miR.609 + miR.520f + miR.519b + miR.130a + miR.29a + miR.362 + miR.369.3p + miR.376a* + miR.494 + miR.518c + miR.639 + miR.338 | 0,81 | 0,064801749 |
| miR.558 + miR.609 + miR.520f + miR.519b + miR.130a + miR.26a + miR.29a + miR.376a* + miR.494 + miR.518c + miR.639 + miR.660 + miR.338 | 0,81 | 0,072759393 |
| miR.558 + miR.609 + miR.603 + miR.520f + miR.220a + miR.519b + miR.130a + miR.26a + miR.29a + miR.376a* + miR.518c + miR.639 + miR.660 | 0,81 | 0,109795784 |
| miR.558 + miR.609 + miR.520f + miR.220a + miR.519b + miR.26a + miR.29a + miR.362 + miR.376a* + miR.494 + miR.518c + miR.639 + miR.338 | 0,81 | 0,129823773 |
| miR.558 + miR.609 + miR.520f + miR.220a + miR.519b + miR.130a + miR.26a + miR.29a + miR.362 + miR.369.3p + miR.376a* + miR.494 + miR.518c | 0,81 | 0,139084816 |
| miR.558 + miR.609 + miR.603 + miR.520f + miR.220a + miR.130a + miR.26a + miR.29a + miR.376a* + miR.494 + miR.518c + miR.639 + miR.338 | 0,81 | 0,011172234 |
| miR.558 + miR.609 + miR.220a + miR.519b + miR.26a + miR.29a + miR.362 + miR.369.3p + miR.376a* + miR.494 + miR.518c + miR.639 + miR.338 | 0,81 | 0,050520222 |
| miR.558 + miR.609 + miR.520f + miR.220a + miR.519b + miR.130a + miR.26a + miR.29a + miR.362 + miR.376a* + miR.494 + miR.518c + miR.338 | 0,81 | 0,14375355 |
| miR.558 + miR.609 + miR.603 + miR.520f + miR.519b + miR.130a + miR.26a + miR.29a + miR.369.3p + miR.376a* + miR.518c + miR.639 + miR.660 | 0,81 | 0,05862995 |
| miR.558 + miR.609 + miR.520f + miR.220a + miR.519b + miR.26a + miR.29a + miR.362 + miR.369.3p + miR.376a* + miR.518c + miR.639 + miR.338 | 0,81 | 0,102879027 |
| miR.558 + miR.609 + miR.603 + miR.520f + miR.220a + miR.519b + miR.130a + miR.29a + miR.362 + miR.376a* + miR.518c + miR.660 + miR.338 | 0,81 | 0,10448496 |
| miR.558 + miR.609 + miR.603 + miR.520f + miR.220a + miR.130a + miR.26a + miR.29a + miR.362 + miR.376a* + miR.494 + miR.518c + miR.660 | 0,81 | 0,056900306 |
| miR.558 + miR.609 + miR.603 + miR.220a + miR.519b + miR.130a + miR.26a + miR.29a + miR.362 + miR.376a* + miR.518c + miR.639 + miR.660 | 0,81 | 0,105228026 |
| miR.558 + miR.609 + miR.603 + miR.520f + miR.220a + miR.519b + miR.130a + miR.26a + miR.29a + miR.376a* + miR.518c + miR.660 + miR.338 | 0,81 | 0,102851595 |
| miR.609 + miR.603 + miR.220a + miR.130a + miR.26a + miR.29a + miR.362 + miR.369.3p + miR.376a* + miR.494 + miR.518c + miR.639 + miR.660 | 0,81 | 0,156983946 |
| miR.558 + miR.609 + miR.520f + miR.519b + miR.26a + miR.29a + miR.362 + miR.369.3p + miR.376a* + miR.494 + miR.518c + miR.639 + miR.660 | 0,81 | 0,011731119 |
| miR.558 + miR.609 + miR.603 + miR.520f + miR.519b + miR.130a + miR.29a + miR.362 + miR.369.3p + miR.376a* + miR.518c + miR.639 + miR.660 | 0,81 | 0,014291383 |
| miR.609 + miR.603 + miR.520f + miR.220a + miR.130a + miR.29a + miR.362 + miR.369.3p + miR.376a* + miR.518c + miR.639 + miR.660 + miR.338 | 0,81 | 0,363021363 |
| miR.558 + miR.609 + miR.520f + miR.220a + miR.519b + miR.130a + miR.26a + miR.29a + miR.362 + miR.369.3p + miR.376a* + miR.518c + miR.338 | 0,81 | 0,084537667 |
| miR.609 + miR.603 + miR.220a + miR.519b + miR.26a + miR.29a + miR.362 + miR.369.3p + miR.376a* + miR.494 + miR.518c + miR.660 + miR.338 | 0,81 | 0,419295859 |
| miR.558 + miR.609 + miR.603 + miR.519b + miR.26a + miR.29a + miR.362 + miR.369.3p + miR.376a* + miR.494 + miR.518c + miR.639 + miR.338 | 0,81 | 0,056449177 |
| miR.558 + miR.609 + miR.603 + miR.520f + miR.220a + miR.519b + miR.26a + miR.29a + miR.362 + miR.376a* + miR.518c + miR.660 + miR.338 | 0,81 | 0,105855453 |
| miR.558 + miR.609 + miR.520f + miR.220a + miR.130a + miR.29a + miR.362 + miR.369.3p + miR.376a* + miR.494 + miR.639 + miR.660 + miR.338 | 0,81 | 0,296783286 |
| miR.558 + miR.609 + miR.603 + miR.220a + miR.130a + miR.26a + miR.29a + miR.362 + miR.376a* + miR.518c + miR.639 + miR.660 + miR.338 | 0,81 | 0,049002286 |
| miR.558 + miR.609 + miR.603 + miR.520f + miR.519b + miR.26a + miR.29a + miR.362 + miR.369.3p + miR.376a* + miR.494 + miR.518c + miR.660 | 0,81 | 0,054974724 |
| miR.558 + miR.609 + miR.220a + miR.519b + miR.130a + miR.26a + miR.29a + miR.362 + miR.369.3p + miR.376a* + miR.518c + miR.639 + miR.338 | 0,81 | 0,07702746 |
| miR.558 + miR.609 + miR.520f + miR.220a + miR.519b + miR.130a + miR.29a + miR.362 + miR.369.3p + miR.376a* + miR.494 + miR.518c + miR.639 | 0,81 | 0,124257893 |
| miR.558 + miR.609 + miR.603 + miR.520f + miR.130a + miR.29a + miR.362 + miR.369.3p + miR.376a* + miR.494 + miR.639 + miR.660 + miR.338 | 0,81 | 0,317759423 |
| miR.609 + miR.603 + miR.520f + miR.220a + miR.519b + miR.26a + miR.29a + miR.362 + miR.369.3p + miR.376a* + miR.494 + miR.518c + miR.660 | 0,81 | 0,195976087 |
| miR.558 + miR.609 + miR.603 + miR.520f + miR.130a + miR.26a + miR.29a + miR.362 + miR.376a* + miR.494 + miR.518c + miR.639 + miR.338 | 0,81 | 0,018509603 |
| miR.609 + miR.603 + miR.520f + miR.220a + miR.519b + miR.130a + miR.29a + miR.362 + miR.369.3p + miR.376a* + miR.494 + miR.518c + miR.660 | 0,81 | 0,176526046 |
| miR.558 + miR.609 + miR.520f + miR.519b + miR.130a + miR.29a + miR.362 + miR.369.3p + miR.376a* + miR.494 + miR.639 + miR.660 + miR.338 | 0,81 | 0,299979233 |
| miR.558 + miR.609 + miR.603 + miR.220a + miR.519b + miR.29a + miR.362 + miR.376a* + miR.494 + miR.518c + miR.639 + miR.660 + miR.338 | 0,81 | 0,081031334 |
| miR.558 + miR.609 + miR.603 + miR.520f + miR.220a + miR.519b + miR.130a + miR.26a + miR.29a + miR.376a* + miR.518c + miR.639 + miR.338 | 0,81 | 0,037621692 |
| miR.558 + miR.609 + miR.603 + miR.519b + miR.130a + miR.26a + miR.29a + miR.362 + miR.369.3p + miR.376a* + miR.494 + miR.518c + miR.338 | 0,81 | 0,055365894 |
| miR.558 + miR.609 + miR.220a + miR.519b + miR.130a + miR.26a + miR.29a + miR.362 + miR.369.3p + miR.376a* + miR.494 + miR.518c + miR.639 | 0,81 | 0,113968637 |
| miR.558 + miR.609 + miR.603 + miR.520f + miR.130a + miR.26a + miR.29a + miR.362 + miR.369.3p + miR.376a* + miR.494 + miR.518c + miR.660 | 0,81 | 0,03505868 |
| miR.558 + miR.609 + miR.603 + miR.519b + miR.130a + miR.26a + miR.29a + miR.369.3p + miR.376a* + miR.494 + miR.518c + miR.660 + miR.338 | 0,81 | 0,050652216 |
| miR.558 + miR.609 + miR.520f + miR.130a + miR.26a + miR.29a + miR.362 + miR.369.3p + miR.376a* + miR.494 + miR.639 + miR.660 + miR.338 | 0,81 | 0,315190456 |
| miR.558 + miR.609 + miR.520f + miR.220a + miR.519b + miR.130a + miR.29a + miR.362 + miR.376a* + miR.494 + miR.518c + miR.639 + miR.660 | 0,81 | 0,114933733 |
| miR.558 + miR.609 + miR.520f + miR.220a + miR.519b + miR.130a + miR.26a + miR.29a + miR.369.3p + miR.376a* + miR.518c + miR.639 + miR.338 | 0,81 | 0,099823206 |
| miR.558 + miR.609 + miR.520f + miR.220a + miR.519b + miR.130a + miR.26a + miR.29a + miR.376a* + miR.494 + miR.518c + miR.639 + miR.660 | 0,81 | 0,115593279 |
| miR.558 + miR.609 + miR.603 + miR.520f + miR.519b + miR.130a + miR.29a + miR.362 + miR.369.3p + miR.376a* + miR.494 + miR.518c + miR.660 | 0,81 | 0,054685768 |
| miR.558 + miR.609 + miR.603 + miR.520f + miR.26a + miR.362 + miR.369.3p + miR.376a* + miR.494 + miR.518c + miR.639 + miR.660 + miR.338 | 0,81 | 0,14649747 |
| miR.558 + miR.609 + miR.603 + miR.220a + miR.130a + miR.29a + miR.362 + miR.376a* + miR.494 + miR.518c + miR.639 + miR.660 + miR.338 | 0,81 | 0,042425785 |
| miR.558 + miR.609 + miR.603 + miR.520f + miR.519b + miR.130a + miR.26a + miR.29a + miR.376a* + miR.494 + miR.518c + miR.639 + miR.338 | 0,81 | 0,023002691 |
| miR.609 + miR.603 + miR.220a + miR.130a + miR.26a + miR.29a + miR.362 + miR.369.3p + miR.376a* + miR.494 + miR.518c + miR.660 + miR.338 | 0,81 | 0,391306838 |
| miR.558 + miR.609 + miR.520f + miR.220a + miR.130a + miR.26a + miR.29a + miR.362 + miR.376a* + miR.494 + miR.518c + miR.660 + miR.338 | 0,81 | 0,043584453 |
| miR.609 + miR.520f + miR.220a + miR.26a + miR.29a + miR.362 + miR.369.3p + miR.376a* + miR.494 + miR.518c + miR.639 + miR.660 + miR.338 | 0,81 | 0,253779002 |
| miR.558 + miR.609 + miR.520f + miR.220a + miR.130a + miR.26a + miR.29a + miR.362 + miR.376a* + miR.518c + miR.639 + miR.660 + miR.338 | 0,81 | 0,023286823 |
| miR.558 + miR.609 + miR.603 + miR.520f + miR.130a + miR.362 + miR.369.3p + miR.376a* + miR.494 + miR.518c + miR.639 + miR.660 + miR.338 | 0,81 | 0,124610367 |
| miR.609 + miR.603 + miR.220a + miR.519b + miR.130a + miR.26a + miR.29a + miR.362 + miR.369.3p + miR.376a* + miR.494 + miR.518c + miR.660 | 0,81 | 0,201195729 |
| miR.609 + miR.603 + miR.520f + miR.220a + miR.26a + miR.29a + miR.362 + miR.369.3p + miR.376a* + miR.518c + miR.639 + miR.660 + miR.338 | 0,81 | 0,347221247 |
| miR.558 + miR.609 + miR.603 + miR.520f + miR.220a + miR.29a + miR.362 + miR.369.3p + miR.376a* + miR.494 + miR.639 + miR.660 + miR.338 | 0,81 | 0,281132438 |
| miR.558 + miR.609 + miR.520f + miR.220a + miR.519b + miR.130a + miR.26a + miR.29a + miR.369.3p + miR.376a* + miR.494 + miR.518c + miR.639 | 0,81 | 0,083637503 |
| miR.609 + miR.520f + miR.220a + miR.130a + miR.29a + miR.362 + miR.369.3p + miR.376a* + miR.494 + miR.518c + miR.639 + miR.660 + miR.338 | 0,81 | 0,291545586 |
| miR.558 + miR.609 + miR.520f + miR.519b + miR.130a + miR.26a + miR.29a + miR.362 + miR.376a* + miR.494 + miR.518c + miR.639 + miR.338 | 0,81 | 0,073905315 |
| miR.558 + miR.609 + miR.520f + miR.220a + miR.519b + miR.130a + miR.29a + miR.362 + miR.369.3p + miR.376a* + miR.518c + miR.639 + miR.338 | 0,81 | 0,087195074 |
| miR.558 + miR.609 + miR.603 + miR.520f + miR.519b + miR.26a + miR.29a + miR.362 + miR.369.3p + miR.376a* + miR.494 + miR.518c + miR.639 | 0,81 | 0,043064954 |
| miR.609 + miR.603 + miR.520f + miR.519b + miR.130a + miR.29a + miR.362 + miR.369.3p + miR.376a* + miR.518c + miR.639 + miR.660 + miR.338 | 0,81 | 0,478390968 |
| miR.558 + miR.609 + miR.603 + miR.520f + miR.519b + miR.130a + miR.26a + miR.29a + miR.369.3p + miR.376a* + miR.494 + miR.518c + miR.660 | 0,81 | 0,061718871 |
| miR.558 + miR.609 + miR.520f + miR.220a + miR.519b + miR.130a + miR.26a + miR.29a + miR.362 + miR.376a* + miR.494 + miR.518c + miR.639 | 0,81 | 0,107551914 |
| miR.558 + miR.609 + miR.603 + miR.520f + miR.519b + miR.130a + miR.29a + miR.362 + miR.376a* + miR.494 + miR.518c + miR.660 + miR.338 | 0,81 | 0,071885439 |
| miR.558 + miR.609 + miR.603 + miR.520f + miR.26a + miR.29a + miR.362 + miR.369.3p + miR.376a* + miR.494 + miR.639 + miR.660 + miR.338 | 0,81 | 0,367884507 |
| miR.558 + miR.609 + miR.520f + miR.220a + miR.26a + miR.29a + miR.362 + miR.369.3p + miR.376a* + miR.494 + miR.639 + miR.660 + miR.338 | 0,81 | 0,295403231 |
| miR.558 + miR.609 + miR.520f + miR.220a + miR.519b + miR.130a + miR.362 + miR.369.3p + miR.376a* + miR.518c + miR.639 + miR.660 + miR.338 | 0,81 | 0,129392208 |
| miR.558 + miR.609 + miR.603 + miR.220a + miR.519b + miR.130a + miR.29a + miR.362 + miR.376a* + miR.518c + miR.639 + miR.660 + miR.338 | 0,81 | 0,079518032 |
| miR.558 + miR.609 + miR.603 + miR.520f + miR.519b + miR.29a + miR.362 + miR.369.3p + miR.376a* + miR.494 + miR.639 + miR.660 + miR.338 | 0,81 | 0,328184604 |
| miR.558 + miR.609 + miR.603 + miR.520f + miR.519b + miR.26a + miR.29a + miR.362 + miR.376a* + miR.494 + miR.518c + miR.639 + miR.338 | 0,81 | 0,048286108 |
| miR.558 + miR.609 + miR.603 + miR.520f + miR.519b + miR.130a + miR.26a + miR.29a + miR.362 + miR.376a* + miR.518c + miR.639 + miR.338 | 0,81 | 0,047126318 |
| miR.609 + miR.603 + miR.220a + miR.519b + miR.130a + miR.29a + miR.362 + miR.369.3p + miR.376a* + miR.494 + miR.518c + miR.639 + miR.660 | 0,81 | 0,197656575 |
| miR.558 + miR.609 + miR.520f + miR.220a + miR.519b + miR.130a + miR.26a + miR.29a + miR.362 + miR.376a* + miR.494 + miR.518c + miR.660 | 0,81 | 0,203443026 |
| miR.609 + miR.603 + miR.220a + miR.519b + miR.26a + miR.29a + miR.369.3p + miR.376a* + miR.494 + miR.518c + miR.639 + miR.660 + miR.338 | 0,81 | 0,113225883 |
| miR.558 + miR.609 + miR.520f + miR.220a + miR.519b + miR.26a + miR.29a + miR.362 + miR.376a* + miR.494 + miR.518c + miR.639 + miR.660 | 0,81 | 0,115644894 |
| miR.558 + miR.609 + miR.603 + miR.519b + miR.130a + miR.26a + miR.29a + miR.362 + miR.369.3p + miR.376a* + miR.494 + miR.518c + miR.660 | 0,81 | 0,092194271 |
| miR.609 + miR.603 + miR.220a + miR.519b + miR.130a + miR.29a + miR.362 + miR.369.3p + miR.376a* + miR.494 + miR.518c + miR.660 + miR.338 | 0,81 | 0,358832089 |
| miR.558 + miR.609 + miR.603 + miR.520f + miR.519b + miR.130a + miR.26a + miR.29a + miR.362 + miR.376a* + miR.494 + miR.518c + miR.338 | 0,81 | 0,033923581 |
| miR.558 + miR.609 + miR.520f + miR.220a + miR.130a + miR.26a + miR.29a + miR.362 + miR.376a* + miR.494 + miR.518c + miR.639 + miR.338 | 0,81 | 0,036992373 |
| miR.558 + miR.609 + miR.520f + miR.220a + miR.519b + miR.29a + miR.362 + miR.369.3p + miR.376a* + miR.494 + miR.639 + miR.660 + miR.338 | 0,81 | 0,284745688 |
| miR.558 + miR.609 + miR.603 + miR.520f + miR.519b + miR.26a + miR.362 + miR.369.3p + miR.376a* + miR.518c + miR.639 + miR.660 + miR.338 | 0,81 | 0,124693966 |
| miR.558 + miR.609 + miR.520f + miR.220a + miR.130a + miR.26a + miR.29a + miR.362 + miR.376a* + miR.494 + miR.518c + miR.639 + miR.660 | 0,81 | 0,044327285 |
| miR.609 + miR.603 + miR.520f + miR.220a + miR.519b + miR.130a + miR.29a + miR.369.3p + miR.376a* + miR.494 + miR.518c + miR.639 + miR.660 | 0,81 | 0,109366469 |
| miR.558 + miR.609 + miR.520f + miR.519b + miR.130a + miR.26a + miR.29a + miR.362 + miR.369.3p + miR.376a* + miR.494 + miR.518c + miR.660 | 0,81 | 0,096025553 |
| miR.558 + miR.609 + miR.520f + miR.220a + miR.519b + miR.26a + miR.29a + miR.362 + miR.376a* + miR.518c + miR.639 + miR.660 + miR.338 | 0,81 | 0,039254335 |
| miR.558 + miR.609 + miR.603 + miR.520f + miR.519b + miR.362 + miR.369.3p + miR.376a* + miR.494 + miR.518c + miR.639 + miR.660 + miR.338 | 0,81 | 0,135571984 |
| miR.558 + miR.609 + miR.603 + miR.520f + miR.519b + miR.26a + miR.29a + miR.362 + miR.376a* + miR.494 + miR.518c + miR.660 + miR.338 | 0,81 | 0,054449119 |
| miR.609 + miR.603 + miR.520f + miR.220a + miR.519b + miR.26a + miR.29a + miR.369.3p + miR.376a* + miR.494 + miR.518c + miR.639 + miR.660 | 0,81 | 0,10526367 |
| miR.558 + miR.609 + miR.520f + miR.220a + miR.519b + miR.130a + miR.26a + miR.29a + miR.362 + miR.369.3p + miR.376a* + miR.518c + miR.639 | 0,81 | 0,110763733 |
| miR.558 + miR.609 + miR.603 + miR.520f + miR.519b + miR.130a + miR.26a + miR.29a + miR.376a* + miR.494 + miR.518c + miR.660 + miR.338 | 0,81 | 0,051269285 |
| miR.558 + miR.609 + miR.520f + miR.220a + miR.519b + miR.130a + miR.29a + miR.362 + miR.376a* + miR.518c + miR.639 + miR.660 + miR.338 | 0,81 | 0,04037021 |

**Table 14: clusters of 14 miRNAs**

| **Cluster of 14 miRs** | **c_tau level** | **pv compared to top signat ure miR.5 58 + miR.6 09 + miR.6 03 + miR.5 20f + miR.1 30a + miR.2 9a + miR.3 62 + miR.3 69.3p + miR.3 76a* + miR.4 94 + miR.5 18c + miR.6 39 + miR.6 60 + miR.3 38** |
|---|---|---|
| miR.558 + miR.609 + miR.603 + miR.520f + miR.130a + miR.29a + miR.362 + miR.369.3p + miR.376a* + miR.494 + miR.518c + miR.639 + miR.660 + miR.338 | 0,82 | NaN |
| miR.558 + miR.609 + miR.603 + miR.130a + miR.26a + miR.29a + miR.362 + miR.369.3p + miR.376a* + miR.494 + miR.518c + miR.639 + miR.660 + miR.338 | 0,82 | 0,301591691 |
| miR.558 + miR.609 + miR.520f + miR.130a + miR.26a + miR.29a + miR.362 + miR.369.3p + miR.376a* + miR.494 + miR.518c + miR.639 + miR.660 + miR.338 | 0,82 | 0,165411135 |
| miR.558 + miR.609 + miR.520f + miR.519b + miR.130a + miR.29a + miR.362 + miR.369.3p + miR.376a* + miR.494 + miR.518c + miR.639 + miR.660 + miR.338 | 0,82 | 0,025209783 |
| miR.558 + miR.609 + miR.520f + miR.220a + miR.130a + miR.29a + miR.362 + miR.369.3p + miR.376a* + miR.494 + miR.518c + miR.639 + miR.660 + miR.338 | 0,82 | 0,003221038 |
| miR.558 + miR.609 + miR.603 + miR.520f + miR.220a + miR.26a + miR.29a + miR.362 + miR.369.3p + miR.376a* + miR.518c + miR.639 + miR.660 + miR.338 | 0,82 | 0,389830678 |
| miR.558 + miR.609 + miR.603 + miR.520f + miR.220a + miR.29a + miR.362 + miR.369.3p + miR.376a* + miR.494 + miR.518c + miR.639 + miR.660 + miR.338 | 0,82 | 0,496123065 |
| miR.558 + miR.609 + miR.603 + miR.220a + miR.26a + miR.29a + miR.362 + miR.369.3p + miR.376a* + miR.494 + miR.518c + miR.639 + miR.660 + miR.338 | 0,81 | 0,444774953 |
| miR.558 + miR.609 + miR.520f + miR.220a + miR.26a + miR.29a + miR.362 + miR.369.3p + miR.376a* + miR.494 + miR.518c + miR.639 + miR.660 + miR.338 | 0,81 | 0,104413155 |
| miR.558 + miR.609 + miR.603 + miR.520f + miR.220a + miR.519b + miR.29a + miR.362 + miR.369.3p + miR.376a* + miR.518c + miR.639 + miR.660 + miR.338 | 0,81 | 0,435667582 |
| miR.558 + miR.609 + miR.603 + miR.520f + miR.220a + miR.130a + miR.29a + miR.362 + miR.369.3p + miR.376a* + miR.518c + miR.639 + miR.660 + miR.338 | 0,81 | 0,48987011 |
| miR.558 + miR.609 + miR.520f + miR.519b + miR.26a + miR.29a + miR.362 + miR.369.3p + miR.376a* + miR.494 + miR.518c + miR.639 + miR.660 + miR.338 | 0,81 | 0,265407378 |
| miR.558 + miR.609 + miR.520f + miR.519b + miR.130a + miR.26a + miR.29a + miR.362 + miR.369.3p + miR.376a* + miR.518c + miR.639 + miR.660 + miR.338 | 0,81 | 0,207501296 |
| miR.558 + miR.609 + miR.603 + miR.520f + miR.26a + miR.29a + miR.362 + miR.369.3p + miR.376a* + miR.494 + miR.518c + miR.639 + miR.660 + miR.338 | 0,81 | 0,418811391 |
| miR.558 + miR.609 + miR.520f + miR.220a + miR.519b + miR.29a + miR.362 + miR.369.3p + miR.376a* + miR.494 + miR.518c + miR.639 + miR.660 + miR.338 | 0,81 | 0,073133149 |
| miR.558 + miR.609 + miR.520f + miR.220a + miR.519b + miR.26a + miR.29a + miR.362 + miR.369.3p + miR.376a* + miR.518c + miR.639 + miR.660 + miR.338 | 0,81 | 0,128223285 |
| miR.558 + miR.609 + miR.603 + miR.520f + miR.519b + miR.29a + miR.362 + miR.369.3p + miR.376a* + miR.494 + miR.518c + miR.639 + miR.660 + miR.338 | 0,81 | 0,495657306 |
| miR.558 + miR.609 + miR.603 + miR.220a + miR.130a + miR.26a + miR.29a + miR.362 + miR.369.3p + miR.376a* + miR.518c + miR.639 + miR.660 + miR.338 | 0,81 | 0,429185148 |
| miR.558 + miR.609 + miR.603 + miR.520f + miR.519b + miR.26a + miR.29a + miR.362 + miR.369.3p + miR.376a* + miR.518c + miR.639 + miR.660 + miR.338 | 0,81 | 0,304888846 |
| miR.558 + miR.609 + miR.603 + miR.220a + miR.519b + miR.26a + miR.29a + miR.362 + miR.369.3p + miR.376a* + miR.518c + miR.639 + miR.660 + miR.338 | 0,81 | 0,426980358 |
| miR.558 + miR.609 + miR.603 + miR.220a + miR.130a + miR.29a + miR.362 + miR.369.3p + miR.376a* + miR.494 + miR.518c + miR.639 + miR.660 + miR.338 | 0,81 | 0,489476287 |
| miR.558 + miR.609 + miR.603 + miR.520f + miR.220a + miR.519b + miR.29a + miR.369.3p + miR.376a* + miR.494 + miR.518c + miR.639 + miR.660 + miR.338 | 0,81 | 0,221998549 |
| miR.558 + miR.609 + miR.603 + miR.520f + miR.130a + miR.26a + miR.29a + miR.362 + miR.369.3p + miR.376a* + miR.518c + miR.639 + miR.660 + miR.338 | 0,81 | 0,398743697 |
| miR.558 + miR.609 + miR.603 + miR.520f + miR.220a + miR.519b + miR.130a + miR.29a + miR.369.3p + miR.376a* + miR.494 + miR.518c + miR.660 + miR.338 | 0,81 | 0,168300629 |
| miR.558 + miR.609 + miR.520f + miR.220a + miR.130a + miR.26a + miR.29a + miR.362 + miR.369.3p + miR.376a* + miR.518c + miR.639 + miR.660 + miR.338 | 0,81 | 0,229918161 |
| miR.558 + miR.609 + miR.603 + miR.520f + miR.220a + miR.519b + miR.26a + miR.29a + miR.369.3p + miR.376a* + miR.494 + miR.518c + miR.660 + miR.338 | 0,81 | 0,199585294 |
| miR.558 + miR.609 + miR.520f + miR.220a + miR.519b + miR.130a + miR.29a + miR.362 + miR.369.3p + miR.376a* + miR.518c + miR.639 + miR.660 + miR.338 | 0,81 | 0,11973122 |
| miR.558 + miR.609 + miR.603 + miR.220a + miR.519b + miR.26a + miR.29a + miR.362 + miR.369.3p + miR.376a* + miR.494 + miR.518c + miR.660 + miR.338 | 0,81 | 0,3394704 |
| miR.558 + miR.609 + miR.603 + miR.520f + miR.220a + miR.130a + miR.29a + miR.369.3p + miR.376a* + miR.494 + miR.518c + miR.639 + miR.660 + miR.338 | 0,81 | 0,071730683 |
| miR.558 + miR.609 + miR.603 + miR.520f + miR.220a + miR.519b + miR.29a + miR.362 + miR.369.3p + miR.376a* + miR.494 + miR.518c + miR.660 + miR.338 | 0,81 | 0,48233317 |
| miR.558 + miR.609 + miR.220a + miR.130a + miR.26a + miR.29a + miR.362 + miR.369.3p + miR.376a* + miR.494 + miR.518c + miR.639 + miR.660 + miR.338 | 0,81 | 0,430921212 |
| miR.558 + miR.609 + miR.603 + miR.520f + miR.519b + miR.130a + miR.29a + miR.362 + miR.369.3p + miR.376a* + miR.518c + miR.639 + miR.660 + miR.338 | 0,81 | 0,489277602 |
| miR.558 + miR.609 + miR.519b + miR.130a + miR.26a + miR.29a + miR.362 + miR.369.3p + miR.376a* + miR.494 + miR.518c + miR.639 + miR.660 + miR.338 | 0,81 | 0,292052047 |
| miR.558 + miR.609 + miR.603 + miR.520f + miR.220a + miR.26a + miR.29a + miR.362 + miR.369.3p + miR.376a* + miR.494 + miR.518c + miR.660 + miR.338 | 0,81 | 0,419601156 |
| miR.558 + miR.609 + miR.603 + miR.220a + miR.519b + miR.130a + miR.26a + miR.29a + miR.369.3p + miR.376a* + miR.494 + miR.518c + miR.660 + miR.338 | 0,81 | 0,211626212 |
| miR.558 + miR.609 + miR.603 + miR.220a + miR.130a + miR.26a + miR.29a + miR.369.3p + miR.376a* + miR.494 + miR.518c + miR.639 + miR.660 + miR.338 | 0,81 | 0,152572763 |
| miR.558 + miR.609 + miR.603 + miR.520f + miR.220a + miR.26a + miR.29a + miR.369.3p + miR.376a* + miR.494 + miR.518c + miR.639 + miR.660 + miR.338 | 0,81 | 0,112197842 |
| miR.558 + miR.609 + miR.603 + miR.220a + miR.519b + miR.130a + miR.29a + miR.362 + miR.369.3p + miR.376a* + miR.494 + miR.518c + miR.660 + miR.338 | 0,81 | 0,24638012 |
| miR.558 + miR.609 + miR.603 + miR.520f + miR.220a + miR.130a + miR.26a + miR.29a + miR.369.3p + miR.376a* + miR.518c + miR.639 + miR.660 + miR.338 | 0,81 | 0,155399238 |
| miR.558 + miR.609 + miR.603 + miR.220a + miR.130a + miR.26a + miR.29a + miR.362 + miR.369.3p + miR.376a* + miR.494 + miR.518c + miR.660 + miR.338 | 0,81 | 0,346202758 |
| miR.558 + miR.609 + miR.603 + miR.520f + miR.220a + miR.519b + miR.26a + miR.29a + miR.362 + miR.369.3p + miR.376a* + miR.518c + miR.660 + miR.338 | 0,81 | 0,413635986 |
| miR.558 + miR.609 + miR.603 + miR.220a + miR.519b + miR.26a + miR.29a + miR.362 + miR.369.3p + miR.376a* + miR.494 + miR.518c + miR.639 + miR.660 | 0,81 | 0,310443044 |
| miR.558 + miR.609 + miR.603 + miR.220a + miR.130a + miR.26a + miR.29a + miR.362 + miR.369.3p + miR.376a* + miR.494 + miR.518c + miR.639 + miR.660 | 0,81 | 0,277978248 |
| miR.558 + miR.609 + miR.603 + miR.520f + miR.220a + miR.26a + miR.29a + miR.362 + miR.369.3p + miR.376a* + miR.494 + miR.518c + miR.639 + miR.660 | 0,81 | 0,272404322 |
| miR.558 + miR.609 + miR.603 + miR.520f + miR.220a + miR.519b + miR.130a + miR.29a + miR.362 + miR.369.3p + miR.376a* + miR.518c + miR.660 + miR.338 | 0,81 | 0,444636774 |
| miR.558 + miR.609 + miR.603 + miR.520f + miR.220a + miR.130a + miR.26a + miR.29a + miR.362 + miR.369.3p + miR.376a* + miR.518c + miR.660 + miR.338 | 0,81 | 0,449201083 |
| miR.558 + miR.609 + miR.603 + miR.220a + miR.519b + miR.26a + miR.29a + miR.369.3p + miR.376a* + miR.494 + miR.518c + miR.639 + miR.660 + miR.338 | 0,81 | 0,1163453 |
| miR.558 + miR.609 + miR.603 + miR.220a + miR.519b + miR.130a + miR.29a + miR.369.3p + miR.376a* + miR.494 + miR.518c + miR.639 + miR.660 + miR.338 | 0,81 | 0,130939588 |
| miR.558 + miR.609 + miR.603 + miR.520f + miR.220a + miR.130a + miR.29a + miR.362 + miR.369.3p + miR.376a* + miR.494 + miR.518c + miR.660 + miR.338 | 0,81 | 0,425067482 |
| miR.558 + miR.609 + miR.603 + miR.520f + miR.220a + miR.519b + miR.26a + miR.29a + miR.362 + miR.369.3p + miR.376a* + miR.494 + miR.518c + miR.660 | 0,81 | 0,322956275 |
| miR.558 + miR.609 + miR.603 + miR.520f + miR.220a + miR.519b + miR.26a + miR.29a + miR.369.3p + miR.376a* + miR.518c + miR.639 + miR.660 + miR.338 | 0,81 | 0,200030524 |
| miR.558 + miR.609 + miR.603 + miR.520f + miR.220a + miR.130a + miR.26a + miR.29a + miR.369.3p + miR.376a* + miR.494 + miR.518c + miR.660 + miR.338 | 0,81 | 0,119283338 |
| miR.558 + miR.609 + miR.603 + miR.220a + miR.519b + miR.130a + miR.26a + miR.29a + miR.362 + miR.369.3p + miR.376a* + miR.494 + miR.518c + miR.660 | 0,81 | 0,321776587 |
| miR.558 + miR.609 + miR.603 + miR.519b + miR.26a + miR.29a + miR.362 + miR.369.3p + miR.376a* + miR.494 + miR.518c + miR.639 + miR.660 + miR.338 | 0,81 | 0,263868072 |
| miR.558 + miR.609 + miR.603 + miR.220a + miR.519b + miR.130a + miR.26a + miR.29a + miR.369.3p + miR.376a* + miR.494 + miR.518c + miR.639 + miR.660 | 0,81 | 0,240312996 |
| miR.558 + miR.609 + miR.603 + miR.519b + miR.130a + miR.29a + miR.362 + miR.369.3p + miR.376a* + miR.494 + miR.518c + miR.639 + miR.660 + miR.338 | 0,81 | 0,221884353 |
| miR.558 + miR.609 + miR.520f + miR.220a + miR.519b + miR.26a + miR.29a + miR.362 + miR.369.3p + miR.376a* + miR.494 + miR.518c + miR.660 + miR.338 | 0,81 | 0,194986273 |
| miR.558 + miR.609 + miR.603 + miR.220a + miR.519b + miR.130a + miR.26a + miR.29a + miR.362 + miR.369.3p + miR.376a* + miR.518c + miR.660 + miR.338 | 0,81 | 0,341674651 |
| miR.558 + miR.609 + miR.603 + miR.520f + miR.220a + miR.519b + miR.26a + miR.29a + miR.369.3p + miR.376a* + miR.494 + miR.518c + miR.639 + miR.660 | 0,81 | 0,237751 |
| miR.558 + miR.609 + miR.520f + miR.220a + miR.130a + miR.26a + miR.29a + miR.362 + miR.369.3p + miR.376a* + miR.494 + miR.518c + miR.660 + miR.338 | 0,81 | 0,314605021 |
| miR.558 + miR.609 + miR.603 + miR.520f + miR.220a + miR.519b + miR.130a + miR.26a + miR.29a + miR.369.3p + miR.376a* + miR.494 + miR.518c + miR.660 | 0,81 | 0,261981571 |
| miR.558 + miR.609 + miR.603 + miR.520f + miR.220a + miR.130a + miR.26a + miR.29a + miR.369.3p + miR.376a* + miR.494 + miR.518c + miR.639 + miR.660 | 0,81 | 0,179522142 |
| miR.558 + miR.609 + miR.603 + miR.520f + miR.220a + miR.519b + miR.130a + miR.29a + miR.369.3p + miR.376a* + miR.518c + miR.639 + miR.660 + miR.338 | 0,81 | 0,234449206 |
| miR.558 + miR.609 + miR.520f + miR.220a + miR.519b + miR.130a + miR.29a + miR.362 + miR.369.3p + miR.376a* + miR.494 + miR.518c + miR.660 + miR.338 | 0,81 | 0,181803011 |
| miR.558 + miR.609 + miR.603 + miR.520f + miR.220a + miR.519b + miR.130a + miR.26a + miR.29a + miR.369.3p + miR.376a* + miR.518c + miR.660 + miR.338 | 0,81 | 0,237494151 |
| miR.558 + miR.609 + miR.603 + miR.520f + miR.220a + miR.519b + miR.26a + miR.29a + miR.362 + miR.369.3p + miR.376a* + miR.518c + miR.639 + miR.660 | 0,81 | 0,279903675 |
| miR.558 + miR.609 + miR.603 + miR.220a + miR.519b + miR.130a + miR.29a + miR.362 + miR.369.3p + miR.376a* + miR.518c + miR.639 + miR.660 + miR.338 | 0,81 | 0,399037769 |
| miR.558 + miR.609 + miR.603 + miR.520f + miR.220a + miR.519b + miR.130a + miR.29a + miR.362 + miR.369.3p + miR.376a* + miR.494 + miR.518c + miR.338 | 0,81 | 0,100024204 |
| miR.558 + miR.609 + miR.603 + miR.520f + miR.220a + miR.519b + miR.29a + miR.362 + miR.369.3p + miR.376a* + miR.494 + miR.518c + miR.639 + miR.660 | 0,81 | 0,256471319 |
| miR.558 + miR.609 + miR.603 + miR.520f + miR.220a + miR.519b + miR.26a + miR.29a + miR.362 + miR.369.3p + miR.376a* + miR.494 + miR.518c + miR.338 | 0,81 | 0,113170435 |
| miR.558 + miR.609 + miR.603 + miR.220a + miR.519b + miR.130a + miR.26a + miR.29a + miR.362 + miR.369.3p + miR.376a* + miR.494 + miR.518c + miR.338 | 0,81 | 0,111280177 |
| miR.558 + miR.609 + miR.603 + miR.520f + miR.220a + miR.519b + miR.130a + miR.29a + miR.369.3p + miR.376a* + miR.494 + miR.518c + miR.639 + miR.660 | 0,81 | 0,218350361 |
| miR.558 + miR.609 + miR.220a + miR.519b + miR.26a + miR.29a + miR.362 + miR.369.3p + miR.376a* + miR.494 + miR.518c + miR.639 + miR.660 + miR.338 | 0,81 | 0,448264063 |
| miR.558 + miR.609 + miR.603 + miR.220a + miR.519b + miR.130a + miR.26a + miR.29a + miR.369.3p + miR.376a* + miR.494 + miR.518c + miR.639 + miR.338 | 0,81 | 0,102185025 |
| miR.558 + miR.609 + miR.603 + miR.520f + miR.220a + miR.519b + miR.130a + miR.26a + miR.29a + miR.369.3p + miR.376a* + miR.494 + miR.518c + miR.338 | 0,81 | 0,105466734 |
| miR.558 + miR.609 + miR.603 + miR.220a + miR.519b + miR.130a + miR.29a + miR.362 + miR.369.3p + miR.376a* + miR.494 + miR.518c + miR.639 + miR.660 | 0,81 | 0,207553067 |
| miR.558 + miR.609 + miR.603 + miR.520f + miR.220a + miR.130a + miR.29a + miR.362 + miR.369.3p + miR.376a* + miR.494 + miR.518c + miR.639 + miR.660 | 0,81 | 0,16636663 |
| miR.558 + miR.609 + miR.603 + miR.519b + miR.130a + miR.26a + miR.29a + miR.362 + miR.369.3p + miR.376a* + miR.518c + miR.639 + miR.660 + miR.338 | 0,81 | 0,444626522 |
| miR.558 + miR.609 + miR.603 + miR.220a + miR.519b + miR.29a + miR.362 + miR.369.3p + miR.376a* + miR.494 + miR.518c + miR.639 + miR.660 + miR.338 | 0,81 | 0,273438607 |
| miR.558 + miR.609 + miR.603 + miR.520f + miR.220a + miR.519b + miR.130a + miR.29a + miR.362 + miR.369.3p + miR.376a* + miR.494 + miR.518c + miR.660 | 0,81 | 0,249720873 |
| miR.558 + miR.609 + miR.603 + miR.220a + miR.519b + miR.130a + miR.26a + miR.29a + miR.362 + miR.369.3p + miR.376a* + miR.518c + miR.639 + miR.660 | 0,81 | 0,350206395 |
| miR.558 + miR.609 + miR.603 + miR.520f + miR.220a + miR.519b + miR.26a + miR.29a + miR.369.3p + miR.376a* + miR.494 + miR.518c + miR.639 + miR.338 | 0,81 | 0,096118835 |
| miR.558 + miR.609 + miR.603 + miR.520f + miR.220a + miR.130a + miR.26a + miR.29a + miR.362 + miR.369.3p + miR.376a* + miR.494 + miR.518c + miR.660 | 0,81 | 0,261276008 |
| miR.558 + miR.609 + miR.220a + miR.519b + miR.130a + miR.29a + miR.362 + miR.369.3p + miR.376a* + miR.494 + miR.518c + miR.639 + miR.660 + miR.338 | 0,81 | 0,365517569 |
| miR.558 + miR.609 + miR.603 + miR.520f + miR.220a + miR.130a + miR.26a + miR.29a + miR.362 + miR.369.3p + miR.376a* + miR.518c + miR.639 + miR.660 | 0,81 | 0,326217437 |
| miR.558 + miR.609 + miR.603 + miR.520f + miR.519b + miR.130a + miR.29a + miR.369.3p + miR.376a* + miR.494 + miR.518c + miR.639 + miR.660 + miR.338 | 0,81 | 0,137393885 |
| miR.558 + miR.609 + miR.603 + miR.520f + miR.519b + miR.26a + miR.29a + miR.369.3p + miR.376a* + miR.494 + miR.518c + miR.639 + miR.660 + miR.338 | 0,81 | 0,133512893 |
| miR.558 + miR.609 + miR.603 + miR.220a + miR.519b + miR.26a + miR.29a + miR.362 + miR.369.3p + miR.376a* + miR.494 + miR.518c + miR.639 + miR.338 | 0,81 | 0,1107797 |
| miR.558 + miR.609 + miR.220a + miR.519b + miR.130a + miR.26a + miR.29a + miR.362 + miR.369.3p + miR.376a* + miR.518c + miR.639 + miR.660 + miR.338 | 0,81 | 0,252485157 |
| miR.558 + miR.609 + miR.603 + miR.520f + miR.220a + miR.519b + miR.29a + miR.362 + miR.369.3p + miR.376a* + miR.494 + miR.518c + miR.639 + miR.338 | 0,81 | 0,113182473 |
| miR.558 + miR.609 + miR.603 + miR.520f + miR.220a + miR.26a + miR.29a + miR.362 + miR.369.3p + miR.376a* + miR.494 + miR.518c + miR.639 + miR.338 | 0,81 | 0,048805842 |
| miR.558 + miR.609 + miR.603 + miR.220a + miR.519b + miR.130a + miR.26a + miR.29a + miR.362 + miR.369.3p + miR.376a* + miR.494 + miR.518c + miR.639 | 0,81 | 0,16622317 |
| miR.558 + miR.609 + miR.603 + miR.520f + miR.220a + miR.519b + miR.130a + miR.29a + miR.369.3p + miR.376a* + miR.494 + miR.518c + miR.639 + miR.338 | 0,81 | 0,090110182 |
| miR.558 + miR.609 + miR.603 + miR.520f + miR.130a + miR.26a + miR.29a + miR.369.3p + miR.376a* + miR.494 + miR.518c + miR.639 + miR.660 + miR.338 | 0,81 | 0,182449951 |
| miR.558 + miR.609 + miR.520f + miR.220a + miR.519b + miR.130a + miR.26a + miR.29a + miR.362 + miR.369.3p + miR.376a* + miR.518c + miR.660 + miR.338 | 0,81 | 0,339667117 |
| miR.558 + miR.609 + miR.603 + miR.220a + miR.519b + miR.130a + miR.26a + miR.29a + miR.369.3p + miR.376a* + miR.518c + miR.639 + miR.660 + miR.338 | 0,81 | 0,213405578 |
| miR.558 + miR.609 + miR.603 + miR.520f + miR.220a + miR.519b + miR.130a + miR.26a + miR.29a + miR.362 + miR.369.3p + miR.376a* + miR.494 + miR.518c | 0,81 | 0,158862961 |
| miR.558 + miR.609 + miR.603 + miR.220a + miR.130a + miR.26a + miR.29a + miR.362 + miR.369.3p + miR.376a* + miR.494 + miR.518c + miR.639 + miR.338 | 0,81 | 0,03808596 |
| miR.558 + miR.609 + miR.603 + miR.520f + miR.220a + miR.519b + miR.130a + miR.26a + miR.29a + miR.362 + miR.369.3p + miR.376a* + miR.518c + miR.660 | 0,81 | 0,29861872 |
| miR.558 + miR.609 + miR.520f + miR.220a + miR.519b + miR.26a + miR.29a + miR.369.3p + miR.376a* + miR.494 + miR.518c + miR.639 + miR.660 + miR.338 | 0,81 | 0,373918099 |
| miR.558 + miR.609 + miR.603 + miR.520f + miR.220a + miR.130a + miR.26a + miR.29a + miR.369.3p + miR.376a* + miR.494 + miR.518c + miR.639 + miR.338 | 0,81 | 0,036356881 |
| miR.558 + miR.609 + miR.520f + miR.220a + miR.519b + miR.130a + miR.29a + miR.369.3p + miR.376a* + miR.494 + miR.518c + miR.639 + miR.660 + miR.338 | 0,81 | 0,299950827 |
| miR.558 + miR.609 + miR.520f + miR.519b + miR.130a + miR.26a + miR.29a + miR.362 + miR.369.3p + miR.376a* + miR.494 + miR.518c + miR.660 + miR.338 | 0,81 | 0,439230664 |
| miR.558 + miR.609 + miR.603 + miR.520f + miR.220a + miR.130a + miR.26a + miR.29a + miR.362 + miR.369.3p + miR.376a* + miR.518c + miR.639 + miR.338 | 0,81 | 0,046614267 |
| miR.558 + miR.609 + miR.603 + miR.520f + miR.519b + miR.130a + miR.29a + miR.362 + miR.369.3p + miR.376a* + miR.494 + miR.518c + miR.660 + miR.338 | 0,81 | 0,290005937 |
| miR.558 + miR.609 + miR.603 + miR.520f + miR.519b + miR.26a + miR.29a + miR.362 + miR.369.3p + miR.376a* + miR.494 + miR.518c + miR.660 + miR.338 | 0,81 | 0,327636819 |
| miR.558 + miR.609 + miR.603 + miR.520f + miR.220a + miR.519b + miR.26a + miR.29a + miR.362 + miR.369.3p + miR.376a* + miR.494 + miR.518c + miR.639 | 0,81 | 0,130968282 |
| miR.558 + miR.609 + miR.603 + miR.520f + miR.130a + miR.26a + miR.29a + miR.362 + miR.369.3p + miR.376a* + miR.494 + miR.518c + miR.660 + miR.338 | 0,81 | 0,299221673 |
| miR.558 + miR.609 + miR.603 + miR.520f + miR.220a + miR.519b + miR.130a + miR.29a + miR.362 + miR.369.3p + miR.376a* + miR.494 + miR.518c + miR.639 | 0,81 | 0,127821482 |
| miR.558 + miR.609 + miR.603 + miR.220a + miR.519b + miR.130a + miR.26a + miR.29a + miR.362 + miR.376a* + miR.494 + miR.518c + miR.639 + miR.338 | 0,81 | 0,101359364 |
| miR.558 + miR.609 + miR.603 + miR.520f + miR.220a + miR.519b + miR.130a + miR.26a + miR.29a + miR.369.3p + miR.376a* + miR.494 + miR.518c + miR.639 | 0,81 | 0,149791793 |
| miR.558 + miR.609 + miR.603 + miR.220a + miR.519b + miR.130a + miR.29a + miR.362 + miR.369.3p + miR.376a* + miR.494 + miR.518c + miR.639 + miR.338 | 0,81 | 0,09738086 |
| miR.558 + miR.609 + miR.603 + miR.520f + miR.220a + miR.130a + miR.26a + miR.29a + miR.362 + miR.369.3p + miR.376a* + miR.494 + miR.518c + miR.338 | 0,81 | 0,03168927 |
| miR.558 + miR.609 + miR.520f + miR.220a + miR.130a + miR.26a + miR.29a + miR.369.3p + miR.376a* + miR.494 + miR.518c + miR.639 + miR.660 + miR.338 | 0,81 | 0,268565804 |
| miR.558 + miR.609 + miR.603 + miR.519b + miR.130a + miR.26a + miR.29a + miR.362 + miR.369.3p + miR.376a* + miR.494 + miR.518c + miR.639 + miR.660 | 0,81 | 0,092238665 |
| miR.558 + miR.609 + miR.603 + miR.520f + miR.519b + miR.130a + miR.26a + miR.29a + miR.362 + miR.369.3p + miR.376a* + miR.518c + miR.660 + miR.338 | 0,81 | 0,281097492 |
| miR.558 + miR.609 + miR.603 + miR.519b + miR.130a + miR.26a + miR.29a + miR.369.3p + miR.376a* + miR.494 + miR.518c + miR.639 + miR.660 + miR.338 | 0,81 | 0,196888705 |
| miR.558 + miR.609 + miR.603 + miR.520f + miR.220a + miR.519b + miR.130a + miR.26a + miR.29a + miR.369.3p + miR.376a* + miR.518c + miR.639 + miR.660 | 0,81 | 0,226900096 |
| miR.558 + miR.609 + miR.603 + miR.220a + miR.519b + miR.130a + miR.26a + miR.29a + miR.376a* + miR.494 + miR.518c + miR.639 + miR.660 + miR.338 | 0,81 | 0,111933784 |
| miR.558 + miR.609 + miR.603 + miR.220a + miR.519b + miR.130a + miR.26a + miR.29a + miR.362 + miR.376a* + miR.494 + miR.518c + miR.639 + miR.660 | 0,81 | 0,14868446 |
| miR.558 + miR.609 + miR.603 + miR.520f + miR.130a + miR.26a + miR.29a + miR.362 + miR.369.3p + miR.376a* + miR.494 + miR.518c + miR.639 + miR.660 | 0,81 | 0,046427614 |
| miR.558 + miR.609 + miR.603 + miR.220a + miR.519b + miR.130a + miR.26a + miR.29a + miR.362 + miR.376a* + miR.494 + miR.518c + miR.660 + miR.338 | 0,81 | 0,104198966 |
| miR.558 + miR.609 + miR.520f + miR.519b + miR.130a + miR.26a + miR.29a + miR.369.3p + miR.376a* + miR.494 + miR.518c + miR.639 + miR.660 + miR.338 | 0,81 | 0,274345721 |
| miR.558 + miR.609 + miR.603 + miR.520f + miR.519b + miR.130a + miR.29a + miR.362 + miR.369.3p + miR.376a* + miR.494 + miR.518c + miR.639 + miR.660 | 0,81 | 0,036277883 |
| miR.558 + miR.609 + miR.603 + miR.520f + miR.220a + miR.519b + miR.130a + miR.29a + miR.362 + miR.369.3p + miR.376a* + miR.518c + miR.639 + miR.660 | 0,81 | 0,263892422 |
| miR.558 + miR.609 + miR.603 + miR.520f + miR.220a + miR.130a + miR.26a + miR.29a + miR.362 + miR.369.3p + miR.376a* + miR.494 + miR.518c + miR.639 | 0,81 | 0,07511349 |
| miR.558 + miR.609 + miR.603 + miR.520f + miR.220a + miR.519b + miR.26a + miR.29a + miR.362 + miR.369.3p + miR.376a* + miR.518c + miR.639 + miR.338 | 0,81 | 0,139866116 |
| miR.558 + miR.609 + miR.520f + miR.220a + miR.130a + miR.26a + miR.29a + miR.362 + miR.369.3p + miR.376a* + miR.494 + miR.518c + miR.639 + miR.660 | 0,81 | 0,314959385 |
| miR.558 + miR.609 + miR.220a + miR.519b + miR.130a + miR.26a + miR.29a + miR.362 + miR.369.3p + miR.376a* + miR.494 + miR.518c + miR.660 + miR.338 | 0,81 | 0,489311352 |
| miR.558 + miR.609 + miR.520f + miR.220a + miR.519b + miR.130a + miR.26a + miR.29a + miR.369.3p + miR.376a* + miR.494 + miR.518c + miR.660 + miR.338 | 0,81 | 0,41094556 |
| miR.558 + miR.609 + miR.603 + miR.520f + miR.220a + miR.519b + miR.130a + miR.26a + miR.29a + miR.362 + miR.369.3p + miR.376a* + miR.518c + miR.338 | 0,81 | 0,108427593 |
| miR.558 + miR.609 + miR.603 + miR.520f + miR.519b + miR.130a + miR.26a + miR.29a + miR.369.3p + miR.376a* + miR.518c + miR.639 + miR.660 + miR.338 | 0,81 | 0,098415273 |
| miR.558 + miR.609 + miR.603 + miR.520f + miR.220a + miR.519b + miR.130a + miR.29a + miR.376a* + miR.494 + miR.518c + miR.639 + miR.660 + miR.338 | 0,81 | 0,105732259 |
| miR.558 + miR.609 + miR.520f + miR.220a + miR.519b + miR.130a + miR.26a + miR.29a + miR.369.3p + miR.376a* + miR.518c + miR.639 + miR.660 + miR.338 | 0,81 | 0,296886249 |
| miR.609 + miR.603 + miR.520f + miR.220a + miR.519b + miR.29a + miR.362 + miR.369.3p + miR.376a* + miR.494 + miR.518c + miR.639 + miR.660 + miR.338 | 0,81 | 0,319886768 |
| miR.558 + miR.609 + miR.603 + miR.520f + miR.220a + miR.519b + miR.130a + miR.26a + miR.29a + miR.369.3p + miR.376a* + miR.518c + miR.639 + miR.338 | 0,81 | 0,121671602 |
| miR.558 + miR.609 + miR.603 + miR.520f + miR.220a + miR.519b + miR.29a + miR.362 + miR.376a* + miR.494 + miR.518c + miR.639 + miR.660 + miR.338 | 0,81 | 0,136061834 |
| miR.558 + miR.609 + miR.603 + miR.520f + miR.220a + miR.519b + miR.26a + miR.29a + miR.376a* + miR.494 + miR.518c + miR.639 + miR.660 + miR.338 | 0,81 | 0,124365975 |
| miR.558 + miR.609 + miR.220a + miR.519b + miR.130a + miR.26a + miR.29a + miR.362 + miR.369.3p + miR.376a* + miR.494 + miR.518c + miR.639 + miR.660 | 0,81 | 0,333427402 |
| miR.558 + miR.609 + miR.603 + miR.520f + miR.220a + miR.519b + miR.130a + miR.29a + miR.362 + miR.376a* + miR.494 + miR.518c + miR.639 + miR.338 | 0,81 | 0,097303 |
| miR.558 + miR.609 + miR.220a + miR.519b + miR.130a + miR.26a + miR.29a + miR.369.3p + miR.376a* + miR.494 + miR.518c + miR.639 + miR.660 + miR.338 | 0,81 | 0,312395205 |
| miR.558 + miR.609 + miR.603 + miR.519b + miR.130a + miR.26a + miR.29a + miR.362 + miR.369.3p + miR.376a* + miR.494 + miR.518c + miR.660 + miR.338 | 0,81 | 0,307052334 |
| miR.558 + miR.609 + miR.603 + miR.520f + miR.220a + miR.519b + miR.130a + miR.26a + miR.29a + miR.362 + miR.369.3p + miR.376a* + miR.518c + miR.639 | 0,81 | 0,143607967 |
| miR.558 + miR.609 + miR.603 + miR.220a + miR.519b + miR.130a + miR.29a + miR.362 + miR.376a* + miR.494 + miR.518c + miR.639 + miR.660 + miR.338 | 0,81 | 0,085158628 |
| miR.558 + miR.609 + miR.603 + miR.520f + miR.220a + miR.519b + miR.130a + miR.26a + miR.29a + miR.362 + miR.376a* + miR.494 + miR.518c + miR.338 | 0,81 | 0,087989213 |
| miR.558 + miR.609 + miR.603 + miR.520f + miR.220a + miR.519b + miR.130a + miR.29a + miR.362 + miR.369.3p + miR.376a* + miR.518c + miR.639 + miR.338 | 0,81 | 0,122044136 |
| miR.558 + miR.609 + miR.603 + miR.520f + miR.220a + miR.519b + miR.130a + miR.26a + miR.29a + miR.376a* + miR.494 + miR.518c + miR.660 + miR.338 | 0,81 | 0,148631783 |
| miR.558 + miR.609 + miR.603 + miR.520f + miR.220a + miR.519b + miR.130a + miR.29a + miR.362 + miR.376a* + miR.494 + miR.518c + miR.660 + miR.338 | 0,81 | 0,144800205 |
| miR.558 + miR.609 + miR.520f + miR.220a + miR.519b + miR.130a + miR.29a + miR.362 + miR.369.3p + miR.376a* + miR.494 + miR.518c + miR.639 + miR.660 | 0,81 | 0,336454773 |
| miR.558 + miR.609 + miR.603 + miR.520f + miR.220a + miR.519b + miR.130a + miR.26a + miR.29a + miR.362 + miR.376a* + miR.494 + miR.518c + miR.639 | 0,81 | 0,128458988 |
| miR.558 + miR.609 + miR.603 + miR.520f + miR.519b + miR.130a + miR.26a + miR.29a + miR.369.3p + miR.376a* + miR.494 + miR.518c + miR.639 + miR.338 | 0,81 | 0,065330518 |
| miR.558 + miR.609 + miR.603 + miR.520f + miR.519b + miR.130a + miR.26a + miR.29a + miR.369.3p + miR.376a* + miR.494 + miR.518c + miR.660 + miR.338 | 0,81 | 0,167923448 |
| miR.558 + miR.609 + miR.603 + miR.520f + miR.220a + miR.519b + miR.26a + miR.29a + miR.362 + miR.376a* + miR.494 + miR.518c + miR.660 + miR.338 | 0,81 | 0,208264538 |
| miR.558 + miR.609 + miR.603 + miR.520f + miR.220a + miR.519b + miR.26a + miR.29a + miR.362 + miR.376a* + miR.494 + miR.518c + miR.639 + miR.338 | 0,81 | 0,105567471 |
| miR.558 + miR.609 + miR.603 + miR.520f + miR.519b + miR.130a + miR.29a + miR.362 + miR.369.3p + miR.376a* + miR.494 + miR.518c + miR.639 + miR.338 | 0,81 | 0,069525217 |
| miR.558 + miR.609 + miR.603 + miR.220a + miR.519b + miR.130a + miR.26a + miR.29a + miR.362 + miR.369.3p + miR.376a* + miR.518c + miR.639 + miR.338 | 0,81 | 0,127177552 |
| miR.558 + miR.609 + miR.603 + miR.520f + miR.130a + miR.26a + miR.29a + miR.362 + miR.369.3p + miR.376a* + miR.494 + miR.518c + miR.639 + miR.338 | 0,81 | 0,064621923 |
| miR.558 + miR.609 + miR.603 + miR.520f + miR.220a + miR.519b + miR.130a + miR.29a + miR.362 + miR.376a* + miR.494 + miR.518c + miR.639 + miR.660 | 0,81 | 0,151849469 |
| miR.558 + miR.609 + miR.520f + miR.220a + miR.519b + miR.26a + miR.29a + miR.362 + miR.369.3p + miR.376a* + miR.494 + miR.518c + miR.639 + miR.660 | 0,81 | 0,28758458 |
| miR.558 + miR.609 + miR.603 + miR.520f + miR.220a + miR.519b + miR.130a + miR.26a + miR.29a + miR.376a* + miR.494 + miR.518c + miR.639 + miR.660 | 0,81 | 0,151317681 |
| miR.558 + miR.609 + miR.603 + miR.520f + miR.220a + miR.519b + miR.130a + miR.26a + miR.29a + miR.362 + miR.376a* + miR.494 + miR.518c + miR.660 | 0,81 | 0,172147629 |
| miR.558 + miR.609 + miR.603 + miR.520f + miR.220a + miR.26a + miR.29a + miR.362 + miR.376a* + miR.494 + miR.518c + miR.639 + miR.660 + miR.338 | 0,81 | 0,026586742 |
| miR.558 + miR.609 + miR.520f + miR.519b + miR.130a + miR.26a + miR.29a + miR.362 + miR.369.3p + miR.376a* + miR.494 + miR.518c + miR.639 + miR.660 | 0,81 | 0,094490807 |
| miR.558 + miR.609 + miR.603 + miR.520f + miR.220a + miR.130a + miR.29a + miR.362 + miR.369.3p + miR.376a* + miR.494 + miR.518c + miR.639 + miR.338 | 0,81 | 0,021233368 |
| miR.558 + miR.609 + miR.603 + miR.520f + miR.220a + miR.130a + miR.29a + miR.362 + miR.376a* + miR.494 + miR.518c + miR.639 + miR.660 + miR.338 | 0,81 | 0,013871553 |
| miR.558 + miR.609 + miR.603 + miR.520f + miR.220a + miR.130a + miR.26a + miR.29a + miR.376a* + miR.494 + miR.518c + miR.639 + miR.660 + miR.338 | 0,81 | 0,020594396 |
| miR.558 + miR.609 + miR.603 + miR.520f + miR.220a + miR.519b + miR.26a + miR.29a + miR.362 + miR.376a* + miR.494 + miR.518c + miR.639 + miR.660 | 0,81 | 0,164857512 |
| miR.558 + miR.609 + miR.603 + miR.220a + miR.130a + miR.26a + miR.29a + miR.362 + miR.376a* + miR.494 + miR.518c + miR.639 + miR.660 + miR.338 | 0,81 | 0,043424385 |
| miR.558 + miR.609 + miR.603 + miR.520f + miR.519b + miR.130a + miR.26a + miR.29a + miR.369.3p + miR.376a* + miR.494 + miR.518c + miR.639 + miR.660 | 0,81 | 0,120865418 |
| miR.558 + miR.609 + miR.520f + miR.220a + miR.519b + miR.130a + miR.26a + miR.29a + miR.369.3p + miR.376a* + miR.494 + miR.518c + miR.639 + miR.660 | 0,81 | 0,281340937 |
| miR.558 + miR.609 + miR.603 + miR.519b + miR.130a + miR.26a + miR.29a + miR.362 + miR.369.3p + miR.376a* + miR.494 + miR.518c + miR.639 + miR.338 | 0,81 | 0,15119666 |
| miR.558 + miR.609 + miR.520f + miR.220a + miR.519b + miR.130a + miR.26a + miR.29a + miR.362 + miR.369.3p + miR.376a* + miR.494 + miR.518c + miR.660 | 0,81 | 0,406136722 |
| miR.609 + miR.603 + miR.520f + miR.220a + miR.26a + miR.29a + miR.362 + miR.369.3p + miR.376a* + miR.494 + miR.518c + miR.639 + miR.660 + miR.338 | 0,81 | 0,136852887 |
| miR.558 + miR.609 + miR.520f + miR.220a + miR.519b + miR.130a + miR.26a + miR.29a + miR.362 + miR.369.3p + miR.376a* + miR.518c + miR.639 + miR.660 | 0,81 | 0,338614254 |
| miR.609 + miR.603 + miR.520f + miR.220a + miR.130a + miR.29a + miR.362 + miR.369.3p + miR.376a* + miR.494 + miR.518c + miR.639 + miR.660 + miR.338 | 0,81 | 0,14186496 |
| miR.558 + miR.609 + miR.603 + miR.520f + miR.519b + miR.26a + miR.29a + miR.362 + miR.369.3p + miR.376a* + miR.494 + miR.518c + miR.639 + miR.660 | 0,81 | 0,047922139 |
| miR.558 + miR.609 + miR.603 + miR.520f + miR.519b + miR.26a + miR.29a + miR.362 + miR.369.3p + miR.376a* + miR.494 + miR.518c + miR.639 + miR.338 | 0,81 | 0,07310338 |
| miR.558 + miR.609 + miR.603 + miR.520f + miR.220a + miR.130a + miR.26a + miR.29a + miR.362 + miR.376a* + miR.494 + miR.518c + miR.639 + miR.338 | 0,81 | 0,024917674 |
| miR.558 + miR.609 + miR.520f + miR.220a + miR.130a + miR.26a + miR.29a + miR.362 + miR.369.3p + miR.376a* + miR.494 + miR.518c + miR.639 + miR.338 | 0,81 | 0,096809607 |
| miR.558 + miR.609 + miR.603 + miR.520f + miR.519b + miR.130a + miR.26a + miR.29a + miR.362 + miR.369.3p + miR.376a* + miR.494 + miR.518c + miR.338 | 0,81 | 0,104729022 |
| miR.558 + miR.609 + miR.603 + miR.220a + miR.519b + miR.26a + miR.29a + miR.362 + miR.376a* + miR.494 + miR.518c + miR.639 + miR.660 + miR.338 | 0,81 | 0,126125163 |
| miR.558 + miR.609 + miR.603 + miR.520f + miR.220a + miR.519b + miR.130a + miR.26a + miR.29a + miR.362 + miR.376a* + miR.518c + miR.639 + miR.338 | 0,81 | 0,103732939 |
| miR.558 + miR.609 + miR.603 + miR.520f + miR.220a + miR.519b + miR.130a + miR.26a + miR.29a + miR.376a* + miR.494 + miR.518c + miR.639 + miR.338 | 0,81 | 0,038656582 |
| miR.558 + miR.609 + miR.520f + miR.220a + miR.519b + miR.130a + miR.29a + miR.362 + miR.376a* + miR.494 + miR.518c + miR.639 + miR.660 + miR.338 | 0,81 | 0,175993851 |
| miR.558 + miR.609 + miR.603 + miR.520f + miR.519b + miR.130a + miR.26a + miR.29a + miR.362 + miR.369.3p + miR.376a* + miR.518c + miR.639 + miR.338 | 0,81 | 0,063042743 |
| miR.558 + miR.609 + miR.603 + miR.520f + miR.220a + miR.130a + miR.362 + miR.369.3p + miR.376a* + miR.494 + miR.518c + miR.639 + miR.660 + miR.338 | 0,81 | 0,134146886 |
| miR.558 + miR.609 + miR.520f + miR.220a + miR.130a + miR.26a + miR.29a + miR.362 + miR.376a* + miR.494 + miR.518c + miR.639 + miR.660 + miR.338 | 0,81 | 0,06959903 |
| miR.609 + miR.603 + miR.220a + miR.130a + miR.26a + miR.29a + miR.362 + miR.369.3p + miR.376a* + miR.494 + miR.518c + miR.639 + miR.660 + miR.338 | 0,81 | 0,381927294 |
| miR.558 + miR.609 + miR.603 + miR.520f + miR.220a + miR.130a + miR.26a + miR.29a + miR.362 + miR.376a* + miR.494 + miR.518c + miR.639 + miR.660 | 0,81 | 0,042926309 |
| miR.609 + miR.603 + miR.520f + miR.220a + miR.519b + miR.130a + miR.29a + miR.362 + miR.369.3p + miR.376a* + miR.494 + miR.518c + miR.660 + miR.338 | 0,81 | 0,39643341 |
| miR.558 + miR.609 + miR.603 + miR.520f + miR.220a + miR.130a + miR.26a + miR.362 + miR.369.3p + miR.376a* + miR.518c + miR.639 + miR.660 + miR.338 | 0,81 | 0,154550367 |
| miR.558 + miR.609 + miR.520f + miR.220a + miR.519b + miR.26a + miR.29a + miR.362 + miR.376a* + miR.494 + miR.518c + miR.639 + miR.660 + miR.338 | 0,81 | 0,24815357 |
| miR.558 + miR.609 + miR.520f + miR.220a + miR.519b + miR.130a + miR.26a + miR.29a + miR.376a* + miR.494 + miR.518c + miR.639 + miR.660 + miR.338 | 0,81 | 0,17067642 |
| miR.609 + miR.603 + miR.520f + miR.220a + miR.519b + miR.26a + miR.29a + miR.362 + miR.369.3p + miR.376a* + miR.494 + miR.518c + miR.660 + miR.338 | 0,81 | 0,396187172 |
| miR.609 + miR.603 + miR.220a + miR.519b + miR.26a + miR.29a + miR.362 + miR.369.3p + miR.376a* + miR.494 + miR.518c + miR.639 + miR.660 + miR.338 | 0,81 | 0,388457399 |
| miR.558 + miR.609 + miR.603 + miR.520f + miR.220a + miR.26a + miR.362 + miR.369.3p + miR.376a* + miR.494 + miR.518c + miR.639 + miR.660 + miR.338 | 0,81 | 0,122979609 |
| miR.558 + miR.609 + miR.520f + miR.220a + miR.519b + miR.26a + miR.29a + miR.362 + miR.369.3p + miR.376a* + miR.494 + miR.518c + miR.639 + miR.338 | 0,81 | 0,156384689 |
| miR.558 + miR.609 + miR.603 + miR.520f + miR.220a + miR.519b + miR.130a + miR.362 + miR.369.3p + miR.376a* + miR.518c + miR.639 + miR.660 + miR.338 | 0,81 | 0,141322411 |
| miR.609 + miR.603 + miR.520f + miR.519b + miR.130a + miR.29a + miR.362 + miR.369.3p + miR.376a* + miR.494 + miR.518c + miR.639 + miR.660 + miR.338 | 0,81 | 0,389267118 |
| miR.609 + miR.603 + miR.520f + miR.519b + miR.26a + miR.29a + miR.362 + miR.369.3p + miR.376a* + miR.494 + miR.518c + miR.639 + miR.660 + miR.338 | 0,81 | 0,43469556 |
| miR.558 + miR.609 + miR.603 + miR.520f + miR.220a + miR.130a + miR.26a + miR.29a + miR.362 + miR.376a* + miR.494 + miR.518c + miR.660 + miR.338 | 0,81 | 0,049259581 |
| miR.609 + miR.603 + miR.520f + miR.130a + miR.26a + miR.29a + miR.362 + miR.369.3p + miR.376a* + miR.494 + miR.518c + miR.639 + miR.660 + miR.338 | 0,81 | 0,35904379 |
| miR.609 + miR.603 + miR.520f + miR.220a + miR.130a + miR.26a + miR.29a + miR.362 + miR.369.3p + miR.376a* + miR.494 + miR.518c + miR.660 + miR.338 | 0,81 | 0,448842699 |
| miR.558 + miR.609 + miR.603 + miR.520f + miR.519b + miR.130a + miR.362 + miR.369.3p + miR.376a* + miR.494 + miR.518c + miR.639 + miR.660 + miR.338 | 0,81 | 0,160209184 |
| miR.558 + miR.609 + miR.520f + miR.220a + miR.519b + miR.130a + miR.26a + miR.29a + miR.362 + miR.369.3p + miR.376a* + miR.494 + miR.518c + miR.338 | 0,81 | 0,140657418 |
| miR.558 + miR.609 + miR.603 + miR.520f + miR.519b + miR.130a + miR.29a + miR.362 + miR.376a* + miR.494 + miR.518c + miR.639 + miR.660 + miR.338 | 0,81 | 0,07626918 |
| miR.558 + miR.609 + miR.603 + miR.520f + miR.519b + miR.130a + miR.26a + miR.29a + miR.362 + miR.376a* + miR.494 + miR.518c + miR.639 + miR.338 | 0,81 | 0,076877765 |
| miR.609 + miR.603 + miR.520f + miR.220a + miR.519b + miR.130a + miR.29a + miR.369.3p + miR.376a* + miR.494 + miR.518c + miR.639 + miR.660 + miR.338 | 0,81 | 0,13790433 |
| miR.558 + miR.609 + miR.520f + miR.220a + miR.519b + miR.130a + miR.26a + miR.29a + miR.369.3p + miR.376a* + miR.494 + miR.518c + miR.639 + miR.338 | 0,81 | 0,129387255 |
| miR.558 + miR.609 + miR.603 + miR.520f + miR.220a + miR.130a + miR.26a + miR.29a + miR.362 + miR.376a* + miR.518c + miR.639 + miR.660 + miR.338 | 0,81 | 0,031372301 |
| miR.558 + miR.609 + miR.520f + miR.220a + miR.519b + miR.130a + miR.29a + miR.362 + miR.369.3p + miR.376a* + miR.494 + miR.518c + miR.639 + miR.338 | 0,81 | 0,181625827 |
| miR.558 + miR.609 + miR.603 + miR.520f + miR.220a + miR.519b + miR.130a + miR.26a + miR.29a + miR.362 + miR.376a* + miR.518c + miR.639 + miR.660 | 0,81 | 0,136519596 |
| miR.558 + miR.609 + miR.603 + miR.520f + miR.220a + miR.519b + miR.362 + miR.369.3p + miR.376a* + miR.494 + miR.518c + miR.639 + miR.660 + miR.338 | 0,81 | 0,089797649 |
| miR.558 + miR.609 + miR.603 + miR.520f + miR.519b + miR.130a + miR.26a + miR.29a + miR.362 + miR.369.3p + miR.376a* + miR.494 + miR.518c + miR.639 | 0,81 | 0,098636684 |
| miR.558 + miR.609 + miR.220a + miR.519b + miR.130a + miR.26a + miR.29a + miR.362 + miR.369.3p + miR.376a* + miR.494 + miR.518c + miR.639 + miR.338 | 0,81 | 0,110669023 |
| miR.558 + miR.609 + miR.520f + miR.519b + miR.130a + miR.26a + miR.29a + miR.362 + miR.376a* + miR.494 + miR.518c + miR.639 + miR.660 + miR.338 | 0,81 | 0,073619589 |
| miR.558 + miR.609 + miR.603 + miR.520f + miR.519b + miR.130a + miR.26a + miR.362 + miR.369.3p + miR.376a* + miR.518c + miR.639 + miR.660 + miR.338 | 0,81 | 0,183658291 |
| miR.558 + miR.609 + miR.603 + miR.520f + miR.130a + miR.26a + miR.362 + miR.369.3p + miR.376a* + miR.494 + miR.518c + miR.639 + miR.660 + miR.338 | 0,81 | 0,169680354 |
| miR.558 + miR.609 + miR.603 + miR.520f + miR.519b + miR.130a + miR.26a + miR.29a + miR.362 + miR.369.3p + miR.376a* + miR.518c + miR.639 + miR.660 | 0,81 | 0,057581379 |
| miR.558 + miR.609 + miR.603 + miR.220a + miR.519b + miR.130a + miR.26a + miR.29a + miR.362 + miR.376a* + miR.518c + miR.639 + miR.660 + miR.338 | 0,81 | 0,093708983 |
| miR.609 + miR.603 + miR.520f + miR.220a + miR.519b + miR.26a + miR.29a + miR.369.3p + miR.376a* + miR.494 + miR.518c + miR.639 + miR.660 + miR.338 | 0,81 | 0,136525761 |
| miR.558 + miR.609 + miR.603 + miR.520f + miR.220a + miR.519b + miR.130a + miR.29a + miR.362 + miR.376a* + miR.518c + miR.639 + miR.660 + miR.338 | 0,81 | 0,087714608 |
| miR.609 + miR.520f + miR.220a + miR.519b + miR.130a + miR.29a + miR.362 + miR.369.3p + miR.376a* + miR.494 + miR.518c + miR.639 + miR.660 + miR.338 | 0,81 | 0,372144025 |
| miR.558 + miR.609 + miR.520f + miR.220a + miR.519b + miR.130a + miR.26a + miR.29a + miR.362 + miR.376a* + miR.494 + miR.518c + miR.660 + miR.338 | 0,81 | 0,264678188 |
| miR.609 + miR.603 + miR.520f + miR.220a + miR.519b + miR.130a + miR.29a + miR.362 + miR.369.3p + miR.376a* + miR.494 + miR.518c + miR.639 + miR.660 | 0,81 | 0,117462912 |
| miR.558 + miR.609 + miR.520f + miR.220a + miR.519b + miR.130a + miR.26a + miR.29a + miR.362 + miR.369.3p + miR.376a* + miR.518c + miR.639 + miR.338 | 0,81 | 0,114780583 |
| miR.609 + miR.520f + miR.220a + miR.519b + miR.26a + miR.29a + miR.362 + miR.369.3p + miR.376a* + miR.494 + miR.518c + miR.639 + miR.660 + miR.338 | 0,81 | 0,316032605 |
| miR.609 + miR.603 + miR.520f + miR.220a + miR.519b + miR.26a + miR.29a + miR.362 + miR.369.3p + miR.376a* + miR.494 + miR.518c + miR.639 + miR.660 | 0,81 | 0,137540765 |
| miR.558 + miR.609 + miR.603 + miR.520f + miR.220a + miR.519b + miR.26a + miR.29a + miR.362 + miR.376a* + miR.518c + miR.639 + miR.660 + miR.338 | 0,81 | 0,090969586 |
| miR.558 + miR.609 + miR.603 + miR.520f + miR.519b + miR.26a + miR.29a + miR.362 + miR.376a* + miR.494 + miR.518c + miR.639 + miR.660 + miR.338 | 0,81 | 0,042006553 |
| miR.558 + miR.609 + miR.603 + miR.520f + miR.220a + miR.519b + miR.130a + miR.26a + miR.29a + miR.376a* + miR.518c + miR.639 + miR.660 + miR.338 | 0,81 | 0,09272833 |
| miR.558 + miR.609 + miR.603 + miR.520f + miR.220a + miR.519b + miR.26a + miR.362 + miR.369.3p + miR.376a* + miR.518c + miR.639 + miR.660 + miR.338 | 0,81 | 0,108268386 |
| miR.558 + miR.609 + miR.520f + miR.519b + miR.130a + miR.26a + miR.29a + miR.362 + miR.369.3p + miR.376a* + miR.494 + miR.518c + miR.639 + miR.338 | 0,81 | 0,181426425 |
| miR.558 + miR.609 + miR.603 + miR.520f + miR.130a + miR.26a + miR.29a + miR.362 + miR.376a* + miR.494 + miR.518c + miR.639 + miR.660 + miR.338 | 0,81 | 0,01410596 |
| miR.609 + miR.603 + miR.220a + miR.519b + miR.130a + miR.29a + miR.362 + miR.369.3p + miR.376a* + miR.494 + miR.518c + miR.639 + miR.660 + miR.338 | 0,81 | 0,302330031 |
| miR.558 + miR.609 + miR.520f + miR.220a + miR.519b + miR.130a + miR.26a + miR.29a + miR.362 + miR.376a* + miR.494 + miR.518c + miR.639 + miR.338 | 0,81 | 0,169836384 |
| miR.558 + miR.609 + miR.520f + miR.220a + miR.519b + miR.130a + miR.26a + miR.29a + miR.362 + miR.369.3p + miR.376a* + miR.494 + miR.518c + miR.639 | 0,81 | 0,186796637 |
| miR.609 + miR.603 + miR.520f + miR.220a + miR.130a + miR.26a + miR.29a + miR.362 + miR.369.3p + miR.376a* + miR.494 + miR.518c + miR.639 + miR.660 | 0,81 | 0,077352304 |
| miR.609 + miR.603 + miR.520f + miR.220a + miR.519b + miR.130a + miR.26a + miR.29a + miR.369.3p + miR.376a* + miR.494 + miR.518c + miR.660 + miR.338 | 0,81 | 0,214717654 |
| miR.558 + miR.609 + miR.603 + miR.520f + miR.220a + miR.130a + miR.29a + miR.362 + miR.369.3p + miR.376a* + miR.494 + miR.639 + miR.660 + miR.338 | 0,81 | 0,261582877 |
| miR.558 + miR.609 + miR.603 + miR.520f + miR.220a + miR.519b + miR.130a + miR.26a + miR.29a + miR.362 + miR.376a* + miR.518c + miR.660 + miR.338 | 0,81 | 0,161945913 |
| miR.609 + miR.603 + miR.520f + miR.220a + miR.519b + miR.130a + miR.29a + miR.362 + miR.369.3p + miR.376a* + miR.518c + miR.639 + miR.660 + miR.338 | 0,81 | 0,291294383 |
| miR.558 + miR.609 + miR.603 + miR.520f + miR.519b + miR.130a + miR.26a + miR.29a + miR.376a* + miR.494 + miR.518c + miR.639 + miR.660 + miR.338 | 0,81 | 0,080526782 |
| miR.558 + miR.609 + miR.520f + miR.220a + miR.130a + miR.26a + miR.29a + miR.362 + miR.369.3p + miR.376a* + miR.494 + miR.639 + miR.660 + miR.338 | 0,81 | 0,248731002 |
| miR.609 + miR.603 + miR.520f + miR.220a + miR.130a + miR.26a + miR.29a + miR.362 + miR.369.3p + miR.376a* + miR.518c + miR.639 + miR.660 + miR.338 | 0,81 | 0,283710814 |
| miR.609 + miR.603 + miR.220a + miR.519b + miR.130a + miR.26a + miR.29a + miR.362 + miR.369.3p + miR.376a* + miR.494 + miR.518c + miR.660 + miR.338 | 0,81 | 0,419568865 |
| miR.609 + miR.603 + miR.220a + miR.519b + miR.130a + miR.26a + miR.29a + miR.362 + miR.369.3p + miR.376a* + miR.494 + miR.518c + miR.639 + miR.660 | 0,81 | 0,180432995 |
| miR.558 + miR.609 + miR.520f + miR.220a + miR.519b + miR.130a + miR.29a + miR.362 + miR.369.3p + miR.376a* + miR.494 + miR.639 + miR.660 + miR.338 | 0,81 | 0,243311863 |
| miR.558 + miR.609 + miR.603 + miR.520f + miR.130a + miR.26a + miR.29a + miR.362 + miR.369.3p + miR.376a* + miR.494 + miR.639 + miR.660 + miR.338 | 0,81 | 0,286216354 |
| miR.558 + miR.609 + miR.603 + miR.520f + miR.519b + miR.130a + miR.29a + miR.362 + miR.369.3p + miR.376a* + miR.494 + miR.639 + miR.660 + miR.338 | 0,81 | 0,251021462 |
| miR.558 + miR.609 + miR.603 + miR.520f + miR.519b + miR.130a + miR.26a + miR.29a + miR.362 + miR.369.3p + miR.376a* + miR.494 + miR.518c + miR.660 | 0,81 | 0,154399614 |
| miR.609 + miR.603 + miR.520f + miR.220a + miR.519b + miR.130a + miR.26a + miR.29a + miR.362 + miR.369.3p + miR.376a* + miR.494 + miR.518c + miR.660 | 0,81 | 0,180502926 |
| miR.558 + miR.609 + miR.520f + miR.519b + miR.130a + miR.26a + miR.29a + miR.362 + miR.369.3p + miR.376a* + miR.494 + miR.639 + miR.660 + miR.338 | 0,81 | 0,290119402 |
| miR.558 + miR.609 + miR.603 + miR.520f + miR.220a + miR.26a + miR.29a + miR.362 + miR.369.3p + miR.376a* + miR.494 + miR.639 + miR.660 + miR.338 | 0,81 | 0,305026907 |
| miR.558 + miR.609 + miR.520f + miR.220a + miR.519b + miR.130a + miR.26a + miR.29a + miR.362 + miR.376a* + miR.494 + miR.518c + miR.639 + miR.660 | 0,81 | 0,183012623 |
| miR.558 + miR.609 + miR.603 + miR.520f + miR.519b + miR.26a + miR.362 + miR.369.3p + miR.376a* + miR.494 + miR.518c + miR.639 + miR.660 + miR.338 | 0,81 | 0,133969406 |
| miR.609 + miR.520f + miR.220a + miR.130a + miR.26a + miR.29a + miR.362 + miR.369.3p + miR.376a* + miR.494 + miR.518c + miR.639 + miR.660 + miR.338 | 0,81 | 0,353970356 |
| miR.609 + miR.603 + miR.520f + miR.220a + miR.519b + miR.26a + miR.29a + miR.362 + miR.369.3p + miR.376a* + miR.518c + miR.639 + miR.660 + miR.338 | 0,81 | 0,320805885 |
| miR.558 + miR.609 + miR.603 + miR.520f + miR.220a + miR.519b + miR.29a + miR.362 + miR.369.3p + miR.376a* + miR.494 + miR.639 + miR.660 + miR.338 | 0,81 | 0,277875226 |
| miR.558 + miR.609 + miR.520f + miR.220a + miR.519b + miR.26a + miR.29a + miR.362 + miR.369.3p + miR.376a* + miR.494 + miR.639 + miR.660 + miR.338 | 0,81 | 0,302816376 |
| miR.558 + miR.609 + miR.520f + miR.220a + miR.519b + miR.130a + miR.362 + miR.369.3p + miR.376a* + miR.494 + miR.518c + miR.639 + miR.660 + miR.338 | 0,81 | 0,160458522 |
| miR.609 + miR.603 + miR.520f + miR.519b + miR.130a + miR.26a + miR.29a + miR.362 + miR.369.3p + miR.376a* + miR.518c + miR.639 + miR.660 + miR.338 | 0,81 | 0,436247623 |
| miR.609 + miR.603 + miR.220a + miR.519b + miR.130a + miR.26a + miR.29a + miR.369.3p + miR.376a* + miR.494 + miR.518c + miR.639 + miR.660 + miR.338 | 0,81 | 0,126768635 |
| miR.609 + miR.603 + miR.520f + miR.220a + miR.519b + miR.130a + miR.26a + miR.29a + miR.369.3p + miR.376a* + miR.494 + miR.518c + miR.639 + miR.660 | 0,81 | 0,094746111 |
| miR.558 + miR.609 + miR.603 + miR.520f + miR.519b + miR.130a + miR.26a + miR.29a + miR.362 + miR.376a* + miR.494 + miR.518c + miR.660 + miR.338 | 0,81 | 0,120212301 |
| miR.558 + miR.609 + miR.220a + miR.519b + miR.130a + miR.26a + miR.29a + miR.362 + miR.376a* + miR.494 + miR.518c + miR.639 + miR.660 + miR.338 | 0,81 | 0,131 7627 |
| miR.558 + miR.609 + miR.520f + miR.220a + miR.519b + miR.130a + miR.26a + miR.29a + miR.362 + miR.376a* + miR.518c + miR.639 + miR.660 + miR.338 | 0,81 | 0,123149461 |
| miR.558 + miR.609 + miR.603 + miR.520f + miR.519b + miR.26a + miR.29a + miR.362 + miR.369.3p + miR.376a* + miR.494 + miR.639 + miR.660 + miR.338 | 0,81 | 0,328620869 |
| miR.609 + miR.520f + miR.220a + miR.519b + miR.130a + miR.26a + miR.29a + miR.362 + miR.369.3p + miR.376a* + miR.518c + miR.639 + miR.660 + miR.338 | 0,81 | 0,341948655 |
| miR.609 + miR.520f + miR.519b + miR.130a + miR.26a + miR.29a + miR.362 + miR.369.3p + miR.376a* + miR.494 + miR.518c + miR.639 + miR.660 + miR.338 | 0,81 | 0,488586805 |
| miR.609 + miR.603 + miR.520f + miR.220a + miR.519b + miR.130a + miR.26a + miR.29a + miR.362 + miR.369.3p + miR.376a* + miR.518c + miR.660 + miR.338 | 0,81 | 0,368413093 |
| miR.558 + miR.609 + miR.603 + miR.520f + miR.519b + miR.130a + miR.26a + miR.29a + miR.362 + miR.376a* + miR.518c + miR.639 + miR.660 + miR.338 | 0,81 | 0,058905172 |
| miR.609 + miR.603 + miR.520f + miR.220a + miR.130a + miR.26a + miR.29a + miR.369.3p + miR.376a* + miR.494 + miR.518c + miR.639 + miR.660 + miR.338 | 0,81 | 0,082143766 |
| miR.609 + miR.603 + miR.520f + miR.220a + miR.519b + miR.130a + miR.26a + miR.29a + miR.362 + miR.369.3p + miR.376a* + miR.518c + miR.639 + miR.660 | 0,81 | 0,222557881 |
| miR.558 + miR.609 + miR.603 + miR.520f + miR.519b + miR.130a + miR.26a + miR.29a + miR.362 + miR.376a* + miR.494 + miR.518c + miR.639 + miR.660 | 0,81 | 0,085203912 |
| miR.558 + miR.609 + miR.520f + miR.220a + miR.519b + miR.130a + miR.26a + miR.362 + miR.369.3p + miR.376a* + miR.518c + miR.639 + miR.660 + miR.338 | 0,81 | 0,180493636 |
| miR.558 + miR.609 + miR.520f + miR.220a + miR.130a + miR.26a + miR.362 + miR.369.3p + miR.376a* + miR.494 + miR.518c + miR.639 + miR.660 + miR.338 | 0,81 | 0,16247103 |
| miR.558 + miR.609 + miR.520f + miR.519b + miR.130a + miR.26a + miR.362 + miR.369.3p + miR.376a* + miR.494 + miR.518c + miR.639 + miR.660 + miR.338 | 0,81 | 0,235809401 |
| miR.558 + miR.609 + miR.603 + miR.519b + miR.130a + miR.26a + miR.29a + miR.362 + miR.376a* + miR.494 + miR.518c + miR.639 + miR.660 + miR.338 | 0,81 | 0,026730385 |
| miR.609 + miR.603 + miR.220a + miR.519b + miR.130a + miR.26a + miR.29a + miR.362 + miR.369.3p + miR.376a* + miR.518c + miR.639 + miR.660 + miR.338 | 0,81 | 0,425981471 |
| miR.558 + miR.609 + miR.520f + miR.220a + miR.519b + miR.26a + miR.362 + miR.369.3p + miR.376a* + miR.494 + miR.518c + miR.639 + miR.660 + miR.338 | 0,81 | 0,162260334 |
| miR.609 + miR.603 + miR.520f + miR.220a + miR.519b + miR.130a + miR.26a + miR.29a + miR.369.3p + miR.376a* + miR.518c + miR.639 + miR.660 + miR.338 | 0,81 | 0,214705087 |
| miR.558 + miR.609 + miR.603 + miR.520f + miR.220a + miR.130a + miR.26a + miR.362 + miR.369.3p + miR.376a* + miR.494 + miR.518c + miR.639 + miR.660 | 0,81 | 0,023712127 |
| miR.558 + miR.609 + miR.603 + miR.520f + miR.220a + miR.519b + miR.130a + miR.362 + miR.369.3p + miR.376a* + miR.494 + miR.518c + miR.639 + miR.660 | 0,81 | 0,024618759 |
| miR.558 + miR.609 + miR.603 + miR.520f + miR.220a + miR.519b + miR.130a + miR.26a + miR.362 + miR.369.3p + miR.376a* + miR.518c + miR.639 + miR.660 | 0,81 | 0,024608771 |
| miR.609 + miR.520f + miR.220a + miR.519b + miR.130a + miR.26a + miR.29a + miR.362 + miR.369.3p + miR.376a* + miR.494 + miR.518c + miR.660 + miR.338 | 0,81 | 0,414060871 |
| miR.558 + miR.609 + miR.603 + miR.520f + miR.220a + miR.519b + miR.130a + miR.26a + miR.369.3p + miR.376a* + miR.518c + miR.639 + miR.660 + miR.338 | 0,81 | 0,129481869 |
| miR.609 + miR.603 + miR.520f + miR.220a + miR.519b + miR.130a + miR.26a + miR.29a + miR.362 + miR.369.3p + miR.376a* + miR.494 + miR.518c + miR.639 | 0,81 | 0,080918235 |
| miR.558 + miR.609 + miR.520f + miR.220a + miR.519b + miR.130a + miR.26a + miR.29a + miR.362 + miR.369.3p + miR.376a* + miR.494 + miR.660 + miR.338 | 0,81 | 0,306562122 |
| miR.609 + miR.603 + miR.519b + miR.130a + miR.26a + miR.29a + miR.362 + miR.369.3p + miR.376a* + miR.494 + miR.518c + miR.639 + miR.660 + miR.338 | 0,81 | 0,109526284 |
| miR.558 + miR.609 + miR.603 + miR.520f + miR.519b + miR.130a + miR.26a + miR.29a + miR.362 + miR.369.3p + miR.376a* + miR.494 + miR.660 + miR.338 | 0,81 | 0,33160657 |
| miR.558 + miR.609 + miR.603 + miR.220a + miR.519b + miR.26a + miR.362 + miR.369.3p + miR.376a* + miR.494 + miR.518c + miR.639 + miR.660 + miR.338 | 0,8 | 0,077144205 |
| miR.558 + miR.609 + miR.603 + miR.520f + miR.220a + miR.519b + miR.130a + miR.369.3p + miR.376a* + miR.494 + miR.518c + miR.639 + miR.660 + miR.338 | 0,8 | 0,12378441 |
| miR.558 + miR.609 + miR.603 + miR.520f + miR.220a + miR.519b + miR.26a + miR.29a + miR.362 + miR.369.3p + miR.376a* + miR.494 + miR.660 + miR.338 | 0,8 | 0,344327611 |
| miR.609 + miR.603 + miR.520f + miR.519b + miR.130a + miR.26a + miR.29a + miR.362 + miR.369.3p + miR.376a* + miR.494 + miR.518c + miR.660 + miR.338 | 0,8 | 0,354329436 |
| miR.558 + miR.609 + miR.603 + miR.520f + miR.220a + miR.130a + miR.26a + miR.369.3p + miR.376a* + miR.494 + miR.518c + miR.639 + miR.660 + miR.338 | 0,8 | 0,098351722 |
| miR.558 + miR.609 + miR.603 + miR.520f + miR.220a + miR.519b + miR.130a + miR.29a + miR.362 + miR.369.3p + miR.376a* + miR.494 + miR.660 + miR.338 | 0,8 | 0,263240613 |
| miR.558 + miR.609 + miR.603 + miR.220a + miR.519b + miR.130a + miR.26a + miR.362 + miR.369.3p + miR.376a* + miR.518c + miR.639 + miR.660 + miR.338 | 0,8 | 0,078061857 |
| miR.558 + miR.609 + miR.603 + miR.220a + miR.130a + miR.26a + miR.362 + miR.369.3p + miR.376a* + miR.494 + miR.518c + miR.639 + miR.660 + miR.338 | 0,8 | 0,071091157 |
| miR.558 + miR.609 + miR.603 + miR.520f + miR.220a + miR.519b + miR.130a + miR.29a + miR.362 + miR.369.3p + miR.376a* + miR.639 + miR.660 + miR.338 | 0,8 | 0,245793065 |
| miR.558 + miR.609 + miR.603 + miR.520f + miR.220a + miR.130a + miR.26a + miR.29a + miR.362 + miR.369.3p + miR.376a* + miR.494 + miR.660 + miR.338 | 0,8 | 0,35909622 |
| miR.609 + miR.520f + miR.220a + miR.519b + miR.130a + miR.26a + miR.29a + miR.362 + miR.369.3p + miR.376a* + miR.494 + miR.518c + miR.639 + miR.660 | 0,8 | 0,046802378 |
| miR.558 + miR.609 + miR.520f + miR.220a + miR.519b + miR.130a + miR.26a + miR.29a + miR.362 + miR.369.3p + miR.376a* + miR.639 + miR.660 + miR.338 | 0,8 | 0,297700793 |
| miR.558 + miR.609 + miR.603 + miR.520f + miR.519b + miR.130a + miR.26a + miR.29a + miR.362 + miR.369.3p + miR.376a* + miR.639 + miR.660 + miR.338 | 0,8 | 0,311833614 |
| miR.558 + miR.609 + miR.603 + miR.520f + miR.220a + miR.519b + miR.26a + miR.29a + miR.362 + miR.369.3p + miR.376a* + miR.639 + miR.660 + miR.338 | 0,8 | 0,276015157 |
| miR.609 + miR.603 + miR.520f + miR.220a + miR.519b + miR.130a + miR.26a + miR.362 + miR.369.3p + miR.376a* + miR.518c + miR.639 + miR.660 + miR.338 | 0,8 | 0,177294609 |
| miR.609 + miR.603 + miR.520f + miR.220a + miR.130a + miR.26a + miR.362 + miR.369.3p + miR.376a* + miR.494 + miR.518c + miR.639 + miR.660 + miR.338 | 0,8 | 0,188464045 |
| miR.609 + miR.603 + miR.520f + miR.220a + miR.519b + miR.130a + miR.29a + miR.362 + miR.369.3p + miR.376a* + miR.494 + miR.518c + miR.639 + miR.338 | 0,8 | 0,055149277 |
| miR.609 + miR.603 + miR.520f + miR.220a + miR.519b + miR.26a + miR.29a + miR.362 + miR.369.3p + miR.376a* + miR.494 + miR.518c + miR.639 + miR.338 | 0,8 | 0,058530001 |
| miR.558 + miR.609 + miR.603 + miR.520f + miR.519b + miR.130a + miR.26a + miR.369.3p + miR.376a* + miR.494 + miR.518c + miR.639 + miR.660 + miR.338 | 0,8 | 0,073419161 |
| miR.609 + miR.603 + miR.520f + miR.220a + miR.519b + miR.130a + miR.362 + miR.369.3p + miR.376a* + miR.494 + miR.518c + miR.639 + miR.660 + miR.338 | 0,8 | 0,160884494 |
| miR.558 + miR.609 + miR.603 + miR.520f + miR.220a + miR.130a + miR.26a + miR.29a + miR.362 + miR.369.3p + miR.376a* + miR.639 + miR.660 + miR.338 | 0,8 | 0,251950223 |
| miR.558 + miR.609 + miR.603 + miR.220a + miR.519b + miR.130a + miR.26a + miR.362 + miR.369.3p + miR.376a* + miR.494 + miR.518c + miR.639 + miR.660 | 0,8 | 0,037889128 |
| miR.609 + miR.603 + miR.520f + miR.220a + miR.519b + miR.130a + miR.26a + miR.29a + miR.362 + miR.369.3p + miR.376a* + miR.494 + miR.518c + miR.338 | 0,8 | 0,059988822 |
| miR.558 + miR.609 + miR.603 + miR.520f + miR.220a + miR.130a + miR.26a + miR.362 + miR.369.3p + miR.376a* + miR.494 + miR.518c + miR.660 + miR.338 | 0,8 | 0,249427092 |
| miR.609 + miR.603 + miR.520f + miR.220a + miR.519b + miR.130a + miR.29a + miR.362 + miR.376a* + miR.494 + miR.518c + miR.639 + miR.660 + miR.338 | 0,8 | 0,046130925 |
| miR.558 + miR.609 + miR.603 + miR.520f + miR.220a + miR.519b + miR.130a + miR.26a + miR.362 + miR.369.3p + miR.376a* + miR.518c + miR.660 + miR.338 | 0,8 | 0,30411399 |
| miR.609 + miR.603 + miR.520f + miR.220a + miR.519b + miR.130a + miR.26a + miR.29a + miR.369.3p + miR.376a* + miR.494 + miR.518c + miR.639 + miR.338 | 0,8 | 0,05301622 |
| miR.558 + miR.609 + miR.603 + miR.520f + miR.220a + miR.519b + miR.26a + miR.369.3p + miR.376a* + miR.494 + miR.518c + miR.639 + miR.660 + miR.338 | 0,8 | 0,076763417 |
| miR.609 + miR.603 + miR.520f + miR.519b + miR.130a + miR.26a + miR.29a + miR.369.3p + miR.376a* + miR.494 + miR.518c + miR.639 + miR.660 + miR.338 | 0,8 | 0,091403692 |
| miR.558 + miR.609 + miR.603 + miR.520f + miR.220a + miR.519b + miR.130a + miR.26a + miR.369.3p + miR.376a* + miR.494 + miR.518c + miR.639 + miR.660 | 0,8 | 0,036778697 |
| miR.609 + miR.603 + miR.520f + miR.220a + miR.130a + miR.26a + miR.29a + miR.362 + miR.369.3p + miR.376a* + miR.494 + miR.518c + miR.639 + miR.338 | 0,8 | 0,03378263 |
| miR.609 + miR.603 + miR.520f + miR.220a + miR.519b + miR.130a + miR.26a + miR.29a + miR.362 + miR.376a* + miR.494 + miR.518c + miR.639 + miR.660 | 0,8 | 0,05034036 |
| miR.609 + miR.603 + miR.520f + miR.519b + miR.130a + miR.26a + miR.362 + miR.369.3p + miR.376a* + miR.494 + miR.518c + miR.639 + miR.660 + miR.338 | 0,8 | 0,171090226 |
| miR.609 + miR.603 + miR.520f + miR.220a + miR.519b + miR.130a + miR.26a + miR.29a + miR.362 + miR.376a* + miR.494 + miR.518c + miR.660 + miR.338 | 0,8 | 0,087606136 |
| miR.609 + miR.220a + miR.519b + miR.130a + miR.26a + miR.29a + miR.362 + miR.369.3p + miR.376a* + miR.494 + miR.518c + miR.639 + miR.660 + miR.338 | 0,8 | 0,224158548 |
| miR.558 + miR.609 + miR.603 + miR.520f + miR.220a + miR.519b + miR.130a + miR.362 + miR.369.3p + miR.376a* + miR.494 + miR.518c + miR.660 + miR.338 | 0,8 | 0,253686996 |
| miR.609 + miR.603 + miR.520f + miR.519b + miR.130a + miR.26a + miR.29a + miR.362 + miR.369.3p + miR.376a* + miR.494 + miR.518c + miR.639 + miR.660 | 0,8 | 0,006637411 |
| miR.609 + miR.603 + miR.220a + miR.519b + miR.130a + miR.26a + miR.29a + miR.362 + miR.369.3p + miR.376a* + miR.494 + miR.518c + miR.639 + miR.338 | 0,8 | 0,086002133 |
| miR.609 + miR.603 + miR.520f + miR.220a + miR.519b + miR.26a + miR.29a + miR.362 + miR.376a* + miR.494 + miR.518c + miR.639 + miR.660 + miR.338 | 0,8 | 0,055090064 |
| miR.609 + miR.603 + miR.520f + miR.220a + miR.519b + miR.130a + miR.26a + miR.29a + miR.376a* + miR.494 + miR.518c + miR.639 + miR.660 + miR.338 | 0,8 | 0,052475325 |
| miR.558 + miR.609 + miR.603 + miR.520f + miR.220a + miR.519b + miR.26a + miR.362 + miR.369.3p + miR.376a* + miR.494 + miR.518c + miR.639 + miR.660 | 0,8 | 0,013558968 |
| miR.558 + miR.609 + miR.603 + miR.520f + miR.220a + miR.519b + miR.130a + miR.26a + miR.369.3p + miR.376a* + miR.494 + miR.518c + miR.660 + miR.338 | 0,8 | 0,104124958 |
| miR.558 + miR.609 + miR.603 + miR.519b + miR.130a + miR.26a + miR.362 + miR.369.3p + miR.376a* + miR.494 + miR.518c + miR.639 + miR.660 + miR.338 | 0,8 | 0,060776353 |
| miR.609 + miR.520f + miR.220a + miR.519b + miR.130a + miR.26a + miR.29a + miR.369.3p + miR.376a* + miR.494 + miR.518c + miR.639 + miR.660 + miR.338 | 0,8 | 0,109279266 |
| miR.609 + miR.603 + miR.520f + miR.220a + miR.130a + miR.26a + miR.29a + miR.362 + miR.376a* + miR.494 + miR.518c + miR.639 + miR.660 + miR.338 | 0,8 | 0,023839263 |
| miR.558 + miR.609 + miR.603 + miR.520f + miR.220a + miR.519b + miR.130a + miR.26a + miR.369.3p + miR.376a* + miR.494 + miR.518c + miR.639 + miR.338 | 0,8 | 0,023504599 |
| miR.609 + miR.603 + miR.520f + miR.220a + miR.519b + miR.26a + miR.29a + miR.362 + miR.369.3p + miR.376a* + miR.494 + miR.639 + miR.660 + miR.338 | 0,8 | 0,325057189 |
| miR.609 + miR.603 + miR.520f + miR.220a + miR.130a + miR.26a + miR.29a + miR.362 + miR.369.3p + miR.376a* + miR.494 + miR.639 + miR.660 + miR.338 | 0,8 | 0,334934386 |
| miR.609 + miR.603 + miR.520f + miR.519b + miR.130a + miR.26a + miR.29a + miR.362 + miR.369.3p + miR.376a* + miR.494 + miR.639 + miR.660 + miR.338 | 0,8 | 0,342581789 |
| miR.558 + miR.609 + miR.603 + miR.520f + miR.220a + miR.130a + miR.26a + miR.362 + miR.369.3p + miR.376a* + miR.494 + miR.518c + miR.639 + miR.338 | 0,8 | 0,007685147 |
| miR.609 + miR.603 + miR.520f + miR.220a + miR.519b + miR.130a + miR.26a + miR.29a + miR.362 + miR.369.3p + miR.376a* + miR.518c + miR.639 + miR.338 | 0,8 | 0,100091253 |
| miR.558 + miR.609 + miR.603 + miR.520f + miR.220a + miR.519b + miR.130a + miR.26a + miR.362 + miR.369.3p + miR.376a* + miR.518c + miR.639 + miR.338 | 0,8 | 0,020942311 |
| miR.558 + miR.609 + miR.603 + miR.520f + miR.220a + miR.519b + miR.130a + miR.362 + miR.369.3p + miR.376a* + miR.494 + miR.518c + miR.639 + miR.338 | 0,8 | 0,024987059 |
| miR.558 + miR.609 + miR.603 + miR.520f + miR.519b + miR.130a + miR.26a + miR.362 + miR.369.3p + miR.376a* + miR.494 + miR.518c + miR.660 + miR.338 | 0,8 | 0,173225559 |
| miR.558 + miR.609 + miR.603 + miR.220a + miR.130a + miR.26a + miR.29a + miR.362 + miR.369.3p + miR.376a* + miR.494 + miR.639 + miR.660 + miR.338 | 0,8 | 0,11496419 |
| miR.558 + miR.609 + miR.603 + miR.519b + miR.130a + miR.26a + miR.29a + miR.362 + miR.369.3p + miR.376a* + miR.494 + miR.639 + miR.660 + miR.338 | 0,8 | 0,110893359 |
| miR.558 + miR.609 + miR.603 + miR.520f + miR.519b + miR.130a + miR.26a + miR.362 + miR.369.3p + miR.376a* + miR.494 + miR.518c + miR.639 + miR.660 | 0,8 | 0,009546435 |
| miR.609 + miR.603 + miR.520f + miR.519b + miR.130a + miR.26a + miR.29a + miR.362 + miR.369.3p + miR.376a* + miR.494 + miR.518c + miR.639 + miR.338 | 0,8 | 0,049406253 |
| miR.558 + miR.609 + miR.603 + miR.520f + miR.220a + miR.130a + miR.26a + miR.362 + miR.376a* + miR.494 + miR.518c + miR.639 + miR.660 + miR.338 | 0,8 | 0,06378174 |
| miR.609 + miR.603 + miR.520f + miR.220a + miR.519b + miR.26a + miR.362 + miR.369.3p + miR.376a* + miR.494 + miR.518c + miR.639 + miR.660 + miR.338 | 0,8 | 0,107424295 |
| miR.609 + miR.520f + miR.220a + miR.519b + miR.130a + miR.26a + miR.29a + miR.362 + miR.369.3p + miR.376a* + miR.494 + miR.639 + miR.660 + miR.338 | 0,8 | 0,308850265 |
| miR.609 + miR.603 + miR.520f + miR.220a + miR.519b + miR.130a + miR.29a + miR.362 + miR.369.3p + miR.376a* + miR.494 + miR.639 + miR.660 + miR.338 | 0,8 | 0,300731562 |
| miR.558 + miR.609 + miR.220a + miR.519b + miR.130a + miR.26a + miR.29a + miR.362 + miR.369.3p + miR.376a^{*} + miR.494 + miR.639 + miR.660 + miR.338 | 0,8 | 0,136518793 |
| miR.558 + miR.609 + miR.603 + miR.520f + miR.220a + miR.519b + miR.130a + miR.26a + miR.362 + miR.369.3p + miR.376a^{*} + miR.494 + miR.518c + miR.660 | 0,8 | 0,05083898 |
| miR.558 + miR.609 + miR.603 + miR.520f + miR.220a + miR.519b + miR.130a + miR.26a + miR.29a + miR.362 + miR.369.3p + miR.376a^{*} + miR.660 + miR.338 | 0,8 | 0,309992112 |
| miR.558 + miR.609 + miR.603 + miR.220a + miR.519b + miR.130a + miR.362 + miR.369.3p + miR.376a* + miR.494 + miR.518c + miR.639 + miR.660 + miR.338 | 0,8 | 0,056993528 |
| miR.609 + miR.603 + miR.520f + miR.220a + miR.519b + miR.130a + miR.26a + miR.29a + miR.362 + miR.376a* + miR.494 + miR.518c + miR.639 + miR.338 | 0,8 | 0,040920039 |
| miR.609 + miR.603 + miR.220a + miR.519b + miR.130a + miR.26a + miR.29a + miR.362 + miR.376a* + miR.494 + miR.518c + miR.639 + miR.660 + miR.338 | 0,8 | 0,042466516 |
| miR.558 + miR.609 + miR.603 + miR.520f + miR.519b + miR.130a + miR.26a + miR.362 + miR.369.3p + miR.376a* + miR.494 + miR.518c + miR.639 + miR.338 | 0,8 | 0,011241388 |
| miR.558 + miR.609 + miR.603 + miR.520f + miR.220a + miR.519b + miR.130a + miR.26a + miR.362 + miR.376a* + miR.518c + miR.639 + miR.660 + miR.338 | 0,8 | 0,076611718 |
| miR.609 + miR.603 + miR.520f + miR.519b + miR.130a + miR.26a + miR.29a + miR.362 + miR.376a* + miR.494 + miR.518c + miR.639 + miR.660 + miR.338 | 0,8 | 0,01127434 |
| miR.558 + miR.609 + miR.603 + miR.220a + miR.519b + miR.130a + miR.26a + miR.369.3p + miR.376a* + miR.494 + miR.518c + miR.639 + miR.660 + miR.338 | 0,8 | 0,057831206 |
| miR.558 + miR.609 + miR.603 + miR.520f + miR.220a + miR.519b + miR.130a + miR.26a + miR.376a* + miR.494 + miR.518c + miR.639 + miR.660 + miR.338 | 0,8 | 0,088643936 |
| miR.558 + miR.609 + miR.603 + miR.520f + miR.519b + miR.130a + miR.26a + miR.362 + miR.376a* + miR.494 + miR.518c + miR.639 + miR.660 + miR.338 | 0,8 | 0,052153102 |
| miR.558 + miR.609 + miR.603 + miR.520f + miR.220a + miR.519b + miR.130a + miR.26a + miR.362 + miR.369.3p + miR.376a* + miR.494 + miR.518c + miR.338 | 0,8 | 0,026733007 |
| miR.558 + miR.609 + miR.603 + miR.520f + miR.220a + miR.519b + miR.130a + miR.26a + miR.362 + miR.376a* + miR.494 + miR.518c + miR.639 + miR.338 | 0,8 | 0,05819873 |
| miR.558 + miR.609 + miR.520f + miR.220a + miR.519b + miR.130a + miR.26a + miR.362 + miR.369.3p + miR.376a* + miR.494 + miR.518c + miR.639 + miR.660 | 0,8 | 0,010025775 |
| miR.558 + miR.609 + miR.603 + miR.220a + miR.519b + miR.130a + miR.26a + miR.362 + miR.369.3p + miR.376a* + miR.494 + miR.518c + miR.660 + miR.338 | 0,8 | 0,078466893 |
| miR.558 + miR.609 + miR.603 + miR.520f + miR.220a + miR.519b + miR.130a + miR.362 + miR.376a* + miR.494 + miR.518c + miR.639 + miR.660 + miR.338 | 0,8 | 0,084470249 |
| miR.558 + miR.609 + miR.520f + miR.220a + miR.519b + miR.130a + miR.26a + miR.362 + miR.369.3p + miR.376a* + miR.494 + miR.518c + miR.660 + miR.338 | 0,8 | 0,293490283 |
| miR.609 + miR.520f + miR.220a + miR.519b + miR.130a + miR.26a + miR.362 + miR.369.3p + miR.376a* + miR.494 + miR.518c + miR.639 + miR.660 + miR.338 | 0,8 | 0,13971314 |
| miR.558 + miR.609 + miR.603 + miR.220a + miR.519b + miR.130a + miR.29a + miR.362 + miR.369.3p + miR.376a* + miR.494 + miR.639 + miR.660 + miR.338 | 0,8 | 0,068026115 |
| miR.558 + miR.609 + miR.220a + miR.519b + miR.130a + miR.26a + miR.362 + miR.369.3p + miR.376a* + miR.494 + miR.518c + miR.639 + miR.660 + miR.338 | 0,8 | 0,061260888 |
| miR.558 + miR.609 + miR.603 + miR.520f + miR.220a + miR.519b + miR.26a + miR.362 + miR.376a* + miR.494 + miR.518c + miR.639 + miR.660 + miR.338 | 0,8 | 0,046879914 |
| miR.558 + miR.609 + miR.520f + miR.220a + miR.519b + miR.130a + miR.26a + miR.369.3p + miR.376a* + miR.494 + miR.518c + miR.639 + miR.660 + miR.338 | 0,8 | 0,108256788 |
| miR.558 + miR.609 + miR.603 + miR.520f + miR.519b + miR.130a + miR.26a + miR.29a + miR.369.3p + miR.376a* + miR.494 + miR.639 + miR.660 + miR.338 | 0,8 | 0,065432315 |
| miR.558 + miR.609 + miR.603 + miR.520f + miR.519b + miR.130a + miR.26a + miR.29a + miR.362 + miR.369.3p + miR.376a* + miR.494 + miR.639 + miR.660 | 0,8 | 0,01172865 |
| miR.558 + miR.609 + miR.603 + miR.520f + miR.220a + miR.519b + miR.130a + miR.26a + miR.362 + miR.369.3p + miR.376a* + miR.494 + miR.518c + miR.639 | 0,8 | 0,030836129 |
| miR.609 + miR.603 + miR.520f + miR.220a + miR.519b + miR.130a + miR.26a + miR.29a + miR.362 + miR.376a* + miR.518c + miR.639 + miR.660 + miR.338 | 0,8 | 0,095192448 |
| miR.558 + miR.609 + miR.603 + miR.520f + miR.220a + miR.130a + miR.26a + miR.29a + miR.362 + miR.369.3p + miR.376a* + miR.494 + miR.639 + miR.660 | 0,8 | 0,013700819 |
| miR.609 + miR.603 + miR.220a + miR.519b + miR.130a + miR.26a + miR.362 + miR.369.3p + miR.376a* + miR.494 + miR.518c + miR.639 + miR.660 + miR.338 | 0,8 | 0,053720299 |
| miR.558 + miR.609 + miR.603 + miR.520f + miR.220a + miR.519b + miR.26a + miR.362 + miR.369.3p + miR.376a* + miR.494 + miR.518c + miR.639 + miR.338 | 0,8 | 0,015063919 |
| miR.558 + miR.609 + miR.603 + miR.520f + miR.220a + miR.519b + miR.130a + miR.29a + miR.369.3p + miR.376a* + miR.494 + miR.639 + miR.660 + miR.338 | 0,8 | 0,043809313 |
| miR.558 + miR.609 + miR.603 + miR.520f + miR.220a + miR.130a + miR.26a + miR.362 + miR.369.3p + miR.376a* + miR.494 + miR.639 + miR.660 + miR.338 | 0,8 | 0,177177824 |
| miR.558 + miR.609 + miR.603 + miR.520f + miR.519b + miR.130a + miR.26a + miR.362 + miR.369.3p + miR.376a* + miR.494 + miR.639 + miR.660 + miR.338 | 0,8 | 0,190168183 |
| miR.558 + miR.609 + miR.603 + miR.520f + miR.220a + miR.519b + miR.26a + miR.29a + miR.369.3p + miR.376a* + miR.494 + miR.639 + miR.660 + miR.338 | 0,8 | 0,090096711 |
| miR.558 + miR.609 + miR.520f + miR.220a + miR.519b + miR.130a + miR.26a + miR.29a + miR.362 + miR.376a* + miR.494 + miR.639 + miR.660 + miR.338 | 0,8 | 0,058666008 |
| miR.558 + miR.609 + miR.603 + miR.520f + miR.220a + miR.519b + miR.130a + miR.29a + miR.362 + miR.369.3p + miR.376a* + miR.494 + miR.639 + miR.660 | 0,8 | 0,013176111 |
| miR.558 + miR.609 + miR.520f + miR.220a + miR.519b + miR.130a + miR.26a + miR.29a + miR.362 + miR.369.3p + miR.376a* + miR.494 + miR.639 + miR.660 | 0,8 | 0,017767517 |
| miR.609 + miR.603 + miR.520f + miR.220a + miR.519b + miR.130a + miR.26a + miR.29a + miR.362 + miR.369.3p + miR.376a* + miR.494 + miR.660 + miR.338 | 0,8 | 0,361479845 |
| miR.609 + miR.603 + miR.520f + miR.220a + miR.519b + miR.130a + miR.26a + miR.369.3p + miR.376a* + miR.494 + miR.518c + miR.639 + miR.660 + miR.338 | 0,8 | 0,059371244 |
| miR.558 + miR.609 + miR.603 + miR.520f + miR.220a + miR.130a + miR.26a + miR.29a + miR.362 + miR.376a* + miR.494 + miR.639 + miR.660 + miR.338 | 0,8 | 0,017379743 |
| miR.558 + miR.609 + miR.603 + miR.520f + miR.220a + miR.519b + miR.130a + miR.29a + miR.362 + miR.376a* + miR.494 + miR.639 + miR.660 + miR.338 | 0,8 | 0,01317354 |
| miR.558 + miR.609 + miR.603 + miR.220a + miR.519b + miR.130a + miR.26a + miR.362 + miR.369.3p + miR.376a* + miR.494 + miR.518c + miR.639 + miR.338 | 0,8 | 0,015154989 |
| miR.609 + miR.520f + miR.220a + miR.519b + miR.130a + miR.26a + miR.29a + miR.362 + miR.376a* + miR.494 + miR.518c + miR.639 + miR.660 + miR.338 | 0,8 | 0,039426385 |
| miR.609 + miR.603 + miR.520f + miR.220a + miR.519b + miR.130a + miR.26a + miR.362 + miR.369.3p + miR.376a* + miR.494 + miR.518c + miR.639 + miR.660 | 0,8 | 0,017648939 |
| miR.558 + miR.609 + miR.520f + miR.220a + miR.519b + miR.130a + miR.26a + miR.29a + miR.369.3p + miR.376a* + miR.494 + miR.639 + miR.660 + miR.338 | 0,8 | 0,102227385 |
| miR.558 + miR.609 + miR.603 + miR.520f + miR.220a + miR.519b + miR.26a + miR.362 + miR.369.3p + miR.376a* + miR.494 + miR.639 + miR.660 + miR.338 | 0,8 | 0,176365286 |
| miR.558 + miR.609 + miR.603 + miR.520f + miR.220a + miR.519b + miR.130a + miR.26a + miR.29a + miR.369.3p + miR.376a* + miR.494 + miR.660 + miR.338 | 0,8 | 0,110508988 |
| miR.558 + miR.609 + miR.603 + miR.520f + miR.220a + miR.519b + miR.130a + miR.26a + miR.362 + miR.369.3p + miR.376a* + miR.639 + miR.660 + miR.338 | 0,8 | 0,261071933 |
| miR.558 + miR.609 + miR.603 + miR.520f + miR.220a + miR.519b + miR.130a + miR.26a + miR.362 + miR.376a* + miR.494 + miR.518c + miR.660 + miR.338 | 0,8 | 0,078135033 |
| miR.558 + miR.609 + miR.603 + miR.520f + miR.220a + miR.130a + miR.26a + miR.29a + miR.369.3p + miR.376a* + miR.494 + miR.639 + miR.660 + miR.338 | 0,8 | 0,028352292 |
| miR.558 + miR.609 + miR.603 + miR.220a + miR.519b + miR.130a + miR.26a + miR.29a + miR.362 + miR.369.3p + miR.376a* + miR.494 + miR.660 + miR.338 | 0,8 | 0,09826272 |
| miR.558 + miR.609 + miR.603 + miR.520f + miR.220a + miR.519b + miR.26a + miR.29a + miR.362 + miR.369.3p + miR.376a* + miR.494 + miR.639 + miR.660 | 0,8 | 0,027125524 |
| miR.558 + miR.609 + miR.603 + miR.220a + miR.519b + miR.26a + miR.29a + miR.362 + miR.369.3p + miR.376a* + miR.494 + miR.639 + miR.660 + miR.338 | 0,8 | 0,107146535 |
| miR.558 + miR.609 + miR.603 + miR.220a + miR.130a + miR.26a + miR.29a + miR.362 + miR.369.3p + miR.494 + miR.518c + miR.639 + miR.660 + miR.338 | 0,8 | 0,01698892 |
| miR.558 + miR.609 + miR.603 + miR.220a + miR.519b + miR.130a + miR.26a + miR.29a + miR.362 + miR.369.3p + miR.494 + miR.518c + miR.639 + miR.660 | 0,8 | 0,010566286 |
| miR.558 + miR.609 + miR.603 + miR.520f + miR.220a + miR.519b + miR.130a + miR.26a + miR.29a + miR.369.3p + miR.376a* + miR.494 + miR.639 + miR.338 | 0,8 | 0,030742831 |
| miR.558 + miR.609 + miR.520f + miR.220a + miR.519b + miR.130a + miR.26a + miR.362 + miR.369.3p + miR.376a* + miR.494 + miR.639 + miR.660 + miR.338 | 0,8 | 0,214792956 |
| miR.558 + miR.609 + miR.603 + miR.520f + miR.220a + miR.519b + miR.26a + miR.362 + miR.369.3p + miR.376a* + miR.494 + miR.518c + miR.660 + miR.338 | 0,8 | 0,061100427 |
| miR.558 + miR.609 + miR.520f + miR.220a + miR.519b + miR.130a + miR.26a + miR.362 + miR.376a* + miR.494 + miR.518c + miR.639 + miR.660 + miR.338 | 0,8 | 0,091496082 |
| miR.558 + miR.609 + miR.603 + miR.520f + miR.519b + miR.130a + miR.26a + miR.29a + miR.362 + miR.369.3p + miR.376a* + miR.494 + miR.639 + miR.338 | 0,8 | 0,026191542 |
| miR.558 + miR.609 + miR.603 + miR.520f + miR.220a + miR.519b + miR.130a + miR.29a + miR.362 + miR.369.3p + miR.494 + miR.518c + miR.639 + miR.660 | 0,8 | 0,006908093 |
| miR.558 + miR.609 + miR.603 + miR.520f + miR.220a + miR.130a + miR.26a + miR.29a + miR.362 + miR.369.3p + miR.518c + miR.639 + miR.660 + miR.338 | 0,8 | 0,015580289 |
| miR.558 + miR.609 + miR.603 + miR.520f + miR.220a + miR.519b + miR.130a + miR.26a + miR.362 + miR.376a* + miR.494 + miR.518c + miR.639 + miR.660 | 0,8 | 0,055096981 |
| miR.558 + miR.609 + miR.603 + miR.520f + miR.220a + miR.130a + miR.26a + miR.29a + miR.362 + miR.369.3p + miR.494 + miR.518c + miR.639 + miR.660 | 0,8 | 0,009438241 |
| miR.558 + miR.609 + miR.603 + miR.520f + miR.220a + miR.519b + miR.130a + miR.26a + miR.29a + miR.362 + miR.369.3p + miR.376a* + miR.494 + miR.660 | 0,8 | 0,033681962 |
| miR.558 + miR.609 + miR.603 + miR.520f + miR.220a + miR.519b + miR.130a + miR.29a + miR.362 + miR.369.3p + miR.376a* + miR.494 + miR.639 + miR.338 | 0,8 | 0,026398977 |
| miR.609 + miR.603 + miR.520f + miR.220a + miR.519b + miR.130a + miR.26a + miR.362 + miR.369.3p + miR.376a* + miR.494 + miR.518c + miR.660 + miR.338 | 0,8 | 0,262528653 |
| miR.558 + miR.609 + miR.603 + miR.220a + miR.519b + miR.130a + miR.26a + miR.29a + miR.362 + miR.369.3p + miR.494 + miR.518c + miR.660 + miR.338 | 0,8 | 0,030567393 |
| miR.558 + miR.609 + miR.603 + miR.220a + miR.519b + miR.130a + miR.26a + miR.29a + miR.362 + miR.369.3p + miR.518c + miR.639 + miR.660 + miR.338 | 0,8 | 0,031207729 |
| miR.558 + miR.609 + miR.603 + miR.520f + miR.220a + miR.519b + miR.26a + miR.29a + miR.362 + miR.376a* + miR.494 + miR.639 + miR.660 + miR.338 | 0,8 | 0,055830281 |
| miR.558 + miR.609 + miR.603 + miR.520f + miR.519b + miR.130a + miR.26a + miR.29a + miR.362 + miR.376a* + miR.494 + miR.639 + miR.660 + miR.338 | 0,8 | 0,051520906 |
| miR.558 + miR.609 + miR.603 + miR.520f + miR.220a + miR.519b + miR.26a + miR.29a + miR.362 + miR.369.3p + miR.376a* + miR.494 + miR.639 + miR.338 | 0,8 | 0,022972698 |
| miR.558 + miR.609 + miR.603 + miR.520f + miR.220a + miR.519b + miR.130a + miR.26a + miR.29a + miR.362 + miR.369.3p + miR.518c + miR.639 + miR.660 | 0,8 | 0,009348737 |
| miR.558 + miR.609 + miR.603 + miR.520f + miR.220a + miR.519b + miR.130a + miR.26a + miR.29a + miR.362 + miR.376a* + miR.494 + miR.660 + miR.338 | 0,8 | 0,113170361 |
| miR.558 + miR.609 + miR.603 + miR.220a + miR.519b + miR.130a + miR.26a + miR.29a + miR.369.3p + miR.494 + miR.518c + miR.639 + miR.660 + miR.338 | 0,8 | 0,028719048 |
| miR.558 + miR.609 + miR.603 + miR.520f + miR.220a + miR.26a + miR.29a + miR.362 + miR.369.3p + miR.494 + miR.518c + miR.639 + miR.660 + miR.338 | 0,8 | 0,012465825 |
| miR.609 + miR.603 + miR.520f + miR.220a + miR.519b + miR.130a + miR.26a + miR.29a + miR.362 + miR.369.3p + miR.376a* + miR.639 + miR.660 + miR.338 | 0,8 | 0,372109999 |
| miR.558 + miR.609 + miR.603 + miR.520f + miR.220a + miR.519b + miR.130a + miR.29a + miR.362 + miR.494 + miR.518c + miR.639 + miR.660 + miR.338 | 0,8 | 0,014803783 |
| miR.558 + miR.609 + miR.603 + miR.520f + miR.220a + miR.519b + miR.130a + miR.29a + miR.362 + miR.369.3p + miR.494 + miR.518c + miR.660 + miR.338 | 0,8 | 0,019467446 |
| miR.558 + miR.609 + miR.603 + miR.520f + miR.220a + miR.519b + miR.130a + miR.26a + miR.29a + miR.369.3p + miR.494 + miR.518c + miR.660 + miR.338 | 0,8 | 0,023002139 |
| miR.558 + miR.609 + miR.603 + miR.520f + miR.220a + miR.519b + miR.26a + miR.29a + miR.362 + miR.369.3p + miR.518c + miR.639 + miR.660 + miR.338 | 0,8 | 0,019047463 |
| miR.558 + miR.609 + miR.603 + miR.520f + miR.220a + miR.519b + miR.130a + miR.362 + miR.369.3p + miR.376a* + miR.494 + miR.639 + miR.660 + miR.338 | 0,8 | 0,178460774 |
| miR.558 + miR.609 + miR.603 + miR.220a + miR.519b + miR.26a + miR.29a + miR.362 + miR.369.3p + miR.494 + miR.518c + miR.639 + miR.660 + miR.338 | 0,8 | 0,028736225 |
| miR.558 + miR.609 + miR.603 + miR.520f + miR.220a + miR.519b + miR.130a + miR.26a + miR.29a + miR.362 + miR.369.3p + miR.376a* + miR.494 + miR.338 | 0,8 | 0,035327934 |
| miR.558 + miR.609 + miR.603 + miR.520f + miR.220a + miR.519b + miR.130a + miR.29a + miR.369.3p + miR.494 + miR.518c + miR.639 + miR.660 + miR.338 | 0,8 | 0,014218463 |
| miR.558 + miR.609 + miR.603 + miR.520f + miR.220a + miR.519b + miR.130a + miR.26a + miR.29a + miR.362 + miR.369.3p + miR.494 + miR.518c + miR.660 | 0,8 | 0,022209399 |
| miR.558 + miR.609 + miR.603 + miR.520f + miR.220a + miR.519b + miR.26a + miR.29a + miR.362 + miR.369.3p + miR.494 + miR.518c + miR.639 + miR.660 | 0,8 | 0,010788415 |
| miR.558 + miR.609 + miR.603 + miR.220a + miR.519b + miR.130a + miR.29a + miR.362 + miR.369.3p + miR.494 + miR.518c + miR.639 + miR.660 + miR.338 | 0,8 | 0,018060186 |
| miR.558 + miR.609 + miR.603 + miR.520f + miR.220a + miR.519b + miR.130a + miR.26a + miR.29a + miR.494 + miR.518c + miR.639 + miR.660 + miR.338 | 0,8 | 0,019141951 |
| miR.558 + miR.609 + miR.603 + miR.520f + miR.220a + miR.519b + miR.130a + miR.26a + miR.29a + miR.362 + miR.494 + miR.518c + miR.660 + miR.338 | 0,8 | 0,024049426 |
| miR.558 + miR.609 + miR.603 + miR.520f + miR.220a + miR.519b + miR.130a + miR.26a + miR.29a + miR.362 + miR.494 + miR.518c + miR.639 + miR.660 | 0,8 | 0,01129977 |
| miR.558 + miR.609 + miR.603 + miR.520f + miR.220a + miR.130a + miR.29a + miR.362 + miR.369.3p + miR.494 + miR.518c + miR.639 + miR.660 + miR.338 | 0,8 | 0,004248511 |
| miR.558 + miR.609 + miR.603 + miR.520f + miR.220a + miR.519b + miR.130a + miR.26a + miR.29a + miR.376a* + miR.494 + miR.639 + miR.660 + miR.338 | 0,8 | 0,068004904 |
| miR.558 + miR.609 + miR.603 + miR.520f + miR.220a + miR.519b + miR.130a + miR.29a + miR.362 + miR.369.3p + miR.518c + miR.639 + miR.660 + miR.338 | 0,8 | 0,007728063 |
| miR.558 + miR.609 + miR.603 + miR.520f + miR.220a + miR.519b + miR.130a + miR.26a + miR.29a + miR.362 + miR.369.3p + miR.518c + miR.660 + miR.338 | 0,8 | 0,038155902 |
| miR.558 + miR.609 + miR.603 + miR.520f + miR.130a + miR.26a + miR.29a + miR.362 + miR.369.3p + miR.494 + miR.518c + miR.639 + miR.660 + miR.338 | 0,8 | 0,005150207 |
| miR.609 + miR.603 + miR.520f + miR.220a + miR.519b + miR.130a + miR.26a + miR.362 + miR.369.3p + miR.376a* + miR.494 + miR.518c + miR.639 + miR.338 | 0,8 | 0,01856064 |
| miR.558 + miR.609 + miR.603 + miR.520f + miR.220a + miR.519b + miR.130a + miR.26a + miR.29a + miR.369.3p + miR.494 + miR.518c + miR.639 + miR.660 | 0,8 | 0,01055317 |
| miR.558 + miR.609 + miR.603 + miR.520f + miR.220a + miR.519b + miR.26a + miR.29a + miR.362 + miR.369.3p + miR.494 + miR.518c + miR.660 + miR.338 | 0,8 | 0,039104023 |
| miR.558 + miR.609 + miR.603 + miR.520f + miR.519b + miR.130a + miR.26a + miR.29a + miR.362 + miR.369.3p + miR.494 + miR.518c + miR.639 + miR.660 | 0,8 | 0,005817217 |
| miR.558 + miR.609 + miR.603 + miR.520f + miR.220a + miR.519b + miR.130a + miR.26a + miR.29a + miR.369.3p + miR.518c + miR.639 + miR.660 + miR.338 | 0,8 | 0,021453956 |
| miR.558 + miR.609 + miR.520f + miR.220a + miR.519b + miR.130a + miR.26a + miR.362 + miR.369.3p + miR.376a* + miR.494 + miR.518c + miR.639 + miR.338 | 0,8 | 0,018697239 |
| miR.558 + miR.609 + miR.520f + miR.220a + miR.519b + miR.130a + miR.26a + miR.29a + miR.362 + miR.369.3p + miR.494 + miR.518c + miR.639 + miR.660 | 0,8 | 0,00494647 |
| miR.558 + miR.609 + miR.603 + miR.220a + miR.519b + miR.130a + miR.26a + miR.29a + miR.362 + miR.494 + miR.518c + miR.639 + miR.660 + miR.338 | 0,8 | 0,026107071 |
| miR.558 + miR.609 + miR.603 + miR.520f + miR.519b + miR.130a + miR.29a + miR.362 + miR.369.3p + miR.494 + miR.518c + miR.639 + miR.660 + miR.338 | 0,8 | 0,002805467 |
| miR.609 + miR.520f + miR.220a + miR.519b + miR.130a + miR.26a + miR.29a + miR.362 + miR.369.3p + miR.376a* + miR.494 + miR.518c + miR.639 + miR.338 | 0,8 | 0,03136434 |
| miR.558 + miR.609 + miR.603 + miR.520f + miR.220a + miR.130a + miR.26a + miR.29a + miR.362 + miR.369.3p + miR.494 + miR.518c + miR.660 + miR.338 | 0,8 | 0,028805568 |
| miR.558 + miR.609 + miR.603 + miR.220a + miR.519b + miR.130a + miR.26a + miR.29a + miR.362 + miR.369.3p + miR.376a* + miR.494 + miR.639 + miR.660 | 0,8 | 0,012489899 |
| miR.558 + miR.609 + miR.520f + miR.220a + miR.519b + miR.130a + miR.29a + miR.362 + miR.369.3p + miR.494 + miR.518c + miR.639 + miR.660 + miR.338 | 0,8 | 0,003124611 |
| miR.558 + miR.609 + miR.520f + miR.220a + miR.519b + miR.130a + miR.26a + miR.29a + miR.362 + miR.369.3p + miR.376a* + miR.494 + miR.639 + miR.338 | 0,8 | 0,025730939 |
| miR.558 + miR.609 + miR.603 + miR.520f + miR.220a + miR.130a + miR.26a + miR.29a + miR.369.3p + miR.494 + miR.518c + miR.639 + miR.660 + miR.338 | 0,8 | 0,009684082 |
| miR.558 + miR.609 + miR.603 + miR.520f + miR.220a + miR.130a + miR.26a + miR.29a + miR.362 + miR.369.3p + miR.376a* + miR.494 + miR.639 + miR.338 | 0,8 | 0,007678091 |
| miR.558 + miR.609 + miR.603 + miR.519b + miR.130a + miR.26a + miR.29a + miR.362 + miR.369.3p + miR.494 + miR.518c + miR.639 + miR.660 + miR.338 | 0,8 | 0,006249143 |
| miR.558 + miR.609 + miR.520f + miR.220a + miR.130a + miR.26a + miR.29a + miR.362 + miR.369.3p + miR.494 + miR.518c + miR.639 + miR.660 + miR.338 | 0,8 | 0,008091387 |
| miR.558 + miR.609 + miR.603 + miR.520f + miR.220a + miR.519b + miR.26a + miR.29a + miR.369.3p + miR.494 + miR.518c + miR.639 + miR.660 + miR.338 | 0,8 | 0,019583277 |
| miR.558 + miR.609 + miR.603 + miR.520f + miR.220a + miR.130a + miR.26a + miR.29a + miR.362 + miR.494 + miR.518c + miR.639 + miR.660 + miR.338 | 0,8 | 0,008544846 |
| miR.558 + miR.609 + miR.603 + miR.520f + miR.220a + miR.519b + miR.130a + miR.26a + miR.29a + miR.369.3p + miR.376a* + miR.639 + miR.660 + miR.338 | 0,8 | 0,050638514 |
| miR.558 + miR.609 + miR.603 + miR.520f + miR.220a + miR.519b + miR.26a + miR.29a + miR.362 + miR.494 + miR.518c + miR.639 + miR.660 + miR.338 | 0,8 | 0,019945097 |
| miR.558 + miR.609 + miR.603 + miR.220a + miR.519b + miR.130a + miR.26a + miR.362 + miR.376a* + miR.494 + miR.518c + miR.639 + miR.660 + miR.338 | 0,8 | 0,053220027 |
| miR.558 + miR.609 + miR.603 + miR.520f + miR.220a + miR.519b + miR.29a + miR.362 + miR.369.3p + miR.494 + miR.518c + miR.639 + miR.660 + miR.338 | 0,8 | 0,011098878 |
| miR.558 + miR.609 + miR.603 + miR.520f + miR.220a + miR.519b + miR.130a + miR.26a + miR.29a + miR.362 + miR.518c + miR.639 + miR.660 + miR.338 | 0,8 | 0,014815491 |
| miR.558 + miR.609 + miR.603 + miR.520f + miR.220a + miR.519b + miR.130a + miR.26a + miR.29a + miR.362 + miR.376a* + miR.494 + miR.639 + miR.338 | 0,8 | 0,0229879 |
| miR.558 + miR.609 + miR.603 + miR.520f + miR.519b + miR.130a + miR.26a + miR.29a + miR.362 + miR.369.3p + miR.518c + miR.639 + miR.660 + miR.338 | 0,8 | 0,005321563 |
| miR.558 + miR.609 + miR.520f + miR.220a + miR.519b + miR.130a + miR.26a + miR.29a + miR.362 + miR.369.3p + miR.518c + miR.639 + miR.660 + miR.338 | 0,8 | 0,006604092 |
| miR.558 + miR.609 + miR.220a + miR.519b + miR.130a + miR.26a + miR.29a + miR.362 + miR.369.3p + miR.494 + miR.518c + miR.639 + miR.660 + miR.338 | 0,8 | 0,010124967 |
| miR.558 + miR.609 + miR.520f + miR.519b + miR.130a + miR.26a + miR.29a + miR.362 + miR.369.3p + miR.494 + miR.518c + miR.639 + miR.660 + miR.338 | 0,8 | 0,003326403 |
| miR.558 + miR.609 + miR.520f + miR.220a + miR.519b + miR.26a + miR.29a + miR.362 + miR.369.3p + miR.494 + miR.518c + miR.639 + miR.660 + miR.338 | 0,8 | 0,008559942 |
| miR.558 + miR.609 + miR.520f + miR.220a + miR.519b + miR.130a + miR.26a + miR.29a + miR.362 + miR.494 + miR.518c + miR.639 + miR.660 + miR.338 | 0,8 | 0,003364812 |
| miR.558 + miR.609 + miR.603 + miR.520f + miR.519b + miR.130a + miR.26a + miR.29a + miR.362 + miR.494 + miR.518c + miR.639 + miR.660 + miR.338 | 0,8 | 0,00434136 |
| miR.609 + miR.603 + miR.520f + miR.220a + miR.519b + miR.130a + miR.26a + miR.362 + miR.376a* + miR.494 + miR.518c + miR.639 + miR.660 + miR.338 | 0,8 | 0,052467223 |
| miR.558 + miR.609 + miR.603 + miR.520f + miR.220a + miR.519b + miR.130a + miR.26a + miR.29a + miR.362 + miR.369.3p + miR.376a* + miR.639 + miR.660 | 0,8 | 0,020388689 |
| miR.558 + miR.609 + miR.520f + miR.220a + miR.519b + miR.130a + miR.26a + miR.29a + miR.362 + miR.369.3p + miR.494 + miR.518c + miR.660 + miR.338 | 0,8 | 0,01053064 |
| miR.558 + miR.609 + miR.603 + miR.520f + miR.519b + miR.26a + miR.29a + miR.362 + miR.369.3p + miR.494 + miR.518c + miR.639 + miR.660 + miR.338 | 0,8 | 0,005301543 |
| miR.558 + miR.609 + miR.603 + miR.520f + miR.519b + miR.130a + miR.26a + miR.29a + miR.369.3p + miR.494 + miR.518c + miR.639 + miR.660 + miR.338 | 0,8 | 0,007014815 |
| miR.558 + miR.609 + miR.520f + miR.220a + miR.519b + miR.130a + miR.26a + miR.29a + miR.369.3p + miR.494 + miR.518c + miR.639 + miR.660 + miR.338 | 0,8 | 0,009009583 |
| miR.558 + miR.609 + miR.603 + miR.520f + miR.220a + miR.519b + miR.130a + miR.26a + miR.29a + miR.362 + miR.369.3p + miR.376a* + miR.639 + miR.338 | 0,8 | 0,022578146 |
| miR.558 + miR.609 + miR.603 + miR.520f + miR.220a + miR.519b + miR.130a + miR.26a + miR.29a + miR.362 + miR.376a* + miR.494 + miR.639 + miR.660 | 0,8 | 0,003876995 |
| miR.609 + miR.603 + miR.520f + miR.220a + miR.519b + miR.130a + miR.26a + miR.362 + miR.369.3p + miR.376a* + miR.494 + miR.639 + miR.660 + miR.338 | 0,8 | 0,176166811 |
| miR.558 + miR.609 + miR.603 + miR.520f + miR.519b + miR.130a + miR.26a + miR.29a + miR.362 + miR.369.3p + miR.494 + miR.518c + miR.660 + miR.338 | 0,8 | 0,011991996 |
| miR.558 + miR.609 + miR.603 + miR.520f + miR.220a + miR.519b + miR.130a + miR.26a + miR.362 + miR.369.3p + miR.376a* + miR.494 + miR.660 + miR.338 | 0,8 | 0,233348404 |
| miR.558 + miR.609 + miR.603 + miR.520f + miR.220a + miR.519b + miR.130a + miR.26a + miR.29a + miR.369.3p + miR.376a* + miR.494 + miR.639 + miR.660 | 0,8 | 0,009690995 |
| miR.558 + miR.609 + miR.603 + miR.220a + miR.519b + miR.130a + miR.26a + miR.29a + miR.362 + miR.369.3p + miR.376a* + miR.639 + miR.660 + miR.338 | 0,8 | 0,048559007 |
| miR.558 + miR.609 + miR.603 + miR.520f + miR.220a + miR.519b + miR.130a + miR.26a + miR.29a + miR.362 + miR.369.3p + miR.494 + miR.518c + miR.338 | 0,79 | 0,032484662 |
| miR.558 + miR.609 + miR.603 + miR.220a + miR.519b + miR.130a + miR.26a + miR.29a + miR.362 + miR.369.3p + miR.494 + miR.518c + miR.639 + miR.338 | 0,79 | 0,033583739 |
| miR.558 + miR.609 + miR.603 + miR.520f + miR.220a + miR.519b + miR.130a + miR.26a + miR.29a + miR.362 + miR.494 + miR.518c + miR.639 + miR.338 | 0,79 | 0,031807754 |
| miR.558 + miR.609 + miR.603 + miR.520f + miR.220a + miR.519b + miR.130a + miR.26a + miR.29a + miR.362 + miR.369.3p + miR.376a* + miR.494 + miR.639 | 0,79 | 0,006085582 |
| miR.558 + miR.609 + miR.603 + miR.520f + miR.220a + miR.519b + miR.130a + miR.26a + miR.29a + miR.362 + miR.376a* + miR.639 + miR.660 + miR.338 | 0,79 | 0,029731462 |
| miR.558 + miR.609 + miR.603 + miR.220a + miR.519b + miR.130a + miR.26a + miR.29a + miR.362 + miR.369.3p + miR.376a* + miR.494 + miR.639 + miR.338 | 0,79 | 0,012041757 |
| miR.609 + miR.603 + miR.520f + miR.220a + miR.519b + miR.130a + miR.26a + miR.29a + miR.362 + miR.369.3p + miR.376a* + miR.494 + miR.639 + miR.660 | 0,79 | 0,021500963 |
| miR.558 + miR.609 + miR.603 + miR.520f + miR.220a + miR.519b + miR.130a + miR.26a + miR.29a + miR.362 + miR.369.3p + miR.518c + miR.639 + miR.338 | 0,79 | 0,027971802 |
| miR.558 + miR.609 + miR.603 + miR.520f + miR.220a + miR.519b + miR.130a + miR.26a + miR.369.3p + miR.376a^{*} + miR.494 + miR.639 + miR.660 + miR.338 | 0,79 | 0,053000691 |
| miR.558 + miR.609 + miR.603 + miR.520f + miR.220a + miR.519b + miR.130a + miR.29a + miR.362 + miR.369.3p + miR.494 + miR.518c + miR.639 + miR.338 | 0,79 | 0,047142301 |
| miR.609 + miR.603 + miR.220a + miR.519b + miR.130a + miR.26a + miR.29a + miR.362 + miR.369.3p + miR.376a^{*} + miR.494 + miR.639 + miR.660 + miR.338 | 0,79 | 0,108525289 |
| miR.609 + miR.603 + miR.520f + miR.220a + miR.519b + miR.130a + miR.26a + miR.29a + miR.369.3p + miR.376a* + miR.494 + miR.639 + miR.660 + miR.338 | 0,79 | 0,060410062 |
| miR.609 + miR.603 + miR.520f + miR.220a + miR.519b + miR.130a + miR.26a + miR.29a + miR.362 + miR.376a* + miR.494 + miR.639 + miR.660 + miR.338 | 0,79 | 0,024830622 |
| miR.558 + miR.609 + miR.603 + miR.220a + miR.519b + miR.130a + miR.26a + miR.29a + miR.369.3p + miR.376a* + miR.494 + miR.639 + miR.660 + miR.338 | 0,79 | 0,003332699 |
| miR.558 + miR.609 + miR.603 + miR.520f + miR.220a + miR.519b + miR.26a + miR.29a + miR.362 + miR.369.3p + miR.494 + miR.518c + miR.639 + miR.338 | 0,79 | 0,022325889 |
| miR.558 + miR.609 + miR.603 + miR.520f + miR.220a + miR.519b + miR.130a + miR.26a + miR.29a + miR.369.3p + miR.494 + miR.518c + miR.639 + miR.338 | 0,79 | 0,032533787 |
| miR.558 + miR.609 + miR.603 + miR.520f + miR.220a + miR.519b + miR.130a + miR.26a + miR.362 + miR.376a* + miR.494 + miR.639 + miR.660 + miR.338 | 0,79 | 0,056348072 |
| miR.558 + miR.609 + miR.603 + miR.520f + miR.220a + miR.130a + miR.26a + miR.29a + miR.362 + miR.369.3p + miR.494 + miR.518c + miR.639 + miR.338 | 0,79 | 0,012745843 |
| miR.558 + miR.609 + miR.520f + miR.220a + miR.519b + miR.130a + miR.26a + miR.29a + miR.362 + miR.369.3p + miR.494 + miR.518c + miR.639 + miR.338 | 0,79 | 0,021305139 |
| miR.558 + miR.609 + miR.603 + miR.520f + miR.220a + miR.519b + miR.130a + miR.26a + miR.29a + miR.362 + miR.369.3p + miR.494 + miR.518c + miR.639 | 0,79 | 0,025363655 |
| miR.558 + miR.609 + miR.603 + miR.520f + miR.220a + miR.519b + miR.130a + miR.26a + miR.362 + miR.369.3p + miR.376a* + miR.494 + miR.639 + miR.338 | 0,79 | 0,008398289 |
| miR.558 + miR.609 + miR.603 + miR.520f + miR.220a + miR.519b + miR.130a + miR.26a + miR.362 + miR.369.3p + miR.376a* + miR.494 + miR.639 + miR.660 | 0,79 | 0,023442674 |
| miR.558 + miR.609 + miR.603 + miR.520f + miR.220a + miR.130a + miR.26a + miR.362 + miR.369.3p + miR.494 + miR.518c + miR.639 + miR.660 + miR.338 | 0,79 | 0,004306052 |
| miR.558 + miR.609 + miR.603 + miR.520f + miR.220a + miR.519b + miR.130a + miR.26a + miR.362 + miR.369.3p + miR.494 + miR.518c + miR.639 + miR.660 | 0,79 | 0,003934717 |
| miR.609 + miR.603 + miR.520f + miR.220a + miR.519b + miR.130a + miR.26a + miR.29a + miR.362 + miR.369.3p + miR.376a* + miR.494 + miR.639 + miR.338 | 0,79 | 0,03639979 |
| miR.558 + miR.609 + miR.603 + miR.220a + miR.519b + miR.130a + miR.26a + miR.362 + miR.369.3p + miR.376a* + miR.494 + miR.639 + miR.660 + miR.338 | 0,79 | 0,061479947 |
| miR.558 + miR.609 + miR.603 + miR.520f + miR.519b + miR.130a + miR.26a + miR.29a + miR.362 + miR.369.3p + miR.494 + miR.518c + miR.639 + miR.338 | 0,79 | 0,023303609 |
| miR.558 + miR.609 + miR.603 + miR.520f + miR.220a + miR.519b + miR.130a + miR.26a + miR.362 + miR.369.3p + miR.518c + miR.639 + miR.660 + miR.338 | 0,79 | 0,005058528 |
| miR.558 + miR.609 + miR.603 + miR.520f + miR.220a + miR.519b + miR.130a + miR.362 + miR.369.3p + miR.494 + miR.518c + miR.639 + miR.660 + miR.338 | 0,79 | 0,005366844 |
| miR.558 + miR.609 + miR.603 + miR.520f + miR.220a + miR.519b + miR.26a + miR.362 + miR.369.3p + miR.494 + miR.518c + miR.639 + miR.660 + miR.338 | 0,79 | 0,005184261 |
| miR.558 + miR.609 + miR.603 + miR.220a + miR.519b + miR.130a + miR.26a + miR.362 + miR.369.3p + miR.494 + miR.518c + miR.639 + miR.660 + miR.338 | 0,79 | 0,00768243 |
| miR.609 + miR.603 + miR.520f + miR.220a + miR.519b + miR.26a + miR.29a + miR.362 + miR.369.3p + miR.494 + miR.518c + miR.639 + miR.660 + miR.338 | 0,79 | 0,007849931 |
| miR.609 + miR.603 + miR.520f + miR.220a + miR.519b + miR.130a + miR.29a + miR.362 + miR.369.3p + miR.494 + miR.518c + miR.639 + miR.660 + miR.338 | 0,79 | 0,00790819 |
| miR.609 + miR.603 + miR.520f + miR.220a + miR.130a + miR.26a + miR.29a + miR.362 + miR.369.3p + miR.494 + miR.518c + miR.639 + miR.660 + miR.338 | 0,79 | 0,005320446 |
| miR.609 + miR.603 + miR.520f + miR.220a + miR.519b + miR.130a + miR.26a + miR.29a + miR.362 + miR.369.3p + miR.494 + miR.518c + miR.639 + miR.660 | 0,79 | 0,004224297 |
| miR.558 + miR.609 + miR.603 + miR.520f + miR.220a + miR.519b + miR.130a + miR.26a + miR.362 + miR.494 + miR.518c + miR.639 + miR.660 + miR.338 | 0,79 | 0,005290236 |
| miR.609 + miR.603 + miR.520f + miR.220a + miR.519b + miR.130a + miR.26a + miR.29a + miR.362 + miR.369.3p + miR.494 + miR.518c + miR.660 + miR.338 | 0,79 | 0,006989892 |
| miR.558 + miR.609 + miR.603 + miR.520f + miR.220a + miR.519b + miR.130a + miR.26a + miR.369.3p + miR.494 + miR.518c + miR.639 + miR.660 + miR.338 | 0,79 | 0,007191019 |
| miR.558 + miR.603 + miR.220a + miR.519b + miR.130a + miR.26a + miR.29a + miR.362 + miR.369.3p + miR.376a* + miR.494 + miR.518c + miR.639 + miR.660 | 0,79 | 0,051643591 |
| miR.558 + miR.609 + miR.603 + miR.220a + miR.519b + miR.130a + miR.26a + miR.29a + miR.362 + miR.376a* + miR.494 + miR.639 + miR.660 + miR.338 | 0,79 | 0,000961238 |
| miR.558 + miR.603 + miR.220a + miR.519b + miR.130a + miR.26a + miR.29a + miR.362 + miR.369.3p + miR.376a* + miR.518c + miR.639 + miR.660 + miR.338 | 0,79 | 0,065690861 |
| miR.558 + miR.603 + miR.520f + miR.220a + miR.519b + miR.130a + miR.29a + miR.362 + miR.369.3p + miR.376a* + miR.518c + miR.639 + miR.660 + miR.338 | 0,79 | 0,036512774 |
| miR.558 + miR.603 + miR.520f + miR.220a + miR.519b + miR.130a + miR.26a + miR.29a + miR.362 + miR.369.3p + miR.376a* + miR.518c + miR.639 + miR.660 | 0,79 | 0,042057641 |
| miR.558 + miR.603 + miR.520f + miR.220a + miR.519b + miR.26a + miR.29a + miR.362 + miR.369.3p + miR.376a* + miR.494 + miR.518c + miR.639 + miR.660 | 0,79 | 0,044866686 |
| miR.558 + miR.603 + miR.520f + miR.220a + miR.519b + miR.130a + miR.29a + miR.362 + miR.369.3p + miR.376a* + miR.494 + miR.518c + miR.639 + miR.660 | 0,79 | 0,03365196 |
| miR.609 + miR.603 + miR.220a + miR.519b + miR.130a + miR.26a + miR.29a + miR.362 + miR.369.3p + miR.494 + miR.518c + miR.639 + miR.660 + miR.338 | 0,79 | 0,005141548 |
| miR.558 + miR.609 + miR.603 + miR.520f + miR.519b + miR.130a + miR.26a + miR.362 + miR.369.3p + miR.494 + miR.518c + miR.639 + miR.660 + miR.338 | 0,79 | 0,00343947 |
| miR.558 + miR.603 + miR.520f + miR.220a + miR.519b + miR.29a + miR.362 + miR.369.3p + miR.376a* + miR.494 + miR.518c + miR.639 + miR.660 + miR.338 | 0,79 | 0,033810602 |
| miR.609 + miR.603 + miR.520f + miR.220a + miR.519b + miR.130a + miR.26a + miR.29a + miR.362 + miR.494 + miR.518c + miR.639 + miR.660 + miR.338 | 0,79 | 0,004106011 |
| miR.558 + miR.603 + miR.520f + miR.220a + miR.519b + miR.130a + miR.26a + miR.29a + miR.362 + miR.369.3p + miR.376a* + miR.494 + miR.518c + miR.639 | 0,79 | 0,040317621 |
| miR.609 + miR.603 + miR.520f + miR.220a + miR.519b + miR.130a + miR.26a + miR.29a + miR.362 + miR.369.3p + miR.518c + miR.639 + miR.660 + miR.338 | 0,79 | 0,007217829 |
| miR.558 + miR.603 + miR.520f + miR.220a + miR.519b + miR.130a + miR.26a + miR.29a + miR.362 + miR.369.3p + miR.376a* + miR.518c + miR.639 + miR.338 | 0,79 | 0,053493608 |
| miR.558 + miR.603 + miR.220a + miR.519b + miR.130a + miR.26a + miR.29a + miR.362 + miR.369.3p + miR.376a* + miR.494 + miR.518c + miR.639 + miR.338 | 0,79 | 0,046238119 |
| miR.558 + miR.603 + miR.520f + miR.220a + miR.519b + miR.130a + miR.29a + miR.362 + miR.369.3p + miR.376a* + miR.494 + miR.518c + miR.639 + miR.338 | 0,79 | 0,02486941 |
| miR.558 + miR.603 + miR.520f + miR.220a + miR.519b + miR.26a + miR.29a + miR.362 + miR.369.3p + miR.376a* + miR.518c + miR.639 + miR.660 + miR.338 | 0,79 | 0,063449632 |
| miR.609 + miR.603 + miR.520f + miR.220a + miR.519b + miR.130a + miR.26a + miR.29a + miR.369.3p + miR.494 + miR.518c + miR.639 + miR.660 + miR.338 | 0,79 | 0,002560894 |
| miR.558 + miR.603 + miR.520f + miR.220a + miR.519b + miR.130a + miR.26a + miR.29a + miR.362 + miR.369.3p + miR.376a* + miR.494 + miR.518c + miR.338 | 0,79 | 0,045666719 |
| miR.558 + miR.603 + miR.520f + miR.220a + miR.519b + miR.130a + miR.26a + miR.29a + miR.362 + miR.369.3p + miR.376a* + miR.494 + miR.518c + miR.660 | 0,79 | 0,045427381 |
| miR.558 + miR.603 + miR.220a + miR.519b + miR.26a + miR.29a + miR.362 + miR.369.3p + miR.376a* + miR.494 + miR.518c + miR.639 + miR.660 + miR.338 | 0,79 | 0,068865951 |
| miR.558 + miR.603 + miR.220a + miR.519b + miR.130a + miR.26a + miR.29a + miR.362 + miR.369.3p + miR.376a* + miR.494 + miR.518c + miR.660 + miR.338 | 0,79 | 0,04441265 |
| miR.558 + miR.603 + miR.520f + miR.220a + miR.519b + miR.26a + miR.29a + miR.362 + miR.369.3p + miR.376a* + miR.494 + miR.518c + miR.660 + miR.338 | 0,79 | 0,06287616 |
| miR.558 + miR.603 + miR.520f + miR.220a + miR.519b + miR.130a + miR.29a + miR.369.3p + miR.376a* + miR.494 + miR.518c + miR.639 + miR.660 + miR.338 | 0,79 | 0,026585437 |
| miR.558 + miR.609 + miR.520f + miR.220a + miR.519b + miR.130a + miR.26a + miR.362 + miR.369.3p + miR.494 + miR.518c + miR.639 + miR.660 + miR.338 | 0,79 | 0,004117076 |
| miR.558 + miR.603 + miR.520f + miR.220a + miR.519b + miR.130a + miR.26a + miR.29a + miR.362 + miR.369.3p + miR.376a* + miR.518c + miR.660 + miR.338 | 0,79 | 0,053326778 |
| miR.558 + miR.603 + miR.220a + miR.519b + miR.130a + miR.29a + miR.362 + miR.369.3p + miR.376a* + miR.494 + miR.518c + miR.639 + miR.660 + miR.338 | 0,79 | 0,039165413 |
| miR.558 + miR.603 + miR.520f + miR.220a + miR.519b + miR.130a + miR.26a + miR.29a + miR.369.3p + miR.376a* + miR.494 + miR.518c + miR.639 + miR.660 | 0,79 | 0,037618768 |
| miR.558 + miR.603 + miR.520f + miR.220a + miR.519b + miR.26a + miR.29a + miR.362 + miR.369.3p + miR.376a* + miR.494 + miR.518c + miR.639 + miR.338 | 0,79 | 0,047400005 |
| miR.558 + miR.609 + miR.603 + miR.520f + miR.220a + miR.519b + miR.130a + miR.26a + miR.362 + miR.369.3p + miR.494 + miR.518c + miR.660 + miR.338 | 0,79 | 0,013092072 |
| miR.609 + miR.520f + miR.220a + miR.519b + miR.130a + miR.26a + miR.29a + miR.362 + miR.369.3p + miR.494 + miR.518c + miR.639 + miR.660 + miR.338 | 0,79 | 0,000581235 |
| miR.558 + miR.603 + miR.520f + miR.220a + miR.519b + miR.130a + miR.26a + miR.29a + miR.369.3p + miR.376a* + miR.518c + miR.639 + miR.660 + miR.338 | 0,79 | 0,034082818 |
| miR.558 + miR.603 + miR.520f + miR.220a + miR.519b + miR.130a + miR.29a + miR.362 + miR.369.3p + miR.376a* + miR.494 + miR.518c + miR.660 + miR.338 | 0,79 | 0,030151994 |
| miR.558 + miR.603 + miR.520f + miR.220a + miR.519b + miR.26a + miR.29a + miR.369.3p + miR.376a* + miR.494 + miR.518c + miR.639 + miR.660 + miR.338 | 0,79 | 0,037594907 |
| miR.558 + miR.603 + miR.520f + miR.220a + miR.519b + miR.130a + miR.26a + miR.29a + miR.369.3p + miR.376a* + miR.494 + miR.518c + miR.639 + miR.338 | 0,79 | 0,035674694 |
| miR.558 + miR.603 + miR.520f + miR.220a + miR.519b + miR.130a + miR.26a + miR.362 + miR.369.3p + miR.376a* + miR.494 + miR.518c + miR.639 + miR.660 | 0,79 | 0,03884114 |
| miR.558 + miR.603 + miR.220a + miR.519b + miR.130a + miR.26a + miR.29a + miR.369.3p + miR.376a* + miR.494 + miR.518c + miR.639 + miR.660 + miR.338 | 0,79 | 0,037521581 |
| miR.558 + miR.603 + miR.520f + miR.220a + miR.519b + miR.130a + miR.26a + miR.29a + miR.369.3p + miR.376a* + miR.494 + miR.518c + miR.660 + miR.338 | 0,79 | 0,04121026 |
| miR.558 + miR.603 + miR.520f + miR.220a + miR.519b + miR.130a + miR.26a + miR.362 + miR.369.3p + miR.376a* + miR.518c + miR.639 + miR.660 + miR.338 | 0,79 | 0,046840855 |
| miR.558 + miR.603 + miR.520f + miR.220a + miR.519b + miR.26a + miR.362 + miR.369.3p + miR.376a* + miR.494 + miR.518c + miR.639 + miR.660 + miR.338 | 0,79 | 0,045365571 |
| miR.609 + miR.603 + miR.520f + miR.519b + miR.130a + miR.26a + miR.29a + miR.362 + miR.369.3p + miR.494 + miR.518c + miR.639 + miR.660 + miR.338 | 0,79 | 0,002045457 |
| miR.558 + miR.603 + miR.520f + miR.220a + miR.519b + miR.130a + miR.362 + miR.369.3p + miR.376a* + miR.494 + miR.518c + miR.639 + miR.660 + miR.338 | 0,79 | 0,035765779 |
| miR.558 + miR.603 + miR.220a + miR.519b + miR.130a + miR.26a + miR.362 + miR.369.3p + miR.376a* + miR.494 + miR.518c + miR.639 + miR.660 + miR.338 | 0,79 | 0,052336529 |
| miR.558 + miR.603 + miR.520f + miR.220a + miR.519b + miR.130a + miR.26a + miR.362 + miR.369.3p + miR.376a* + miR.494 + miR.518c + miR.639 + miR.338 | 0,79 | 0,036657315 |
| miR.558 + miR.603 + miR.220a + miR.130a + miR.26a + miR.29a + miR.362 + miR.369.3p + miR.376a* + miR.494 + miR.518c + miR.639 + miR.660 + miR.338 | 0,79 | 0,030590384 |
| miR.558 + miR.609 + miR.603 + miR.520f + miR.220a + miR.519b + miR.130a + miR.26a + miR.362 + miR.369.3p + miR.494 + miR.518c + miR.639 + miR.338 | 0,79 | 0,006176138 |
| miR.558 + miR.603 + miR.520f + miR.220a + miR.130a + miR.26a + miR.29a + miR.362 + miR.369.3p + miR.376a* + miR.494 + miR.518c + miR.639 + miR.660 | 0,78 | 0,030580245 |
| miR.558 + miR.603 + miR.520f + miR.220a + miR.26a + miR.29a + miR.362 + miR.369.3p + miR.376a* + miR.494 + miR.518c + miR.639 + miR.660 + miR.338 | 0,78 | 0,029932015 |
| miR.558 + miR.603 + miR.520f + miR.220a + miR.519b + miR.130a + miR.26a + miR.362 + miR.369.3p + miR.376a* + miR.494 + miR.518c + miR.660 + miR.338 | 0,78 | 0,038297104 |
| miR.558 + miR.603 + miR.520f + miR.220a + miR.519b + miR.130a + miR.26a + miR.369.3p + miR.376a* + miR.494 + miR.518c + miR.639 + miR.660 + miR.338 | 0,78 | 0,035548638 |
| miR.558 + miR.603 + miR.520f + miR.220a + miR.130a + miR.26a + miR.362 + miR.369.3p + miR.376a* + miR.494 + miR.518c + miR.639 + miR.660 + miR.338 | 0,78 | 0,021213589 |
| miR.558 + miR.603 + miR.520f + miR.220a + miR.130a + miR.26a + miR.29a + miR.362 + miR.369.3p + miR.376a* + miR.518c + miR.639 + miR.660 + miR.338 | 0,78 | 0,025821406 |
| miR.558 + miR.603 + miR.520f + miR.220a + miR.130a + miR.26a + miR.29a + miR.362 + miR.369.3p + miR.376a* + miR.494 + miR.518c + miR.639 + miR.338 | 0,78 | 0,011696159 |
| miR.558 + miR.603 + miR.520f + miR.220a + miR.130a + miR.29a + miR.362 + miR.369.3p + miR.376a* + miR.494 + miR.518c + miR.639 + miR.660 + miR.338 | 0,78 | 0,018113387 |
| miR.558 + miR.603 + miR.520f + miR.220a + miR.519b + miR.130a + miR.29a + miR.362 + miR.376a* + miR.494 + miR.518c + miR.639 + miR.660 + miR.338 | 0,78 | 0,015425693 |
| miR.558 + miR.603 + miR.220a + miR.519b + miR.130a + miR.26a + miR.29a + miR.362 + miR.376a* + miR.494 + miR.518c + miR.639 + miR.660 + miR.338 | 0,78 | 0,023097884 |
| miR.558 + miR.603 + miR.520f + miR.220a + miR.519b + miR.130a + miR.26a + miR.29a + miR.362 + miR.376a* + miR.494 + miR.518c + miR.639 + miR.660 | 0,78 | 0,024569319 |
| miR.558 + miR.603 + miR.520f + miR.220a + miR.130a + miR.26a + miR.29a + miR.369.3p + miR.376a* + miR.494 + miR.518c + miR.639 + miR.660 + miR.338 | 0,78 | 0,008888786 |
| miR.558 + miR.603 + miR.520f + miR.220a + miR.519b + miR.130a + miR.26a + miR.29a + miR.362 + miR.376a* + miR.494 + miR.518c + miR.660 + miR.338 | 0,78 | 0,020894465 |
| miR.558 + miR.603 + miR.520f + miR.220a + miR.519b + miR.130a + miR.26a + miR.29a + miR.362 + miR.376a* + miR.494 + miR.518c + miR.639 + miR.338 | 0,78 | 0,013299731 |
| miR.558 + miR.603 + miR.520f + miR.220a + miR.519b + miR.130a + miR.26a + miR.29a + miR.376a* + miR.494 + miR.518c + miR.639 + miR.660 + miR.338 | 0,78 | 0,013858012 |
| miR.558 + miR.603 + miR.520f + miR.220a + miR.519b + miR.130a + miR.26a + miR.29a + miR.362 + miR.376a* + miR.518c + miR.639 + miR.660 + miR.338 | 0,78 | 0,017900737 |
| miR.558 + miR.603 + miR.520f + miR.220a + miR.519b + miR.26a + miR.29a + miR.362 + miR.376a* + miR.494 + miR.518c + miR.639 + miR.660 + miR.338 | 0,78 | 0,017263067 |
| miR.609 + miR.603 + miR.520f + miR.220a + miR.519b + miR.130a + miR.26a + miR.362 + miR.369.3p + miR.494 + miR.518c + miR.639 + miR.660 + miR.338 | 0,78 | 0,005967248 |
| miR.558 + miR.603 + miR.520f + miR.519b + miR.130a + miR.26a + miR.29a + miR.362 + miR.369.3p + miR.376a* + miR.518c + miR.639 + miR.660 + miR.338 | 0,78 | 0,023698113 |
| miR.558 + miR.603 + miR.520f + miR.519b + miR.130a + miR.29a + miR.362 + miR.369.3p + miR.376a* + miR.494 + miR.518c + miR.639 + miR.660 + miR.338 | 0,78 | 0,021111105 |
| miR.558 + miR.603 + miR.520f + miR.220a + miR.130a + miR.26a + miR.29a + miR.362 + miR.369.3p + miR.376a* + miR.494 + miR.518c + miR.660 + miR.338 | 0,78 | 0,016725226 |
| miR.558 + miR.603 + miR.520f + miR.519b + miR.26a + miR.29a + miR.362 + miR.369.3p + miR.376a* + miR.494 + miR.518c + miR.639 + miR.660 + miR.338 | 0,78 | 0,019684948 |
| miR.558 + miR.603 + miR.520f + miR.519b + miR.130a + miR.26a + miR.29a + miR.362 + miR.369.3p + miR.376a* + miR.494 + miR.518c + miR.639 + miR.338 | 0,78 | 0,018456147 |
| miR.558 + miR.609 + miR.603 + miR.520f + miR.519b + miR.130a + miR.26a + miR.29a + miR.362 + miR.369.3p + miR.494 + miR.639 + miR.660 + miR.338 | 0,78 | 0,006056795 |
| miR.558 + miR.609 + miR.603 + miR.520f + miR.220a + miR.519b + miR.130a + miR.29a + miR.362 + miR.369.3p + miR.494 + miR.639 + miR.660 + miR.338 | 0,78 | 0,004485767 |
| miR.609 + miR.603 + miR.520f + miR.220a + miR.519b + miR.130a + miR.26a + miR.29a + miR.362 + miR.369.3p + miR.494 + miR.518c + miR.639 + miR.338 | 0,78 | 0,003432279 |
| miR.558 + miR.609 + miR.520f + miR.220a + miR.519b + miR.130a + miR.26a + miR.29a + miR.362 + miR.369.3p + miR.494 + miR.639 + miR.660 + miR.338 | 0,78 | 0,003576897 |
| miR.558 + miR.603 + miR.520f + miR.220a + miR.519b + miR.130a + miR.26a + miR.362 + miR.376a* + miR.494 + miR.518c + miR.639 + miR.660 + miR.338 | 0,78 | 0,01023259 |
| miR.558 + miR.603 + miR.520f + miR.519b + miR.130a + miR.26a + miR.362 + miR.369.3p + miR.376a* + miR.494 + miR.518c + miR.639 + miR.660 + miR.338 | 0,78 | 0,013120117 |
| miR.558 + miR.603 + miR.519b + miR.130a + miR.26a + miR.29a + miR.362 + miR.369.3p + miR.376a* + miR.494 + miR.518c + miR.639 + miR.660 + miR.338 | 0,78 | 0,039952459 |
| miR.558 + miR.609 + miR.603 + miR.520f + miR.220a + miR.130a + miR.26a + miR.29a + miR.362 + miR.369.3p + miR.494 + miR.639 + miR.660 + miR.338 | 0,78 | 0,0032938 |
| miR.558 + miR.603 + miR.520f + miR.130a + miR.26a + miR.29a + miR.362 + miR.369.3p + miR.376a* + miR.494 + miR.518c + miR.639 + miR.660 + miR.338 | 0,78 | 0,009754549 |
| miR.558 + miR.609 + miR.603 + miR.520f + miR.220a + miR.519b + miR.130a + miR.26a + miR.29a + miR.362 + miR.369.3p + miR.494 + miR.639 + miR.660 | 0,78 | 0,005980976 |
| miR.558 + miR.609 + miR.603 + miR.520f + miR.220a + miR.519b + miR.130a + miR.26a + miR.29a + miR.362 + miR.494 + miR.639 + miR.660 + miR.338 | 0,78 | 0,00562775 |
| miR.558 + miR.603 + miR.520f + miR.519b + miR.130a + miR.26a + miR.29a + miR.362 + miR.369.3p + miR.376a* + miR.494 + miR.518c + miR.639 + miR.660 | 0,78 | 0,022485569 |
| miR.603 + miR.520f + miR.220a + miR.519b + miR.26a + miR.29a + miR.362 + miR.369.3p + miR.376a* + miR.494 + miR.518c + miR.639 + miR.660 + miR.338 | 0,78 | 0,015872651 |
| miR.558 + miR.609 + miR.603 + miR.520f + miR.220a + miR.519b + miR.26a + miR.29a + miR.362 + miR.369.3p + miR.494 + miR.639 + miR.660 + miR.338 | 0,78 | 0,00451448 |
| miR.603 + miR.520f + miR.220a + miR.519b + miR.130a + miR.26a + miR.29a + miR.362 + miR.369.3p + miR.376a* + miR.518c + miR.639 + miR.660 + miR.338 | 0,78 | 0,01868585 |
| miR.603 + miR.520f + miR.220a + miR.519b + miR.130a + miR.29a + miR.362 + miR.369.3p + miR.376a* + miR.494 + miR.518c + miR.639 + miR.660 + miR.338 | 0,78 | 0,014360381 |
| miR.603 + miR.520f + miR.220a + miR.519b + miR.130a + miR.26a + miR.29a + miR.362 + miR.369.3p + miR.376a* + miR.494 + miR.518c + miR.639 + miR.660 | 0,78 | 0,013393427 |
| miR.603 + miR.220a + miR.519b + miR.130a + miR.26a + miR.29a + miR.362 + miR.369.3p + miR.376a* + miR.494 + miR.518c + miR.639 + miR.660 + miR.338 | 0,78 | 0,019659735 |
| miR.603 + miR.520f + miR.220a + miR.519b + miR.130a + miR.26a + miR.362 + miR.369.3p + miR.376a* + miR.494 + miR.518c + miR.639 + miR.660 + miR.338 | 0,78 | 0,017653305 |
| miR.558 + miR.603 + miR.220a + miR.519b + miR.130a + miR.26a + miR.29a + miR.362 + miR.369.3p + miR.494 + miR.518c + miR.639 + miR.660 + miR.338 | 0,78 | 0,027608733 |
| miR.558 + miR.609 + miR.603 + miR.520f + miR.220a + miR.519b + miR.130a + miR.26a + miR.29a + miR.362 + miR.369.3p + miR.494 + miR.660 + miR.338 | 0,78 | 0,006852495 |
| miR.558 + miR.603 + miR.520f + miR.220a + miR.519b + miR.130a + miR.26a + miR.29a + miR.362 + miR.369.3p + miR.494 + miR.518c + miR.660 + miR.338 | 0,78 | 0,025823419 |
| miR.558 + miR.603 + miR.520f + miR.519b + miR.130a + miR.26a + miR.29a + miR.369.3p + miR.376a* + miR.494 + miR.518c + miR.639 + miR.660 + miR.338 | 0,78 | 0,015729623 |
| miR.558 + miR.603 + miR.520f + miR.519b + miR.130a + miR.26a + miR.29a + miR.362 + miR.369.3p + miR.376a* + miR.494 + miR.518c + miR.660 + miR.338 | 0,78 | 0,03289423 |
| miR.558 + miR.603 + miR.520f + miR.220a + miR.130a + miR.26a + miR.29a + miR.362 + miR.376a* + miR.494 + miR.518c + miR.639 + miR.660 + miR.338 | 0,78 | 0,001930455 |
| miR.603 + miR.520f + miR.220a + miR.519b + miR.130a + miR.26a + miR.29a + miR.362 + miR.369.3p + miR.376a* + miR.494 + miR.518c + miR.639 + miR.338 | 0,78 | 0,00955262 |
| miR.603 + miR.520f + miR.220a + miR.130a + miR.26a + miR.29a + miR.362 + miR.369.3p + miR.376a* + miR.494 + miR.518c + miR.639 + miR.660 + miR.338 | 0,78 | 0,011944047 |
| miR.558 + miR.603 + miR.520f + miR.220a + miR.519b + miR.130a + miR.26a + miR.29a + miR.369.3p + miR.494 + miR.518c + miR.639 + miR.660 + miR.338 | 0,78 | 0,019952903 |
| miR.558 + miR.603 + miR.520f + miR.220a + miR.519b + miR.130a + miR.26a + miR.29a + miR.362 + miR.369.3p + miR.494 + miR.518c + miR.639 + miR.338 | 0,78 | 0,030585667 |
| miR.603 + miR.520f + miR.220a + miR.519b + miR.130a + miR.26a + miR.29a + miR.362 + miR.369.3p + miR.376a* + miR.494 + miR.518c + miR.660 + miR.338 | 0,78 | 0,02036552 |
| miR.558 + miR.603 + miR.520f + miR.220a + miR.519b + miR.130a + miR.26a + miR.29a + miR.362 + miR.369.3p + miR.518c + miR.639 + miR.660 + miR.338 | 0,78 | 0,022737674 |
| miR.558 + miR.609 + miR.603 + miR.520f + miR.220a + miR.519b + miR.130a + miR.26a + miR.29a + miR.362 + miR.369.3p + miR.639 + miR.660 + miR.338 | 0,78 | 0,002284354 |
| miR.558 + miR.603 + miR.520f + miR.220a + miR.519b + miR.130a + miR.29a + miR.362 + miR.369.3p + miR.494 + miR.518c + miR.639 + miR.660 + miR.338 | 0,78 | 0,017434767 |
| miR.558 + miR.603 + miR.520f + miR.220a + miR.519b + miR.130a + miR.26a + miR.29a + miR.362 + miR.369.3p + miR.494 + miR.518c + miR.639 + miR.660 | 0,78 | 0,019895237 |
| miR.603 + miR.520f + miR.220a + miR.519b + miR.130a + miR.26a + miR.29a + miR.369.3p + miR.376a* + miR.494 + miR.518c + miR.639 + miR.660 + miR.338 | 0,78 | 0,006389498 |
| miR.558 + miR.603 + miR.520f + miR.220a + miR.519b + miR.26a + miR.29a + miR.362 + miR.369.3p + miR.494 + miR.518c + miR.639 + miR.660 + miR.338 | 0,78 | 0,023506388 |
| miR.558 + miR.603 + miR.520f + miR.220a + miR.519b + miR.130a + miR.26a + miR.29a + miR.362 + miR.494 + miR.518c + miR.639 + miR.660 + miR.338 | 0,78 | 0,012217828 |
| miR.558 + miR.603 + miR.520f + miR.519b + miR.130a + miR.26a + miR.29a + miR.362 + miR.376a* + miR.494 + miR.518c + miR.639 + miR.660 + miR.338 | 0,78 | 0,004743746 |
| miR.558 + miR.603 + miR.520f + miR.220a + miR.519b + miR.130a + miR.26a + miR.362 + miR.369.3p + miR.494 + miR.518c + miR.639 + miR.660 + miR.338 | 0,78 | 0,010958841 |
| miR.558 + miR.609 + miR.603 + miR.220a + miR.519b + miR.130a + miR.26a + miR.29a + miR.362 + miR.369.3p + miR.494 + miR.639 + miR.660 + miR.338 | 0,78 | 0,004310703 |
| miR.558 + miR.609 + miR.603 + miR.520f + miR.220a + miR.519b + miR.130a + miR.26a + miR.29a + miR.369.3p + miR.494 + miR.639 + miR.660 + miR.338 | 0,78 | 0,001676862 |
| miR.558 + miR.603 + miR.520f + miR.220a + miR.130a + miR.26a + miR.29a + miR.362 + miR.369.3p + miR.494 + miR.518c + miR.639 + miR.660 + miR.338 | 0,77 | 0,010269422 |
| miR.558 + miR.520f + miR.220a + miR.519b + miR.130a + miR.29a + miR.362 + miR.369.3p + miR.376a* + miR.494 + miR.518c + miR.639 + miR.660 + miR.338 | 0,77 | 0,010915222 |
| miR.558 + miR.520f + miR.220a + miR.519b + miR.130a + miR.26a + miR.29a + miR.362 + miR.369.3p + miR.376a* + miR.518c + miR.639 + miR.660 + miR.338 | 0,77 | 0,018014321 |
| miR.558 + miR.609 + miR.603 + miR.520f + miR.220a + miR.519b + miR.130a + miR.26a + miR.362 + miR.369.3p + miR.494 + miR.639 + miR.660 + miR.338 | 0,77 | 0,00816644 |
| miR.558 + miR.520f + miR.220a + miR.519b + miR.26a + miR.29a + miR.362 + miR.369.3p + miR.376a* + miR.494 + miR.518c + miR.639 + miR.660 + miR.338 | 0,77 | 0,011387087 |
| miR.558 + miR.520f + miR.220a + miR.519b + miR.130a + miR.26a + miR.362 + miR.369.3p + miR.376a* + miR.494 + miR.518c + miR.639 + miR.660 + miR.338 | 0,77 | 0,011993783 |
| miR.558 + miR.520f + miR.220a + miR.130a + miR.26a + miR.29a + miR.362 + miR.369.3p + miR.376a* + miR.494 + miR.518c + miR.639 + miR.660 + miR.338 | 0,77 | 0,010071406 |
| miR.558 + miR.520f + miR.220a + miR.519b + miR.130a + miR.26a + miR.29a + miR.362 + miR.369.3p + miR.376a* + miR.494 + miR.518c + miR.639 + miR.660 | 0,77 | 0,011844227 |
| miR.558 + miR.520f + miR.220a + miR.519b + miR.130a + miR.26a + miR.29a + miR.362 + miR.369.3p + miR.376a* + miR.494 + miR.518c + miR.639 + miR.338 | 0,77 | 0,022616737 |
| miR.603 + miR.520f + miR.220a + miR.519b + miR.130a + miR.26a + miR.29a + miR.362 + miR.376a* + miR.494 + miR.518c + miR.639 + miR.660 + miR.338 | 0,77 | 0,002355668 |
| miR.558 + miR.520f + miR.519b + miR.130a + miR.26a + miR.29a + miR.362 + miR.369.3p + miR.376a* + miR.494 + miR.518c + miR.639 + miR.660 + miR.338 | 0,77 | 0,002827991 |
| miR.558 + miR.603 + miR.520f + miR.519b + miR.130a + miR.26a + miR.29a + miR.362 + miR.369.3p + miR.494 + miR.518c + miR.639 + miR.660 + miR.338 | 0,77 | 0,008202613 |
| miR.603 + miR.520f + miR.519b + miR.130a + miR.26a + miR.29a + miR.362 + miR.369.3p + miR.376a* + miR.494 + miR.518c + miR.639 + miR.660 + miR.338 | 0,77 | 0,002968385 |
| miR.558 + miR.520f + miR.220a + miR.519b + miR.130a + miR.26a + miR.29a + miR.369.3p + miR.376a* + miR.494 + miR.518c + miR.639 + miR.660 + miR.338 | 0,77 | 0,014012981 |
| miR.558 + miR.520f + miR.220a + miR.519b + miR.130a + miR.26a + miR.29a + miR.362 + miR.369.3p + miR.376a* + miR.494 + miR.518c + miR.660 + miR.338 | 0,77 | 0,014131251 |
| miR.609 + miR.603 + miR.520f + miR.220a + miR.519b + miR.130a + miR.26a + miR.29a + miR.362 + miR.369.3p + miR.494 + miR.639 + miR.660 + miR.338 | 0,77 | 0,003183899 |
| miR.558 + miR.609 + miR.603 + miR.520f + miR.220a + miR.519b + miR.130a + miR.26a + miR.29a + miR.362 + miR.369.3p + miR.494 + miR.639 + miR.338 | 0,77 | 0,001665673 |
| miR.558 + miR.220a + miR.519b + miR.130a + miR.26a + miR.29a + miR.362 + miR.369.3p + miR.376a* + miR.494 + miR.518c + miR.639 + miR.660 + miR.338 | 0,77 | 0,018514106 |
| miR.558 + miR.520f + miR.220a + miR.519b + miR.130a + miR.26a + miR.29a + miR.362 + miR.376a* + miR.494 + miR.518c + miR.639 + miR.660 + miR.338 | 0,77 | 0,004861981 |
| miR.603 + miR.520f + miR.220a + miR.519b + miR.130a + miR.26a + miR.29a + miR.362 + miR.369.3p + miR.494 + miR.518c + miR.639 + miR.660 + miR.338 | 0,77 | 0,001455528 |
| miR.558 + miR.520f + miR.220a + miR.519b + miR.130a + miR.26a + miR.29a + miR.362 + miR.369.3p + miR.494 + miR.518c + miR.639 + miR.660 + miR.338 | 0,77 | 0,004785135 |
| miR.520f + miR.220a + miR.519b + miR.130a + miR.26a + miR.29a + miR.362 + miR.369.3p + miR.376a* + miR.494 + miR.518c + miR.639 + miR.660 + miR.338 | 0,76 | 0,000466356 |
| miR.558 + miR.603 + miR.520f + miR.519b + miR.130a + miR.26a + miR.29a + miR.362 + miR.369.3p + miR.376a* + miR.494 + miR.639 + miR.660 + miR.338 | 0,76 | 0,001480874 |
| miR.558 + miR.603 + miR.520f + miR.220a + miR.519b + miR.130a + miR.26a + miR.362 + miR.369.3p + miR.376a* + miR.494 + miR.639 + miR.660 + miR.338 | 0,76 | 0,0016 66107 |
| miR.558 + miR.603 + miR.520f + miR.220a + miR.519b + miR.26a + miR.29a + miR.362 + miR.369.3p + miR.376a* + miR.494 + miR.639 + miR.660 + miR.338 | 0,76 | 0,002647249 |
| miR.558 + miR.603 + miR.520f + miR.220a + miR.519b + miR.130a + miR.29a + miR.362 + miR.369.3p + miR.376a* + miR.494 + miR.639 + miR.660 + miR.338 | 0,76 | 0,002038337 |
| miR.558 + miR.603 + miR.520f + miR.220a + miR.519b + miR.130a + miR.26a + miR.29a + miR.362 + miR.369.3p + miR.376a* + miR.639 + miR.660 + miR.338 | 0,75 | 0,000724525 |
| miR.558 + miR.603 + miR.520f + miR.220a + miR.130a + miR.26a + miR.29a + miR.362 + miR.369.3p + miR.376a* + miR.494 + miR.639 + miR.660 + miR.338 | 0,75 | 0,000438559 |
| miR.558 + miR.603 + miR.520f + miR.220a + miR.519b + miR.130a + miR.26a + miR.29a + miR.362 + miR.369.3p + miR.376a* + miR.494 + miR.660 + miR.338 | 0,75 | 0,004191131 |
| miR.558 + miR.603 + miR.520f + miR.220a + miR.519b + miR.130a + miR.26a + miR.29a + miR.362 + miR.369.3p + miR.376a* + miR.494 + miR.639 + miR.338 | 0,75 | 0,001704329 |
| miR.558 + miR.603 + miR.520f + miR.220a + miR.519b + miR.130a + miR.26a + miR.29a + miR.362 + miR.369.3p + miR.376a* + miR.494 + miR.639 + miR.660 | 0,75 | 0,000807655 |
| miR.558 + miR.520f + miR.220a + miR.519b + miR.130a + miR.26a + miR.29a + miR.362 + miR.369.3p + miR.376a* + miR.494 + miR.639 + miR.660 + miR.338 | 0,75 | 0,001349938 |
| miR.558 + miR.603 + miR.520f + miR.220a + miR.519b + miR.130a + miR.26a + miR.29a + miR.362 + miR.376a* + miR.494 + miR.639 + miR.660 + miR.338 | 0,75 | 0,000296002 |
| miR.603 + miR.520f + miR.220a + miR.519b + miR.130a + miR.26a + miR.29a + miR.362 + miR.369.3p + miR.376a* + miR.494 + miR.639 + miR.660 + miR.338 | 0,75 | 0,00077583 |
| miR.558 + miR.603 + miR.520f + miR.220a + miR.519b + miR.130a + miR.26a + miR.29a + miR.369.3p + miR.376a* + miR.494 + miR.639 + miR.660 + miR.338 | 0,75 | 0,000781236 |
| miR.558 + miR.603 + miR.220a + miR.519b + miR.130a + miR.26a + miR.29a + miR.362 + miR.369.3p + miR.376a* + miR.494 + miR.639 + miR.660 + miR.338 | 0,75 | 0,002537045 |
| miR.558 + miR.603 + miR.520f + miR.220a + miR.519b + miR.130a + miR.26a + miR.29a + miR.362 + miR.369.3p + miR.494 + miR.639 + miR.660 + miR.338 | 0,74 | 0,00066002 |

**Table 15: clusters of 15 miRNAs**

| **Cluster of 15 miRs** | **c_tau level** | **pv compared to top signat ure miR.5 58 + miR.6 09 + miR.6 03 + miR.5 20f + miR.1 30a + miR.2 6a + miR.2 9a + miR.3 62 + miR.3 69.3p + miR.3 76a* + miR.4 94 + miR.5 18c + miR.6 39 + miR.6 60 + miR.3 38** |
|---|---|---|
| miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 | 0,82 | NaN |
| miR.558 + miR.609 + miR.603 + miR.520f + miR.519b + miR.130a + miR.29a + miR.362 + miR.369.3p + miR.376a. + miR.494 + miR.518c + miR.639 + miR.660 + miR.338 | 0,82 | 0,41789311 2 |
| miR.558 + miR.609 + miR.603 + miR.220a + miR.130a + miR.26a + miR.29a + miR.362 + miR.369.3p + miR.376a. + miR.494 + miR.518c + miR.639 + miR.660 + miR.338 | 0,82 | 0,42194783 4 |
| miR.558 + miR.609 + miR.603 + miR.520f + miR.220a + miR.130a + miR.29a + miR.362 + miR.369.3p + miR.376a. + miR.494 + miR.518c + miR.639 + miR.660 + miR.338 | 0,82 | 0,45672365 9 |
| miR.558 + miR.609 + miR.603 + miR.520f + miR.220a + miR.26a + miR.29a + miR.362 + miR.369.3p + miR.376a. + miR.494 + miR.518c + miR.639 + miR.660 + miR.338 | 0,82 | 0,43647204 2 |
| miR.558 + miR.609 + miR.603 + miR.519b + miR.130a + miR.26a + miR.29a + miR.362 + miR.369.3p + miR.376a. + miR.494 + miR.518c + miR.639 + miR.660 + miR.338 | 0,82 | 0,26611971 3 |
| miR.558 + miR.609 + miR.520f + miR.519b + miR.130a + miR.26a + miR.29a + miR.362 + miR.369.3p + miR.376a. + miR.494 + miR.518c + miR.639 + miR.660 + miR.338 | 0,82 | 0,02691938 3 |
| miR.558 + miR.609 + miR.603 + miR.520f + miR.220a + miR.519b + miR.29a + miR.362 + miR.369.3p + miR.376a. + miR.494 + miR.518c + miR.639 + miR.660 + miR.338 | 0,82 | 0,450 22922 9 |
| miR.558 + miR.609 + miR.603 + miR.520f + miR.220a + miR.130a + miR.26a + miR.29a + miR.362 + miR.369.3p + miR.376a. + miR.518c + miR.639 + miR.660 + miR.338 | 0,82 | 0,45004679 7 |
| miR.558 + miR.609 + miR.520f + miR.220a + miR.130a + miR.26a + miR.29a + miR.362 + miR.369.3p + miR.376a. + miR.494 + miR.518c + miR.639 + miR.660 + miR.338 | 0,82 | 0,00669368 2 |
| miR.558 + miR.609 + miR.520f + miR.220a + miR.519b + miR.130a + miR.29a + miR.362 + miR.369.3p + miR.376a. + miR.494 + miR.518c + miR.639 + miR.660 + miR.338 | 0,82 | 0,00927235 5 |
| miR.558 + miR.609 + miR.603 + miR.520f + miR.220a + miR.519b + miR.26a + miR.29a + miR.362 + miR.369.3p + miR.376a. + miR.518c + miR.639 + miR.660 + miR.338 | 0,82 | 0,45529395 4 |
| miR.558 + miR.609 + miR.603 + miR.220a + miR.519b + miR.26a + miR.29a + miR.362 + miR.369.3p + miR.376a. + miR.494 + miR.518c + miR.639 + miR.660 + miR.338 | 0,81 | 0,48228721 3 |
| miR.558 + miR.609 + miR.603 + miR.220a + miR.519b + miR.130a + miR.29a + miR.362 + miR.369.3p + miR.376a. + miR.494 + miR.518c + miR.639 + miR.660 + miR.338 | 0,81 | 0,31621487 3 |
| miR.558 + miR.609 + miR.520f + miR.220a + miR.519b + miR.26a + miR.29a + miR.362 + miR.369.3p + miR.376a. + miR.494 + miR.518c + miR.639 + miR.660 + miR.338 | 0,81 | 0,07478367 2 |
| miR.558 + miR.609 + miR.603 + miR.520f + miR.220a + miR.519b + miR.130a + miR.29a + miR.362 + miR.369.3p + miR.376a. + miR.518c + miR.639 + miR.660 + miR.338 | 0,81 | 0,43528859 1 |
| miR.558 + miR.609 + miR.603 + miR.520f + miR.519b + miR.26a + miR.29a + miR.362 + miR.369.3p + miR.376a. + miR.494 + miR.518c + miR.639 + miR.660 + miR.338 | 0,81 | 0,47884557 8 |
| miR.558 + miR.609 + miR.603 + miR.520f + miR.220a + miR.519b + miR.130a + miR.29a + miR.369.3p + miR.376a. + miR.494 + miR.518c + miR.639 + miR.660 + miR.338 | 0,81 | 0,21054605 7 |
| miR.558 + miR.609 + miR.603 + miR.520f + miR.220a + miR.519b + miR.26a + miR.29a + miR.362 + miR.369.3p + miR.376a. + miR.494 + miR.518c + miR.660 + miR.338 | 0,81 | 0,46822726 2 |
| miR.558 + miR.609 + miR.603 + miR.520f + miR.220a + miR.519b + miR.26a + miR.29a + miR.369.3p + miR.376a. + miR.494 + miR.518c + miR.639 + miR.660 + miR.338 | 0,81 | 0,22355025 2 |
| miR.558 + miR.609 + miR.603 + miR.220a + miR.519b + miR.130a + miR.26a + miR.29a + miR.362 + miR.369.3p + miR.376a. + miR.494 + miR.518c + miR.660 + miR.338 | 0,81 | 0,28674531 7 |
| miR.558 + miR.609 + miR.603 + miR.520f + miR.220a + miR.519b + miR.130a + miR.29a + miR.362 + miR.369.3p + miR.376a. + miR.494 + miR.518c + miR.660 + miR.338 | 0,81 | 0,39198935 3 |
| miR.558 + miR.609 + miR.520f + miR.220a + miR.519b + miR.130a + miR.26a + miR.29a + miR.362 + miR.369.3p + miR.376a. + miR.518c + miR.639 + miR.660 + miR.338 | 0,81 | 0,10336815 8 |
| miR.558 + miR.609 + miR.603 + miR.220a + miR.519b + miR.130a + miR.26a + miR.29a + miR.362 + miR.369.3p + miR.376a. + miR.518c + miR.639 + miR.660 + miR.338 | 0,81 | 0,48975352 2 |
| miR.558 + miR.609 + miR.603 + miR.520f + miR.220a + miR.130a + miR.26a + miR.29a + miR.369.3p + miR.376a. + miR.494 + miR.518c + miR.639 + miR.660 + miR.338 | 0,81 | 0,09274832 1 |
| miR.558 + miR.609 + miR.603 + miR.520f + miR.519b + miR.130a + miR.26a + miR.29a + miR.362 + miR.369.3p + miR.376a. + miR.518c + miR.639 + miR.660 + miR.338 | 0,81 | 0,47465141 1 |
| miR.558 + miR.609 + miR.603 + miR.220a + miR.519b + miR.130a + miR.26a + miR.29a + miR.369.3p + miR.376a. + miR.494 + miR.518c + miR.639 + miR.660 + miR.338 | 0,81 | 0,16270038 7 |
| miR.558 + miR.609 + miR.603 + miR.520f + miR.220a + miR.519b + miR.130a + miR.26a + miR.29a + miR.369.3p + miR.376a. + miR.494 + miR.518c + miR.660 + miR.338 | 0,81 | 0,17522995 |
| miR.558 + miR.609 + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,34357476 |
| miR.558 + miR.609 + miR.603 + miR.520f + miR.220a + miR.130a + miR.26a + miR.29a + miR.362 + miR.369.3p + miR.376a. + miR.494 + miR.518c + miR.660 + miR.338 | 0,81 | 0,41295407 5 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.660 | 0,81 | 0,27763172 8 |
| miR.558 + miR.609 + miR.603 + miR.520f + miR.220a + miR.519b + miR.130a + miR.26a + miR.29a + miR.362 + miR.369.3p + miR.376a. + miR.518c + miR.660 + miR.338 | 0,81 | 0,45082842 9 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.660 | 0,81 | 0,28836596 5 |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 | 0,81 | 0,2350529 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.660 | 0,81 | 0,21540011 6 |
| miR.558 + miR.609 + miR.603 + miR.520f + miR.220a + miR.519b + miR.130a + miR.26a + miR.29a + miR.369.3p + miR.376a. + miR.518c + miR.639 + miR.660 + miR.338 | 0,81 | 0,19469033 8 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.660 | 0,81 | 0,30961795 9 |
| miR.558 + miR.609 + miR.520f + miR.220a + miR.519b + miR.130a + miR.26a + miR.29a + miR.362 + miR.369.3p + miR.376a. + miR.494 + miR.518c + miR.660 + miR.338 | 0,81 | 0,16129346 7 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.660 | 0,81 | 0,21528266 7 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.338 | 0,81 | 0,10211321 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.338 | 0,81 | 0,09339476 4 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.338 | 0,81 | 0,08156321 5 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.338 | 0,81 | 0,09391331 1 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.338 | 0,81 | 0,10776340 7 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.660 | 0,81 | 0,32796379 1 |
| miR.558 + miR.609 + miR.603 + miR.520f + miR.519b + miR.130a + miR.26a + miR.29a + miR.369.3p + miR.376a. + miR.494 + miR.518c + miR.639 + miR.660 + miR.338 | 0,81 | 0,11880583 6 |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.338 | 0,81 | 0,03601632 3 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 | 0,81 | 0,15016789 8 |
| miR.558 + miR.609 + miR.520f + miR.220a + miR.519b + miR.130a + miR.26a + miR.29a + miR.369.3p + miR.376a. + miR.494 + miR.518c + miR.639 + miR.660 + miR.338 | 0,81 | 0,33108117 2 |
| miR.558 + miR.609 + miR.603 + miR.520f + miR.519b + miR.130a + miR.26a + miR.29a + miR.362 + miR.369.3p + miR.376a. + miR.494 + miR.518c + miR.660 + miR.338 | 0,81 | 0,41319679 1 |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.660 | 0,81 | 0,03822282 |
| miR.558 + miR.609 + miR.603 + miR.220a + miR.519b + miR.130a + miR.26a + miR.29a + miR.362 + miR.376a. + miR.494 + miR.518c + miR.639 + miR.660 + miR.338 | 0,81 | 0,10992064 7 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.338 | 0,81 | 0,12719531 1 |
| miR.558 + miR.609 + miR.603 + miR.520f + miR.220a + miR.519b + miR.130a + miR.26a + miR.29a + miR.376a. + miR.494 + miR.518c + miR.639 + miR.660 + miR.338 | 0,81 | 0,11221176 2 |
| miR.558 + miR.609 + miR.603 + miR.520f + miR.220a + miR.519b + miR.130a + miR.29a + miR.362 + miR.376a. + miR.494 + miR.518c + miR.639 + miR.660 + miR.338 | 0,81 | 0,12574555 5 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.660 | 0,81 | 0,37661079 3 |
| miR.558 + miR.609 + miR.603 + miR.520f + miR.220a + miR.519b + miR.26a + miR.29a + miR.362 + miR.376a. + miR.494 + miR.518c + miR.639 + miR.660 + miR.338 | 0,81 | 0,11235135 3 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.338 | 0,81 | 0,09679765 7 |
| miR.558 + miR.609 + miR.603 + miR.520f + miR.220a + miR.519b + miR.130a + miR.26a + miR.29a + miR.362 + miR.376a. + miR.494 + miR.518c + miR.660 + miR.338 | 0,81 | 0,14969933 9 |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.338 | 0,81 | 0,06398421 2 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.660 | 0,81 | 0,13144661 7 |
| miR.558 + miR.609 + miR.603 + miR.520f + miR.220a + miR.130a + miR.26a + miR.29a + miR.362 + miR.376a. + miR.494 + miR.518c + miR.639 + miR.660 + miR.338 | 0,81 | 0,01068059 5 |
| miR.609 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0,81 | 0,14424039 5 |
| miR.609 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0,81 | 0,13282591 4 |
| miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,81 | 0,16150016 2 |
| miR.558 + miR.609 + miR.520f + miR.220a + miR.519b + miR.130a + miR.26a + miR.29a + miR.362 + miR.376a. + miR.494 + miR.518c + miR.639 + miR.660 + miR.338 | 0,81 | 0,17520392 2 |
| miR.558 + miR.609 + miR.603 + miR.520f + miR.220a + miR.519b + miR.130a + miR.362 + miR.369.3p + miR.376a. + miR.494 + miR.518c + miR.639 + miR.660 + miR.338 | 0,81 | 0,16756380 6 |
| miR.609 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0,81 | 0,39306794 3 |
| miR.558 + miR.609 + miR.603 + miR.520f + miR.220a + miR.130a + miR.26a + miR.362 + miR.369.3p + miR.376a. + miR.494 + miR.518c + miR.639 + miR.660 + miR.338 | 0,81 | 0,14989393 7 |
| miR.609 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,34632805 |
| miR.558 + miR.609 + miR.603 + miR.520f + miR.220a + miR.519b + miR.130a + miR.26a + miR.362 + miR.369.3p + miR.376a. + miR.518c + miR.639 + miR.660 + miR.338 | 0,81 | 0,14584867 5 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.338 | 0,81 | 0,16786015 3 |
| miR.558 + miR.609 + miR.603 + miR.520f + miR.220a + miR.519b + miR.26a + miR.362 + miR.369.3p + miR.376a. + miR.494 + miR.518c + miR.639 + miR.660 + miR.338 | 0,81 | 0,12681659 4 |
| miR.609 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0,81 | 0,37047733 2 |
| miR.558 + miR.609 + miR.603 + miR.520f + miR.519b + miR.130a + miR.26a + miR.362 + miR.369.3p + miR.376a. + miR.494 + miR.518c + miR.639 + miR.660 + miR.338 | 0,81 | 0,22825136 9 |
| miR.558 + miR.609 + miR.603 + miR.520f + miR.220a + miR.519b + miR.130a + miR.26a + miR.29a + miR.362 + miR.376a. + miR.518c + miR.639 + miR.660 + miR.338 | 0,81 | 0,07319244 5 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0,81 | 0,11654659 5 |
| miR.609 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0,81 | 0,12213319 2 |
| miR.558 + miR.609 + miR.603 + miR.520f + miR.220a + miR.130a + miR.26a + miR.29a + miR.362 + miR.369.3p + miR.376a. + miR.494 + miR.639 + miR.660 + miR.338 | 0,81 | 0,21979618 2 |
| miR.609 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,29535657 6 |
| miR.558 + miR.609 + miR.603 + miR.520f + miR.220a + miR.519b + miR.130a + miR.29a + miR.362 + miR.369.3p + miR.376a. + miR.494 + miR.639 + miR.660 + miR.338 | 0,81 | 0,22575434 6 |
| miR.558 + miR.609 + miR.603 + miR.520f + miR.519b + miR.130a + miR.26a + miR.29a + miR.362 + miR.376a. + miR.494 + miR.518c + miR.639 + miR.660 + miR.338 | 0,81 | 0,04511966 8 |
| miR.609 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0,81 | 0,21255894 2 |
| miR.558 + miR.609 + miR.520f + miR.220a + miR.519b + miR.130a + miR.26a + miR.29a + miR.362 + miR.369.3p + miR.376a. + miR.494 + miR.639 + miR.660 + miR.338 | 0,81 | 0,26387376 9 |
| miR.558 + miR.609 + miR.603 + miR.520f + miR.519b + miR.130a + miR.26a + miR.29a + miR.362 + miR.369.3p + miR.376a. + miR.494 + miR.639 + miR.660 + miR.338 | 0,81 | 0,26345260 2 |
| miR.558 + miR.609 + miR.603 + miR.520f + miR.220a + miR.519b + miR.26a + miR.29a + miR.362 + miR.369.3p + miR.376a. + miR.494 + miR.639 + miR.660 + miR.338 | 0,81 | 0,31341978 |
| miR.558 + miR.609 + miR.520f + miR.220a + miR.519b + miR.130a + miR.26a + miR.362 + miR.369.3p + miR.376a. + miR.494 + miR.518c + miR.639 + miR.660 + miR.338 | 0,81 | 0,19833790 1 |
| miR.558 + miR.609 + miR.603 + miR.520f + miR.220a + miR.519b + miR.130a + miR.26a + miR.369.3p + miR.376a. + miR.494 + miR.518c + miR.639 + miR.660 + miR.338 | 0,81 | 0,17267101 9 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.660 | 0,81 | 0,01833704 7 |
| miR.558 + miR.609 + miR.603 + miR.520f + miR.220a + miR.519b + miR.130a + miR.26a + miR.29a + miR.362 + miR.369.3p + miR.376a. + miR.494 + miR.660 + miR.338 | 0,81 | 0,36498723 5 |
| miR.558 + miR.609 + miR.603 + miR.220a + miR.519b + miR.130a + miR.26a + miR.362 + miR.369.3p + miR.376a. + miR.494 + miR.518c + miR.639 + miR.660 + miR.338 | 0,8 | 0,07090830 9 |
| miR.558 + miR.609 + miR.603 + miR.520f + miR.220a + miR.519b + miR.130a + miR.26a + miR.29a + miR.362 + miR.369.3p + miR.376a. + miR.639 + miR.660 + miR.338 | 0,8 | 0,30185850 2 |
| miR.609 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0,8 | 0,17291455 9 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.338 | 0,8 | 0,08524150 4 |
| miR.558 + miR.609 + miR.603 + miR.520f + miR.220a + miR.519b + miR.130a + miR.26a + miR.362 + miR.369.3p + miR.376a. + miR.494 + miR.518c + miR.660 + miR.338 | 0,8 | 0,27591258 2 |
| miR.609 + miR.603 + miR.520f + miR.220a + miR.519b + miR.130a + miR.26a + miR.29a + miR.362 + miR.376a. + miR.494 + miR.518c + miR.639 + miR.660 + miR.338 | 0,8 | 0,04872587 9 |
| miR.609 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0,8 | 0,34024859 1 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.338 | 0,8 | 0,02394564 8 |
| miR.558 + miR.609 + miR.603 + miR.220a + miR.519b + miR.130a + miR.26a + miR.29a + miR.362 + miR.369.3p + miR.376a. + miR.494 + miR.639 + miR.660 + miR.338 | 0,8 | 0,11951914 7 |
| miR.558 + miR.609 + miR.603 + miR.520f + miR.220a + miR.519b + miR.130a + miR.26a + miR.362 + miR.376a. + miR.494 + miR.518c + miR.639 + miR.660 + miR.338 | 0,8 | 0,10336376 5 |
| miR.558 + miR.609 + miR.603 + miR.520f + miR.220a + miR.519b + miR.130a + miR.26a + miR.29a + miR.369.3p + miR.376a. + miR.494 + miR.639 + miR.660 + miR.338 | 0,8 | 0,10512413 5 |
| miR.558 + miR.609 + miR.603 + miR.520f + miR.220a + miR.519b + miR.130a + miR.26a + miR.362 + miR.369.3p + miR.376a. + miR.494 + miR.639 + miR.660 + miR.338 | 0,8 | 0,19821761 3 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.660 | 0,8 | 0,00876563 8 |
| miR.558 + miR.609 + miR.603 + miR.220a + miR.519b + miR.130a + miR.26a + miR.29a + miR.362 + miR.369.3p + miR.494 + miR.518c + miR.639 + miR.660 + miR.338 | 0,8 | 0,02127004 3 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.660 | 0,8 | 0,01027400 1 |
| miR.558 + miR.609 + miR.603 + miR.520f + miR.220a + miR.519b + miR.130a + miR.29a + miR.362 + miR.369.3p + miR.494 + miR.518c + miR.639 + miR.660 + miR.338 | 0,8 | 0,00871955 7 |
| miR.558 + miR.609 + miR.603 + miR.520f + miR.220a + miR.130a + miR.26a + miR.29a + miR.362 + miR.369.3p + miR.494 + miR.518c + miR.639 + miR.660 + miR.338 | 0,8 | 0,01205239 9 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.338 | 0,8 | 0,02236004 1 |
| miR.558 + miR.609 + miR.603 + miR.520f + miR.220a + miR.519b + miR.130a + miR.26a + miR.29a + miR.362 + miR.369.3p + miR.518c + miR.639 + miR.660 + miR.338 | 0,8 | 0,01132788 7 |
| miR.558 + miR.609 + miR.603 + miR.520f + miR.220a + miR.519b + miR.130a + miR.26a + miR.29a + miR.362 + miR.376a. + miR.494 + miR.639 + miR.660 + miR.338 | 0,8 | 0,07566166 8 |
| miR.558 + miR.609 + miR.603 + miR.520f + miR.220a + miR.519b + miR.130a + miR.26a + miR.29a + miR.362 + miR.369.3p + miR.494 + miR.518c + miR.660 + miR.338 | 0,8 | 0,02446797 3 |
| miR.558 + miR.609 + miR.603 + miR.520f + miR.220a + miR.519b + miR.130a + miR.26a + miR.29a + miR.369.3p + miR.494 + miR.518c + miR.639 + miR.660 + miR.338 | 0,8 | 0,02081330 6 |
| miR.558 + miR.609 + miR.603 + miR.520f + miR.220a + miR.519b + miR.130a + miR.26a + miR.29a + miR.362 + miR.494 + miR.518c + miR.639 + miR.660 + miR.338 | 0,8 | 0,01599405 |
| miR.558 + miR.609 + miR.603 + miR.520f + miR.220a + miR.519b + miR.26a + miR.29a + miR.362 + miR.369.3p + miR.494 + miR.518c + miR.639 + miR.660 + miR.338 | 0,8 | 0,01434367 4 |
| miR.558 + miR.609 + miR.603 + miR.520f + miR.519b + miR.130a + miR.26a + miR.29a + miR.362 + miR.369.3p + miR.494 + miR.518c + miR.639 + miR.660 + miR.338 | 0,8 | 0,00378449 1 |
| miR.558 + miR.609 + miR.520f + miR.220a + miR.519b + miR.130a + miR.26a + miR.29a + miR.362 + miR.369.3p + miR.494 + miR.518c + miR.639 + miR.660 + miR.338 | 0,8 | 0,00477199 4 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.338 | 0,79 | 0,02585565 8 |
| miR.558 + miR.609 + miR.603 + miR.520f + miR.220a + miR.519b + miR.130a + miR.26a + miR.362 + miR.369.3p + miR.494 + miR.518c + miR.639 + miR.660 + miR.338 | 0,79 | 0,00384726 7 |
| miR.609 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0,79 | 0,00639978 7 |
| miR.558 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0,79 | 0,05275078 8 |
| miR.558 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0,79 | 0,04517926 6 |
| miR.558 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0,79 | 0,02591738 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0,79 | 0,04349600 8 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + R.338 | 0,79 | 0,03485767 2 |
| miR.558 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0,79 | 0,04018239 8 |
| miR.558 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0,79 | 0,05514381 |
| miR.558 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0,79 | 0,03572174 4 |
| miR.558 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0,79 | 0,03721832 9 |
| miR.558 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,78 | 0,02064292 3 |
| miR.558 + miR.603 + miR.520f + miR.220a + miR.519b + miR.130a + miR.26a + miR.29a + miR.362 + miR.376a. + miR.494 + miR.518c + miR.639 + miR.660 + miR.338 | 0,78 | 0,01405088 4 |
| miR.558 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0,78 | 0,02130120 8 |
| miR.558 + miR.609 + miR.603 + miR.520f + miR.220a + miR.519b + miR.130a + miR.26a + miR.29a + miR.362 + miR.369.3p + miR.494 + miR.639 + miR.660 + miR.338 | 0,78 | 0,00588310 5 |
| miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,78 | 0,01330627 4 |
| miR.558 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0,78 | 0,01784349 9 |
| miR.558 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,77 | 0,01028608 3 |
| miR.558 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0,76 | 0,00164717 |

| **Table 16: clusters of 16 miRNAs** | | |
|---|---|---|
| **Cluster of 16 miRs** | **c_ tau level** | **pv compared to top signa ture miR. 558 + miR. 609 + miR. 603 + miR. 520f + miR. 220a + miR. 130a + miR. 26a + miR. 29a + miR. 362 + miR. 369.3 p + miR. 376a* + miR. 494 + miR. 518c + miR. 639 + miR. 660 + miR. 338** |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0, 82 | NaN |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0, 82 | 0,34679455 4 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0, 82 | 0,37405084 1 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0, 82 | 0,31028963 5 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0, 82 | 0,36610205 4 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0, 82 | 0,15814461 3 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0, 82 | 0,44507839 3 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0, 81 | 0,10950789 4 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0, 81 | 0,20065095 2 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0, 81 | 0,21778575 3 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.338 | 0, 81 | 0,06650593 9 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.660 + miR.338 | 0, 81 | 0,07388535 7 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0, 81 | 0,30347294 6 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0, 81 | 0,18501505 3 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0, 81 | 0,21667920 9 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0, 8 | 0,00887054 6 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0, 79 | 0,03881643 5 |

| **Table 17: best clusters of miRNAs** | | | |
|---|---|---|---|
| **Comparaison between the top clusters of miRs** | **c_tau level** | **pv compared to UICC** | **pv compared to top signature miR. 558 + miR. 609 + miR. 603 + miR. 520f + miR. 220a + miR. 519b + miR. 130a + miR. 26a + miR. 29a + miR. 362 + miR. 369. 3p + miR. 376a .+ miR. 494 + miR. 518c + miR.** |
| miR.558 + miR.609 + miR.603 + miR.520f + miR.220a + miR.519b + miR.130a + miR.26a + miR.29a + miR.362 + miR.369.3p + miR.376a.+ miR.494 + miR.518c + miR.639 + miR.660 + miR.338 | 0,82 | 3,89 631E -09 | NaN |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,82 | 5,31 322E -09 | 0,273786 656 |
| miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 | 0,82 | 1,07 178E -09 | 0,469592 99 |
| miR.558 + miR.609 + miR.603 + miR.520f + miR.130a + miR.29a + miR.362 + miR.369.3p + miR.376a. + miR.494 + miR.518c + miR.639 + miR.660 + miR.338 | 0,82 | 1,57 648E -09 | 0,496622 615 |
| miR.558 + miR.609 + miR.520f + miR.130a + miR.29a + miR.362 + miR.369.3p + miR.376a. + miR.494 + miR.518c + miR.639 + miR.660 + miR.338 | 0,82 | 3,57 144E -09 | 0,358285 772 |
| miR.558 + miR.609 + miR.520f + miR.220a + miR.29a + miR.362 + miR.369.3p + miR.376a. + miR.518c + miR.639 + miR.660 + miR.338 | 0,81 | 4,82 435E -09 | 0,231087 673 |
| miR.558 + miR.609 + miR.520f + miR.29a + miR.362 + miR.369.3p + miR.376a. + miR.518c + miR.639 + miR.660 + miR.338 | 0,81 | 3,58 012E -09 | 0,455646 227 |
| miR.558 + miR.609 + miR.603 + miR.220a + miR.26a + miR.29a + miR.369.3p + miR.376a. + miR.518c + miR.660 | 0,81 | 2,32 944E -08 | 0,062628 078 |
| miR.558 + miR.609 + miR.603 + miR.220a + miR.26a + miR.29a + miR.369.3p + miR.376a. + miR.518c | 0,81 | 1,12 929E -08 | 0,021753 942 |
| miR.558 + miR.609 + miR.603 + miR.220a + miR.29a + miR.369.3p + miR.376a. + miR.518c | 0,81 | 1,05 401E -08 | 0,003784 432 |
| miR.558 + miR.609 + miR.220a + miR.29a + miR.369.3p + miR.376a. + miR.518c | 0,8 | 1,70 664E -07 | 0,016562 471 |
| miR.558 + miR.609 + miR.220a + miR.29a + miR.376a. + miR.518c | 0,8 | 2,75 189E -07 | 0,005183 149 |
| miR.558 + miR.609 + miR.29a + miR.376a. + miR.518c | 0,79 | 8,33 665E -07 | 0,007189 529 |
| miR.609 + miR.29a + miR.376a. + miR.518c | 0,77 | 0,00 0258 704 | 0,001288 932 |
| miR.558 + miR.609 + miR.518c | 0,76 | 3,7808E-05 | 8,30546E -05 |
| miR.603 + miR.518c | 0,73 | 1,38 325E -05 | 8,07086E -06 |
| miR.609 | 0,66 | 0,07 1282 935 | 7,46688E -10 |
| UICC | 0,59 | NaN | 3,89631E -09 |

### REFERENCES:

Throughout this application, various references describe the state of the art to which this invention pertains.
Galon J, Costes A, Sanchez-Cabo F, Kirilovsky A, Mlecnik B, Lagorce-Pagès C, Tosolini M, Camus M, Berger A, Wind P, Zinzindohoué F, Bruneval P, Cugnenc PH, Trajanoski Z, Fridman WH, Pages F. Type, density, and location of immune cells within human colorectal tumors predict clinical outcome. Science. 2006 Sep 29;313(5795):1960-4.
Harrell FE Jr, Lee KL, Mark DB.Multivariable prognostic models: issues in developing models, evaluating assumptions and adequacy, and measuring and reducing errors. Stat Med. 1996 Feb 28;15(4):361-87. Review.
Heagerty PJ, Zheng Y. Survival model predictive accuracy and ROC curves. Biometrics. 2005 Mar;61(1):92-105.
Pencina MJ, D'Agostino RB Sr, D'Agostino RB Jr, Vasan RS. Evaluating the added predictive ability of a new marker: from area under the ROC curve to reclassification and beyond. Stat Med. 2008 Jan 30;27(2): 157-72; discussion 207-12.

## Claims

1. A method for predicting the outcome of a colorectal cancer in a patient comprising a step consisting of determining the expression level of a miRNA cluster in a tumor sample obtained from said patient, wherein said miRNA cluster comprises miR.609.

2. The method according to claim 1 wherein said miRNA cluster consists in the combination miR.558 + miR.609 + miR.518c.

3. The method according to claim 1 wherein said miRNA cluster consists in the combination miR.609 + miR.29a + miR.376a. + miR.518c.

4. The method according to claim 1 wherein said miRNA cluster consists in the combination miR.558 + miR.609 + miR.29a + miR.376a. + miR.518c.

5. The method according to claim 1 wherein said miRNA cluster consists in the combination miR.558 + miR.609 + miR.220a + miR.29a + miR.376a. + miR.518c.

6. The method according to claim 1 wherein said miRNA cluster consists in the combination miR.558 + miR.609 + miR.220a + miR.29a + miR.369.3p + miR.376a. + miR.518c.

7. The method according to claim 1 wherein said miRNA cluster consists in the combination miR.558 + miR.609 + miR.603 + miR.220a + miR.26a + miR.29a + miR.369.3p + miR.376a. + miR.518c.

8. The method according to claim 1 wherein said miRNA cluster consists in the combination miR.558 + miR.609 + miR.603 + miR.220a + miR.26a + miR.29a + miR.369.3p + miR.376a. + miR.518c + miR.660.

9. The method according to claim 1 wherein said miRNA cluster consists in the combination miR.558 + miR.609 + miR.520f + miR.29a + miR.362 + miR.369.3p + miR.376a. + miR.518c + miR.639 + miR.660 + miR.338.

10. The method according to claim 1 wherein said miRNA cluster consists in the combination miR.558 + miR.609 + miR.520f + miR.220a + miR.29a + miR.362 + miR.369.3p + miR.376a. + miR.518c + miR.639 + miR.660 + miR.338.

11. The method according to claim 1 wherein said miRNA cluster consists in the combination miR.558 + miR.609 + miR.520f + miR.130a + miR.29a + miR.362 + miR.369.3p + miR.376a. + miR.494 + miR.518c + miR.639 + miR.660 + miR.338.

12. The method according to claim 1 wherein said miRNA cluster consists in the combination miR.558 + miR.609 + miR.603 + miR.520f + miR.130a + miR.29a + miR.362 + miR.369.3p + miR.376a. + miR.494 + miR.518c + miR.639 + miR.660 + miR.338.

13. The method according to claim 1 wherein said miRNA cluster consists in the combination miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338.

14. The method according to claim 1 wherein said miRNA cluster consists in the combination miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338.

15. The method according to claim 1 wherein said miRNA cluster consists in the combination miR.558 + miR.609 + miR.603 + miR.520f + miR.220a + miR.519b + miR.130a + miR.26a + miR.29a + miR.362 + miR.369.3p + miR.376a*+ miR.494 + miR.518c + miR.639 + miR.660 + miR.338.

16. The method according to any of claims 1 to 15 which further comprises a step consisting of comparing the expression level of said miRNA cluster determined in the sample of the patient with a reference expression level of said miRNA cluster.

## Patentansprüche

1. Ein Verfahren zur Vorhersage des Verhaltens von kolorektalem Krebs in einem Patienten, umfassend einen Schritt bestehend aus der Bestimmung des Expressionsniveaus eines miRNA-Clusters in einer Tumorprobe, die von einem Patienten erhalten wurde, wobei der miRNA-Cluster miR.609 umfasst.

2. Das Verfahren nach Anspruch 1, wobei das miRNA-Cluster aus einer Kombination von miR.558 + miR.609 + miR.518c besteht.

3. Das Verfahren nach Anspruch 1, wobei das miRNA-Cluster aus einer Kombination von miR.609 + miR.29a + miR.376a. + miR.518c besteht.

4. Das Verfahren nach Anspruch 1, wobei das miRNA-Cluster aus einer Kombination von miR.558 + miR.609 + miR.29a + miR.376a. + miR.518c besteht.

5. Das Verfahren nach Anspruch 1, wobei das miRNA-Cluster aus einer Kombination von miR.558 + miR.609 + miR.220a + miR.29a + miR.376a. + miR.518c besteht.

6. Das Verfahren nach Anspruch 1, wobei das miRNA-Cluster aus einer Kombination von miR.558 + miR.609 + miR.220a + miR.29a + miR.369.3p + miR.376a. + miR.518c besteht.

7. Das Verfahren nach Anspruch 1, wobei das miRNA-Cluster aus einer Kombination von miR.558 + miR.609 + miR.603 + miR.220a + miR.26a + miR.29a + miR.369.3p + miR.376a. + miR.518c besteht.

8. Das Verfahren nach Anspruch 1, wobei das miRNA-Cluster aus einer Kombination von miR.558 + miR.609 + miR.603 + miR.220a + miR.26a + miR.29a + miR.369.3p + miR.376a. + miR.518c + miR.660 besteht.

9. Das Verfahren nach Anspruch 1, wobei das miRNA-Cluster aus einer Kombination von miR.558 + miR.609 + miR.520f + miR.29a + miR.362 + miR.369.3p + miR.376a. + miR.518c + miR.639 + miR.660 + miR.338 besteht.

10. Das Verfahren nach Anspruch 1, wobei das miRNA-Cluster aus einer Kombination von miR.558 + miR.609 + miR.520f + miR.220a + miR.29a + miR.362 + miR.369.3p + miR.376a. + miR.518c + miR.639 + miR.660 + miR.338 besteht.

11. Das Verfahren nach Anspruch 1, wobei das miRNA-Cluster aus einer Kombination von miR.558 + miR.609 + miR.520f + miR.130a + miR.29a + miR.362 + miR.369.3p + miR.376a. + miR.494 + miR.518c + miR.639 + miR.660 + miR.338 besteht.

12. Das Verfahren nach Anspruch 1, wobei das miRNA-Cluster aus einer Kombination von miR.558 + miR.609 + miR.603 + miR.520f + miR.130a + miR.29a + miR.362 + miR.369.3p + miR.376a. + miR.494 + miR.518c + miR.639 + miR.660 + miR.338 besteht.

13. Das Verfahren nach Anspruch 1, wobei das miRNA-Cluster aus einer Kombination von miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 besteht.

14. Das Verfahren nach Anspruch 1, wobei das miRNA-Cluster aus einer Kombination von miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 besteht.

15. Das Verfahren nach Anspruch 1, wobei das miRNA-Cluster aus einer Kombination von miR.558 + miR.609 + miR.603 + miR.520f + miR.220a + miR.519b + miR.130a + miR.26a + miR.29a + miR.362 + miR.369.3p + miR.376a*+ miR.494 + miR.518c + miR.639 + miR.660 + miR.338 besteht.

16. Das Verfahren nach einem der Ansprüche 1 bis 15, welches weiterhin einen Schritt umfasst, bestehend aus dem Vergleich des Expressionsniveaus des miRNA-Clusters, wie in der Probe des Patienten bestimmt, mit einem Referenzexpressionsniveau dieses miRNA-Clusters.

## Revendications

1. Procédé permettant de prédire l'issue d'un cancer colorectal chez un patient comprenant une étape consistant à déterminer le taux d'expression d'un groupe de miARN dans un échantillon tumoral obtenu à partir dudit patient, où ledit groupe de miARN comprend le miR.609.

2. Procédé selon la revendication 1, dans lequel ledit groupe de miARN est constitué de la combinaison miR.558 + miR.609 + miR.518c.

3. Procédé selon la revendication 1, dans lequel ledit groupe de miARN est constitué de la combinaison miR.609 + miR.29a + miR.376a. + miR.518c.

4. Procédé selon la revendication 1, dans lequel ledit groupe de miARN est constitué de la combinaison miR.558 + miR.609 + miR.29a + miR.376a. + miR.518c.

5. Procédé selon la revendication 1, dans lequel ledit groupe de miARN est constitué de la combinaison miR.558 + miR.609 + miR.220a + miR.29a + miR.376a. + miR.518c.

6. Procédé selon la revendication 1, dans lequel ledit groupe de miARN est constitué de la combinaison miR.558 + miR.609 + miR.220a + miR.29a + miR.369.3p + miR.376a. + miR.518c.

7. Procédé selon la revendication 1, dans lequel ledit groupe de miARN est constitué de la combinaison miR.558 + miR.609 + miR.603 + miR.220a + miR.26a + miR.29a + miR.369.3p + miR.376a. + miR.518c.

8. Procédé selon la revendication 1, dans lequel ledit groupe de miARN est constitué de la combinaison miR.558 + miR.609 + miR.603 + miR.220a + miR.26a + miR.29a + miR.369.3p + miR.376a. + miR.518c + miR.660.

9. Procédé selon la revendication 1, dans lequel ledit groupe de miARN est constitué de la combinaison miR.558 + miR.609 + miR.520f + miR.29a + miR.362 + miR.369.3p + miR.376a. + miR.518c + miR.639 + miR.660 + miR.338.

10. Procédé selon la revendication 1, dans lequel ledit groupe de miARN est constitué de la combinaison miR.558 + miR.609 + miR.520f + miR.220a + miR.29a + miR.362 + miR.369.3p + miR.376a. + miR.518c + miR.639 + miR.660 + miR.338.

11. Procédé selon la revendication 1, dans lequel ledit groupe de miARN est constitué de la combinaison miR.558 + miR.609 + miR.520f + miR.130a + miR.29a + miR.362 + miR.369.3p + miR.376a. + miR.494 + miR.518c + miR.639 + miR.660 + miR.338.

12. Procédé selon la revendication 1, dans lequel ledit groupe de miARN est constitué de la combinaison miR.558 + miR.609 + miR.603 + miR.520f + miR.130a + miR.29a + miR.362 + miR.369.3p + miR.376a. + miR.494 + miR.518c + miR.639 + miR.660 + miR.338.

13. Procédé selon la revendication 1, dans lequel ledit groupe de miARN est constitué de la combinaison miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338.

14. Procédé selon la revendication 1, dans lequel ledit groupe de miARN est constitué de la combinaison miR.558 + miR.609 + miR.603 + miR.520f +miR.220a + miR.130a + miR.26a + miR.29a + miR.362 + miR.369.3p + miR.376a. + miR.494 + miR.518c + miR.639 + miR.660 + miR.338.

15. Procédé selon la revendication 1, dans lequel ledit groupe de miARN est constitué de la combinaison miR.558 + miR.609 + miR.603 + miR.520f +miR.220a + miR.519b + miR.130a + miR.26a + miR.29a + miR.362 + miR.369.3p + miR.376a* + miR.494 + miR.518c + miR.639 + miR.660 + miR.338.

16. Procédé selon l'une quelconque des revendications 1 à 15 qui comprend en outre une étape consistant à comparer le taux d'expression dudit groupe de miARN déterminé dans l'échantillon du patient à un taux d'expression de référence dudit groupe de miARN.
